# EUROPEAN PATENT APPLICATION

(11) **EP 4 631 942 A1**
(43) Date of publication of application: **15.10.2025**
(21) Application number: 23879241.0
(22) Date of filing: 23.10.2023
(51) Int. Cl.: C07D 471/04, C07D 519/00, C07D 239/94, C07D 239/95, A61K 31/517, A61K 31/519, A61P 35/00

(54) **PAN-KRAS DEGRADING AGENT, AND PREPARATION METHOD THEREFOR AND USE THEREOF**

(30) Priority: 21.10.2022 CN 202211297444; 20.01.2023 CN 202310079075; 11.07.2023 CN 202310841974
(71) Applicant: Leadingtac Pharmaceutical (Shaoxing) Co., Ltd., Shaoxing, Zhejiang 312030 (CN)
(72) Inventor: YE, Zhengqing, Shanghai 201203 (CN); FENG, Yan, Shanghai 201203 (CN); DING, Chenli, Shanghai 201203 (CN)
(74) Representative: Murgitroyd & Company
(86) International application number: PCT/CN2023/125974
(87) International publication number: WO 2024/083256

(57) **Abstract**

Provided in the present invention are a pan-KRAS degrading agent, and a preparation method therefor and the use thereof. Specifically, disclosed in the present invention are a small molecule compound for targeted degradation of the pan-KRAS protein, and a preparation method therefor and the use thereof as a therapeutic agent for preventing and/or treating cancers. The compound of the present invention can effectively degrade and/or inhibit the pan-KRAS protein in cells, and can be used in the preparation of a drug for treating and/or preventing relevant diseases or conditions mediated by pan-KRAS.

## Description

The present application claims priority to the Chinese Patent Application 2022112974447 filed on Oct. 21, 2022, the Chinese Patent Application 2023100790752 filed on Jan. 20, 2023, and the Chinese Patent Application 2023108419741 filed on Jul. 11, 2023, which are incorporated herein by reference in their entirety.

### TECHNICAL FIELD

The present disclosure relates to the technical field of pharmaceuticals, and particularly relates to a pan-KRAS degrader, a preparation method therefor and use thereof as a therapeutic agent preventing and/or treating cancer.

### BACKGROUND

RAS (rat sarcoma) is one of the oncogenes with the highest mutation rate in tumors, and its mutations are present in about 30% of human malignancies. The RAS family includes KRAS, NRAS, and HRAS, among which KRAS (kirsten rat sarcoma viral oncogene) is more prone to mutations compared to the other two RAS subtypes. KRAS mutations account for approximately 85% of all RAS mutations and are particularly common in solid tumors (Cancer Res. 2020, 80, 2969-2974). The mutation of the KRAS gene exists in 30%-40% of colorectal cancers, 90% of pancreatic cancers, and 15%-20% of lung cancers. After KRAS is activated, it regulates the functions such as the proliferation, differentiation, and survival of cells through downstream signaling pathways such as RAF-MEK-ERK and PI3K-AKT-mTOR. After the mutation of the KRAS gene, the protein is continuously activated, resulting in continuous activation of downstream signaling pathways, and thus promoting tumorigenesis (Nat. Rev. Cancer 2015, 15, 290-301). Therefore, KRAS is an important therapeutic target in cancer treatment, with efforts focused on targeting the KRAS protein itself, its post-translational modifications, membrane localization, protein-protein interactions, and the downstream signaling pathways of RAS.

Due to the ultra-high affinity of KRAS for binding with GTP or GDP (in the picomolar concentration range) and the smooth surface of the RAS protein, which lacks ideal binding sites for small molecules, the development of competitive inhibitors directly targeting RAS proteins is considered highly challenging (Nat. Rev. Drug Discov. 2020, 19, 533-552).

At present, according to action modes of inhibitors, KRAS inhibitors (Cancer Discov. 2022, 12, 924-937) may be:
1) Inhibitors directly targeting KRAS, such as KRAS G12C, KRAS G12D, KRAS G12R (J. Am. Chem. Soc. 2022, 144, 35, 15916-15921), and KRAS G12S (Nat. Chem. Biol. 2022 Jul 21. doi: 10.1038/s41589-022-01065-9); or
2) Inhibitors acting indirectly on KRAS, such as SHP2 (SHP 099, Nature 2016, 535, 148-152 & J. Med. Chem. 2016, 59, 7773-7782; RMC4550, Nat. Cell Biol. 2018, 20, 1064-1073; TNO155, J. Med. Chem. 2020, 63, 22, 13578-13594; RMC4630, WO 2021142026A1; JAB-3068, WO2017211303A1, and the like), SOS1 inhibitors (Bay-293, Proc. Natl. Acad. Sci. U S A. 2019, 116, 2551-2560; BI-3406, Cancer Discov. 2021, 11, 142-157; MRTX0902, J. Med. Chem. 2022, 65, 9678-9690, and the like), and KRAS (on) (WO2021091982A1, WO2022060836A1).

In recent years, with the continuous advancement in KRAS research, significant progress has been made in the development of KRAS inhibitors (Cancer Discov. 2022, 12, 924-937), and the specific KRAS-G12C inhibitor is the first to break through.

In the design of KRAS-G12C inhibitors, the cysteine residue at codon 12 is primarily utilized due to its ability to form covalent bonds. Covalent targeting of this cysteine residue has become a significant breakthrough in the development of KRAS drugs. Shokat discovered a new allosteric binding pocket in Switch-II, called the Switch-II pocket, and developed the first batch of KRAS-G12C covalent inhibitors (Nature 2013, 503, 548-551; Nat. Rev. Drug Discov. 2016, 15, 771-785). On May 28, 2021, the FDA granted accelerated approval to LumaKRAS (Sotorasib, AMG510) (Nature 2019, 575, 217-223; J. Med. Chem. 2020, 63, 52-65), developed by Amgen, for the treatment of patients with non-small cell lung cancer harboring the KRAS-G12C mutation (N. Engl. J. Med. 2021, 384, 2371-2381). Currently, multiple small molecule drugs targeting KRAS-G12C are undergoing clinical development worldwide. On Feb. 16, 2022, the FDA accepted the New Drug Application submitted by Mirati Therapeutics for its second KRAS-G12C inhibitor, Adagrasib (MRTX849) (Cancer Discov. 2020, 10, 54-71 & J. Med. Chem. 2020, 63, 6679-6693). This inhibitor is intended for the treatment of patients with non-small cell lung cancer harboring the KRAS G12C mutation who have received at least one systemic therapy *(*N. Engl. J. Med. 2022, 387, 120-131). Adagrasib is expected to become the second KRAS-targeted drug worldwide.

Unlike the KRAS G12C mutation, the most common mutation in patients with non-small cell lung cancer (NSCLC), KRAS G12D is the most common mutation in colorectal cancer and pancreatic cancer. The KRAS G12C mutant contains a cysteine (Cys) residue at codon 12, which facilitates the formation of covalent bonds. This structural characteristic enables the covalent binding of the mutant to small molecule inhibitors. In contrast, the KRAS G12D mutant has aspartic acid (Asp) at codon 12, which precludes the use of this covalent binding strategy.

The activation of KRAS-G12D is primarily mediated by protein-protein interactions. Considering the lack of distinct binding pockets on the surface of KRAS proteins and the high binding affinity of cyclic peptides for protein surfaces, peptide molecules are used as the first choice for targeting KRAS G12D (Biochem. Biophys. Res. Commun. 2017, 484, 605-611; Bioorg. Med. Chem. Lett. 2017, 27, 2757-2761; ACS Med. Chem. Lett. 2017, 8, 732-736; Sci. Rep. 2020, 10, 21671). At present, several potent cyclic peptides targeting KRAS G12D have been reported *(*MedChemComm 2013, 4, 378-382; Angew. Chem., Int. Ed. 2015, 54, 7602-7606; J. Med. Chem. 2021, 64, 13038), but they have relatively poor cell membrane permeability. The ability of cells to uptake peptides depends on the regulation of the cell surface receptor, neuropilin 1 (NRP1), and this protein is overexpressed on the cell membrane of human lung cancer cells. Therefore, cyclic peptides targeting NRP1 and KRAS G12D are picked out, and in this way, the cyclic peptides not only have relatively high cell uptake ability, but also act on KRAS G12D to exert an anti-tumor effect *(*J. Am. Chem. Soc. 2022, 144, 7117-7128).

On Dec. 10, 2021, the first non-covalent, potent, and highly selective KRAS-G12D inhibitor MRTX1133 *(*J. Med. Chem. 2022, 65, 3923-3942; WO2021041671A1) was reported by Mirati Therapeutics. MRTX1133 can inhibit KRAS-G12D mutant cells in an activated or inactivated state, but does not inhibit wild-type tumor cells, with a specificity of more than 1000 times. Dose-dependent inhibition was shown in *in vivo* xenograft tumor models of both pancreatic cancer and colorectal cancer.

Patents such as WO2021041671A1, WO2022015375A1, WO2022031678A1, WO2022066646A1, WO2022098625A1, WO2022192790A1, WO2022192794A1, WO2021106231A1, WO2021107160A1, and WO2022173870A1 disclose several classes of KRAS G12D specific inhibitors.

The inhibitors described above that directly target KRAS, such as KRAS-G12C and KRAS-G1D inhibitors, are specific inhibitors, while the inhibitors that indirectly target KRAS, such as SHP2, SOS1, and KRAS (on) inhibitors, are pan-KRAS inhibitors. The SOS1 inhibitor can act on various KRAS mutants when indirectly targeting KRAS, including KRAS-G12C/G12D/G12V/G13D (BI-3406, Cancer Discov. 2021, 11, 142-157; MRTX0902, J. Med. Chem. 2022, 65, 9678-9690), etc.

Patents such as WO2022132200A1 and WO2022133038A1 disclose several classes of pan-KRAS inhibitors.

Targeted protein degraders have become a globally popular research and development technology in recent years *(*Nat. Rev. Drug Discov. 2017, 16, 101-114; Nat. Rev. Drug Discov. 2019, 18, 949-963; Nat. Rev. Drug Discov. 2022, 21, 181-200). The technology has the following characteristics: the development of the "druggable" target encounters a bottleneck, the potential of the "non-druggable" target is infinite, and the PROTAC technology is expected to solve the development problem of the "non-druggable" target; the PROTAC drugs have the advantages of good druggability, high selectivity, capability of overcoming drug resistance, multiple administration routes, small dose, low toxicity, etc.

Thus, there is an unmet clinical need for pan-KRAS-targeted protein degraders.

### SUMMARY

The present disclosure provides a compound of formula I or I', and/or a stereoisomer, an enantiomer, a diastereomer, an atropisomer, a deuteride, a hydrate, a solvate or a prodrug thereof and/or a pharmaceutically acceptable salt thereof: 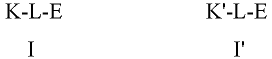 wherein:
K is K1: 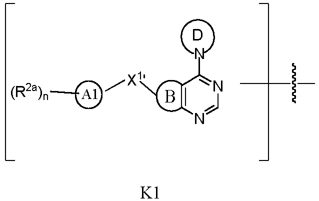
K¹ is K2: 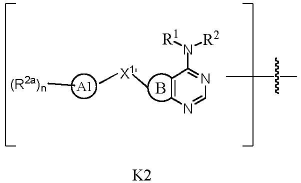
each R^{2a} heteroalkyl, C₃-C₈ alkynyl, triazolyl, - alkyl-, HC(=O)-, - heteroalkyl, C₃-C₈ alkynyl, -O-C₁-C₆ substituted with 1 o
each R^{3a} i C₆ alkynyl;
or two R wherein the 4- to 12 hydroxy, cyano, am
each ring membered heterocy
each ring to 16-membered het or 5- to 16-member
ring D is optionally substitut heteroatom or heter
each R^{a} i membered heterocy C₃ alkyl-, C₁-C₃ a heterocycloalkyl, or alkyl), -OC₁-C₆ hete -S(C₃-C₈ cycloalkyl 3- to 8-membered h deuterium, halogen,
each R^{4a}
or two heterocycloalkyl is
each X¹' i
each R¹ a **is independently** halogen, deuterium, cyano, amino, hydroxy, C₁-C₆ alkyl, C₁-C₆ cycloalkyl, 3- to 8-membered heterocycloalkyl, -S-C₁-C₆ alkyl, C₂-C₆ alkenyl, C₂-C₆ O-C₁-C₆ alkyl, -O-C₃-C₈ cycloalkyl, -CH₂C(=O)N(R^{3a})₂, N(R^{3a})₂, C₁-C₃ alkyl-O-C₁-C₃ CO₂R^{3a}, -CON(R^{3a})₂, or 5- to 6-membered heteroaryl, wherein the C₁-C₆ alkyl, C₁-C₆ cycloalkyl, 3- to 8-membered heterocycloalkyl, -S-C₁-C₆ alkyl, C₂-C₆ alkenyl, C₂-C₆ alkyl, (NR^{3a})₂, -O-C₃-C₈ cycloalkyl, or 5- to 6-membered heteroaryl is optionally r more deuterium, halogen, hydroxy, cyano, amino, nitro, C₁-C₃ alkoxy, or C₁-C₃ alkyl; s independently selected from H, C₁-C₆ alkyl, C₃-C₈ cycloalkyl, C₃-C₆ alkenyl, and C₃-
^{3a}, together with the atom linked thereto, form 4- to 12-membered heterocycloalkyl, -membered heterocycloalkyl is optionally substituted with 1 or more deuterium, halogen, ino, C₁-C₃ alkoxy, or C₁-C₃ alkyl;
A1 is independently absent, C₆-C₁₀ aryl, 5- to 10-membered heteroaryl, or 5- to 10-cloalkyl;
B is independently C₆-C₁₅ aryl, 5- to 15-membered heteroaryl, C₅-C₁₅ cycloalkyl, or 5-erocycloalkyl, wherein the C₆-C₁₅ aryl, 5- to 15-membered heteroaryl, C₅-C₁₅ cycloalkyl, ed heterocycloalkyl is optionally substituted with 1 or more R^{a};
4- to 12-membered heterocycloalkyl, wherein the 4- to 12-membered heterocycloalkyl is ed with 1 or more R^{a}, and the 4- to 12-membered heterocycloalkyl contains at least one oatom group selected from N, S, and O;
s independently hydrogen, hydroxy, halogen, cyano, -N(R^{3a})₁₋₂, -CH₂N(R^{3a})₁₋₂, 3- to 8-cloalkyl, C₁-C₆ alkyl, HC(=O)-, -CO₂R^{4a}, -CON(R^{4a})₁₋₂, C₁-C₃ alkoxy, (C₁-C₃ alkoxy)-C₁-lkyl-N(R^{4a})₁₋₂, C₂-C₄ alkenyl, C₂-C₆ alkynyl, C₃-C₈ cycloalkyl, 3- to 8-membered r 5- to 6-membered heteroaryl, wherein the C₁-C₆ alkyl, C₁-C₆ heteroalkyl, -O(C₁-C₆ roalkyl, -O(C₃-C₈ cycloalkyl), -O(3- to 8-membered heterocycloalkyl), -S(C₁-C₆ alkyl), l), -O-phenyl, -O-pyridyl, C₁-C₃ alkoxy, C₂-C₄ alkenyl, C₂-C₆ alkynyl, C₃-C₈ cycloalkyl, eterocycloalkyl, or 5- to 6-membered heteroaryl is optionally substituted with 1 or more , cyano, hydroxy, amino, nitro, C₁-C₃ alkyl, or C₁-C₃ alkoxy;
is independently hydrogen, C₁-C₄ alkyl, or C₁-C₃ hydroxyalkyl;
R^{4a}, together with the atom linked thereto, form heterocycloalkyl, wherein the optionally substituted with 1 or more deuterium, -OH, -NH₂, C₁-C₃ alkyl, or C₁-C₃ alkoxy;
s independently a bond or -C(O)-;
nd each R² are independently hydrogen, C₁-C₆ alkyl, C₁-C₆ alkoxy, C₃-C₈ cycloalkyl, or 3- to 8-membered heterocycloalkyl, wherein the C₁-C₆ alkyl, C₃-C₈ cycloalkyl, or 3- to 8-membered heterocycloalkyl is optionally substituted with one or more deuterium, halogen, hydroxy, amino, -C₁-C₃ alkyl, -S-C₁-C₃ alkyl, -O-C₁-C₃ alkyl, or -NH-C₁-C₃ alkyl;
n is 0, 1, 2, 3, or 4;
L is a linking chain, which links K and E by means of covalent bonds;
L is a linking chain, which links K' and E by means of covalent bonds;
each E is independently E1, E2, or E3: 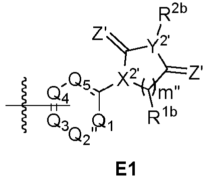
wherein in E1:
   Z' is O, S, or CH₂;
   X²' is CH or N;
   Y²' is CH, N, O, or S;
   Q₁, Q₂, Q₃, Q₄, and Q₅ are each independently CR^{3b} or N;
   each R^{3b} is independently hydrogen, deuterium, hydroxy, amino, cyano, halogen, nitro, sulfhydryl, C₁-C₆ alkyl, C₁-C₆ heteroalkyl, C₂-C₆ alkenyl, C₂-C₆ alkynyl, C₃-C₈ cycloalkyl, 3- to 8-membered heterocycloalkyl, C₆-C₁₀ aryl, 5- to 10-membered heteroaryl, -O-(C₁-C₆ alkyl), -O-(C₁-C₆ heteroalkyl), -O-(C₃-C₈ cycloalkyl), -O-(3- to 8-membered heterocycloalkyl), -S-(C₁-C₆ alkyl), -S-(C₁-C₆ heteroalkyl), -S-(C₃-C₈ cycloalkyl), -S-(3- to 8-membered heterocycloalkyl), -N(C₁-C₆ alkyl)₁₋₂, -N(C₁-C₆ heteroalkyl)₁₋₂, - N(C₃-C₈ cycloalkyl)₁₋₂, -N(3- to 8-membered heterocycloalkyl)₁₋₂, -O-(C₆-C₁₀ aryl), or -O-(5- to 10-membered heteroaryl); the C₁-C₆ alkyl, C₁-C₆ heteroalkyl, C₂-C₆ alkenyl, C₂-C₆ alkynyl, C₃-C₈ cycloalkyl, 3- to 8-membered heterocycloalkyl, C₆-C₁₀ aryl, or 5- to 10-membered heteroaryl is optionally substituted with 1-3 groups independently selected from deuterium, hydroxy, halogen, cyano, amino, O(C₁-C₆ alkyl), O-(C₃-C₈ cycloalkyl), -O-(3- to 8-membered heterocycloalkyl), N(C₁-C₆ alkyl)₁₋₂, -NH(C₃-C₈ cycloalkyl), - NH(3- to 8-membered heterocycloalkyl), -O-(C₆-C₁₀ aryl), and -O-(5- to 10-membered heteroaryl); or R^{3b}, together with the atom linked thereto, form cycloalkyl, heterocycloalkyl, heteroaryl, or aryl;
   m" is 1, 2, or 3;
   each R^{1b} is independently hydrogen, deuterium, hydroxy, amino, cyano, halogen, C₁-C₆ alkyl, C₁-C₆ heteroalkyl, C₃-C₈ cycloalkyl, C₃-C₈ heterocycloalkyl, C₆-C₁₀ aryl, 5- to 10-membered heteroaryl, -O(C₁-C₆ alkyl), -O-(C₃-C₈ cycloalkyl), -O-(3- to 8-membered heterocycloalkyl), -N(C₁-C₆ alkyl)₁₋₂, -NH(C₃-C₈ cycloalkyl), -NH(3- to 8-membered heterocycloalkyl), -O-(C₆-C₁₀ aryl), or -O-(5- to 10-membered heteroaryl), wherein the alkyl, cycloalkyl, heterocycloalkyl, aryl, or heteroaryl is optionally substituted with 1-3 groups independently selected from hydroxy, halogen, cyano, hydroxy, and amino;
   R^{2b} is absent, hydrogen, deuterium, C₁-C₆ alkyl, or C₃-C₆ cycloalkyl, wherein the C₁-C₆ alkyl and C₃-C₆ cycloalkyl are optionally substituted with 1-3 groups independently selected from hydroxy, halogen, cyano, hydroxy, amino, and -OC(O)(C₁-C₆ alkyl); 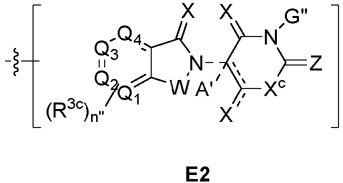
   wherein in E2:
      Q₁, Q₂, Q₃, and Q₄ are each independently CR^{3b} or N;
      W is CR^{1c}R^{2c}, C(S), C(O), SO₂, -OC=R^{4c}-, -SC=R^{4c}-, -C=R^{4c}NR^{5c}-, or -N=CA'-;
      X is CH₂, O, or S;
      X^{c}is -CH₂- or -NG'-;
      Z is CH₂, O, or S;
      G' and G" are each independently selected from hydrogen, deuterium, C₁-C₆ alkyl, OH, C₃-C₆ cycloalkyl, -CH₂-heterocycloalkyl, and -CH₂-phenyl, wherein the C₁-C₆ alkyl, C₃-C₆ cycloalkyl, -CH₂-heterocycloalkyl, or -CH₂-phenyl is optionally substituted with 1 or more hydroxy, halogen, cyano, and amino;
      A' is hydrogen, deuterium, C₁-C₆ alkyl, C₃-C₈ cycloalkyl, or halogen;
      R^{1c}, R^{2c}, and R^{3c} are each independently selected from hydrogen, deuterium, hydroxy, halogen, - NH₂, -N(C₁-C₆ alkyl)₁₋₂, C₁-C₆ alkyl, C₁-C₆ alkoxy, C₁-C₆ haloalkyl, -CONR_{'}R_{"}, -OR_{'}, -NR_{'}R_{"}, -SR_{'}, -SO₂R_{'}, -SO₂NR_{'}R_{"}, -CR_{'}R_{"}, -CR_{'}NR_{'}R_{"}, C₆-C₁₀ aryl, 5- to 10-membered heteroaryl, C₃-C₈ cycloalkyl, 3- to 8-membered heterocycloalkyl, -P(O)(OR_{'})R_{"}, -P(O)R_{'}R_{"}, -OP(O)(OR_{'})R_{"}, -CN, -NR_{'}SO₂NR_{'}R_{"}, - NR_{'}C(O)NR_{'}R_{"}, -C(O)N_{'}C(O)R_{"}, -NR_{'}C(=N-CN)NR_{'}R_{"}, -C(=N-CN)NR_{'}R_{"}, -NR_{'}C(=N-CN)R_{"}, -NR_{'}C(=C-NO₂)NR_{'}R_{"}, -SO₂NR_{'}COR_{"}, -NO₂, -COR_{'}, -C(C=N-OR_{'})R_{"}, -CR_{'}=CR_{'}R_{"}, -CCR_{'}, -S(C=O)(C=N-R_{'})R_{"}, -SF₅ and -OCF₃;
      R^{4c} is O or S;
      R^{5c} is H, C₁-C₆ alkyl, C₆-C₁₀ aryl, 5- to 12-membered heteroaryl, C₃-C₈ cycloalkyl, or 3- to 8-membered heterocycloalkyl;
      R_{'} and R_{"} are each independently selected from a bond, hydrogen, deuterium, C₁-C₆ alkyl, C₃-C₈ cycloalkyl, C₆-C₁₀ aryl, 5- to 10-membered heteroaryl, and 3- to 8-membered heterocycloalkyl;
      n^{"} is 0, 1, 2, 3, or 4;
      -̅ -̅ -̅ is a single bond or a double bond;
      ----- is a bond, which may be an R stereoisomer, an S stereoisomer, or a non-stereoisomer; 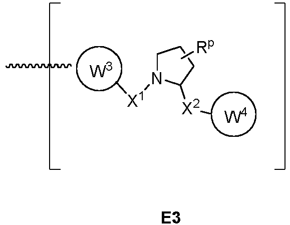
      wherein in E3:
         X¹ and X² are each independently a bond, O, C(O), C(S), NR^{1d}, or CR^{1d}R^{2d};
         R^{1d} and R^{2d} are each independently selected from H, deuterium, and C₁-C₆ alkyl, wherein the C₁-C₆ alkyl is optionally substituted with 1 or more halogen or C₁-C₆ alkoxy;
         R^{P} is independently selected from H, deuterium, halogen, -OH, and C₁-C₃ alkyl, wherein the C₁-C₃ alkyl is optionally substituted with 1 or more halogen, hydroxy, or C₁-C₃ alkoxy;
         W³ is C₁-C₆ alkyl, -T-N(R^{3d}R^{4d}), -T-N(R^{3d}R^{4d})X³, -T-(C₆-C₁₀) aryl, -T-(5- to 10-membered)heteroaryl, -T-(4- to 12-membered) heterocycloalkyl, -NR^{5d}-T-(C₆-C₁₀) aryl, -NR^{5d}-T-(5- to 10-membered)heteroaryl, or -NR^{5d}-T-(4- to 12-membered) heterocycloalkyl, wherein the C₁-C₆ alkyl, -T-N(R^{3d}R^{4d}), -T-N(R^{3d}R^{4d})X³, -T-(C₆-C₁₀) aryl, -T-(5- to 10-membered)heteroaryl, -T-(4- to 12-membered)heterocycloalkyl, -NR^{5d}-T-(C₆-C₁₀) aryl, -NR^{5d}-T-(5- to 10-membered)heteroaryl, or -NR^{5d}-T-(4-to 12-membered)heterocycloalkyl is optionally substituted;
         X³ is C(O), R^{3d}, R^{4d}, or R^{5d};
         R^{3d}, R^{4d}, or R^{5d} is each independently selected from H, deuterium, and C₁-C₆ alkyl, wherein the C₁-C₆ alkyl is optionally substituted with 1 or more halogen, -OH, R^{1d}C(O), R^{1d}C(S), R^{1d}SO, R^{1d}SO₂, NR^{1d}R^{2d}C(O), NR^{1d}R^{2d}C(S), NR^{1d}R^{2d}SO, or NR^{1d}R^{2d}SO₂;
         T is C₁₋C₆ alkyl or -(CH₂)_{n'}-, wherein 1 or more methylene groups in the -(CH₂)_{n'}- are optionally substituted with groups selected from deuterium, halogen, and C₁₋C₆ alkyl, and the C₁₋C₆ alkyl is optionally substituted with halogen, -OH, or amino;
         n' is 0, 1, 2, 3, 4, 5, or 6;
         W⁴ is selected from 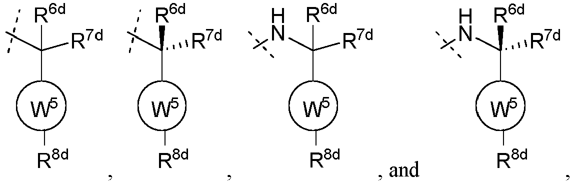 wherein the 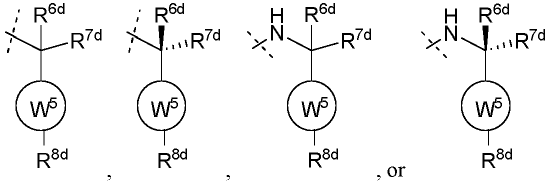 is optionally substituted;
         R^{6d} and R^{7d} are each independently selected from H, deuterium, C₃-C₈ cycloalkyl, and C₁₋C₆ alkyl, wherein the C₃-C₈ cycloalkyl or C₁₋C₆ alkyl is optionally substituted with halogen, -OH, CN, NO₂, or amino;
         W⁵ is selected from 6- to 10-membered aryl and 5- to 10-membered heteroaryl;
         R^{8d} is H, deuterium, halogen, CN, OH, NO₂, NR^{6d}R^{7d}, OR^{6d}, COR^{6d}R^{7d}, NR^{6d}COR^{7d}, SO₂R^{6d}R^{7d}, R^{6d}SO₂R^{7d}, C₁-C₆ alkyl, C₁₋C₆ alkoxy, C₆-C₁₀ aryl, 5- to 10-membered heteroaryl, C₃-C₈ cycloalkyl, or 3- to 8-membered heterocycloalkyl, wherein the C₁₋C₆ alkyl or C₁₋C₆ alkoxy is optionally substituted with deuterium, halogen, -OH, CN, NO₂, or amino.
         Preferably, in certain embodiments of the present disclosure, in formula I, the definitions of one or more of the groups R^{2a}, R^{3a}, R^{a}, and R^{4a} are also replaced by the following definitions, provided that L is linked to E via -C(O)-;
         R^{2a} is sulfhydryl, (C₁-C₃ alkoxy)C₁-C₃ alkyl-, -O-C₁-C₆ heteroalkyl, -OCOR^{3a}, -NH(C₁-C₆ heteroalkyl), -N(C₁-C₆ heteroalkyl)(C₁-C₆ heteroalkyl), or -C₃-C₄ alkynyl-N(R^{5a'})₂;
         in R^{2a}, the hydroxy is independently optionally substituted with 1 or more deuterium, C₁-C₃ alkoxy, C₁-C₃ alkyl, C₃-C₈ cycloalkyl, -Si-C₁-C₃ alkyl, 3- to 8-membered heterocycloalkyl, or -CON(R^{3a})₂;
         in R^{2a}, the C₁₋C₆ alkyl, C₁-C₆ heteroalkyl, C₃-C₈ cycloalkyl, 3- to 8-membered heterocycloalkyl, - S-C₁-C₆ alkyl, C₂-C₆ alkenyl, C₂-C₆ alkynyl, -O-C₁-C₆ heteroalkyl, -O-C₃-C₈ cycloalkyl, and 5- to 6-membered heteroaryl are independently optionally substituted with 1 or more C₃-C₈ cycloalkyl, -Si-C₁-C₃ alkyl, 3- to 8-membered heterocycloalkyl, or -CON(R^{3a})₂;
         each R^{3a} is deuterium or -NH-C₁-C₃ alkyl;
         R^{a} is independently a bond, deuterium, oxo, cyanomethyl, C₁₋C₆ heteroalkyl, -O(C₁-C₆ alkyl), - O(C₃-C₈ cycloalkyl), -O(C₁-C₆ heteroalkyl), -O(3- to 8-membered heterocycloalkyl), -S(C₁-C₆ alkyl), -S(C₃-Cs cycloalkyl), -O-phenyl, -O-pyridyl, triazolyl, -CH₂C(=O)N(R^{3a})₂, or -C₃-C₄ alkynyl-N(R^{3a})₂;
         or two adjacent R^{a}, together with the atoms linked thereto, form 6- to 10-membered aryl, 5- to 10-membered heteroaryl, 5- to 8-membered cycloalkyl, or 5- to 8-membered heterocycloalkyl, wherein the 6-to 10-membered aryl, 5- to 10-membered heteroaryl, 5- to 8-membered cycloalkyl, or 5- to 8-membered heterocycloalkyl is optionally substituted with 1 or more hydroxy, amino, halogen, cyano, or nitro;
         in R^{a}, the C₁₋C₆ alkyl, C₁-C₃ alkoxy, C₂-C₄ alkenyl, C₃-C₈ cycloalkyl, 3- to 8-membered heterocycloalkyl, or 5- to 6-membered heteroaryl is optionally substituted with 1 or more C₁-C₃ haloalkyl;
         in R^{a}, the C₁₋C₆ heteroalkyl, phenyl, pyridyl, C₂-C₄ alkynyl, triazolyl, -CH₂C(=O)N(R^{3a})₁₋₂, or - C₃-C₄ alkynyl-N(R^{3a})₂ is optionally substituted with 1 or more deuterium, halogen, cyano, hydroxy, amino, nitro, C₁-C₃ haloalkyl, C₁-C₃ alkyl, or C₁-C₃ alkoxy;
         each R^{4a} is independently C₁₋C₆ alkyl.

Preferably, in certain embodiments of the present disclosure, in each R^{2a}, the C₁-C₆ heteroalkyl is C₃ heteroalkyl, wherein in the heteroalkyl, methylene is replaced by -C(O)NH-, such as

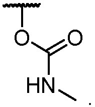

Preferably, in certain embodiments of the present disclosure, in each R^{2a}, the C₃-C₈ may be optionally substituted with 1 or more C₁-C₃ alkyl.

Preferably, in certain embodiments of the present disclosure, in each R^{2a}, the C₃-C₈ cycloalkyl may be optionally substituted with 1 or more C₁-C₃ alkyl.

Preferably, in certain embodiments of the present disclosure, in each R^{2a}, the 3- to 8-membered heterocycloalkyl is 3- to 6-membered heterocycloalkyl containing 1, 2, or 3 heteroatoms being one or more of N, S, and O.

Preferably, in certain embodiments of the present disclosure, in each R^{2a}, the C₂-C₆ alkynyl is C₂-C₄ alkynyl, such as ethynyl.

Preferably, in certain embodiments of the present disclosure, each R^{2a} is optionally substituted with substituents, wherein in the substituents, the halogen is F, Cl, Br, or I, such as F.

Preferably, in certain embodiments of the present disclosure, each R^{2a} is optionally substituted with substituents, wherein in the substituents, the C₁-C₃ alkoxy is methoxy, ethoxy, n-propoxy, or isopropoxy, such as methoxy.

Preferably, in certain embodiments of the present disclosure, each R^{2a} is optionally substituted with substituents, wherein in the substituents, the C₁-C₃ alkyl is methyl, ethyl, n-propyl, or isopropyl, such as methyl.

Preferably, in certain embodiments of the present disclosure, in each ring A1, the C₆-C₁₀ aryl is phenyl or naphthyl.

Preferably, in certain embodiments of the present disclosure, in each ring A1, 5- to 15-membered heteroaryl in the 5- to 15-membered heteroaryl is 5- to 10-membered heteroaryl, and may also be 6-, 7-, 8-, or 9-membered monocyclic or bicyclic heteroaryl, containing 1 or 2 heteroatoms being one or more of N, S, and O, such as pyridyl, 1H-indazolyl, phenyl[dlthiazole, or oxazolophenyl.

Preferably, in certain embodiments of the present disclosure, in each ring A1, the 5- to 10-membered heteroaryl is bicyclic heteroaryl, containing 1 or 2 heteroatoms being one or more of N, S, and O, such as pyridyl, 1H-indazolyl, phenyl[dlthiazole, or oxazolophenyl.

Preferably, in certain embodiments of the present disclosure, in each ring A1, the 5- to 10-membered heterocycloalkyl contains 1 or 2 heteroatoms being one or more of N, S, and O.

Preferably, in certain embodiments of the present disclosure, in each ring B, the C₆-C₁₅ aryl is C₆-C₁₀ aryl, such as phenyl or naphthyl.

Preferably, in certain embodiments of the present disclosure, in each ring B, the 5- to 15-membered heteroaryl is 5- to 10-membered heteroaryl, and may also be 5- to 6-membered heteroaryl, containing 1 or 2 heteroatoms of N, such as pyridyl.

Preferably, in certain embodiments of the present disclosure, in each ring B, the C₅-C₁₅ cycloalkyl is C₅-C₇ monocyclic saturated cycloalkyl, C₅-C₇ cycloalkenyl, or C₈-C₁₅ tricyclic cycloalkyl, such as 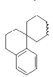 or 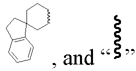 indicates that the cycloalkyl is fused to a ring linked thereto via the bond herein.

Preferably, in certain embodiments of the present disclosure, in each ring B, the 5- to 16-membered heterocycloalkyl is 5- to 7-membered monocyclic heterocycloalkyl containing 1 or 2 heteroatoms of N and/or O, such as pyrrolidinyl, piperidinyl, or tetrahydro-2H-pyranyl.

Preferably, in certain embodiments of the present disclosure, in each ring D, ring D is not:

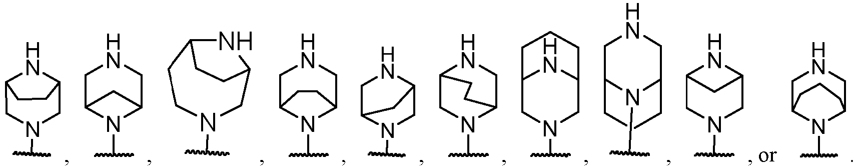

Preferably, in certain embodiments of the present disclosure, in each ring D, the 4- to 12-membered heterocycloalkyl is 5- to 7-membered monocyclic saturated heterocycloalkyl, containing 1, 2, or 3 heteroatoms being one or more of N, O, and S (including heteroatom groups formed by each heteroatom, such as -C(O)-, -S(O)-, -S(O)₂-, -C(O)O-, -OC(O)-), C(O)NH-, -NHC(O)-, and the like), for example:

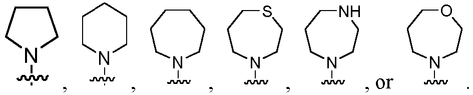

Preferably, in certain embodiments of the present disclosure, in each ring D, the 4- to 12-membered heterocycloalkyl is 8-, 9-, or 10-membered bridged heterocycloalkyl or spiroheterocycloalkyl, containing 1, 2, or 3 heteroatoms being one or more of N, O, and S (including heteroatom groups formed by each heteroatom, such as -C(O)-, -S(O)-, -S(O)₂-, -C(O)O-, -OC(O)-), C(O)NH-, -NHC(O)-, and the like), for example:

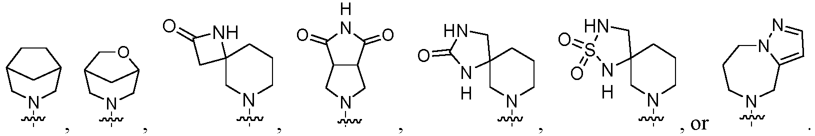

Preferably, in certain embodiments of the present disclosure, in formula I, the definitions of one or more of the groups R^{2a}, R^{3a}, C₁-C₄ alkylene in L', R^{a}, and R^{b} may also be replaced by the following definitions, provided that L is linked to E via -C(O)-;
R^{2a} is sulfhydryl, (C₁-C₃ alkoxy)C₁-C₃ alkyl-, -O-C₁-C₆ heteroalkyl, -NH(C₁-C₆ heteroalkyl), -N(C₁-C₆ heteroalkyl)(C₁-C₆ heteroalkyl), or -C₃-C₄ alkynyl-N(R^{5a'})₂;
in R^{2a}, the hydroxy is independently optionally substituted with 1 or more deuterium, halogen, hydroxy, cyano, amino, nitro, C₁-C₃ alkoxy, C₁-C₃ alkyl, C₃-C₈ cycloalkyl, -Si-C₁-C₃ alkyl, 3- to 8-membered heterocycloalkyl, or -CON(R^{3a})₂;
in R^{2a}, the C₁₋C₆ alkyl, C₁-C₆ heteroalkyl, C₃-C₈ cycloalkyl, 3- to 8-membered heterocycloalkyl, - S-C₁-C₆ alkyl, C₂-C₆ alkenyl, C₂-C₆ alkynyl, -O-C₁-C₆ heteroalkyl, -O-C₃-C₈ cycloalkyl, and 5- to 6-membered heteroaryl are independently optionally substituted with 1 or more C₃-C₈ cycloalkyl, -Si-C₁-C₃ alkyl, 3- to 8-membered heterocycloalkyl, or -CON(R^{3a})₂;
each R^{3a} is deuterium;
R^{a} is independently a bond, deuterium, oxo, cyanomethyl, C₁₋C₆ heteroalkyl, -O(C₁-C₆ alkyl), - O(C₃-C₈ cycloalkyl), -O(C₁-C₆ heteroalkyl), -O(3- to 8-membered heterocycloalkyl), -S(C₁-C₆ alkyl), -S(C₃-Cs cycloalkyl), -O-phenyl, -O-pyridyl, triazolyl, -CH₂C(=O)N(R^{3a})₂, or -C₃-C₄ alkynyl-N(R^{3a})₂;
or two adjacent R^{a}, together with the atoms linked thereto, form 6- to 10-membered aryl, 5- to 10-membered heteroaryl, 5- to 8-membered cycloalkyl, or 5- to 8-membered heterocycloalkyl, wherein the 6-to 10-membered aryl, 5- to 10-membered heteroaryl, 5- to 8-membered cycloalkyl, or 5- to 8-membered heterocycloalkyl is optionally substituted with 1 or more hydroxy, amino, halogen, cyano, or nitro;
in R^{a}, the C₁₋C₆ alkyl, C₁-C₃ alkoxy, C₂-C₄ alkenyl, C₃-C₈ cycloalkyl, 3- to 8-membered heterocycloalkyl, or 5- to 6-membered heteroaryl is optionally substituted with 1 or more C₁-C₃ haloalkyl;
in R^{a}, the C₁₋C₆ heteroalkyl, phenyl, pyridyl, C₂-C₄ alkynyl, triazolyl, -CH₂C(=O)N(R^{3a})₁₋₂, or - C₃-C₄ alkynyl-N(R^{3a})₂ is optionally substituted with 1 or more deuterium, halogen, cyano, hydroxy, amino, nitro, C₁-C₃ haloalkyl, C₁-C₃ alkyl, or C₁-C₃ alkoxy;
each R^{4a} is independently C₁₋C₆ alkyl.

Preferably, in certain embodiments of the present disclosure, K1 is K1-I or K1-II: 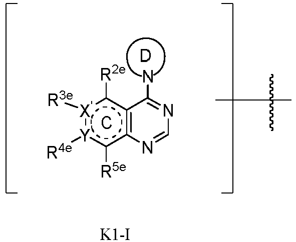 wherein in K1-I:
ring C is C₆-C₁₀ aryl, 5- to 10-membered heteroaryl, C₆-C₁₀ cycloalkyl, or 5- to 10-membered heterocycloalkyl;
X' is a bond, C, or N;
Y' is C or N; 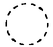
is one or more double bonds that are optionally present;
R^{2e} is H, deuterium, halogen, hydroxy, amino, C₁-C₆ alkyl, C₃-C₈ cycloalkyl, O(C₁-C₆ alkyl), or O(C₃-C₈ cycloalkyl);
R^{3e} is a bond, H, deuterium, halogen, cyano, oxo, O(C₁-C₆ alkyl), O(C₁-C₆ heteroalkyl), O(C₃-C₈ cycloalkyl), O(3- to 8-membered heterocycloalkyl), S(C₁-C₆ alkyl), S(C₃-C₈ cycloalkyl), -O-phenyl, or -O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
R^{4e} is 6- to 10-membered aryl or 5- to 10-membered heteroaryl, wherein the 6- to 10-membered aryl or 5- to 10-membered heteroaryl is optionally substituted with 1 or more R^{a'};
or R^{4e}, together with the carbon atom linked thereto, forms C₆-C₁₂ aryl or 5- to 12-membered heteroaryl (i.e., R^{4e}, together with Y' linked thereto, forms a spiro ring structure), wherein the C₆-C₁₂ aryl or 5- to 12-membered heteroaryl is optionally substituted with H, deuterium, halogen, hydroxy, amino, C₁-C₆ alkyl, -S(C₁-C₆ alkyl), or -O(C₁-C₆ alkyl);
R^{5e} is hydrogen, deuterium, halogen, cyano, C₁-C₆ alkyl, C₃-C₈ cycloalkyl, -O(C₁-C₆ alkyl), or - O(C₃-C₈ cycloalkyl);
R^{a'} is independently selected from halogen, cyano, hydroxy, C₁₋C₆ alkyl, C₁₋C₆ heteroalkyl, -S-C₁-C₆ alkyl, C₂-C₆ alkenyl, C₂-C₆ alkynyl, triazolyl, -O-C₁-C₆ alkyl, -O-C₁-C₆ heteroalkyl, -N(C₁-C₆ alkyl)₁₋₂, - N(C₁-C₆ heteroalkyl)₁₋₂, -CH₂C(=O)N(R^{5a'})₂, -C₃-C₄ alkynyl(NR^{5a'})₂, -N(R^{5a'})₂, (C₁-C₃ alkoxy)C₁-C₃ alkyl-, C₃-C₆ cycloalkyl, and 3- to 6-membered heterocycloalkyl, wherein the hydroxy, C₁₋C₆ alkyl, C₁-C₆ heteroalkyl, -S-C₁-C₆ alkyl, C₂-C₆ alkenyl, C₂-C₆ alkynyl, -O-C₁-C₆ alkyl, -O-C₁-C₆ heteroalkyl, -N(C₁-C₆ alkyl)₁₋₂, -N(C₁-C₆ heteroalkyl)₁₋₂, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-(C₁-C₃ alkyl)₃;
each R^{5a'} is independently selected from H, deuterium, and C₁₋C₆ alkyl;
ring D is as defined and described in the present disclosure; 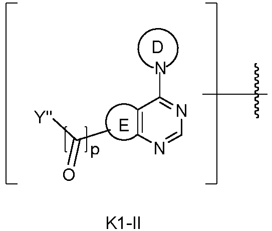
wherein in K1-II:
   ring E is C₅-C₇ cycloalkyl, 5- to 7-membered heterocycloalkyl, C₆-C₁₀ aryl, 5- to 10-membered heteroaryl, C₅-C₇ cycloalkenyl, or a 8- to 10-membered spiro ring, and a fused ring is formed between ring E and a pyrimidine ring, wherein the C₅-C₇ cycloalkyl, 5- to 7-membered heterocycloalkyl, C₆-C₁₀ aryl, 5-to 10-membered heteroaryl, C₅-C₇ cycloalkenyl, or 8- to 10-membered spiro ring is optionally substituted with R^{b"}, and the heterocycloalkyl contains at least one heteroatom selected from N, S, and O;
   Y" is 6- to 10-membered heterocycloalkyl, 6- to 10-membered aryl, or 5- to 10-membered heteroaryl, wherein the 6- to 10-membered heterocycloalkyl or 5- to 10-membered heteroaryl contains at least one heteroatom selected from N, S, and O, and is optionally substituted with R^{b‴};
   R^{b'} is C₁-C₃ alkyl, hydroxy, -O(C₁-C₃ alkyl), cyano, halogen, -N(R^{x'})₂, -CH₂N(R^{x'})₂, cyanomethyl, or 3- to 8-membered heterocycloalkyl;
   R^{b"} is halogen, deuterium, cyano, hydroxy, C₁-C₄ alkyl, -S-C₁-C₃ alkyl, C₂-C₄ alkenyl, C₂-C₄ alkynyl, triazolyl, -O-C₁-C₃ alkyl, -CH₂C(=O)N(R^{x'})₂, -C₃-C₄ alkynyl(NR^{x'})₂, -N(R^{x'})₂, or C₁-C₃ alkoxy-C₁-C₃ alkyl-, wherein the C₁-C₄ alkyl, -S-C₁-C₃ alkyl, C₂-C₄ alkenyl, C₂-C₄ alkynyl, or C₁-C₃ alkoxy is optionally substituted with deuterium, halogen, hydroxy, cyano, amino, nitro, C₁-C₃ alkoxy, or C₁-C₃ alkyl;
   R^{b‴} is hydrogen, hydroxy, halogen, cyano, C₁-C₆ alkyl, C₂-C₄ alkynyl, C₂-C₄ alkenyl, O(C₁-C₆ alkyl), HC(=O)-, -CO₂R^{x'}, -CO₂N(R^{x'})₂, or 5- to 6-membered heteroaryl, wherein the C₁₋C₆ alkyl is optionally substituted with halogen, hydroxy, cyano, or amino;
   each R^{x'} is independently selected from H and C₁-C₃ alkyl;
   p is 0 or 1; and
   ring D is as defined and described in the present disclosure.

Preferably, in certain embodiments of the present disclosure, K1 is K1-I, K1-II, or K2-I: 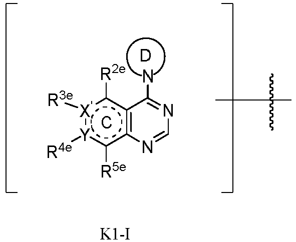 wherein in K1-I:
ring C is C₆-C₁₅ aryl, 5- to 10-membered heteroaryl, C₆-C₁₅ cycloalkyl, or 5- to 10-membered heterocycloalkyl;
X' is a bond, C, O, or N;
Y' is C or N; 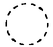
is one or more double bonds that are optionally present;
R^{2e} is H, deuterium, halogen, hydroxy, amino, C₁-C₆ alkyl, C₃-C₈ cycloalkyl, O(C₁-C₆ alkyl), or O(C₃-C₈ cycloalkyl);
R^{3e} is a bond, H, deuterium, halogen, cyano, oxo, O(C₁-C₆ alkyl), O(C₁-C₆ heteroalkyl), O(C₃-C₈ cycloalkyl), O(3- to 8-membered heterocycloalkyl), S(C₁-C₆ alkyl), S(C₃-C₈ cycloalkyl), -O-phenyl, or -O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
R^{4e} is 6- to 10-membered aryl or 5- to 10-membered heteroaryl, wherein the 6- to 10-membered aryl or 5- to 10-membered heteroaryl is optionally substituted with 1 or more R^{a'};
or R^{4e}, together with the carbon atom linked thereto, forms C₆-C₁₂ aryl or 5- to 12-membered heteroaryl, wherein the C₆-C₁₂ aryl or 5- to 12-membered heteroaryl is optionally substituted with H, deuterium, halogen, hydroxy, amino, C₁-C₆ alkyl, S(C₁-C₆ alkyl), or O(C₁-C₆ alkyl);
R^{5e} is hydrogen, deuterium, halogen, cyano, C₁-C₆ alkyl, C₃-C₈ cycloalkyl, O(C₁-C₆ alkyl), or O(C₃-C₈ cycloalkyl);
R^{a'} is independently selected from halogen, cyano, hydroxy, C₁₋C₆ alkyl, C₁₋C₆ heteroalkyl, -S-C₁-C₆ alkyl, C₂-C₆ alkenyl, C₂-C₆ alkynyl, triazolyl, -O-C₁-C₆ alkyl, -O-C₁-C₆ heteroalkyl, -N(C₁-C₆ alkyl)₁₋₂, - N(C₁-C₆ heteroalkyl)₁₋₂, -CH₂C(=O)N(R^{5a'})₂, -C₃-C₄ alkynyl(NR^{5a'})₂, -N(R^{5a'})₂, (C₁-C₃ alkoxy)C₁-C₃ alkyl-, C₃-C₆ cycloalkyl, and 3- to 6-membered heterocycloalkyl, wherein the hydroxy, C₁₋C₆ alkyl, C₁-C₆ heteroalkyl, -S-C₁-C₆ alkyl, C₂-C₆ alkenyl, C₂-C₆ alkynyl, -O-C₁-C₆ alkyl, -O-C₁-C₆ heteroalkyl, -N(C₁-C₆ alkyl)₁₋₂, -N(C₁-C₆ heteroalkyl)₁₋₂, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-(C₁-C₃ alkyl)₃;
each R^{5a'} is independently selected from H, deuterium, and C₁₋C₆ alkyl;
ring D is as defined and described in the present disclosure; 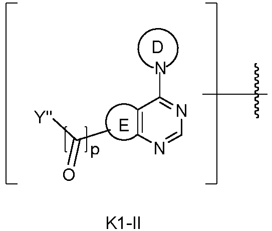
wherein in K1-II:
   ring E is C₅-C₇ cycloalkyl, 5- to 7-membered heterocycloalkyl, C₆-C₁₀ aryl, 5- to 10-membered heteroaryl, C₅-C₇ cycloalkenyl, or a 8- to 10-membered spiro ring, and a fused ring is formed between ring E and a pyrimidine ring, wherein the C₅-C₇ cycloalkyl, 5- to 7-membered heterocycloalkyl, C₆-C₁₀ aryl, 5-to 10-membered heteroaryl, C₅-C₇ cycloalkenyl, or 8- to 10-membered spiro ring is optionally substituted with R^{b"}, and the heterocycloalkyl contains at least one heteroatom selected from N, S, and O;
   Y" is 6- to 10-membered heterocycloalkyl, 6- to 10-membered aryl, or 5- to 10-membered heteroaryl, wherein the 6- to 10-membered heterocycloalkyl or 5- to 10-membered heteroaryl contains at least one heteroatom selected from N, S, and O, and is optionally substituted with R^{b‴};
   R^{b"} is hydrogen, oxo, halogen, deuterium, cyano, hydroxy, amino, C₁-C₆ alkyl, C₁₋C₆ heteroalkyl, C₃-C₆ cycloalkyl, -S-C₁-C₃ alkyl, C₂-C₄ alkenyl, C₂-C₄ alkynyl, triazolyl, -O-C₁-C₆ alkyl, -CH₂C(=O)N(R^{x'})₂, -C₃-C₄ alkynyl(NR^{x'})₂, -N(R^{x'})₂, or C₁-C₃ alkoxy-C₁-C₃ alkyl-, wherein the C₁₋C₆ alkyl, C₁-C₆ heteroalkyl, C₃-C₆ cycloalkyl, -O-C₁-C₆ alkyl, -S-C₁-C₃ alkyl, C₂-C₄ alkenyl, or C₂-C₄ alkynyl is optionally substituted with deuterium, halogen, hydroxy, cyano, amino, nitro, C₁-C₃ alkoxy, or C₁-C₃ alkyl;
   R^{b‴} is hydrogen, hydroxy, halogen, cyano, C₁-C₆ alkyl, C₂-C₄ alkynyl, C₂-C₄ alkenyl, O(C₁-C₆ alkyl), HC(=O)-, -CO₂R^{x'}, -CO₂N(R^{x'})₂, or 5- to 6-membered heteroaryl, wherein the C₁₋C₆ alkyl is optionally substituted with halogen, hydroxy, cyano, or amino;
   each R^{x'} is independently selected from H and C₁-C₃ alkyl;
   p is 0 or 1; and
   ring D is as defined and described in the present disclosure; 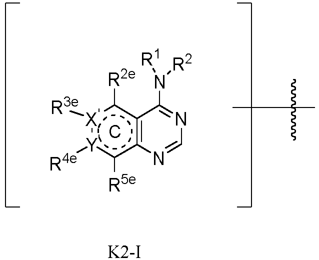
   wherein in K2-I:
   ring C is C₆-C₁₀ aryl, 5- to 10-membered heteroaryl, C₆-C₁₀ cycloalkyl, or 5- to 10-membered heterocycloalkyl;
   X' is a bond, C, O, or N;
   Y' is C or N; 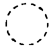
   is one or more double bonds that are optionally present;
   R^{2e} is H, deuterium, halogen, hydroxy, amino, C₁-C₆ alkyl, C₃-C₈ cycloalkyl, O(C₁-C₆ alkyl), or O(C₃-C₈ cycloalkyl);
   R^{3e} is a bond, H, deuterium, halogen, cyano, oxo, O(C₁-C₆ alkyl), O(C₁-C₆ heteroalkyl), O(C₃-C₈ cycloalkyl), O(3- to 8-membered heterocycloalkyl), S(C₁-C₆ alkyl), S(C₃-C₈ cycloalkyl), -O-phenyl, or -O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
   R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
   or R^{4e}, together with the carbon atom linked thereto, forms C₆-C₁₂ aryl or 5- to 12-membered heteroaryl, wherein the C₆-C₁₂ aryl or 5- to 12-membered heteroaryl is optionally substituted with H, deuterium, halogen, hydroxy, amino, C₁-C₆ alkyl, S(C₁-C₆ alkyl), or O(C₁-C₆ alkyl);
   R^{5e} is hydrogen, deuterium, halogen, cyano, C₁-C₆ alkyl, C₃-C₈ cycloalkyl, O(C₁-C₆ alkyl), or O(C₃-C₈ cycloalkyl);
   R^{a'} is independently selected from halogen, cyano, hydroxy, amino, sulfhydryl, C₁₋C₆ alkyl, C₁-C₆ heteroalkyl, -S-C₁-C₆ alkyl, C₂-C₆ alkenyl, C₂-C₆ alkynyl, triazolyl, -O-C₁-C₆ alkyl, -O-C₁-C₆ heteroalkyl, -N(C₁-C₆ alkyl)₁₋₂, -N(C₁-C₆ heteroalkyl)₁₋₂, -CH₂C(=O)N(R^{5a'})₂, -C₃-C₄ alkynyl(NR^{5a'})₂, -N(R^{5a'})₂, (C₁-C₃ alkoxy)C₁-C₃ alkyl-, C₃-C₆ cycloalkyl, and 3- to 6-membered heterocycloalkyl, wherein the hydroxy, C₁-C₆ alkyl, C₁-C₆ heteroalkyl, -S-C₁-C₆ alkyl, C₂-C₆ alkenyl, C₂-C₆ alkynyl, -O-C₁-C₆ alkyl, -O-C₁-C₆ heteroalkyl, -N(C₁-C₆ alkyl)₁₋₂, -N(C₁-C₆ heteroalkyl)₁₋₂, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-(C₁-C₃ alkyl)₃;
   each R^{5a'} is independently selected from H, deuterium, and C₁₋C₆ alkyl;
   R¹ and R² are as defined and described in the present disclosure.

Preferably, in certain embodiments of the present disclosure, K1 is K1-I-a or K1-II-a: 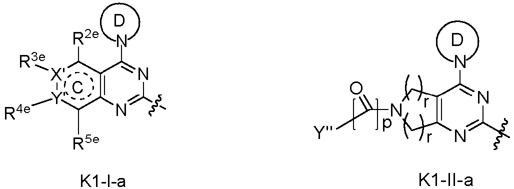 wherein:
r is 1 or 2;
t is 1, 2, or 3;
wherein ring C, ring D, R^{2e}, R^{3e}, R^{4e}, R^{5e}, Y", and p in K1-I-a or K1-II-a are as defined and described in the present disclosure.

Preferably, in certain embodiments of the present disclosure, K1 is K1-I-a, K1-II-a, or K2-I-a: 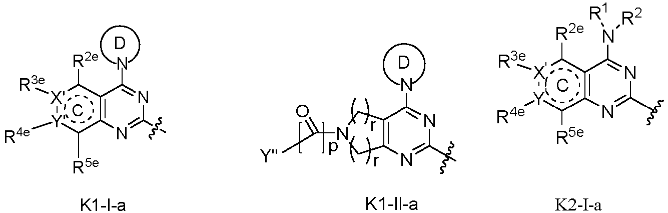 wherein:
r is 1 or 2;
t is 1, 2, or 3;
wherein ring C, ring D, R^{2e}, R^{3e}, R^{4e}, R^{5e}, X', Y', R¹, R², Y", and p in K1-I-a or K1-II-a are as defined and described in the present disclosure.

Preferably, in certain embodiments of the present disclosure, K1 is K1-I-a1, K1-I-a2, K1-I-a3, K1-I-a4, K1-I-a5, K1-I-a6, K1-II-a1, K1-II-a2, K1-II-a3, or K1-II-a4: 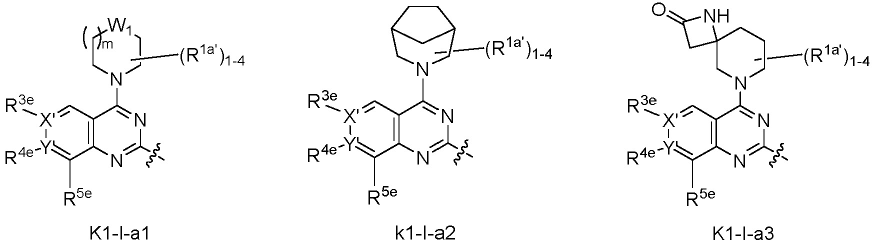 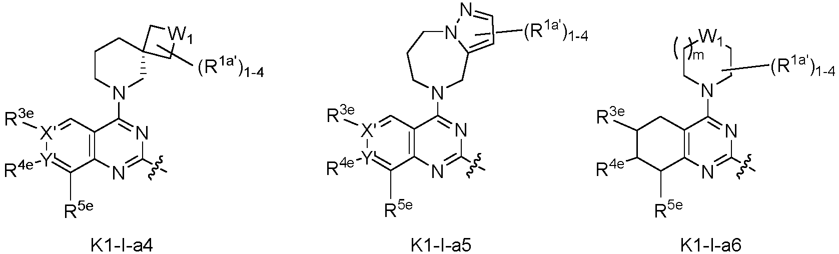 wherein in K1-I-a1, K1-I-a2, K1-I-a3, K1-I-a4, K1-I-a5, or K1-I-a6:
R^{1a'} is hydrogen, hydroxy, halogen, cyano, C₁-C₆ alkyl, C₃-C₈ cycloalkyl, HC(=O)-, -CO₂R^{5a'}, - CON(R^{5a'})₁₋₂, or 5- to 6-membered heteroaryl, wherein the C₁₋C₆ alkyl or C₃-C₈ cycloalkyl is optionally substituted with halogen, hydroxy, cyano, or amino;
each R^{5a'} is independently selected from hydrogen and C₁-C₄ alkyl;
or two R^{5a'}, together with the N atom linked thereto, form 4- to 8-membered heterocycloalkyl, wherein the 4- to 8-membered heterocycloalkyl contains 1 or more N, O, or S heteroatoms or heteroatom groups;
W₁ is CH₂, O, NH, or S;
m is 0, 1, 2, or 3;
R^{3e}, R^{4e}, R^{5e}, X', and Y' are as defined and described in the present disclosure; 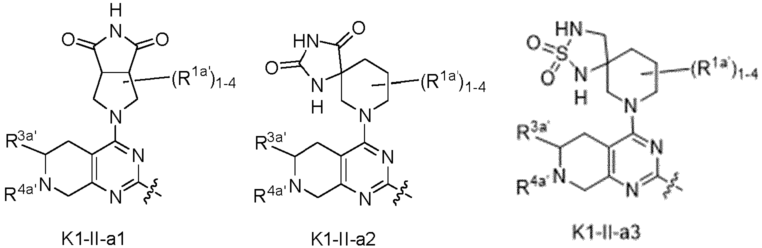 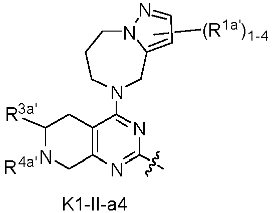
wherein in K1-II-a1, K1-II-a2, K1-II-a3, or K1-II-a4:
   R^{1a'} is hydrogen, hydroxy, halogen, cyano, C₁-C₆ alkyl, C₃-C₈ cycloalkyl, HC(=O)-, -CO₂R^{5a'}, - CON(R^{5a'})₁₋₂, or 5- to 6-membered heteroaryl, wherein the C₁₋C₆ alkyl or C₃-C₈ cycloalkyl is optionally substituted with halogen, hydroxy, cyano, or amino;
   each R^{5a'} is independently selected from hydrogen and C₁-C₆ alkyl;
   or two R^{5a'}, together with the N atom linked thereto, form 4- to 8-membered heterocycloalkyl, wherein the 4- to 8-membered heterocycloalkyl contains 1 or more N, O, or S heteroatoms or heteroatom groups;
   R^{4a'} is selected from 6- to 10-membered aryl and 5- to 10-membered heteroaryl, wherein the 6- to 10-membered aryl or 5- to 10-membered heteroaryl may be optionally substituted with one or more R^{8a'};
   R^{5a'} is independently selected from H and C₁-C₃ alkyl;
   each R^{8a'} is independently halogen, deuterium, cyano, hydroxy, amino, C₁-C₆ alkyl, C₁-C₆ heteroalkyl, C₃-C₆ cycloalkyl, -S-C₁-C₃ alkyl, C₂-C₄ alkenyl, C₂-C₄ alkynyl, triazolyl, -O-C₁-C₆ alkyl, N(R^{5a'})₁₋₂, or -O-C₁-C₆ alkyl, wherein the C₁₋C₆ alkyl, C₁-C₆ heteroalkyl, C₃-C₆ cycloalkyl, -S-C₁-C₃ alkyl, C₂-C₄ alkenyl, C₂-C₄ alkynyl, or -O-C₁-C₆ alkyl is optionally substituted with halogen, hydroxy, cyano, or amino;
   R^{3a'} is hydrogen or oxo.

Preferably, in certain embodiments of the present disclosure, K1 is K1-I-a1, K1-I-a2, K1-I-a3, K1-I-a4, K1-I-a5, K1-I-a6, K1-I-a7, K1-II-a1, K1-II-a2, K1-II-a3, K1-II-a4, K1-II-a5, or K2-I-a1: 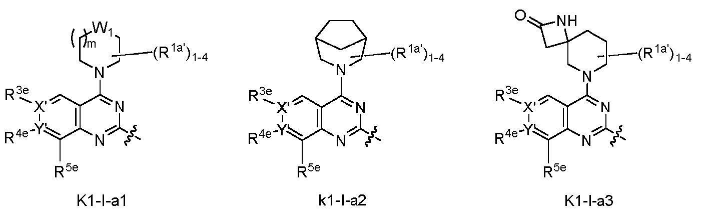 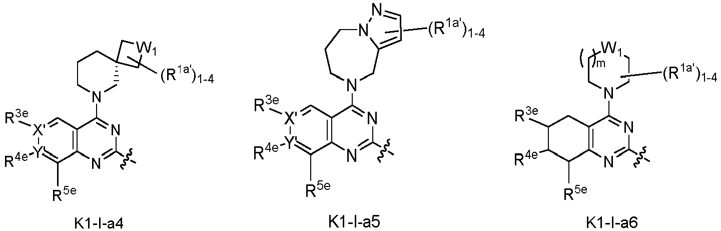 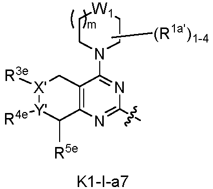 wherein in K1-I-a1, K1-I-a2, K1-I-a3, K1-I-a4, K1-I-a5, K1-I-a6, or K1-I-a7:
R^{1a'} is hydrogen, hydroxy, halogen, cyano, amino, C₁-C₆ alkyl, C₃-C₈ cycloalkyl, HC(=O)-, - CO₂R^{5a'}, -CON(R^{5a'})₁₋₂, or 5- to 6-membered heteroaryl, wherein the C₁₋C₆ alkyl or C₃-C₈ cycloalkyl is optionally substituted with halogen, hydroxy, cyano, or amino;
each R^{5a'} is independently selected from hydrogen and C₁-C₆ alkyl;
or two R^{5a'}, together with the N atom linked thereto, form 4- to 8-membered heterocycloalkyl, wherein the 4- to 8-membered heterocycloalkyl contains 1 or more N, O, or S heteroatoms or heteroatom groups;
W₁ is CH₂, O, NH, or S;
m is 0, 1, 2, or 3;
R^{3e}, R^{4e}, R^{5e}, X', and Y' are as defined and described in K1-I-a of the present disclosure; 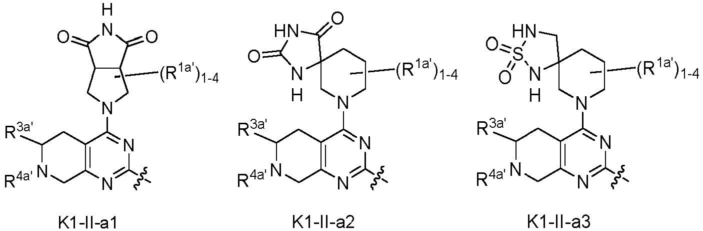 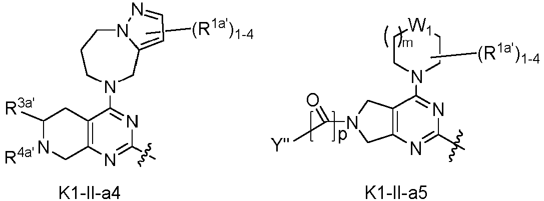
wherein in K1-II-a1, K1-II-a2, K1-II-a3, K1-II-a4, or K1-II-a5:
   R^{1a'} is hydrogen, hydroxy, halogen, cyano, C₁-C₆ alkyl, C₃-C₈ cycloalkyl, HC(=O)-, -CO₂R^{5a'}, - CON(R^{5a'})₁₋₂, or 5- to 6-membered heteroaryl, wherein the C₁₋C₆ alkyl or C₃-C₈ cycloalkyl is optionally substituted with halogen, hydroxy, cyano, or amino;
   each R^{5a'} is independently selected from hydrogen and C₁-C₆ alkyl;
   or two R^{5a'}, together with the N atom linked thereto, form 4- to 8-membered heterocycloalkyl, wherein the 4- to 8-membered heterocycloalkyl contains 1 or more N, O, or S heteroatoms or heteroatom groups;
   R^{4a'} is selected from 6- to 10-membered aryl and 5- to 10-membered heteroaryl, wherein the 6- to 10-membered aryl or 5- to 10-membered heteroaryl may be optionally substituted with one or more R^{8a'};
   R^{5a'} is independently selected from H and C₁-C₃ alkyl;
   each R^{8a'} is independently halogen, deuterium, cyano, hydroxy, amino, C₁-C₆ alkyl, C₁-C₆ heteroalkyl, C₃-C₆ cycloalkyl, -S-C₁-C₃ alkyl, C₂-C₄ alkenyl, C₂-C₄ alkynyl, triazolyl, N(R^{5a'})₁₋₂, or -O-C₁-C₆ alkyl, wherein the C₁₋C₆ alkyl, C₁-C₆ heteroalkyl, C₃-C₆ cycloalkyl, -S-C₁-C₃ alkyl, C₂-C₄ alkenyl, C₂-C₄ alkynyl, or -O-C₁-C₆ alkyl is optionally substituted with halogen, hydroxy, cyano, or amino;
   R^{3a'} is hydrogen or oxo;
   W₁ is CH₂, O, NH, or S;
   m is 0, 1, 2, or 3;
   Y" is as defined and described in the present disclosure; 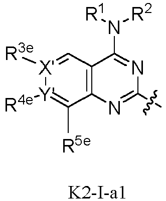
   wherein in K2-I-a1:
      R^{3e}, R^{4e}, R^{5e}, X', Y', R¹, and R² are as defined and described in the present disclosure.

Preferably, in certain embodiments of the present disclosure, K1 is K1-I-a1; 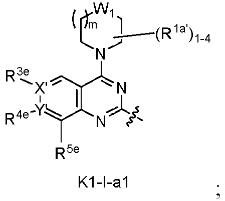
wherein X' is C or N; Y' is C
R^{3e} is H or absent; R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R^{1a'} is hydrogen, hydroxy, NH₂, halogen, or C₁-C₆ alkyl;
W₁ is CH₂ or O;
m is 0, 1, 2, or 3;
K2 is K2-I-a1; 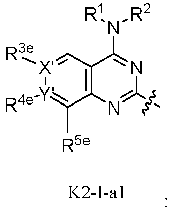
wherein X' is C or N; Y' is C;
R^{3e} is H or absent; R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R¹ and R² are independently hydrogen, C₁-C₆ alkyl, or C₃-C₈ cycloalkyl, wherein the C₁-C₆ alkyl or C₃-C₈ cycloalkyl is optionally substituted with one or more halogen, hydroxy, or amino.

Preferably, in certain embodiments of the present disclosure, K1 is K1-I-a1; 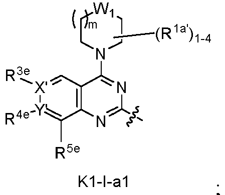
wherein X' is C or N; Y' is C;
R^{3e} is H or absent; R^{4e} is C₆-C₁₀ aryl, wherein the C₆-C₁₀ aryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R^{1a'} is hydrogen, hydroxy, or C₁-C₆ alkyl;
W₁ is CH₂ or O;
m is 1 or 2;
K2 is K2-I-a1; 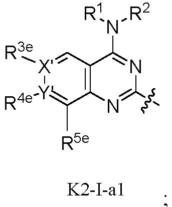
wherein X' is N; Y' is C;
R^{3e} is absent; R^{4e} is C₆-C₁₀ aryl, wherein the C₆-C₁₀ aryl is optionally substituted with 1 or more R^{a'};
R^{a'} is independently selected from halogen, hydroxy, and C₂-C₆ alkynyl;
R^{5e} is halogen;
R¹ and R² are independently hydrogen, C₁-C₆ alkyl, or C₃-C₈ cycloalkyl.

Preferably, in certain embodiments of the present disclosure, the structural unit 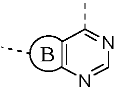 is 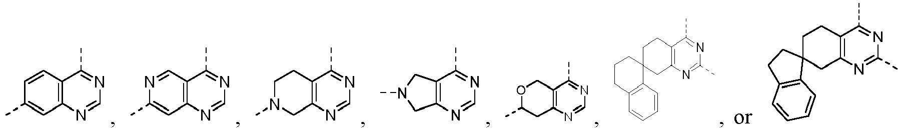 ; the structural unit 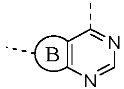 is optionally substituted with F, Cl, Br, I, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -(3- to 8-membered heterocycloalkyl), -O(C₁-C₆ alkyl), -O(C₃-C₈ cycloalkyl), -S(C₁-C₆ alkyl), -S(C₃-C₈ cycloalkyl), -N(C₁-C₆ alkyl)₁₋₂, or -N(C₃-Cs cycloalkyl)₁₋₂.

Preferably, in certain embodiments of the present disclosure, the structural unit 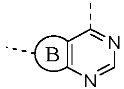 is 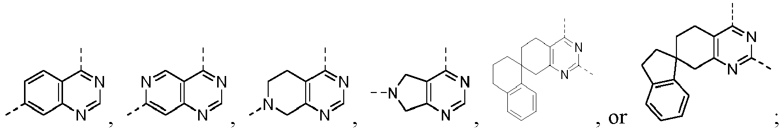 the structural unit 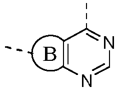 is optionally substituted with F, Cl, Br, I, hydroxy, amino, cyano, -C₁-C₆ alkyl, -C₁-C₆ heteroalkyl, -C₃-C₈ cycloalkyl, -C₃-C₆ alkenyl, -C₃-C₆ alkynyl, -C₃-C₈ heterocycloalkyl, -O(C₁-C₆ alkyl), - O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂.

More preferably, in certain embodiments of the present disclosure, the structural unit 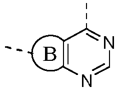 is 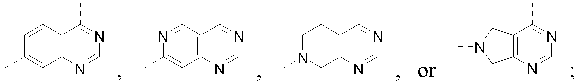 the structural unit 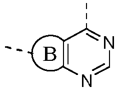 is optionally substituted with F, Cl Br, I, hydroxy, amino, cyano, -C₁-C₆ alkyl, -C₁-C₆ heteroalkyl, -C₃-C₈ cycloalkyl, -C₃-C₆ alkenyl, -C₃-C₆ alkynyl, -C₃-C₈ heterocycloalkyl, -O(C₁-C₆ alkyl), - O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂.

Preferably, in certain embodiments of the present disclosure, ring A1 is C₆-C₁₅ aryl or 5- to 15-membered heteroaryl, wherein the C₆-C₁₅ aryl or 5- to 15-membered heteroaryl is optionally substituted with -OH, F, Cl, Br, I, CN, -NH₂, -CH₃, -CH₂CH₃, -CH₂CH₂CH₃, -CH(CH₃)₂-, -OCH₃, OCH₂CH₃, -C≡CH, 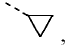 or -SCH₃, the -CH₃, -CH₂CH₃, -CH₂CH₂CH₃, -CH(CH₃)₂, 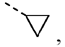 or -SCH₃ being optionally substituted with halogen, CN, -OH, -NH₂, or C₁-C₃ alkyl.

Preferably, in certain embodiments of the present disclosure, ring A1 is C₆-C₁₀ aryl or 5- to 10-membered heteroaryl, wherein the C₆-C₁₀ aryl or 5- to 10-membered heteroaryl is optionally substituted with -OH, F, Cl, Br, I, CN, -NH₂, -CH₃, -CH₂CH₃, -CH₂CH₂CH₃, -CH(CH₃)₂-, -OCH₃, OCH₂CH₃, -C≡CH, 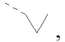 or -SCH₃, the -CH₃, -CH₂CH₃, -CH₂CH₂CH₃, -CH(CH₃)₂, 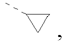 or -SCH₃ being optionally substituted with halogen, CN, -OH, -NH₂, or C₁-C₃ alkyl.

More preferably, in certain embodiments of the present disclosure, ring A1 is phenyl, pyridyl, naphthyl, 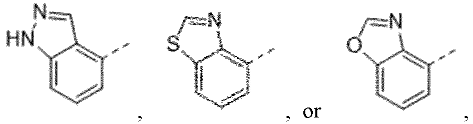 wherein the phenyl, pyridyl, naphthyl, 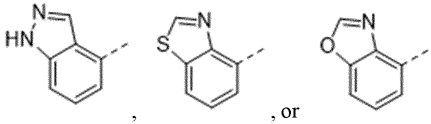 is optionally substituted with -OH, F, Cl, Br, I, CN, -NH₂, -CH₃, -CH₂CH₃, -CH₂CH₂CH₃, -CH(CH₃)₂-, -OCH₃, OCH₂CH₃, -C≡CH, 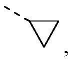 or -SCH₃, the -CH₃, - CH₂CH₃, -CH₂CH₂CH₃, -CH(CH₃)₂, 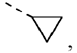 or -SCH₃ being optionally substituted with halogen, CN, -OH, -NH₂, or C₁-C₃ alkyl.

Preferably, in certain embodiments of the present disclosure, the structural unit 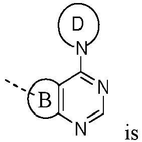 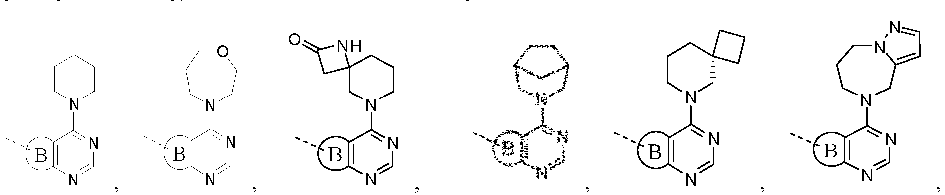 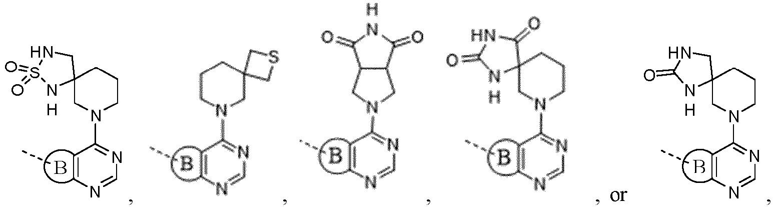 and the structural unit 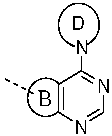 is optionally substituted with 1, 2, or 3 R^{a}.

Preferably, in certain embodiments of the present disclosure, the structural unit 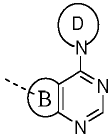 is 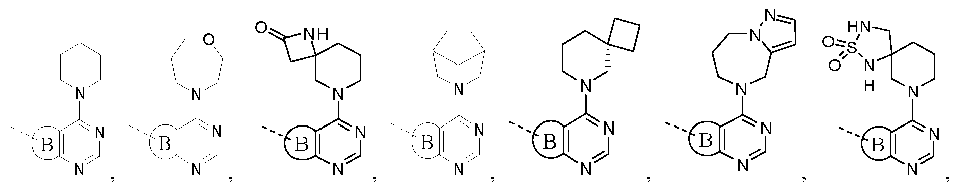 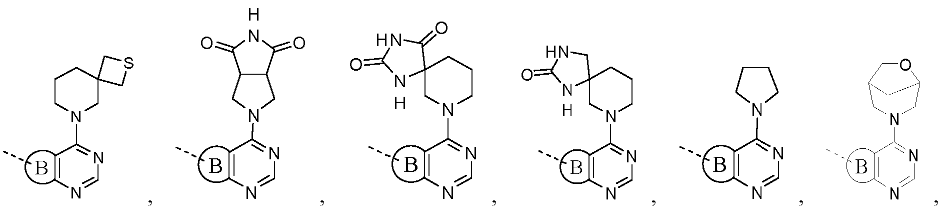 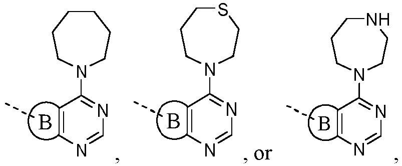 and the structural unit 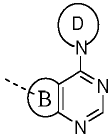 is optionally substituted with 1, 2, or 3 R^{a}.

Preferably, in certain embodiments of the present disclosure, the structural unit 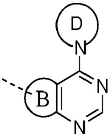 is 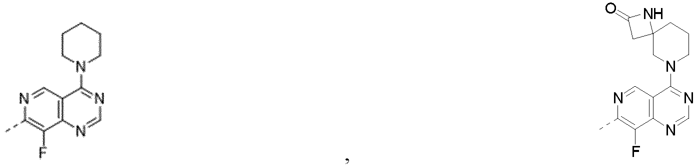 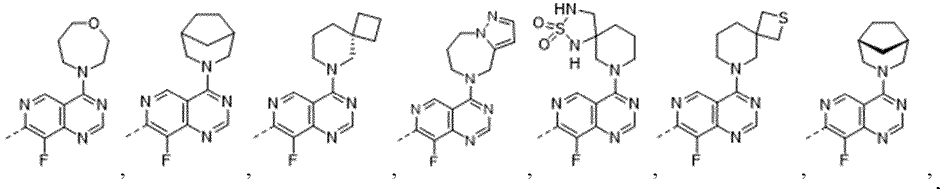 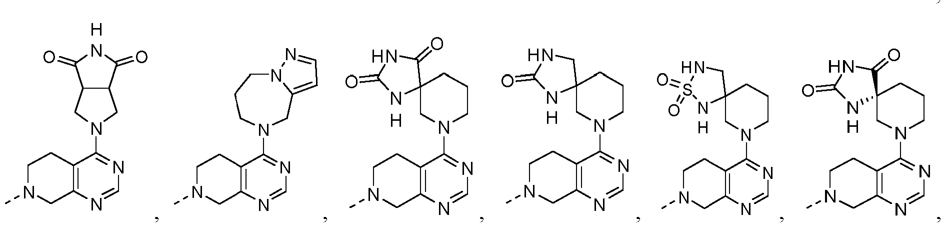 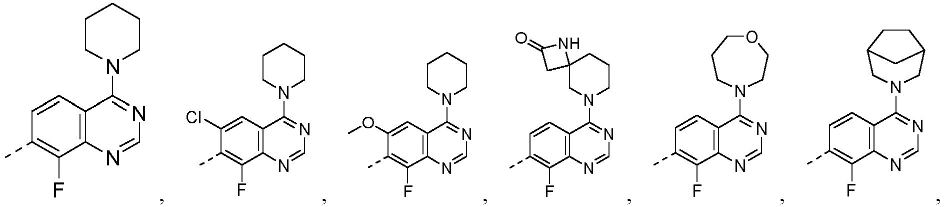 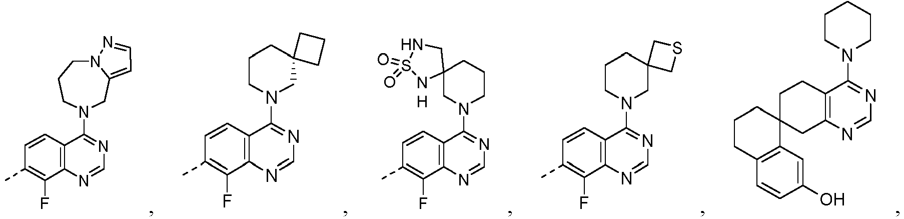 or 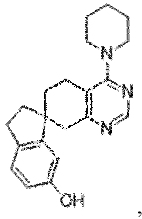 and the structural unit 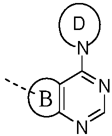 is optionally substituted with 1, 2, or 3 R^{a}.

Preferably, in certain embodiments of the present disclosure, the structural unit 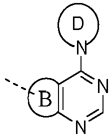 is 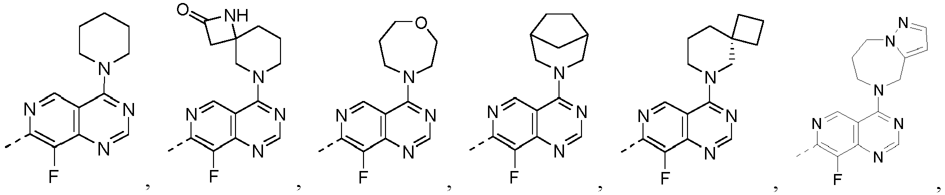 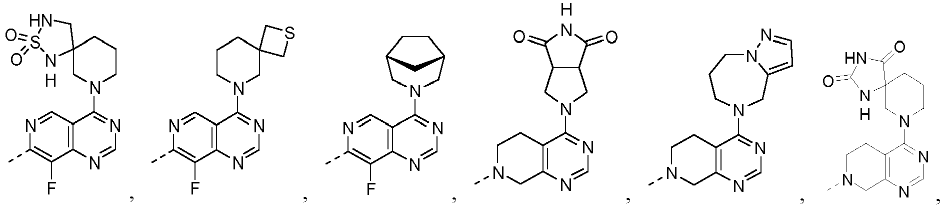 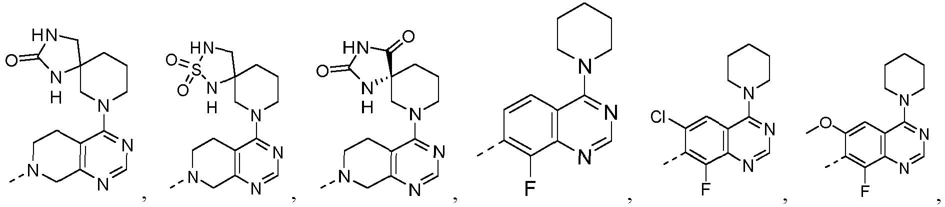 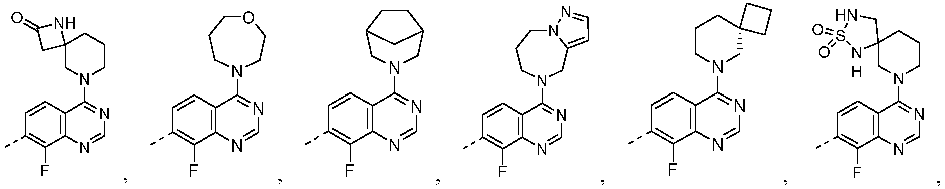 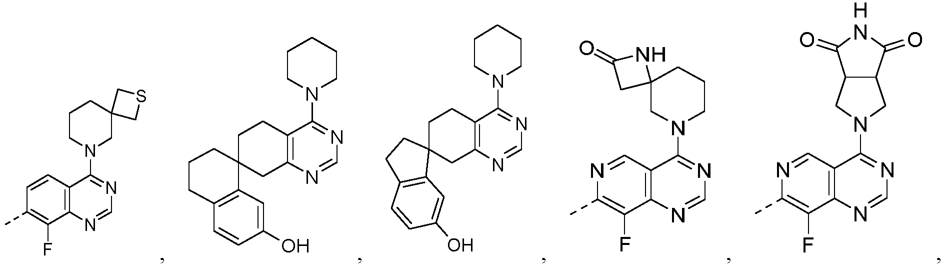 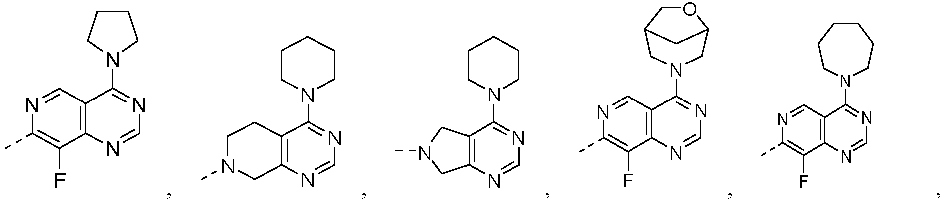 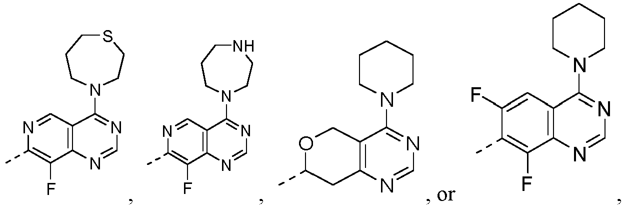 and the structural unit 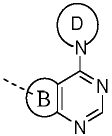 is optionally substituted with 1, 2, or 3 R^{a}.

More preferably, in certain embodiments of the present disclosure, the structural unit 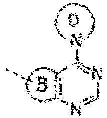 is

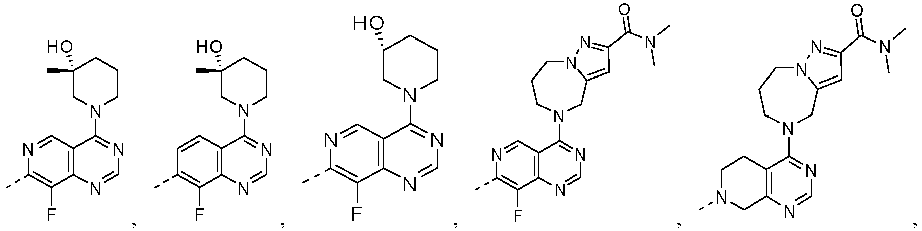

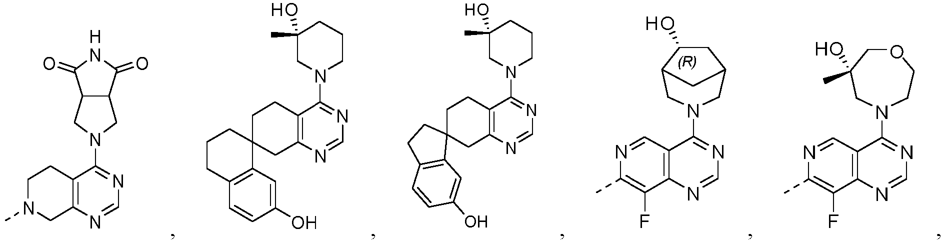

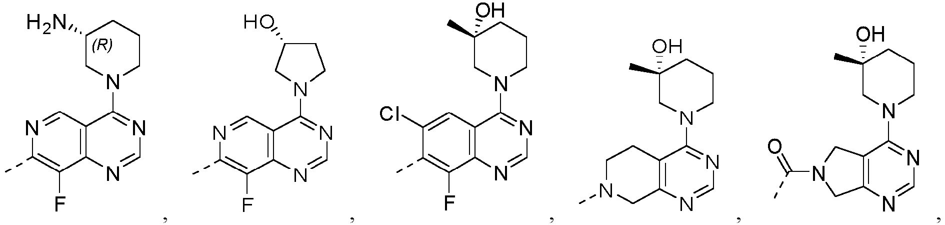

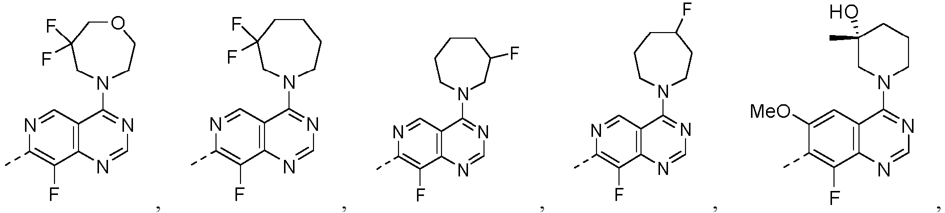

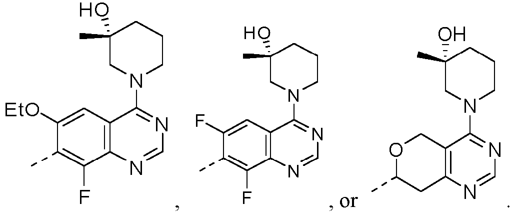

More preferably, in certain embodiments of the present disclosure, the structural unit 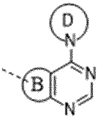 is

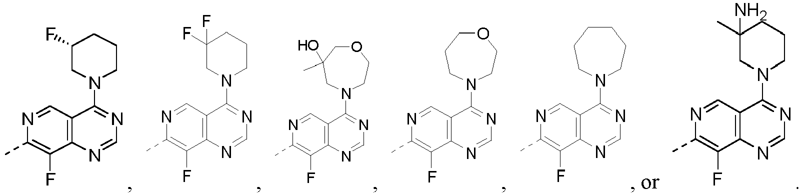

More preferably, in certain embodiments of the present disclosure, the structural unit

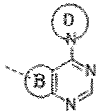

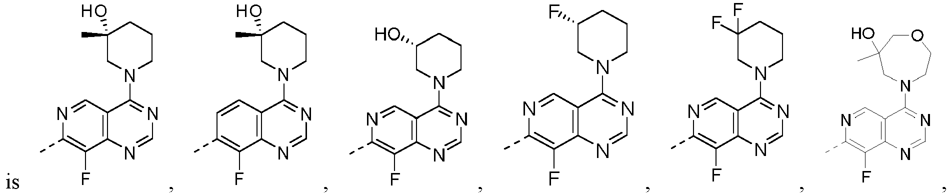

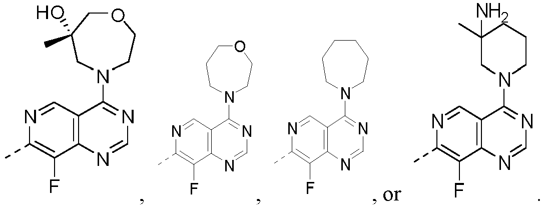

Preferably, in certain embodiments of the present disclosure, the structural unit 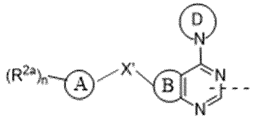 is: 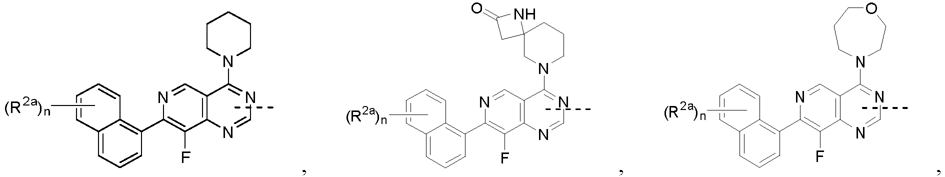 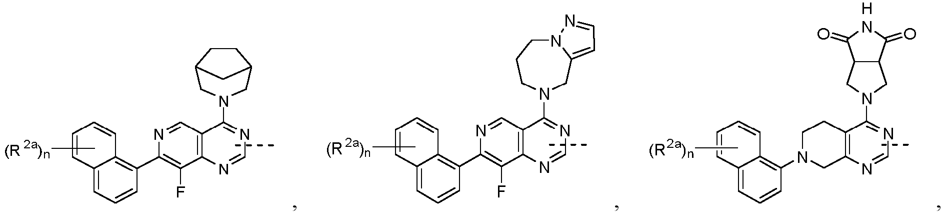 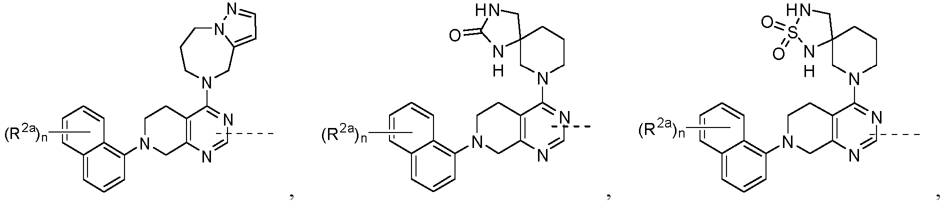 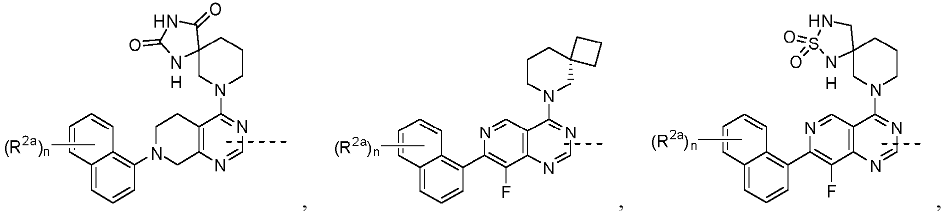 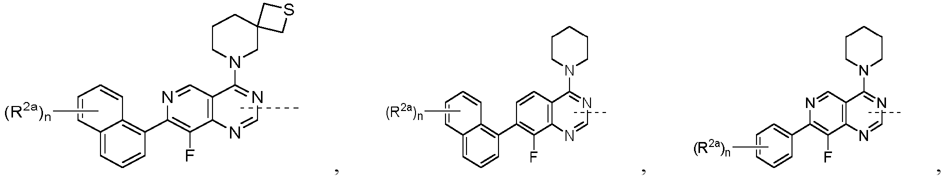 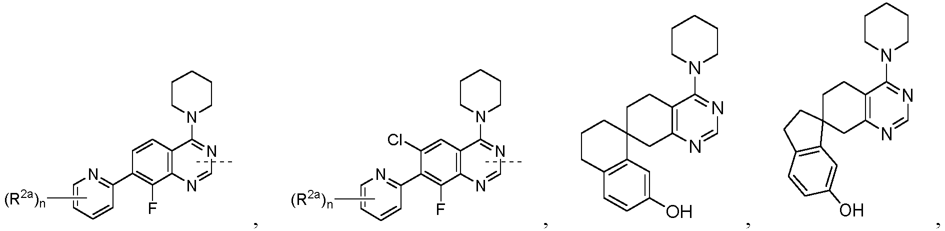 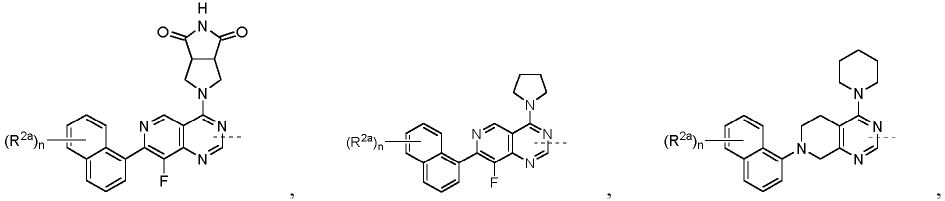 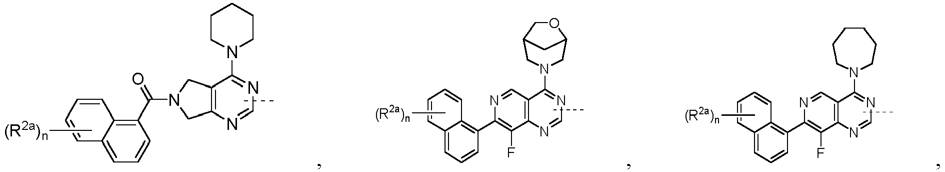 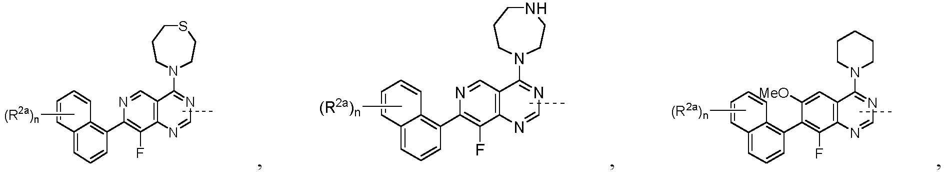 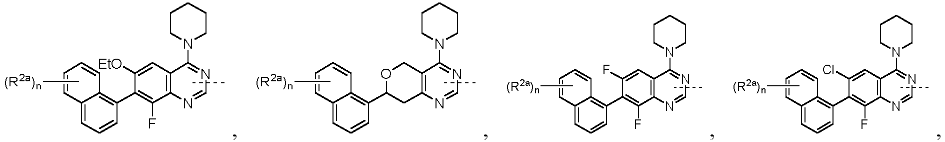 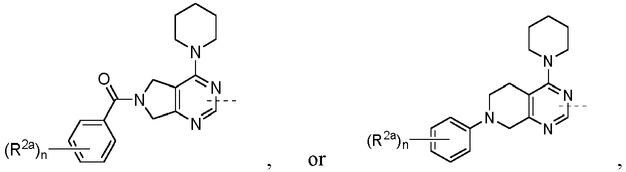 and the structural unit 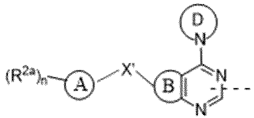 is optionally substituted with 1, 2, or 3 R^{a}.

Preferably, in certain embodiments of the present disclosure, the structural unit 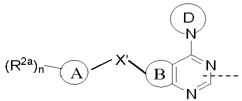 is: 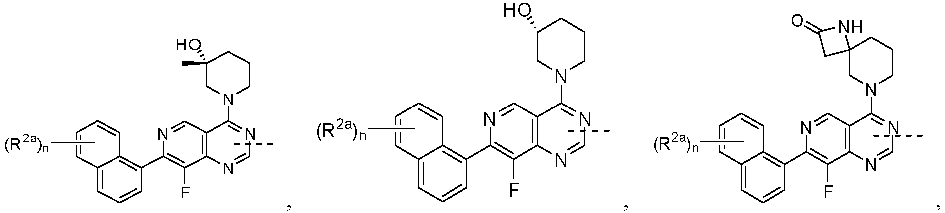 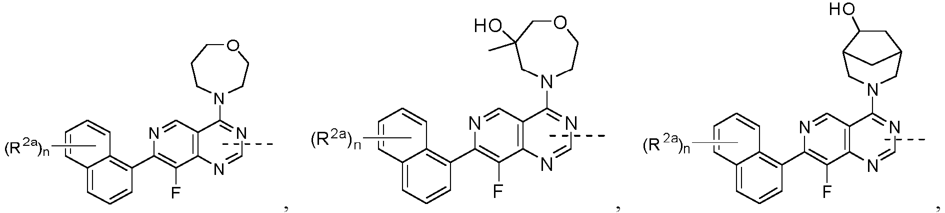 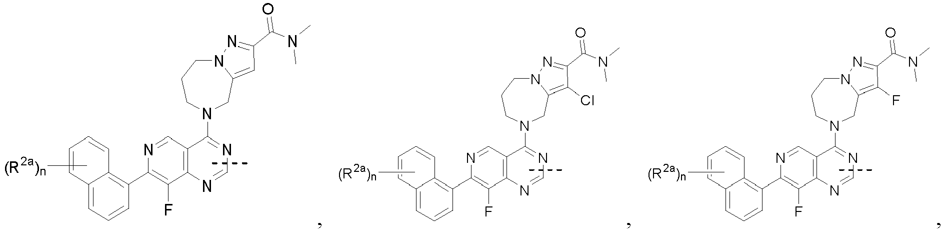 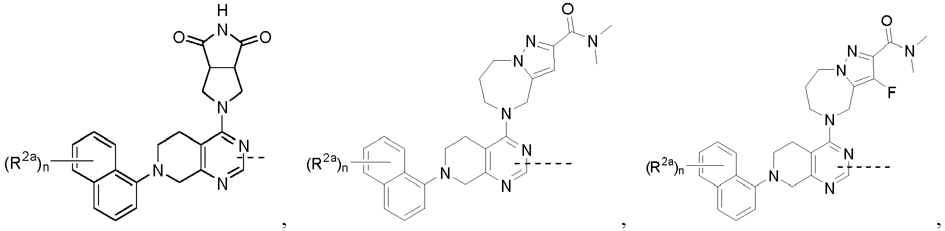 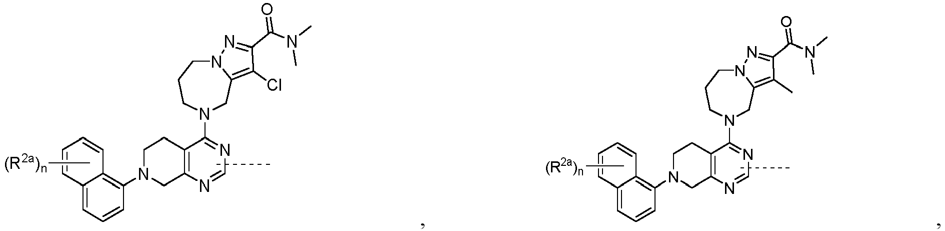 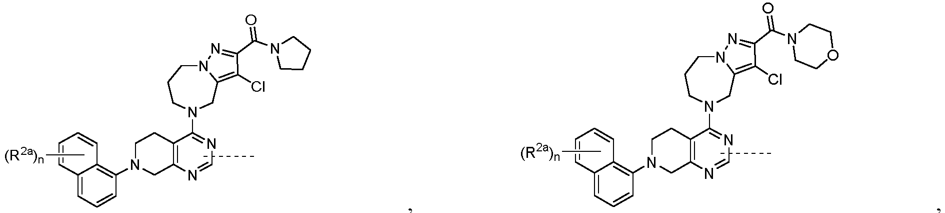 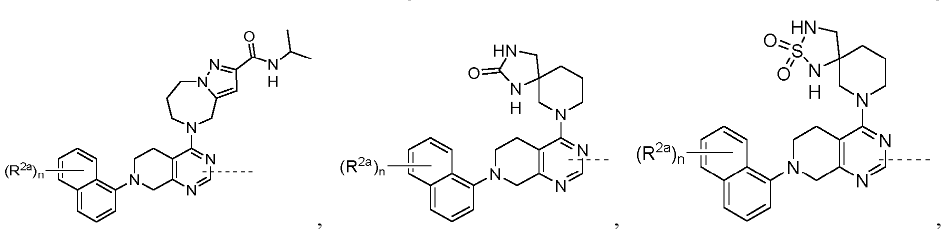 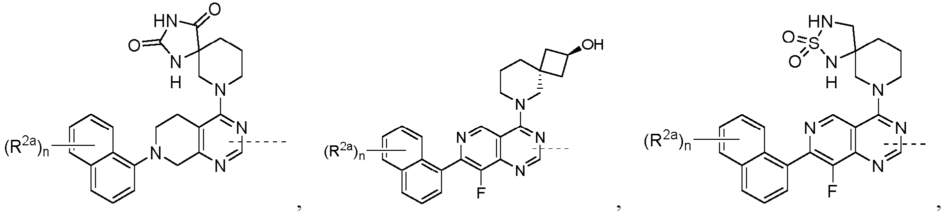 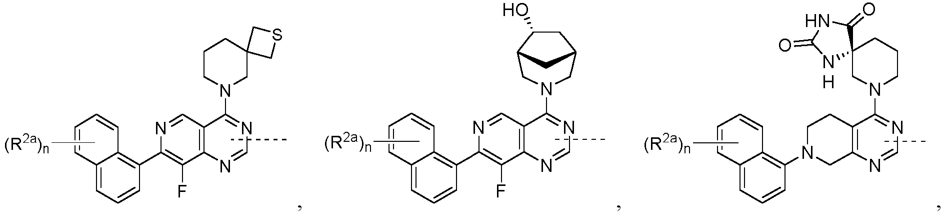 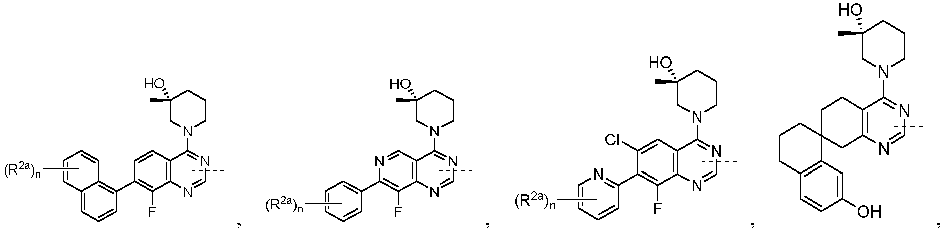 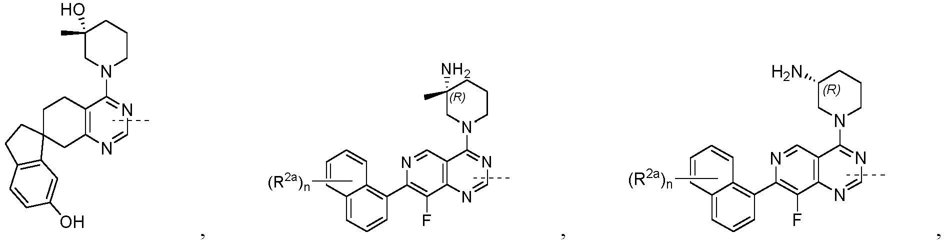 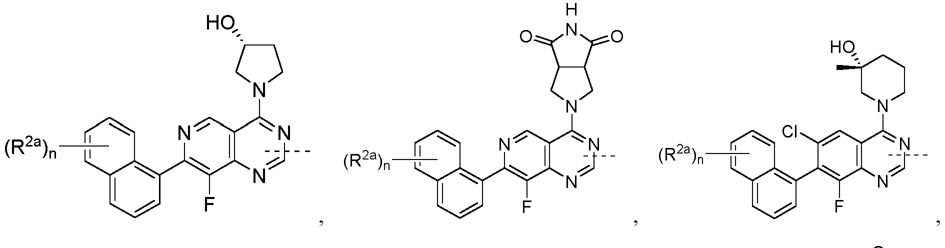 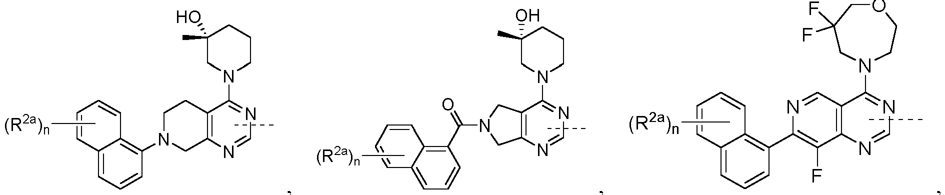 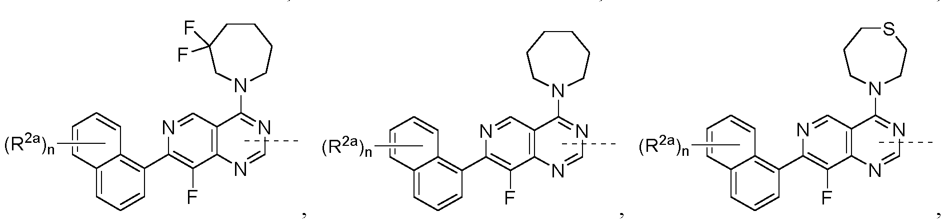 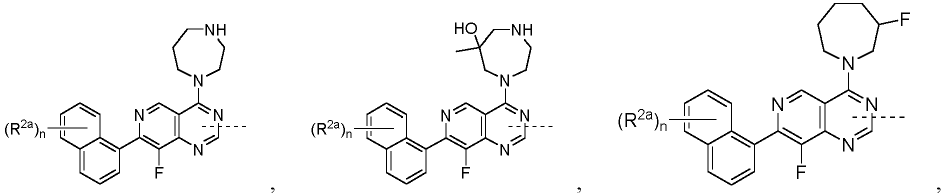 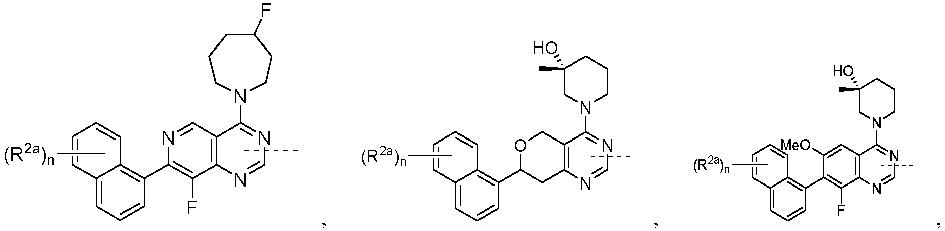 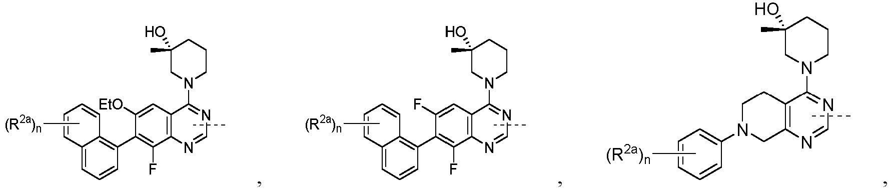 or

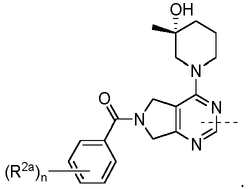

Preferably, in certain embodiments of the present disclosure, the structural unit 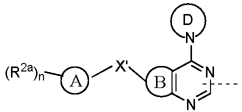 is

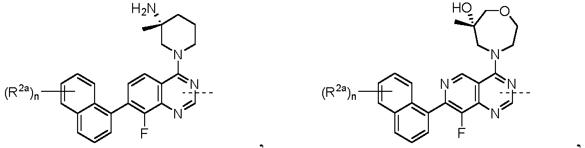

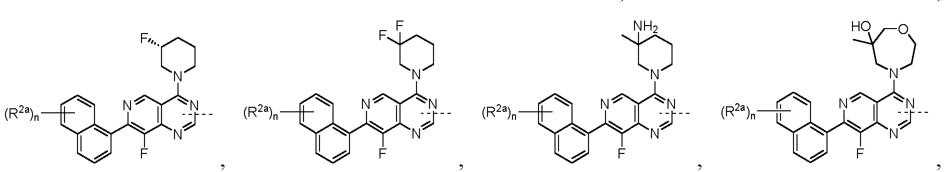

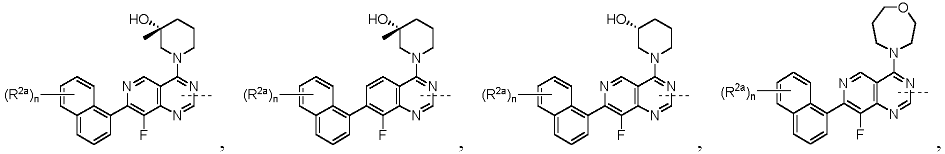

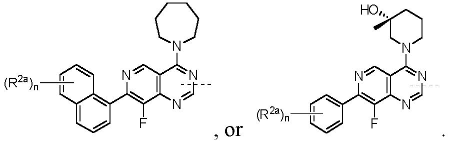

Preferably, in certain embodiments of the present disclosure, the structural unit 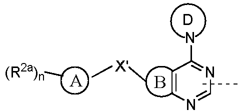 is 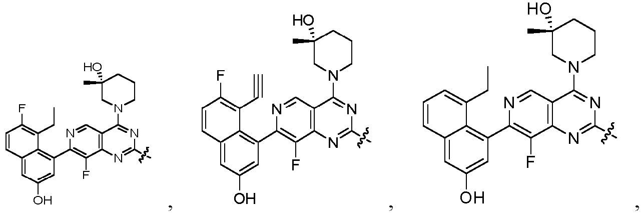 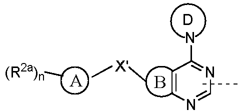 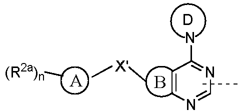 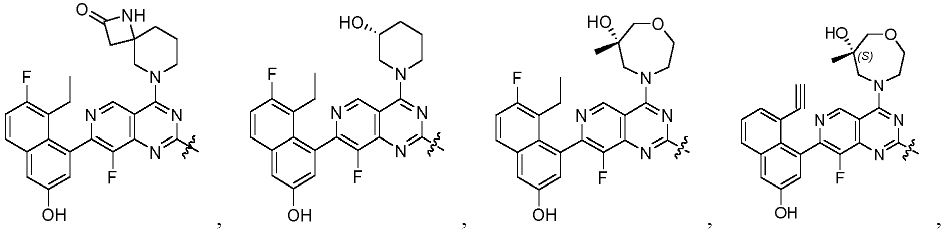 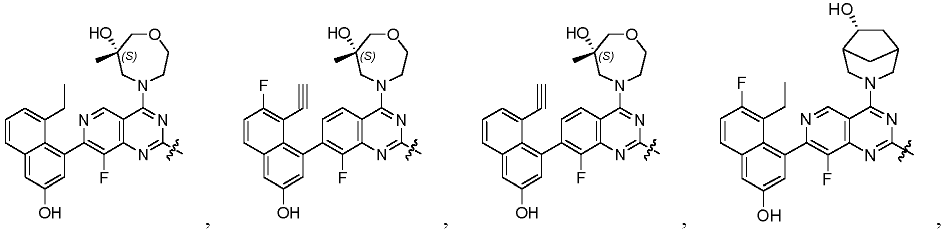 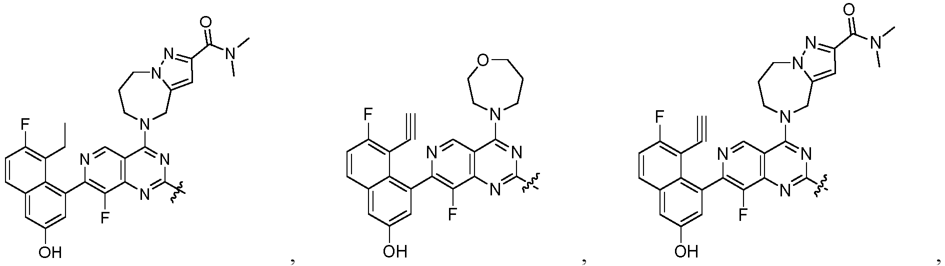 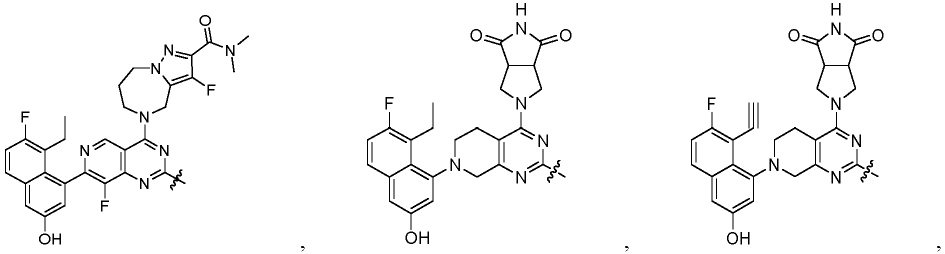 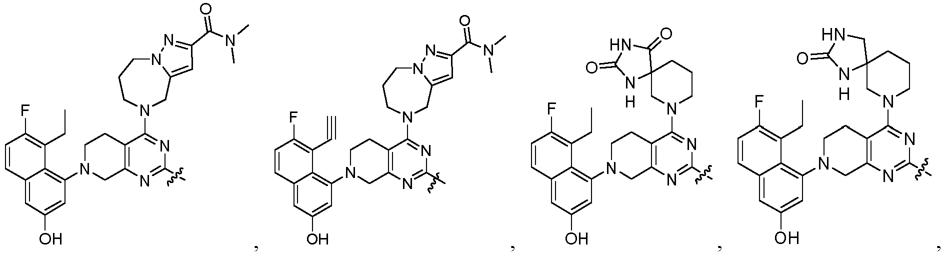 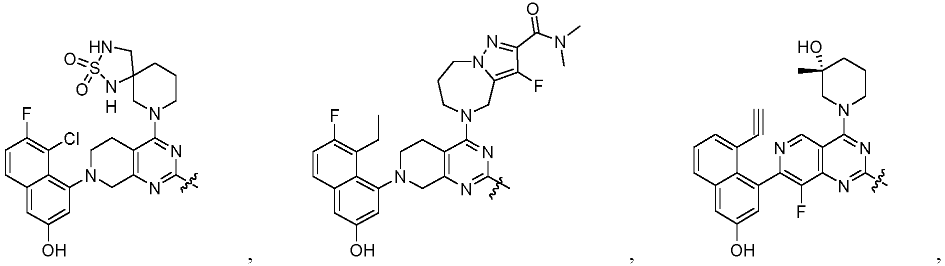 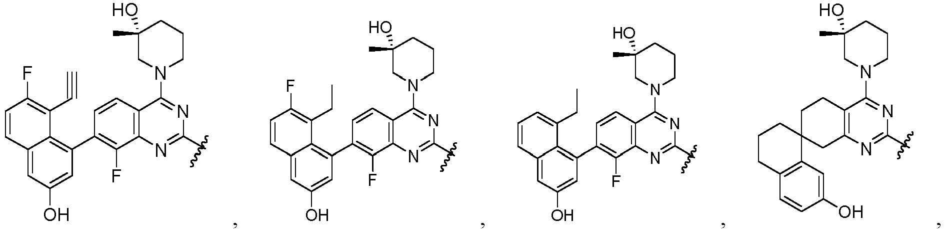 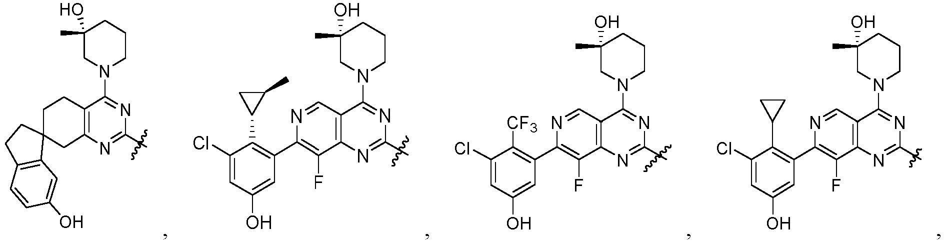 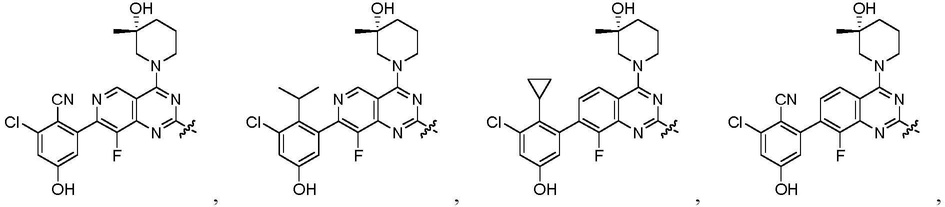 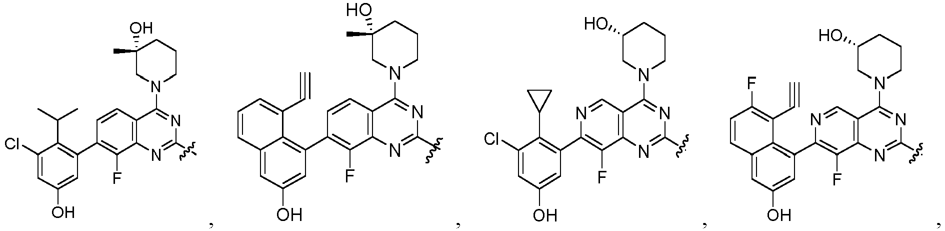 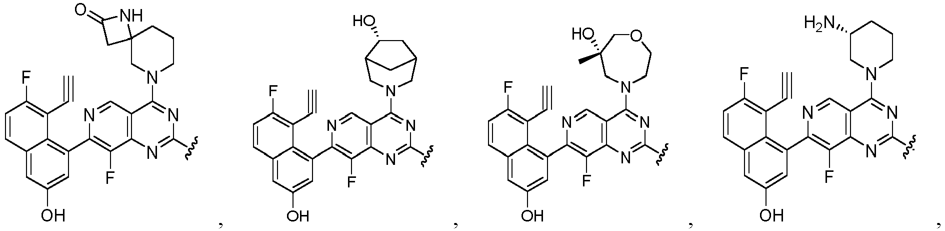 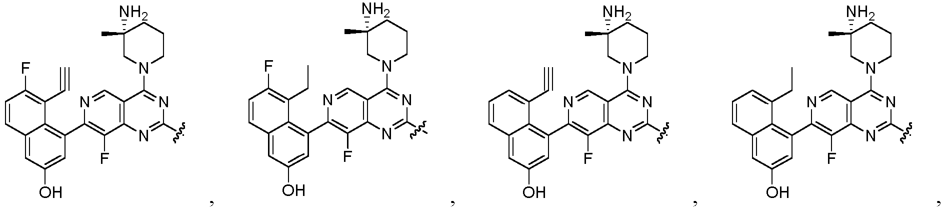 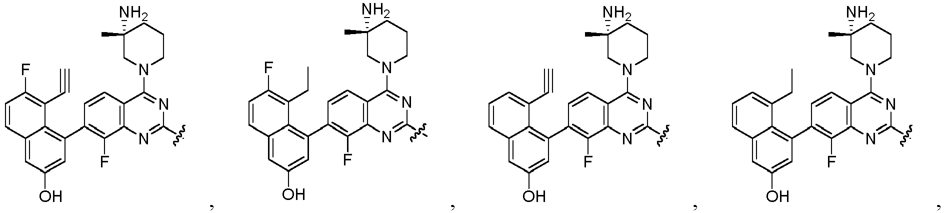 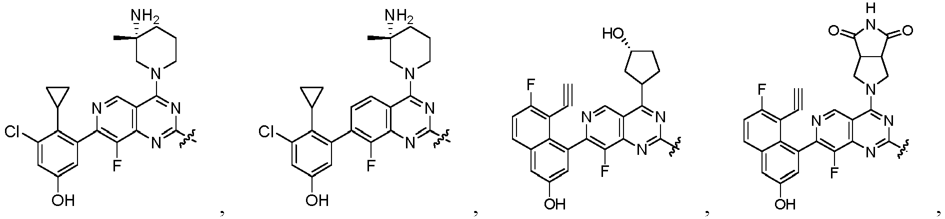 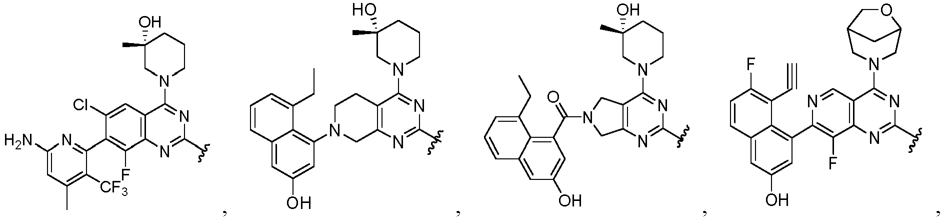 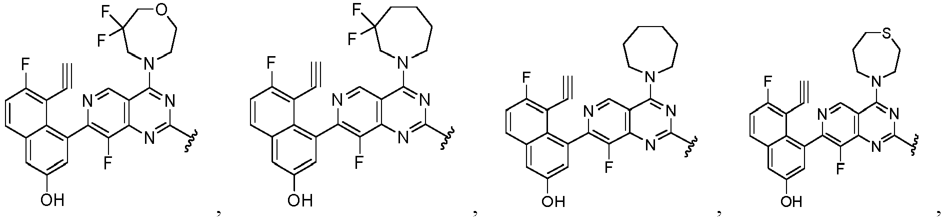 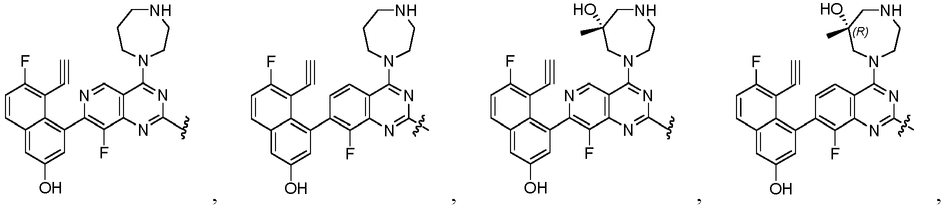 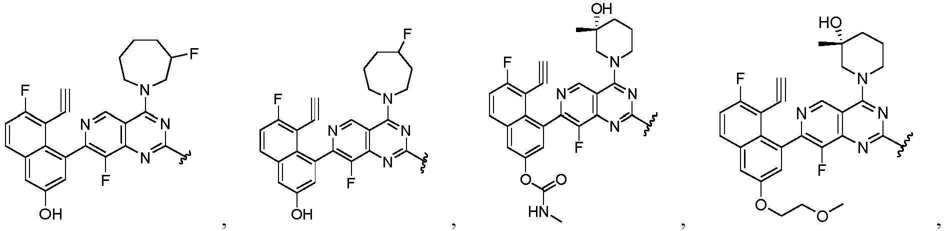 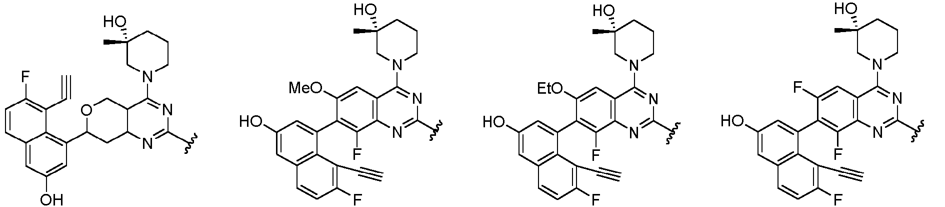 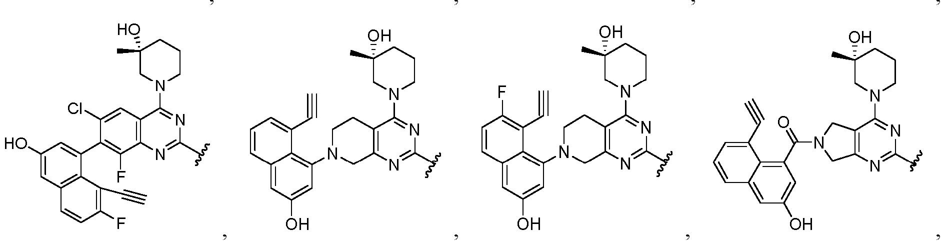 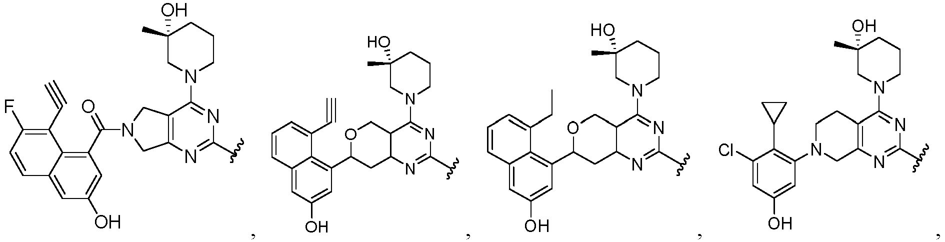 or

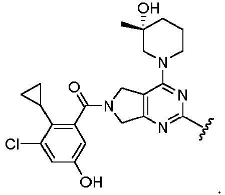

Preferably, in certain embodiments of the present disclosure, the structural unit 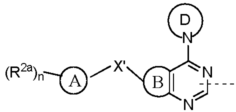 is

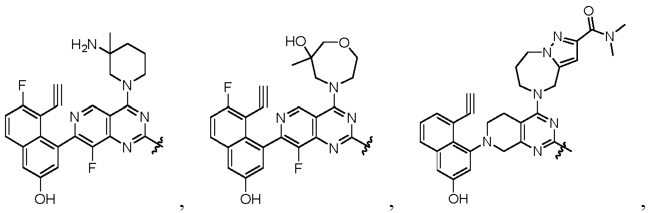

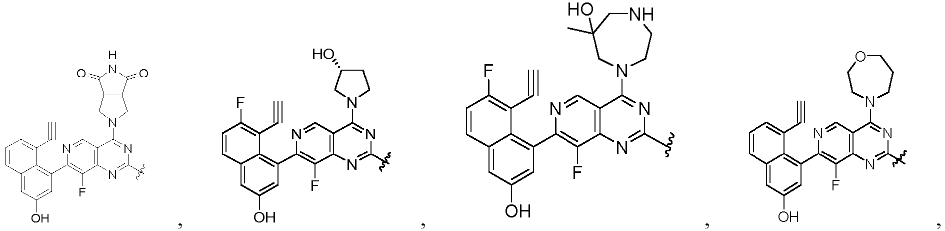

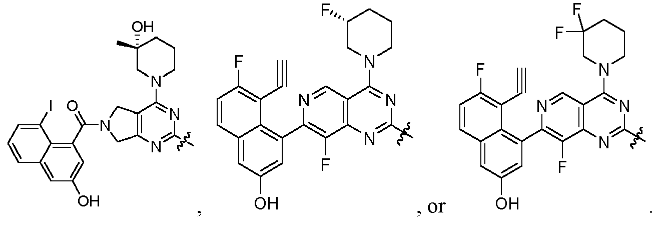

Preferably, in certain embodiments of the present disclosure, the structural unit 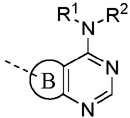 is 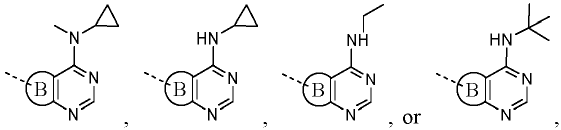 and the structural unit 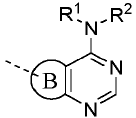 is optionally substituted with one or more deuterium, F, Cl, Br, hydroxy, amino, -CH₃, or -S-CH₃.

Preferably, in certain embodiments of the present disclosure, the structural unit 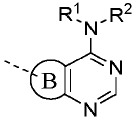 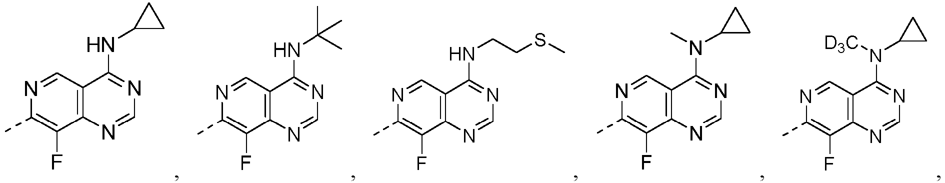 is

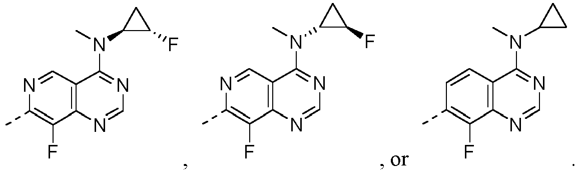

Preferably, in certain embodiments of the present disclosure, the structural unit 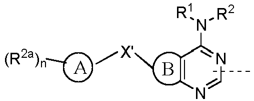 is: 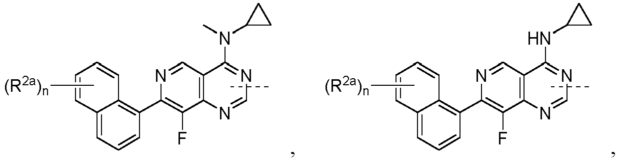 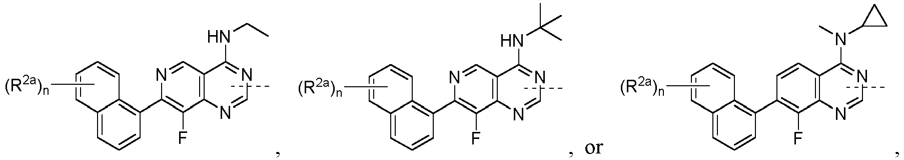 and the structural unit 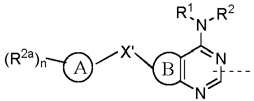 is optionally substituted with one or more deuterium, F, Cl, Br, hydroxy, amino, -CH₃, or -S-CH₃.

Preferably, in certain embodiments of the present disclosure, the structural unit 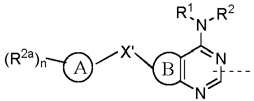 is:

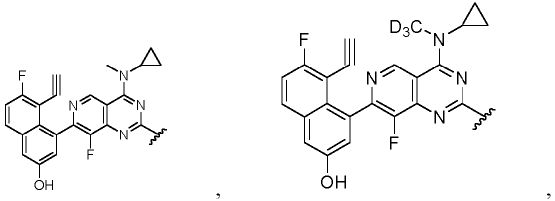

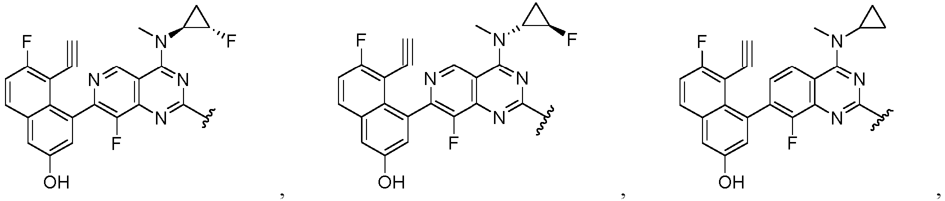

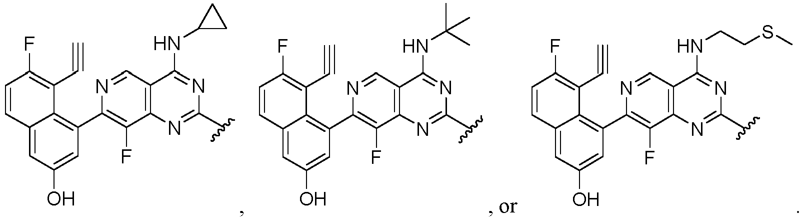

Preferably, in certain embodiments of the present disclosure, L is -(CH₂)ⱼ-, wherein 1 or more methylene groups in the -(CH₂)ⱼ- are optionally replaced by a group selected from -NR^{3'}-, -O-, -CR^{1'}R^{2'}-,-C(O)-, -S(O)-, -S(O)₂-, -C(O)O-, -OC(O)-, -C(O)NR^{3'}-, -NR^{3'}C(O)-, -S(O)₂NR^{3'}-, -NR^{3'}S(O)₂-, ethenylene, ethynylene, phenyl, 8- to 10-membered bicyclic arylene, saturated or partially unsaturated 3- to 7-membered cycloalkylene, saturated or partially unsaturated 5- to 11-membered spirocycloalkylene, saturated or partially unsaturated 5- to 11-membered fused cycloalkylene, saturated or partially unsaturated 8- to 10-membered bicyclic cycloalkylene, saturated or partially unsaturated 4- to 7-membered heterocycloalkylene having 1-2 heteroatoms independently selected from nitrogen, oxygen, and sulfur, saturated or partially unsaturated 5-to 11-membered spiroheterocycloalkylene having 1-2 heteroatoms independently selected from nitrogen, oxygen, and sulfur, saturated or partially unsaturated 5- to 11-membered fused heterocycloalkylene having 1-2 heteroatoms independently selected from nitrogen, oxygen, and sulfur, saturated or partially unsaturated 8- to 10-membered bicyclic heterocycloalkylene having 1-2 heteroatoms independently selected from nitrogen, oxygen, and sulfur, 5- to 6-membered heteroarylene having 1-4 heteroatoms independently selected from nitrogen, oxygen, and sulfur, and 8- to 10-membered bicyclic heteroaryl having 1-5 heteroatoms selected from nitrogen, oxygen, and sulfur, wherein the ethenylene, ethynylene, cycloalkylene, heterocycloalkylene, phenyl, spiroheterocycloalkylene, fused heterocycloalkylene, spirocycloalkylene, fused cycloalkylene, and heteroarylene are each independently optionally substituted with 1 or more substituents selected from halogen, oxo, -NR^{3'}R^{4'}, -OR^{3'}, nitro, -CN, C₁-C₆ alkyl, C₃-C₁₀ cycloalkyl, and C₃-C₁₀ heterocycloalkyl, the alkyl, cycloalkyl, or heterocycloalkyl being optionally substituted with 1 or more substituents selected from halogen, -OH, -NH₂, -CN, C₁-C₄ alkyl, and C₃-C₆ cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl),-NH(C₁-C₄ alkyl), -NH(C₁-C₄ alkyl), C₃-C₆ cycloalkyl, -O(C₃-C₆ cycloalkyl), -NH(C₃-C₆ cycloalkyl), C₃-C₆ heterocycloalkyl, -O(C₃-C₆ heterocycloalkyl), or -NH(C₃-C₆ cycloalkyl); R^{3'} and R^{4'} are each independently hydrogen, deuterium, C₁-C₄ alkyl, C₃-C₆ cycloalkyl, or C₃-C₆ heterocycloalkyl; j is 2, 3, 4, 5, 6, 7, 8, 9, 10, 11, or 12.

Preferably, in certain embodiments of the present disclosure, L is: 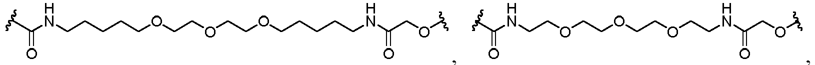 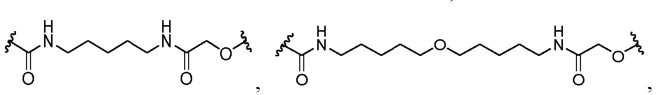 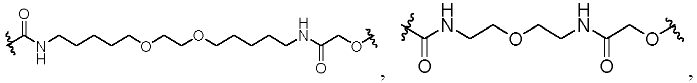 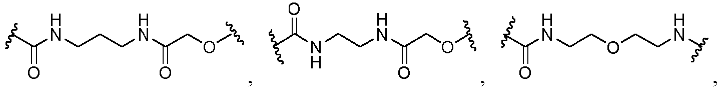 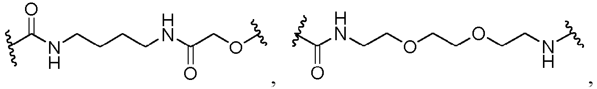 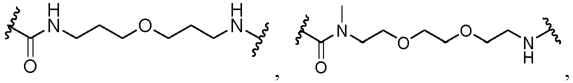 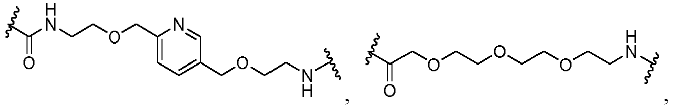 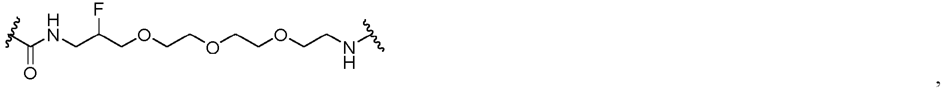 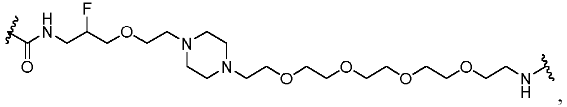 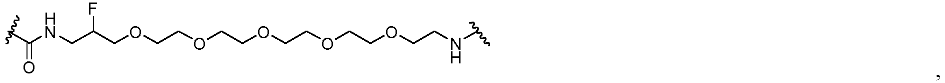 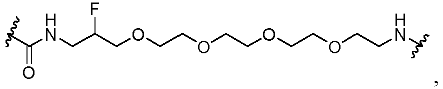 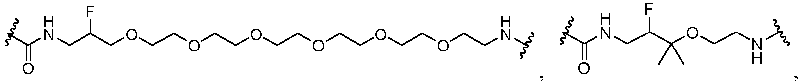 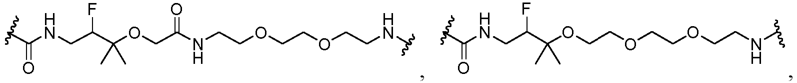 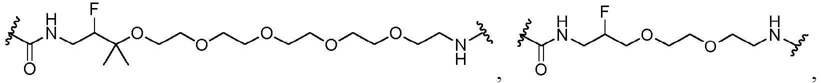 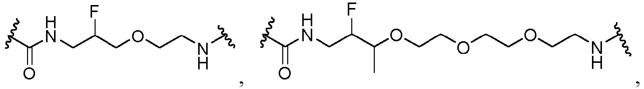 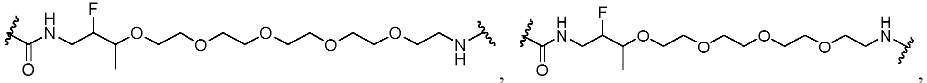 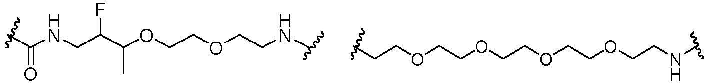 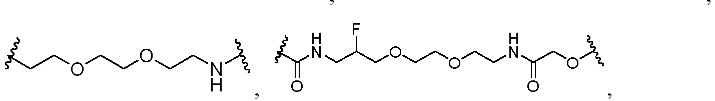 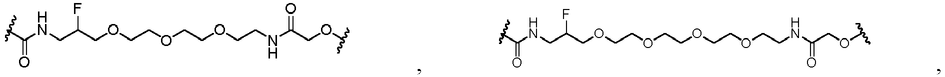 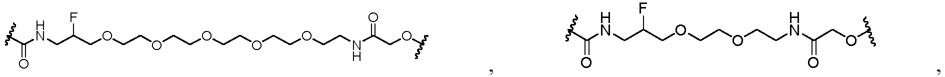 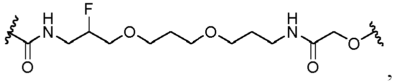 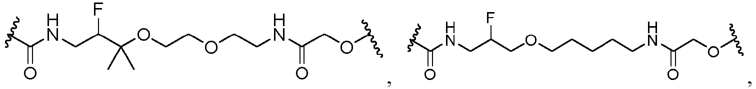 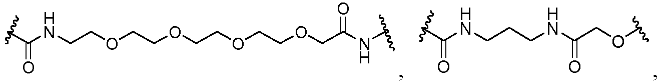 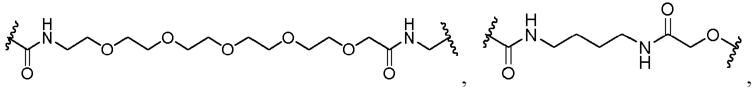 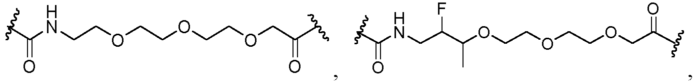 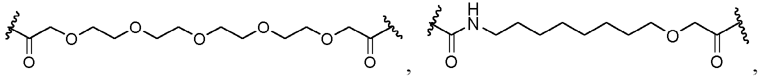 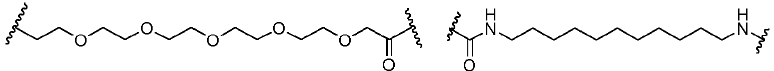 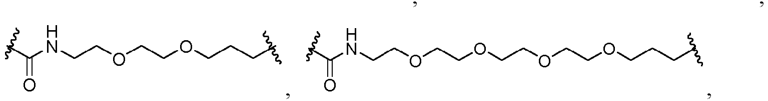 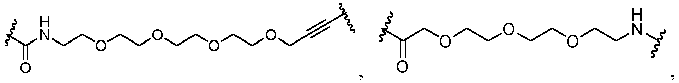 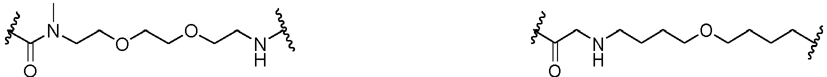 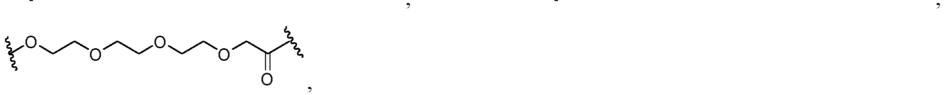 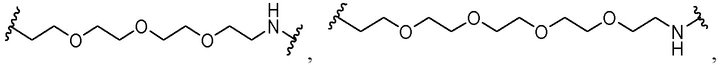 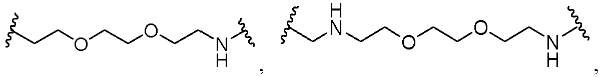 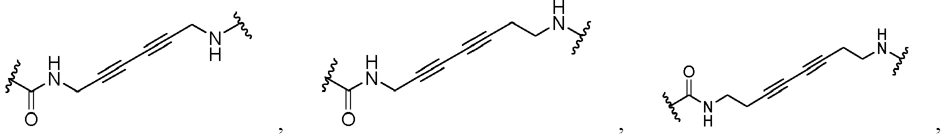 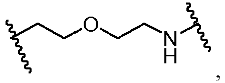 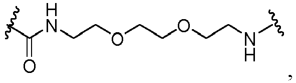 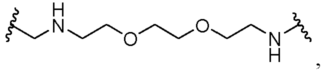 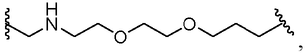 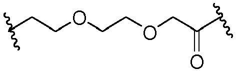 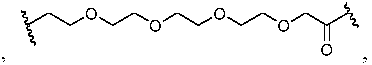 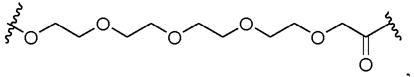 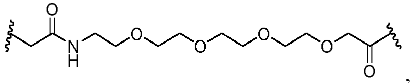 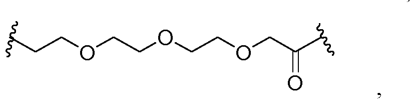 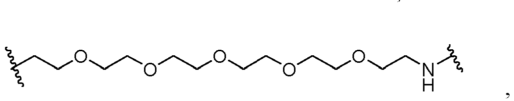 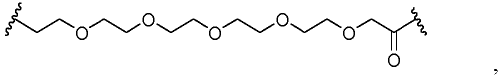 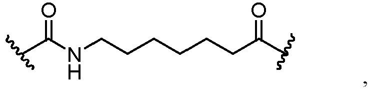 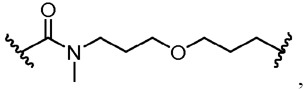 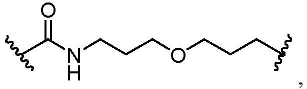 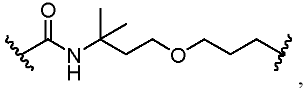 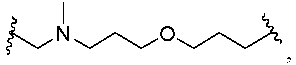 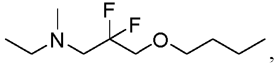 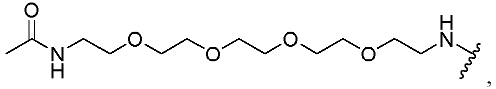 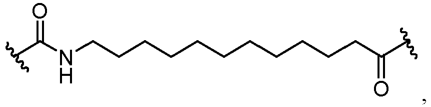 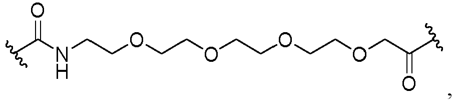 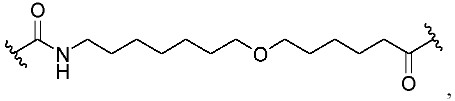 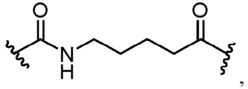 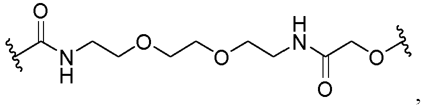 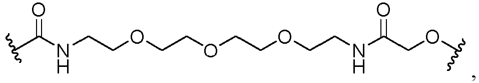 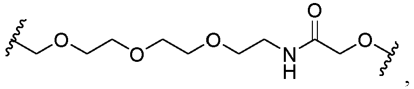 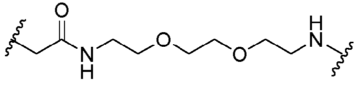 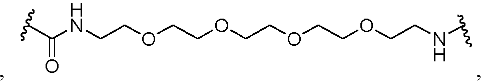 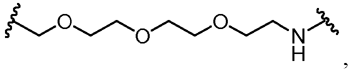 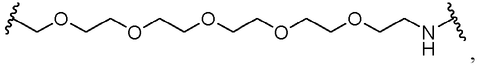 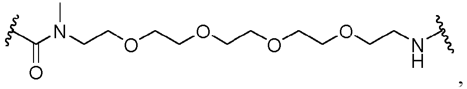 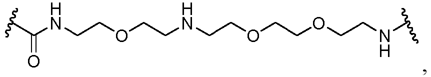 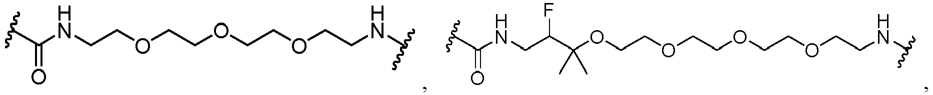 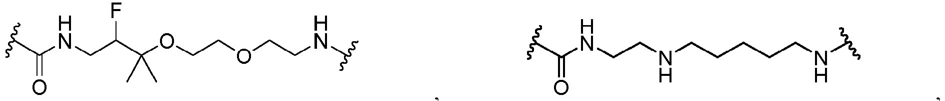 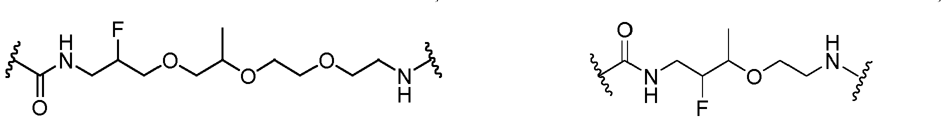 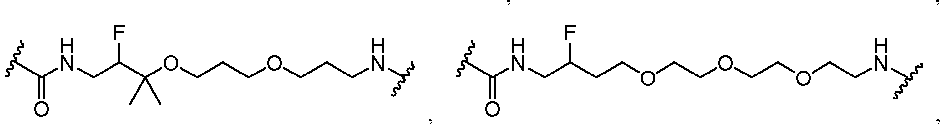 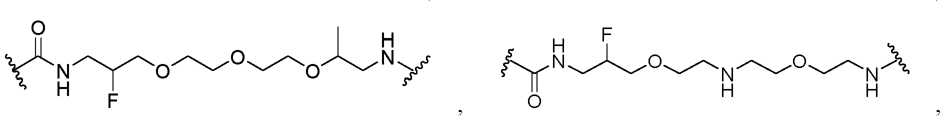 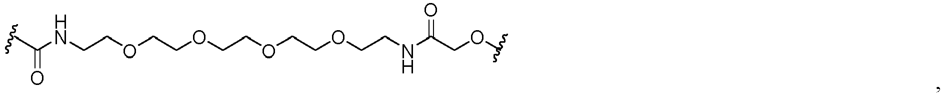 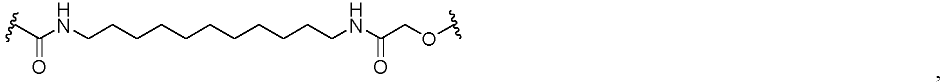 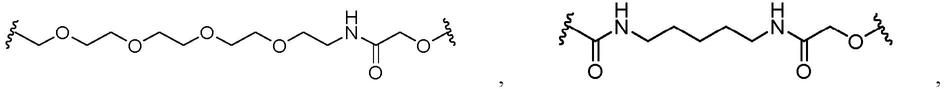 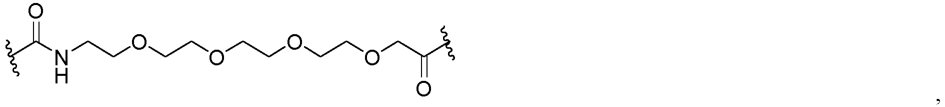 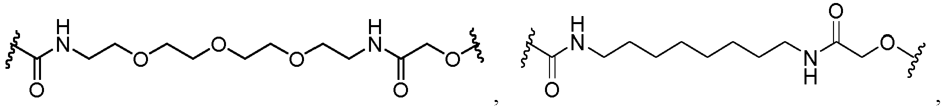 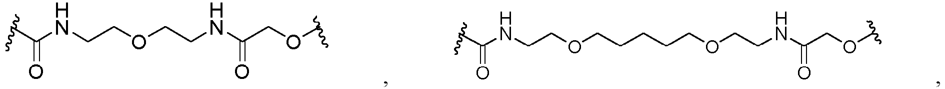 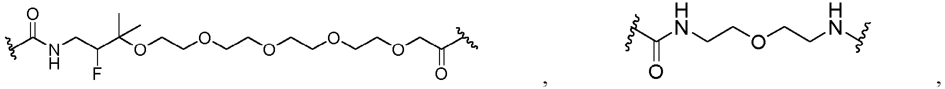 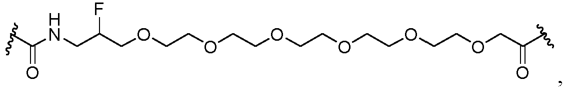 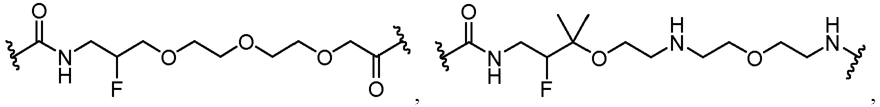 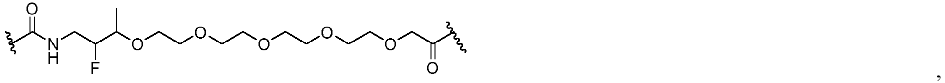 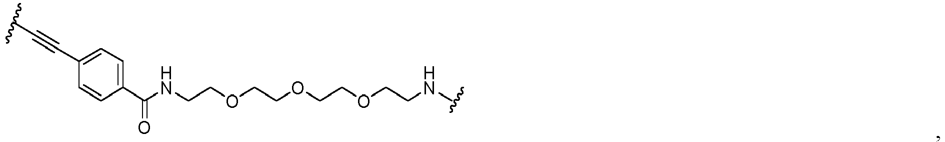 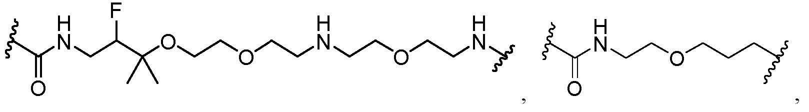 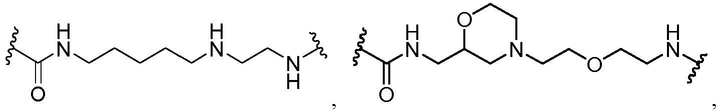 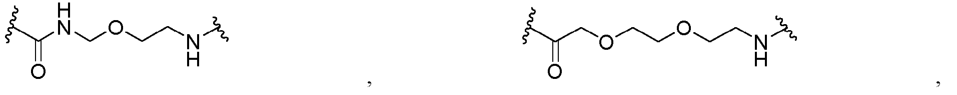 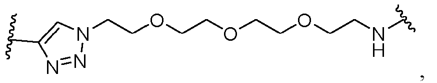 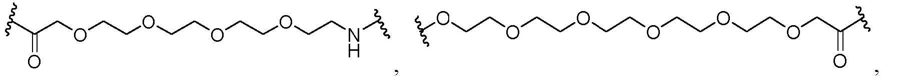 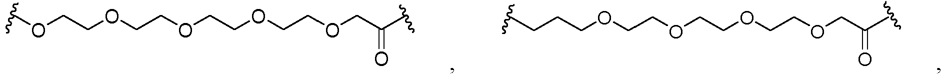 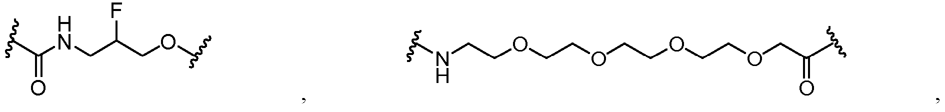 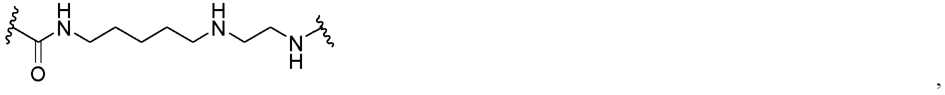 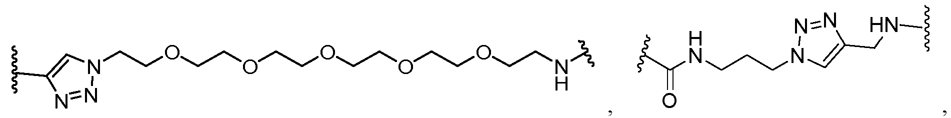 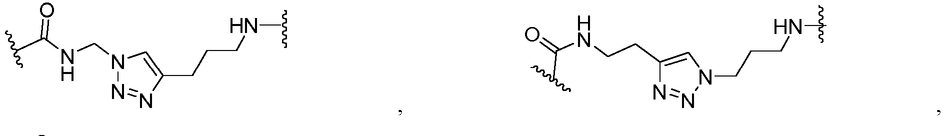 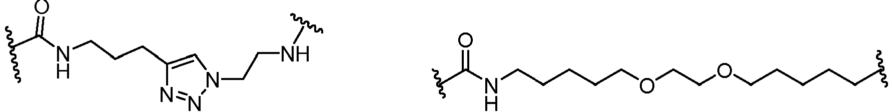 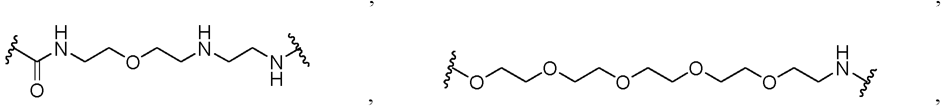 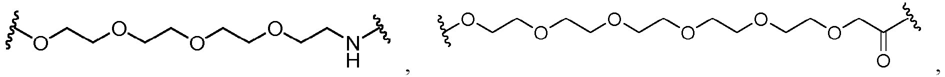 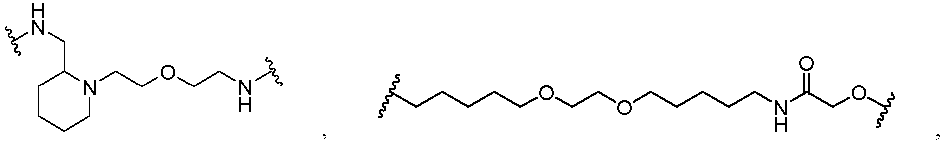 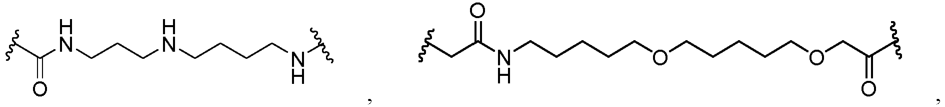 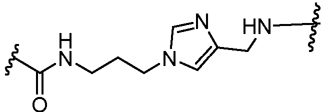 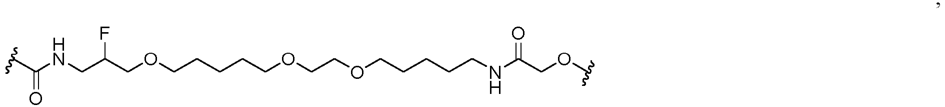 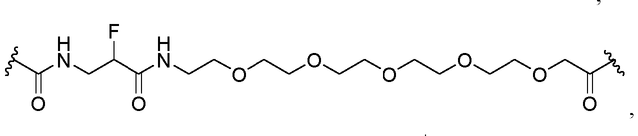 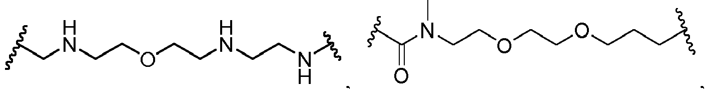 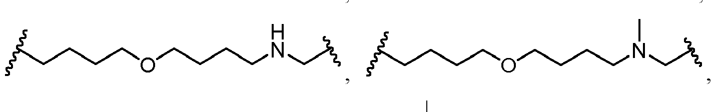 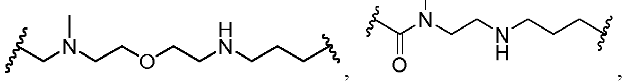 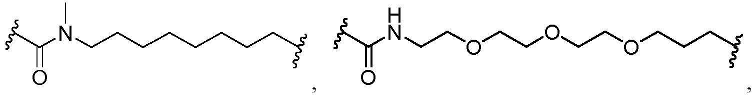 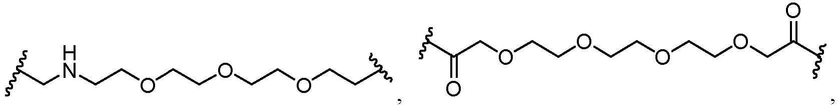 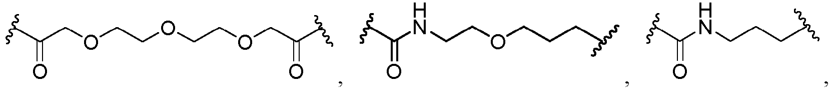 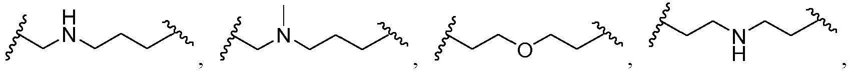 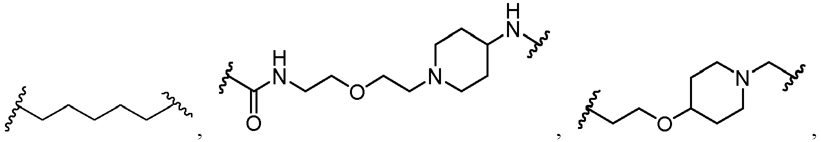 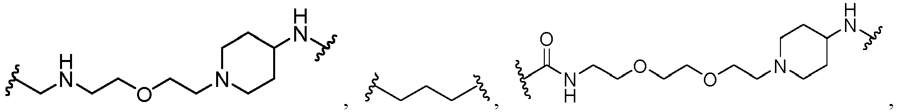 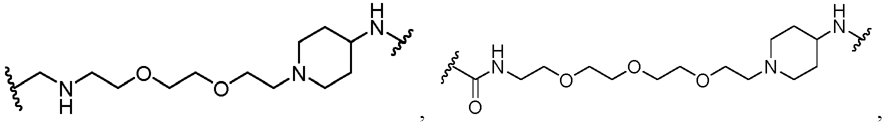 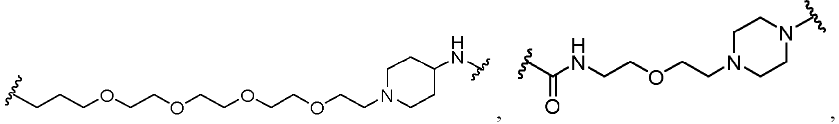 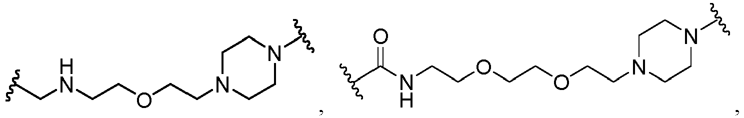 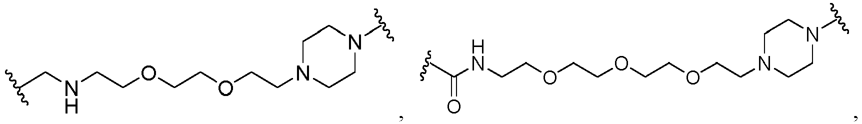 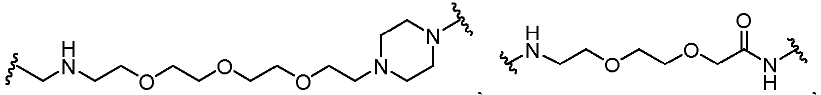 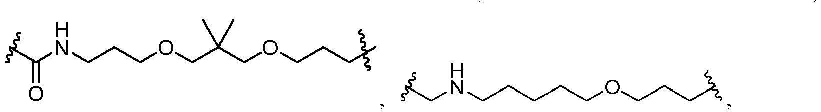 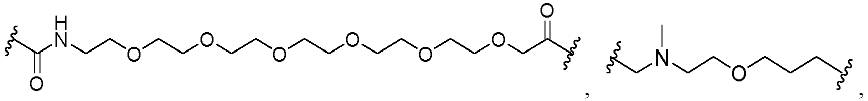 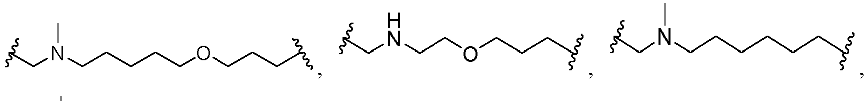 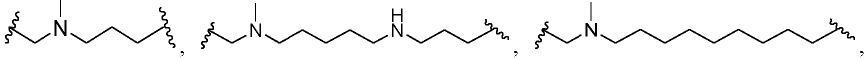 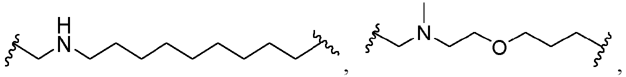 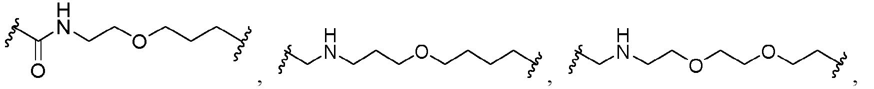 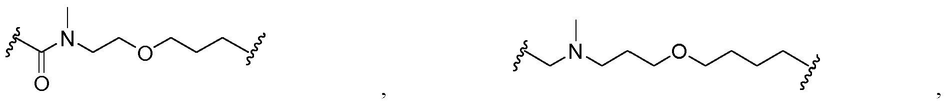 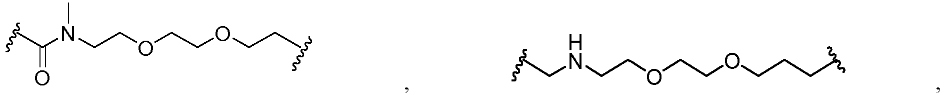 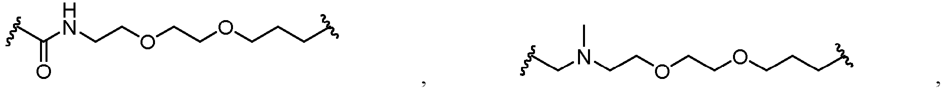 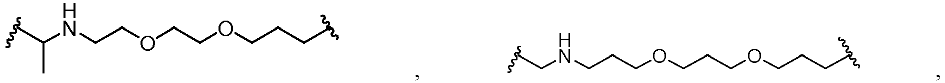 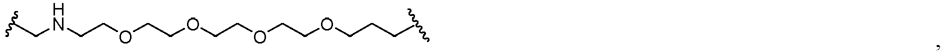 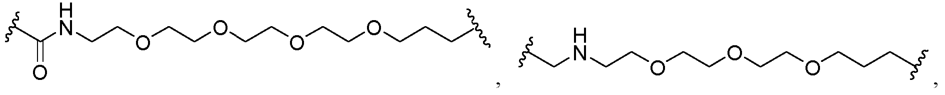 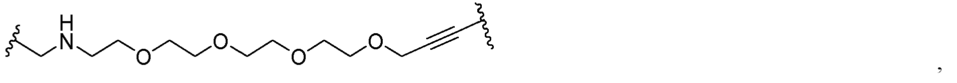 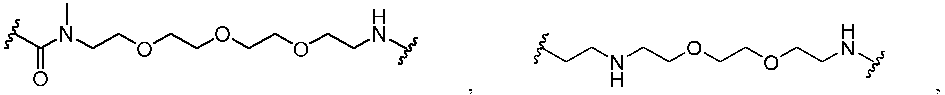 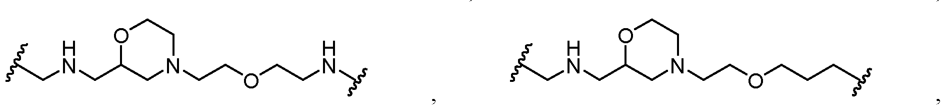 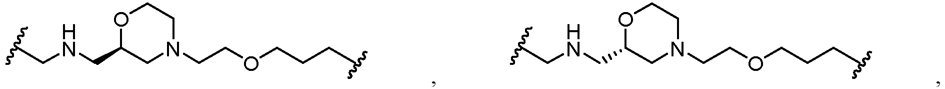 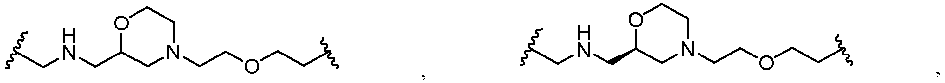 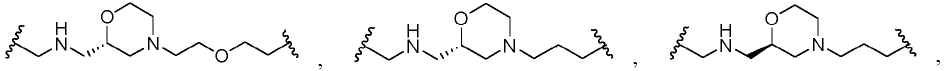 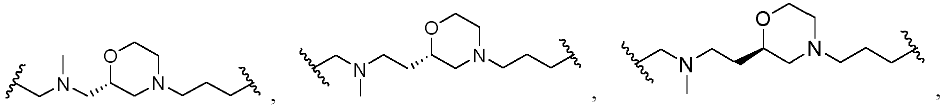 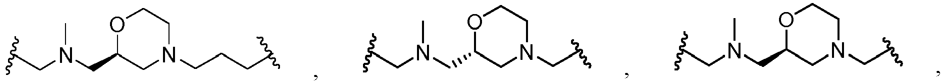 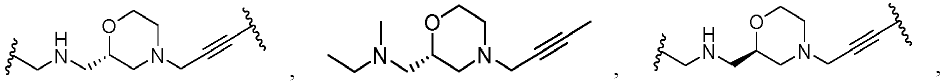 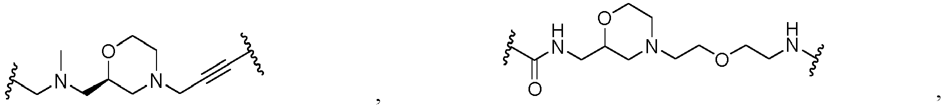 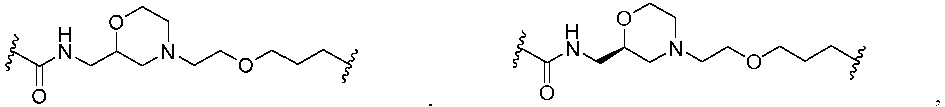 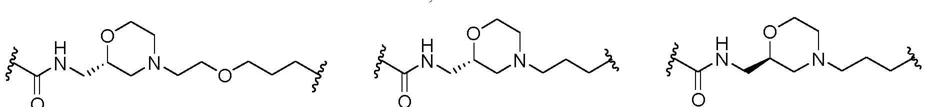 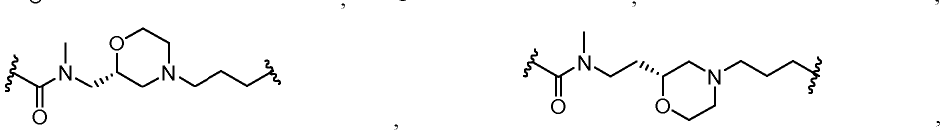 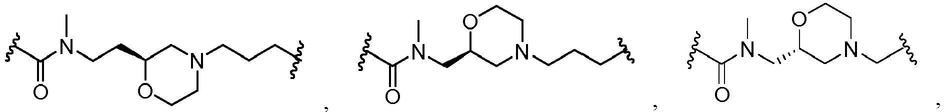 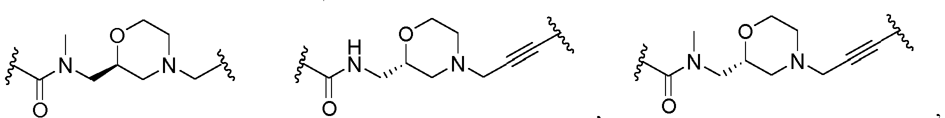 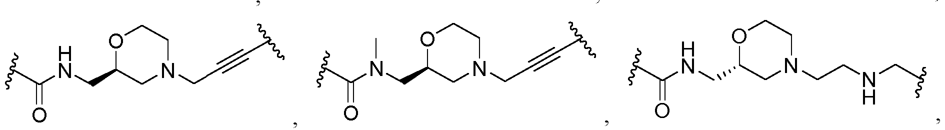 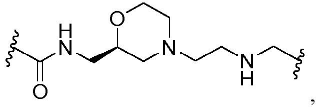 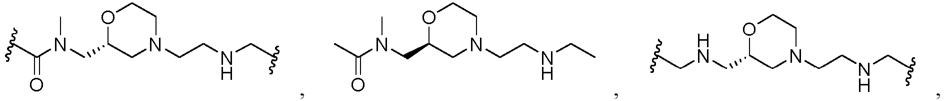 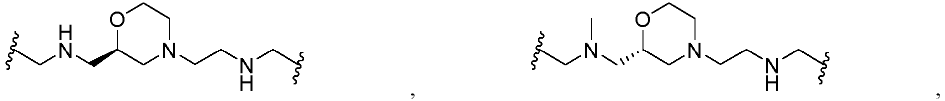 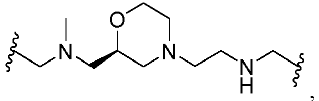 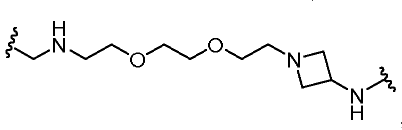 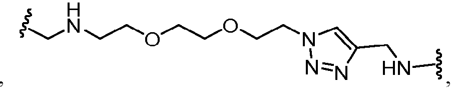 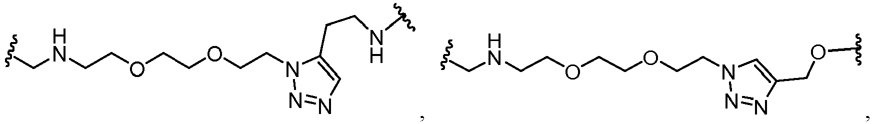 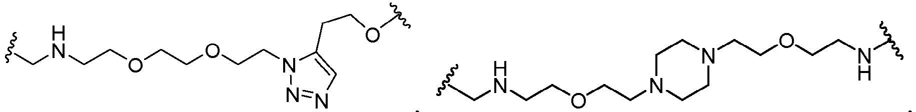 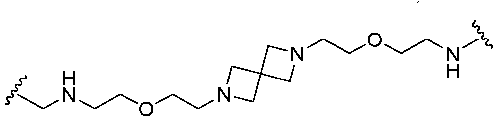 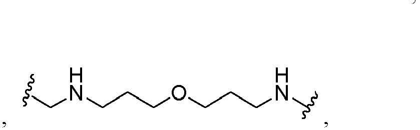 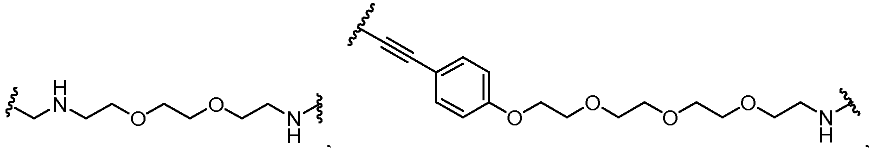 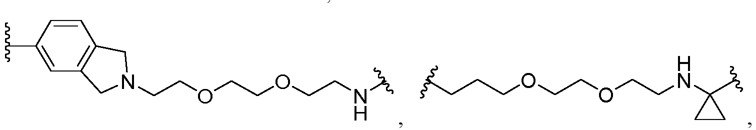 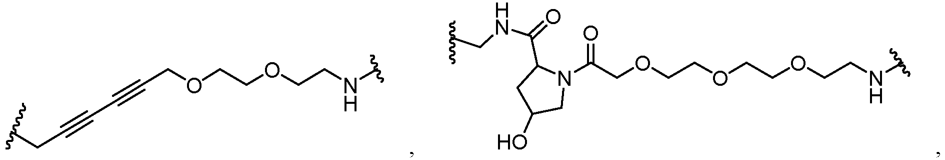 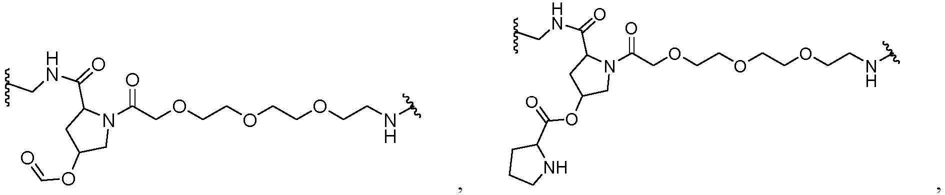 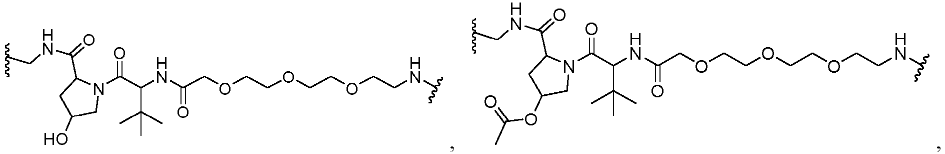 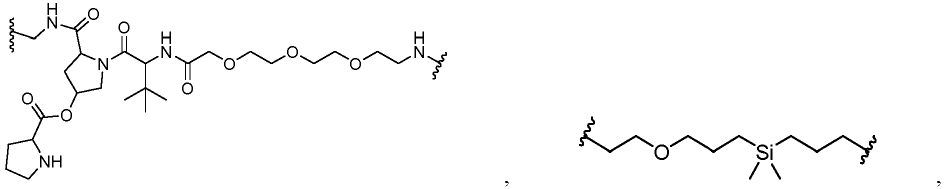 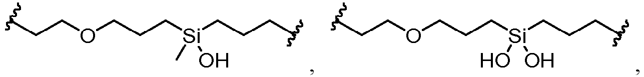 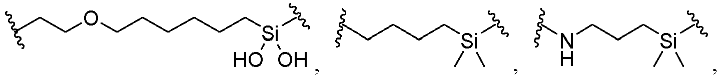 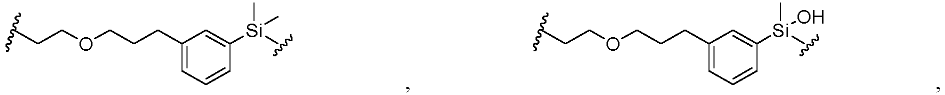 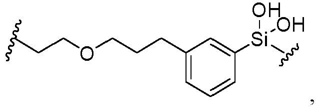 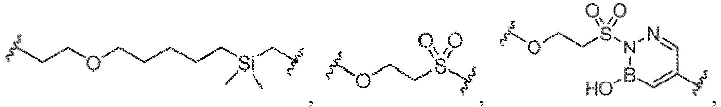 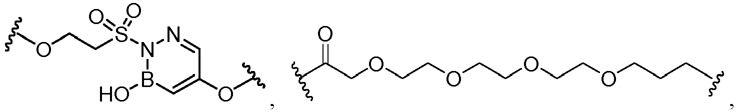 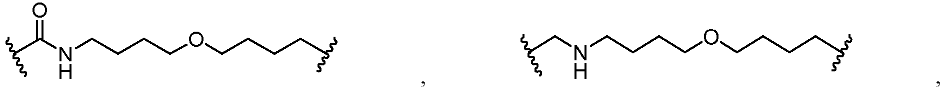 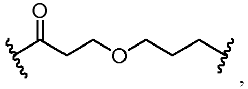 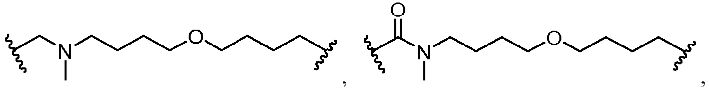 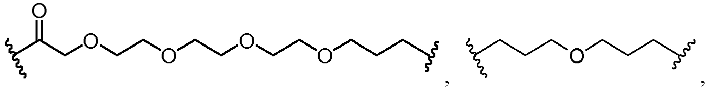 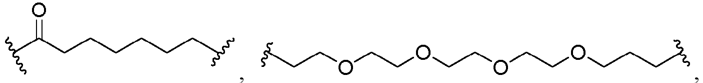 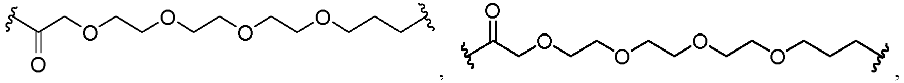 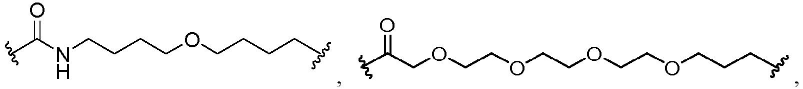 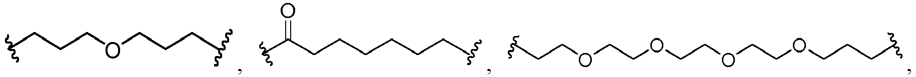 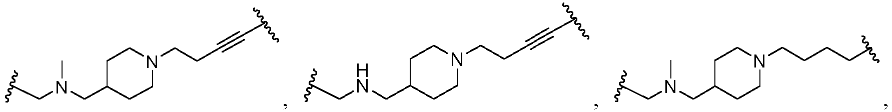 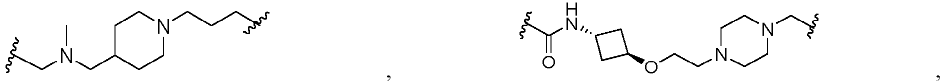 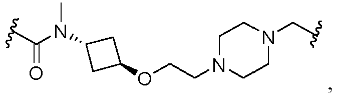 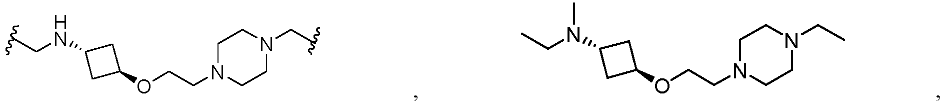 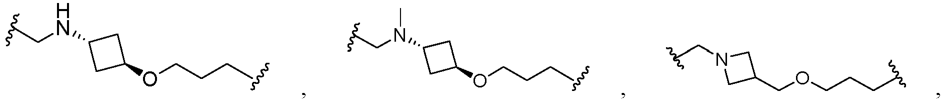 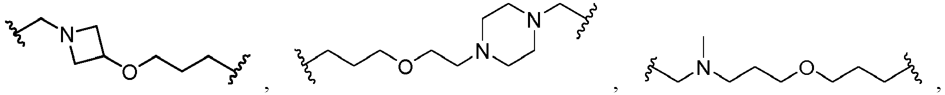 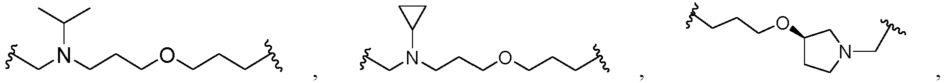 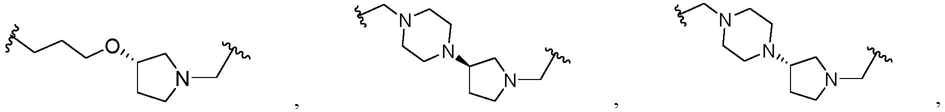 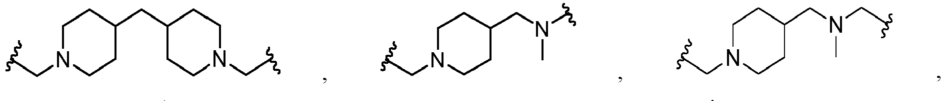 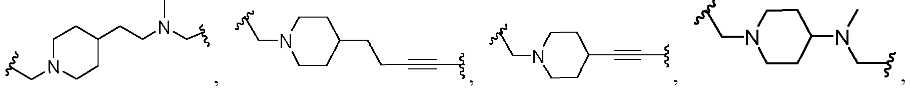 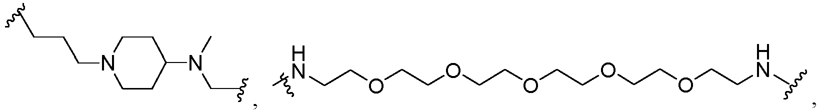 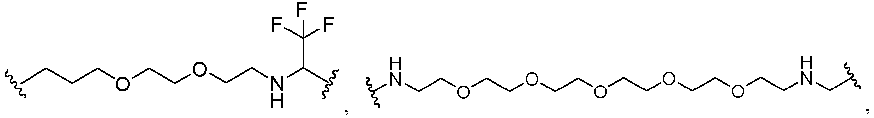 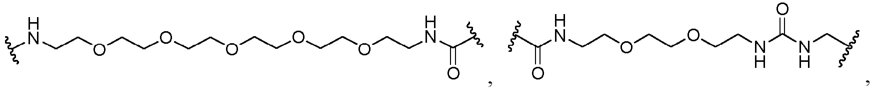 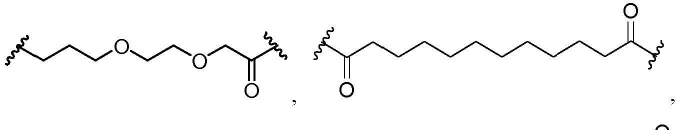 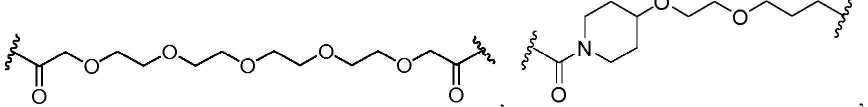 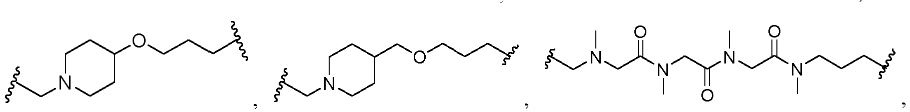 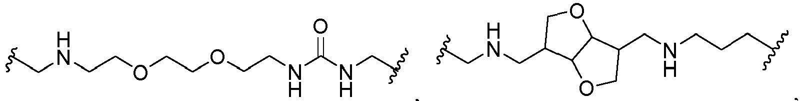 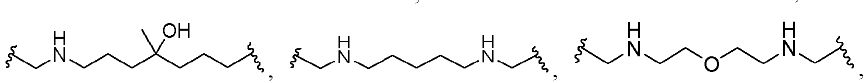 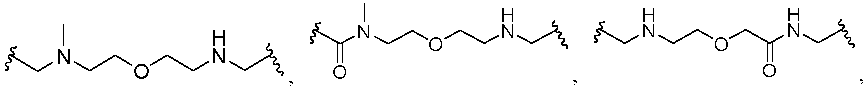 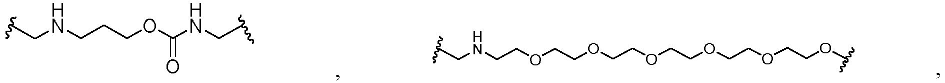 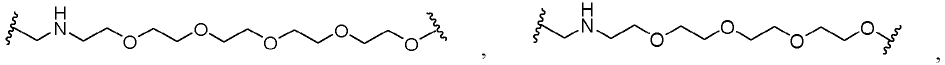 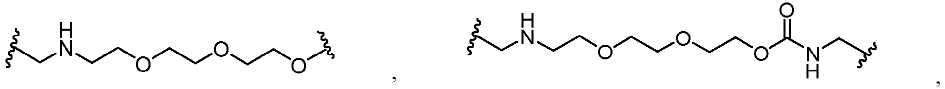 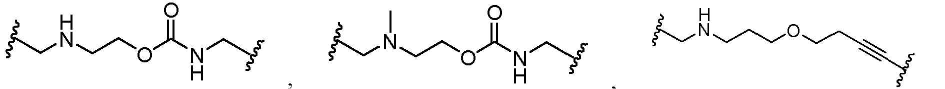 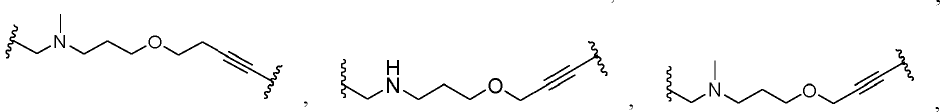 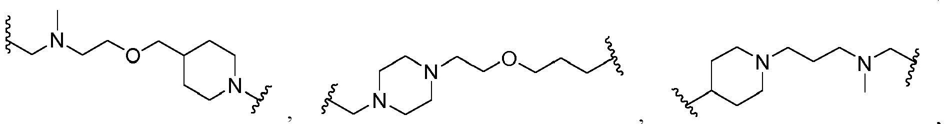 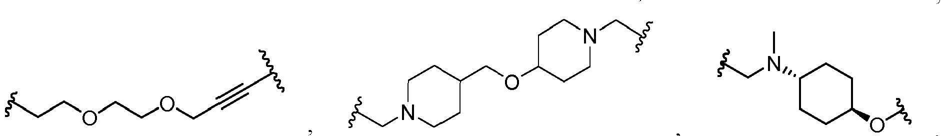 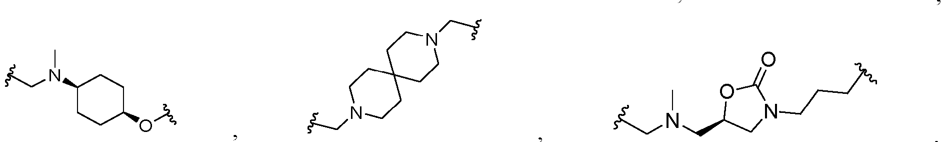 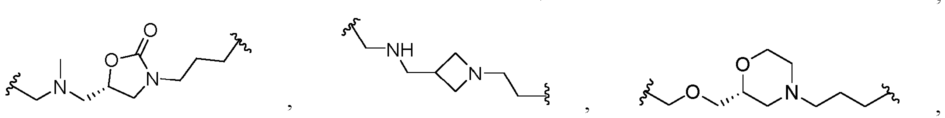 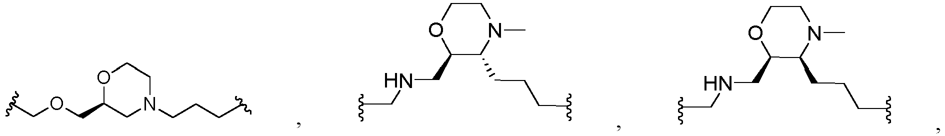 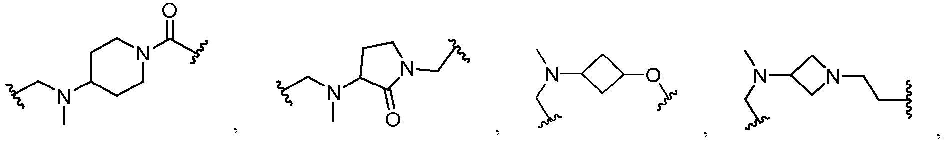 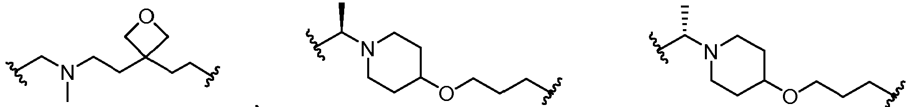 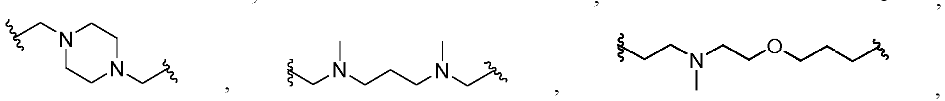 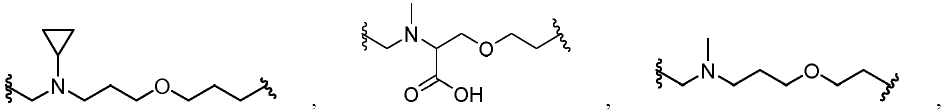 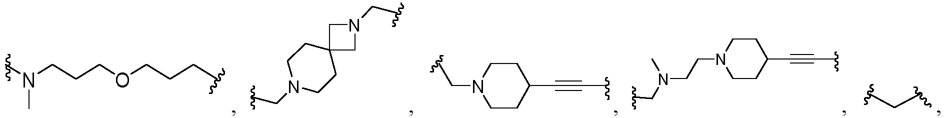 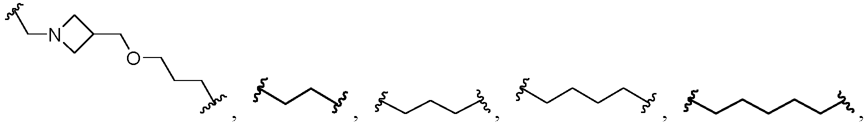 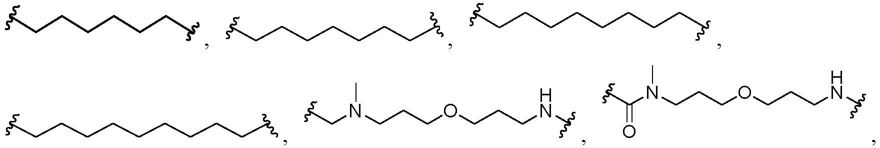 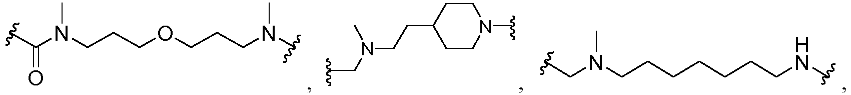 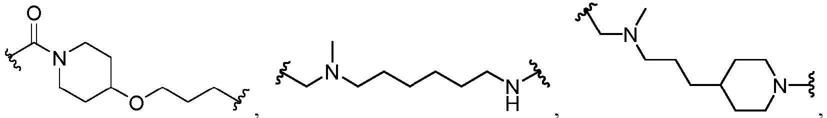 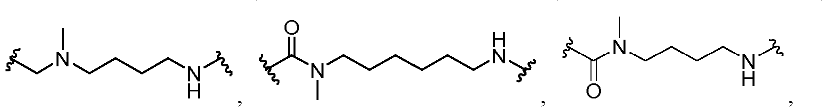 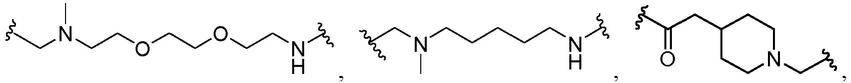 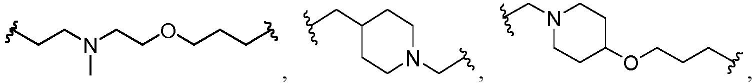 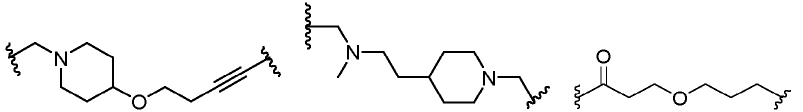 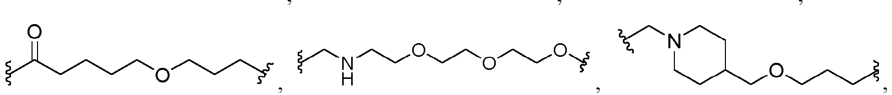 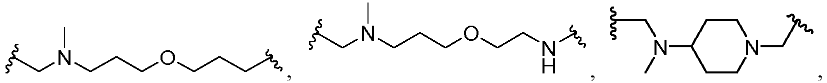 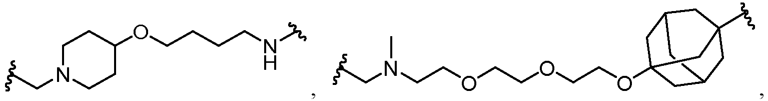 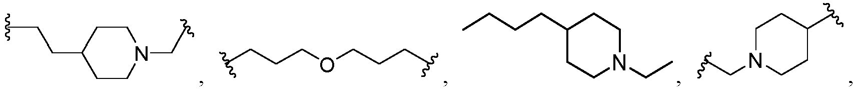 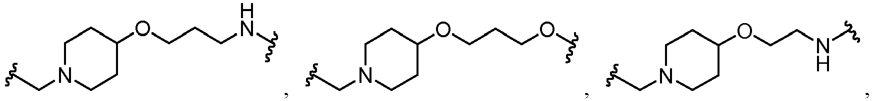 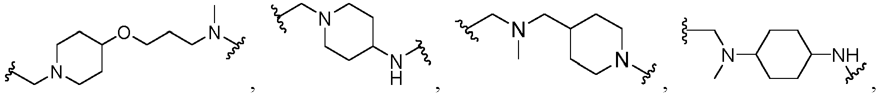 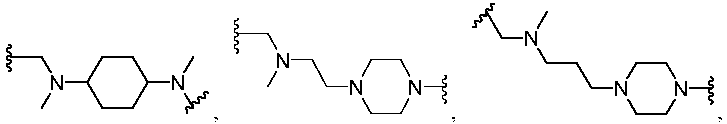 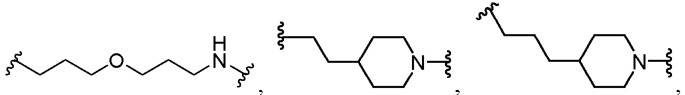 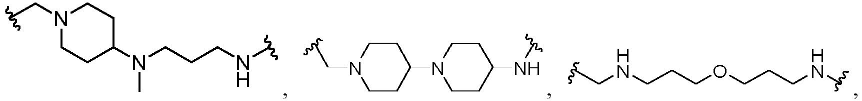 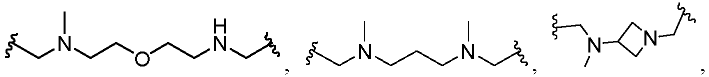 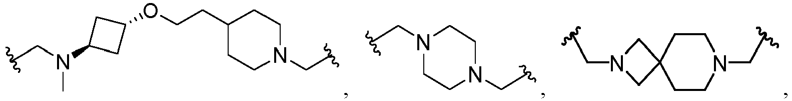 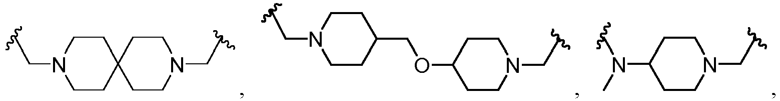 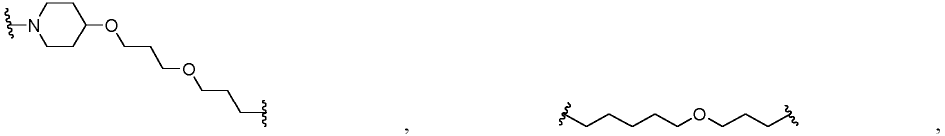 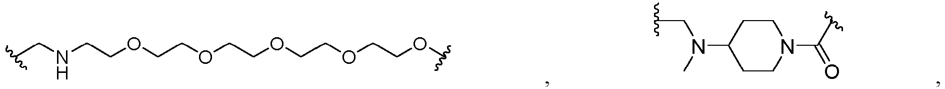 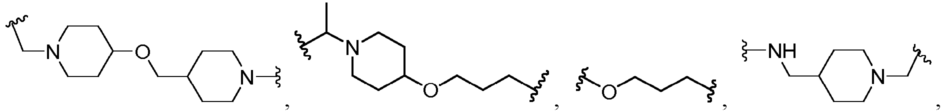 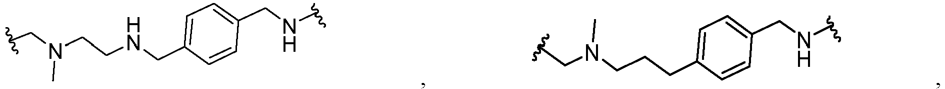 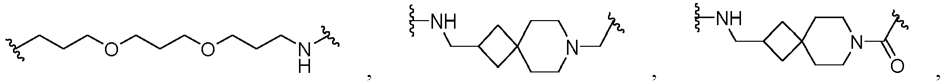 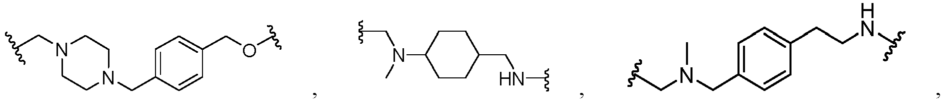 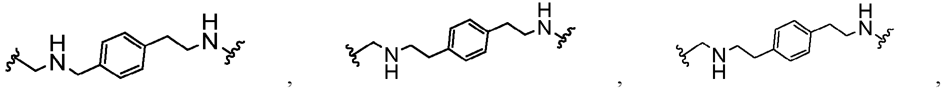 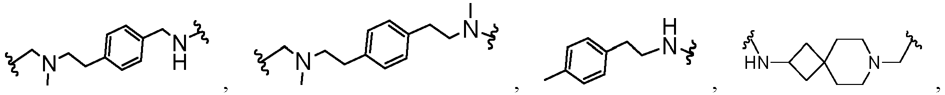 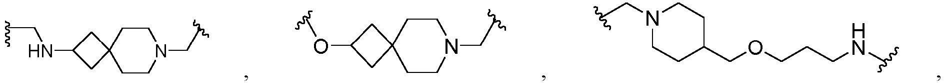 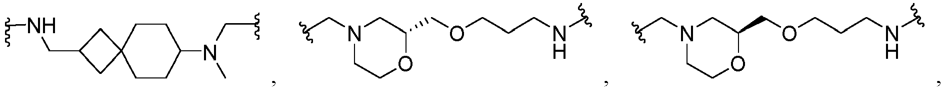 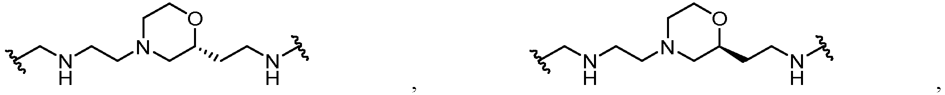 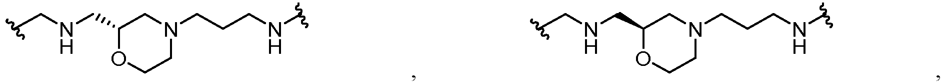 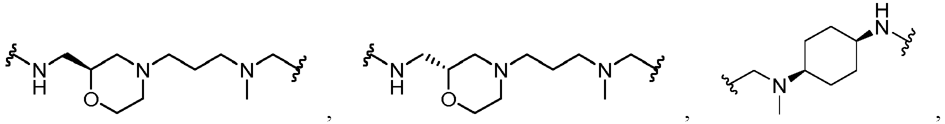 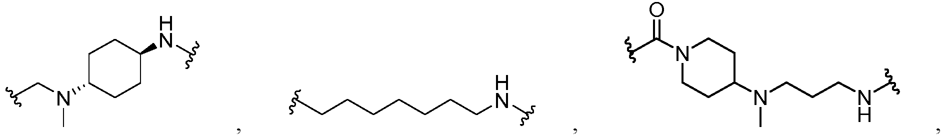 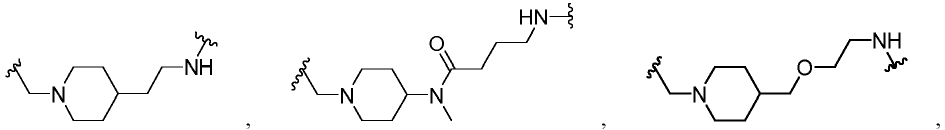 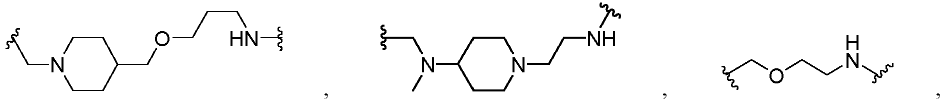 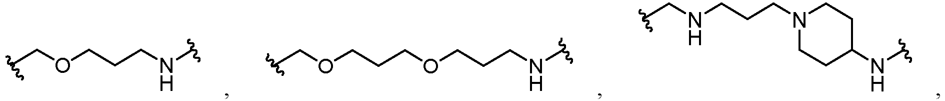 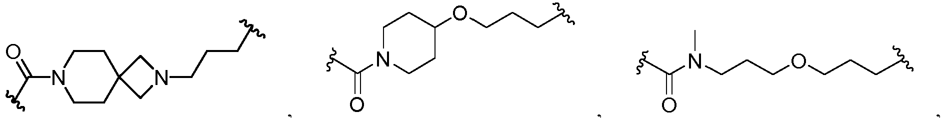 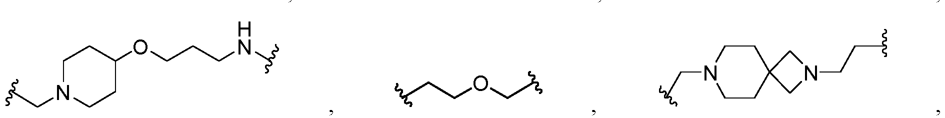 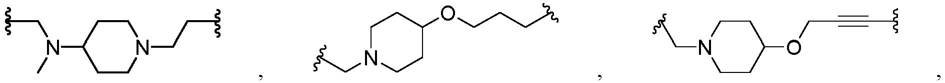 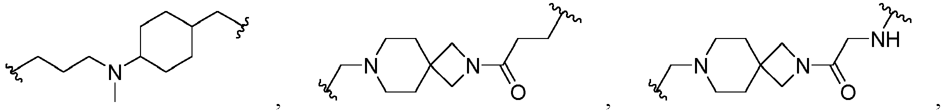 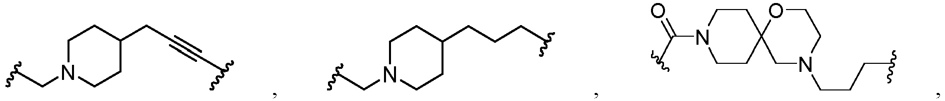 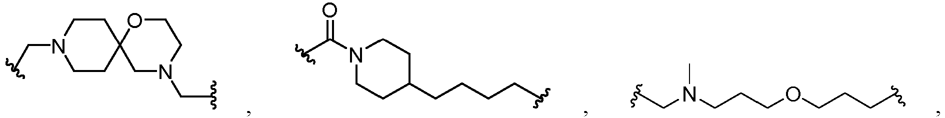 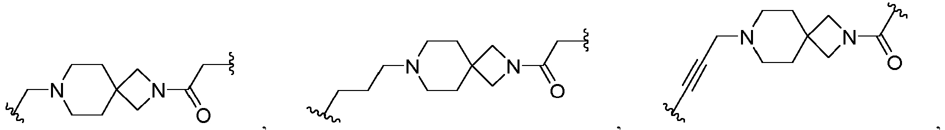 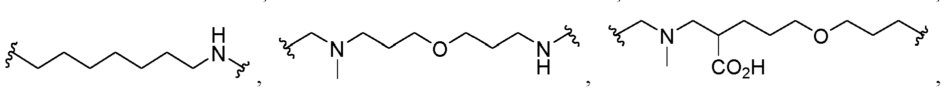 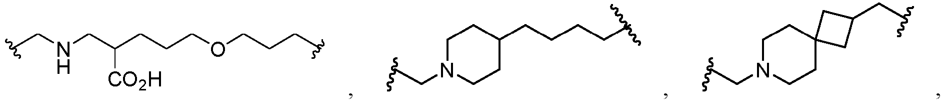 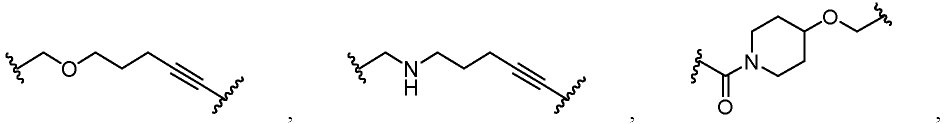 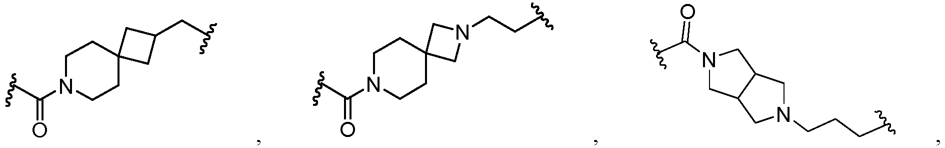 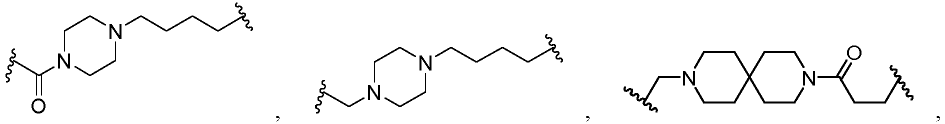 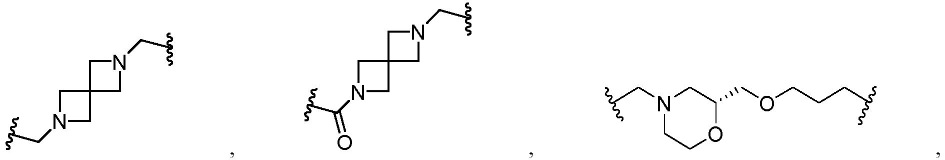 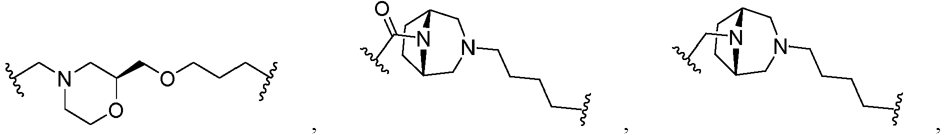 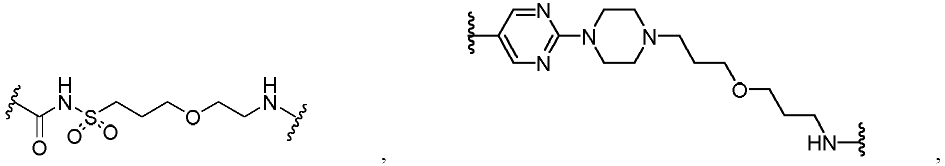 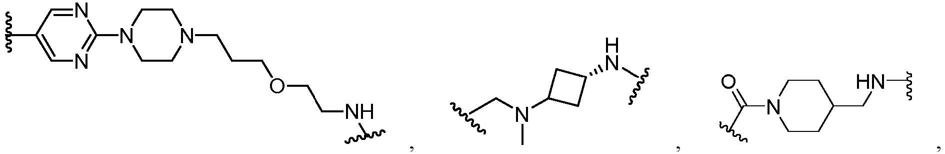 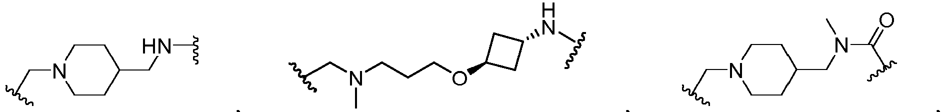 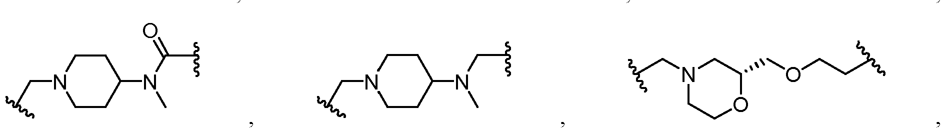 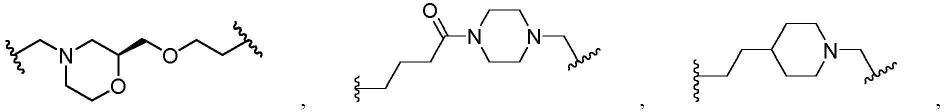 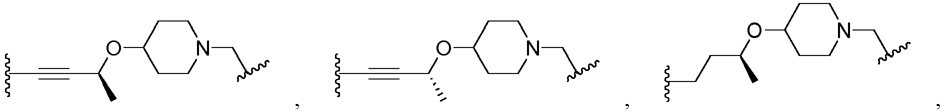 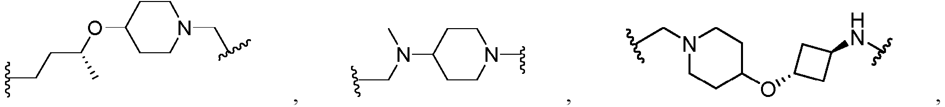 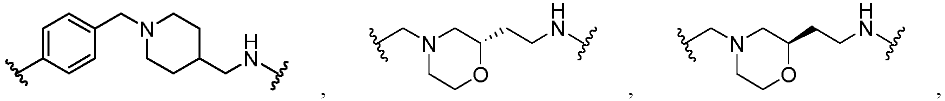 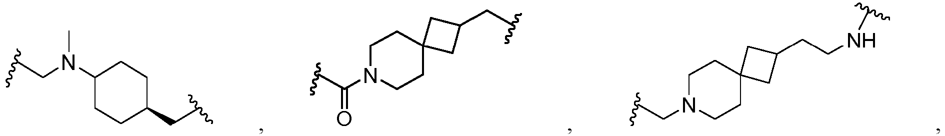 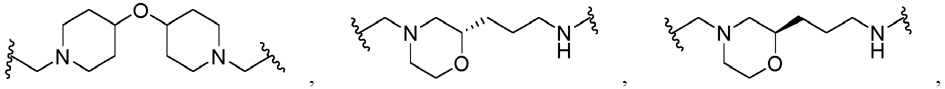 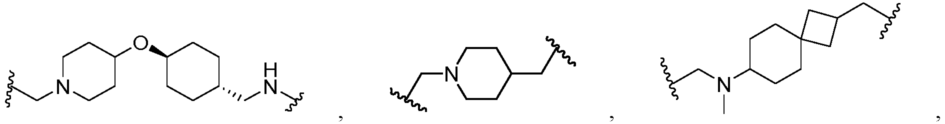 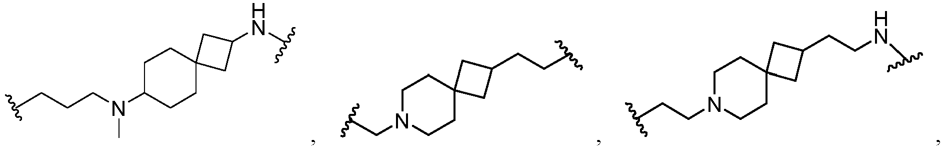 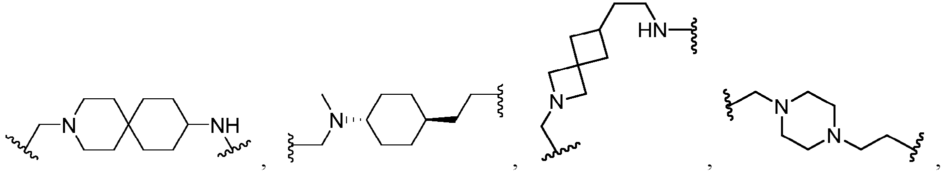 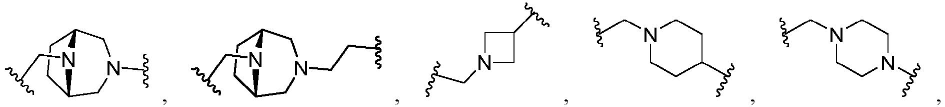 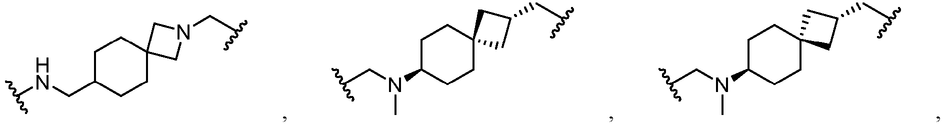 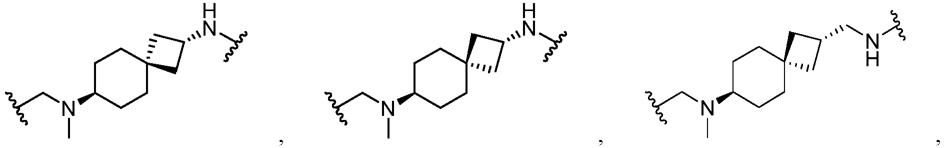 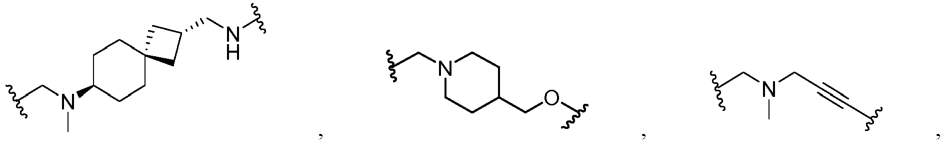 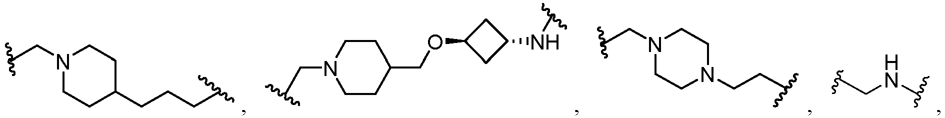 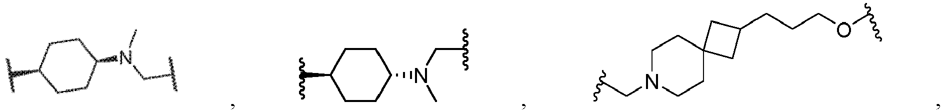 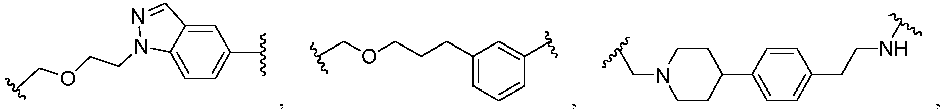 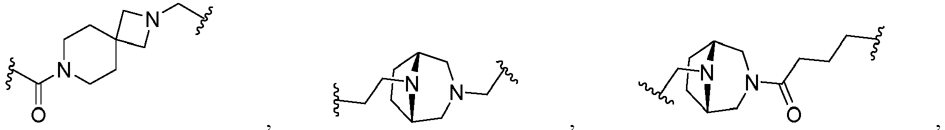 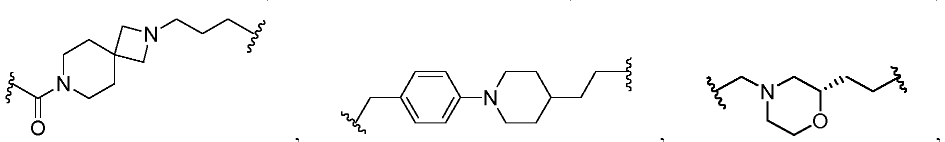 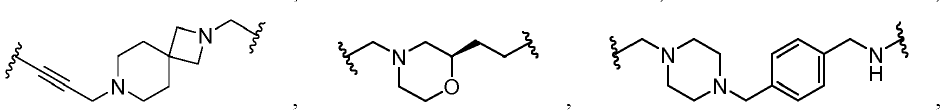 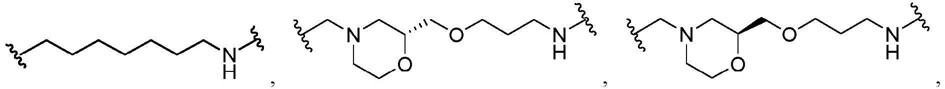 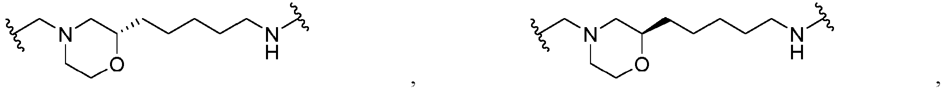 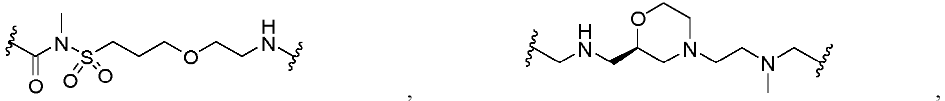 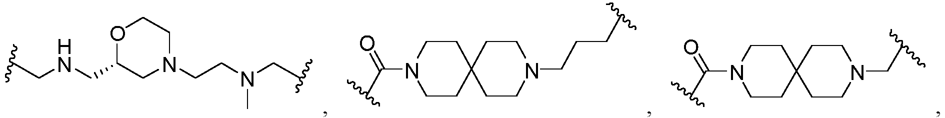 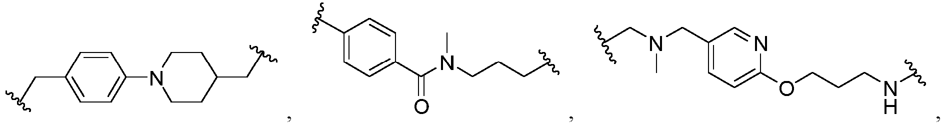 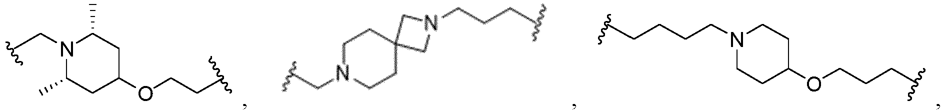 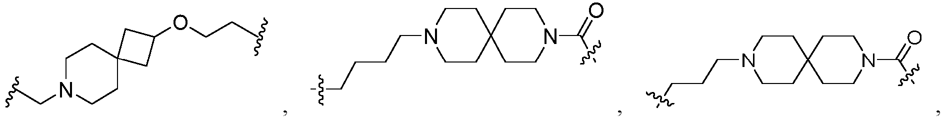 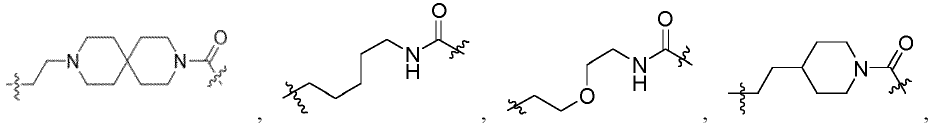 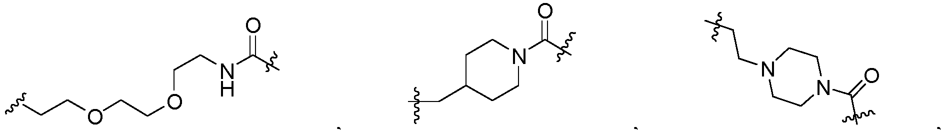 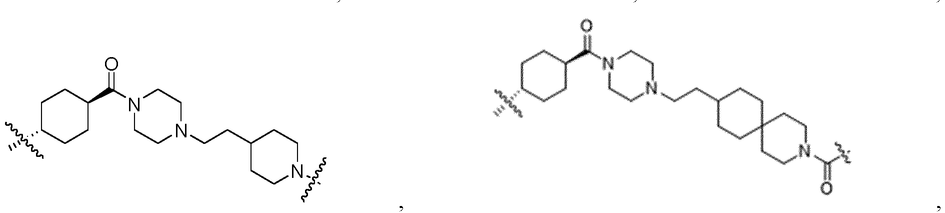 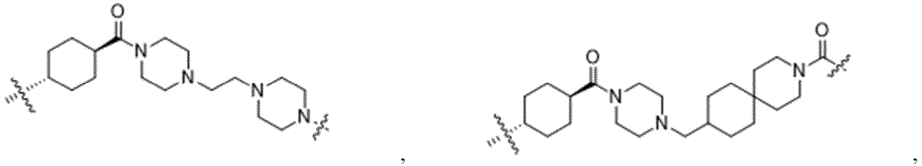 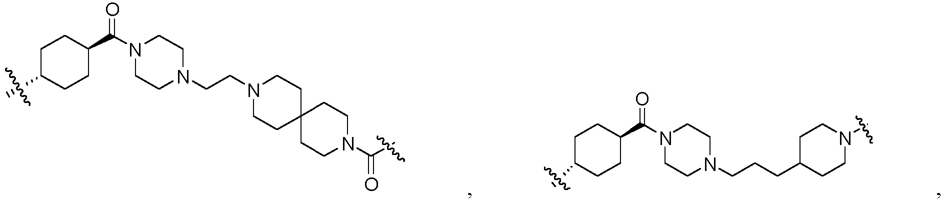 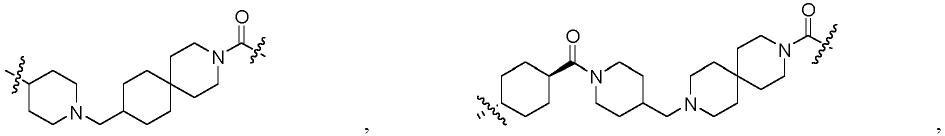 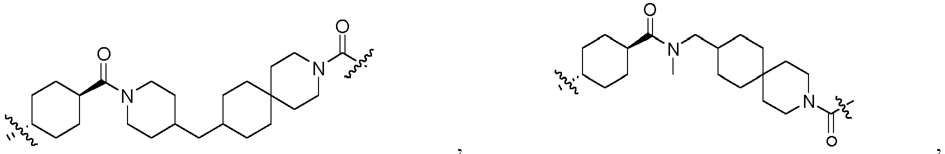 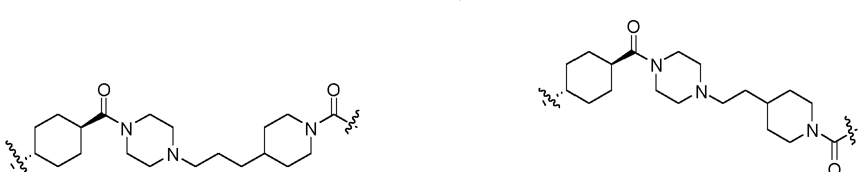 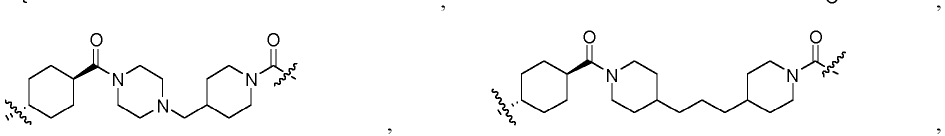 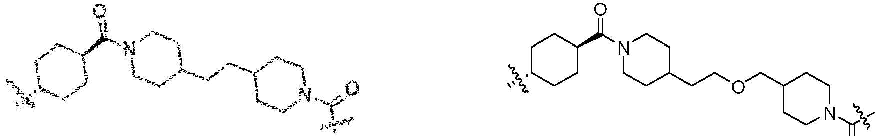 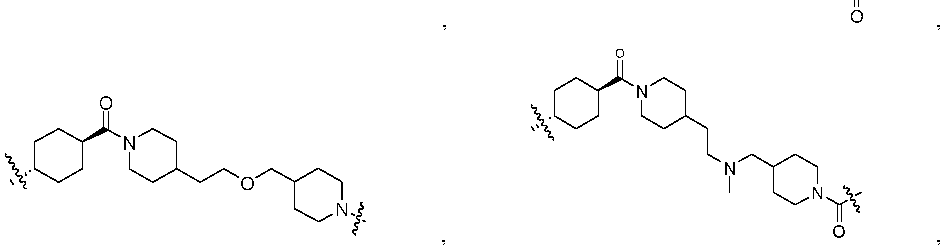 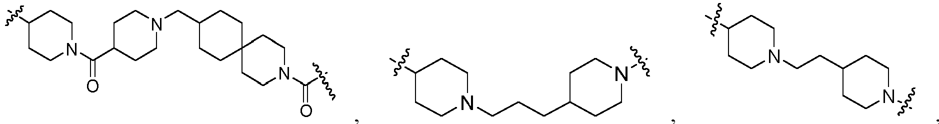 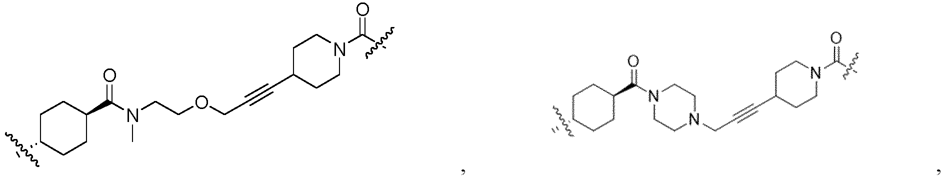 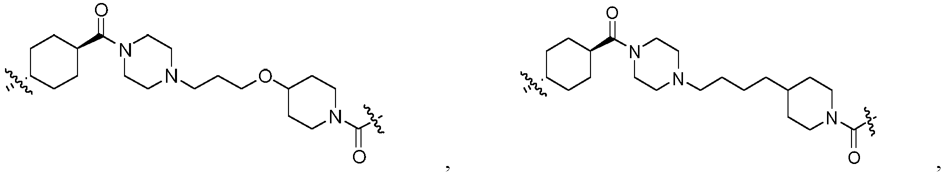 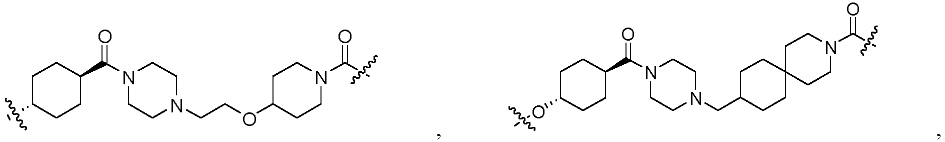 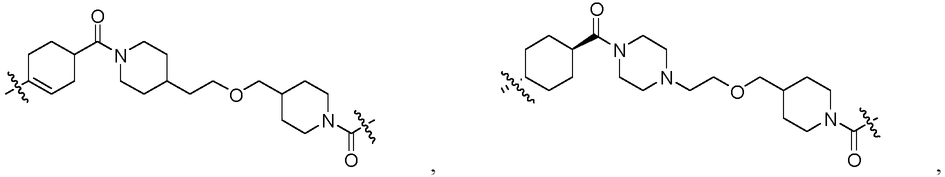 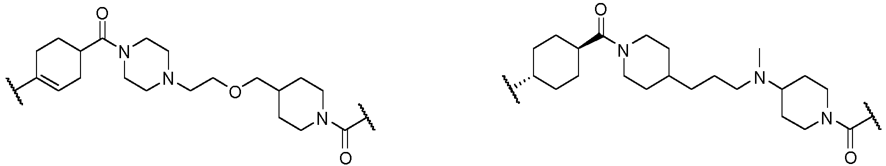 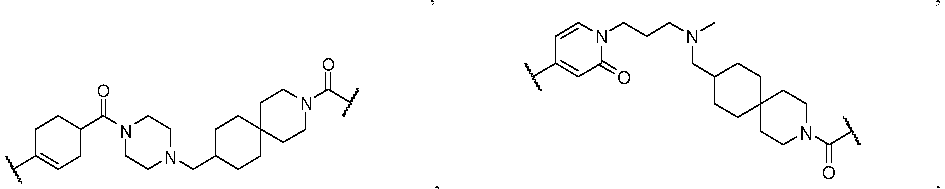 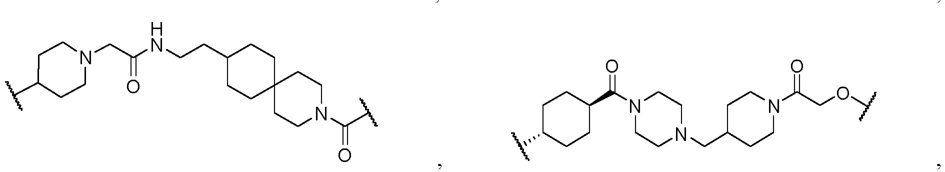 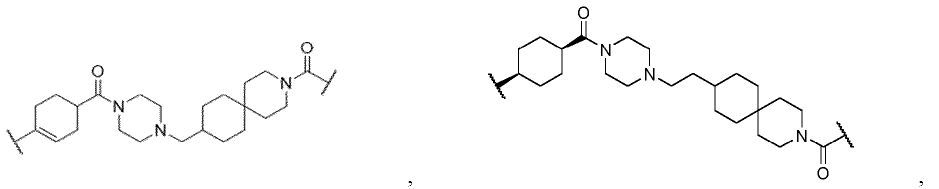 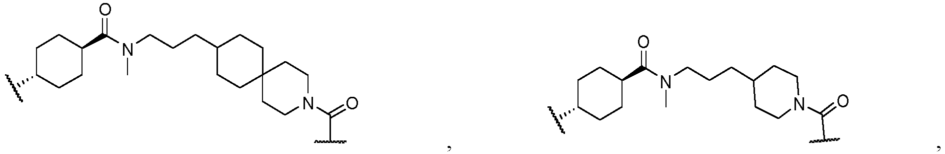 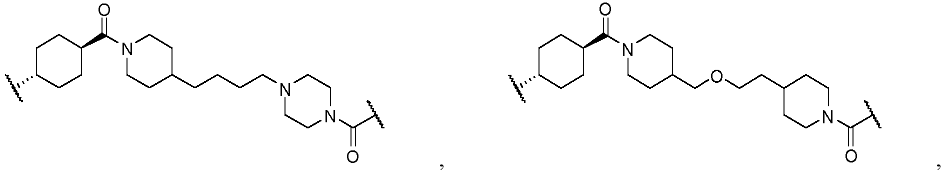 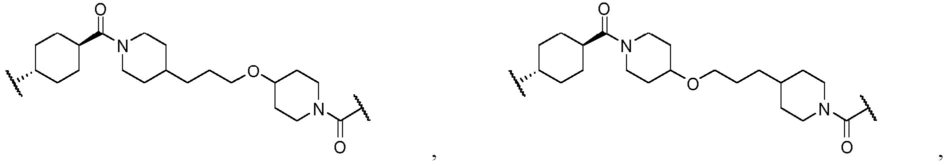 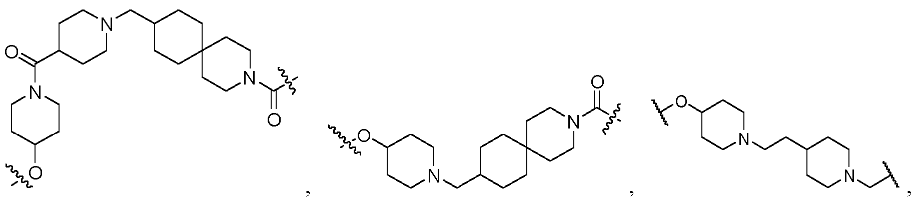 or

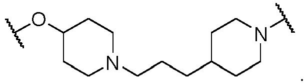

Preferably, in certain embodiments of the present disclosure, L is LA: 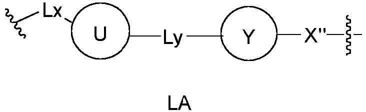 wherein in LA:
ring U is C₃-C₁₂ cycloalkylene or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
ring Y is a bond, C₃-C₁₂ cycloalkylene, or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
X" is a bond, -C(O)-, -NH-, -NCH₃-, -O-, -C(CH₃)₂-, -S-, -C=C-, -C≡C-, -CHF-, -CHCF₃-,-(CH₂)_{q}C(O)-, -S(O)-, -S(O)₂-, -C(O)O-, -OC(O)-, -(CH₂)_{q}C(O)NH-, -C(O)NCH₃-, -NHC(O)-, -NCH₃C(O)-, or -C(O)CH₂O-;
q is 1 or 2;
Lx is -(CH₂)ᵥ-, wherein one or two methylene groups in Lx are optionally replaced by a group selected from -O-, -S-, -NH-, -C≡C-, -N(C₁-C₆ alkyl)-, -N(C₁-C₆ haloalkyl)-, -C(O)-, -N(C₁-C₆ hydroxyalkyl)-, -N(C₃-C₈ cycloalkyl)-, and -CRaRₑ-; v is 1, 2, 3, 4, 5, 6, or 7;
R_{d} and Rₑ are each independently H, -OH, C₁-C₆ alkyl, or C₁-C₆ alkoxy;
or R_{d} and Rₑ, together with the C atom linked thereto, form C₃-C₈ cycloalkyl or 3- to 8-membered heterocycloalkyl;
Ly is -(CH₂)ₖ-, wherein one or two methylene groups in Ly are optionally replaced by a group selected from -O-, -NH-, -C≡C-, -N(C₁-C₆ alkyl)-, -N(C₁-C₆ haloalkyl)-, -C(O)-, -N(C₁-C₆ hydroxyalkyl)-, and -N(C₃-C₈ cycloalkyl)-; k is 1, 2, 3, 4, 5, 6, 7, or 8.

Preferably, in certain embodiments of the present disclosure, L is LA: 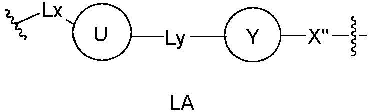 wherein in LA:
ring U is C₃-C₁₂ cycloalkylene or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
ring Y is a bond, C₃-C₁₂ cycloalkylene, or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
X" is a bond, -C(O)-, -NH-, -NCH₃-, -O-, -C(CH₃)₂-, -S-, -C=C-, -CHF-, -CHCF₃-, -(CH₂)_{q}C(O)-, -S(O)-, -S(O)₂-, -C(O)O-, -OC(O)-, -(CH₂)_{q}C(O)NH-, -C(O)NCH₃-, -NHC(O)-, -NCH₃C(O)-, or-C(O)CH₂O-;
q is 1 or 2;
Lx is -(CH₂)ᵥ-, wherein one or two methylene groups in Lx are optionally replaced by a group selected from -O-, -S-, -NH-, -C≡C-, -N(C₁-C₆ alkyl)-, -N(C₁-C₆ haloalkyl)-, -C(O)-, -N(C₁-C₆ hydroxyalkyl)-, -N(C₃-C₈ cycloalkyl)-, and -CRaRₑ-; v is 1, 2, 3, 4, 5, 6, or 7;
R_{d} and Rₑ are each independently H, -OH, C₁-C₆ alkyl, or C₁-C₆ alkoxy;
or R_{d} and Rₑ, together with the C atom linked thereto, form C₃-C₈ cycloalkyl or 3- to 8-membered heterocycloalkyl;
Ly is -(CH₂)ₖ-, wherein one or two methylene groups in Ly are optionally replaced by a group selected from -O-, -NH-, -C≡C-, -N(C₁-C₆ alkyl)-, -N(C₁-C₆ haloalkyl)-, -C(O)-, -N(C₁-C₆ hydroxyalkyl)-, and -N(C₃-C₈ cycloalkyl)-; k is 1, 2, 3, 4, 5, 6, 7, or 8.

Preferably, in certain embodiments of the present disclosure, L is LA: 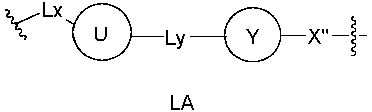 wherein in LA:
ring U is C₃-C₁₂ cycloalkylene or 3- to 12-membered heterocycloalkylene containing 1-3 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
ring Y is a bond, C₃-C₁₂ cycloalkylene, or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
X" is a bond, -C(O)-, -NH-, -NCH₃-, -O-, -C(CH₃)₂-, -S-, -C=C-, -CHF-, -CHCF₃-, -(CH₂)_{q}C(O)-, -S(O)-, -S(O)₂-, -C(O)O-, -OC(O)-, -(CH₂)_{q}C(O)NH-, -C(O)NCH₃-, -NHC(O)-, -NCH₃C(O)-, or-C(O)CH₂O-;
q is 1 or 2;
Lx is -(CH₂)ᵥ-, wherein one or two methylene groups in Lx are optionally replaced by a group selected from -O-, -S-, -NH-, -C≡C-, -N(C₁-C₆ alkyl)-, -N(C₁-C₆ haloalkyl)-, -C(O)-, -N(C₁-C₆ hydroxyalkyl)-, -N(C₃-C₈ cycloalkyl)-, and -CRaRₑ-; v is 1, 2, 3, 4, 5, 6, or 7;
R_{d} and Rₑ are each independently H, -OH, -NH₂, C₁-C₆ haloalkyl, C₁-C₆ alkyl, or C₁-C₆ alkoxy;
or R_{d} and Rₑ, together with the C atom linked thereto, form C₃-C₈ cycloalkyl or 3- to 8-membered heterocycloalkyl;
Ly is -(CH₂)ₖ-, wherein one or two methylene groups in Ly are optionally replaced by a group selected from -O-, -NH-, -C≡C-, -N(C₁-C₆ alkyl)-, -N(C₁-C₆ haloalkyl)-, -C(O)-, -N(C₁-C₆ hydroxyalkyl)-, and -N(C₃-C₈ cycloalkyl)-; k is 1, 2, 3, 4, 5, 6, 7, or 8.

Preferably, in certain embodiments of the present disclosure, ring U in LA is 4- to 8-membered saturated monocyclic heterocycloalkylene or 6- to 10-membered fused heterocycloalkylene, containing 1 or 2 nitrogen heteroatoms; ring Y is 6- to 8-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond or -C(O)-; Lx is -(CH₂)ᵥ-, and v is 1, 2, 3, 4, or 5; Ly is -(CH₂)ₖ-, and k is 1, 2, 3, 4, or 5.

Preferably, in certain embodiments of the present disclosure, ring U in LA is 4- to 8-membered saturated monocyclic heterocycloalkylene, 6- to 10-membered fused heterocycloalkylene, or 7- to 11-membered spiroheterocycloalkylene containing 1 or 2 nitrogen heteroatoms; ring Y is 6- to 8-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond, -C(O)CH₂O-, or -C(O)-; Lx is -(CH₂)ᵥ-, and v is 1, 2, 3, 4, or 5; Ly is -(CH₂)ₖ-, and k is 1, 2, 3, 4, or 5.

Preferably, in certain embodiments of the present disclosure, LA is LA-1 or LA-2: 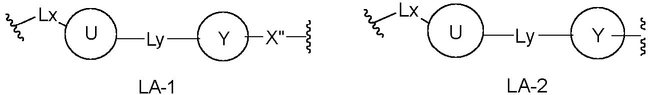
X" is -C(O)- or -C(O)NH-;
in LA-1 and LA-2, ring U, ring Y, Lx, Ly, and q are as defined and described in the present disclosure.

Preferably, in certain embodiments of the present disclosure, LA is LA-1 or LA-2: 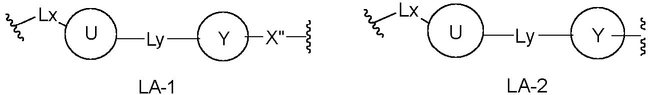
X" is -C(O)-, -C(O)NH-, or -C(O)CH₂O-;
in LA-1 and LA-2, ring U, ring Y, Lx, Ly, and q are as defined and described in the present disclosure.

Preferably, in certain embodiments of the present disclosure, LA is LA-3, LA-4, or LA-5: 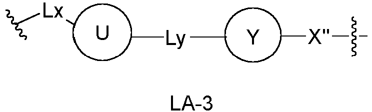 wherein in LA-3:
Lx is -(CH₂)ᵥ-, wherein one or two CH₂ contained in Lx are each independently optionally replaced by -O-, -S-, -NH-, -NMe-, or -CR_{d}Rₑ-; -CR_{d}Rₑ- is 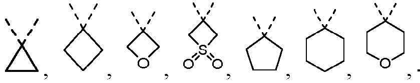 -C(CH₃)₂, -CH(CH₃)-, -CH(OH)-, -CH(OCH₃)-, or C(CH₃)(OH)-; v is 1, 2, 3, 4, 5, or 6;
ring U is 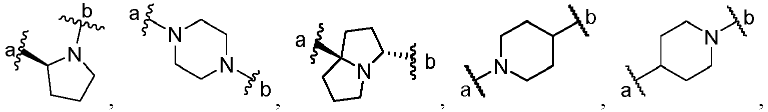 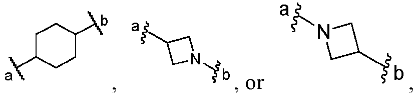 wherein end a is linked to Lx, and end b is linked to Ly; 1, 2, 3, or 4 hydrogen atoms in ring U are optionally substituted with F;
ring Y is a bond, 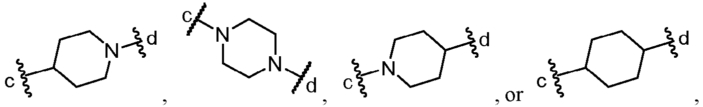 wherein end c is linked to Ly, and end d is linked to X"; 1, 2, 3, or 4 hydrogen atoms in ring Y are optionally substituted with F;
Ly is -(CH₂)ₖ-, wherein one or two CH₂ contained in Ly are each independently optionally replaced by -O-, -C≡C-, or -N(C₁-C₆ alkyl)-; k is 1, 2, 3, 4, 5, or 6;
X" is a bond, -C(O)-, -(CH₂)₂C(O)-, or -(CH₂)₂C(O)NH-; 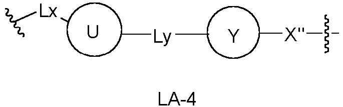
wherein in LA-4:
   Lx is -(CH₂)ᵥ-, wherein one or two CH₂ contained in Lx are each independently optionally replaced by -O-, -S-, -NH-, or -CRaRₑ-; -CR_{d}Rₑ- is 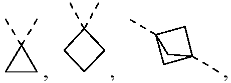 -C(CH₃)₂-, -CH(CH₃)-, -CH(OCH₃)-, -CH(OH)-, or -C(CH₃)(OH)-; v is 1, 2, 3, 4, 5, or 6;
   ring U is 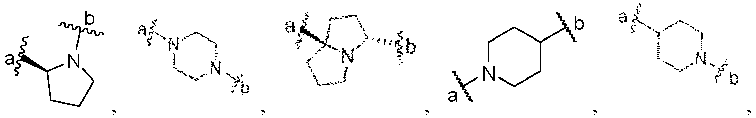 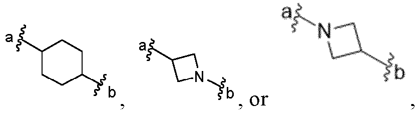 wherein end a is linked to Lx, and end b is linked to Ly; 1, 2, 3, or 4 hydrogen atoms in ring U are optionally substituted with F;
   ring Y is 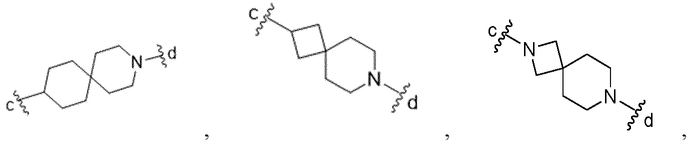 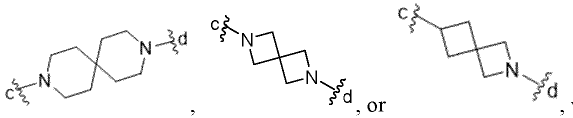 wherein end c is linked to Ly, and end d is linked to X"; 1, 2, 3, or 4 hydrogen atoms in ring Y are optionally substituted with F;
   Ly is -(CH₂)ₖ-, wherein one or two CH₂ contained in Ly are each independently optionally replaced by -O- or -N(C₁-C₆ alkyl)-; k is 1, 2, 3, 4, 5, or 6;
   X" is -C(O)-; 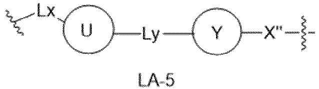
   wherein in LA-5:
      Lx is -(CH₂)ᵥ-, wherein one or two CH₂ contained in Lx are each independently optionally replaced by -O-; v is 1, 2, 3, or 4;
      ring U is 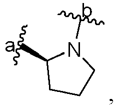 wherein end a is linked to Lx, and end b is linked to Ly; 1, 2, 3, or 4 hydrogen atoms in ring U are optionally substituted with F;
      ring Y is a bond;
      Ly is -(CH₂)ₖ-, wherein one or two CH₂ contained in Ly are each independently optionally replaced by -O-, -C(O)-, or -NH-; k is 1, 2, 3, 4, 5, 6, 7, or 8;
      X" is -C(O)NH-; 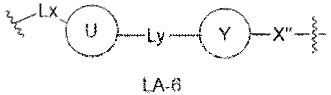
      wherein in LA-6:
         Lx is -(CH₂)ᵥ-, wherein one or two CH₂ contained in Lx are each independently optionally replaced by -O- or -CRaRₑ-; -CR_{d}Rₑ- is 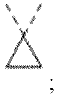 v is 1, 2, 3, or 4;
         ring U is 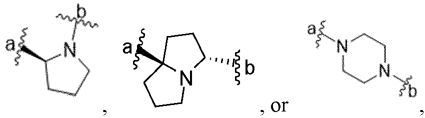 wherein end a is linked to Lx, and end b is linked to Ly;
         ring Y is 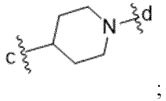
         Ly is -(CH₂)ₖ-, wherein one or two CH₂ contained in Ly are each independently optionally replaced by -O-, -C(O)-, or -NH-; k is 1, 2, 3, 4, 5, or 6;
         X" is a bond.

Preferably, in certain embodiments of the present disclosure, LA is LA-3, LA-4, LA-5, or LA-6: 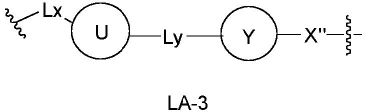 wherein in LA-3:
Lx is -(CH₂)ᵥ-, wherein one or two CH₂ contained in Lx are each independently optionally replaced by -O-, -S-, -NH-, -NMe-, or -CR_{d}Rₑ-; -CR_{d}Rₑ- is 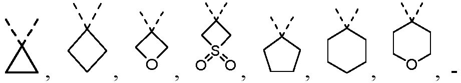 C(CH₃)₂-, -CH(CH₃)-, -CH(OH)-, -CH(OCH₃)-, or C(CH₃)(OH)-; v is 1, 2, 3, 4, 5, or 6;
ring U is 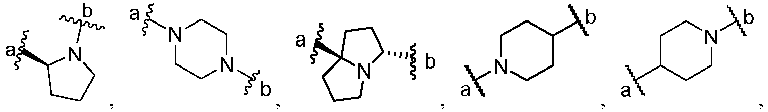 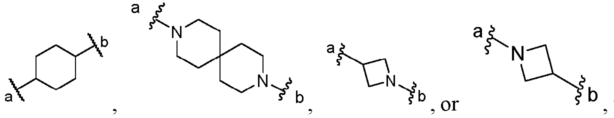 wherein end a is linked to Lx and end b is linked to Ly; 1, 2, 3, or 4 hydrogen atoms in ring U are optionally substituted with F;
ring Y is a bond, 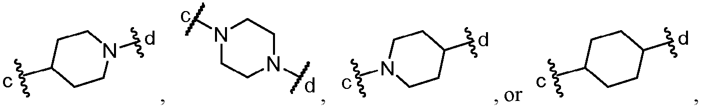 wherein end c is linked to Ly, and end d is linked to X"; 1, 2, 3, or 4 hydrogen atoms in ring Y are optionally substituted with F;
Ly is -(CH₂)ₖ-, wherein one or two CH₂ contained in Ly are each independently optionally replaced by -O-, -C≡C-, -C(O)-, or -N(C₁-C₆ alkyl)-; k is 1, 2, 3, 4, 5, or 6;
X" is a bond, -C(O)-, -(CH₂)₁₋₂C(O)-, -CH₂-C≡C-, -C(O)CH₂O-, or -(CH₂)₁₋₂C(O)NH-; 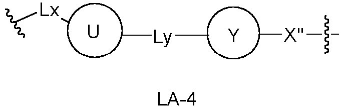
wherein in LA-4:
   Lx is -(CH₂)ᵥ-, wherein one or two CH₂ contained in Lx are each independently optionally replaced by -O-, -S-, -NH-, or -CR_{d}Rₑ-; -CR_{d}Rₑ- is 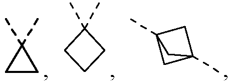 -C(CH₃)₂-, -CH(CH₃)-, -CH(OCH₃)-, -CH(OH)-, or -C(CH₃)(OH)-; v is 1, 2, 3, 4, 5, or 6;
   ring U is 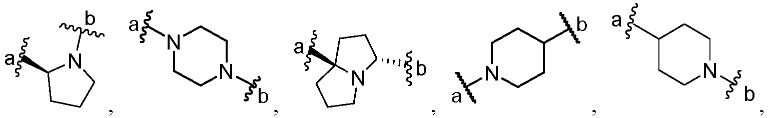 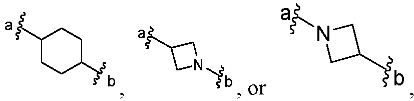 wherein end a is linked to Lx, and end b is linked to Ly; 1, 2, 3, or 4 hydrogen atoms in ring U are optionally substituted with F;
   ring Y is 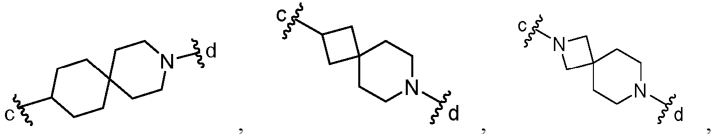 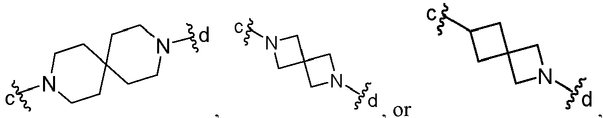 wherein end c is linked to Ly, and end d is linked to X"; 1, 2, 3, or 4 hydrogen atoms in ring Y are optionally substituted with F;
   Ly is -(CH₂)ₖ-, wherein one or two CH₂ contained in Ly are each independently optionally replaced by -O- or -N(C₁-C₆ alkyl)-; k is 1, 2, 3, 4, 5, or 6;
   X" is -C(O)-; 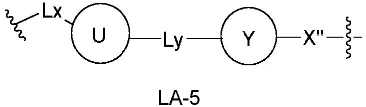
   wherein in LA-5:
      Lx is -(CH₂)ᵥ-, wherein one or two CH₂ contained in Lx are each independently optionally replaced by -O-; v is 1, 2, 3, or 4;
      ring U is 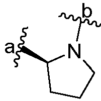 hydrogen atoms in ring U are optionally substituted with F;
      ring Y is a bond;
      Ly is -(CH₂)ₖ-, wherein one or two CH₂ contained in Ly are each independently optionally replaced by -O-, -C(O)-, or -NH-; k is 1, 2, 3, 4, 5, 6, 7, or 8;
      X" is -C(O)NH-; 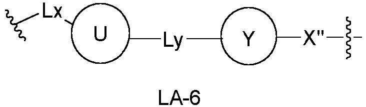
      wherein in LA-6:
      Lx is -(CH₂)ᵥ-, wherein one or two CH₂ contained in Lx are each independently optionally replaced by -O- or -CRaRₑ-; -CR_{d}Rₑ- is 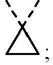 v is 1, 2, 3, or 4;
      ring U is 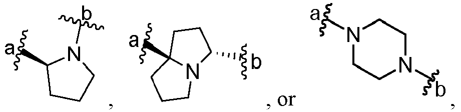 wherein end a is linked to Lx, and end b is linked to Ly;
      ring Y is 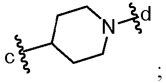
      Ly is -(CH₂)ₖ-, wherein one or two CH₂ contained in Ly are each independently optionally replaced by -O-, -C(O)-, or -NH-; k is 1, 2, 3, 4, 5, or 6;
      X" is a bond.

Preferably, in certain embodiments of the present disclosure, LA is LA-7 or LA-8: 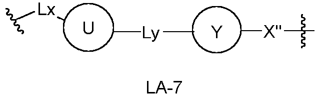 wherein in LA-7:
Lx is -(CH₂)ᵥ-, wherein one or two CH₂ contained in Lx are each independently optionally replaced by -O-, -S-, -NH-, -NMe-, or -CR_{d}Rₑ-; -CR_{d}Rₑ- is 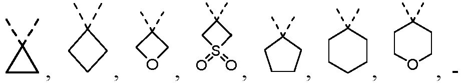 C(CH₃)₂-, -CH(CH₃)-, -CH(OH)-, -CH(OCH₃)-, or C(CH₃)(OH)-; v is 1, 2, 3, 4, 5, or 6;
ring U is 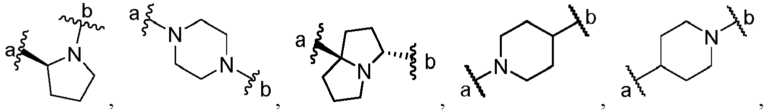 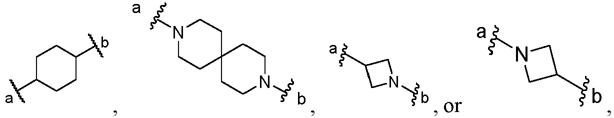 wherein end a is linked to Lx, and end b is linked to Ly; 1, 2, 3, or 4 hydrogen atoms in ring U are optionally substituted with F;
ring Y is a bond, 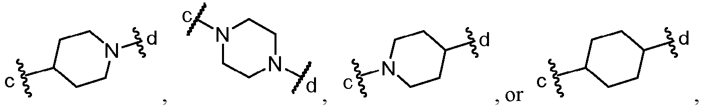 wherein end c is linked to Ly, and end d is linked to X"; 1, 2, 3, or 4 hydrogen atoms in ring Y are optionally substituted with F;
Ly is -(CH₂)ₖ-, wherein one or two CH₂ contained in Ly are each independently optionally replaced by -O-, -C≡C-, -C(O)-, or -N(C₁-C₆ alkyl)-; k is 1, 2, 3, 4, 5, or 6;
X" is a bond, -C(O)-, -(CH₂)₁₋₂C(O)-, -CH₂-C≡C-, -C(O)CH₂O-, or -(CH₂)₁₋₂C(O)NH-; 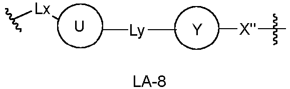
wherein in LA-8:
   Lx is -(CH₂)ᵥ-, wherein one or two CH₂ contained in Lx are each independently optionally replaced by -O-, -S-, -NH-, or -CR_{d}Rₑ-; -CR_{d}Rₑ- is 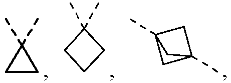 -C(CH₃)₂-, -CH(CH₃)-, -CH(OCH₃)-, -CH(OH)-, -C(CH₃)(OH)-, -CH(CHF₂)-, -CH(CH(CH₃)₂)-, -CH(CH₂CF₃)-, or -CH(NH₂)-; v is 1, 2, 3, 4, 5, or 6;
   ring U is 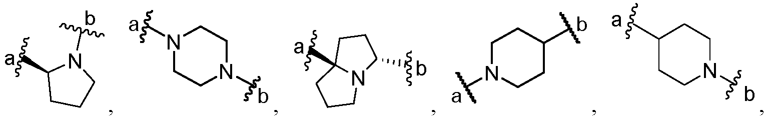 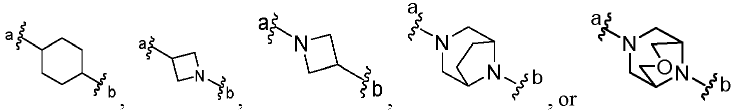 , wherein end a is linked to Lx, and end b is linked to Ly; 1, 2, 3, or 4 hydrogen atoms in ring U are optionally substituted with F, cyano, C₁₋₆ alkyl, or C₁-C₆ hydroxyalkyl;
   ring Y is 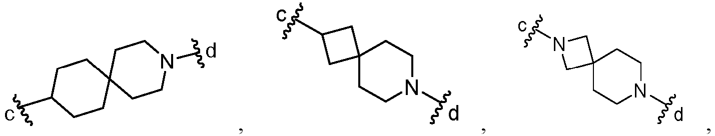 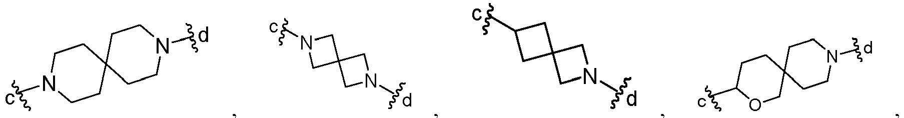 or 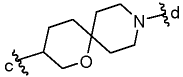 , wherein end c is linked to Ly, and end d is linked to X"; 1, 2, 3, or 4 hydrogen atoms in ring Y are optionally substituted with F;
   Ly is -(CH₂)ₖ-, wherein one or two CH₂ contained in Ly are each independently optionally replaced by -O- or -N(C₁-C₆ alkyl)-; k is 1, 2, 3, 4, 5, or 6;
   X" is -C(O)-.

Preferably, in certain embodiments of the present disclosure, LA is LA-9 or LA-10: 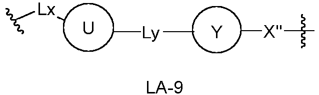 wherein in LA-9:
Lx is -(CH₂)ᵥ-, wherein one or two CH₂ contained in Lx are each independently optionally replaced by -O- or -CRaRₑ-; -CR_{d}Rₑ- is 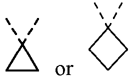 ; v is 1, 2, 3, 4, 5, or 6;
ring U is 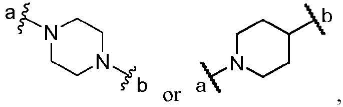 wherein end a is linked to Lx, and end b is linked to Ly;
ring Y is 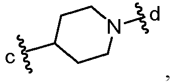 wherein end c is linked to Ly, and end d is linked to X";
Ly is -(CH₂)ₖ-, wherein one or two CH₂ contained in Ly are each independently optionally replaced by -O-, -C≡C-, or -N(C₁-C₆ alkyl)-; k is 1, 2, 3, 4, 5, or 6;
X" is -C(O)-; 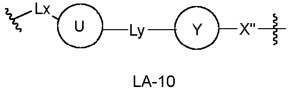
wherein in LA-10:
   Lx is -(CH₂)ᵥ-, wherein one or two CH₂ contained in Lx are each independently optionally replaced by -O- or -CR_{d}Rₑ-; -CR_{d}Rₑ- is 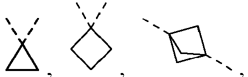 -CH(CH₃)-, -CH(OCH₃)-, -CH(OH)-, or - CH(NH₂)-; v is 1, 2, 3, 4, 5, or 6;
   ring U is 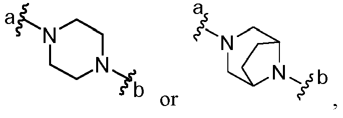 wherein end a is linked to Lx, and end b is linked to Ly;
   ring Y is 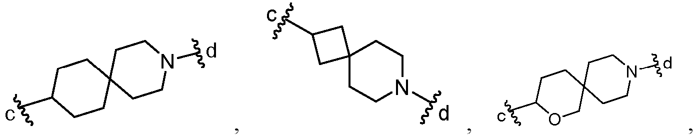 or 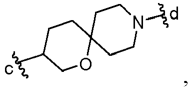 wherein end c is linked to Ly, and end d is linked to X";
   Ly is -(CH₂)ₖ-, and k is 1, 2, 3, 4, 5, or 6;
   X" is -C(O)-.

Preferably, in certain embodiments of the present disclosure, LA is LA-11 or LA-12: 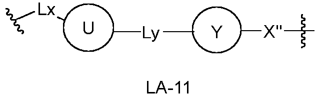 wherein in LA-11:
Lx is -(CH₂)ᵥ-, wherein one or two CH₂ contained in Lx are each independently optionally replaced by -O- or -CR_{d}Rₑ-; -CR_{d}Rₑ- is 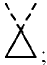 v is 1 2 3 4 5, or 6;
ring U is 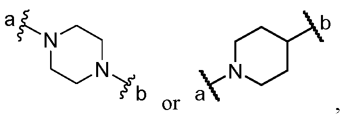 wherein end a is linked to Lx, and end b is linked to Ly;
ring Y is 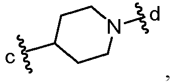 wherein end c is linked to Ly, and end d is linked to X";
Ly is -(CH₂)ₖ-, wherein one or two CH₂ contained in Ly are each independently optionally replaced by -O- or -C≡C-; k is 1, 2, 3, 4, 5, or 6;
X" is -CO-; 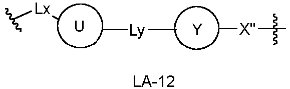
wherein in LA-12:
   Lx is -(CH₂)ᵥ-, wherein one or two CH₂ contained in Lx are each independently optionally replaced by -O- or -CRaRₑ-; -CR_{d}Rₑ- is 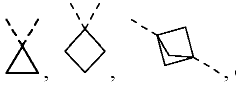 , or -CH(CH₃)-; v is 1, 2, 3, 4, 5, or 6;
   ring U is 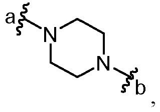 , wherein end a is linked to Lx, and end b is linked to Ly;
   ring Y is 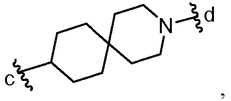 , wherein end c is linked to Ly, and end d is linked to X";
   Ly is -(CH₂)ₖ-, and k is 1, 2, 3, 4, 5, or 6;
   X" is -C(O)-.

Preferably, in certain embodiments of the present disclosure, Lx is

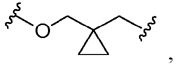

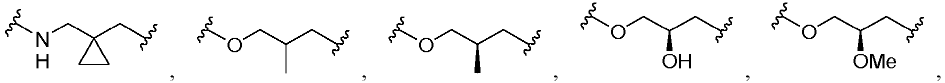

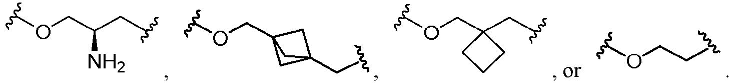

Preferably, in certain embodiments of the present disclosure, Ly is -CH₂-, -CH₂CH₂-,-CH₂CH₂CH₂-, -CH₂CH₂CH₂CH₂-,

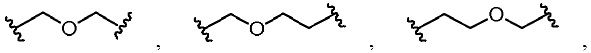

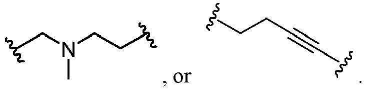

Preferably, in certain embodiments of the present disclosure, LA is of any one of the following structures: 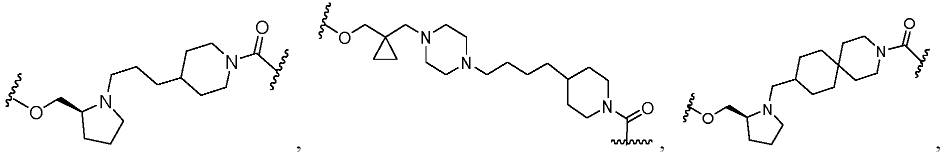 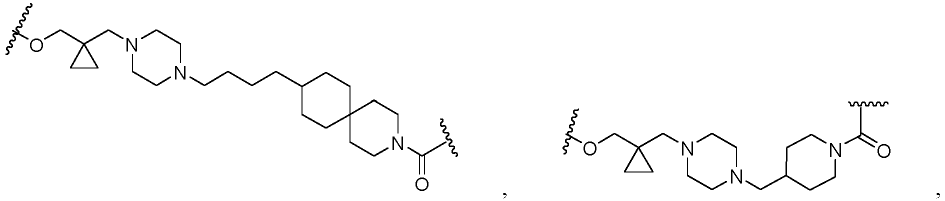 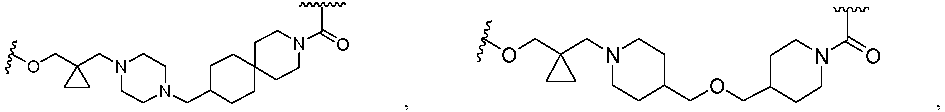 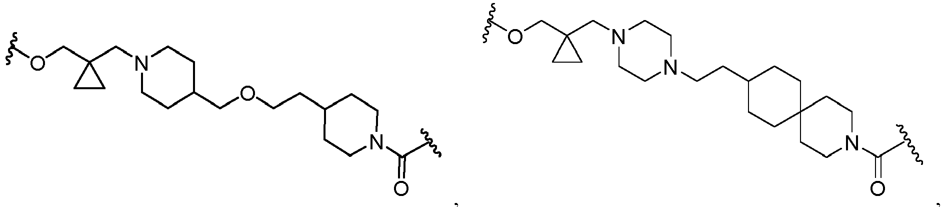 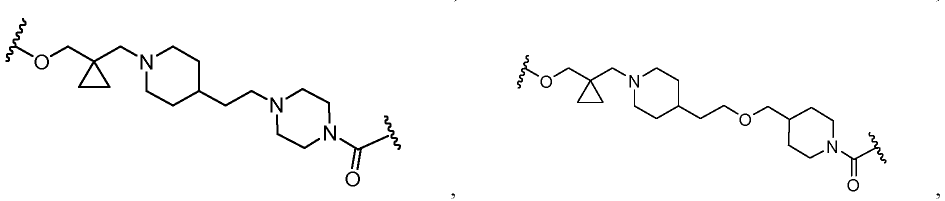 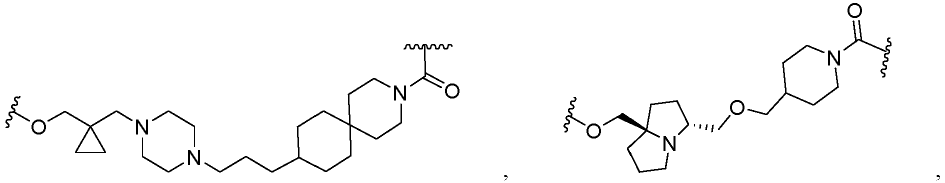 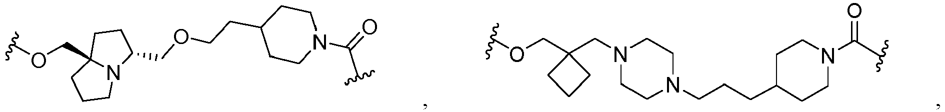 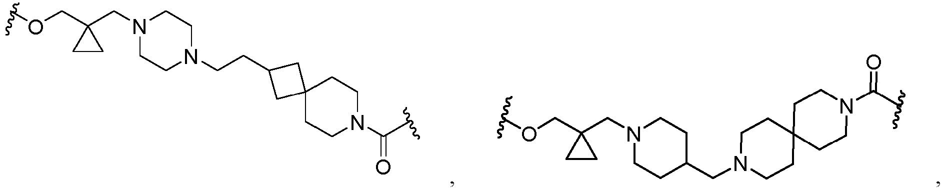 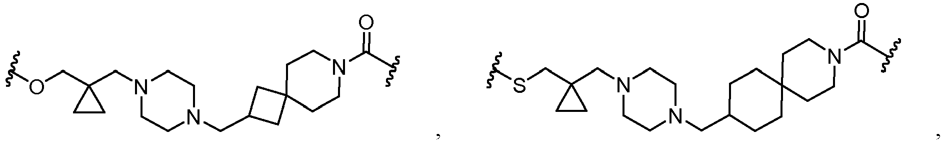 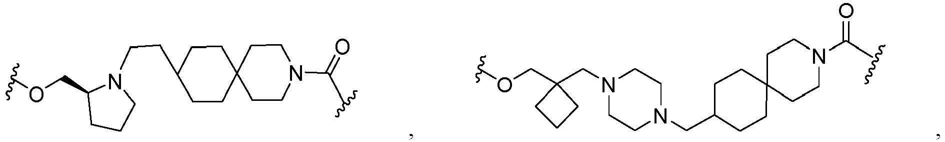 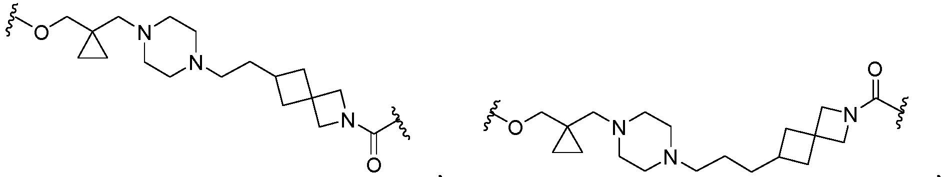 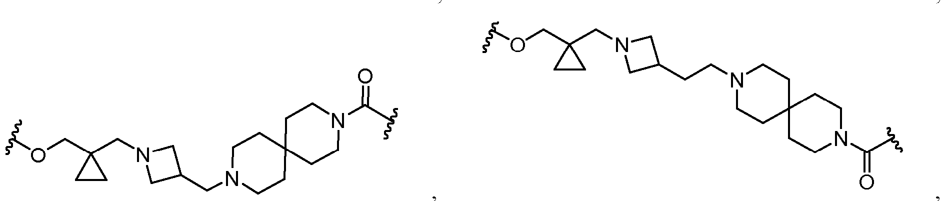 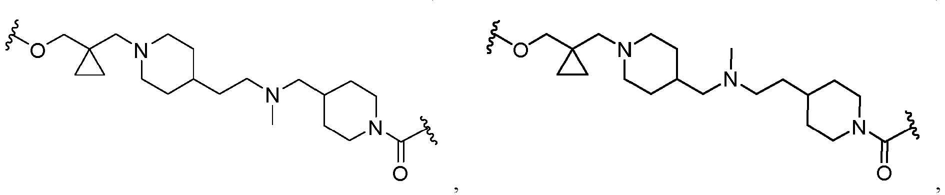 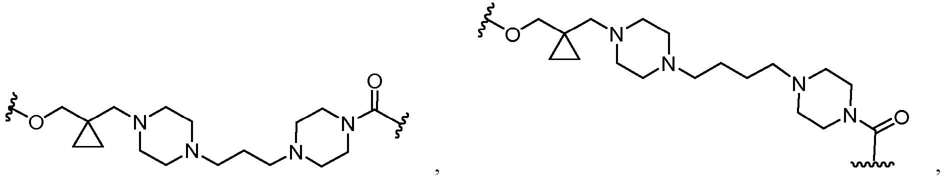 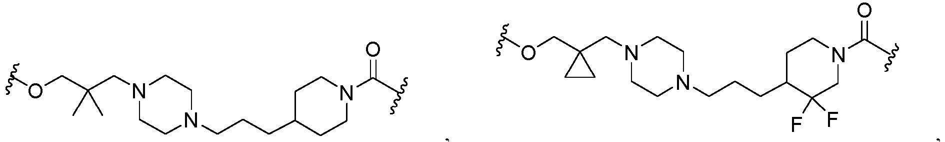 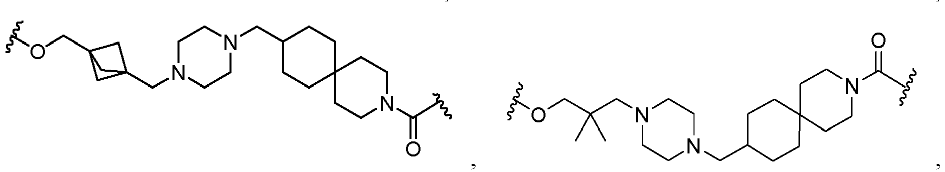 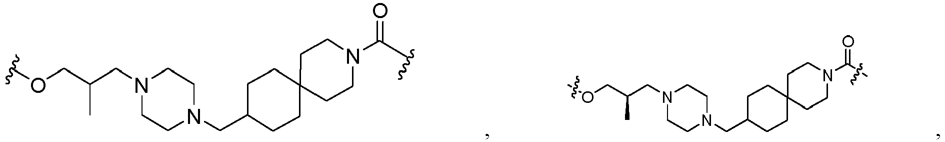 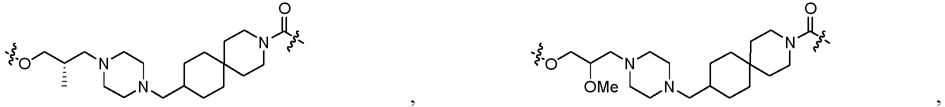 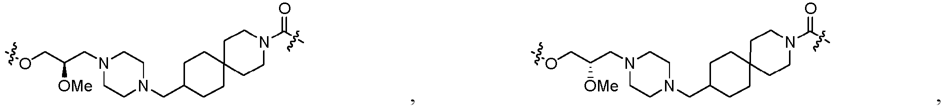 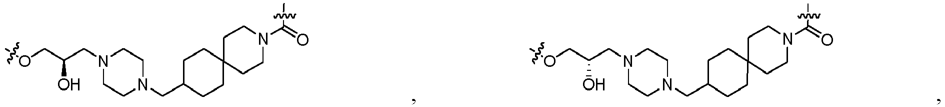 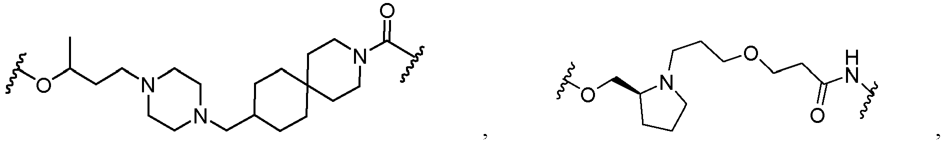 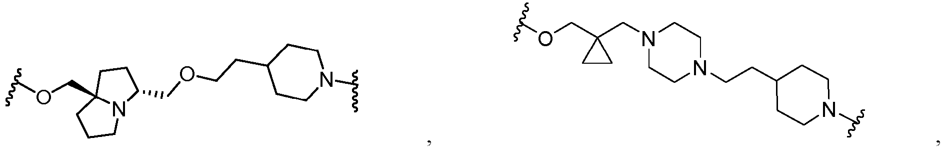 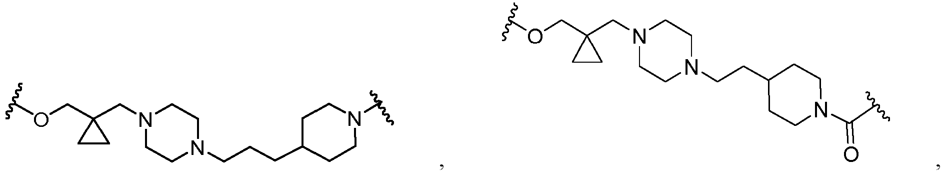 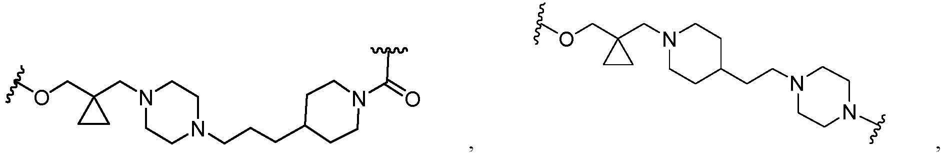 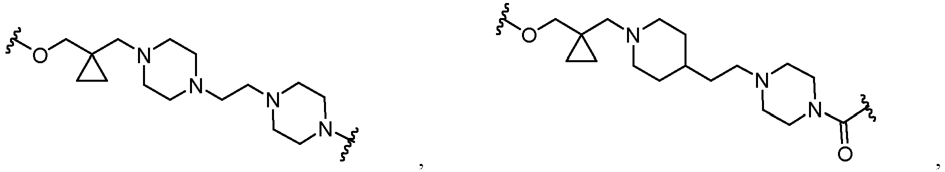 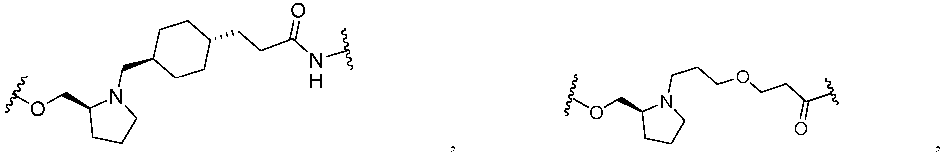 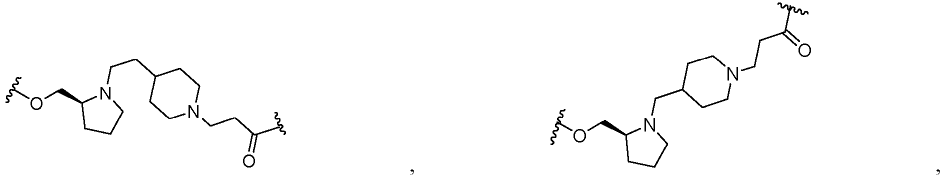 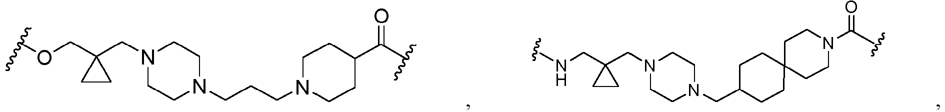 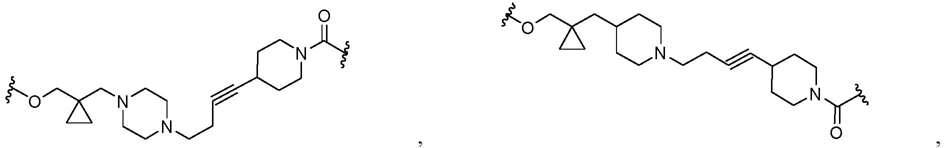 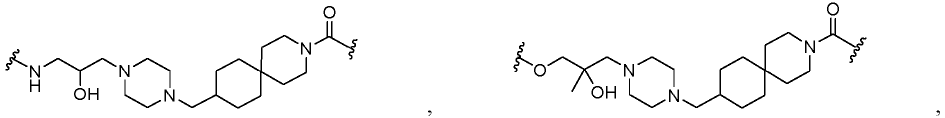 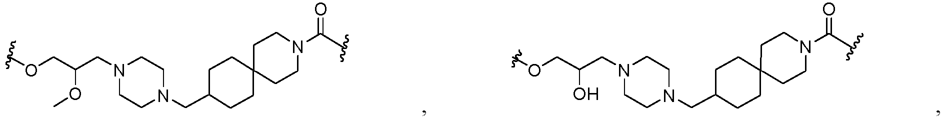 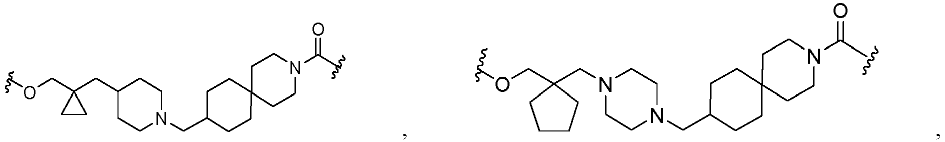 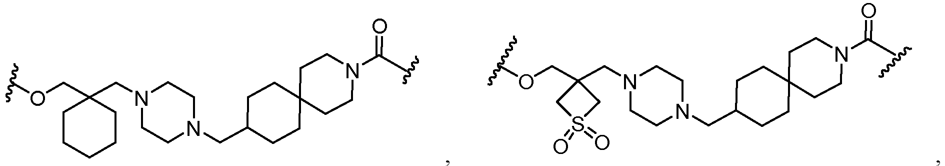 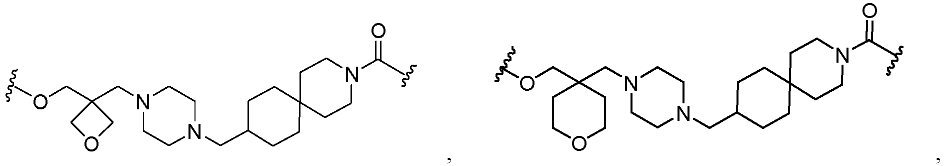 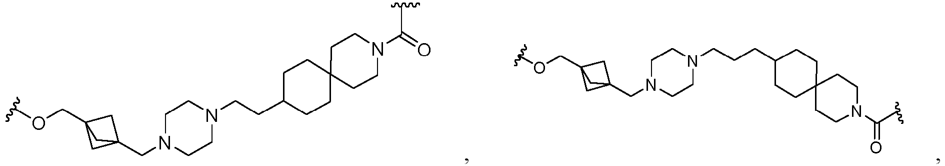 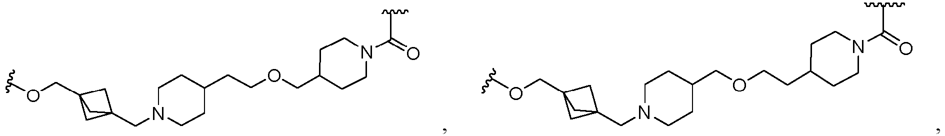 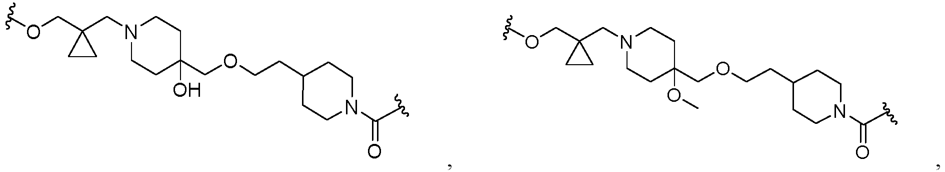 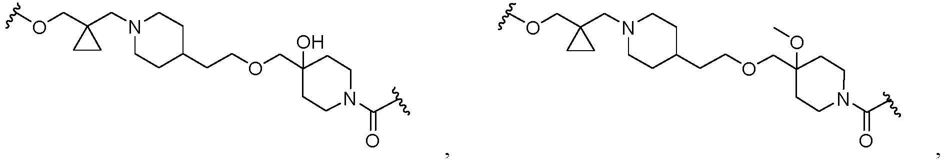 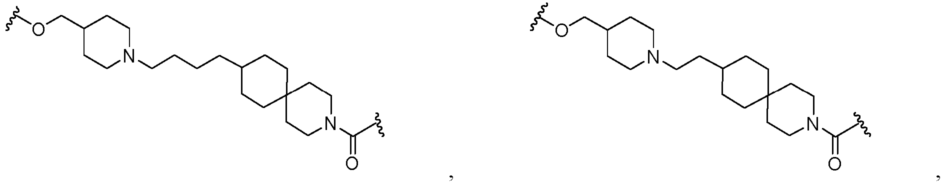 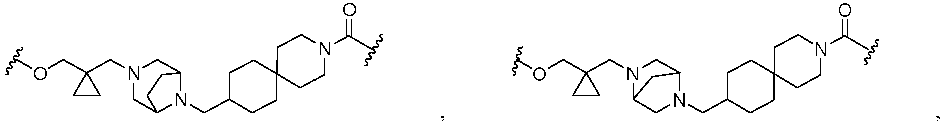 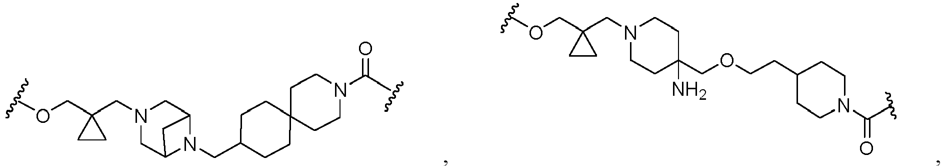 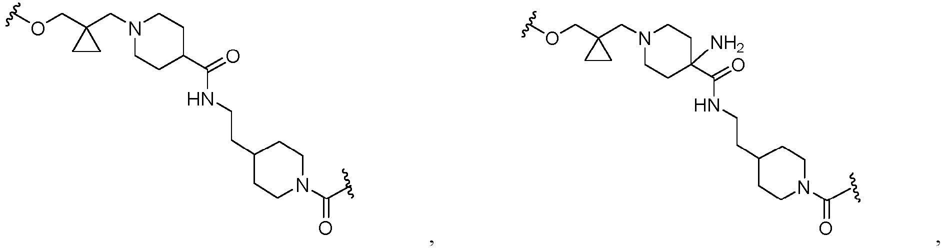 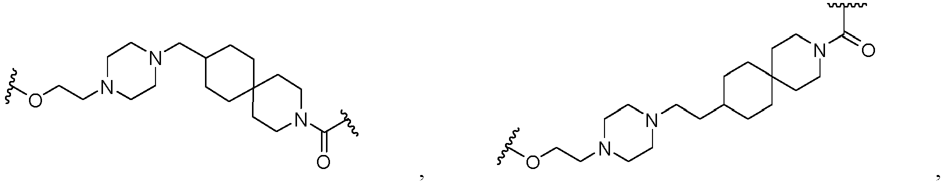 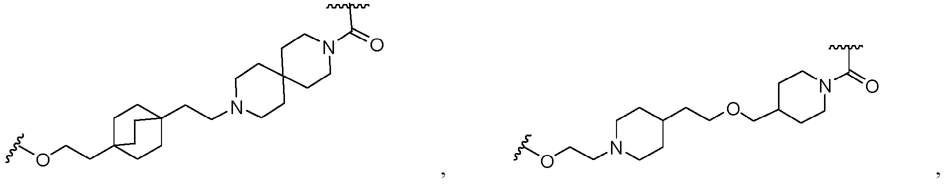 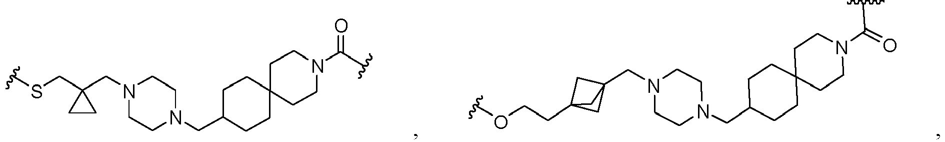 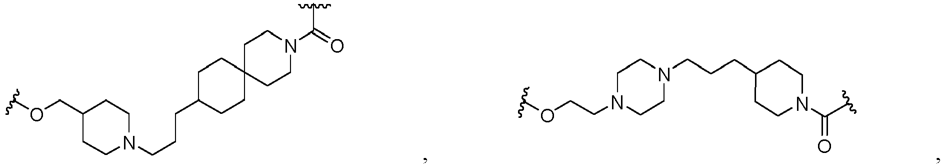 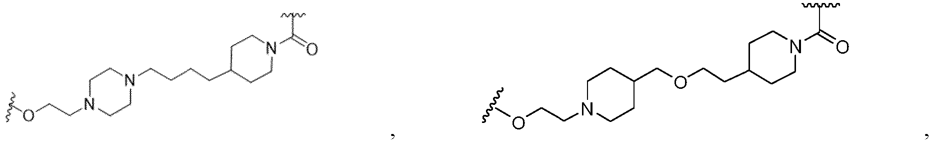 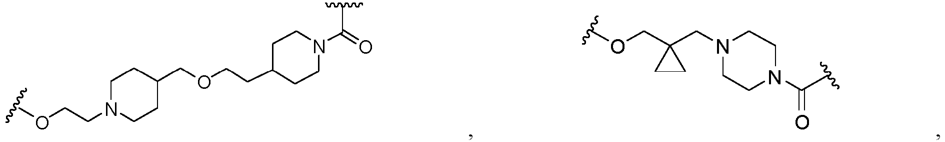 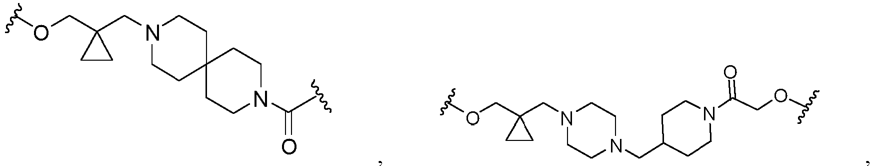 or

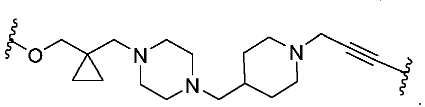

Preferably, in certain embodiments of the present disclosure, LA is of any one of the following structures:

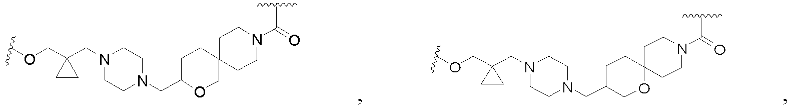

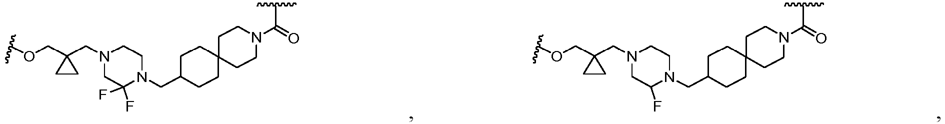

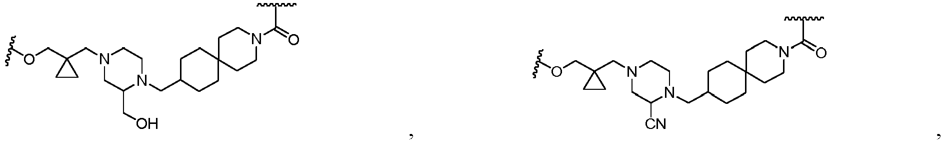

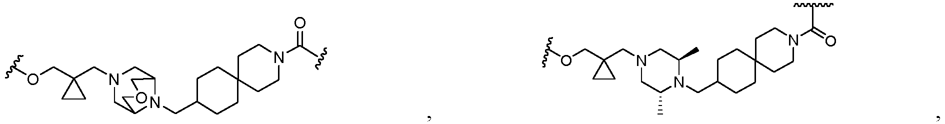

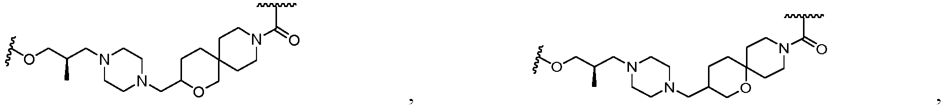

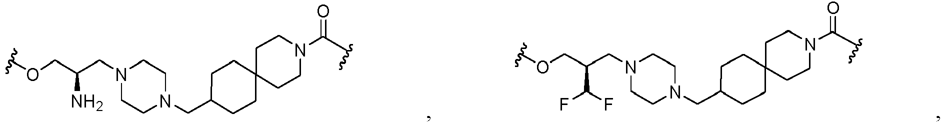

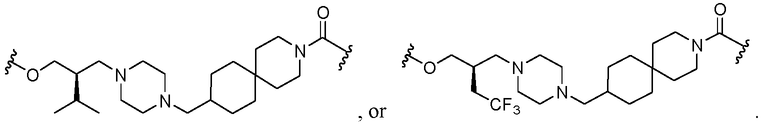

Preferably, in certain embodiments of the present disclosure, LA is of any one of the following structures: 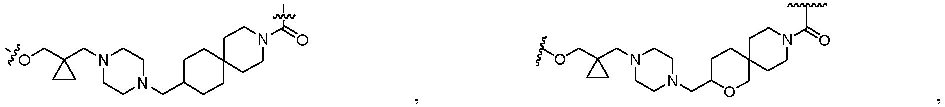 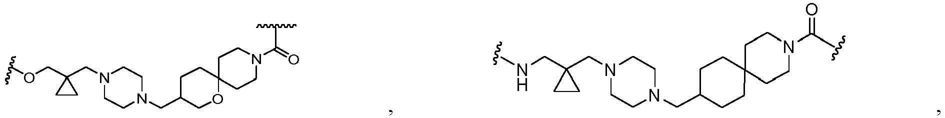 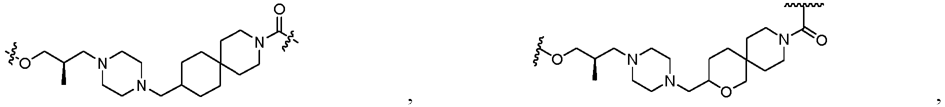 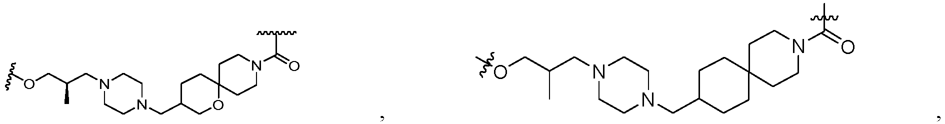 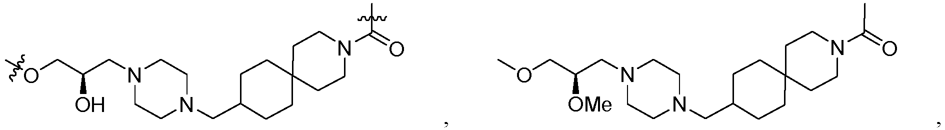 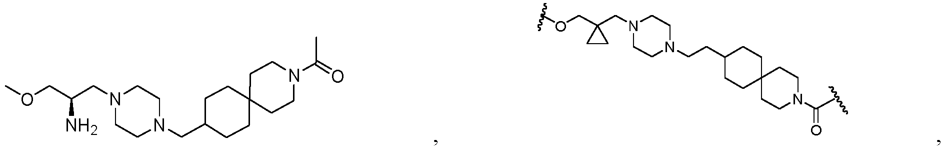 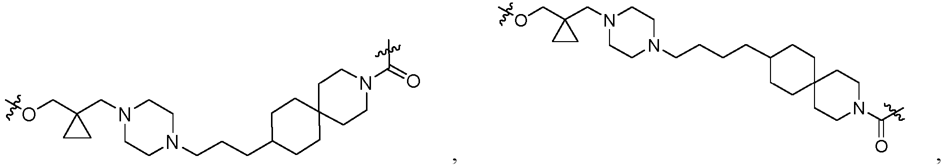 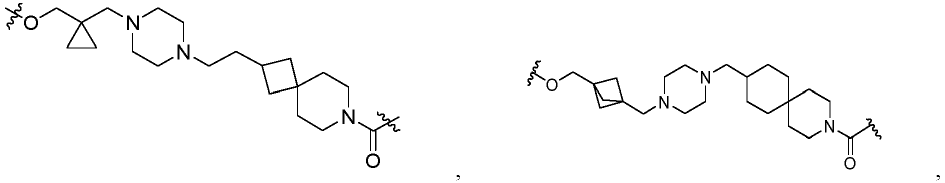 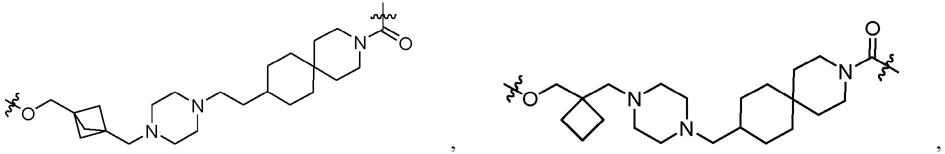 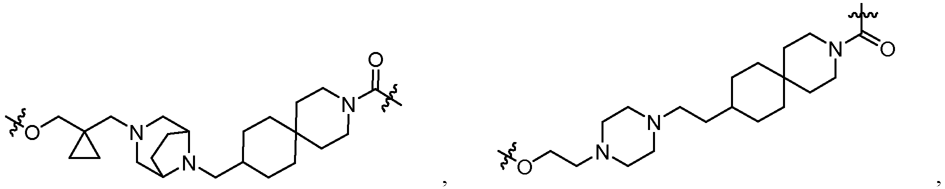 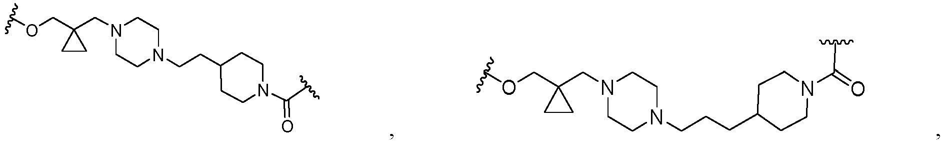 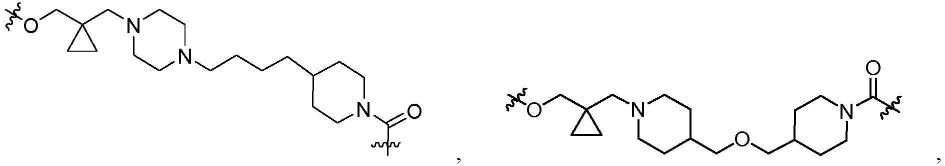 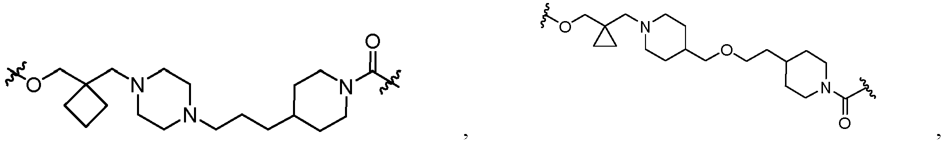 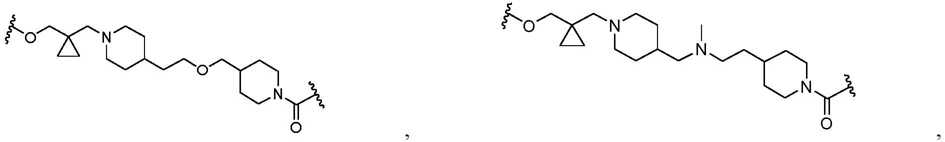 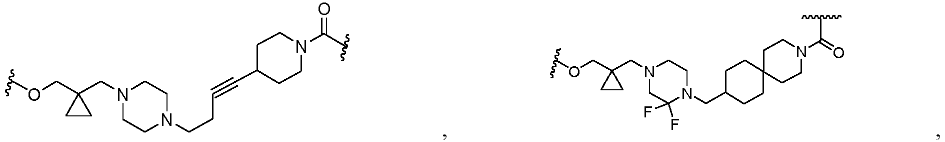 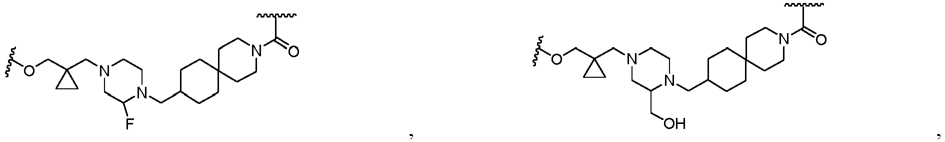 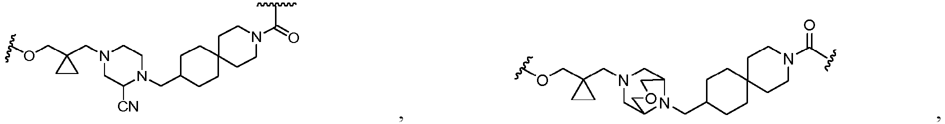 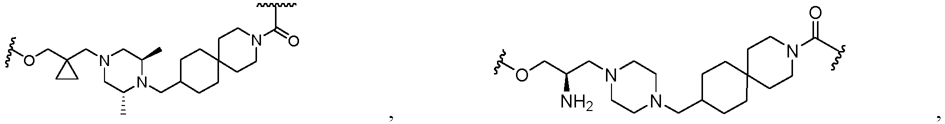 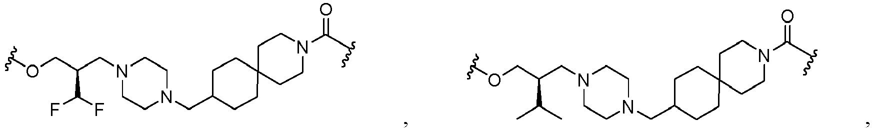 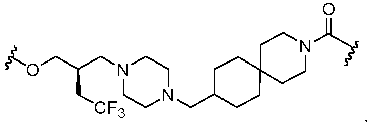 or

Preferably, in certain embodiments of the present disclosure, E1 has a structure of formula E1-1a, E1-1b, E1-1c, E1-1d, E1-1e, E1-1f, E1-1g, E1-1aa, E1-1h, 1-1bb, E1-1cc, E1-1dd, E1-1ee, E1-1ff, E1-1gg, or E1-1hh: 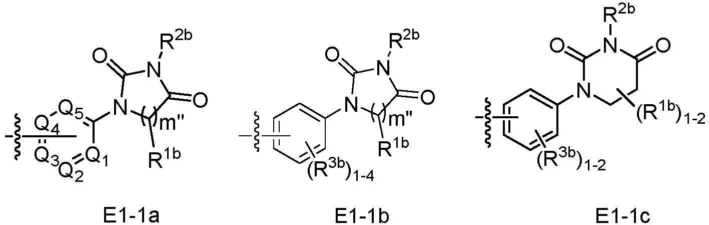 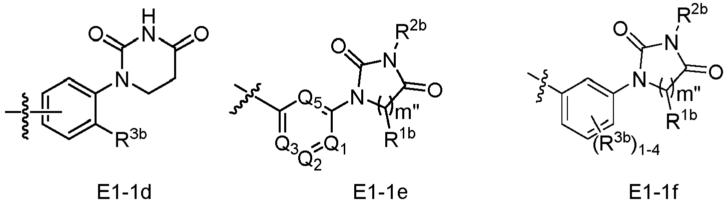 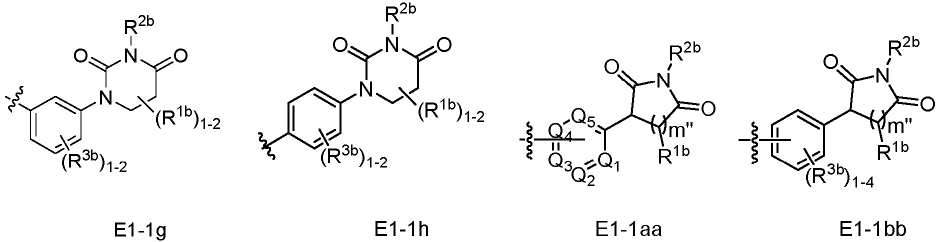 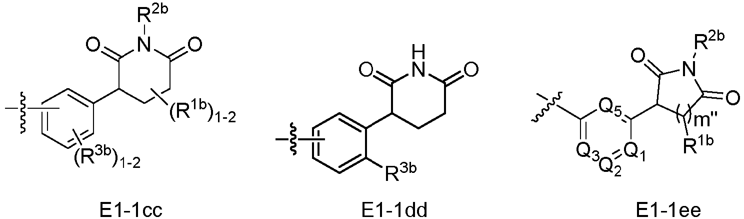 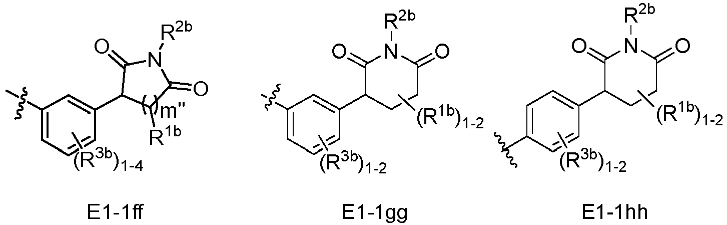 wherein Q₁, Q₂, Q₃, Q₄, Q₅, R₁", R₂", R₃", and m" are as defined and described in the present disclosure.

Preferably, in certain embodiments of the present disclosure, E1 has a structure of formula E1-1h", E1-1i', E1-1j', or E1-1h'h': 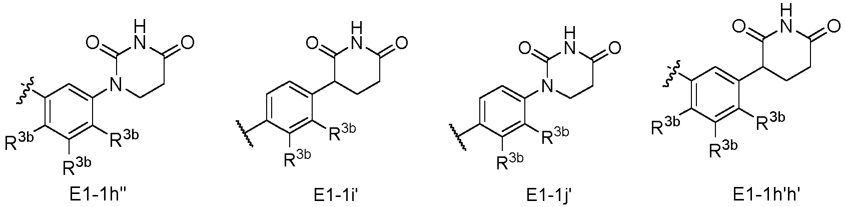 wherein R^{3b} is as defined and described in the present disclosure.

Preferably, in certain embodiments of the present disclosure, R^{3b} is hydrogen, halogen, cyano, - OH, -NH₂, C₁-C₆ alkyl, C₃-C₈ cycloalkyl, 3- to 8-membered heterocycloalkyl, -O(C₁-C₆ alkyl), -O(C₃-C₈ cycloalkyl), -O(3- to 8-membered heterocycloalkyl), -N(C₁-C₆ alkyl)₁₋₂, -N(C₃-C₈ cycloalkyl)₁₋₂, or -S(C₁-C₆ alkyl), wherein the C₁-C₆ alkyl, C₃-C₈ cycloalkyl, 3- to 8-membered heterocycloalkyl, -O(C₁-C₆ alkyl), - O(C₃-C₈ cycloalkyl), -O(3- to 8-membered heterocycloalkyl), -N(C₁-C₆ alkyl)₁₋₂, -N(C₃-C₈ cycloalkyl)₁₋₂, or -S(C₁-C₆ alkyl) is optionally substituted with 1-3 halogen, cyano, -OH, and -NH₂.

Preferably, in certain embodiments of the present disclosure, E1 has a structure of formula E1-1h": 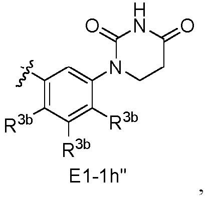 wherein each R^{3b} is independently hydrogen, halogen, C₁-C₆ alkyl, or -O(C₁-C₆ alkyl), the C₁-C₆ alkyl and -O(C₁-C₆ alkyl) being optionally substituted with deuterium, halogen, cyano, -OH, or -NH₂, and the number of substitution is 1, 2, or 3.

Preferably, in certain embodiments of the present disclosure, E1 is:

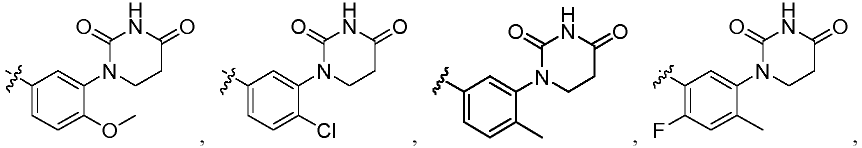

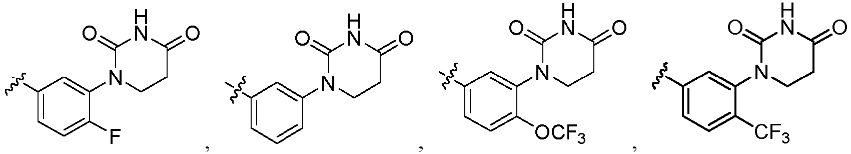

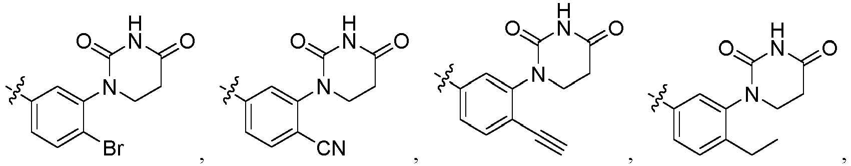

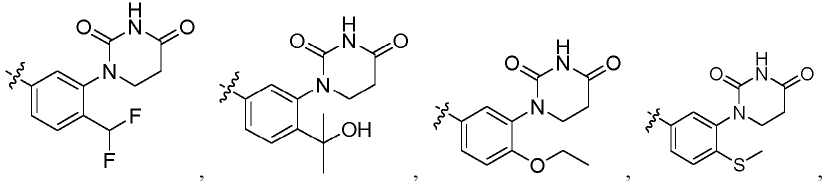

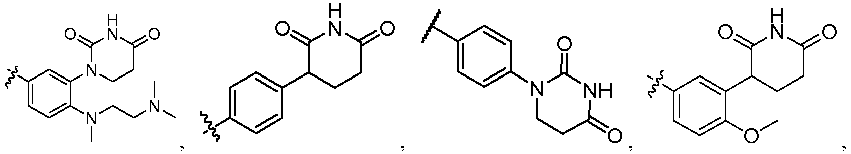

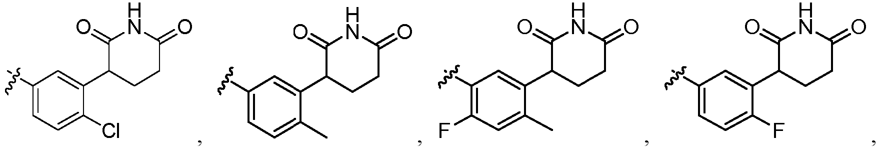

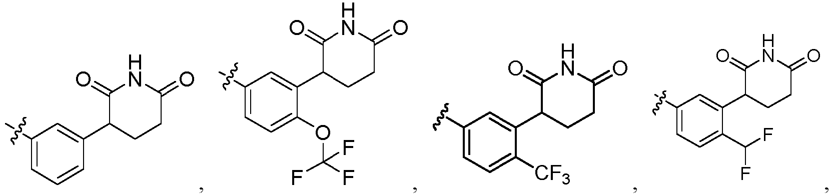

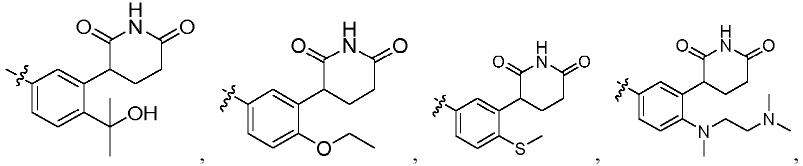

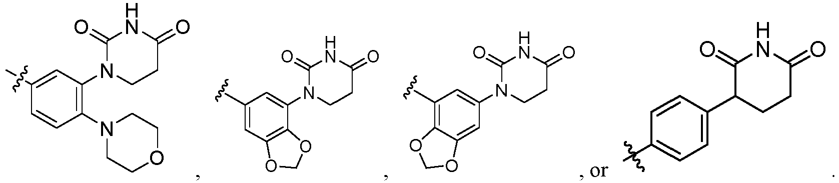

Preferably, in certain embodiments of the present disclosure, E1 is 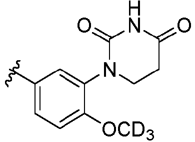 or

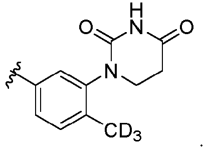

Preferably, in certain embodiments of the present disclosure, E1 is

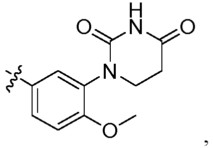

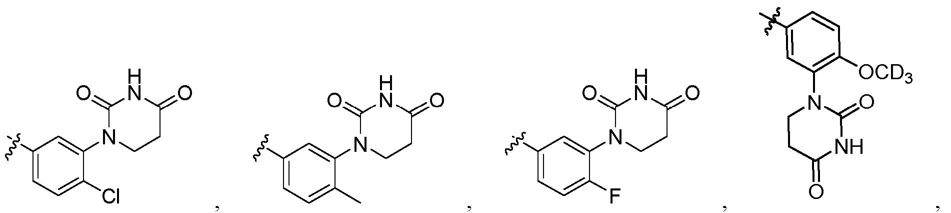

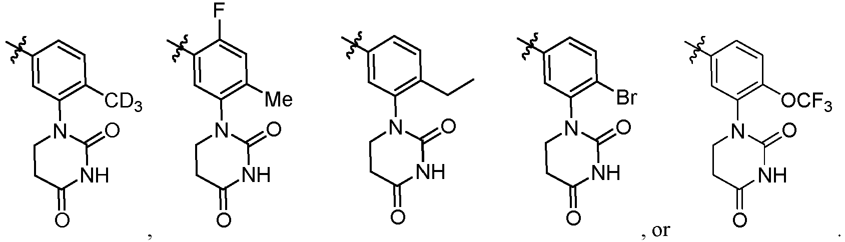

Preferably, in certain embodiments of the present disclosure, E2 has a structure of formula E2-1a, E2-1b, E2-1c, E2-1d, E2-1e, or E2-1f: 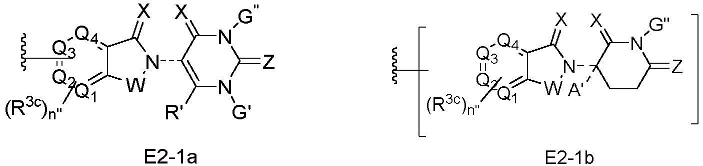 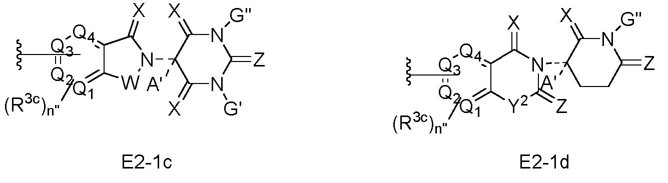 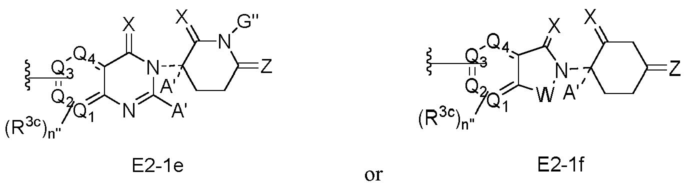 wherein in E2-1a, E2-1b, E2-1c, E2-1d, E2-1e, and E2-1f:
W is CR^{1c}R^{2c}, C(S), C(O), or SO₂;
X is CH₂, O, or S;
Y² is NH, N-alkyl, N-aryl, N-heteroaryl, N-cycloalkyl, N-heterocycloalkyl, O, or S;
Z is CH₂, O, or S;
G" and G' are each independently selected from hydrogen, deuterium, C₁-C₆ alkyl, OH, C₃-C₆ cycloalkyl, -CH₂-heterocycloalkyl, or -CH₂-phenyl, wherein the C₁-C₆ alkyl, C₃-C₆ cycloalkyl, -CH₂-heterocycloalkyl, or -CH₂-phenyl is optionally substituted with 1-3 groups independently selected from hydroxy, halogen, cyano, and amino;
Q₁, Q₂, Q₃, and Q₄ are each independently CR^{3b} or N;
A' is hydrogen, deuterium, C₁-C₆ alkyl, C₃-C₆ cycloalkyl, or halogen;
R^{1c}, R^{2c}, and R^{3c} are each independently selected from hydrogen, hydroxy, halogen, -NH₂, -N(C₁-C₆ alkyl)₁₋₂, C₁-C₆ alkyl, C₁-C₆ alkoxy, C₁-C₆ haloalkyl, -CONR_{'}R_{"} -OR_{'}, -NR_{'}R_{"}, -SR_{'} -SO₂R_{'} -SO₂NR_{'}R_{"} -CR_{'}R_{"} -CR_{'}NR_{'}R_{"}, aryl, heteroaryl, C3-C8 cycloalkyl, 3- to 8-membered heterocycloalkyl, -P(O)(OR_{'})R_{"},-P(O)R_{'}R_{"}, -OP(O)(OR_{'})R_{"}, -Cl, -F, -Br, -I, -CF₃, -CN, -NR_{'}SO₂NR_{'}R_{"}, -NR_{'}C(O)NR_{'}R_{"}, -C(O)NR_{'}C(O)R_{"},-NR_{'}C(=N-CN)NR_{'}R_{"}, -C(=N-CN)NR_{'}R_{"}, -NR_{'}C(=N-CN)R_{"}, -NR_{'}C(=C-NO₂)NR_{'}R_{"}, -SO₂NR_{'}COR_{"}, -NO₂,-COR_{'}, -C(C=N-OR_{'})R_{"}, -CR_{'}=CR_{'}R_{"}, -CCR_{'}, -S(C=O)(C=N-R_{'})R_{"}, -SF₅, and -OCF₃;
R_{'} and R_{"} are each independently selected from a bond, hydrogen, deuterium, C₁-C₆ alkyl, C₃-C₈ cycloalkyl, C₆-C₁₀ aryl, 5- to 10-membered heteroaryl, and 3- to 8-membered heterocycloalkyl;
n^{"} is 1, 2, 3, or 4;
----- is a bond, which may be an *R* stereoisomer, an *S* stereoisomer, or a non-stereoisomer;
R^{3b} is as described and defined in the present disclosure;

Preferably, in certain embodiments of the present disclosure, E2 has a structure of formula E2-1bb or E2-1aa: 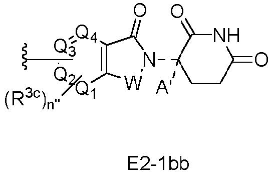 wherein in E2-1bb:
Q₁, Q₂, Q₃, and Q₄ are each independently CR^{3b} or N;
W is C(O) or CH₂;
A' is hydrogen, deuterium, C₁-C₆ alkyl, or halogen;
R^{3c} is selected from hydrogen, deuterium, hydroxy, halogen, -NH₂, -N(C₁-C₆ alkyl)₁₋₂, C₁-C₆ alkyl, C₁-C₆ alkoxy, and C₁-C₆ haloalkyl;
n^{"} is 1, 2, 3, or 4;
----- is a bond, which may be an R stereoisomer, an S stereoisomer, or a non-stereoisomer;
R^{3b} is as described and defined in the present disclosure; 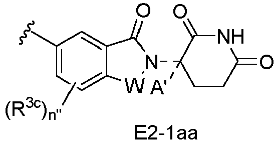
wherein E2-1aa:
   W is CH₂ or C(O);
   A' is hydrogen, methyl, Cl, or F;
   each R^{3c} is independently selected from hydrogen, hydroxy, NH₂, C₁-C₆ alkyl, and C₁-C₆ alkoxy;
   n^{"} is 1, 2, 3, or 4;
   ----- is a bond, which may be an R stereoisomer, an S stereoisomer, or a non-stereoisomer.

Preferably, in certain embodiments of the present disclosure, E2 is:

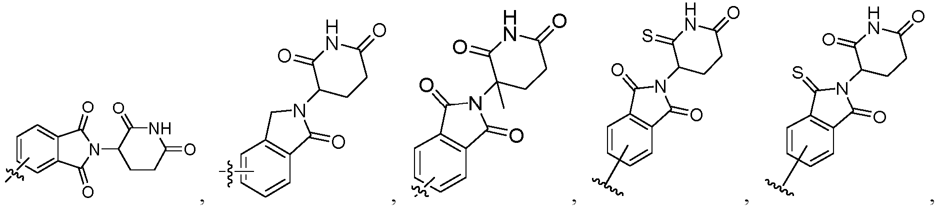

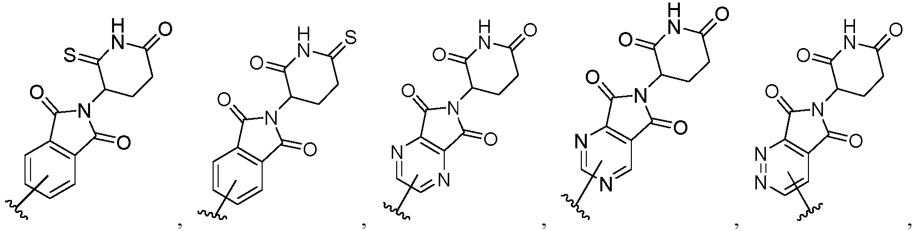

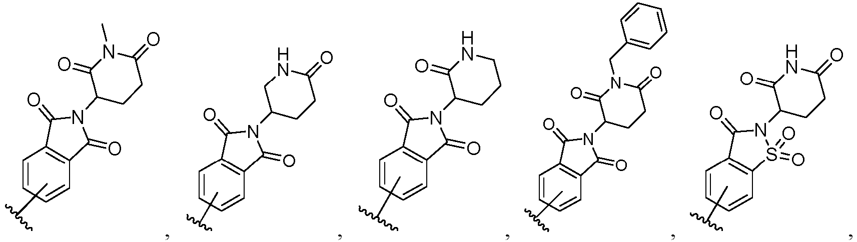

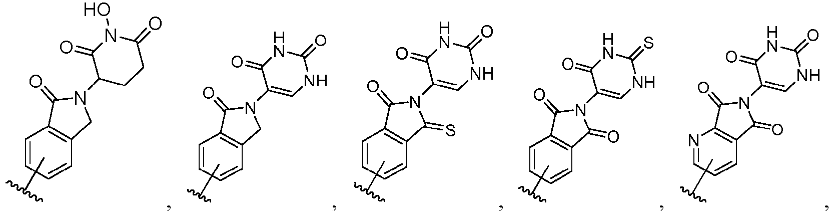

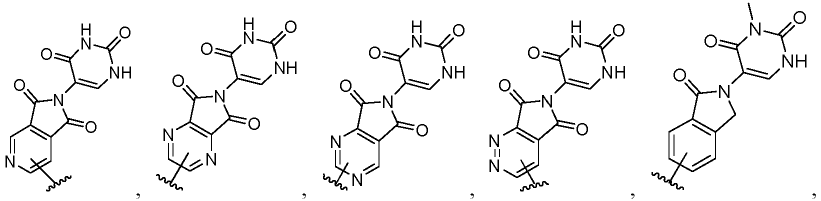

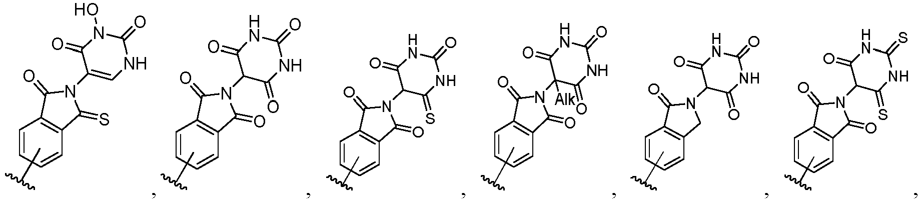

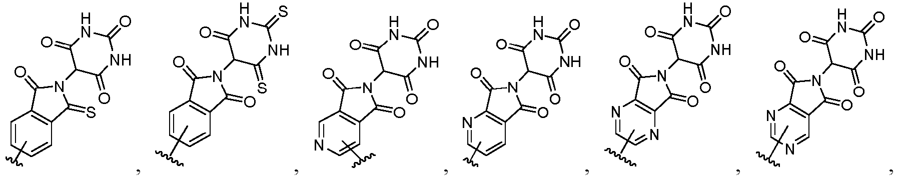

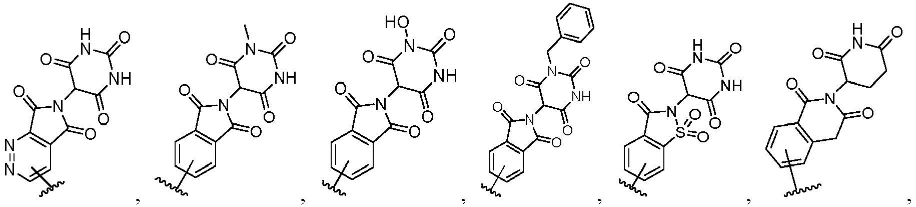

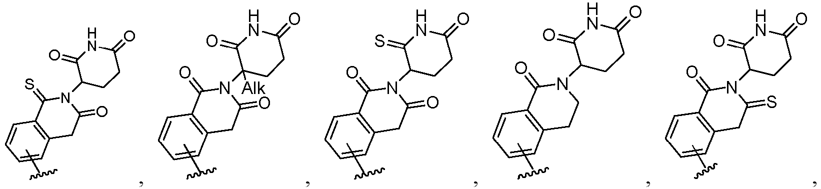

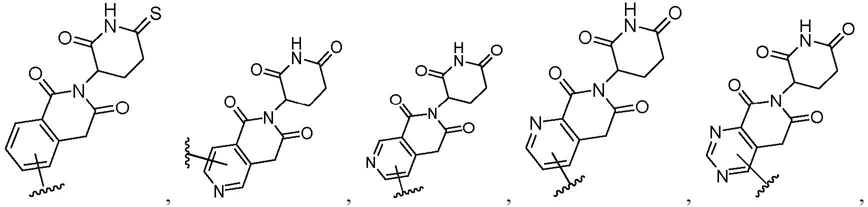

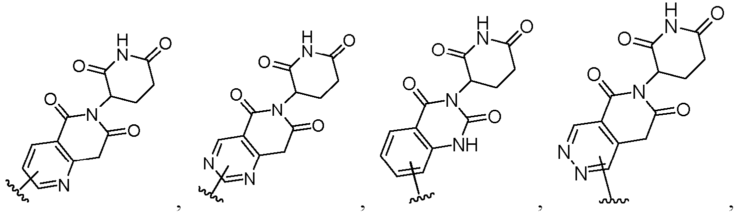

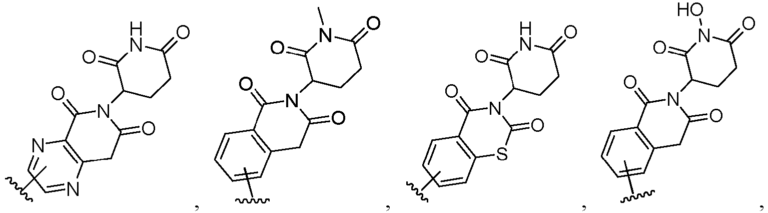

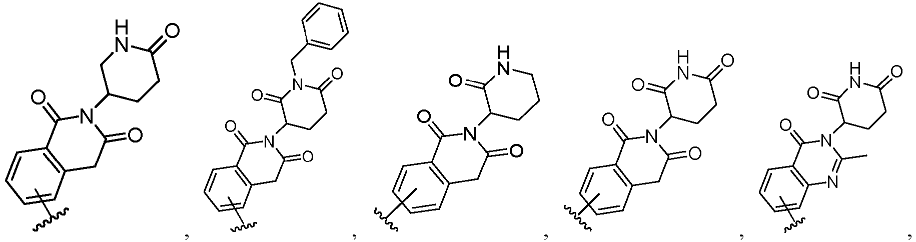

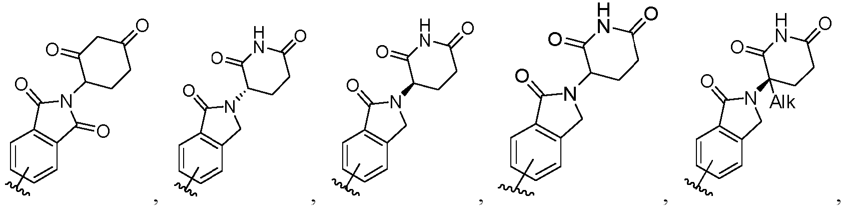

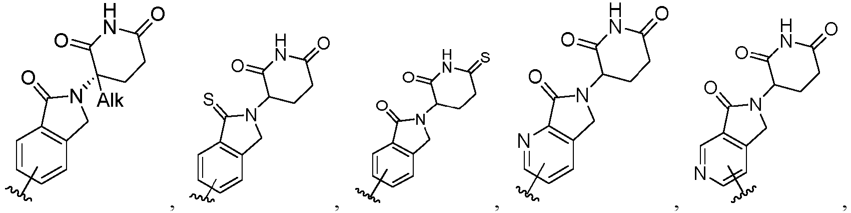

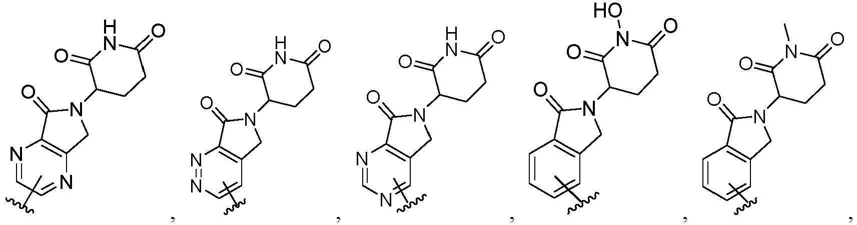

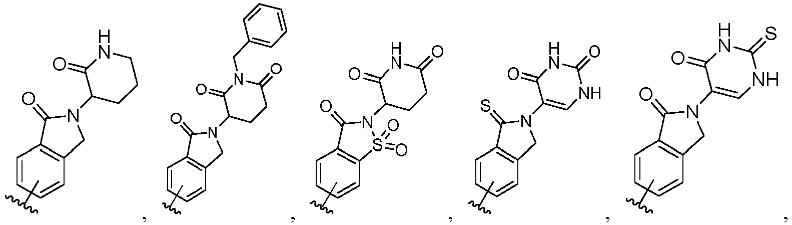

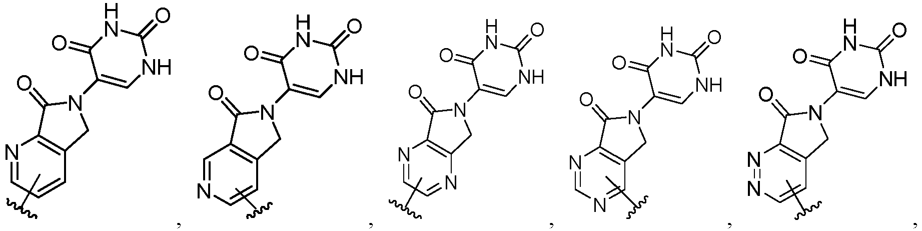

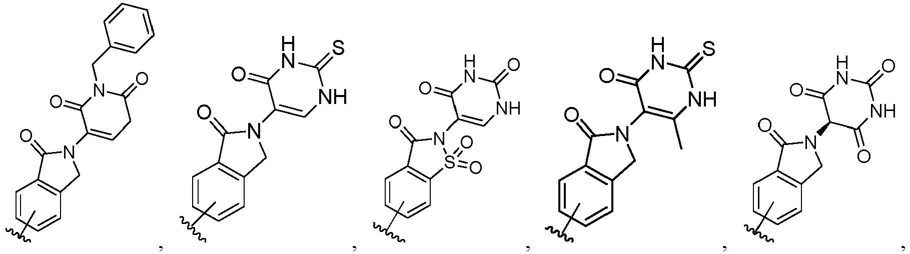

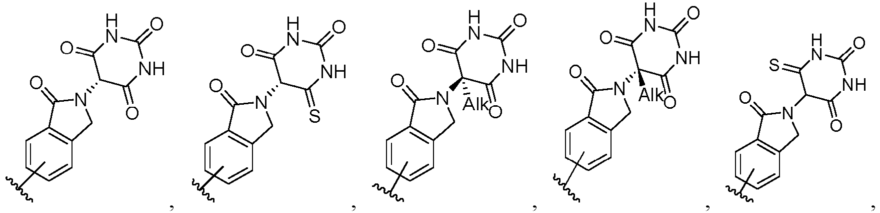

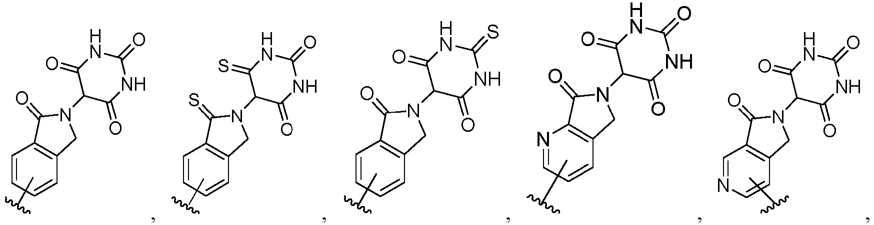

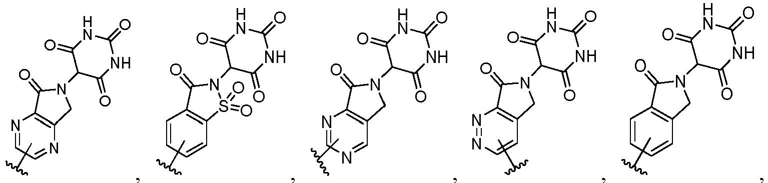

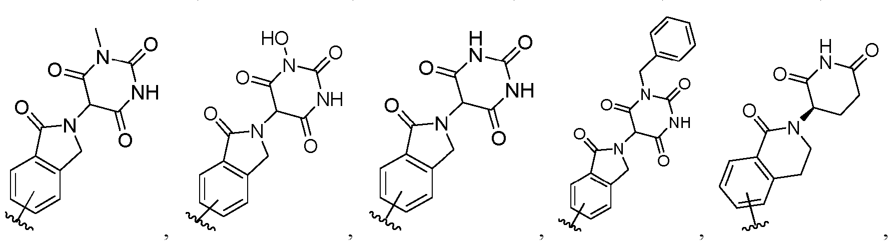

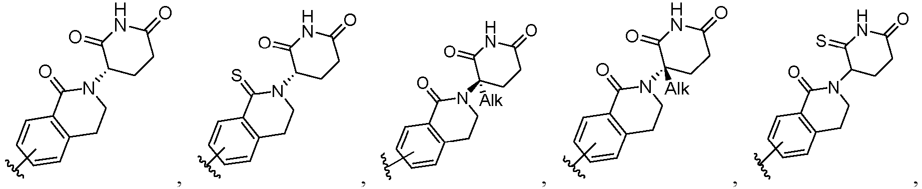

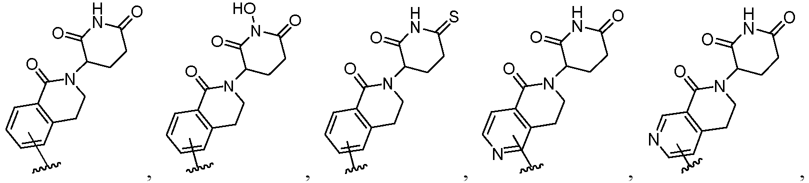

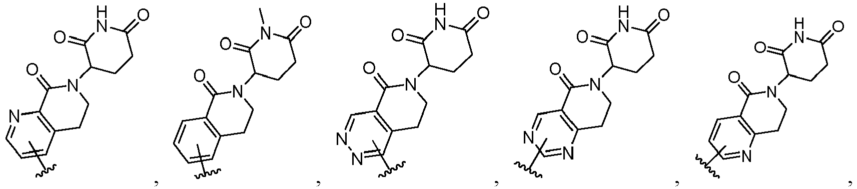

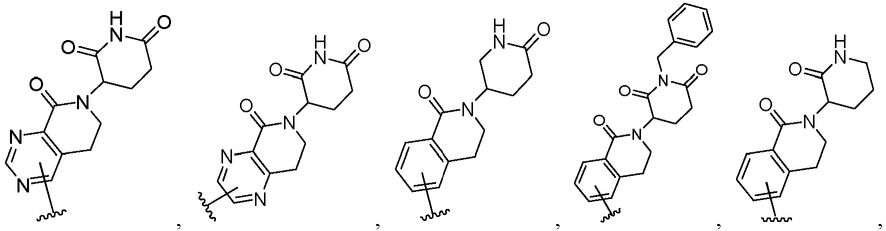

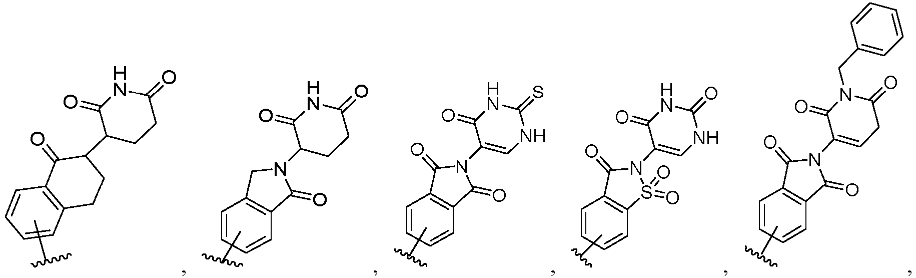

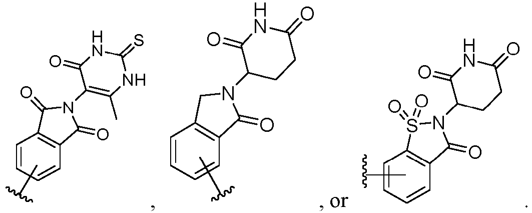

Preferably, in certain embodiments of the present disclosure, E2 is 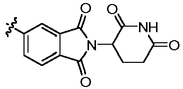 or

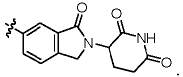

Preferably, in certain embodiments of the present disclosure, E3 has a structure of formula E3-I: 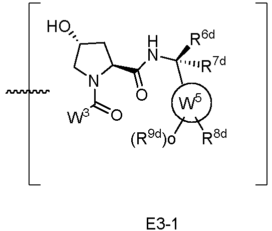
W³ is aryl, heteroaryl, or 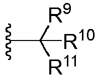 , wherein the aryl or heteroaryl is optionally substituted;
R⁹ and R¹⁰ are each independently selected from hydrogen, alkyl, C₃-C₈ cycloalkyl, and 5- to 10-membered heteroaryl, wherein the alkyl, C₃-C₈ cycloalkyl, or 5- to 10-membered heteroaryl is optionally substituted with 1 or more -OH, halogen, or -NH₂;
or R⁹ and R¹⁰, together with the carbon atom linked thereto, form C₃-C₈ cycloalkyl, wherein the C₃-C₈ cycloalkyl is optionally substituted with -OH, halogen, -NH₂, or C₁-C₃ alkyl;
R¹¹ is selected from C₁-C₆ alkyl, C₁-C₆ alkoxy, 3- to 8-membered heterocycloalkyl, C₆-C₁₀ aryl, 5-to 10-membered heteroaryl, and 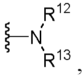 wherein the C₁-C₆ alkyl, C₁-C₆ alkoxy, 3- to 8-membered heterocycloalkyl, C₆-C₁₀ aryl, or 5- to 10-membered heteroaryl is optionally substituted with 1 or more -OH, halogen, or -NH₂;
R¹² is selected from H, C(O), and substituted alkyl;
R¹³ is selected from H, alkyl, -alkyl CO-, -(cycloalkyl)alkyl CO-, -aralkyl CO-, -aryl CO-, - (heterocycloalkyl)CO-, and arylalkyl, wherein the alkyl, -alkyl CO-, -(cycloalkyl)alkyl CO-, -aralkyl CO-, - aryl CO-, -(heterocycloalkyl)CO-, or arylalkyl is optionally substituted;
R¹⁶ is H, halogen, -OH, alkyl, or alkoxy, wherein the alkyl or alkoxy is optionally substituted with halogen;
● is 1, 2, 3, or 4;
R^{8d}, R^{14a}, R^{14b}, and R¹⁵ are as described and defined in the present disclosure.

Preferably, in certain embodiments of the present disclosure, E3 has a structure of formula E3-1a, E3-1b, or E3-1c: 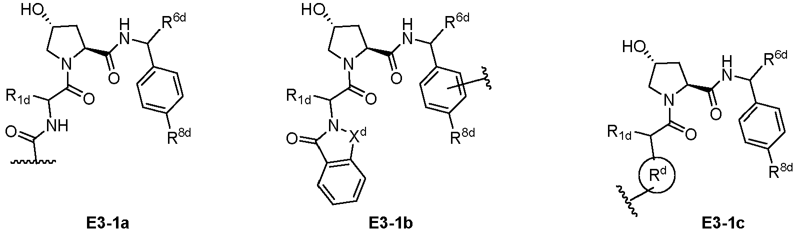 wherein,
R_{1d} is H, ethyl, isopropyl, *tert*-butyl, *sec*-butyl, cyclopropyl, cyclobutyl, cyclopentyl, cyclohexyl, alkyl, hydroxyalkyl, heteroaryl, or haloalkyl, wherein the alkyl, hydroxyalkyl, or heteroaryl is optionally substituted;
R^{6d} is H, -CH₃, -CH₂F, -CH₂OH, ethyl, isopropyl, or cyclopropyl;
R^{8d} is H, halogen, CN, OH, NO₂, aryl, heteroaryl, C₁-C₆ alkyl, C₁-C₆ alkoxy, cycloalkyl, or heterocycloalkyl, wherein the aryl, heteroaryl, C₁-C₆ alkyl, C₁-C₆ alkoxy, cycloalkyl, or heterocycloalkyl is optionally substituted with halogen, -OH, CN, NO₂, or amino;
X^{d} is CH₂ or C(O);
R_{d} is 5- to 6-membered heteroaryl, wherein the 5- to 6-membered heteroaryl is optionally substituted.

Preferably, in certain embodiments of the present disclosure, E3 has a structure of formula E3-1aa: 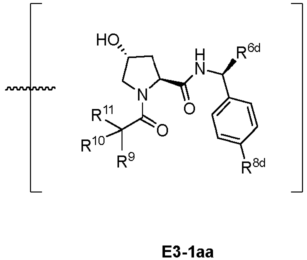 wherein,
R^{6d} is H, -CH₃, -CH₂F, -CH₂OH, ethyl, isopropyl, or cyclopropyl;
R⁹ is H;
R¹⁰ is H, ethyl, isopropyl, *tert*-butyl, *sec*-butyl, cyclopropyl, cyclobutyl, cyclopentyl, or cyclohexyl;
R¹¹ is selected from 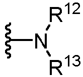 and 5- to 10-membered heteroaryl, wherein the 5- to 10-membered heteroaryl is optionally substituted with 1 or more -OH, halogen, or -NH₂;
R¹² is H or C(O);
R¹³ is H, alkyl, -alkyl CO-, -(cycloalkyl)alkyl CO-, -aralkyl CO-, -aryl CO-,-(heterocycloalkyl)CO-, or arylalkyl, wherein the alkyl, -alkyl CO-, -(cycloalkyl)alkyl CO-, -aralkyl CO-,-aryl CO-, -(heterocycloalkyl)CO-, or arylalkyl is optionally substituted;

R^{8d} is H, halogen, CN, OH, NO₂,

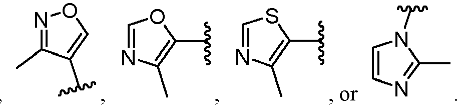

Preferably, in certain embodiments of the present disclosure, E3 is:

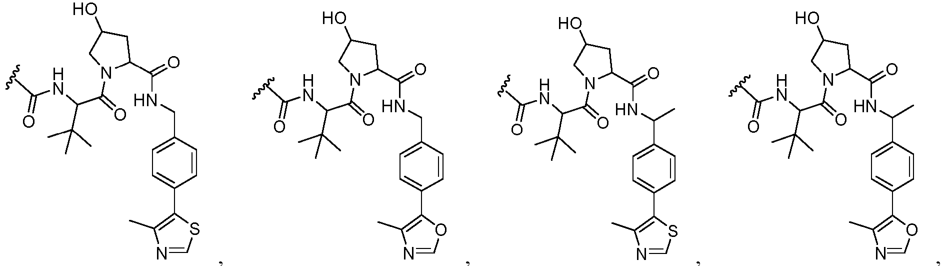

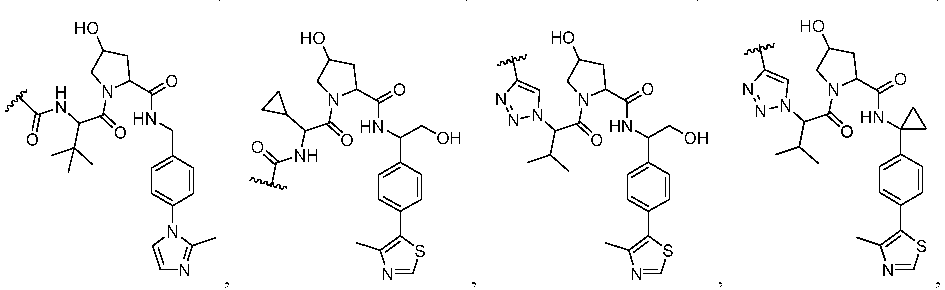

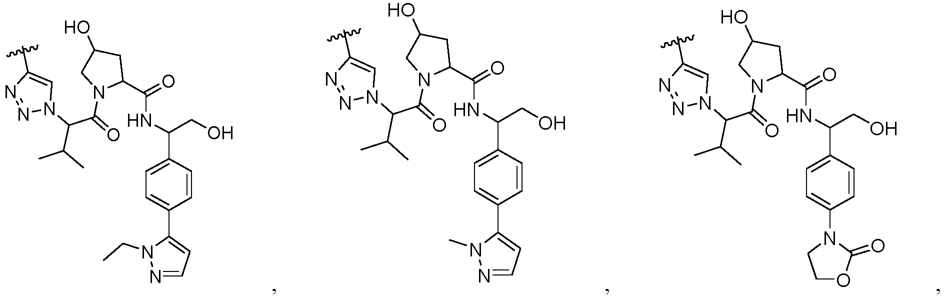

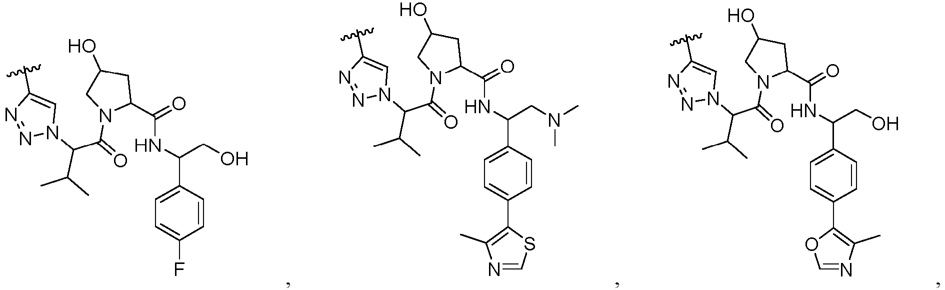

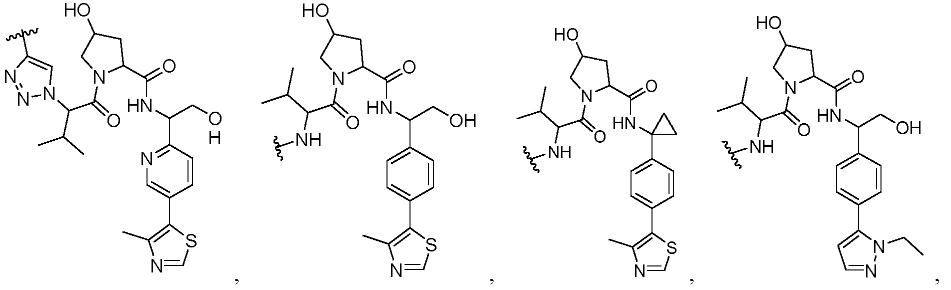

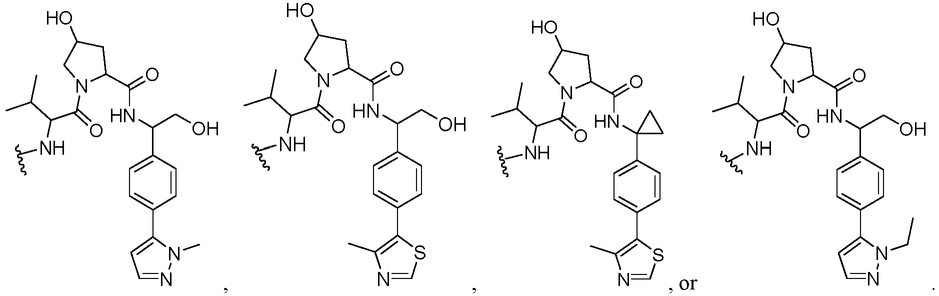

Preferably, in certain embodiments of the present disclosure, E3 is: 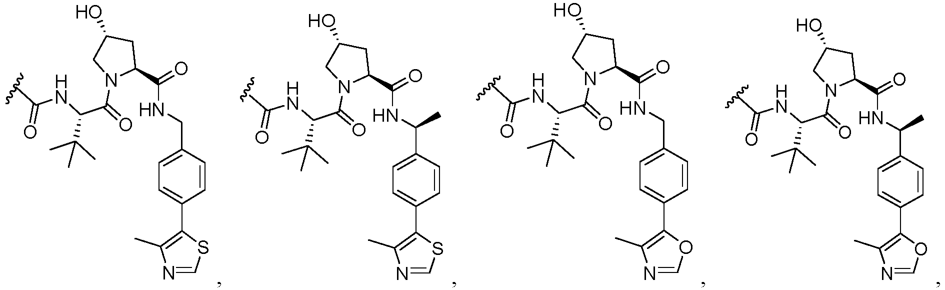 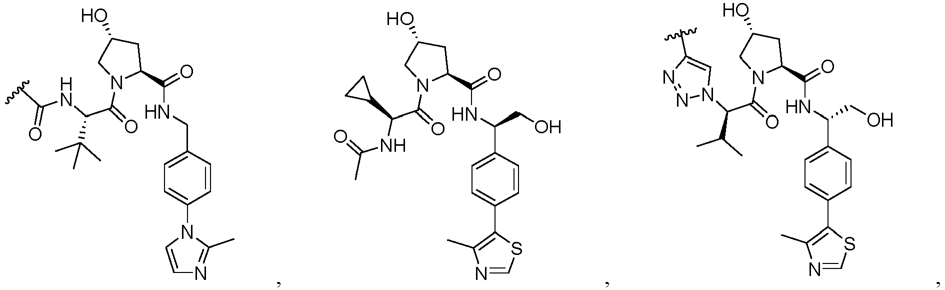 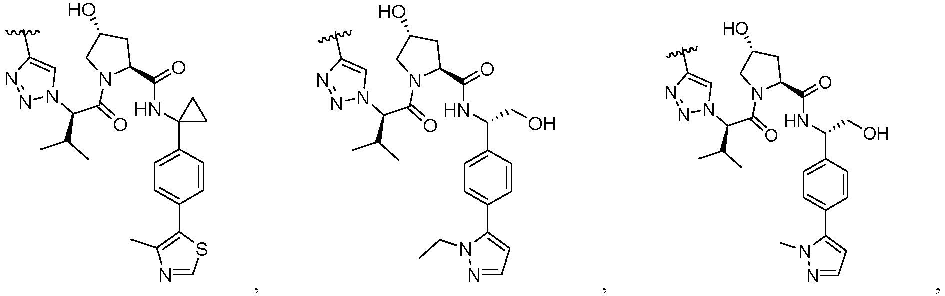 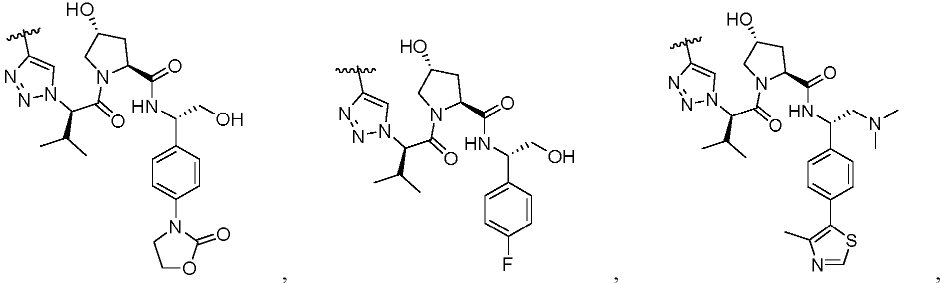 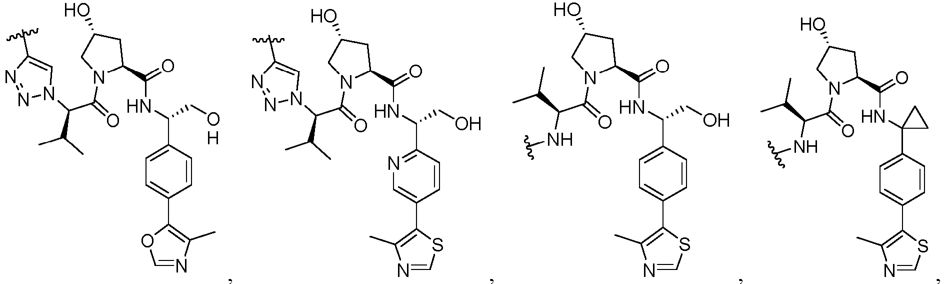 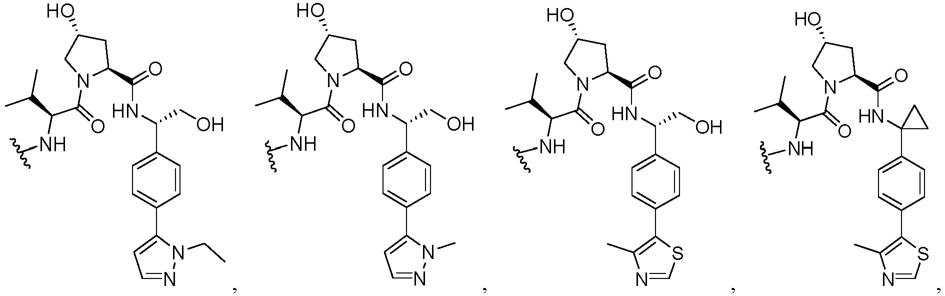 or

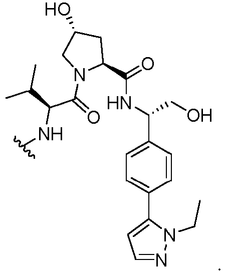

Preferably, in certain embodiments of the present disclosure, K is K1-I-a1 or K' is K2-I-a1; 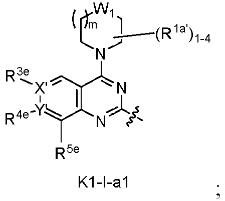 wherein in K1-I-a1, X' is C or N; Y' is C;
R^{3e} is H or absent; R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R^{1a'} is hydrogen, hydroxy, NH₂, halogen, or C₁-C₆ alkyl;
W₁ is CH₂ or O;
m is 0, 1, 2, or 3; 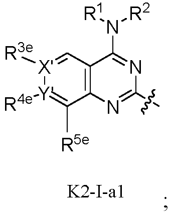
wherein in K2-I-a1, X' is C or N; Y' is C;
R^{3e} is H or absent; R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R¹ and R² are independently hydrogen, C₁-C₆ alkyl, or C₃-C₈ cycloalkyl, wherein the C₁-C₆ alkyl or C₃-C₈ cycloalkyl is optionally substituted with one or more halogen, hydroxy, or amino;
L is LA-9 or LA-10; 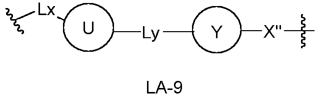
wherein in LA-9:
   Lx is -(CH₂)ᵥ-, wherein one or two CH₂ contained in Lx are each independently optionally replaced by -O- or -CRaRₑ-; -CR_{d}Rₑ- is 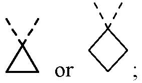 v is 1, 2, 3, 4, 5, or 6;
   ring U is to Ly; 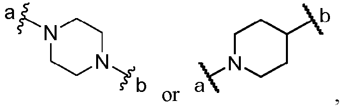 wherein end a is linked to Lx, and end b is linked
   ring Y is 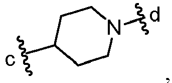 wherein end c is linked to Ly, and end d is linked to X'';
   Ly is -(CH₂)ₖ-, wherein one or two CH₂ contained in Ly are each independently optionally replaced by -O-, -C≡C-, or -N(C₁-C₆ alkyl)-; k is 1, 2, 3, 4, 5, or 6;
   X" is -C(O)-; 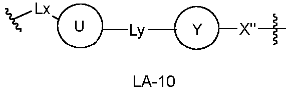
   wherein in LA-10:
      Lx is -(CH₂)ᵥ-, wherein one or two CH₂ contained in Lx are each independently optionally replaced by -O- or -CRaRₑ-; -CR_{d}Rₑ- is 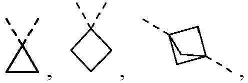 -CH(CH₃)-, -CH(OCH₃)-, -CH(OH)-, or - CH(NH₂)-; v is 1, 2, 3, 4, 5, or 6;
      ring U is 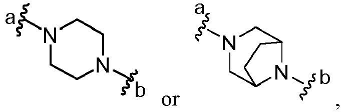 wherein end a is linked to Lx, and end b is linked to Ly;
      ring Y is 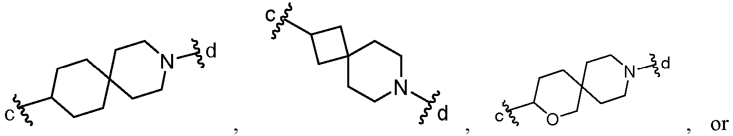 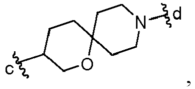 wherein end c is linked to Ly, and end d is linked to X";
      Ly is -(CH₂)ₖ-, and k is 1, 2, 3, 4, 5, or 6;
      X" is -C(O)-;
      E is E1-1h" or E2-1aa; 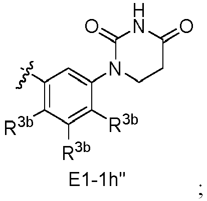
      wherein in E1-1h", each R^{3b} is independently hydrogen, halogen, C₁-C₆ alkyl, or -O(C₁-C₆ alkyl), the C₁-C₆ alkyl and -O(C₁-C₆ alkyl) being optionally substituted with deuterium, halogen, cyano, -OH, or - NH₂, and the number of substitution is 1, 2, or 3; 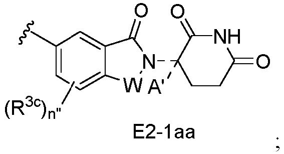
      wherein in E2-1aa,
      W is CH₂ or C(O);
      A' is hydrogen, methyl, Cl, or F;
      each R^{3c} is independently selected from hydrogen, hydroxy, NH₂, C₁-C₆ alkyl, and C₁-C₆ alkoxy;
      n^{"} is 1, 2, 3, or 4;
      ----- is a bond, which is an R stereoisomer, an S stereoisomer, or a non-stereoisomer.

Preferably, in certain embodiments of the present disclosure, K1 is K1-I-a1 or K2 is K2-I-a1; 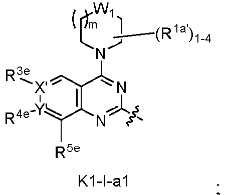
wherein in K1-I-a1, X' is C or N; Y' is C;
R^{3e} is H or absent; R^{4e} is C₆-C₁₀ aryl, wherein the C₆-C₁₀ aryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R^{1a'} is hydrogen, hydroxy, or C₁-C₆ alkyl;
W₁ is CH₂ or O;
m is 1 or 2; 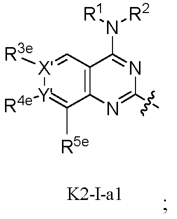
wherein in K2-I-a1, X' is N; Y' is C;
R^{3e} is absent; R^{4e} is C₆-C₁₀ aryl, wherein the C₆-C₁₀ aryl is optionally substituted with 1 or more R^{a'};
R^{a'} is independently selected from halogen, hydroxy, and C₂-C₆ alkynyl;
R^{5e} is halogen;
R¹ and R² are independently hydrogen, C₁-C₆ alkyl, or C₃-C₈ cycloalkyl;
L is LA-11 or LA-12; 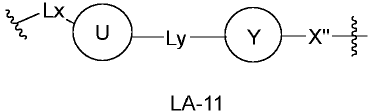
wherein in LA-11:
   Lx is -(CH₂)ᵥ-, wherein one or two CH₂ contained in Lx are each independently optionally replaced by -O- or -CRaRₑ-; -CR_{d}Rₑ- is 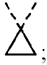 v is 1, 2, 3, 4, 5, or 6;
   ring U is 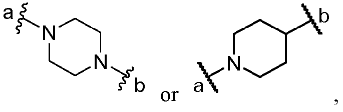 , wherein end a is linked to Lx, and end b is linked to Ly;
   ring Y is 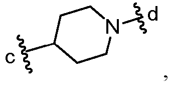 , wherein end c is linked to Ly, and end d is linked to X";
   Ly is -(CH₂)ₖ-, wherein one or two CH₂ contained in Ly are each independently optionally replaced by -O- or -C≡C-; k is 1, 2, 3, 4, 5, or 6;
   X" is -C(O)-; 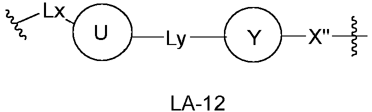
   wherein in LA-12:
      Lx is -(CH₂)ᵥ-, wherein one or two CH₂ contained in Lx are each independently optionally replaced by -O- or -CRaRₑ-; -CR_{d}Rₑ- is 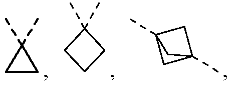 , or -CH(CH₃)-; v is 1, 2, 3, 4, 5, or 6;
      ring U is 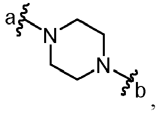 wherein end a is linked to Lx, and end b is linked to Ly;
      ring Y is 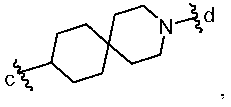 wherein end c is linked to Ly, and end d is linked to X";
      Ly is -(CH₂)ₖ-, and k is 1, 2, 3, 4, 5, or 6;
      X" is -C(O)-;
      E is E1-1h"; 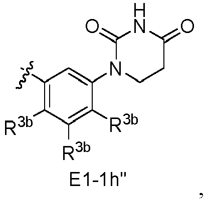
      wherein each R^{3b} is independently hydrogen, halogen, C₁-C₆ alkyl, or -O(C₁-C₆ alkyl), the C₁-C₆ alkyl and -O(C₁-C₆ alkyl) being optionally substituted with deuterium, halogen, cyano, -OH, or -NH₂, and the number of substitution is 1, 2, or 3.

Preferably, in certain embodiments of the present disclosure, the compound of formula I or I' is:

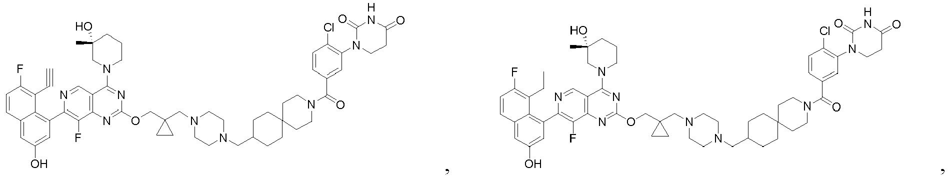

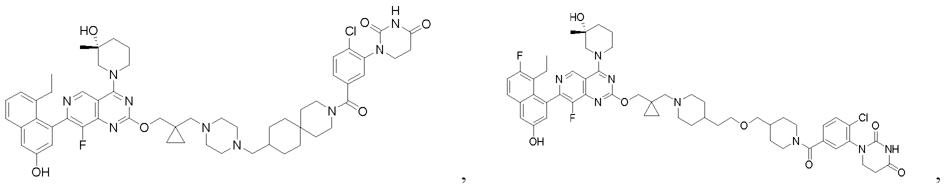

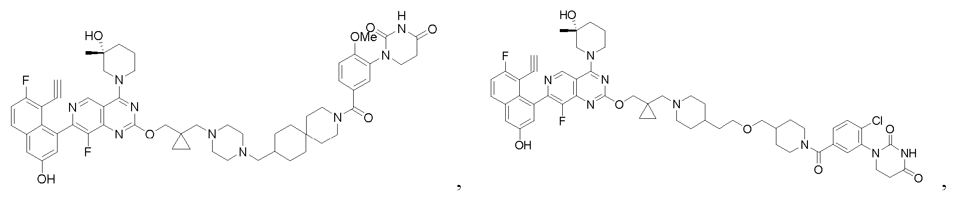

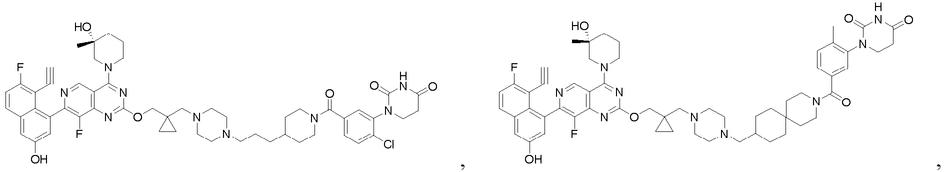

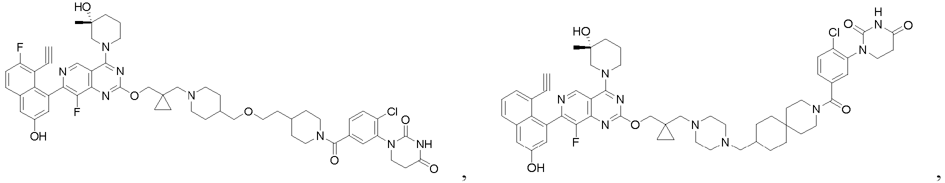

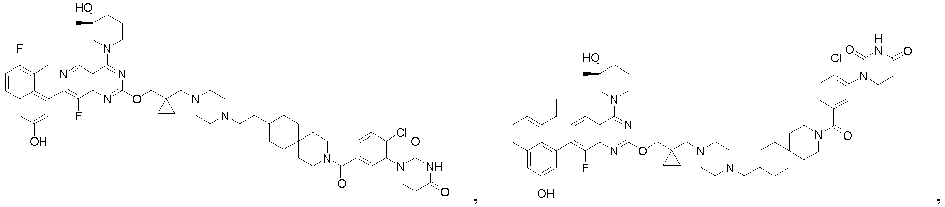

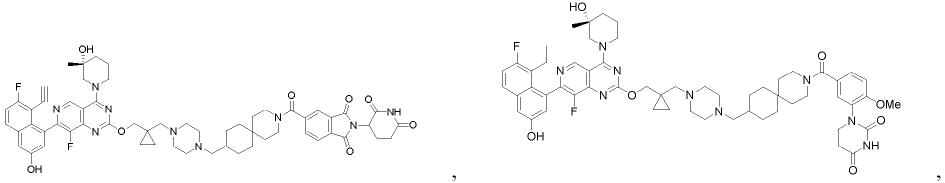

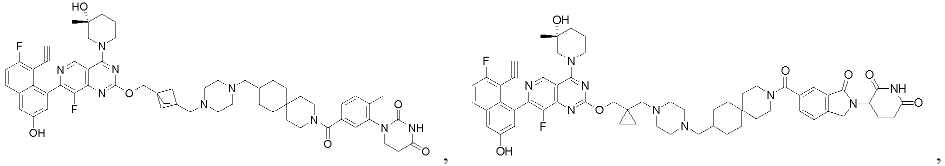

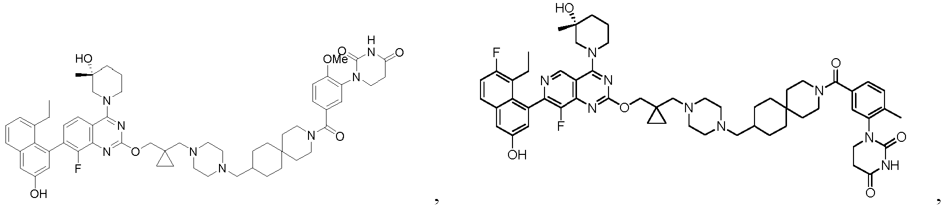

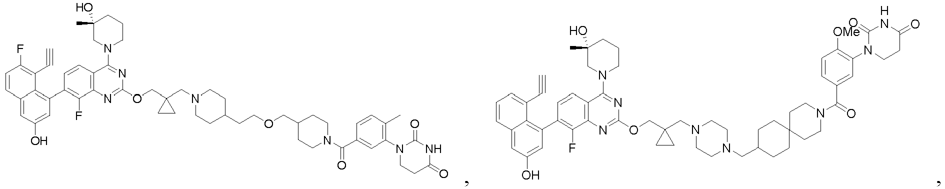

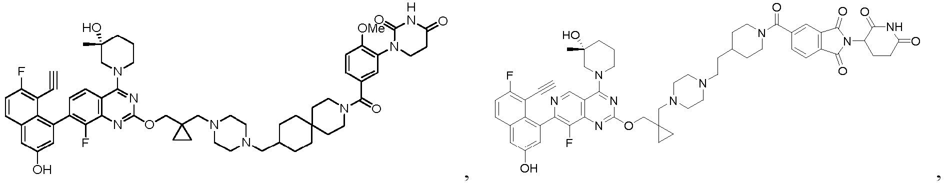

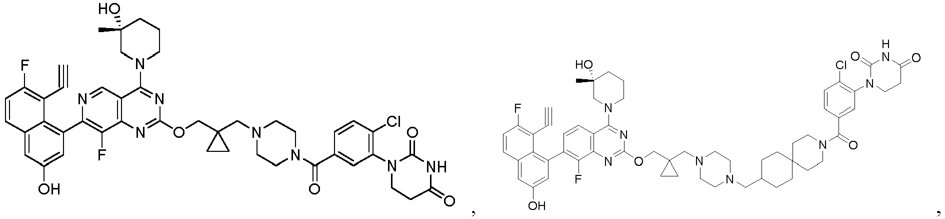

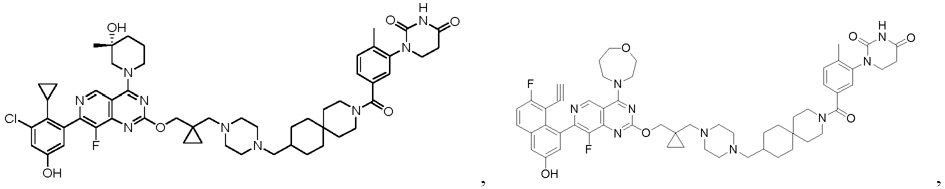

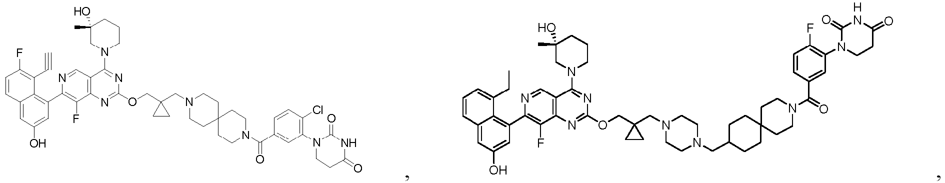

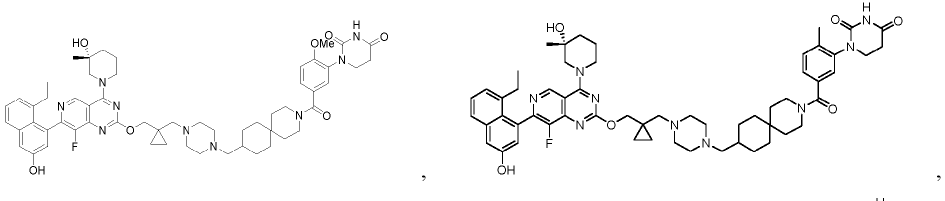

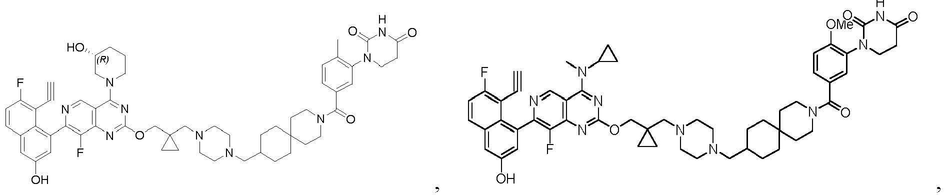

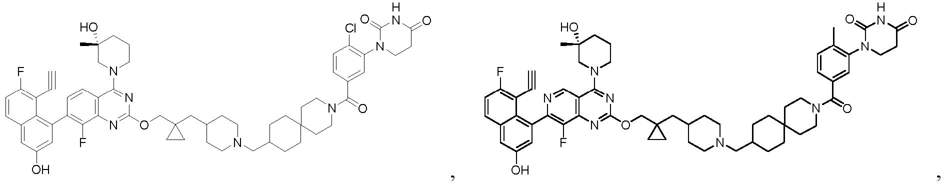

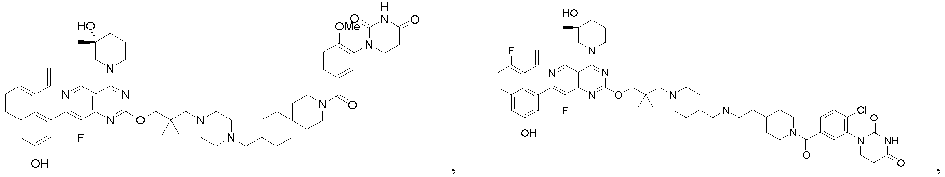

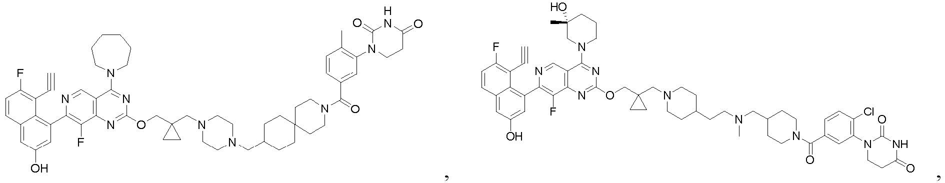

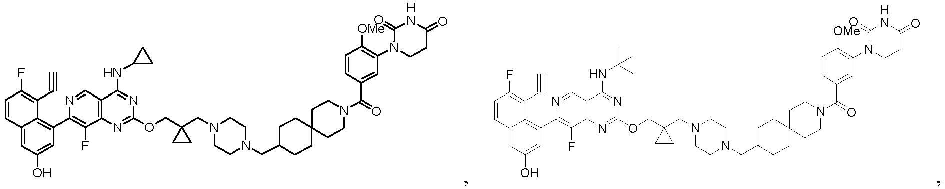

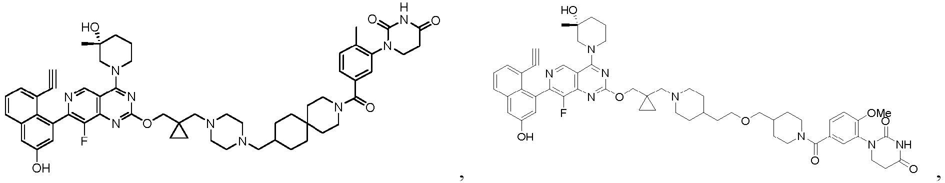

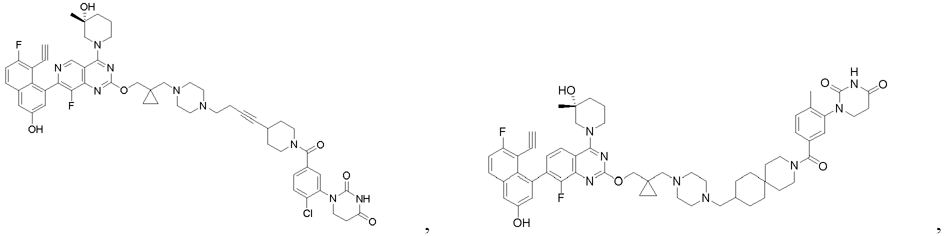

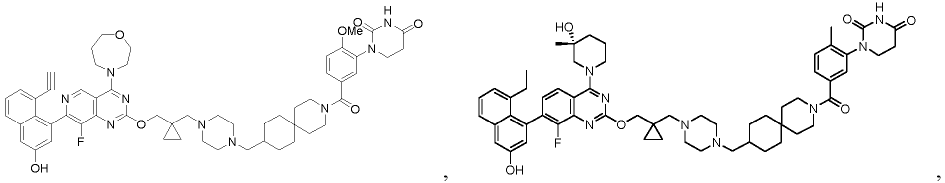

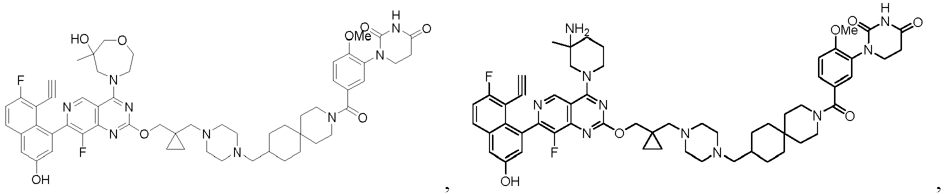

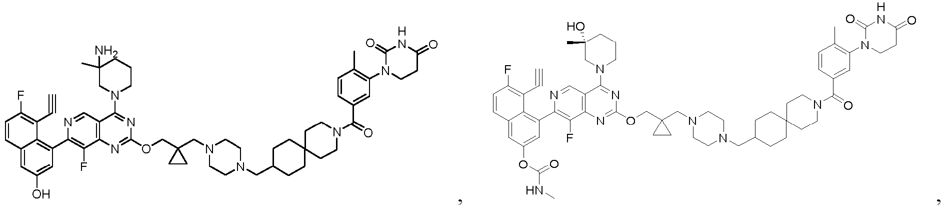

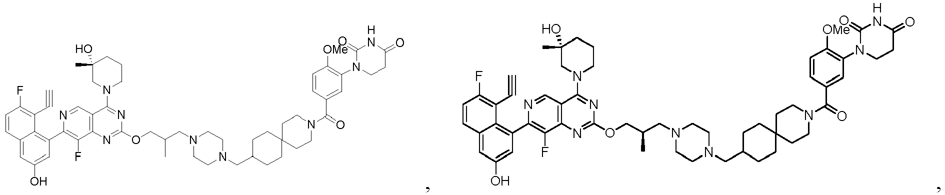

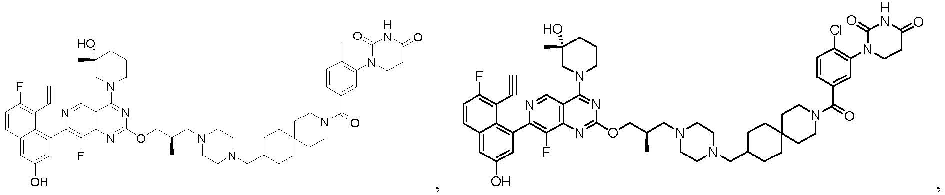

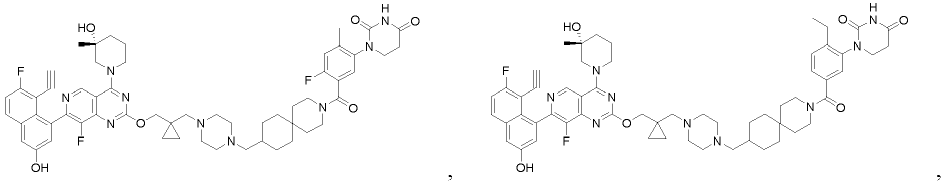

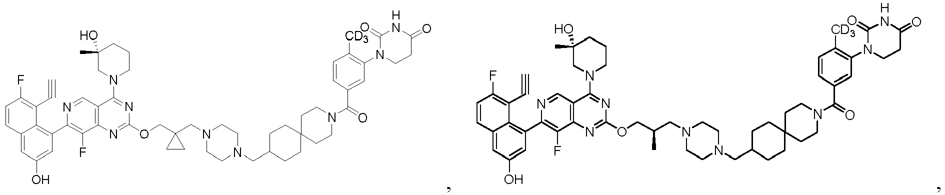

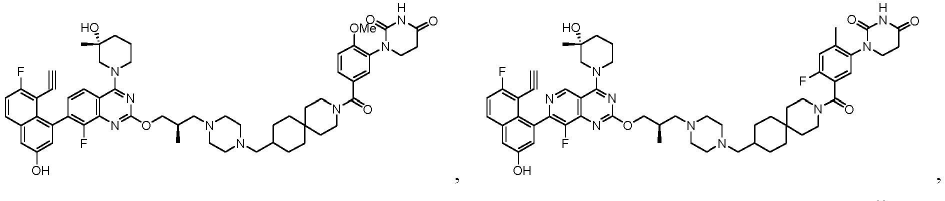

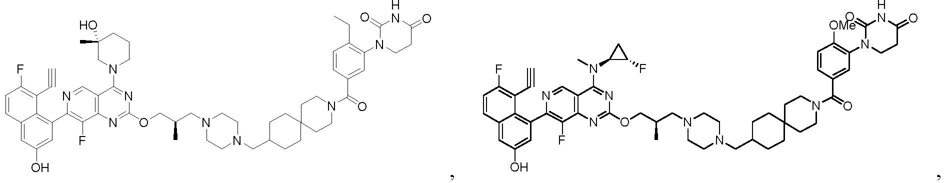

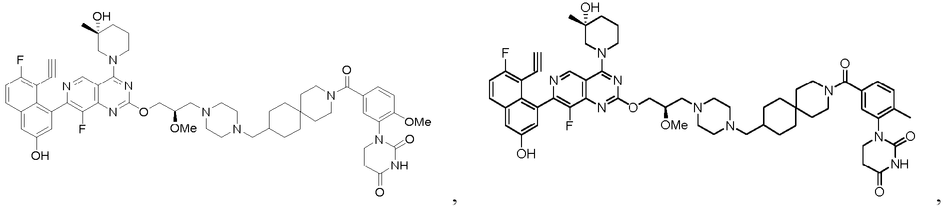

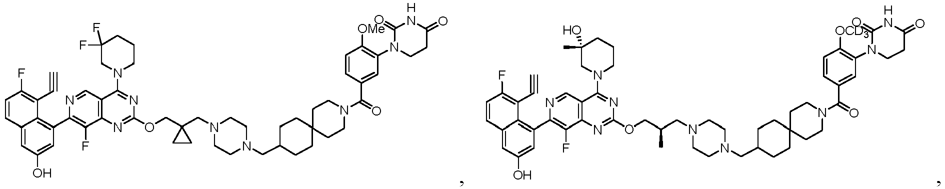

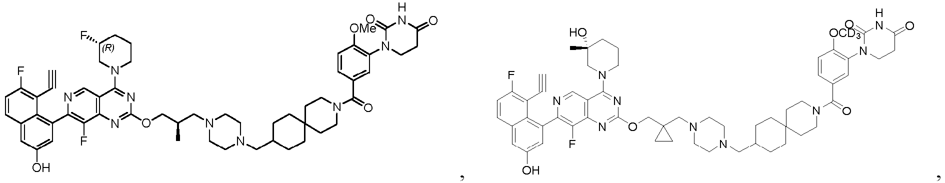

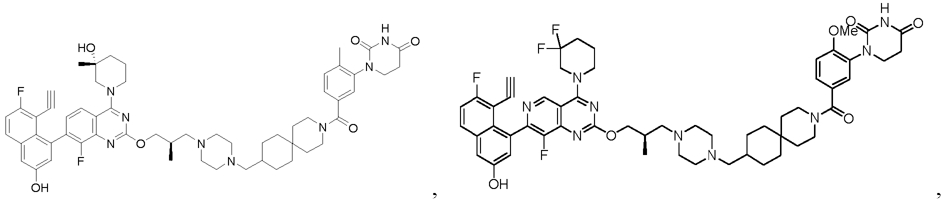

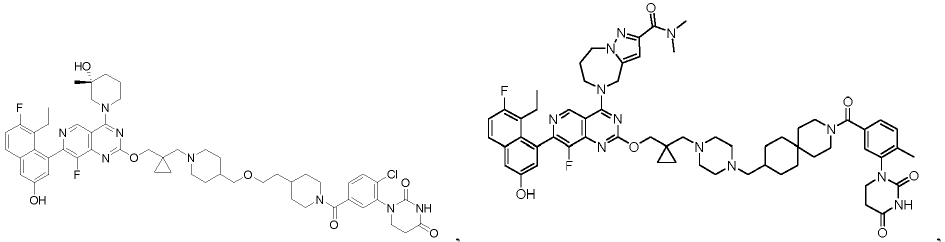

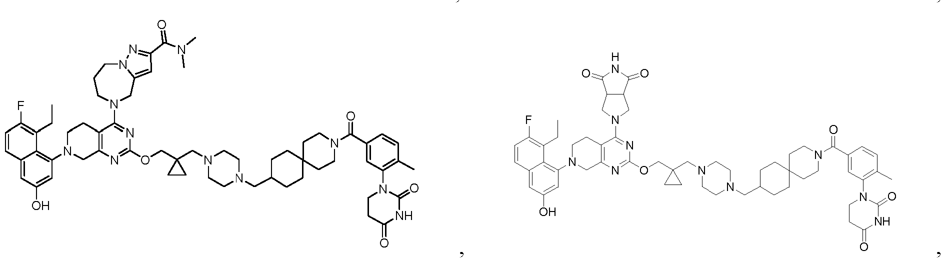

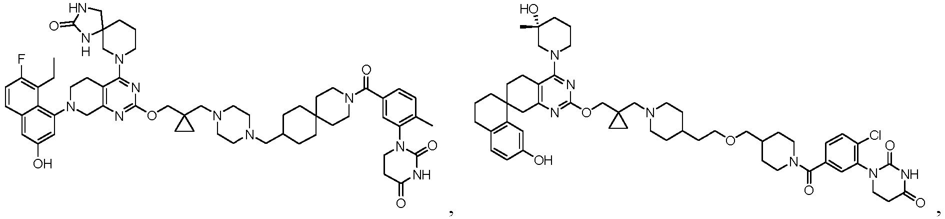

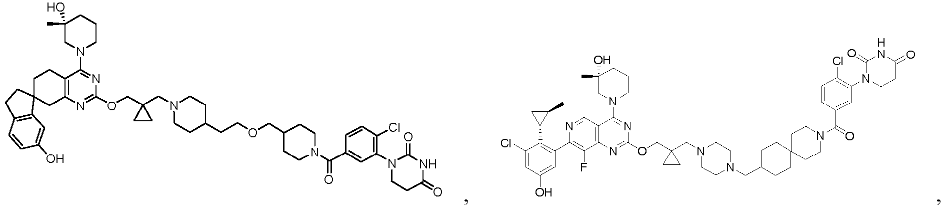

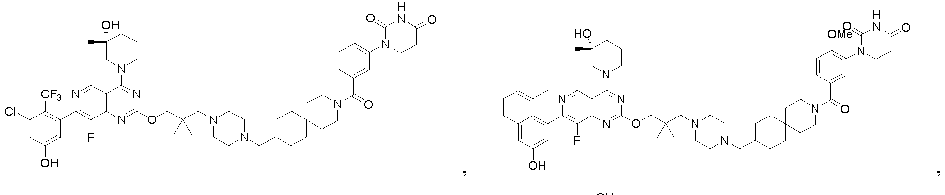

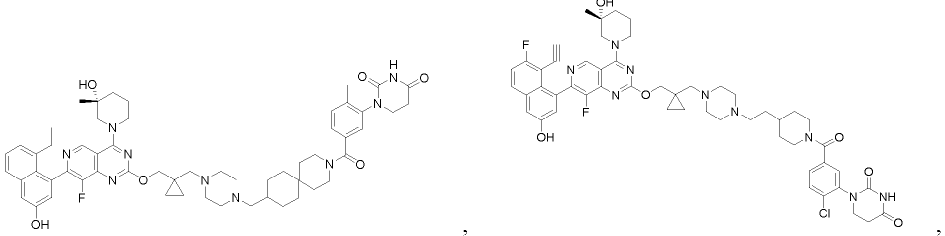

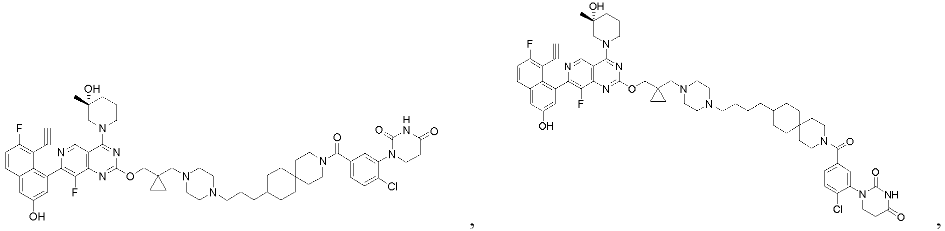

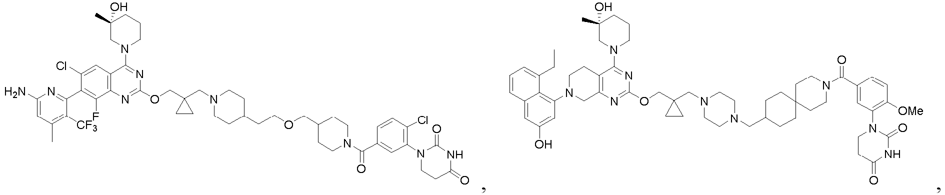

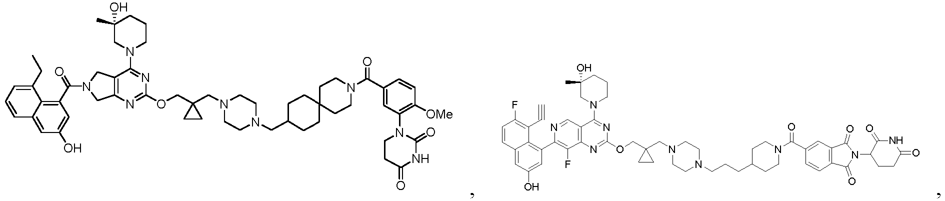

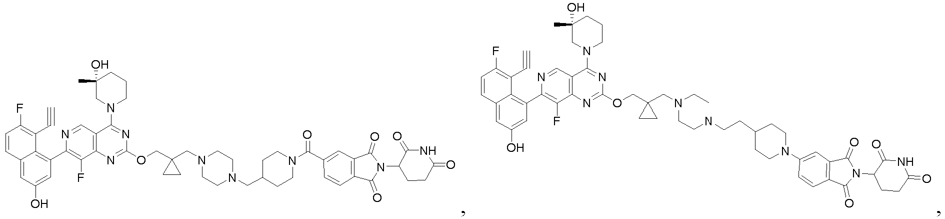

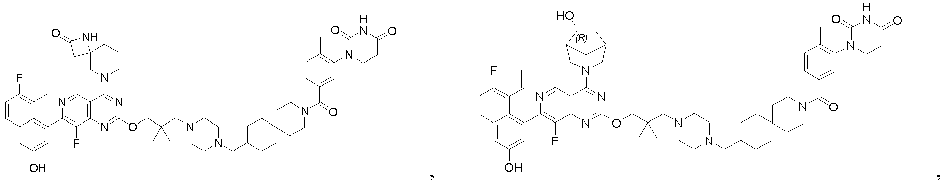

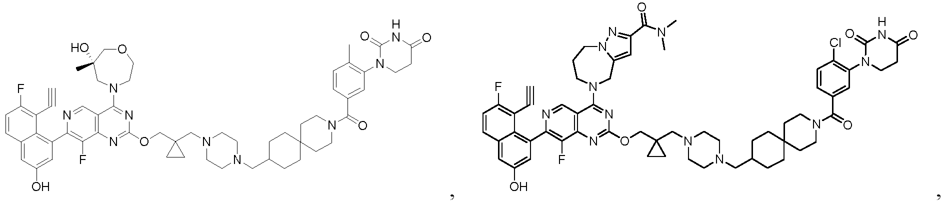

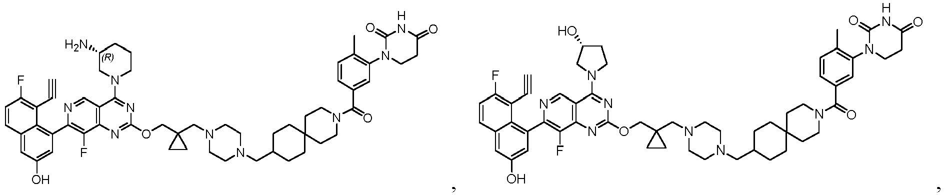

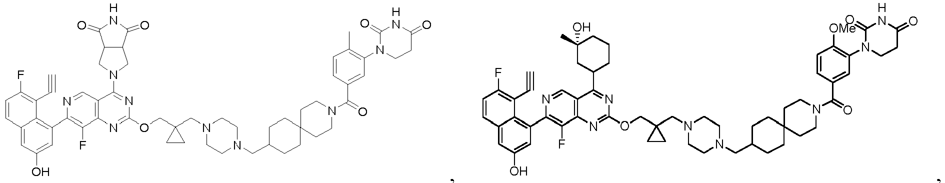

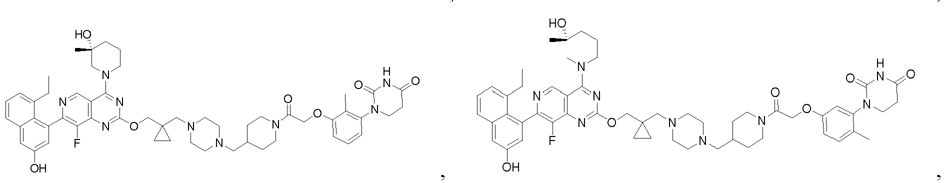

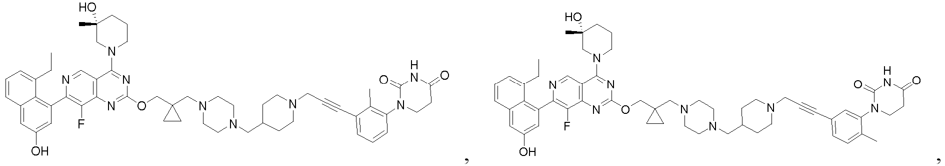

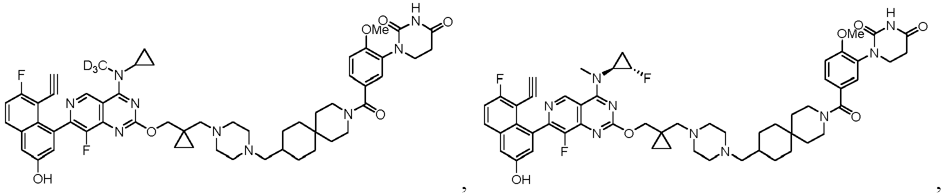

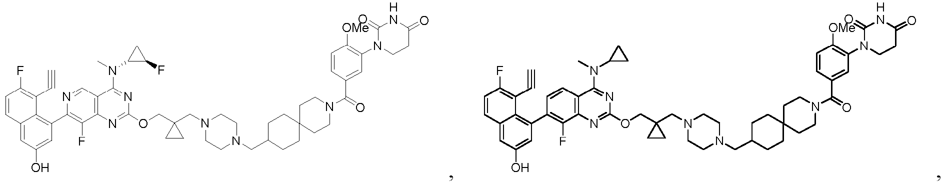

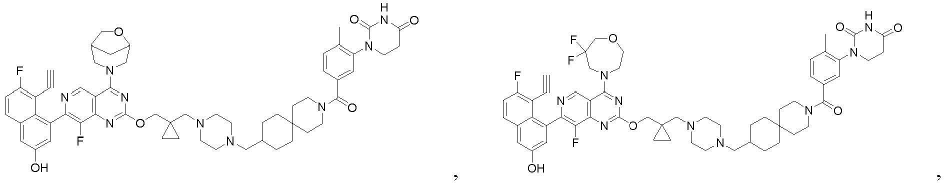

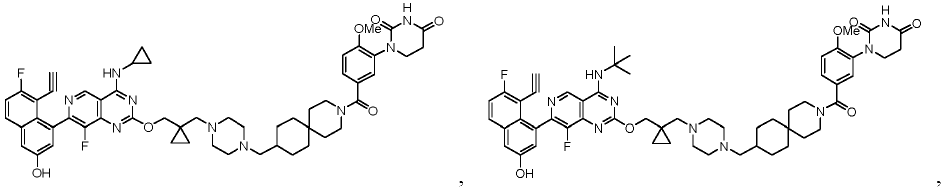

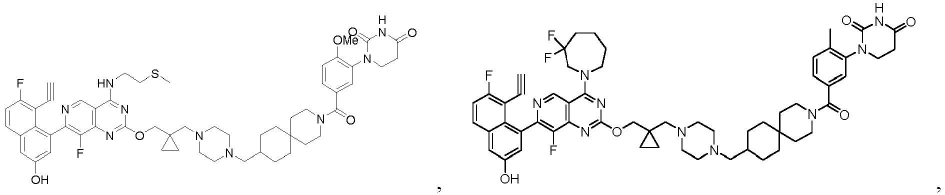

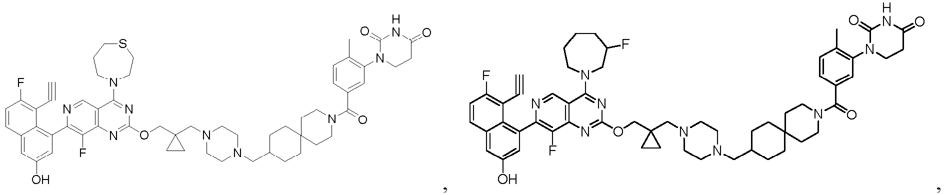

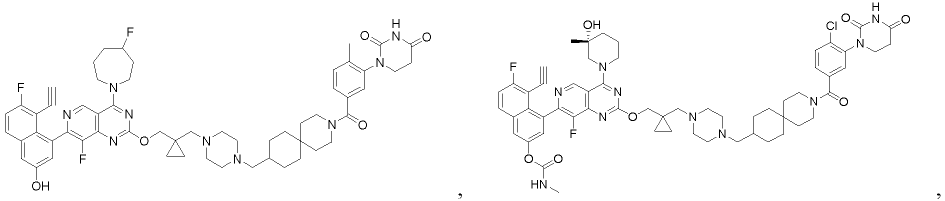

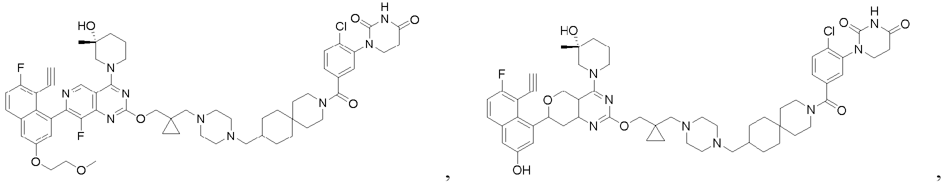

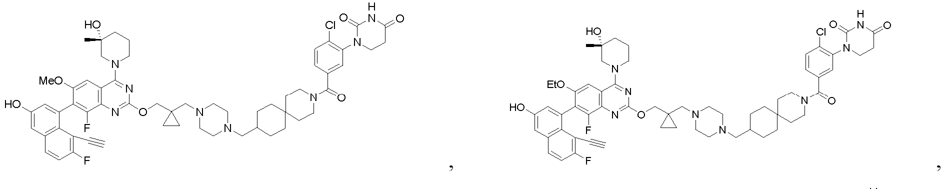

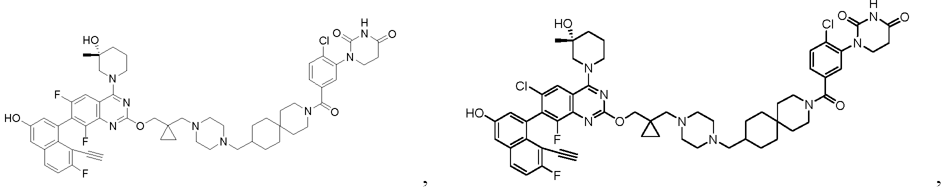

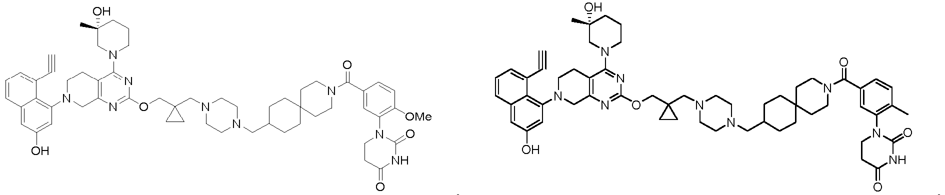

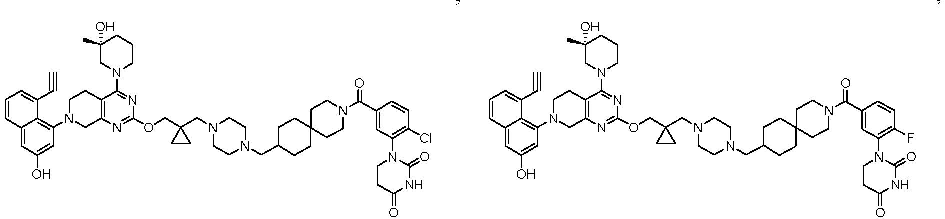

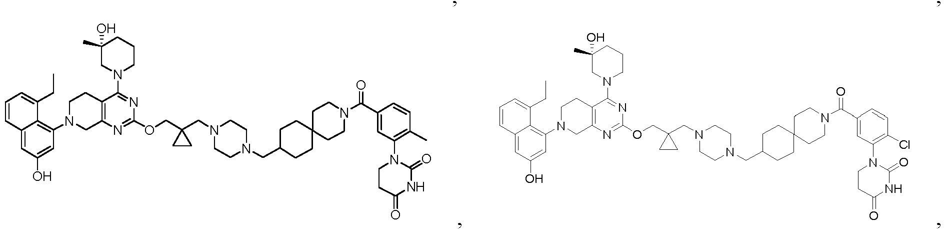

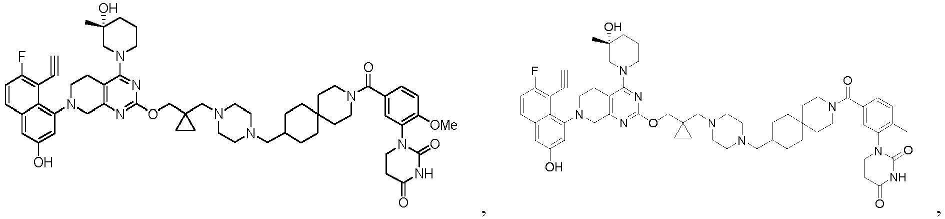

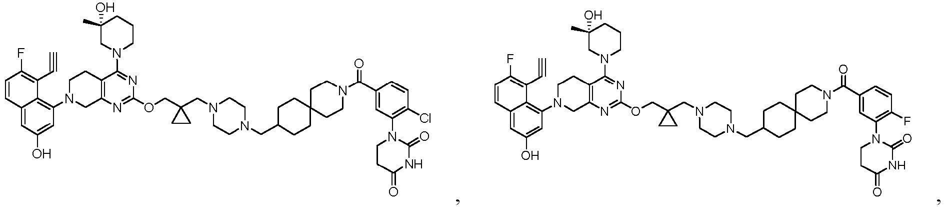

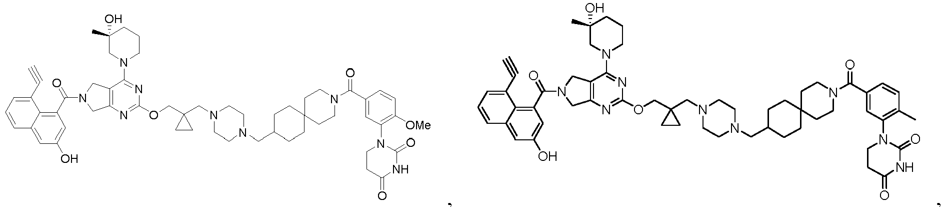

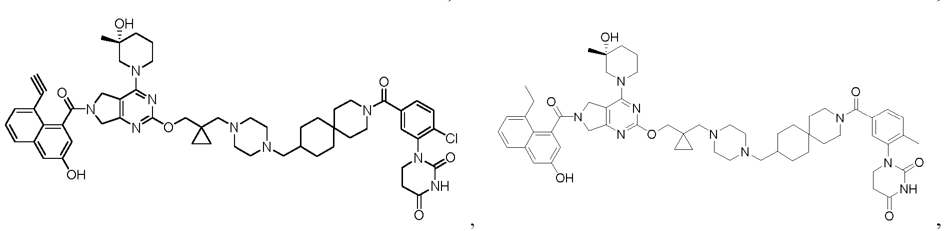

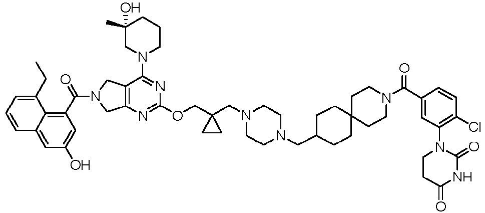

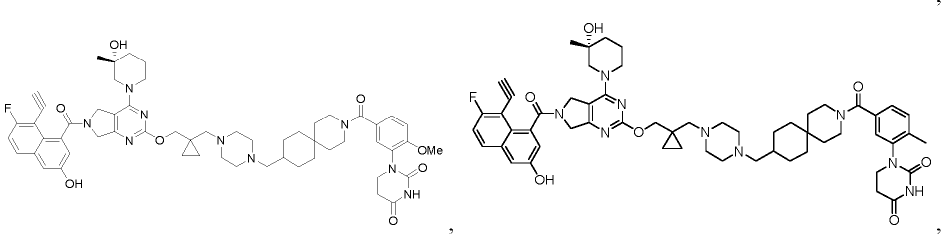

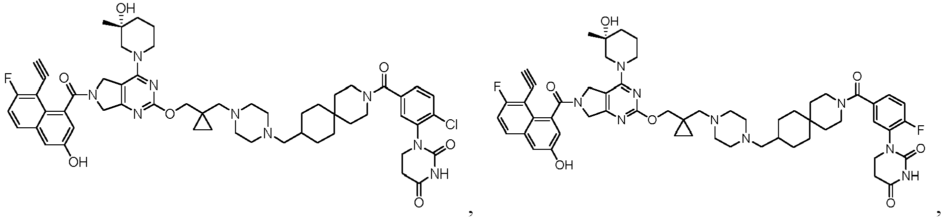

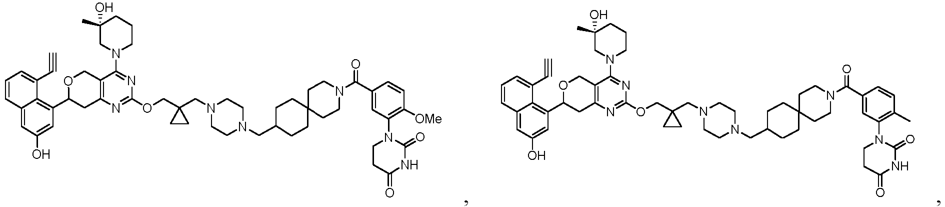

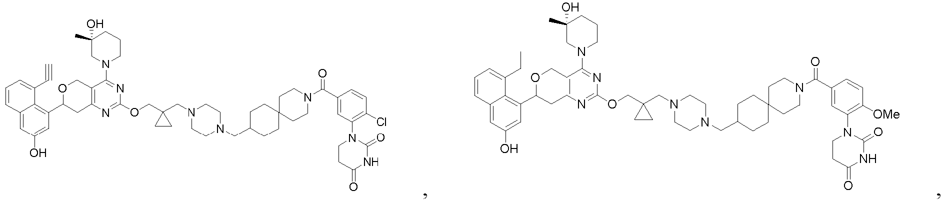

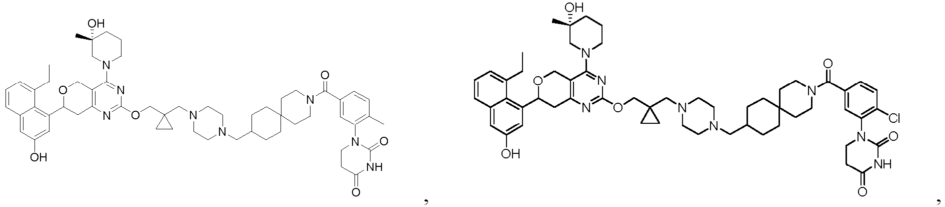

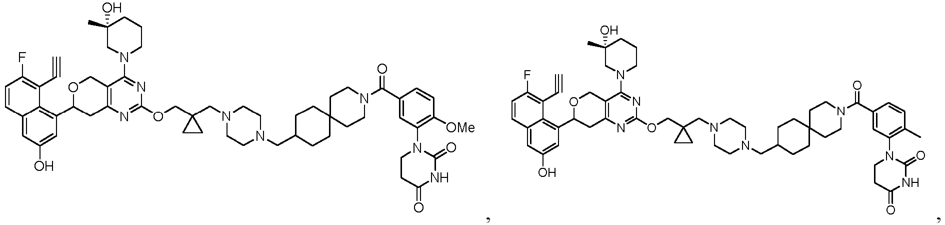

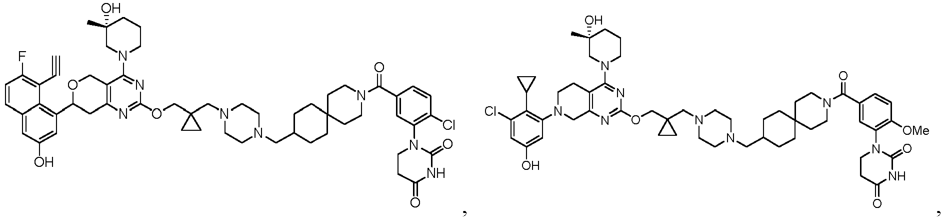

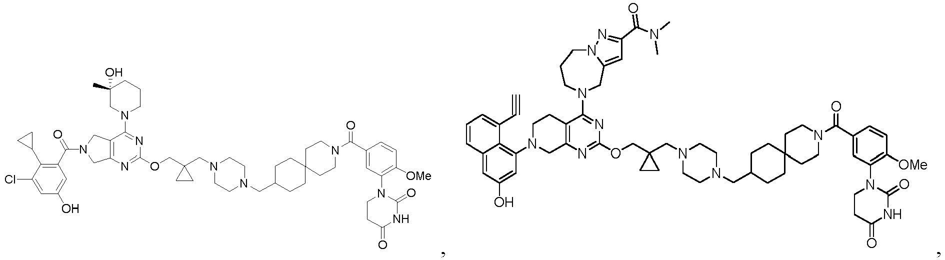

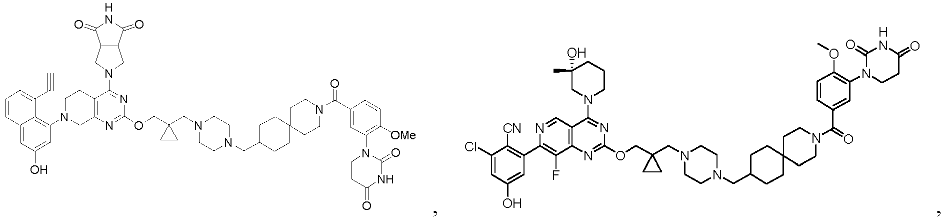

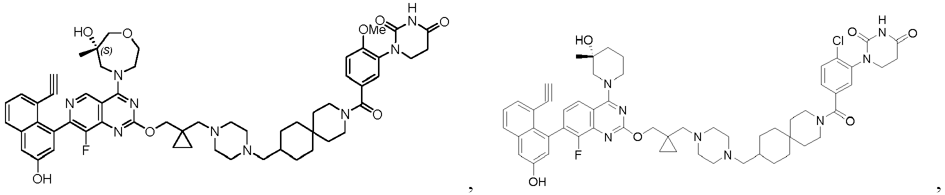

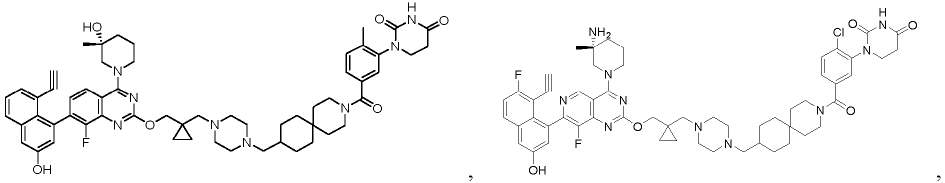

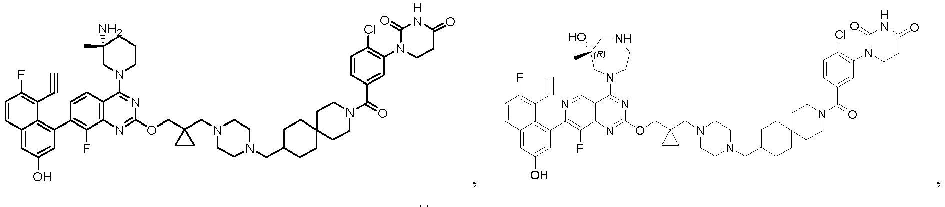

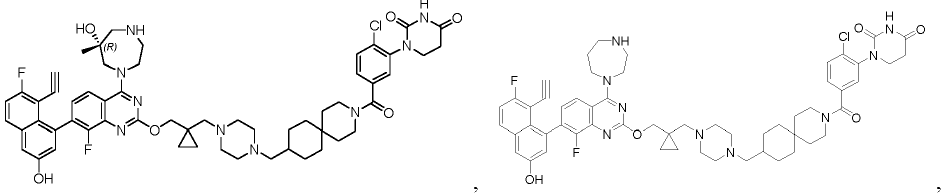

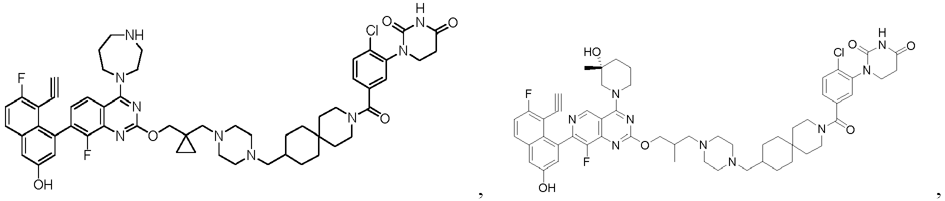

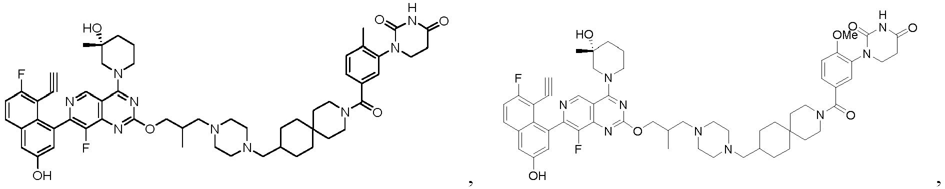

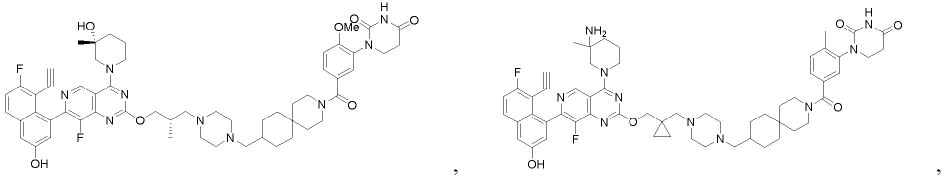

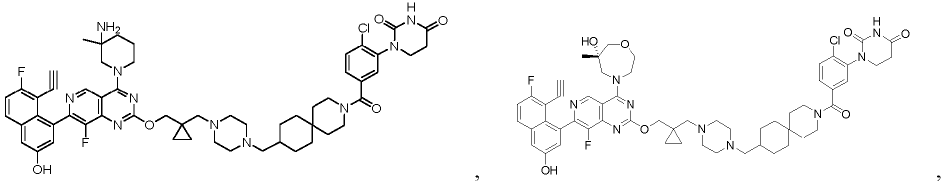

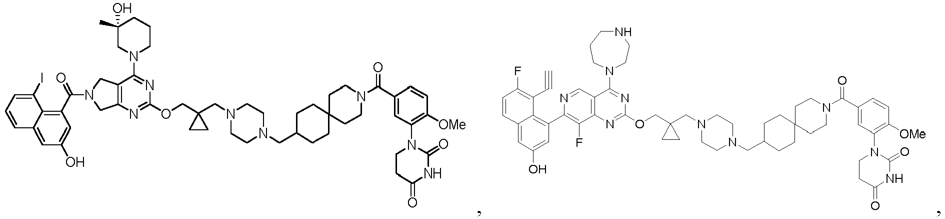

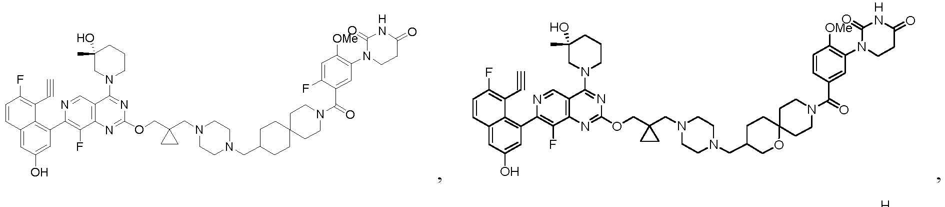

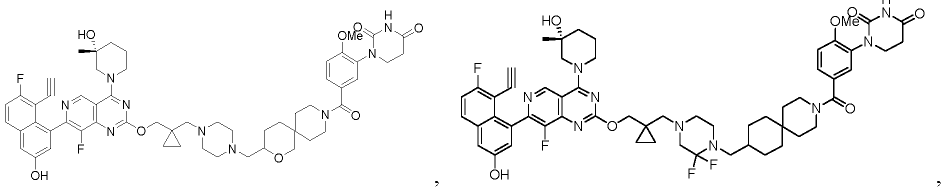

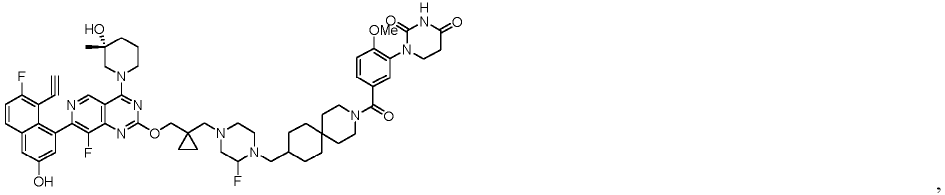

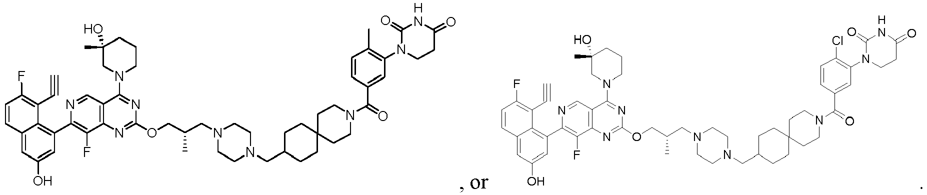

The present disclosure further provides a compound of formula S-1, S-2, S-3, or S-4: 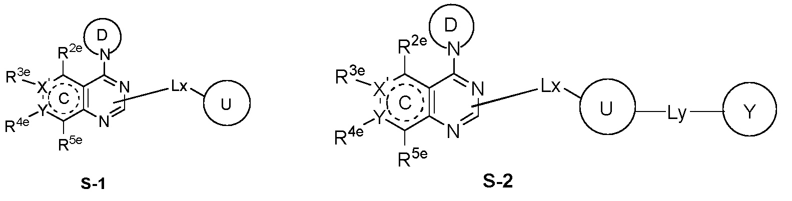 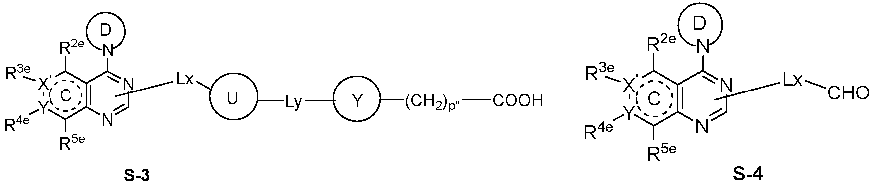 wherein ring C, ring D, R^{2e}, R^{3e}, R^{4e}, and R^{5e} are as defined and described in the present disclosure; Lx, ring U, Ly, and ring Y, as well as the linking relationships among them are as defined and described in the present disclosure; p" is 1, 2, 3, or 4.

Preferably, formula S-1 is formula S-1-1, formula S-2 is formula S-2-1, formula S-3 is formula S-3-1, and formula S-4 is formula S-4-1: 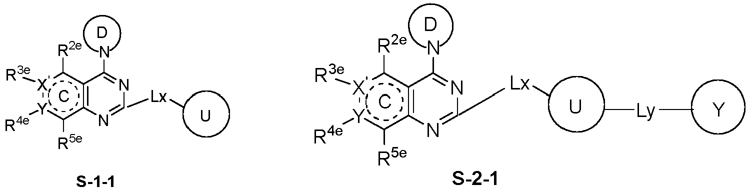 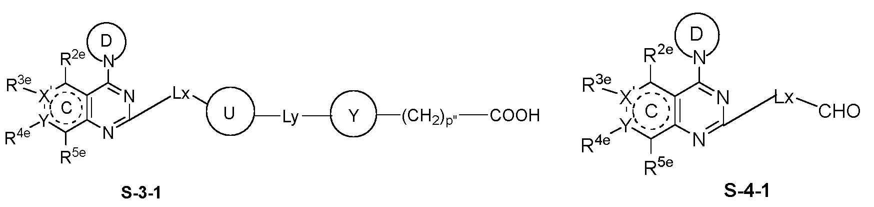 wherein ring C, ring D, R^{2e}, R^{3e}, R^{4e}, R^{5e}, Lx, ring U, Ly, ring Y, and p" are as defined and described in the present disclosure.

Preferably, the compound of formula S-1, S-2, S-3, or S-4 is selected from:

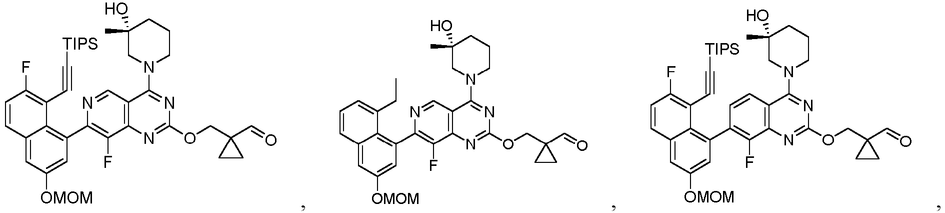

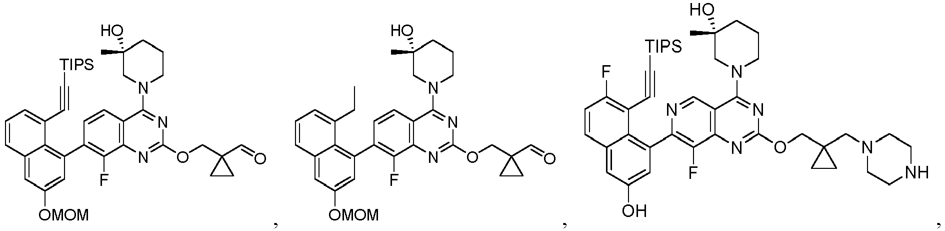

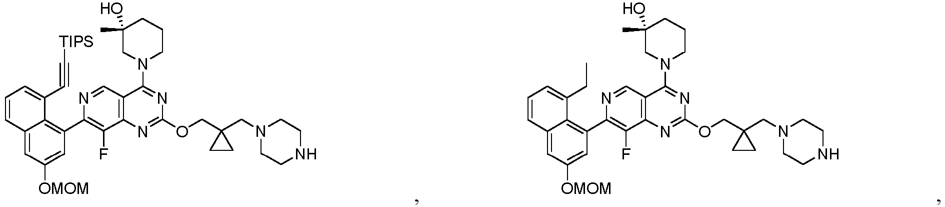

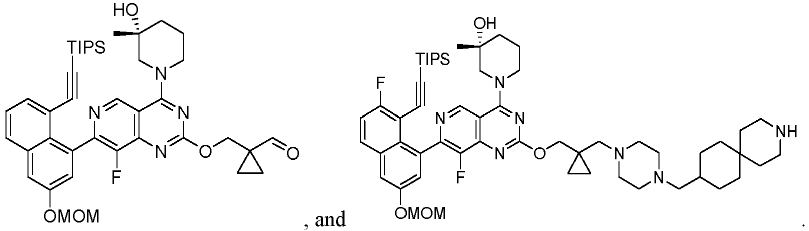

The present disclosure provides a method for preparing the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof.

The present disclosure provides a pharmaceutical composition comprising a therapeutically effective amount of the compound of formula I or I', and/or the stereoisomer, the enantiomer, the diastereomer, the atropisomer, the deuteride, the hydrate, the solvate or the prodrug thereof and/or the pharmaceutically acceptable salt thereof, and a pharmaceutically acceptable carrier, diluent, or excipient.

The present disclosure provides a method for degrading pan-KRAS protein, comprising contacting the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof with the pan-KRAS protein.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof as a medicament for the treatment or prevention of a pan-KRAS-mediated disease or disorder.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof as a medicament for the treatment or prevention of a disease or disorder (cancer) caused by pan-KRAS.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof in preparing a medicament for the treatment or prevention of a disease or disorder (e.g., cancer) mediated by pan-KRAS.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof in preparing a medicament for the treatment or prevention of cancer.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof in preparing a medicament for the treatment or prevention of pancreatic cancer, lung cancer, colorectal cancer, cholangiocarcinoma, multiple myeloma, melanoma, uterine cancer, endometrial cancer, thyroid cancer, acute myeloid leukemia, bladder cancer, urothelial cancer, gastric cancer, cervical cancer, head and neck squamous cell carcinoma, diffuse large B-cell lymphoma, esophageal cancer, chronic lymphocytic leukemia, hepatocellular carcinoma, breast cancer, ovarian cancer, prostate cancer, glioblastoma, renal cancer, and sarcoma.

The present disclosure provides a method for treating or preventing a pan-KRAS-mediated disease or disorder, comprising administering to a patient in need thereof a therapeutically effective amount of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof.

The present disclosure provides a method for treating or preventing a disease or disorder (e.g., cancer) regulated by pan-KRAS, comprising administering to a patient in need thereof a therapeutically effective amount of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof.

In certain embodiments of the present disclosure, the cancer is selected from:
heart: sarcoma (angiosarcoma, fibrosarcoma, rhabdomyosarcoma, and liposarcoma), myxoma, rhabdomyoma, fibroma, lipoma, and teratoma;
lung: bronchial cancer (squamous cell carcinoma, undifferentiated small cell carcinoma, undifferentiated large cell carcinoma, and adenocarcinoma), bronchioloalveolar carcinoma (bronchiolar carcinoma), bronchial adenoma, sarcoma, lymphoma, chondroma, hamartoma, mesothelioma, lung cancer, and small cell lung cancer;
gastrointestinal tract: esophagus cancer (squamous cell carcinoma, adenocarcinoma, leiomyosarcoma, and lymphoma), stomach (cancer, lymphoma, and leiomyosarcoma), pancreas (ductal adenocarcinoma, insulinoma, glucagonoma, gastrinoma, carcinoid tumor, and vipoma), small intestine (adenocarcinoma, lymphoma, carcinoid tumor, Kaposi's sarcoma, leiomyoma, hemangioma, lipoma, neurofibroma, and fibroma), large intestine (adenocarcinoma, tubular adenoma, villous adenoma, hamartoma, and leiomyoma), appendiceal cancer, gastrointestinal neuroendocrine tumor, esophageal gastric cancer, anal cancer, and gastrointestinal stromal tumor;
genitourinary system: kidney (adenocarcinoma, embryonal carcinosarcoma (Wilms' tumor), lymphoma, and leukemia), bladder and urinary tract (squamous cell carcinoma, transitional cell carcinoma, and adenocarcinoma), prostate (adenocarcinoma and sarcoma), testis (seminoma, teratoma, embryonal carcinoma, teratoma, choriocarcinoma, sarcoma, interstitial cell carcinoma, fibroma, fibroadenoma, adenomatoid tumor, and lipoma), germinal cell tumor, bladder cancer, and prostate cancer;
liver: liver cancer (hepatocellular carcinoma), intrahepatic cholangiocarcinoma, hepatoblastoma, malignant hemangioendothelioma, hepatocellular adenoma, and hemangioma;
biliary tract: cholangiocarcinoma, gallbladder cancer, ampullary cancer, and intrahepatic cholangiocarcinoma;
bone: osteogenic sarcoma (osteosarcoma), fibrosarcoma, malignant fibrous histiocytoma, chondrosarcoma, Ewing's sarcoma, malignant lymphoma (reticulosarcoma), multiple myeloma, malignant giant cell tumor, chordoma, chondroma (osteochondral exostosis), benign chondroma, chondroblastoma, chondromyxoid fibroma, osteoid osteoma, giant cell tumor, and bone cancer;
nervous system: skull (osteoma, hemangioma, granuloma, xanthoma, and osteitis deformans), meninges (meningioma, meningosarcoma, and glioma), brain (astrocytoma, medulloblastoma, glioma, ependymoma, germinal cell tumor (pinealoma), glioblastoma multiforme, oligodendroglioma, neurilemmoma, eye cancer, and congenital tumor), spinal neurofibroma, meningioma, glioma, and sarcoma);
gynaecology and obstetrics: uterus (endometrial cancer (serous cystadenocarcinoma, mucinous cystadenocarcinoma, and unclassified cancer), granulosa theca cell tumor, ovarian Sertoli-Leydig cell tumor, dysgerminoma, and malignant teratoma), vulva (squamous cell carcinoma, intraepithelial carcinoma, adenocarcinoma, fibrosarcoma, and melanoma), vagina (clear cell carcinoma, squamous cell carcinoma, botulinum sarcoma (embryonal rhabdomyosarcoma), fallopian tube (cancer), cervical cancer, and endometrioid carcinoma;
hematology: blood (myeloid leukemia (acute and chronic), acute lymphoblastic leukemia, chronic lymphocytic leukemia, myeloproliferative disease, multiple myeloma, and myelodysplastic syndrome), Hodgkin's disease, and non-Hodgkin's lymphoma (malignant lymphoma);
skin: malignant melanoma, basal cell carcinoma, squamous cell carcinoma, Kaposi's sarcoma, dysplastic nevus, lipoma, hemangioma, dermatofibroma, keloid, and psoriasis; and
adrenal gland: neuroblastoma.

In certain embodiments of the present disclosure, the cancer is a cancer associated with KRAS G12A, KRAS G12C, KRAS G12D, KRAS G12R, KRAS G12S, KRAS G12V, KRAS G13D, or KRAS Q61H protein mutation.

In certain embodiments of the present disclosure, the cancer is a KRAS G12A-associated cancer: non-small cell lung cancer, ovarian cancer, or colorectal cancer.

In certain embodiments of the present disclosure, the cancer is a KRAS G12C-associated cancer: non-small cell lung cancer, colorectal cancer, or pancreatic cancer.

In certain embodiments of the present disclosure, the cancer is a KRAS G12D-associated cancer: biliary tract cancer, endometrial cancer, pancreatic cancer, colorectal cancer, non-small cell lung cancer, ovarian cancer, or rectal cancer.

In certain embodiments of the present disclosure, the cancer is a KRAS G12R-associated cancer: pancreatic cancer or non-small cell lung cancer.

In certain embodiments of the present disclosure, the cancer is a KRAS G12S-associated cancer: rectal cancer, intestine adenocarcinoma, or colorectal cancer.

In certain embodiments of the present disclosure, the cancer is a KRAS G12V-associated cancer: pancreatic cancer, colorectal cancer, non-small cell lung cancer, or ovarian cancer.

In certain embodiments of the present disclosure, the cancer is a KRAS G13D-associated cancer: colorectal cancer.

In certain embodiments of the present disclosure, the cancer is non-small cell lung cancer, small cell lung cancer, colorectal cancer, rectal cancer, or pancreatic cancer.

The present disclosure further provides the compound of formula I or I' or the stereoisomer thereof, the atropisomer thereof, the pharmaceutically acceptable salt thereof, the pharmaceutically acceptable salt of the stereoisomer thereof, or the pharmaceutically acceptable salt of the atropisomer thereof described above for use in the treatment of a disease or disorder associated with pan-KRAS mutant protein.

The present disclosure further provides the compound of formula I or I', wherein the disease or disorder associated with the pan-KRAS mutant protein is a cancer associated with pan-KRAS protein mutation.

In certain embodiments of the present disclosure, the cancer is selected from pancreatic cancer, colorectal cancer, endometrial cancer, and lung cancer.

In certain embodiments of the present disclosure, the lung cancer is selected from non-small cell lung cancer and small cell lung cancer.

The present disclosure provides a method for preparing the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof:
when K is K1-I-a 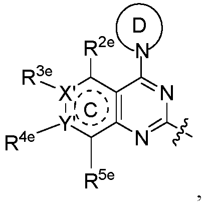 L is LA 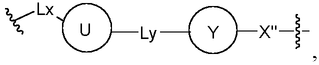 and ring U contains an NH group, the preparation method is: 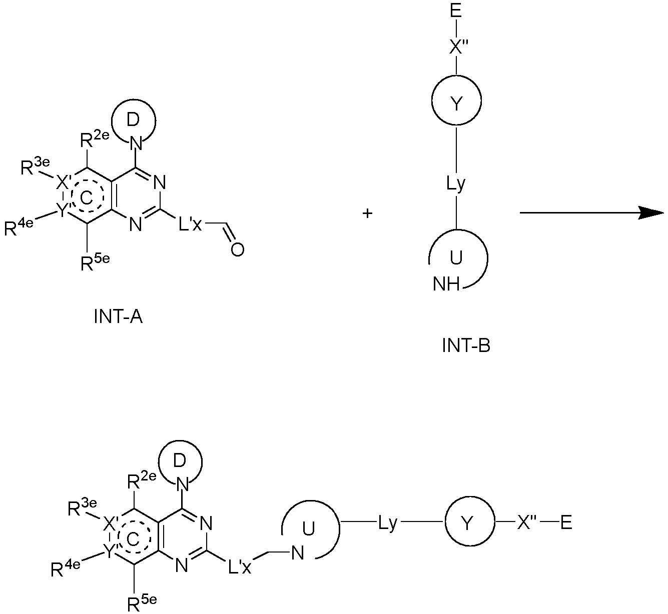
subjecting INT-A and INT-B to a reductive amination reaction to give the target compound, wherein the reductant for the reductive amination includes, but is not limited to, Pd/C, sodium borohydride, sodium cyanoborohydride, borane, and sodium triacetoxyborohydride, wherein 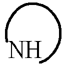 refers to ring U containing an NH group; ring C, ring D, R^{2e}, R^{3e}, R^{4e}, R^{5e}, and Y" are as defined and described in K1-I-a; 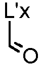 refers to Lx containing an aldehyde group; ring U, ring Y, X", Lx, and Ly are as defined in LA; E is as defined and described in the present disclosure, and preferably, E is E1-1a, E1-1b, E1-1c, E1-1d, E1-1e, E1-1f, E1-1g, E1-1aa, E E1-1h, 1-1bb, E1-1cc, E1-1dd, E1-1ee, E1-1ff, E1-1gg, or E1-1hh.
when K is K1-I-a 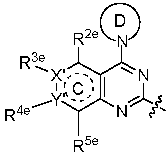 , L is LA 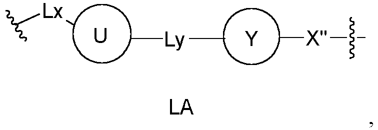 ring Y contains an NH group, and X" is C(O), the preparation method is: 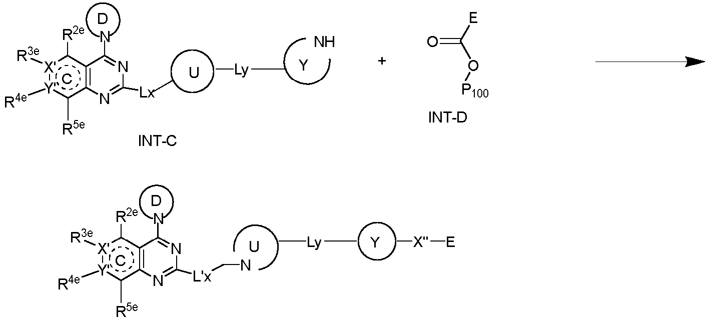
subjecting INT-C and INT-D to a substitution reaction under an alkaline condition to give the target compound, wherein the base includes, but is not limited to, triethylamine, *N,N*-diisopropylethylamine, potassium carbonate, sodium carbonate, and sodium bicarbonate, wherein 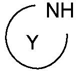 refers to ring Y containing an NH group; P₁₀₀ is pentafluorophenyl or *p*-nitrophenyl; ring C, ring D, R^{2e}, R^{3e}, R^{4e}, R^{5e}, and Y" are as defined and described in K1-I-a; ring U, ring Y, X", Lx, and Ly are as defined in LA; E is as defined and described in the present disclosure, and preferably, E is E1-1a, E1-1b, E1-1c, E1-1d, E1-1e, E1-1f, E1-1g, E1-1aa, E E1-1h, 1-1bb, E1-1cc, E1-1dd, E1-1ee, E1-1ff, E1-1gg, or E1-1hh.

Detailed description: Unless otherwise stated, the following terms used in the specification and claims have the following meanings.

"Alkyl" refers to a saturated aliphatic hydrocarbon group, including linear or branched alkyl; for example, C₁-C₈ alkyl refers to an alkyl group containing 1-8 carbon atoms, and the C₁-C₈ alkyl includes C₁-C₂, C₁-C₃, C₁-C₄, C₁-C₅, C₂-C₃, C₂-C₄ alkyl, etc., such as methyl, ethyl, *n*-propyl, isopropyl, *n*-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, or various branched isomers thereof, preferably C₁-C₆ alkyl, and more preferably C₁-C₄ alkyl. The alkyl may be substituted or unsubstituted. In some embodiments, the alkyl is C₁, C₂, C₃, C₄, C₅, C₆, C₇, or C₈ alkyl.

"Heteroalkyl" means that the methylene group (-CH₂-) in a saturated aliphatic hydrocarbon group is substituted with a heteroatom (e.g., O, S, or N), a heteroatom group (e.g., -C(O)-, -S(O)-, -S(O)₂-, -C(O)O-, or -OC(O)-), C(O)NH-, -NHC(O)-, ethenylene, or ethynylene, and includes linear or branched heteroalkyl; C₁-C₈ heteroalkyl means that at least one methylene group in an alkyl group containing 1-8 carbon atoms is substituted with a heteroatom or a heteroatom group, such as -C(O)-CH₃, -CH₂-O-CH₃, -CH₂-S-CH₃, -CH₂-S(O)-CH₃, -CH₂-S(O)₂-CH₃, -CH₂-S-CH₂-CH₃, -CH₂-O-CH₂-CH₃, -CH₂-O-CH₂-CH₃, -CH₂-S(O)-CH₂-CH₃, or various branched isomers thereof, preferably C₁-C₆ heteroalkyl, and more preferably C₁-C₄ heteroalkyl. The heteroalkyl may be substituted or unsubstituted. In some embodiments, the heteroalkyl is C₁, C₂, C₃, C₄, C₅, C₆, C₇, or C₈ heteroalkyl.

Unless otherwise specified, the number of atoms on a ring is generally defined as the member number of the ring. For example, "5- to 7-membered ring" refers to a "ring" on which 5 to 7 atoms are arranged in a circle.

Unless otherwise specified, Cₙ₋ₙ₊ₘ or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁, and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, etc. Similarly, n- to n+m-membered represents that the number of atoms on the ring is n to n+m. For example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. Also, any range within n to n+m may be included. For example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, etc.

"Cycloalkyl" refers to a saturated or partially unsaturated, monocyclic or polycyclic hydrocarbon substituent; "C₃-C₁₁ cycloalkyl" refers to a cycloalkyl group containing 3 to 11 carbon atoms, and the C₃-C₁₁ cycloalkyl includes C₃-C₁₁, C₃-C₁₀, C₃-C₈, C₄-C₁₁, C₄-C₁₀, C₄-C₈, C₄-C₇, C₄-C₆, C₄-C₅, etc.; "C₃-C₈ cycloalkyl" refers to a cycloalkyl group containing 3 to 8 carbon atoms, and the C₃-C₈ cycloalkyl includes C₃-C₈, C₃-C₆, C₃-C₅, C₄-C₈, C₄-C₇, C₄-C₆, C₄-C₅, etc.

Non-limiting examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl, etc., preferably cyclopropyl, cyclobutyl, cyclopentyl, and cyclohexyl, preferably C₃-C₈ cycloalkyl, and more preferably C₃-C₆ cycloalkyl.

Polycyclic cycloalkyl includes spiro, fused, and bridged cycloalkyl. "Spirocycloalkyl" refers to a polycyclic group in which a carbon atom (referred to as a spiro atom) is shared between monocyclic rings; they may contain one or more double bonds, but none of the rings has a fully conjugated π-electron system. According to the number of spiro atoms shared between rings, the spirocycloalkyl may include monospirocycloalkyl, bispirocycloalkyl, or polyspirocycloalkyl, and is preferably 7- to 12-membered bispirocycloalkyl. Non-limiting examples of the spirocycloalkyl include: 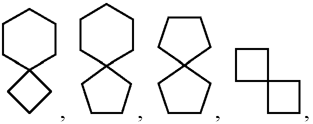 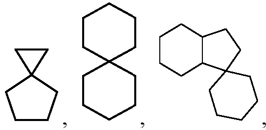 etc.

"Fused cycloalkyl" refers to an all-carbon polycyclic group in which each ring shares a pair of adjacent carbon atoms with the other rings in the system, wherein one or more of the rings may contain one or more double bonds, but none of the rings has a fully conjugated π-electron system. According to the number of constituent rings, the fused cycloalkyl may include bicyclic, tricyclic, tetracyclic, or polycyclic fused cycloalkyl, and is preferably bicyclic fused cycloalkyl. Non-limiting examples of the fused cycloalkyl include: 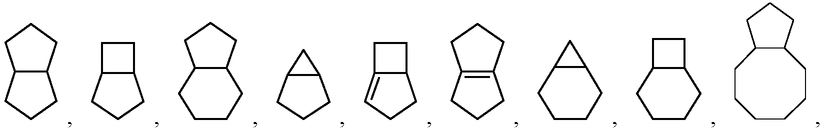 and

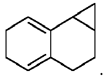

"Bridged cycloalkyl" refers to an all-carbon polycyclic group in which any two rings share two carbon atoms not directly connected; they may contain one or more double bonds, but none of the rings has a fully conjugated π-electron system. According to the number of constituent rings, the bridged cycloalkyl may include bicyclic, tricyclic, tetracyclic, or polycyclic bridged cycloalkyl. Non-limiting examples of the bridged cycloalkyl include: 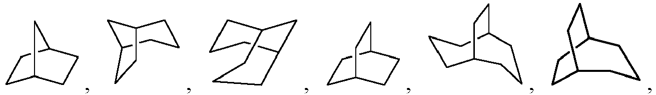 and

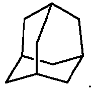

The cycloalkyl ring may be fused to an aryl, heteroaryl, or heterocycloalkyl ring, wherein the ring attached to the parent structure is cycloalkyl; non-limiting examples include indanyl, tetrahydronaphthyl, benzocycloheptyl, etc. The cycloalkyl may be optionally substituted or unsubstituted.

In some embodiments, the cycloalkyl is C₃, C₄, C₅, C₆, C₇, C₈, C₉, C₁₀, C₁₁ or C₁₂ monocyclic or polycyclic (e.g., spiro, fused or bridged) cycloalkyl.

"Heterocycloalkyl" refers to a saturated or partially unsaturated, monocyclic or polycyclic hydrocarbon substituent, wherein one or more (e.g., 2, 3, 4, or 5) of the ring atoms are selected from nitrogen, oxygen, and S(O)ᵣ (wherein r is an integer of 0, 1, or 2), but a ring portion of -O-O-, -O-S-, or -S-S- is not included, and the remaining ring atoms are carbon atoms, wherein the nitrogen atom is optionally quaternized, and the nitrogen and sulfur heteroatoms may optionally be oxidized (i.e., NO and S(O)p, and p is 1 or 2). "3- to 11-membered heterocycloalkyl" refers to a cyclic group containing 3 to 11 ring atoms, "5-to 10-membered heterocycloalkyl" refers to a cyclic group containing 5 to 10 ring atoms, and "3- to 8-membered heterocycloalkyl" refers to a cyclic group containing 3 to 8 ring atoms. "3- to 11-membered heterocycloalkyl" containing 1-2 heteroatoms selected from N, O, and S is preferred, and 3- to 11-membered heterocycloalkyl containing 1 or 2 N atoms is more preferred, such as 3- to 10-membered heterocycloalkyl, including 3- to 8-membered, 3- to 6-membered, 3- to 5-membered, 4- to 6-membered, 5- to 6-membered, 4-membered, 5-membered, and 6-membered heterocycloalkyl, etc. Examples of the 3- to 10-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, etc.

Monocyclic heterocycloalkyl is preferably 3- to 8-membered monocyclic heterocycloalkyl containing 1-2 N heteroatoms; non-limiting examples of the monocyclic heterocycloalkyl include pyrrolidinyl, piperidinyl, piperazinyl, morpholinyl, thiomorpholinyl, homopiperazinyl, etc., preferably piperidinyl and piperazinyl.

The polycyclic heterocycloalkyl includes spiro, fused, and bridged heterocycloalkyl. "Spiroheterocycloalkyl" refers to a polycyclic heterocycloalkyl group in which an atom (called spiro atom) is shared among monocyclic rings, wherein one or more of the ring atoms are selected from nitrogen, oxygen and S(O)ᵣ (wherein r is an integer of 0, 1 or 2), and the remaining ring atoms are carbon atoms. They may contain one or more double bonds, but none of the rings has a fully conjugated π-electron system. According to the number of spiro atoms shared between rings, the spirocycloalkyl may include monospiroheterocycloalkyl, bispiroheterocycloalkyl, or polyspiroheterocycloalkyl, and is preferably saturated "3- to 11-membered bispiroheterocycloalkyl" containing 1-2 heteroatoms selected from N, O, and S, and more preferably saturated "7- to 11-membered bispiroheterocycloalkyl" containing 1 or 2 N atoms. Non-limiting examples of the spiroheterocycloalkyl include: 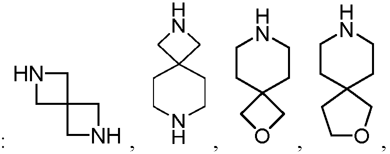 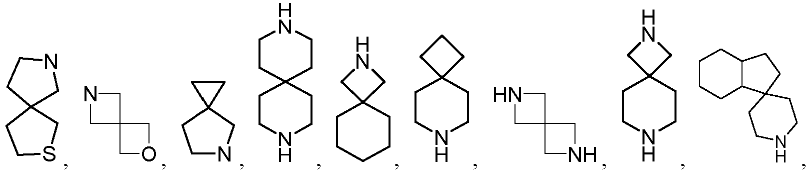 and the like.

"Fused heterocycloalkyl" refers to a polycyclic heterocycloalkyl group in which each ring shares a pair of adjacent atoms with the other rings in the system, wherein one or more of the rings may contain one or more double bonds, but none of the rings has a fully conjugated π-electron system, wherein one or more of the ring atoms are selected from nitrogen, oxygen and S(O)ᵣ (wherein r is an integer of 0, 1 or 2), and the remaining ring atoms are carbon atoms. According to the number of constituent rings, the fused heterocycloalkyl may include bicyclic, tricyclic, tetracyclic, or polycyclic fused heterocycloalkyl, and is preferably "3- to 11-membered bicyclic fused heterocycloalkyl" containing 1-3 heteroatoms selected from N, O, and S, and more preferably saturated "3- to 11-membered bicyclic fused heterocycloalkyl" containing 1 or 2 N atoms. Non-limiting examples of the fused heterocycloalkyl include: 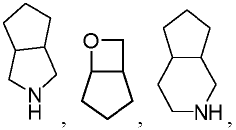 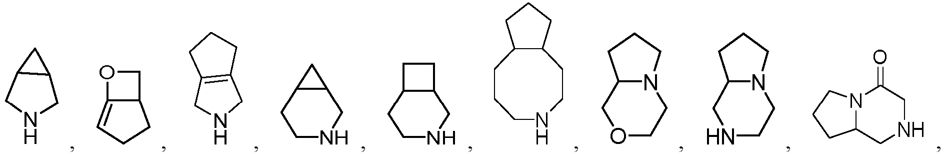 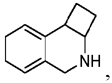 etc.

"Bridged heterocycloalkyl" refers to a polycyclic heterocycloalkyl group in which any two rings share two atoms not directly connected, and they may contain one or more double bonds, but none of the rings has a fully conjugated π-electron system, wherein one or more of the ring atoms are selected from nitrogen, oxygen or S(O)ᵣ (wherein r is an integer of 0, 1 or 2), and the other ring atoms are carbon atoms. According to the number of constituent rings, the bridged heterocycloalkyl may include bicyclic, tricyclic, tetracyclic, or polycyclic bridged cycloalkyl; non-limiting examples of the bridged heterocycloalkyl include: 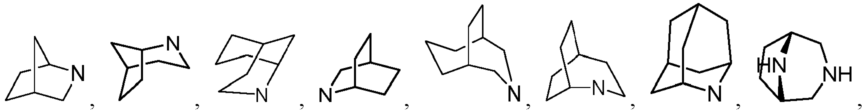 etc.

The heterocycloalkyl ring may be fused to an aryl, heteroaryl, or cycloalkyl ring, wherein the ring attached to the parent structure is heterocycloalkyl; non-limiting examples include: 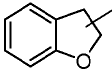 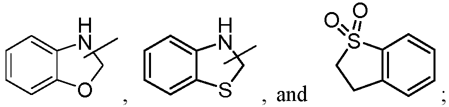 the heterocycloalkyl may be optionally substituted or unsubstituted. In some embodiments, the heterocycloalkyl is 3-, 4-, 5-, 6-, 7-, 8-, 9-, 10-, 11-, or 12-membered monocyclic or polycyclic (e.g., spiro, fused, or bridged) heterocycloalkyl, wherein the number of the heteroatoms may be 1, 2, 3, 4, or 5, each heteroatom being independently nitrogen, oxygen, or S(O)ᵣ (wherein r is an integer of 0, 1, or 2).

"Aryl" refers to an all-carbon monocyclic or fused polycyclic group (i.e., rings that share pairs of adjacent carbon atoms) and a polycyclic group having a conjugated π-electron system; "6- to 10-membered aryl" refers to an all-carbon aryl group containing 6-10 carbon atoms, such as phenyl and naphthyl, preferably phenyl. The aryl ring may be fused to a heteroaryl, heterocycloalkyl, or cycloalkyl ring, wherein the ring attached to the parent structure is an aryl ring; non-limiting examples include: 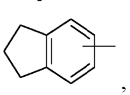 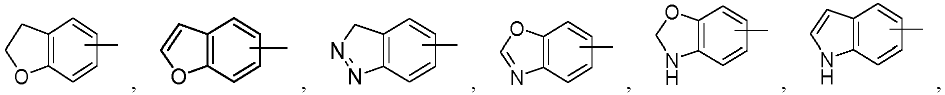 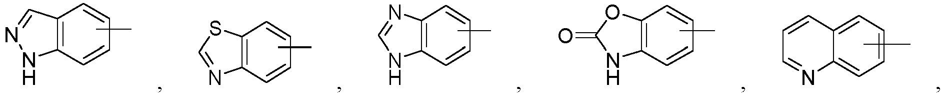 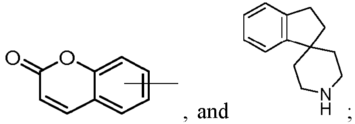 the aryl may be optionally substituted or unsubstituted. In some embodiments, the aryl is 6- to 10-membered aryl.

"Heteroaryl" refers to a heteroaromatic system containing 1 to 4 heteroatoms, and the heteroatoms include nitrogen, oxygen, or S(O)ᵣ (wherein r is an integer of 0, 1, or 2); 5- to 6-membered heteroaryl refers to a heteroaromatic system containing 5-6 ring atoms; 5- to 10-membered heteroaryl refers to a heteroaromatic system containing 5-10 ring atoms; preferably 5- to 6-membered heteroaryl; more preferably 5- to 6-membered heteroaryl containing 1 or 2 N atoms; non-limiting examples include furanyl, thienyl, pyridyl, pyrrolyl, N-alkylpyrrolyl, pyrimidinyl, pyrazinyl, pyrazole, imidazolyl, triazolyl, tetrazolyl, and the like, preferably pyridyl. The heteroaryl ring may be fused to an aryl, heterocycloalkyl, or cycloalkyl ring, wherein the ring attached to the parent structure is a heteroaryl ring; non-limiting examples include: 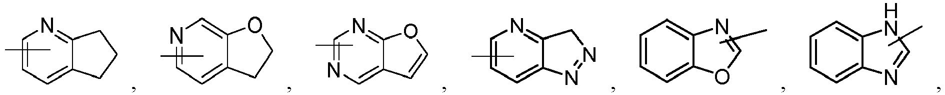 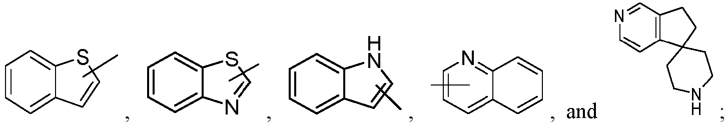 the heteroaryl may be optionally substituted or unsubstituted. In some embodiments, the heteroaryl is 5-, 6-, 7-, 8-, 9-, or 10-membered heteroaryl, wherein the number of the heteroatoms may be 1, 2, 3, 4, or 5, each heteroatom being independently nitrogen, oxygen, or S.

"Alkenyl" refers to an alkyl group as defined above that consists of at least two carbon atoms and at least one carbon-carbon double bond; "C₂-₈ alkenyl" refers to a linear or branched alkenyl group containing 2-8 carbon atoms, including but not limited to, ethenyl, 1-propenyl, 2-propenyl, 1-, 2- or 3-butenyl, etc., preferably "C₂-₆ alkenyl", and more preferably "C₂-₄ alkenyl". The alkenyl may be substituted or unsubstituted. In some embodiments, the alkenyl is C₂, C₃, C₄, C₅, C₆, C₇ or C₈ alkenyl.

"Alkynyl" refers to an alkyl group as defined above that consists of at least two carbon atoms and at least one carbon-carbon triple bond; "C₂-₈ alkynyl" refers to a linear or branched alkynyl group containing 2-8 carbon atoms, including but not limited to, ethynyl, 1-propynyl, 2-propynyl, 1-, 2-, or 3-butynyl, preferably "C₂-₆ alkynyl", and more preferably "C₂-₄ alkynyl". The alkynyl may be substituted or unsubstituted. In some embodiments, the alkynyl is C₂, C₃, C₄, C₅, C₆, C₇ or C₈ alkynyl.

"-ylene" refers to a divalent group. For example, alkylene refers to a divalent alkyl group; alkenylene refers to a divalent alkenyl group; alkynylene refers to a divalent alkynyl group; cycloalkylene refers to a divalent cycloalkyl group; heterocycloalkylene refers to a divalent heterocycloalkyl group; arylene refers to a divalent aryl group; heteroarylene refers to a divalent heteroaryl group; the alkyl, alkenyl, alkynyl, cycloalkyl, heterocycloalkyl, aryl, and heteroaryl groups are as defined above; the "-ylene" groups may be optionally substituted or unsubstituted.

"Haloalkyl" refers to an alkyl group optionally substituted with one or more fluorine, chlorine, bromine, or iodine atoms, wherein the alkyl group is as defined above; non-limiting examples include difluoromethyl, dichloromethyl, dibromomethyl, trifluoromethyl, trichloromethyl, tribromomethyl, etc.

"Hydroxyalkyl" refers to an alkyl group optionally substituted with one or more -OH, wherein the alkyl group is as defined above; non-limiting examples include hydroxymethyl, hydroxyethyl, hydroxypropyl, and hydroxyisopropyl.

"Alkoxy" refers to -O-alkyl, wherein the alkyl is as defined above; non-limiting examples include methoxy, ethoxy, isopropoxy, *tert*-butoxy, etc.

"Cycloalkoxy" refers to -O-cycloalkyl, wherein the cycloalkyl is as defined above; non-limiting examples include cyclopropaneoxy, cyclobutaneoxy, cyclopentanyloxy, cyclohexyloxy, etc.

"Heterocycloalkoxy" refers to -O-heterocycloalkyl, wherein the heterocycloalkyl is as defined above; non-limiting examples include azetidinyloxy, azacyclopentyloxy, piperidinyloxy, piperazinyloxy, oxolanyloxy, oxanyloxy, etc.

"-C(O)C₁-C₃ alkyl" refers to -C(O)-CH₃, -C(O)-CH₂CH₃, etc.

"Cyano" refers to -CN.

"Hydroxy" refers to -OH.

"Sulfonyl" refers to -S(O)₂-.

"Carboxyl" or "carboxylic acid" refers to -COOH.

"Oxo" refers to the =O group.

"Halogen" refers to fluorine, chlorine, bromine, or iodine.

"EA or EtOAc" refers to ethyl acetate.

"THF" refers to tetrahydrofuran.

"DCM" refers to dichloromethane.

"cataCXium A Pd-G3" refers to mesylate[n-butyl di(1-adamantyl)phosphine](2-amino-1,1'-biphenyl-2-yl)palladium(II).

"POCl₃" refers to phosphorus oxychloride.

"NaHCO₃" refers to sodium bicarbonate.

"NaCl" refers to sodium chloride.

"Na₂SO₄" refers to sodium sulfate.

"NH₄Cl" refers to ammonium chloride.

"Cs₂CO₃" refers to cesium carbonate.

"NaBH(OAc)₃" refers to sodium triacetoxyborohydride.

"Triton B" refers to benzyltrimethylammonium hydroxide.

"NaBH₃CN" refers to sodium cyanoborohydride.

"CsF" refers to cesium fluoride.

"Na₂CO₃" refers to sodium carbonate.

"HOAc" refers to acetic acid.

"TPAP" refers to tetrapropylammonium perruthenate.

"NMO" refers to N-methylmorpholine oxide.

"Cs₂CO₃" refers to cesium carbonate.

"NaH" refers to sodium hydride.

"*i*-PrOH" refers to isopropanol.

*"*DMF" refers to *N*,*N*-dimethylformamide.

"MeOH" refers to methanol.

"Pd/C" refers to palladium/carbon.

"DMSO" refers to dimethyl sulfoxide.

"TFA" refers to trifluoroacetic acid.

"DIEA" refers to *N,N*-diisopropylethylamine.

"Dess-Martin" refers to Dess-Martin reagent.

"PBS" refers to phosphate-buffered saline.

"SDS-PAGE" refers to sodium dodecyl sulfate-polyacrylamide gel electrophoresis.

"PVDF" refers to polyvinylidene fluoride.

"PE" refers to petroleum ether.

"AcOH" refers to acetic acid.

"HCl" refers to hydrochloric acid.

"NH₃" refers to ammonia water.

"EDCI" refers to 1-ethyl-(3-dimethylaminopropyl)carbodiimide hydrochloride.

"HOBT" refers to 1-hydroxybenzotriazole.

"HMPA" refers to hexamethylphosphoramide.

"Pd(dppf)Cl₂" refers to [1,1'-bis(diphenylphosphino)ferrocene]palladium(II) dichloride.

"LAH" refers to lithium aluminum hydride.

"Prep-HPLC" refers to preparative high-performance liquid chromatography.

"sat." refers to a saturated solution.

"aq" refers to an aqueous solution.

In the chemical structure herein, "-" as a linking bond represents a single bond, and "=" represents a double bond (in the case of an undefined configuration, it may be *trans* or *cis*)*.*

"Optional" or "optionally" means that the event or circumstance subsequently described may, but does not necessarily, occur; this description includes instances where the event or circumstance occurs or does not occur. For example, "a heterocycloalkyl group optionally substituted with an alkyl group" means that the alkyl group may, but does not necessarily, exist; this description includes instances where the heterocycloalkyl group is substituted with the alkyl group and instances where it is not.

"Substituted" means that one or more, preferably up to 5, and more preferably 1-3, hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when amino or hydroxy having a free hydrogen is bound to a carbon atom having an unsaturated (such as olefin) bond.

As used herein, unless otherwise indicated, the term "optionally substituted" may be unsubstituted or substituted; when it is substituted, the substituent may be one or more (e.g., 2, 3, 4, 5 or 6) groups independently selected from alkyl, alkenyl, alkynyl, hydroxy, hydroxyalkyl, haloalkyl, alkoxy, amino, aminoalkyl, cyano, halogen, oxo, cycloalkyl, heterocycloalkyl, aryl and heteroaryl, and the alkyl, the alkenyl, the alkynyl, the hydroxy, the hydroxyalkyl, the haloalkyl, the alkoxy, the amino and the aminoalkyl are optionally substituted with one or more (e.g., 2, 3, 4, 5 or 6) cycloalkyl, heterocycloalkyl, aryl or heteroaryl; the cycloalkyl, the heterocycloalkyl, the aryl and the heteroaryl are optionally substituted with one or more groups selected from alkyl, alkenyl, alkynyl, hydroxy, hydroxyalkyl, haloalkyl, alkoxy, amino, aminoalkyl, cyano, halogen and oxo.

Unless otherwise indicated, the following terms and phrases used herein are intended to have the following meanings. A specific term or phrase should not be considered indefinite or unclear in the absence of a specific definition, but should be understood in the conventional sense. When a trade name appears herein, it is intended to refer to its corresponding commodity or active ingredient thereof.

"Pharmaceutical composition" refers to a mixture containing one or more of the compounds or the physiologically/pharmaceutically acceptable salts or prodrugs thereof described herein and other chemical components, as well as other components such as physiologically/pharmaceutically acceptable carriers and excipients. The pharmaceutical composition is intended to promote the administration to an organism, so as to facilitate the absorption of the active ingredient, thereby exerting biological activity.

The present disclosure further provides a pharmaceutically acceptable salt of the compound of formula (I). The term "pharmaceutically acceptable salt" refers to a relatively non-toxic acid addition salt or base addition salt of the compound of the present disclosure. The acid addition salt is a salt formed from the compound of formula (I) of the present disclosure and a suitable inorganic acid or organic acid. These salts may be prepared during the final isolation and purification of the compound or by reacting the purified compound of formula (I) in its free base form with a suitable organic acid or inorganic acid. The representative acid addition salt includes hydrochloride, tartrate, hydrobromide, sulfate, bisulfate, sulfite, acetate, oxalate, valerate, oleate, palmitate, stearate, laurate, borate, benzoate, lactate, phosphate, biphosphate, carbonate, bicarbonate, toluate, citrate, maleate, fumarate, succinate, methanesulfonate, p-toluenesulfonate, gluconate, lactobionate, lauryl sulfonate, and the like. The base addition salt is a salt formed from the compound of formula (I) and a suitable inorganic base or organic base, including, for example, salts formed with alkali metal, alkaline earth metal, and quaternary ammonium cations, such as sodium salts, lithium salts, potassium salts, calcium salts, magnesium salts, tetramethyl quaternary ammonium salts, tetraethyl quaternary ammonium salts, and the like; amine salts include salts formed with ammonia (NH₃) and primary, secondary, or tertiary amines, such as methylamine salts, dimethylamine salts, trimethylamine salts, triethylamine salts, ethylamine salts, and the like.

The pharmaceutically acceptable salts of the present disclosure can be synthesized from a parent compound containing an acidic or basic group using conventional chemical methods. In general, such salts are prepared by subjecting the compounds in a free acid or base form to a reaction with a stoichiometric amount of appropriate base or acid in water or an organic solvent or a mixture thereof.

The term "pharmaceutically acceptable excipient" refers to an inert substance administered with an active ingredient to facilitate administration of the active ingredient, including but not limited to, any glidant, sweetener, diluent, preservative, dye/coloring agent, flavor enhancer, surfactant, wetting agent, dispersant, disintegrant, suspending agent, stabilizer, isotonizing agent, solvent, or emulsifier acceptable for use in humans or animals (e.g., domesticated animals) as permitted by the National Medical Products Administration, PRC.

As used herein and as is familiar in the art, "treatment" or "treating" is a method for obtaining beneficial or desired results, including clinical results. The beneficial or desired clinical results may include, but are not limited to, reduction in the tumor progression, reduction in the tumor size, decrease in the tumor growth rate, reduction in tumor invasion and metastatic potential, alleviation or amelioration of one or more symptoms or conditions, decrease in the extent of disease, stabilized (i.e., not worsening) disease state, prevention of disease spread, delay or slowing down of the disease progression, amelioration or mitigation of the disease state, and alleviation (whether partial or total), whether detectable or undetectable. "Treatment" or "treating" may also refers to prolonging survival as compared to the expected survival if not receiving treatment.

The therapeutic dose of the compound of the present application may be determined, for example, according to: the specific use of the treatment, the route of administration of the compound, the health and state of the patient, and the judgment of the prescriber. The proportion or concentration of the compound of the present application in a pharmaceutical composition may not be constant and depends on a variety of factors including doses, chemical properties (e.g., hydrophobicity), and administration routes.

The term "treatment" or "treating" refers to administering the compound or formulation described herein to ameliorate or eliminate a disease or one or more symptoms related to the disease, and includes:
(i) inhibiting a disease or disease state, i.e., arresting its progression; and
(ii) alleviating a disease or disease state, i.e., causing regression of the disease or the disease state.

The term "therapeutically effective amount" refers to an amount of the compound of the present application for (i) treating a specific disease, condition, or disorder, (ii) alleviating, ameliorating, or eliminating one or more symptoms of a specific disease, condition, or disorder, or (iii) preventing or delaying onset of one or more symptoms of the specific disease, condition, or disorder described herein. The amount of the compound of the present application composing the "therapeutically effective amount" varies depending on the compound, the disease state, and its severity, the administration mode, and the age of the mammal to be treated, but may be determined routinely by those skilled in the art in accordance with their knowledge and the present disclosure.

Unless otherwise required, the word "comprise" and variations thereof such as "comprises" and "comprising", used in the specification and the claims which follow, should be understood in an open-ended and non-exclusive sense, i.e., "including, but not limited to".

"In some embodiments", "in an embodiment", "in another embodiment", or "in certain embodiments" used in the specification means that a specific reference element, structure, or feature described in connection with the embodiment is included in at least one embodiment. Thus, the phrase "in some embodiments", "in an embodiment", "in another embodiment", or "in certain embodiments" in various places throughout the specification are not necessarily all referring to the same embodiment. Furthermore, the specific elements, structures, or features may be combined in any suitable manner in one or more embodiments.

Unless otherwise indicated, the term "isomer" is intended to include geometric isomers, *cis*-*trans* isomers, stereoisomers, enantiomers, optical isomers, diastereomers, and tautomers.

The compounds of the present disclosure may be in the form of a specific geometric isomer or stereoisomer. All such compounds are contemplated herein, including *cis* and *trans* isomers, (-)- and (+)-enantiomers, (R)- and (S)-enantiomers, diastereomers, (D)-isomers, (L)-isomers, and racemic mixtures and other mixtures thereof, such as enantiomer or diastereomer enriched mixtures, all of which are encompassed within the scope of the present disclosure. Additional asymmetric carbon atoms may be present in substituents such as alkyl. All these isomers and mixtures thereof are encompassed within the scope of the present disclosure.

Unless otherwise indicated, the term "enantiomer" or "optical isomer" refers to stereoisomers that are mirror images of each other.

Unless otherwise indicated, the term *"cis-trans* isomer" or "geometric isomer" results from the inability of a double bond or a single bond of a ring carbon atom to rotate freely.

Unless otherwise indicated, the term "diastereomer" refers to stereoisomers whose molecules have two or more chiral centers and are not mirror images of each other.

Unless otherwise indicated, the absolute configuration of a stereogenic center is represented by a wedged solid bond and a wedged dashed bond, and the relative configuration of a stereogenic center is represented by a straight solid bond and a straight dashed bond. A wavy line represents a wedged solid bond or a wedged dashed bond, or a wavy line represents a straight solid bond or a straight dashed bond.

Unless otherwise indicated, the term "diastereomer" refers to stereoisomers whose molecules have two or more chiral centers and are not mirror images of each other.

Unless otherwise indicated, "(+)" represents dextrorotation, "(-)" represents levorotation, and "(±)" represents racemization.

"Optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted with substituents, wherein the substituents may include deuterium and hydrogen variants, as long as the valence of the specific atom is normal and the substituted compound is stable. When the substituent is oxygen (i.e., =O), it means that two hydrogen atoms are substituted. Substitution with oxygen does not occur on aromatic groups.

The term "optionally substituted" means that an atom may or may not be substituted with a substituent. Unless otherwise specified, the type and number of the substituent may be arbitrary as long as being chemically achievable.

The term "prodrug" refers to a chemical derivative of the compound of the present disclosure that, if chemically reacted *in vivo,* is converted to a compound of general formula I.

When any variable (e.g., R) occurs once or more in the constitution or structure of a compound, the definition of the variable in each case is independent. Thus, for example, if a group is substituted with 0-2 R, the group may be optionally substituted with up to two R, and the definition of R in each case is independent. Furthermore, a combination of the substituent and/or the variant thereof is permissible only if the combination can result in a stable compound.

When the number of a linking group is 0, for example, -(CRR)0-, it means that the linking group is a single bond.

When one of the variables is selected from a single bond, it means that the two groups connected to it are linked directly. For example, in A-L-Z, when L represents a single bond, it means that the structure is actually A-Z.

When a substituent is absent, it means that there is no such a substituent. For example, when X in A-X is absent, the structure is actually A. When it is not specified by which atom the listed substituent is linked to the group to be substituted, the substituent may be linked via any atom of the group. For example, pyridyl as a substituent may be linked to the group to be substituted via any carbon atom on the pyridine ring.

When the direction for linkage of the listed linking group is not specified, the direction for linkage is arbitrary. For example, when the linking group L is -M-W-, -M-W- may either link ring A to ring B in a direction same as left-to-right reading order to form, or link ring A to ring B in a direction opposite to the left-to-right reading order to form. A combination of the linking group, the substituent, and/or the variant thereof is permissible only if the combination can result in a stable compound.

Unless otherwise specified, when a group has one or more linkable sites, any one or more of the sites of the group may be linked to other groups by chemical bonds. If there is no designated linking mode for chemical bonds and H atoms are present at a linkable site, when the linkable site is linked to chemical bonds, the number of the H atoms at the linkable site is correspondingly reduced based on the number of the linked chemical bonds, so that the resulting groups have corresponding valences. The chemical bond that links the site to another group may be represented by a straight solid bond, a straight dashed bond, or a wavy line. For example, the straight solid bond in -OCH₃ indicates that the group is linked to another group through the oxygen atom; the straight dashed bond indicates that the group is linked to another group through the two ends of the nitrogen atom; the wavy line indicates that the phenyl group is linked to another group through the carbon atoms on positions 1 and 2.

Unless otherwise specified, 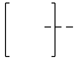 is used to indicate that a hydrogen atom at any position of a group within 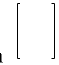 may be substituted.

Unless otherwise indicated, the term "enriched with one isomer", "isomer enriched", "enriched with one enantiomer", or "enantiomer enriched" means that the content of one of the isomers or enantiomers is less than 100% and more than or equal to 60%, or more than or equal to 70%, or more than or equal to 80%, or more than or equal to 90%, or more than or equal to 95%, or more than or equal to 96%, or more than or equal to 97%, or more than or equal to 98%, or more than or equal to 99%, or more than or equal to 99.5%, or more than or equal to 99.6%, or more than or equal to 99.7%, or more than or equal to 99.8%, or more than or equal to 99.9%.

Unless otherwise indicated, the term "isomeric excess" or "enantiomeric excess" refers to the difference between the relative percentages of two isomers or enantiomers. For example, if the content of one of the isomers or enantiomers is 90% and the content of the other isomer or enantiomer is 10%, the isomeric or enantiomeric excess (ee) is 80%.

Optically active (R)- and (S)-enantiomers, as well as D- and L-isomers may be prepared by chiral synthesis, chiral reagents, or other conventional techniques. If one enantiomer of a certain compound of the present disclosure is to be obtained, the desired pure enantiomer may be prepared by asymmetric synthesis or derivatization using a chiral auxiliary, wherein the resulting diastereoisomeric mixture is separated and the auxiliary group is cleaved. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

The compound of the present disclosure may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen may be substituted with deuterium to form a deuterated drug, and the bond formed by deuterium and carbon is firmer than that formed by common hydrogen and carbon. Compared with an un-deuterated drug, the deuterated drug has the advantages of reduced toxic and side effects, increased pharmaceutical stability, enhanced efficacy, prolonged biological half-life, and the like. All isotopic variations of the compounds of the present disclosure, whether radioactive or not, are encompassed within the scope of the present disclosure.

The compounds of the present disclosure may be prepared by a variety of synthetic methods well known to those skilled in the art, including the specific embodiments listed below, embodiments formed by combinations thereof with other chemical synthetic methods, and equivalents thereof well known to those skilled in the art. Preferred embodiments include, but are not limited to, the examples of the present disclosure.

The structures of the compounds of the present disclosure may be confirmed by conventional methods well known to those skilled in the art, and if the present disclosure relates to an absolute configuration of the compound, the absolute configuration may be confirmed by means of conventional techniques in the art. For example, in single crystal X-ray diffraction (SXRD), intensity data of diffraction of the single crystal grown are collected with a Bruker D8 venture diffractometer, with the light source being Cu-Kα radiation and the scanning mode being scanning; after related data are collected, a direct method (Shelxs97) is further employed to analyze the crystal structure, and thus the absolute configuration can be confirmed.

The compound or the pharmaceutically acceptable salt thereof of the present disclosure may be administered to mammals including humans, and may be administered orally, rectally, parenterally (intravenously, intramuscularly, or subcutaneously), topically (powders, ointments, or drops), or intratumorally.

The compound of the present disclosure may be administered at a dose of about 0.05-300 mg/kg body weight/day, preferably 10-300 mg/kg body weight/day, and more preferably 10-200 mg/kg body weight/day.

The compound or the pharmaceutically acceptable salt thereof of the present disclosure may be formulated into a solid dosage form for oral administration, including but not limited to, capsules, tablets, pills, pulvis, granules, and the like. In these solid dosage forms, the compound of formula (I) of the present disclosure, as an active ingredient, is mixed with at least one conventional inert excipient (or carrier), such as sodium citrate or dicalcium phosphate, or with the following ingredients: (1) fillers or extenders, such as starch, lactose, sucrose, glucose, mannitol, silicic acid, and the like; (2) binders, such as hydroxymethyl cellulose, alginate, gelatin, polyvinylpyrrolidone, sucrose, acacia, and the like; (3) humectants, such as glycerol and the like; (4) disintegrants, such as agar, calcium carbonate, potato or tapioca starch, alginic acid, certain complex silicates, sodium carbonate, and the like; (5) solution retarders, such as paraffin and the like; (6) absorption accelerators, such as quaternary ammonium compounds and the like; (7) wetting agents, such as cetyl alcohol, glycerol monostearate, and the like; (8) adsorbents, such as kaolin and the like; and (9) lubricants, such as talc, calcium stearate, magnesium stearate, solid polyethylene glycol, sodium lauryl sulfate, and the like, or mixtures thereof. Capsules, tablets, and pills may further comprise buffers.

The solid dosage forms such as tablets, dragees, capsules, pills, and granules may be coated or microencapsulated using coatings and shells such as enteric coatings and other materials well known in the art. They may comprise opacifying agents, and the active ingredient in such a composition may be released in a certain part of the digestive tract in a delayed manner. Examples of embedding components that can be used are polymeric substances and wax-based substances. If necessary, the active ingredient can also be in microcapsule form with one or more of the above-mentioned excipients.

The compound or the pharmaceutically acceptable salt thereof of the present disclosure may be formulated into a liquid dosage form for oral administration, including but not limited to, pharmaceutically acceptable emulsions, solutions, suspensions, syrups, tinctures, and the like. In addition to the compound of formula (I) or the pharmaceutically acceptable salt thereof as an active ingredient, the liquid dosage form may comprise inert diluents commonly used in the art, such as water and other solvents, solubilizers, and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, sesame oil, and the like, or mixtures of these substances. In addition to these inert diluents, the liquid dosage form of the present disclosure may further comprise conventional adjuvants, such as wetting agents, emulsifiers and suspending agents, sweeteners, flavoring agents, perfuming agents, and the like.

The suspending agents include, for example, ethoxylated isostearyl alcohols, polyoxyethylene sorbitol and sorbitan esters, microcrystalline cellulose, aluminum methylate, agar, and the like, or mixtures of these substances.

The compound or the pharmaceutically acceptable salt thereof of the present disclosure may be formulated into a dosage form for parenteral injection, including but not limited to, physiologically acceptable sterile aqueous or water-free solutions, dispersions, suspensions, or emulsions, and sterile powders for re-dissolution to form sterile injectable solutions or dispersions. Suitable carriers, diluents, solvents, or excipients include water, ethanol, polyols, and suitable mixtures thereof.

The compound or the pharmaceutically acceptable salt thereof of the present disclosure may also be formulated into a dosage form for topical administration, including, for example, ointments, pulvis, suppositories, drops, sprays, inhalants, and the like. The compound of formula (I) or the pharmaceutically acceptable salt thereof of the present disclosure as an active ingredient is mixed under sterile conditions with a physiologically acceptable carrier and optional preservatives, buffers, or propellants that may be required if necessary.

The present disclosure further provides a pharmaceutical composition comprising the compound of formula (I) or the pharmaceutically acceptable salt thereof of the present disclosure as an active ingredient, and a pharmaceutically acceptable carrier, excipient, or diluent. In the preparation of the pharmaceutical composition, the compound of formula (I) or the pharmaceutically acceptable salt thereof of the present disclosure is typically mixed with the pharmaceutically acceptable carrier, excipient, or diluent.

The composition of the present disclosure may be formulated into a conventional pharmaceutical formulation according to a conventional preparation method. Examples include tablets, pills, capsules, pulvis, granules, emulsions, suspensions, dispersions, solutions, syrups, elixirs, ointments, drops, suppositories, inhalants, sprays, and the like.

The compound or the pharmaceutically acceptable salt thereof described herein may be administered alone or, if desired, in combination with other pharmaceutically acceptable therapeutic agents, such as other anti-tumor medicaments. The ingredients to be combined may be administered simultaneously or sequentially, and administered in a single formulation form or in different formulation forms. The combination may include not only a combination of the compound of the present disclosure and one additional active agent but also a combination of the compound of the present disclosure and two or more additional active agents.

In the present disclosure, other pharmaceutically acceptable therapeutic agents that may be used together with or in combination with the pan-KRAS degrader, the compound of formula (I), may be: EGFR and/or a mutant inhibitor thereof, ErbB2(Her2) and/or a mutant inhibitor thereof, ALK and/or a mutant inhibitor thereof, MEK and/or a mutant inhibitor thereof, KRAS and/or a mutant inhibitor thereof, BCR-ABL and/or a mutant inhibitor thereof, FGFR1/FGFR2/FGFR3 and/or a mutant inhibitor thereof, ROS1 and/or a mutant inhibitor thereof, c-MET and/or a mutant inhibitor thereof, AXL and/or a mutant inhibitor thereof, NTRK1 and/or a mutant inhibitor thereof, RET and/or a mutant inhibitor thereof, taxane, a platinum-containing compound, an antimetabolite, a mitotic kinase inhibitor, an immunotherapeutic agent, an anti-angiogenic drug, a topoisomerase inhibitor, A-Raf/B-Raf/C-RAf and/or a mutant inhibitor thereof, ERK and/or a mutant inhibitor thereof, an apoptosis inhibitor, AKT and/or a mutant inhibitor thereof, an mTOR inhibitor, an epigenetic modulator, an IGF1/2 and/or IGF1-R inhibitor, Ras GEF and/or a mutant inhibitor thereof, SOS1 and/or a mutant inhibitor thereof, SHP2 and/or a mutant inhibitor thereof, PI3K and/or a mutant inhibitor thereof, or a PD-1/PD-L1 inhibitor; EGFR and/or a mutant degrader thereof, ErbB2(Her2) and/or a mutant degrader thereof, ALK and/or a mutant degrader thereof, MEK and/or a mutant degrader thereof, KRAS and/or a mutant degrader thereof, BCR-ABL and/or a mutant degrader thereof, FGFR1/FGFR2/FGFR3 and/or a mutant degrader thereof, ROS1 and/or a mutant degrader thereof, c-MET and/or a mutant degrader thereof, AXL and/or a mutant degrader thereof, NTRK1 and/or a mutant degrader thereof, RET and/or a mutant degrader thereof, A-Raf/B-Raf/C-RAf and/or a mutant degrader thereof, ERK and/or a mutant degrader thereof, AKT and/or a mutant degrader thereof, an IGF1/2 and/or IGF1-R degrader, Ras GEF and/or a mutant degrader thereof, SOS1 and/or a mutant degrader thereof, SHP2 and/or a mutant degrader thereof, or PI3K and/or a mutant degrader thereof; EGFR and/or a mutant monoclonal antibody thereof, ErbB2(Her2) and/or a mutant monoclonal antibody thereof, a PD-1/PD-L1 monoclonal antibody, or a CTLA-4 monoclonal antibody; a PD-L1/TIGHT bispecific antibody, a PD-L1/CTLA-4 bispecific antibody, an EGFR/MET bispecific antibody, an EGFR/CD3 bispecific antibody, an EGFR/4-IBB bispecific antibody, a PD-L1/4-IBB bispecific antibody, or an HER2/CD3 bispecific antibody.

In the present disclosure, other pharmaceutically acceptable therapeutic agents that may be used together with or in combination with the pan-KRAS degrader, the compound of formula (I), may be: afatinib, erlotinib, gefitinib, lapatinib, cetuximab, panitumumab, osimertinib, olmutinib, EGF-816, trastuzumab, pertuzumab, crizotinib, alectinib, entrectinib, brigatinib, trametinib, cobimetinib, binimetinib, selumetinib, refametinib, imatinib, dasatinib, nilotinib, nintedanib, crizotinib, lorlatinib, ceritinib, merestinib, paclitaxel, nab-paclitaxel, docetaxel, cisplatin, carboplatin, oxaliplatin, 5-fluorouracil, capecitabine, floxuridine, cytarabine, gemcitabine, a combination of trifluridine and tipiracil (=TAS102), palbociclib, ribociclib, abemaciclib, ipilimumab, nivolumab, pembrolizumab, atezolizumab, avelumab, durvalumab, pidilizumab, PDR-001 (=spartalizumab), bevacizumab, irinotecan, liposomal irinotecan, topotecan, ulixertinib, rapamycin, temsirolimus, everolimus, ridaforolimus, JQ-1, GSK 525762, OTX 015 (=MK8628), CPI 0610, TEN-010 (=RO6870810), xentuzumab (antibody 60833 in WO 2010/066868) or MEDI-573 (=dusigitumab).

On the basis of the general knowledge in the art, the preferred conditions described above may be combined arbitrarily to obtain preferred embodiments of the present disclosure.

The reagents and starting materials used in the present disclosure are commercially available.

Compounds are named according to conventional nomenclature rules in the art or Chemdraw^{®} software, and supplier's catalog names are given for commercially available compounds.

According to the present disclosure, KRAS G12C, KRAS G12D, or KRAS G12V protein binding activity tests proves that the compound of formula I or I' described herein is able to effectively bind to pan-KRAS target protein to generate the effect of inhibiting downstream channel, and it is proved that the compound of formula I or I' is able to effectively degrade KRAS G12C protein in NCI-H358 cells, KRAS G12D protein in A427 cells, KRAS G12D protein in AsPc-1 cells, KRAS G12V protein in SW900 cells, KRAS G12V protein in SW620 cells, KRAS 13D protein in DLD-1 cells, and KRAS WT (expanded) protein in MKN1 cells by means of Western-Blot. The compound of formula I or I', and/or the stereoisomer, the enantiomer, the diastereomer, the deuteride, the hydrate, the solvate, the metabolite or the prodrug thereof and/or the pharmaceutically acceptable salt thereof described herein can effectively degrade KRAS G12C, KRAS G12D, KRAS G12V, KRAS G13D, KRAS WT (expanded) protein, KRAS G12V, KRAS G12D, and KRAS G12C protein, thereby achieving the effect of preventing or treating a disease or disorder caused by mutations in KRAS G13D, KRAS WT (expanded) protein, KRAS G12V, KRAS G12D, and KRAS G12C.

### DETAILED DESCRIPTION

The present disclosure is further explained in detail below with reference to examples; however, the examples are not intended to limit the present disclosure, and the present disclosure is not limited to the contents of the examples. The starting materials in the examples of the present disclosure are known and commercially available, or may be synthesized by using or following methods known in the art. Unless otherwise stated, experimental methods without specific conditions indicated in the examples of the present disclosure were generally conducted under conventional conditions or conditions recommended by the manufacturers of the starting materials or commercial products.

### Compound Preparation Examples

### Intermediate 1: 2,4,7-trichloro-8-fluoropyrido[4,3-d]pyrimidine

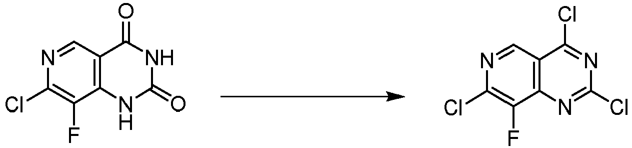

7-Chloro-8-fluoro-1,2,3,4-tetrahydropyrido[4,3-*d*]pyrimidine-2,4-dione (9.30 g, 43.14 mol) was dissolved in POCl₃ (100 mL). The mixture was cooled to 0 °C, and DIEA (16.69 g, 129.42 mol) was slowly added dropwise with stirring. The reaction liquid was heated to 120 °C and reacted for 16 h. After the reaction was completed, the reaction liquid was concentrated, and the concentrate was diluted with ethyl acetate. The mixture was slowly poured into ice water to quench the reaction and extracted with ethyl acetate (100 mL × 3), and the organic phase was separated. The organic phase was sequentially washed with a cooled aqueous NaHCO₃ solution and a saturated NaCl solution, dried over anhydrous Na₂SO₄, filtered, and concentrated under reduced pressure to give 2,4,7-trichloro-8-fluoropyrido[4,3-*d*]pyrimidine.

### Intermediate 2: (R)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropane-1-carbaldehyde

### Step 1: preparation of (R)-1-(2,7-dichloro-8-fluoropyrido[4,3-d]pyrimidin-4-yl)-3-methylpiperidin-3-ol

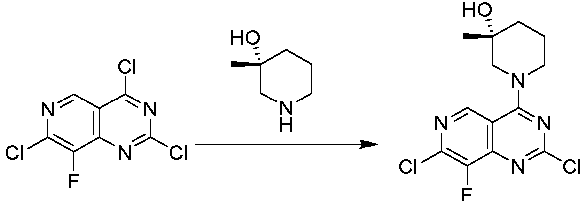

2,4,7-Trichloro-8-fluoropyrido[4,3-*d*]pyrimidine (1.9 g, 7.53 mmol) and triethylamine (1.9 g, 18.82 mmol) were dissolved in THF (20 mL), and the mixture was cooled to -78 °C. (*R*)-3-methylpiperidin-3-ol hydrochloride (0.9 g, 6.02 mmol) was added, and the mixture was reacted at -78 °C for 3 h. After the reaction was completed, the reaction liquid was poured into a saturated aqueous ammonium chloride solution (20 mL), and the mixture was extracted with ethyl acetate (30 mL × 3). The organic phases were combined, washed with saturated brine, dried over anhydrous sodium sulfate, and filtered. The filtrate was concentrated, and the resulting crude product was purified by silica gel column chromatography (DCM:MeOH = 100:1 to 50:1) to give (*R*)-1-(2,7-dichloro-8-fluoropyrido[4,3-*d*]pyrimidin-4-yl)-3-methylpiperidin-3-ol.

LC-MS: (ESI, m/z): [M+H]⁺ = 331.1.

### Step 2: preparation of (R)-1-(2-((1-(((tert-butyldiphenylsilyl)oxy)methyl)cyclopropyl)methoxy)-7-chloro-8-fluoropyrido[4,3-d]pyrimidin-4-yl)-3-methylpiperidin-3-ol

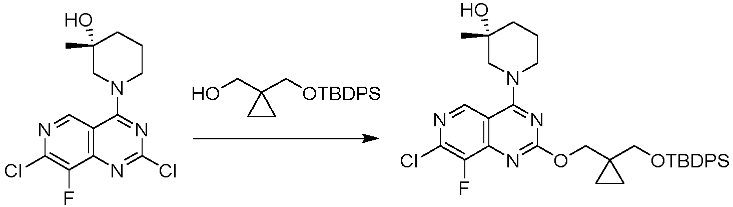

(*R*)-1-(2,7-dichloro-8-fluoropyrido[4,3-*d*]pyrimidin-4-yl)-3-methylpiperidin-3-ol (971 mg, 2.93 mmol) and (1-(((*tert*-butyldiphenylsilyl)oxy)methyl)cyclopropyl)methanol (2.0 g, 5.86 mmol) were added to anhydrous acetonitrile (10 mL), and then cesium carbonate (1.4 g, 4.40 mmol) and 1,4-diazabicyclo[2.2.2]octane (164 mg, 1.47 mmol) were added. The mixture was stirred at 25 °C for 1 h. After the reaction was completed, saturated brine (30 mL) was added to quench the reaction, and then the mixture was extracted with ethyl acetate (50 mL × 3). The organic phases were combined, dried over anhydrous sodium sulfate, and concentrated under reduced pressure. The concentrate was purified by silica gel column chromatography (PE:EA = 5:1) to give (*R*)-1-(2-((1-(((*tert*butyldiphenylsilyl)oxy)methyl)cyclopropyl)methoxy)-7-chloro-8-fluoropyrido[4,3-*d*]pyrimidin-4-yl)-3-methylpiperidin-3-ol.

LC-MS: (ESI, m/z): [M+H]⁺ = 635.4.

### Step 3: preparation of (R)-1-(7-chloro-8-fluoro-2-((1-(hydroxymethyl)cyclopropyl)methoxy)pyrido[4,3-d]pyrimidin-4-yl)-3-methylpiperidin-3-ol

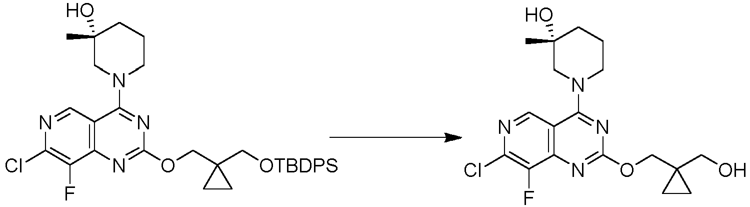

*(R)-1-(2-((1-(((tert-butyldiphenylsilyl)oxy)methyl)cyclopropyl)methoxy)-7-chloro-8-*fluoropyrido[4,3-*d*]pyrimidin-4-yl)-3-methylpiperidin-3-ol (988 mg, 1.56 mmol) was dissolved in DMF (10 mL), and cesium fluoride (709 mg, 4.67 mmol) was added. The mixture was heated to 50 °C and reacted for 6 h. After the reaction was completed, the mixture was cooled to room temperature. Water (30 mL) was added to quench the reaction, and the mixture was extracted with ethyl acetate (60 mL × 3). The organic phases were combined, dried over anhydrous sodium sulfate, and concentrated under reduced pressure. The concentrate was purified by silica gel column chromatography (DCM:MeOH = 98:2) to give (R)-1-(7-chloro-8-fluoro-2-((1-(hydroxymethyl)cyclopropyl)methoxy)pyrido[4,3-*d*]pyrimidin-4-yl)-3-methylpiperidin-3-ol.

LC-MS: (ESI, m/z): [M+H]⁺ = 397.1.

¹H NMR (400 MHz, CDCl₃) δ 8.93 (s, 1H), 4.50 - 4.36 (m, 4H), 3.65 - 3.56 (m, 1H), 3.46 - 3.32 (m, 3H), 3.26 (d, *J* = 13.6 Hz, 1H), 2.52 - 2.40 (m, 1H), 2.15 - 1.99 (m, 1H), 1.92 - 1.83 (m, 1H), 1.78 - 1.66 (m, 2H), 1.33 (s, 3H), 0.71 - 0.66 (m, 2H), 0.63 - 0.58 (m, 2H).

### Step 4: preparation of (R)-1-(8-fluoro-7-(7-fluoro-3-(methoxymethoxy)-8-((triisopropylsilyl)ethynyl) naphthalen-1-yl)-2-((1-(hydroxymethyl)cyclopropyl)methoxy)pyrido[4,3-d]pyrimidin-4-yl)-3-methylpiperidin-3-ol

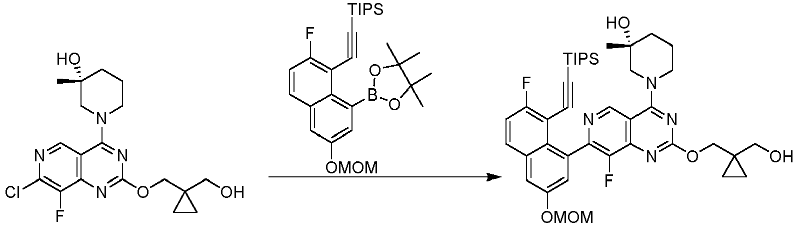

(*R*)-1-(7-chloro-8-fluoro-2-((1-(hydroxymethyl)cyclopropyl)methoxy)pyrido[4,3-*d*]pyrimidin-4-yl)-3-methylpiperidin-3-ol (85 mg, 0.214 mmol), cesium carbonate (210 mg, 0.642 mmol), ((2-fluoro-6-(methoxymethoxy)-8-(4,4,5,5-tetramethyl-1,3,2-dioxaborolan-2-yl)naphthalen-1-yl)ethynyl)triisopropylsilane, and cataXium A Pd G3 (31 mg, 0.043 mmol) were added to 1,4-dioxane/H₂O (2.5 mL/0.5 mL). The mixture was reacted at 80 °C overnight under N₂ atmosphere. The mixture was filtered and concentrated, and the resulting crude product was purified by silica gel column chromatography (DCM:MeOH = 100:1 to 10:1) to give (*R*)-1-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido[4,3-*d*]pyrimidin-4-yl)-3-methylpiperidin-3-ol.

LC-MS: (ESI, m/z): [M+H]⁺ = 747.4.

### Step 5: preparation of (R)-1-(((8-fluoro-7-(7-fluoro-3-(methoxymethoxy)-8-((triisopropylsilyl)ethynyl) naphthalen-1-yl)-4-(3-hydroxy-3-methylpiperidin-1-yl)pyrido[4,3-d]pyrimidin-2-yl)oxy)methyl) cyclopropane-1-carbaldehyde

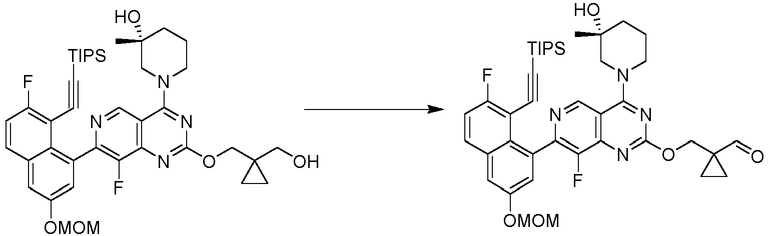

A solution of (*R*)-1-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido[4,3-*d*]pyrimidin-4-yl)-3-methylpiperidin-3-ol (80 mg, 0.107 mmol) in DCM (2 mL) was cooled to 0 °C, and then Dess-Martin oxidant (91 mg, 0.214 mmol) was added. The mixture was reacted at room temperature for 0.5 h. After the reaction was completed, a saturated sodium thiosulfate solution (5 mL) was added to quench the reaction, and then the mixture was extracted with dichloromethane (10 mL × 3). The organic phases were combined, dried over anhydrous sodium sulfate, and then concentrated under reduced pressure. The concentrate was purified by silica gel column chromatography (DCM:MeOH = 10:1) to give (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde.

LC-MS: (ESI, m/z): [M+H]⁺ = 745.4.

### Intermediate 3: (R)-1-(((7-(8-ethyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropane-1-carbaldehyde

### Step 1: preparation of (R)-1-(((7-(8-ethynyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropane-1-carbaldehyde

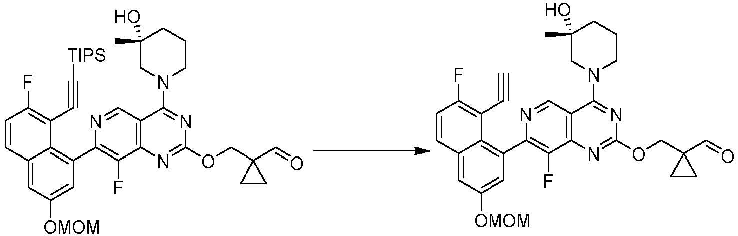

CsF (143 mg, 0.94 mmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde (140 mg, 0.188 mmol) in DMF (2 mL) at room temperature, and the mixed solution was reacted at room temperature for 30 min. After the reaction was completed, water was added, and the mixture was extracted with ethyl acetate. The organic phase was concentrated and washed with saturated brine. The crude product was purified by silica gel column chromatography (DCM:MeOH = 50:1 to 10:1) to give (*R*)-1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde.

LC-MS: (ESI, m/z): [M+H]⁺ = 589.2.

### Step 2: preparation of (R)-1-(((7-(8-ethyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropane-1-carbaldehyde

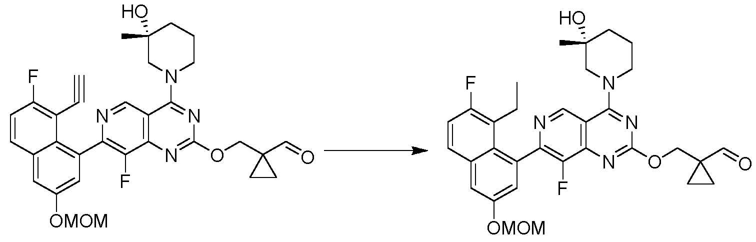

Pd/C (50 mg) was added to a solution of (*R*)-1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde (95 mg, 0.161 mmol) in MeOH (2 mL), and the mixture was stirred at 30 °C for 3 h. The mixture was filtered and concentrated to give (*R*)-1-(((7-(8-ethyl-7-fluoro-3-(methoxymethoxy)naphthalen-1-yl)-8-fluoro-4-(3-hydroxy-3-methylpiperidin-1-yl)pyrido [4,3-*d*]pyrimidin-2-yl)oxy)methyl)cyclopropane-1-carbaldehyde. The crude product was directly used in the next step without purification.

LC-MS: (ESI, m/z): [M+H]⁺ = 593.2.

### Intermediate 4: 1-(2-chloro-5-(4-((2-(piperidin-4-yl)ethoxy)methyl)piperidine-1-carbonyl)phenyl) dihydropyrimidine-2,4(1H,3H)-dione

### Step 1: preparation of tert-butyl 4-(2-(pyridin-4-ylmethoxy)ethyl)piperidine-1-carboxylate

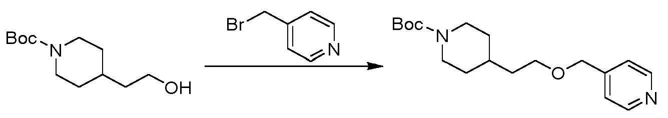

NaH (1.75 g, 43.6 mmol, 60%) was added to a solution of *tert*-butyl 4-(2-hydroxyethyl)piperidine-1-carboxylate (2 g, 8.72 mmol) in THF (10 mL) at 0 °C. The mixture was stirred at room temperature for 0.5 h, and then 4-(bromomethyl)pyridine (3.4 g, 13.08 mmol) was added. The reaction liquid was stirred at room temperature for 12 h. An aqueous ammonium chloride solution was added to quench the reaction liquid, and the mixture was extracted with ethyl acetate. The organic phase was dried and concentrated, and the resulting crude product was purified by silica gel column chromatography (DCM:MeOH = 100:1) to give *tert*-butyl 4-(2-(pyridin-4-ylmethoxy)ethyl)piperidine-1-carboxylate.

LC-MS: (ESI, m/z): [M+H]⁺= 321.2.

### Step 2: preparation of tert-butyl 4-(2-(piperidin-4-ylmethoxy)ethyl)piperidine-1-carboxylate

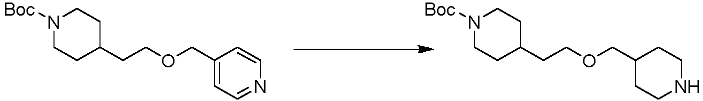

Pd/C (500 mg) was added to a solution of *tert*-butyl 4-(2-(pyridin-4-ylmethoxy)ethyl)piperidine-1-carboxylate (2.4 g, 7.49 mmol) in *i*-PrOH/H₂O (20 mL/20 mL), and the mixture was stirred at 75 °C for 16 h under hydrogen atmosphere. The reaction liquid was filtered through celite, and the filtrate was concentrated under reduced pressure. The resulting crude product was purified by silica gel column chromatography (MeOH:DCM = (0-1):4) to give *tert*-butyl 4-(2-(piperidin-4-ylmethoxy)ethyl)piperidine-1-carboxylate.

¹H NMR (400 MHz, CDCl₃) δ 4.05 (s, 1H), 3.44 (t, *J =* 6.3 Hz, 2H), 3.23 (d, *J =* 6.0 Hz, 2H), 3.11 - 3.04 (m, 2H), 2.74 - 2.55 (m, 4H), 1.93 (s, 2H), 1.75 - 1.62 (m, 5H), 1.59 - 1.42 (m, 12H), 1.19 - 1.04 (m, 4H).

### Step 3: preparation of tert-butyl 4-(2-((1-(4-chloro-3-(2,4-dioxotetrahydropyrimidin-1(2H)-yl)benzoyl)piperidin-4-yl)methoxy)ethyl)piperidine-1-carboxylate

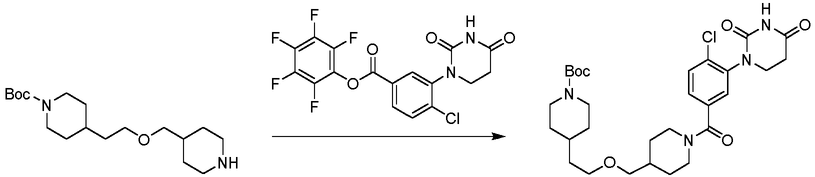

Pentafluorophenyl 4-chloro-3-(2,4-dioxotetrahydropyrimidin-1(2*H*)-yl)benzoate (0.28 g, 0.64 mmol) and DIEA (0.24 g, 1.84 mmol) were added to a solution of *tert-butyl* 4-(2-(piperidin-4-ylmethoxy)ethyl)piperidine-1-carboxylate (200 mg, 0.61 mmol) in DMSO (5 mL). The mixture was stirred at room temperature for 1 h. The crude product was purified by Pre-HPLC (CAN/H₂O = 0%-100%) to give *tert*-butyl 4-(2-((1-(4-chloro-3-(2,4-dioxotetrahydropyrimidin-1(2*H*)-yl)benzoyl)piperidin-4-yl)methoxy)ethyl)piperidine-1-carboxylate.

LC-MS: (ESI, m/z): [M+Na]⁺ = 599.2.

### Step 4: preparation of 1-(2-chloro-5-(4-((2-(piperidin-4-yl)ethoxy)methyl)piperidine-1-carbonyl)phenyl) dihydropyrimidine-2,4(1H,3H)-dione

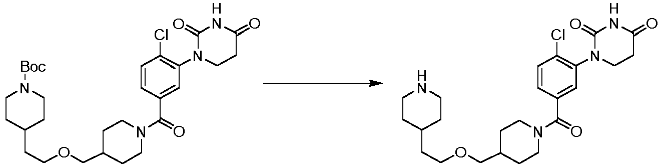

TFA (2 mL) was added to a solution of *tert-butyl* 4-(2-((1-(4-chloro-3-(2,4-dioxotetrahydropyrimidin-1(2*H*)-yl)benzoyl)piperidin-4-yl)methoxy)ethyl)piperidine-1-carboxylate (120 mg, 0.21 mmol) in DCM (4 mL). The mixture was stirred at room temperature for 1 h. The reaction liquid was directly concentrated under reduced pressure to give 1-(2-chloro-5-(4-((2-(piperidin-4-yl)ethoxy)methyl)piperidine-1-carbonyl)phenyl) dihydropyrimidine-2,4(1*H*,3*H*)-dione. The resulting crude product was directly used in the next step.

LC-MS: (ESI, m/z): [M+H]⁺= 477.2.

### Intermediate 5: preparation of 1-(2-chloro-5-(9-(piperazin-1-ylmethyl)-3-azaspiro[5.5]undecane-3-carbonyl)phenyl)dihydropyrimidine-2,4(1H,3H)-dione

### Step 1: preparation of (9H-fluoren-9-yl)methyl 4-((3-azaspiro[5.5]undecan-9-yl)methyl)piperazine-1-carboxylate

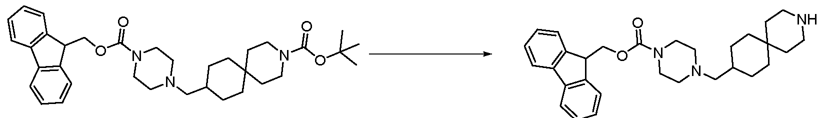

A solution of *tert-butyl* 9-((4-(((9*H*-fluorenyl-9-yl)methoxy)carbonyl)piperazin-1-yl)methyl)-3-azaspiro[5.5]
undecane-3-carboxylate (500 mg, 0.87 mmol) in HCl/1,4-dioxane (4 M, 3 mL) was stirred at room temperature for 1 h. The reaction liquid was concentrated under reduced pressure to give (9*H*-fluoren-9-yl)methyl 4-((3-azaspiro[5.5]undecan-9-yl)methyl)piperazine-1-carboxylate. The resulting product was directly used in the next step.

LC-MS: (ESI, *m*/*z*): [M+H]⁺= 474.2.

### Step 2: preparation of (9H-fluoren-9-yl)methyl 4-((3-(4-chloro-3-(2,4-dioxotetrahydropyrimidin-1(2H)-yl)benzoyl)-3-azaspiro[5.5]undecan-9-yl)methyl)piperazine-1-carboxylate

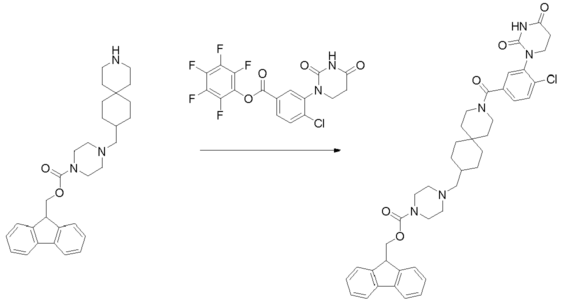

(9*H*-fluorenyl-9-yl)methyl 4-((3-azaspiro[5.5]undecan-9-yl)methyl)piperazine-1-carboxylate (400 mg, 0.84 mmol) was dissolved in DMSO (4 mL), and then pentafluorophenyl 4-chloro-3-(2,4-dioxotetrahydropyrimidin-1(2*H*)-yl)benzoate (367 mg, 0.84 mmol) and DIEA (325 mg, 2.5 mmol) were added to the solution. The reaction liquid was stirred at room temperature for 16 h. The reaction liquid was poured into water, extracted with EA (20 mL × 3), dried over anhydrous Na₂SO₄ and concentrated under reduced pressure. The resulting crude product was purified by column chromatography (DCM:MeOH = 19:1) to give ((9*H*-fluoren-9-yl)methyl 4-((3-(4-chloro-3-(2,4-dioxotetrahydropyrimidin-1(2*H*)-yl)benzoyl)-3-azaspiro[5.5]
undecan-9-yl)methyl)piperazine-1-carboxylate.

LC-MS: (ESI, *m*/*z*): [M+H]⁺= 724.3.

### Step 3: preparation of 1-(2-chloro-5-(9-(piperazin-1-ylmethyl)-3-azaspiro[5.5]undecane-3-carbonyl)phenyl) dihydropyrimidine-2,4(1H,3H)-dione

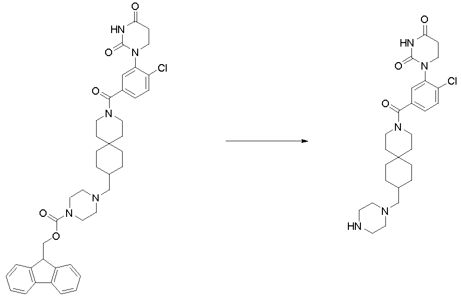

Piperidine (587 mg, 6.9 mmol) was added to a solution of ((9*H*-fluoren-9-yl)methyl 4-((3-(4-chloro-3-(2,4-dioxotetrahydropyrimidin-1(2*H*)-yl)benzoyl)-3-azaspiro[5.5]undecan-9-yl)methyl)piperazine-1-carboxylate (500 mg, 0.69 mmol) in DCM (5 mL). The reaction liquid was stirred at room temperature for 3 h. The reaction liquid was poured into water, extracted with DCM (20 mL × 3), dried over anhydrous Na₂SO₄ and concentrated under reduced pressure. The resulting crude product was purified by column chromatography (DCM:MeOH = 19:1) to give 1-(2-chloro-5-(9-(piperazin-1-ylmethyl)-3-azaspiro[5.5]undecane-3-carbonyl)phenyl)
dihydropyrimidine-2,4(1H,3H)-dione.

LC-MS: (ESI, *m*/*z*): [M+H]⁺ = 502.4.

### Intermediate 6: preparation of 1-(2-methoxy-5-(9-(piperazin-1-ylmethyl)-3-azaspiro[5.5]undecane-3-carbonyl) phenyl)dihydropyrimidine-2,4(1H,3H)-dione

### Step 1: preparation of tert-butyl 9-((4-((benzyloxy)carbonyl)piperazin-1-yl)methyl)-3-azaspiro[5.5]undecane-3-carboxylate

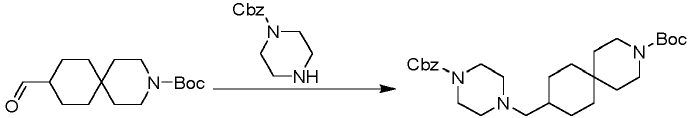

AcOH (0.2 mL) was added to a mixed solution of *tert-butyl* 9-formyl-3-azaspiro[5.5]undecane-3-carboxylate (1.30 g, 4.62 mmol) and *tert*-butyl piperazine-1-carboxylate (1.82 g, 8.28 mmol) in DCM/MeOH (15 mL/5 mL), and the mixture was stirred at room temperature for 0.5 h. NaBH(OAc)₃ (2.25 g, 10.60 mmol) was then added in batches to the above mixture at 0-5 °C, and the mixture was stirred for another 0.5 h. The reaction liquid was directly concentrated under reduced pressure, and the resulting crude product was purified by silica gel column chromatography (MeOH:DCM = 150:1 to 20:1) to give *tert*-butyl 9-((4-((benzyloxy)carbonyl)piperazin-1-yl)methyl)-3-azaspiro[5.5]undecane-3-carboxylate.

LC-MS: (ESI, m/z): [M+H] ⁺= 486.3.

### Step 2: preparation of benzyl 4-((3-azaspiro[5.5]undecan-9-yl)methyl)piperazine-1-carboxylate

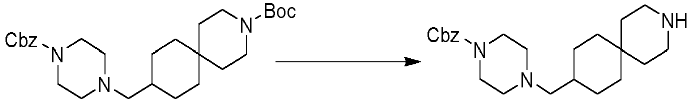

HCl/1,4-dioxane (6 N, 5 mL) was added to a solution of *tert*-butyl 9-((4-((benzyloxy)carbonyl)piperazin-1-yl)methyl)-3-azaspiro[5.5]undecane-3-carboxylate (750 mg, 4.54 mmol) in 1,4-dioxane (5 mL). The mixture was stirred at room temperature for 1 h, and the reaction liquid was directly concentrated under reduced pressure to give benzyl 4-((3-azaspiro[5.5]undecan-9-yl)methyl)piperazine-1-carboxylate. The resulting crude product was directly used in the next step without purification.

### Step 3: preparation of benzyl 4-((3-(3-(2,4-dioxotetrahydropyrimidin-1(2H)-yl)-4-methoxybenzoyl)-3-azaspiro[5.5]undecan-9-yl)methyl)piperazine-1-carboxylate

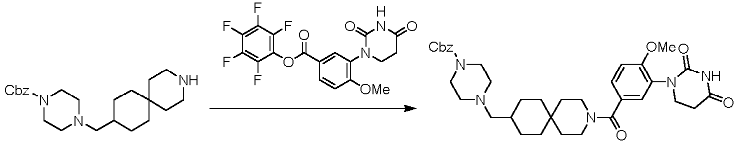

Pentafluorophenyl 3-(2,4-dioxotetrahydropyrimidin-1(2*H*)-yl)-4-methoxybenzoate (430 mg, 1.00 mmol) and DIEA (516 mg, 4.00 mmol) were added to a solution of benzyl 4-((3-azaspiro[5.5]undecan-9-yl)methyl)piperazine-1-carboxylate (632 mg, crude) in DMSO (10 mL). The mixture was stirred at room temperature for 1 h and then purified by pre-HPLC to give benzyl 4-((3-(3-(2,4-dioxotetrahydropyrimidin-1(2*H*)-yl)-4-methoxybenzoyl)-3-azaspiro[5.5]undecan-9-yl)methyl)piperazine-1-carboxylate.

LC-MS: (ESI, m/z): [M+H] ⁺= 632.3.

### Step 4: preparation of 1-(2-methoxy-5-(9-(piperazin-1-ylmethyl)-3-azaspiro[5.5]undecane-3-carbonyl)phenyl) dihydropyrimidine-2,4(1H,3H)-dione

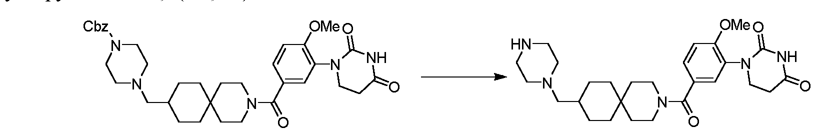

Pd/C (300 mg) was added to a solution of benzyl 4-((3-(3-(2,4-dioxotetrahydropyrimidin-1(2H)-yl)-4-methoxybenzoyl)-3-azaspiro[5.5]undecan-9-yl)methyl)piperazine-1-carboxylate (586 mg, 0.93 mmol) in ethyl acetate (20 mL), and the mixture was stirred at room temperature for 16 h. The reaction liquid was filtered through celite, and the filtrate was concentrated under reduced pressure to give 1-(2-methoxy-5-(9-(piperazin-1-ylmethyl)-3-azaspiro[5.5]undecane-3-carbonyl)phenyl)dihydropyrimidine-2,4(1*H*,3*H*)-dione.

LC-MS: (ESI, m/z): [M+H] ⁺= 498.2.

### Intermediate 7: 1-(2-chloro-5-(4-(2-(piperidin-4-ylmethoxy)ethyl)piperidine-1-carbonyl)phenyl) dihydropyrimidine-2,4(1H,3H)-dione

### Step 1: preparation of tert-butyl 4-(2-((1-benzylpiperidin-4-yl)methoxy)ethyl)piperidine-1-carboxylate

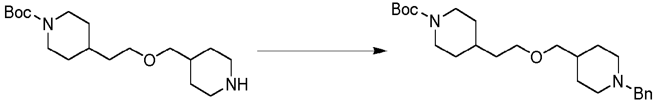

Acetic acid (0.01 mL) was added to a mixed solution of *tert-butyl* 4-(2-(piperidin-4-ylmethoxy)ethyl)piperidine-1-carboxylate (100 mg, 0.31 mmol) and benzaldehyde (39 mg, 0.37 mmol) in 1,2-dichloroethane/methanol (0.5 mL/0.5 mL). The mixture was stirred at 30 °C for 1 h. NaBH₃CN (39 mg, 0.62 mmol) was added at 0 °C, and the mixture was reacted at room temperature for 2 h. After the reaction was completed, H₂O (10 mL) was added, and the mixture was extracted with ethyl acetate (30 mL × 3). The combined organic phase was dried over anhydrous sodium sulfate, filtered, and concentrated in vacuum. The crude product was purified by silica gel column chromatography (DCM:MeOH = 20:1) to give *tert*-butyl 4-(2-((1-benzylpiperidin-4-yl)methoxy)ethyl)piperidine-1-carboxylate.

LC-MS: (ESI, m/z): [M+H]⁺ = 417.3.

### Step 2: preparation of 1-benzyl-4-((2-(piperidin-4-yl)ethoxy)methyl)piperidine

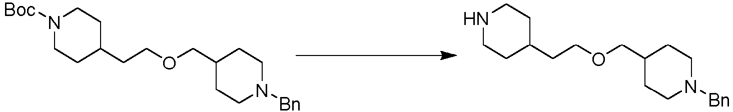

TFA (1 mL) was added to a solution of *tert-butyl* 4-(2-((1-benzylpiperidin-4-yl)methoxy)ethyl)piperidine-1-carboxylate (100 mg, 0.24 mmol) in DCM (2 mL). The mixture was stirred at room temperature for 1 h, and the reaction liquid was directly concentrated under reduced pressure to give 1-benzyl-4-((2-(piperidin-4-yl)ethoxy)methyl)piperidine, which was directly used in the next step.

### Step 3: preparation of 1-(5-(4-(2-((1-benzylpiperidin-4-yl)methoxy)ethyl)piperidine-1-carbonyl)-2-chlorophenyl) dihydropyrimidine-2,4(1H,3H)-dione

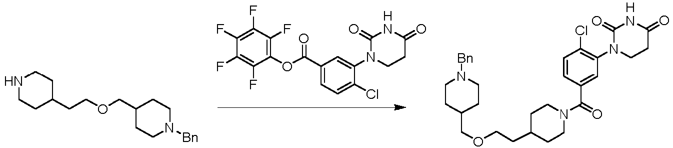

1- Benzyl-4-((2-(piperidin-4-yl)ethoxy)methyl)piperidine (100 mg, 0.32 mmol) was dissolved in DMSO (2 mL), and pentafluorophenyl 4-chloro-3-(2,4-dioxotetrahydropyrimidin-1(2*H*)-yl)benzoate (110 mg, 0.34 mmol) and DIEA (93 mg, 0.96 mmol) were added. The reaction liquid was stirred at room temperature for 2 h. Water (20 mL) was added, and the mixture was extracted with ethyl acetate (20 mL × 2). The organic phases were combined, washed with saturated brine (30 mL × 3), dried over anhydrous sodium sulfate, and filtered. The filtrate was concentrated and purified by silica gel column chromatography (DCM:MeOH = 100:1 to 5:1) to give 1-(5-(4-(2-((1-benzylpiperidin-4-yl)methoxy)ethyl)piperidine-1-carbonyl)-2-chlorophenyl)
dihydropyrimidine-2,4(1*H*,3*H*)-dione.

LC-MS: (ESI, m/z): [M+H]⁺= 567.2.

### Step 4: preparation of 1-(2-chloro-5-(4-(2-(piperidin-4-ylmethoxy)ethyl)piperidine-1-carbonyl)phenyl) dihydropyrimidine-2,4(1H,3H)-dione

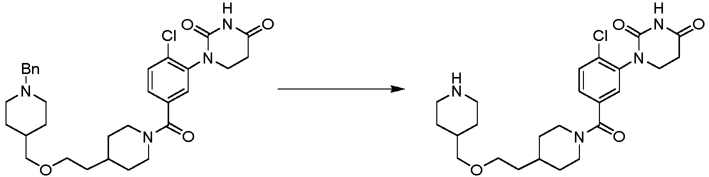

1-1-(5-(4-(2-((1-Benzylpiperidin-4-yl)methoxy)ethyl)piperidine-1-carbonyl)-2-chlorophenyl)dihydropyrimidine-2,4(1*H*,3*H*)-dione (200 mg, 0.35 mmol) was dissolved in DCM (2 mL), and 1-chloroethyl chloroformate (150 mg, 1.05 mmol) and DIEA (90 mg, 0.7 mmol) were added. The mixture was reacted at room temperature for 30 min and concentrated under reduced pressure. MeOH (2 mL) was added to the concentrate for dissolution, and the mixture was heated to 50 °C and reacted for 30 min. After the reaction was completed, the mixture was concentrated and purified by silica gel column chromatography (DCM:MeOH (NH₃) = 100:1 to 9:1) to give 1-(2-chloro-5-(4-(2-(piperidin-4-ylmethoxy)ethyl)piperidine-1- carbonyl)phenyl)dihydropyrimidine-2,4(1*H*,3*H*)-dione.

LC-MS: (ESI, m/z): [M+H]⁺ = 477.3.

### Intermediate 8: tert-butyl 4-((1-(hydroxymethyl)cyclopropyl)methyl)piperidine-1-carboxylate

### Step 1: preparation of methyl 4-hydroxy-2-methylenebutanoate

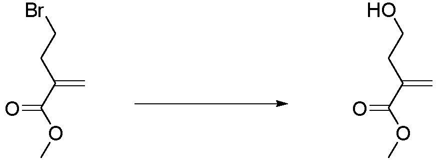

Indium powder (3.53 g, 30.74 mmol) was added to a solution of methyl 4-bromo-2-methylenebutanoate (5.00 g, 27.93 mmol) and 37% aqueous formaldehyde (4.53 g, 55.86 mmol) in ethanol (50 mL) and water (50 mL) at room temperature, and the mixture was stirred at 25 °C for 24 h under nitrogen atmosphere. Dichloromethane (200 mL) and water (200 mL) were added to the reaction liquid, and the mixture was stirred for 30 min and filtered through celite. The organic phase was washed with saturated brine (100 mL), and the aqueous phase was extracted with dichloromethane (200 mL × 2). The organic phase was dried over anhydrous sodium sulfate, filtered, and concentrated under reduced pressure to give methyl 4-hydroxy-2-methylenebutanoate.

LC-MS: (ESI, m/z): [M+H]⁺ = 131.2.

### Step 2: preparation of methyl 4-chloro-2-methylenebutanoate

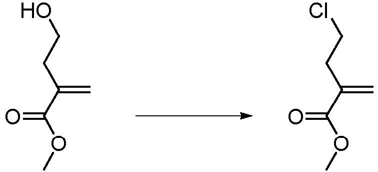

Pyridine (2.4 mL) and thionyl chloride (4.5 mL, 59.93 mmol) were added to a solution of methyl 4-hydroxy-2-methylenebutanoate (3.05 g, 23.46 mmol) in dichloromethane (120 mL) at room temperature. After the system was degassed, the mixture was stirred at 50 °C for 18 h under nitrogen atmosphere. Petroleum ether (100 mL) was added to the reaction liquid, and then the mixture was poured into water (100 mL) and extracted with dichloromethane (20 mL × 2). The combined organic phase was washed with saturated sodium chloride (100 mL), dried over anhydrous sodium sulfate, filtered, and concentrated under reduced pressure. The crude product was purified by silica gel column chromatography (petroleum ether:ethyl acetate = 100:1 to 10:1) to give methyl 4-chloro-2-methylenebutanoate.

¹H NMR (400 MHz, CDCl₃) δ 6.31 (s, 1H), 5.71 (s, 1H), 3.77 (s, 3H), 3.67 (t, *J =* 6.8 Hz, 2H), 2.77 (t, *J* = 6.8 Hz, 2H).

### Step 3: preparation of tert-butyl 4-((1-(methoxycarbonyl)cyclopropyl)methyl)piperidine-1-carboxylate

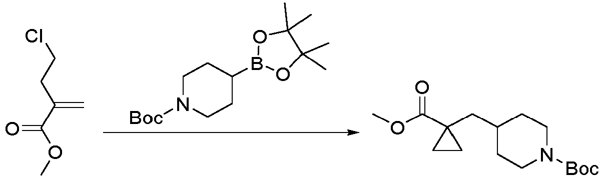

Phenyllithium (1.0 M, 10 mL, 10.00 mmol) was added to a solution of *tert-butyl* 4-(4,4,5,5-tetramethyl-1,3,2-dioxaborolan-2-yl)piperidine-1-carboxylate (2.87 g, 9.22 mmol) in tetrahydrofuran (30 mL) at 0 °C, and the mixture was stirred at 25 °C for 10 min. The mixture was then concentrated under reduced pressure, and the residue was dissolved in N,N-dimethylformamide (30 mL). Methyl 4-chloro-2-methylenebutanoate (1.25 g, 8.38 mmol) and 2,4,5,6-tetrakis(9-carbazolyl)-isophthalonitrile (331 mg, 0.42 mmol) were added, and the mixture was stirred at 25 °C for 24 h under nitrogen atmosphere. The reaction liquid was poured into water (300 mL), and the mixture was extracted with ethyl acetate (50 mL × 3). The combined organic phase was separately washed with water (100 mL) and saturated sodium chloride (100 mL), and the organic phase was dried over anhydrous sodium sulfate, filtered and concentrated under reduced pressure. The crude product was purified by silica gel column chromatography (petroleum ether:ethyl acetate = 100:1 to 10:1) to give *tert*-butyl 4-((1-(methoxycarbonyl)cyclopropyl)methyl)piperidine-1-carboxylate.

LC-MS: (ESI, m/z): [M-tBu+H]⁺ = 242.2.

### Step 4: preparation of tert-butyl 4-((1-(hydroxymethyl)cyclopropyl)methyl)piperidine-1-carboxylate

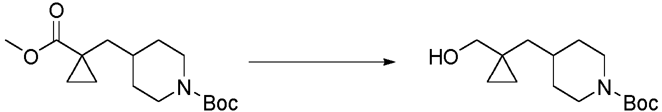

Lithium aluminum hydride (1.0 M, 7.3 mL, 7.3 mmol) was added to a solution of *tert*-butyl 4-((1-(methoxycarbonyl)cyclopropyl)methyl)piperidine-1-carboxylate (1.69 mg, 5.69 mmol) in tetrahydrofuran (90 mL) at 0 °C. The mixture was stirred at 0 °C for 1 h under nitrogen atmosphere. After the reaction was completed, a saturated potassium sodium tartrate solution (1 mL) was added to quench the reaction, and the mixture was dried over anhydrous sodium sulfate, filtered, and concentrated under reduced pressure to give a crude product of *tert-butyl* 4-((1-(hydroxymethyl)cyclopropyl)methyl)piperidine-1-carboxylate, which was directly used in the next step.

### Intermediate 9: (R)-1-(7-bromo-8-fluoro-2-((1-(hydroxymethyl)cyclopropyl)methoxy)quinazolin-4-yl)-3-methylpiperidin-3-ol

### Step 1: preparation of (R)-1-(7-bromo-2-chloro-8-fluoroquinazolin-4-yl)-3-methylpiperidin-3-ol

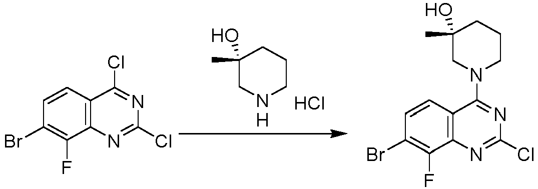

TEA (695 mg, 6.87 mmol) was added to a solution of 7-bromo-2,4-dichloro-8-fluoroquinazoline (678 mg, 2.29 mmol) in THF (30 mL) at -50 °C. (*R*)-3-methylpiperidin-3-ol hydrochloride (347 mg, 2.29 mmol) was added, and the mixture was stirred for 5 h. H₂O (50 mL) was added to the reaction liquid, and the mixture was then extracted with EtOAc (30 mL × 3). The organic phases were combined, dried over anhydrous sodium sulfate, and concentrated under reduced pressure, and the concentrate was purified by silica gel column chromatography (PE:EtOAc = 5:1) to give (*R*)-1-(7-bromo-2-chloro-8-fluoroquinazolin-4-yl)-3-methylpiperidin-3-ol.

LC-MS: (ESI, m/z): [M+H]⁺ = 375.8.

### Step 2: preparation of (R)-1-(7-bromo-8-fluoro-2-((1-(hydroxymethyl)cyclopropyl)methoxy)quinazolin-4-yl)-3-methylpiperidin-3-ol

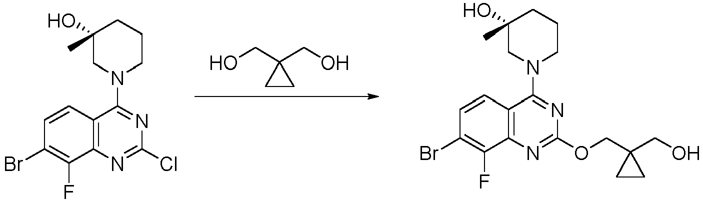

Triethylenediamine (90 mg, 0.80 mmol) and anhydrous cesium carbonate (783 mg, 22.40 mmol) were added to a solution of (*R*)-1-(7-bromo-2-chloro-8-fluoroquinazolin-4-yl)-3-methylpiperidin-3-ol and cyclopropane-1,1-dimethanol (327 mg, 3.20 mmol) in anhydrous acetonitrile (30 mL), and the mixture was stirred at 25 °C for 18 h. After the reaction was completed, the mixture was poured into water (100 mL) and extracted with ethyl acetate (20 mL × 2). The organic phases were combined, washed with saturated sodium chloride (100 mL), dried over anhydrous sodium sulfate, and concentrated under reduced pressure. The resulting crude product was purified by silica gel column chromatography (dichloromethane:methanol = 100:1 to 15:1) to give (R)-1-(7-bromo-8-fluoro-2-((1-(hydroxymethyl)cyclopropyl)methoxy)quinazolin-4-yl)-3-methylpiperidin-3-ol.

LC-MS: (ESI, m/z): [M+H]⁺ = 439.8.

### Intermediate 10: (R)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropane-1-carbaldehyde

### Step 1: preparation of (R)-1-(8-fluoro-7-(7-fluoro-3-(methoxymethoxy)-8-((triisopropylsilyl)ethynyl) naphthalen-1-yl)-2-((1-(hydroxymethyl)cyclopropyl)methoxy)quinazolin-4-yl)-3-methylpiperidin-3-ol

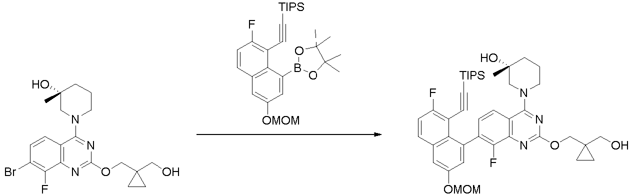

Anhydrous cesium carbonate (1344 mg, 4088.01 µmol) and cataCXium(R) A Pd G3 (149 mg, 204.40 µmol) were added to a solution of (*R*)-1-(7-bromo-8-fluoro-2-((1-(hydroxymethyl)cyclopropyl)methoxy)quinazolin-4-yl)-3-methylpiperidin-3-ol (600 mg, 1362.67 µmol) and ((2-fluoro-6-(methoxymethoxy)-8-(4,4,5,5-tetramethyl-1,3,2-dioxaborolan-2-yl)naphthalen-1-yl)ethynyl)triisopropylsilane (838 mg, 1635.21 µmol) in 1,4-dioxane (40 mL) and water (10 mL) at room temperature. The mixture was stirred at 75 °C for 18 h under nitrogen atmosphere. The reaction liquid was poured into water (100 mL), and the mixture was extracted with ethyl acetate (20 mL × 2). The combined organic phase was washed with saturated sodium chloride (100 mL), dried over anhydrous sodium sulfate, filtered and concentrated under reduced pressure. The crude product was purified by silica gel column chromatography (dichloromethane:methanol = 80:1, 0.1% NH₃) to give (*R*)-1-(8-fluoro-7-(7-fluoro-3-(methoxymethoxy)-8-((triisopropylsilyl)ethynyl)naphthalen-1-yl)-2-((1-(hydroxymethyl) cyclopropyl)methoxy)quinazolin-4-yl)-3-methylpiperidin-3-ol.

LC-MS: (ESI, m/z): [M+H]⁺ = 746.4.

### Step 2: preparation of (R)-1-(((8-fluoro-7-(7-fluoro-3-(methoxymethoxy)-8-((triisopropylsilyl)ethynyl) naphthalen-1-yl)-4-(3-hydroxy-3-methylpiperidin-1-yl)quinazolin-2-yl)oxy)methyl)cyclopropane-1-carbaldehyde

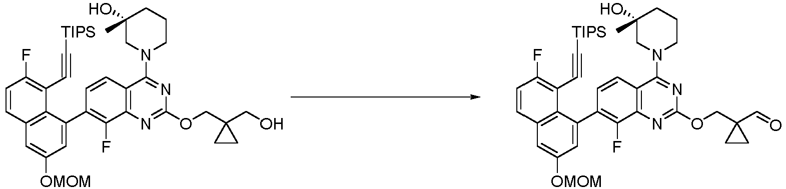

A solution of (R)-1-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)quinazolin-4-yl)-3-methylpiperidin-3-ol (752 mg, 1.008 mmol) in dichloromethane (30 mL) was cooled to 0 °C from room temperature, and Dess-Martin reagent (555 mg, 1.309 mmol) was added. The mixture was stirred at 25 °C for 1 h. After the reaction was completed, the mixture was poured into a saturated aqueous sodium bicarbonate solution (100 mL), and extracted with dichloromethane (20 mL × 2). The combined organic phase was washed with saturated sodium chloride (100 mL), dried over anhydrous sodium sulfate, filtered and concentrated under reduced pressure. The crude product was purified by silica gel column chromatography (petroleum ether:ethyl acetate =10:1 to 1/1, 0.1% NH₃) to give (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropane-1-carbaldehyde.

LC-MS: (ESI, m/z): [M+H]⁺ = 743.9.

### Intermediate 11: 3-(4-chloro-3-(2,4-dioxotetrahydropyrimidin-1(2H)-yl)benzoyl)-3-azaspiro[5.5]undecane-9-carbaldehyde

### Step 1: preparation of 3-azaspiro[5.5]undecane-9-carbaldehyde

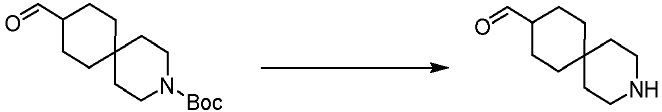

*tert-Butyl* 9-formyl-3-azaspiro[5.5]undecane-3-carboxylate (400 mg, 1.42 mmol) was dissolved in DCM (4 mL), and then TFA (2 mL) was added. The mixture was stirred at room temperature for 1 h and concentrated to give 3-azaspiro[5.5]undecane-9-carbaldehyde trifluoroacetate, which was directly used in the next step.

### Step 2: preparation of 3-(4-chloro-3-(2,4-dioxotetrahydropyrimidin-1(2H)-yl)benzoyl)-3-azaspiro[5.5] undecane-9-carbaldehyde

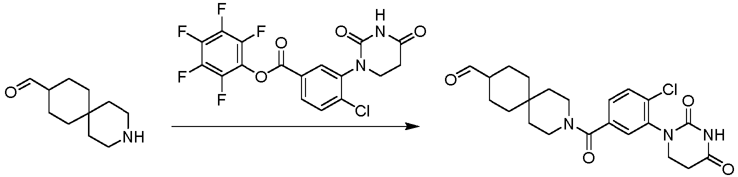

DIEA (720 mg, 5.61 mmol) and pentafluorophenyl 4-chloro-3-(2,4-dioxotetrahydropyrimidin-1(2*H*)-yl)benzoate (850 mg, 1.96 mmol) were added to a solution of 3-azaspiro[5.5]undecane-9-carbaldehyde (500 mg, 1.87 mmol) in DMSO (5 mL), and the reaction liquid was stirred at room temperature for 1 h. Water was added, and the mixture was extracted with ethyl acetate. The organic phases were combined, dried over anhydrous sodium sulfate, and filtered. The filtrate was concentrated and purified by silica gel column chromatography (DCM:MeOH = 100:1 to 5:1) to give 3-(4-chloro-3-(2,4-dioxotetrahydropyrimidin-1(2*H*)-yl)benzoyl)-3-azaspiro[5.5]undecane-9-carbaldehyde.

MS (ESI) m/z: [M+H]⁺ = 432.0.

### Intermediate 12: preparation of 1-(2-chloro-5-(4-(3-(piperazin-1-yl)propyl)piperidine-1-carbonyl)phenyl) dihydropyrimidine-2,4(1H,3H)-dione

### Step 1: preparation of benzyl 4-(3-hydroxypropyl)piperidine-1-carboxylate

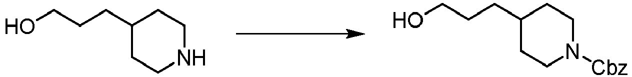

A sodium bicarbonate solution (42 mL) was added to a solution of 3-(piperidin-4-yl)propan-1-ol (3 g, 20.9 mmol) in tetrahydrofuran (12 mL). After the mixture was cooled to 0 °C, benzyl chloroformate (3.9 g, 23.0 mmol) was slowly added dropwise. The mixture was reacted at room temperature for 2 h and then extracted with ethyl acetate (30 mL × 3). The organic phase was dried, concentrated, and purified by silica gel column chromatography (PE:EA = 30:1) to give benzyl 4-(3-hydroxypropyl)piperidine-1-carboxylate.

LC-MS: (ESI, m/z): [M+H]⁺ = 278.1.

### Step 2: preparation of benzyl 4-(3-oxopropyl)piperidine-1-carboxylate

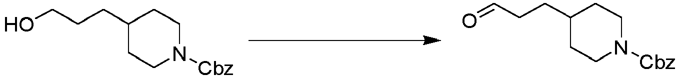

Dess-Martin (7 g, 17.3 mmol) was added to a solution of benzyl 4-(3-hydroxypropyl)piperidine-1-carboxylate (4 g, 14.4 mmol) in dichloromethane (50 mL). The mixture was stirred at room temperature for 2 h. After the reaction was completed, a sodium thiosulfate solution (50 mL) and a sodium bicarbonate solution (50 mL) were added to quench the reaction, and the mixture was extracted with dichloromethane (50 mL × 3). The organic phases were combined, dried over anhydrous sodium sulfate, and concentrated under reduced pressure. The concentrate was purified by silica gel column chromatography (PE:EA = 2:1) to give benzyl 4-(3-oxopropyl)piperidine-1-carboxylate.

LC-MS: (ESI, m/z): [M+H]⁺ = 276.1.

### Step 3: preparation of tert-butyl 4-(3-(1-((benzyloxy)carbonyl)piperidin-4-yl)propyl)piperazine-1-carboxylate

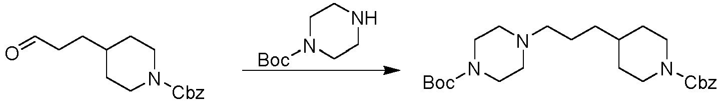

*tert-Butyl* piperazine-1-carboxylate (6.7 g, 36.0 mmol) and acetic acid (2 mL) were added to a solution of benzyl 4-(3-oxopropyl)piperidine-1-carboxylate (3.3 g, 12.0 mmol) in 1,2-dichloroethane (60 mL), and the mixture was reacted at room temperature for 2 h. After the mixture was cooled to 0 °C, sodium triacetoxyborohydride (5.3 g, 24.0 mmol) was slowly added. The mixture was reacted at room temperature for 16 h. After the reaction was completed, water (60 mL) was added to quench the reaction, and the mixture was extracted with dichloromethane (60 mL × 3). The organic phase was dried, concentrated, and purified by silica gel column chromatography (PE:EA = 1:1) to give *tert-butyl* 4-(3-(1-((benzyloxy)carbonyl)piperidin-4-yl)propyl)piperazine-1-carboxylate.

LC-MS: (ESI, m/z): [M+H]⁺ = 446.2.

### Step 4: preparation of tert-butyl 4-(3-(piperidin-4-yl)propyl)piperazine-1-carboxylate

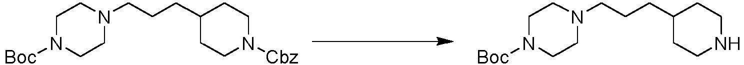

Pd/C (597 mg) was added to a solution of *tert-butyl* 4-(3-(1-((benzyloxy)carbonyl)piperidin-4-yl)propyl)piperazine-1-carboxylate (2.5 g, 5.6 mmol) in methanol (40 mL), and the mixture was stirred at 30 °C for 16 h. The mixture was filtered and concentrated to give *tert-butyl* 4-(3-(piperidin-4-yl)propyl)piperazine-1-carboxylate.

LC-MS: (ESI, m/z): [M+H]⁺ = 312.2.

### Step 5: preparation of tert-butyl 4-(3-(1-(4-chloro-3-(2,4-dioxotetrahydropyrimidin-1(2H)-yl)benzoyl) piperidin-4-yl)propyl)piperazine-1-carboxylate

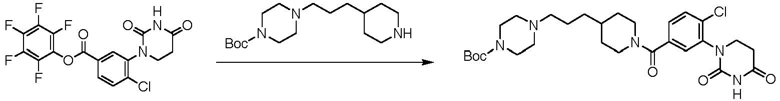

Perfluorophenyl 4-chloro-3-(2,4-dioxotetrahydropyrimidin-1(2*H*)-yl)benzoate (348 mg, 0.8 mmol) and DIEA (310 mg, 2.4 mmol) were added to a solution of *tert-butyl* 4-(3-(piperidin-4-yl)propyl)piperazine-1-carboxylate (250 mg, 0.8 mmol) in DMF (3 mL). The mixture was stirred at room temperature for 1 h. After the reaction was completed, water (5 mL) was added to quench the reaction, and the mixture was extracted with ethyl acetate (5 mL × 3). The organic phase was dried, concentrated, and purified by silica gel column chromatography (DCM:MeOH = 20:1) to give *tert-butyl* 4-(3-(1-(4-chloro-3-(2,4-dioxotetrahydropyrimidin-1(2*H*)-yl)benzoyl)piperidin-4-yl)propyl)piperazine-1-carboxylate.

LC-MS: (ESI, m/z): [M+H]⁺ = 562.2.

### Step 6: preparation of 1-(2-chloro-5-(4-(3-(piperazin-1-yl)propyl)piperidine-1-carbonyl)phenyl) dihydropyrimidine-2,4(1H,3H)-dione

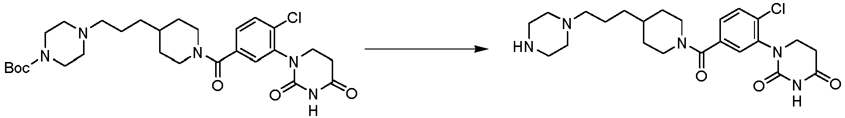

HCl/1,4-dioxane (6 N, 1 mL) was added to a solution of *tert-butyl* 4-(3-(1-(4-chloro-3-(2,4-dioxotetrahydropyrimidin-1(2*H*)-yl)benzoyl)piperidin-4-yl)propyl)piperazine-1-carboxylate (400 mg, 0.71 mmol) in 1,4-dioxane (2 mL). The mixture was stirred at room temperature for 1 h, concentrated, and purified by silica gel column chromatography (DCM:MeOH (NH₃) = 20:1) to give 1-(2-chloro-5-(4-(3-(piperazin-1-yl)propyl)piperidine-1-carbonyl)phenyl)dihydropyrimidine-2,4(1*H*,3*H*)-dione.

LC-MS: (ESI, m/z): [M+H]⁺ = 462.2.

### Intermediate 13: preparation of 1-(2-methyl-5-(9-(piperazin-1-ylmethyl)-3-azaspiro[5.5]undecane-3-carbonyl)phenyl)dihydropyrimidine-2,4(1H,3H)-dione

### Step 1: preparation of benzyl 4-((3-(3-(2,4-dioxotetrahydropyrimidin-1(2H)-yl)-4-methylbenzoyl)-3-azaspiro[5.5]undecan-9-yl)methyl)piperazine-1-carboxylate

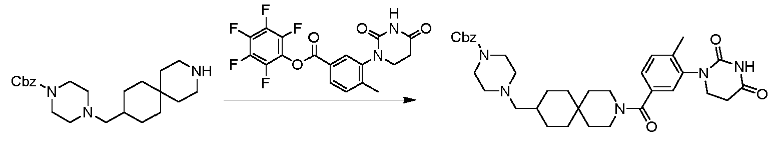

Pentafluorophenyl 3-(2,4-dioxotetrahydropyrimidin-1(2*H*)-yl)-4-methylbenzoate (921 mg, 2.3 mmol) and DIEA (995 mg, 7.7 mmol) were added to a solution of benzyl 4-((3-azaspiro[5.5]undecan-9-yl)methyl)piperazine-1-carboxylate hydrochloride (930 mg, 2.2 mmol) in DMSO (20 mL). The mixture was stirred at room temperature for 1 h and purified by Pre-HPLC to give benzyl 4-((3-(3-(2,4-dioxotetrahydropyrimidin-1(2*H*)-yl)-4-methylbenzoyl)-3-azaspiro[5.5]undecan-9-yl)methyl)piperazine-1-carboxylate.

LC-MS: (ESI, m/z): [M+H]⁺ = 616.3.

### Step 2: preparation of 1-(2-methyl-5-(9-(piperazin-1-ylmethyl)-3-azaspiro[5.5]undecane-3-carbonyl)phenyl) dihydropyrimidine-2,4(1H,3H)-dione

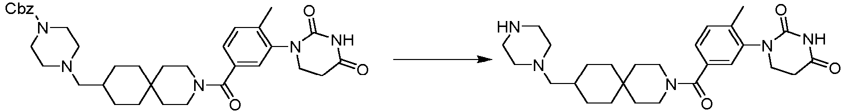

Pd/C (100 mg) was added to a solution of benzyl 4-((3-(3-(2,4-dioxotetrahydropyrimidin-1(2*H*)-yl)-4-methylbenzoyl)-3-azaspiro[5.5]undecan-9-yl)methyl)piperazine-1-carboxylate (900 mg, 1.46 mmol) in ethyl acetate (20 mL), and the mixture was stirred at room temperature for 16 h. The reaction liquid was filtered through celite, and the filtrate was concentrated to give 1-(2-methyl-5-(9-(piperazin-1-ylmethyl)-3-azaspiro[5.5]undecane-3-carbonyl)phenyl)dihydropyrimidine-2,4(1*H*,3*H*)-dione.

LC-MS: (ESI, m/z): [M+H]⁺ = 482.2.

### Intermediate 14:tert-butyl 4-(2-(piperazin-1-yl)ethyl)piperidine-1-carboxylate

### Step 1: preparation of benzyl 4-(2-(1-(tert-butoxycarbonyl)piperidin-4-yl)ethyl)piperazine-1-carboxylate

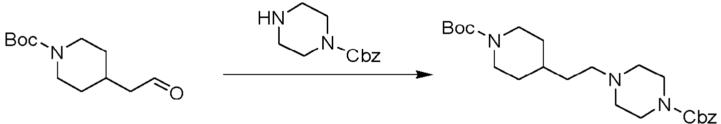

*tert-Butyl* 4-(2-oxoethyl)piperidine-1-carboxylate (2 g, 8.8 mmol) was dissolved in 1,2-dichloroethane (30 mL), and acetic acid (1 mL) and benzyl piperazine-1-carboxylate (5.8 g, 26.4 mmol) were added. The mixture was stirred at 30 °C for 2 h, and then NaBH₃CN (1.1 g, 17.6 mmol) was slowly added in an ice bath. The mixture was stirred at 30 °C for 16 h. After the reaction was completed, the mixture was diluted with water (30 mL) and extracted with ethyl acetate (50 mL × 3), dried over anhydrous Na₂SO₄, filtered, and concentrated under reduced pressure. The crude product was purified by silica gel column chromatography (DCM:MeOH = 20:1) to give benzyl 4-(2-(1-(*tert*-butoxycarbonyl)piperidin-4-yl)ethyl)piperazine-1-carboxylate.

LC-MS: (ESI, m/z): [M+H]⁺= 432.2.

### Step 2: preparation of tert-butyl 4-(2-(piperazin-1-yl)ethyl)piperidine-1-carboxylate

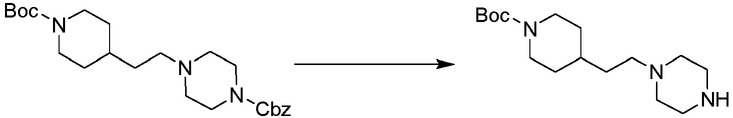

Pd/C (490 mg) was added to a solution of benzyl 4-(2-(1-(tert-butoxycarbonyl)piperidin-4-yl)ethyl)piperazine-1-carboxylate (2 g, 4.6 mmol) in methanol (10 mL), and the mixture was stirred at 30 °C for 16 h under hydrogen atmosphere. After the reaction was completed, the mixture was filtered through celite, and the filtrate was concentrated under reduced pressure to give *tert-butyl* 4-(2-(piperazin-1-yl)ethyl)piperidine-1-carboxylate. The crude product was directly used in the next step without further purification.

### Intermediate 15: tert-butyl 9-(piperazin-1-ylmethyl)-3-azaspiro[5.5]undecane-3-carboxylate

### Step 1: preparation of tert-butyl 9-((4-((benzyloxy)carbonyl)piperazin-1-yl)methyl)-3-azaspiro[5.5]undecane-3-carboxylate

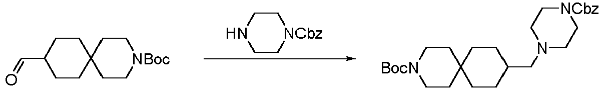

*tert*-Butyl 9-formyl-3-azaspiro[5.5]undecane-3-carboxylate (300 mg, 1.06 mmol) and benzyl piperazine-1-carboxylate (235 mg, 1.06 mmol) were dissolved in tetrahydrofuran (10 mL), and sodium triacetoxyborohydride (674 mg, 3.18 mmol) was added with stirring. The reaction liquid was stirred at room temperature for 2 h. Water (50 mL) was added, and the mixture was extracted with dichloromethane (30 mL × 3). The organic phase was washed with saturated brine (100 mL), dried over anhydrous sodium sulfate, filtered, and concentrated under reduced pressure. The crude product was purified by column chromatography (methanol:dichloromethane = 1:9) to give *tert-butyl* 9-((4-((benzyloxy)carbonyl)piperazin-1-yl)methyl)-3-azaspiro [5.5]undecane-3 -carboxylate.

LC-MS: (ESI, *m*/*z*): [M+H] ⁺ = 486.2.

### Step 2: preparation of tert-butyl 9-(piperazin-1-ylmethyl)-3-azaspiro[5.5]undecane-3-carboxylate

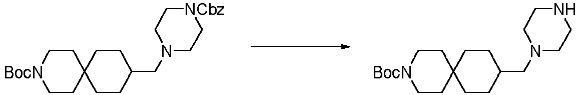

*tert*-Butyl 9-((4-((benzyloxy)carbonyl)piperazin-1-yl)methyl)-3-azaspiro[5.5]undecane-3-carboxylate (500 mg, 1.02 mmol) was dissolved in ethyl acetate (15 mL), and Pd(OH)₂/C (250 mg, 0.35 mmol) was added. The mixture was heated to 70 °C under hydrogen atmosphere and stirred overnight. The reaction liquid was cooled to room temperature and filtered, and the filtrate was concentrated under reduced pressure to give *tert*-butyl 9-(piperazin-1-ylmethyl)-3-azaspiro[5.5]undecane-3-carboxylate. The crude product was directly used in the next step without purification.

LC-MS: (ESI, *m*/*z*): [M+H] ⁺ = 352.2.

### Intermediate 16: 2-(3-(4-chloro-3-(2,4-dioxotetrahydropyrimidin-1(2H)-yl)benzoyl)-3-azaspiro[5.5]undecan-9-yl)acetaldehyde

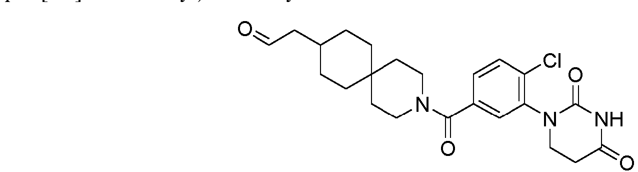

The intermediate was prepared with reference to intermediate 11.

### Intermediate 17: 1-(2-chloro-5-(piperazine-1-carbonyl)phenyl)dihydropyrimidine-2,4(1H,3H)-dione

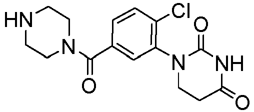

The intermediate was prepared with reference to Example 40 in the patent WO2023025159A1.

### Intermediate 18: 3-(3-(2,4-dioxotetrahydropyrimidin-1(2H)-yl)-4-methylbenzoyl)-3-azaspiro [5 .5]undecane-9-carbaldehyde

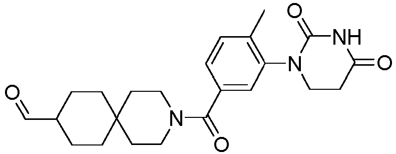

The intermediate was prepared with reference to Example 32 in the patent WO2023025159A1.

### Intermediate 19: (R)-1-(8-fluoro-7-(7-fluoro-3-(methoxymethoxy)-8-((triisopropylsilyl)ethynyl)naphthalen-1-yl)-2-((R)-2-methyl-3-(piperazin-1-yl)propoxy)pyrido[4,3-d]pyrimidin-4-yl)-3-methylpiperidin-3-ol

### Step 1: preparation of (R)-1-(2-((R)-3-(4-benzylpiperazin-1-yl)-2-methylpropoxy)-7-chloro-8-fluoropyrido [4,3-d]pyrimidin-4-yl)-3-methylpiperidin-3-ol

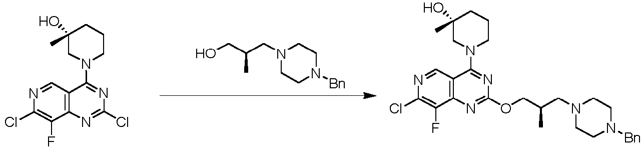

(*R*)-1-(2,7-dichloro-8-fluoropyrido[4,3-*d*]pyrimidin-4-yl)-3-methylpiperidin-3-ol (3.6 g, 10.87 mmol) and (*R*)-3-(4-benzylpiperazin-1-yl)-2-methylpropan-1-ol (5.4 g, 21,74 mmol) were dissolved in THF (50 mL) and added to a sealed tube reactor, and Cs₂CO₃ (10.63 g, 32.61 mmol) was added. The mixture was reacted at 90 °C for 16 h. After the reaction was completed, the mixture was cooled to room temperature. Water (30 mL) was added, and the mixture was extracted with ethyl acetate (30 mL × 3). The organic phases were combined, washed with a saturated NaCl solution, dried over anhydrous Na₂SO₄, and filtered, and the filtrate was concentrated. The resulting crude product was purified by silica gel column chromatography (DCM:MeOH = 20:1) to give (*R*)-1-(2-((*R*)-3-(4-benzylpiperazin-1-yl)-2-methylpropoxy)-7-chloro-8-fluoropyrido[4,3-*d*]pyrimidin-4-yl)-3-methylpiperidin-3-ol.

LC-MS: (ESI, m/z): [M+H]⁺ = 543.3.

### Step 2: preparation of (R)-1-(2-((R)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3-methylpiperidin-3-ol

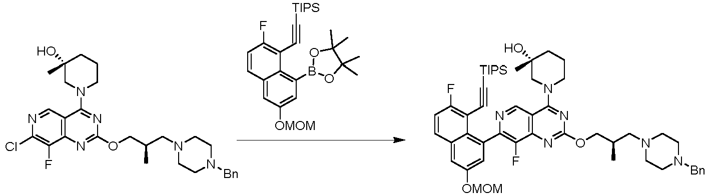

CataCXium A Pd G3 (134 mg, 0.18 mmol) and cesium carbonate (1.8 g, 5.52 mmol) were added to a mixed solution of (*R*)-1-(2-((*R*)-3-(4-benzylpiperazin-1-yl)-2-methylpropoxy)-7-chloro-8-fluoropyrido[4,3-*d*]pyrimidin-4-yl)-3-methylpiperidin-3-ol (1 g, 1.84 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (1.13 g, 2.21 mmol) in 1,4-dioxane (6 mL) and H₂O (1.2 mL). The mixture was reacted at 85 °C for 16 h under nitrogen atmosphere. Water (10 mL) was added, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 20:1) to give (*R*)-1-(2-((*R*)-3-(4-benzylpiperazin-1-yl)-2-methylpropoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3-methylpiperidin-3-ol.

LC-MS: (ESI, m/z): [M+H]⁺ = 893.5.

### Step 3: preparation of (R)-1-(8-fluoro-7-(7-fluoro-3-(methoxymethoxy)-8-((triisopropylsilyl)ethynyl) naphthalen-1-yl)-2-((R)-2-methyl-3-(piperazin-1-yl)propoxy)pyrido[4,3-d]pyrimidin-4-yl)-3-methylpiperidin-3-ol

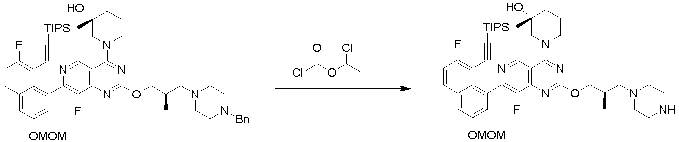

(*R*)-1-(2-((*R*)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3-methylpiperidin-3-ol (1 g, 1.12 mmol) was dissolved in DCM (5 mL), and 1-chloroethyl chloroformate (480 mg, 3.36 mmol) and DIEA (434 mg, 3.36 mmol) were added. The mixture was reacted at room temperature for 1 h and concentrated under reduced pressure. MeOH (5 mL) was added to the concentrate, and the mixture was heated to 50 °C and reacted for 10 min. After the reaction was completed, the mixture was concentrated under reduced pressure. The concentrate was purified by silica gel column chromatography (DCM:MeOH (NH₃) = 20:1) to give (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3-methylpiperidin-3-ol.

LC-MS: (ESI, m/z): [M/2+H]⁺ = 402.3.

### Intermediate 20: (R)-1-(8-fluoro-7-(7-fluoro-3-(methoxymethoxy)-8-((triisopropylsilyl)ethynyl)naphthalen-1-yl)-2-((R)-2-methyl-3-(piperazin-1-yl)propoxy)quinazolin-4-yl)-3-methylpiperidin-3-ol

### Step 1: preparation of (R)-1-(2-((R)-3-(4-benzylpiperazin-1-yl)-2-methylpropoxy)-7-bromo-8-fluoroquinazolin-4-yl)-3-methylpiperidin-3-ol

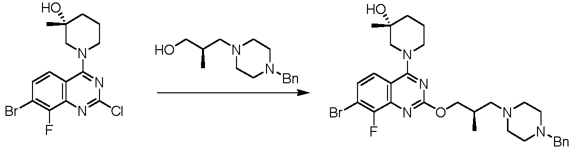

(*R*)-1-(7-bromo-2-chloro-8-fluoroquinazolin-4-yl)-3-methylpiperidin-3-ol (1.2 g, 3.20 mmol) and (*R*)-3-(4-benzylpiperazin-1-yl)-2-methylpropan-1-ol (1.03 g, 4.16 mmol) were dissolved in acetonitrile (15 mL). Cs₂CO₃ (3.13 g, 9.60 mmol) and DABCO (CAS: 280-57-9, 72 mg, 0.64 mmol) were added, and the mixture was reacted at room temperature for 10 h. After the reaction was completed, the mixture was filtered, and the filtrate was concentrated. The resulting crude product was purified by silica gel column chromatography (DCM:MeOH = 20:1) to give (*R*)-1-(2-((*R*)-3-(4-benzylpiperazin-1-yl)-2-methylpropoxy)-7-bromo-8-fluoroquinazolin-4-yl)-3-methylpiperidin-3-ol.

LC-MS: (ESI, m/z): [M+H]⁺ = 586.2.

### Step 2: preparation of (R)-1-(2-((R)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3-methylpiperidin-3-ol

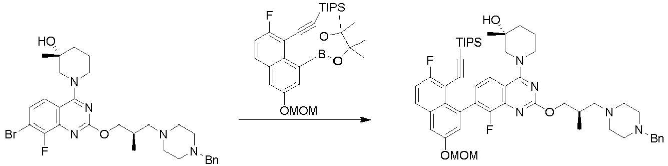

[1,1'-bis(di-*tert*-butylphosphino)ferrocene]palladium dichloride (289 mg, 0.44 mmol) and cesium carbonate (2.17 g, 6.66 mmol) were added to a mixed solution of (*R*)-1-(2-((*R*)-3-(4-benzylpiperazin-1-yl)-2-methylpropoxy)-7-bromo-8-fluoroquinazolin-4-yl)-3-methylpiperidin-3-ol (1.3 g, 2.22 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (1.37 g, 2.66 mmol) in 1,4-dioxane (10 mL) and H₂O (2 mL). The mixture was reacted at 85 °C for 16 h under nitrogen atmosphere. Water (20 mL) was added to the reaction liquid, and the mixture was extracted with ethyl acetate (30 mL × 3). The organic phases were combined, dried over anhydrous sodium sulfate, and concentrated under reduced pressure. The concentrate was purified by silica gel column chromatography (DCM:MeOH = 20:1) to give (*R*)-1-(2-((*R*)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3-methylpiperidin-3-ol.

LC-MS: (ESI, m/z): [M+H]⁺ = 892.5.

### Step 3: preparation of (R)-1-(8-fluoro-7-(7-fluoro-3-(methoxymethoxy)-8-((triisopropylsilyl)ethynyl) naphthalen-1-yl)-2-((R)-2-methyl-3-(piperazin-1-yl)propoxy)quinazolin-4-yl)-3-methylpiperidin-3-ol

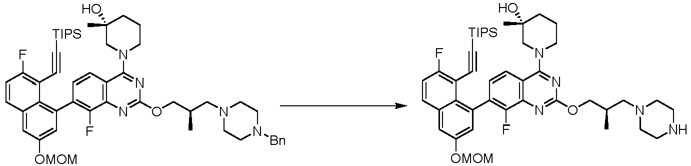

*(R)-1-(2-((R)-3-(4-benzylpiperazin-1-yl)-2-methylpropoxy)-8-fluoro-7-(7-fluoro-3-*(methoxymethoxy)-8-((triisopropylsilyl)ethynyl)naphthalen-1-yl)quinazolin-4-yl)-3-methylpiperidin-3-ol (1.5 g, 1.68 mmol) was dissolved in DCM (8 mL), and 1-chloroethyl carbonochloridate (721 mg, 5.04 mmol) and DIEA (651 mg, 5.04 mmol) were added. The mixture was reacted at room temperature for 1 h. The reaction liquid was directly concentrated under reduced pressure, and the concentrate was dissolved in MeOH (8 mL). The mixture was heated to 50 °C and reacted for 30 min. After the reaction was completed, the mixture was concentrated under reduced pressure. The concentrate was purified by silica gel column chromatography (DCM:MeOH (NH₃) = 30:1) to give (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)quinazolin-4-yl)-3-methylpiperidin-3-ol.

LC-MS: (ESI, m/z): [M+H]⁺ = 802.5.

### Intermediate 21: 2,7-dichloro-4-(3,3-difluoropiperidin-1-yl)-8-fluoropyrido[4,3-d]pyrimidine

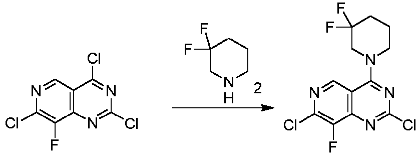

2,4,7-Trichloro-8-fluoropyrido[4,3-*d*]pyrimidine (3.0 g, 11.88 mmol) and triethylamine (3.61 g, 35.64 mmol) were dissolved in THF (40 mL), and the mixture was cooled to -50 °C. 3,3-Difluoropiperidine (1.68 g, 10.69 mmol) was added, and the mixture was reacted at -50 °C for 3 h. After the reaction was completed, the reaction liquid was poured into a saturated aqueous ammonium chloride solution (20 mL), and the mixture was extracted with ethyl acetate (20 mL × 3). The organic phases were combined, washed with saturated brine, dried over anhydrous sodium sulfate, and filtered. The filtrate was concentrated, and the resulting crude product was purified by silica gel column chromatography (PE:EtOAc = 3:1) to give 2,7-dichloro-4-(3,3-difluoropiperidin-1-yl)-8-fluoropyrido[4,3-*d*]pyrimidine.

LC-MS: (ESI, m/z): [M+H]⁺ = 337.1.

### Intermediate 22: (R)-1-(2-((1-((4-((3-azaspiro[5.5]undecan-9-yl)methyl)piperazin-1-yl)methyl)cyclopropyl) methoxy)-8-fluoro-7-(7-fluoro-3-hydroxy-8-((triisopropylsilyl)ethynyl)naphthalen-1-yl)pyrido[4,3-d]pyrimidin-4-yl)-3-methylpiperidin-3-ol

### Step 1: preparation of tert-butyl (R)-9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carboxylate

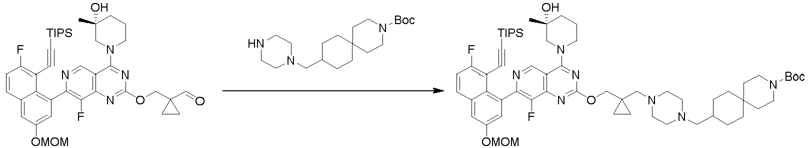

Tetraisopropyl titanate (1145 mg, 4027.0 µmol) and glacial acetic acid (0.4 mL) were added to a stirred solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde (518 mg, 695.34 µmol) and *tert*-butyl 9-(piperazin-1-ylmethyl)-3-azaspiro[5.5]undecane-3-carboxylate (566 mg, 1610.80 µmol) in dichloromethane (30 mL) at room temperature, and the mixture was stirred at 25 °C for 4 h. Then sodium triacetoxyborohydride (442 mg, 2086.02 µmol) was added, and the mixture was reacted at 25 °C for another 4 h. The mixture was concentrated under reduced pressure at 40 °C, and the resulting crude product was purified by silica gel column chromatography (dichloromethane:methanol =100:1 to 10:1) to give *tert*-butyl (*R*)-9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carboxylate.

LC-MS: (ESI, m/z): [M+H]⁺ = 1080.6.

### Step 2: preparation of (R)-1-(2-((1-((4-((3-azaspiro[5.5]undecan-9-yl)methyl)piperazin-1-yl)methyl) cyclopropyl)methoxy)-8-fluoro-7-(7-fluoro-3-hydroxy-8-((triisopropylsilyl)ethynyl)naphthalen-1-yl)pyrido[4,3-d]pyrimidin-4-yl)-3-methylpiperidin-3-ol

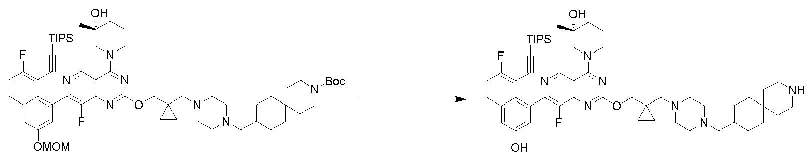

A hydrogen chloride/1,4-dioxane solution (4.0 M, 3 mL) was added to a solution of crude *tert-*butyl (*R*)-9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carboxylate (511.6 mg, 473.49 µmol) in a 1,4-dioxane solution (2 mL) at 0 °C, and the mixture was reacted at room temperature for 1.5 h. After the reaction was completed, the mixture was concentrated under reduced pressure at 30 °C. A solution of ammonia in methanol (5 mL) was added to the concentrate, and the mixture was concentrated under reduced pressure at 30 °C to give (*R*)-1-(2-((1-((4-((3-azaspiro[5.5]undecan-9-yl)methyl)piperazin-1-yl)methyl)cyclopropyl)methoxy)-8-fluoro-7-(7-fluoro-3-hydroxy-8-((triisopropylsilyl)ethynyl)naphthalen-1-yl)pyrido[4,3-*d*]pyrimidin-4-yl)-3-methylpiperidin-3-ol.

LC-MS: (ESI, m/z): [M+H]⁺ = 936.5.

### Intermediate 23: tert-butyl (1-(8-fluoro-7-(7-fluoro-3-(methoxymethoxy)-8-((triisopropylsilyl)ethynyl) naphthalen-1-yl)-2-((1-formylcyclopropyl)methoxy)pyrido[4,3-d]pyrimidin-4-yl)-3-methylpiperidin-3-yl)carboxylate

### Step 1: preparation of tert-butyl (1-(2,7-dichloro-8-fluoropyrido[4,3-d]pyrimidin-4-yl)-3-methylpiperidin-3-yl)carboxylate

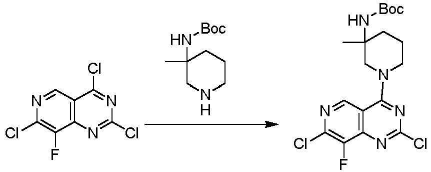

TEA (2.4 g, 23.7 mmol) was added to a solution of 2,4,7-trichloro-8-fluoropyrido[4,3-*d*]pyrimidine (2.0 g, 7.9 mmol) in DCM (20 mL) at -78 °C, and the mixture was stirred for 30 min. *tert-*Butyl (3-methylpiperidin-3-yl)carbamate (1.35 g, 2.0 mmol) was added, and the mixture was stirred for 4 h. After the reaction was completed, the mixture was naturally warmed to room temperature. H₂O (30 mL) was added, and the mixture was extracted with DCM (30 mL × 3). The organic phases were combined, dried over anhydrous sodium sulfate, and concentrated under reduced pressure, and the concentrate was purified by silica gel column chromatography (PE:EtOAc = 5:1) to give *tert-butyl* (1-(2,7-dichloro-8-fluoropyrido[4,3-*d*]pyrimidin-4-yl)-3-methylpiperidin-3-yl)carboxylate.

LC-MS: (ESI, m/z): [M+H]⁺ = 430.1.

### Step 2: preparation of tert-butyl (1-(7-chloro-8-fluoro-2-((1-(hydroxymethyl)cyclopropyl)methoxy)pyrido [4,3-d]pyrimidin-4-yl)-3-methylpiperidin-3-yl)carboxylate

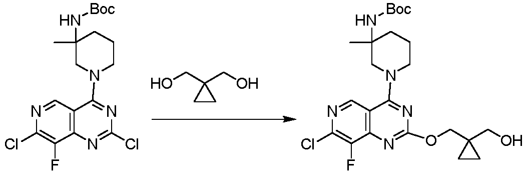

Cs₂CO₃ (1.3 g, 4.2 mmol) and DABCO (1.1 g, 0.7 mmol) were added to a solution of *tert-butyl* (1-(2,7-dichloro-8-fluoropyrido[4,3-*d*]pyrimidin-4-yl)-3-methylpiperidin-3-yl)carboxylate (2.3 g, 5.3 mmol) and cyclopropane-1,1-diyldimethanol (286 mg, 10.6 mmol) in acetonitrile (5 mL), and the mixture was stirred at room temperature for 30 min. After the reaction was completed, water (20 mL) was added, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH = 20:1) to give *tert-butyl* (1-(7-chloro-8-fluoro-2-((1-(hydroxymethyl)
cyclopropyl)methoxy)pyrido[4,3-*d*]pyrimidin-4-yl)-3-methylpiperidin-3-yl)carboxylate.

LC-MS: (ESI, m/z): [M+H]⁺ = 496.1.

### Step 3: preparation of tert-butyl (1-(8-fluoro-7-(7-fluoro-3-(methoxymethoxy)-8-((triisopropylsilyl)ethynyl) naphthalen-1-yl)-2-((1-(hydroxymethyl)cyclopropyl)methoxy)pyrido[4,3-d]pyrimidin-4-yl)-3-methylpiperidin-3-yl)carboxylate

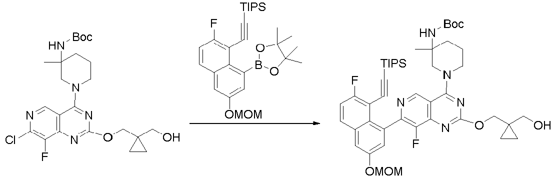

((2-Fluoro-6-(methoxymethoxy)-8-(4,4,5,5-tetramethyl-1,3,2-dioxaborolan-2-yl)naphthalen-1-yl)ethynyl) triisopropylsilane (496 mg, 0.97 mmol), cataCXium A Pd G3 (118 mg, 0.36 mmol), and cesium carbonate (788 mg, 2.42 mmol) were added to a mixed solution of *tert*-butyl (1-(7-chloro-8-fluoro-2-((1-(hydroxymethyl)cyclopropyl)methoxy)pyrido[4,3-*d*]pyrimidin-4-yl)-3-methylpiperidin-3-yl)carboxylate (400 mg, 0.81 mmol) in 1,4-dioxane (12 mL) and H₂O (2 mL). The mixture was reacted at 85 °C for 16 h under nitrogen atmosphere. After the reaction was completed, water (10 mL) was added to the reaction liquid, and the mixture was extracted with ethyl acetate (10 mL × 3). The organic phase was dried over anhydrous sodium sulfate and concentrated under reduced pressure. The concentrate was purified by silica gel column chromatography (DCM:MeOH = 10:1) to give *tert-*butyl (1-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido [4,3-*d*]pyrimidin-4-yl)-3-methylpiperidin-3-yl)carboxylate.

LC-MS: (ESI, m/z): [M+H]⁺ = 846.4.

### Step 4: preparation of tert-butyl (1-(8-fluoro-7-(7-fluoro-3-(methoxymethoxy)-8-((triisopropylsilyl)ethynyl) naphthalen-1-yl)-2-((1-formylcyclopropyl)methoxy)pyrido[4,3-d]pyrimidin-4-yl)-3-methylpiperidin-3-yl)carboxylate

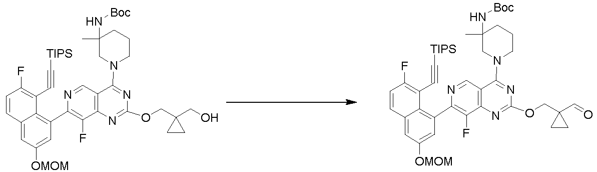

A solution of *tert*-butyl (1-(8-fluoro-7-(7-fluoro-3-(methoxymethoxy)-8-((triisopropylsilyl)ethynyl) naphthalen-1-yl)-2-((1-(hydroxymethyl)cyclopropyl)methoxy)pyrido[4,3-*d*]pyrimidin-4-yl)-3-methylpiperidin-3-yl)carboxylate (500 mg, 0.59 mmol) in DCM (5 mL) was cooled to 0 °C, and Dess-Martin oxidant (501 mg, 1.18 mmol) was added. The mixture was reacted at room temperature for 1 h. After the reaction was completed, a sodium thiosulfate solution (5 mL) and a sodium bicarbonate solution (5 mL) were added to quench the reaction, and the mixture was extracted with dichloromethane (5 mL × 3). The organic phases were combined, dried over anhydrous sodium sulfate, and concentrated under reduced pressure. The concentrate was purified by silica gel column chromatography (DCM:MeOH = 20:1) to give *tert-butyl* (1-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3-methylpiperidin-3-yl)carboxylate.

LC-MS: (ESI, m/z): [M+H]⁺ = 844.4.

### Intermediate 24: (R)-1-(8-fluoro-7-(7-fluoro-3-(methoxymethoxy)-8-((triisopropylsilyl)ethynyl)naphthalen-1-yl)-2-((R)-2-methoxy-3-(piperazin-1-yl)propoxy)pyrido[4,3-d]pyrimidin-4-yl)-3-methylpiperidin-3-ol

### Step 1: preparation of (R)-1-(2-((R)-3-(4-benzylpiperazin-1-yl)-2-methoxypropoxy)-7-chloro-8-fluoropyrido [4,3-d]pyrimidin-4-yl)-3-methylpiperidin-3-ol

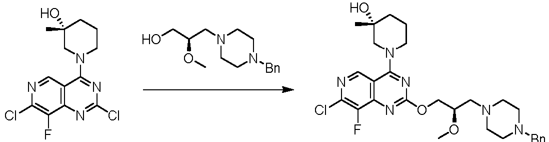

(*R*)-1-(2,7-dichloro-8-fluoropyrido[4,3-*d*]pyrimidin-4-yl)-3-methylpiperidin-3-ol (1.50 g, 4.53 mmol) and (*R*)-3-(4-benzylpiperazin-1-yl)-2-methoxypropan-1-ol (2.39 g, 9.06 mmol) were dissolved in acetonitrile (20 mL). Cs₂CO₃ (2.95 g, 9.06 mmol) and DABCO (51 mg, 0.45 mmol) were added, and the mixture was reacted at room temperature for 2 h. After the reaction was completed, the mixture was cooled to room temperature. Water (30 mL) was added, and the mixture was extracted with ethyl acetate (30 mL × 3). The organic phases were combined, washed with a saturated NaCl solution, dried over anhydrous Na₂SO₄, and filtered, and the filtrate was concentrated. The resulting crude product was purified by silica gel column chromatography (DCM:MeOH = 20:1) to give (R)-1-(2-((R)-3-(4-benzylpiperazin-1-yl)-2-methoxypropoxy)-7-chloro-8-fluoropyrido[4,3-*d*]pyrimidin-4-yl)-3-methylpiperidin-3-ol.

LC-MS: (ESI, m/z): [M+H]⁺ = 559.2.

### Step 2: preparation of (R)-1-(2-((R)-3-(4-benzylpiperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3-methylpiperidin-3-ol

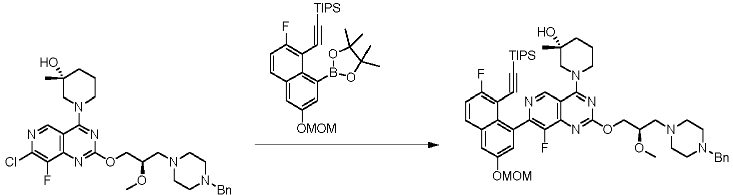

Ruphos Pd G3 (130 mg, 0.18 mmol) and cesium carbonate (1.20 g, 3.60 mmol) were added to a mixed solution of (*R*)-1-(2-((*R*)*-*3-(4-benzylpiperazin-1-yl)-2-methoxypropoxy)-7-chloro-8-fluoropyrido[4,3-*d*]pyrimidin-4-yl)-3-methylpiperidin-3-ol (1.00 g, 1.80 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (1.20 g, 2.30 mmol) in 1,4-dioxane/water (10 mL/2 mL). The mixture was reacted at 85 °C for 16 h under nitrogen atmosphere. Water (20 mL) was added, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH = 20:1) to give (*R*)-1-(2-((*R*)-3-(4-benzylpiperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3-methylpiperidin-3-ol.

LC-MS: (ESI, m/z): [M/2+H]⁺ = 455.4.

### Step 3: preparation of (R)-1-(8-fluoro-7-(7-fluoro-3-(methoxymethoxy)-8-((triisopropylsilyl)ethynyl) naphthalen-1-yl)-2-((R)-2-methoxy-3-(piperazin-1-yl)propoxy)pyrido[4,3-d]pyrimidin-4-yl)-3-methylpiperidin-3-ol

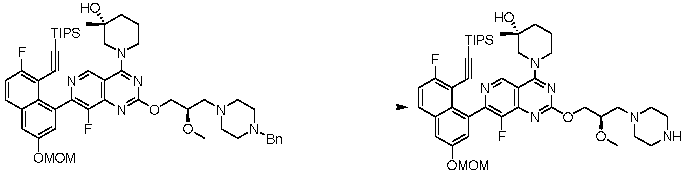

1- Chloroethyl chloroformate (0.472 g, 3.300 mmol) was added to a stirred solution of (*R*)-1-(2-((*R*)-3-(4-benzylpiperazin-1-yl)-2-methoxypropoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3-methylpiperidin-3-ol (1.000 g, 1.099 mmol) in dichloromethane (30 mL) and *N*,*N*-diisopropylethylamine (0.426 g, 3.300 mmol) at room temperature. The mixture was stirred at 25 °C for 1 h and concentrated under reduced pressure. Anhydrous methanol (30 mL) was dissolved in the concentrate, and the mixture was reacted at 50 °C for another 1 h and concentrated under reduced pressure at 40 °C to give a crude product of (R)-1-(8-fluoro-7-(7-fluoro-3-(methoxymethoxy)-8-((triisopropylsilyl)ethynyl)naphthalen-1-yl)-2-((R)-2-methoxy-3-(piperazin-1-yl)propoxy)pyrido[4,3-*d*]pyrimidin-4-yl)-3-methylpiperidin-3-ol.

LC-MS: (ESI, m/z): [M+H]⁺ = 819.4.

### Intermediate 25: 3-(3-(2,4-dioxotetrahydropyrimidin-1(2H)-yl)-4-methoxybenzoyl)-3-azaspiro[5.5] undecane-9-carbaldehyde

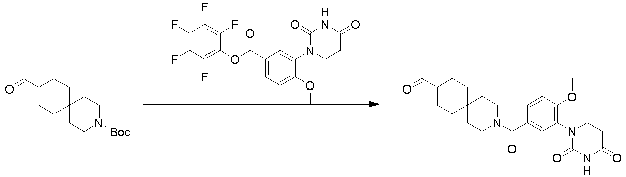

TFA (50 mL) was added to a solution of *tert-butyl* 9-formyl-3-azaspiro[5.5]undecane-3-carboxylate (10 g, 35.6 mmol) in DCM (200 mL) at 25 °C, and the mixture was reacted at 25 °C for 1 h. The reaction liquid was concentrated, and the concentrate was dissolved in *N*,*N*-dimethylformamide (200 mL). DIEA (46 g, 356 mmol) and pentafluorophenyl 3-(2,4-dioxotetrahydropyrimidin-1(2*H*)-yl)-4-methoxybenzoate (18 g, 42.7 mmol) were added, and the mixture was stirred at 25 °C for 16 h. After the reaction was completed, the reaction liquid was directly purified by preparative high-performance liquid chromatography to give 3-(3-(2,4-dioxotetrahydropyrimidin-1(2*H*)-yl)-4-methoxybenzoyl)-3-azaspiro[5.5]undecane-9-carbaldehyde.

LC-MS: (ESI, m/z): [M+H]⁺ = 428.3.

### Intermediate 26: preparation of 1-(2-ethyl-5-(9-(piperazin-1-ylmethyl)-3-azaspiro[5.5]undecane-3-carbonyl)phenyl)dihydropyrimidine-2,4(1H,3H)-dione

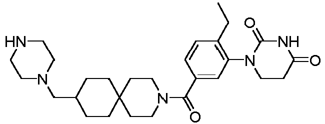

The intermediate was prepared with reference to intermediate 12.

### Intermediate 27: pentafluorophenyl 3-(2,4-dioxotetrahydropyrimidin-1(2H)-yl)-4-(methyl-d3)benzoate

### Step 1: preparation of methyl 3-((tert-butoxycarbonyl)amino)-4-iodobenzoate

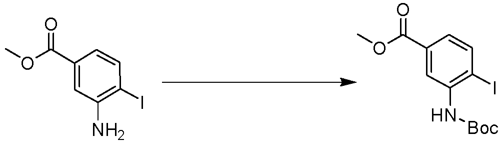

Methyl 3-amino-4-iodobenzoate (20.0 g, 72.2 mmol) was dissolved in tetrahydrofuran (200 mL). Boc₂O (47.2 g, 216.6 mmol) and DMAP (0.9 g, 7.22 mmol) were added, and the mixture was reacted at 25 °C for 5 h. After the reaction was completed, the reaction liquid was poured into a saturated aqueous ammonium chloride solution (100 mL), and the mixture was extracted with ethyl acetate (100 mL × 2). The organic phases were combined, washed with saturated brine, dried over anhydrous sodium sulfate, and filtered. The filtrate was concentrated to give a crude product of the intermediate. The intermediate was dissolved in DCM (300 mL). Trifluoroacetic acid (10 mL) was added at 0-5 °C, and the mixture was reacted at 0-5 °C for another 1 h. The reaction liquid was slowly added dropwise to a sodium carbonate solution at 0-5 °C, and the mixture was extracted with ethyl acetate (100 mL × 2). The organic phase was washed with a saturated aqueous sodium chloride solution, dried over anhydrous sodium sulfate, and concentrated. The resulting crude product was purified by silica gel column chromatography (PE:EA = 100:1 to 20:1) to give methyl 3-((*tert*-butoxycarbonyl)amino)-4-iodobenzoate.

LC-MS: (ESI, m/z): [M-Boc+H]⁺ = 278.0.

### Step 2: preparation of methyl 3-((tert-butoxycarbonyl)amino)-4-(methyl-d3)benzoate

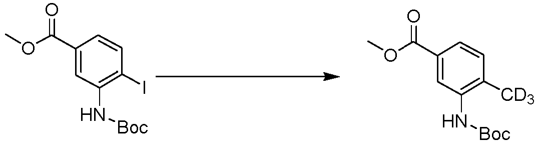

Methyl 3-((*tert*-butoxycarbonyl)amino)-4-iodobenzoate (13.5 g, 35.8 mmol) and deuterated iodomethane (15.6 g, 107.4 mmol) were dissolved in anhydrous THF (150 mL). Sodium iodide (2.7 g, 17.9 mmol), zinc powder (7.0 g, 107.4 mmol), and 1,3-bis(diphenylphosphinopropane)nickel dichloride (1.9 g, 3.58 mmol) were added. The mixture was reacted at 25 °C for 16 h under nitrogen atmosphere. After the reaction was completed, the reaction liquid was filtered, and the filtrate was concentrated under reduced pressure. The concentrate was purified by silica gel column chromatography (PE:EA = 200:1 to 50:1) to give methyl 3-((*tert*-butoxycarbonyl)amino)-4-(methyl-d3)benzoate.

LC-MS: (ESI, m/z): [M-tBu+H]⁺ = 213.2.

### Step 3: preparation of 3-((tert-butoxycarbonyl)amino)-4-(methyl-d3)benzoic acid

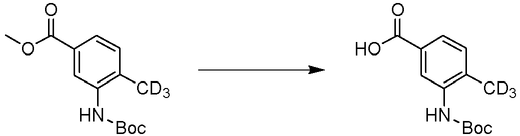

Methyl 3-((*tert*-butoxycarbonyl)amino)-4-(methyl-d3)benzoate (2.0 g, 7.46 mmol) was dissolved in THF (15 mL) and H₂O (15 mL). Lithium hydroxide (1.25 g, 29.9 mmol) was added, and the mixture was reacted at 25 °C for 3 h. The mixture was extracted with methyl *tert-butyl* ether (20 mL). The aqueous phase was adjusted to pH 4 with dilute hydrochloric acid and extracted with ethyl acetate (30 mL × 2). The organic phases were combined, washed with a saturated sodium chloride solution, dried over anhydrous sodium sulfate, and filtered. The filtrate was concentrated to give 3-((*tert*-butoxycarbonyl)amino)-4-(methyl-d3)benzoic acid.

LC-MS: (ESI, m/z): [M-tBu+H]⁺ = 199.1.

### Step 4: preparation of 3-amino-4-(methyl-d3)benzoic acid

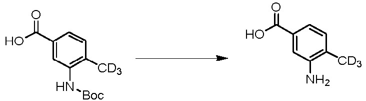

3-((tert-Butoxycarbonyl)amino)-4-(methyl-d3)benzoic acid (1.5 g, 5.90 mmol) was dissolved in 1,4-dioxane (10 mL). HCl/1,4-dioxane (4 N, 10 mL) was added, and the mixture was reacted at 25 °C for 3 h. The reaction liquid was concentrated to give 3-amino-4-(methyl-d3)benzoic acid, which was directly used in the next step.

### Step 5: preparation of 3-(2,4-dioxotetrahydropyrimidin-1(2H)-yl)-4-(methyl-d3)benzoic acid

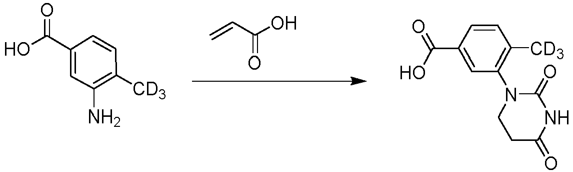

A mixture of 3-amino-4-(methyl-d3)benzoic acid (1.00 g, 6.62 mmol) and acrylic acid (1.4 g, 19.87 mmol) was heated to 100 °C and stirred for 3 h. The mixture was then cooled to room temperature, and glacial acetic acid (10 mL) was added. The mixture was then heated to 100 °C for 10 min, and urea (2.38 g, 39.72 mmol) was added. The mixture was then heated to 120 °C and stirred for 16 h. After the reaction was completed, the reaction liquid was cooled to room temperature and poured into ice dilute hydrochloric acid (1 N, 30 mL), and a large amount of solid precipitated. The mixture was stirred for another 1 h and filtered to give 3-(2,4-dioxotetrahydropyrimidin-1(2*H*)-yl)-4-(methyl-d3)benzoic acid.

LC-MS: (ESI, m/z): [M+H]⁺ = 252.1.

### Step 6: preparation of pentafluorophenyl 3-(2,4-dioxotetrahydropyrimidin-1(2H)-yl)-4-(methyl-d3)benzoate

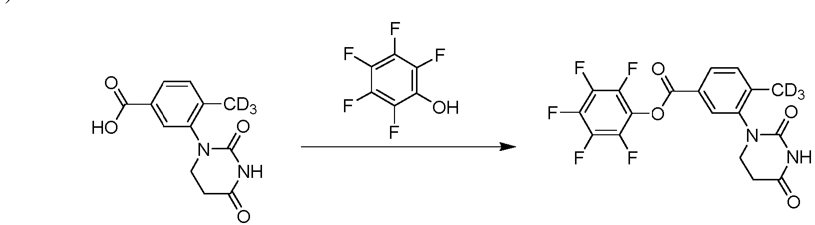

*N*,*N'*-dicyclohexylcarbodiimide (393 mg, 1.91 mmol) was added to a solution of 3-(2,4-dioxotetrahydropyrimidin-1(2*H*)-yl)-4-(methyl-d3)benzoic acid (400 mg, 1.59 mmol) and 2,3,4,5,6-pentafluorophenol (351 mg, 1.91 mmol) in DMF (2 mL) at room temperature, and the mixture was stirred at 25 °C for 3 h. Water (20 mL) was added, and the mixture was extracted with ethyl acetate (20 mL × 3). The organic phases were combined, dried over anhydrous sodium sulfate, and concentrated under reduced pressure. The resulting crude product was purified by silica gel column chromatography (DCM/MeOH = 20/1 to 10/1) to give pentafluorophenyl 3-(2,4-dioxotetrahydropyrimidin-1(2*H*)-yl)-4-(methyl-d3)benzoate.

LC-MS: (ESI, m/z): [M+H]⁺ = 418.1.

### Intermediate 28: 3-(3-(2,4-dioxotetrahydropyrimidin-1(2H)-yl)-4-(methyl-d3)benzoyl)-3-azaspiro[5.5] undecane-9-carbaldehyde

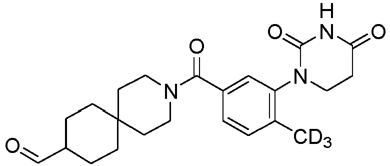

The intermediate was prepared with reference to intermediate 11.

### Intermediate 29: 3-(3-(2,4-dioxotetrahydropyrimidin-1(2H)-yl)-4-ethylbenzoyl)-3-azaspiro[5.5]undecane-9-carbaldehyde

### Step 1: preparation of 3-(2,4-dioxotetrahydropyrimidin-1(2H)-yl)-4-ethylbenzoic acid

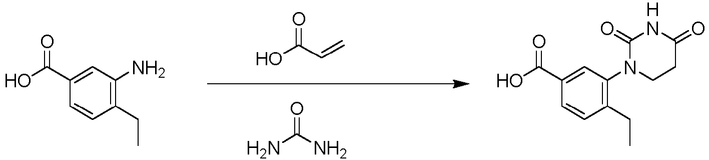

A mixture of 3-amino-4-ethylbenzoic acid (3.00 g, 18.19 mmol) and acrylic acid (3.9 g, 54.55 mmol) was heated to 100 °C and stirred for 3 h. The mixture was then cooled to room temperature, and glacial acetic acid (20 mL) was added. The mixture was then heated to 100 °C for 10 min, and urea (6.5 g, 109.08 mmol) was added. The mixture was then heated to 120 °C and stirred for 16 h. After the reaction was completed, the reaction liquid was cooled to room temperature and poured into ice dilute hydrochloric acid (1 N, 100 mL), and a large amount of solid precipitated. The mixture was stirred for another 1 h and filtered to give 3-(2,4-dioxotetrahydropyrimidin-1(2*H*)-yl)-4-ethylbenzoic acid.

LC-MS: (ESI, m/z): [M+H]⁺ = 263.1.

### Step 2: preparation of pentafluorophenyl 3-(2,4-dioxotetrahydropyrimidin-1(2H)-yl)-4-ethylbenzoate

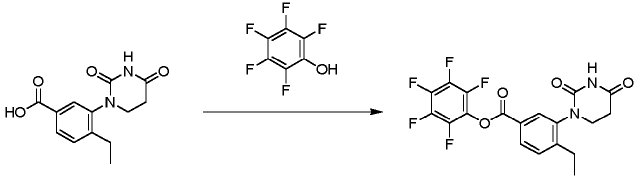

*N*,*N*-dicyclohexylcarbodiimide (3.5 g, 16.92 mmol) and DMAP (172 mg, 1.41 mmol) were added to a stirred solution of 3-(2,4-dioxotetrahydropyrimidin-1(2*H*)-yl)-4-ethylbenzoic acid (3.7 g, 14.1 mmol) and pentafluorophenol (3.1 g, 16.92 mmol) in *N*,*N*-dimethylformamide (70 mL) at room temperature, and the mixture was stirred at 25 °C for 16 h. After the reaction was completed, the reaction liquid was poured into water (100 mL), and the mixture was extracted with ethyl acetate (100 mL × 3). The organic phase was washed with saturated brine (100 mL × 2). The organic phases were combined, dried over anhydrous sodium sulfate, filtered, and concentrated under reduced pressure to give a crude product, which was purified by silica gel column chromatography (MeOH:DCM = 1:20) to give pentafluorophenyl 3-(2,4-dioxotetrahydropyrimidin-1(2*H*)-yl)-4-ethylbenzoate.

LC-MS: (ESI, m/z): [M+H]⁺ = 429.1.

### Step 3: preparation of 3-(3-(2,4-dioxotetrahydropyrimidin-1(2H)-yl)-4-ethylbenzoyl)-3-azaspiro[5.5] undecane-9-carbaldehyde

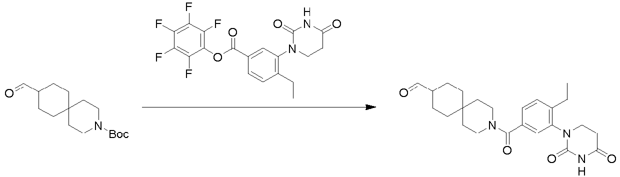

TFA (50 mL) was added to a solution of *tert*-butyl 9-formyl-3-azaspiro[5.5]undecane-3-carboxylate (2 g, 7.1 mmol) in DCM (200 mL) at 25 °C, and the mixture was reacted at 25 °C for 1 h. The reaction liquid was concentrated, and the concentrate was dissolved in *N,N*-dimethylformamide (20 mL). DIEA (4.6 g, 35.6 mmol) and pentafluorophenyl 3-(2,4-dioxotetrahydropyrimidin-1(2*H*)-yl)-4-ethylbenzoate (3.0 g, 7.1 mmol) were added, and the mixture was stirred at 25 °C for 16 h. After the reaction was completed, the reaction liquid was directly purified by reversed-phase chromatography (acetonitrile/water = 5/95 to 95/95), and the prepared solution was freeze-dried to give 3-(3-(2,4-dioxotetrahydropyrimidine-1(2*H*)-yl)-4-ethylbenzoyl)-3-azaspiro[5.5]undecane-9-carbaldehyde.

LC-MS: (ESI, m/z): [M+H]⁺ = 426.3.

### Intermediate 30: 3-(5-(2,4-dioxotetrahydropyrimidin-1(2H)-yl)-2-fluoro-4-methylbenzoyl)-3-azaspiro[5.5] undecane-9-carbaldehyde

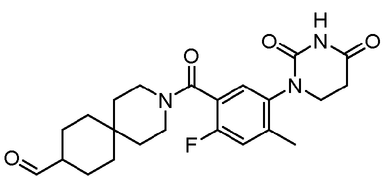

The intermediate was prepared with reference to intermediate 11.

### Intermediate 31: 3-(3-(2,4-dioxotetrahydropyrimidin-1(2H)-yl)-4-(methoxy-d3)benzoyl)-3-azaspiro[5.5] undecane-9-carbaldehyde

### Step 1: preparation of methyl 4-(methoxy-d3)-3-nitrobenzoate

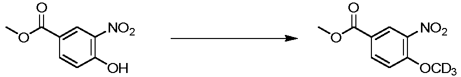

Deuterated iodomethane (20.00 g, 137.97 mmol) was added to a mixed solution of methyl 4-hydroxy-3-nitrobenzoate (25.00 g, 126.81 mmol) and anhydrous potassium carbonate (52.58 g, 380.42 mmol) in *N,N*-dimethylformamide (150 mL) at 25 °C, and the mixture was stirred at 65 °C for 16 h. After the reaction was completed, the reaction liquid was poured into water (500 mL), and the mixture was extracted with ethyl acetate (200 mL × 3). The organic phase was washed with saturated brine (500 mL × 2). The organic phases were combined, dried over anhydrous sodium sulfate, filtered, and concentrated under reduced pressure. The crude product was stirred with petroleum ether/ethyl acetate (20/1), and filtered to give methyl 4-(methoxy-d3)-3-nitrobenzoate.

LC-MS: (ESI, m/z): [M+H]⁺ = 215.1.

### Step 2: preparation of methyl 3-amino-4-(methoxy-d3)benzoate

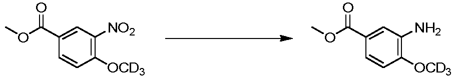

Zinc powder (78.87 g, 1206.27 mmol) was added to a stirred solution of methyl 4-(methoxy-d3)-3-nitrobenzoate (25.84 g, 120.626 mmol) in glacial acetic acid (250 mL) at 25 °C, and the mixture was stirred at 50 °C for 16 h. The mixture was filtered through celite to remove insoluble substances, and the filtrate was concentrated to dryness under reduced pressure. A saturated aqueous sodium carbonate solution (200 mL) was added to the residue, and the mixture was extracted with ethyl acetate (100 mL × 3). The organic phase was washed with saturated brine (200 mL), and the organic phases were combined, dried over anhydrous sodium sulfate, filtered, and concentrated under reduced pressure. The resulting crude product was purified by silica gel column chromatography (petroleum ether:ethyl acetate = 20:1 to 1:1) to give methyl 3-amino-4-(methoxy-d3)benzoate.

LC-MS: (ESI, m/z): [M+H]⁺ = 185.2.

### Step 3: preparation of 3-amino-4-(methoxy-d3)benzoic acid

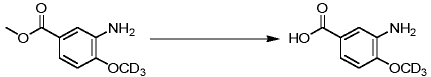

Lithium hydroxide, (19.70 g, 469.57 mmol) was added to a stirred solution of methyl 3-amino-4-(methoxy-d3)benzoate (17.30 g, 93.92 mmol) in tetrahydrofuran (170 mL) and water (170 mL) at 25 °C, and the mixture was stirred at 50 °C for 16 h. The reaction liquid was concentrated to dryness under reduced pressure, and the residue was dissolved in ethyl acetate and dried over anhydrous sodium sulfate. Insoluble matter was filtered out, and the filtrate was concentrated under reduced pressure to give 3-amino-4-(methoxy-d3)benzoic acid.

LC-MS: (ESI, m/z): [M+H]⁺ = 171.2.

### Step 4: preparation of 3-(2,4-dioxotetrahydropyrimidin-1(2H)-yl)-4-(methoxy-d3)benzoic acid

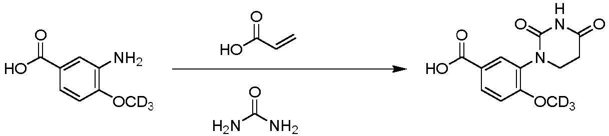

A mixture of 3-amino-4-(methoxy-d3)benzoic acid (2.00 g, 11.75 mmol) and acrylic acid (3.39 g, 47.01 mmol) was heated to 100 °C and stirred for 3 h. The mixture was then cooled to room temperature, and glacial acetic acid (14 mL) was added. The mixture was then heated to 100 °C for 10 min, and urea (4.38 g, 72.86 mmol) was added. The mixture was then heated to 120 °C and stirred for 16 h. After the reaction was completed, the reaction liquid was cooled to room temperature and poured into ice dilute hydrochloric acid (1 N, 150 mL), and a large amount of solid precipitated. The mixture was stirred for another 1 h and filtered to give 3-(2,4-dioxotetrahydropyrimidin-1(*2H*)-yl)-4-(methoxy-d3)benzoic acid.

LC-MS: (ESI, m/z): [M+H]⁺ = 268.1.

### Step 5: preparation of methyl 3-(2,4-dioxotetrahydropyrimidin-1(2H)-yl)-4-(methoxy-d3)benzoate

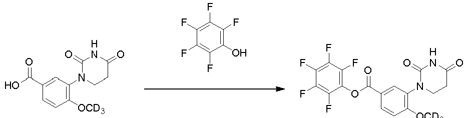

A tetrahydrofuran solution (10 mL) containing *N,N*-dicyclohexylcarbodiimide (20.00 g, 137.97 mmol) was added to a stirred solution of 3-(2,4-dioxotetrahydropyrimidin-1(*2H*)-yl)-4-(methoxy-d3)benzoic acid (1.83 g, 6.84 mmol) and pentafluorophenol (1.39 g, 7.53 mmol) in *N,N*-dimethylformamide (60 mL) at -10 °C, and the mixture was stirred at 25 °C for 16 h. After the reaction was completed, the reaction liquid was poured into water (200 mL), and the mixture was extracted with ethyl acetate (50 mL × 3). The organic phase was washed with saturated brine (200 mL × 2). The organic phases were combined, dried over anhydrous sodium sulfate, filtered, and concentrated under reduced pressure. The crude product was stirred with petroleum ether/tetrahydrofuran (5/1), and filtered to give methyl 3-(2,4-dioxotetrahydropyrimidin-1(2*H*)-yl)-4-(methoxy-d3)benzoate.

LC-MS: (ESI, m/z): [M+H]⁺ = 434.1.

### Step 6: preparation of 3-(3-(2,4-dioxotetrahydropyrimidin-1(2H)-yl)-4-(methoxy-d3)benzoyl)-3-azaspiro[5.5] undecane-9-carbaldehyde

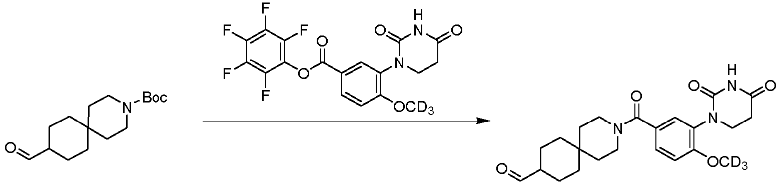

TFA(2 mL) was added to a solution of *tert*-butyl 9-formyl-3-azaspiro[5.5]undecane-3-carboxylate (0.56 g, 2.01 mmol) in DCM (4 mL) at 25 °C, and the mixture was reacted at 25 °C for 1 h. The reaction liquid was concentrated, and the concentrate was dissolved in *N,N*-dimethylformamide (10 mL). DIEA (2.98 g, 23.08 mmol) and methyl 3-(2,4-dioxotetrahydropyrimidin-1(*2H*)-yl)-4-(methoxy-d3)benzoate (1.00 g, 2.01 mmol) were added, and the mixture was stirred at 25 °C for 16 h. After the reaction was completed, the reaction liquid was directly purified by preparative high-performance liquid chromatography (acetonitrile:water = 5:95 to 95:95) to give 3-(3-(2,4-dioxotetrahydropyrimidin-1(*2H*)-yl)-4-(methoxy-d3)benzoyl)-3-azaspiro[5.5]
undecane-9-carbaldehyde.

LC-MS: (ESI, m/z): [M+H]⁺ = 431.3.

### Intermediate 32: (R)-1-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido [4,3-d]pyrimidin-4-yl)-3-methylpiperidin-3-ol

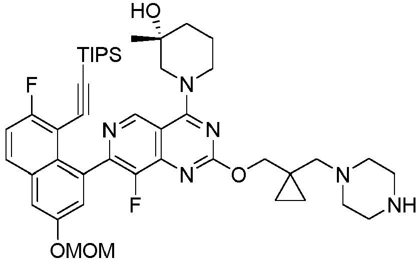

The intermediate was prepared with reference to intermediate 11.

### Intermediate 33: (R)-3-(4-benzylpiperazin-1-yl)-2-methylpropan-1-ol

### Step 1: preparation of methyl (S)-3-((tert-butyldimethylsilyl)oxy)-2-methylpropanoate

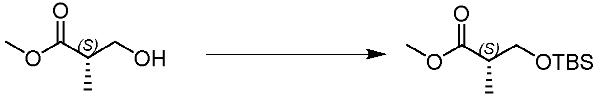

Methyl (*S*)-3-hydroxy-2-methylpropanoate (30 g, 254 mmol) and imidazole (34.5 g, 508 mmol) were dissolved in dichloromethane (120 mL), and *tert*-butyldimethylchlorosilane (46 g, 305 mmol) was added. The mixture was reacted at room temperature for 3 h. After the reaction was completed, the reaction liquid was poured into a saturated aqueous ammonium chloride solution (20 mL), and the mixture was extracted with ethyl acetate (200 mL × 3). The organic phases were combined, washed with saturated brine, dried over anhydrous sodium sulfate, and filtered. The filtrate was concentrated, and the resulting crude product was purified by silica gel column chromatography (PE:EtOAc = 30:1) to give methyl (*S*)-3-((*tert*butyldimethylsilyl)oxy)-2-methylpropanoate.

¹H NMR (400 MHz, CDCl₃) δ3.80 - 3.73 (m, 1H), 3.70 - 3.58 (m, 4H), 2.69 - 2.57(m, 1H), 1.14 (d, *J* = 7.0 Hz, 3H), 0.87 (s, 9H), 0.03 (d, *J* = 1.4 Hz, 6H).

### Step 2: preparation of (R)-3-((tert-butyldimethylsilyl)oxy)-2-methylpropan-1-ol

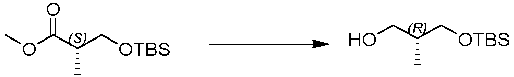

Methyl (*S*)-3-((*tert*-butyldimethylsilyl)oxy)-2-methylpropanoate (49 g, 211 mmol) was dissolved in THF/MeOH (500 mL/100 mL), and the mixture was cooled to 0-5 °C. Lithium borohydride (7 g, 317 mmol) was added, and the mixture was reacted at room temperature for 16 h. An aqueous ammonium chloride solution was first added to quench the reaction, and the mixture was then extracted with ethyl acetate (200 mL × 3). The organic phases were combined, washed with saturated brine, dried over anhydrous sodium sulfate, and filtered, and the filtrate was concentrated to give (*R*)-3-((*tert*-butyldimethylsilyl)oxy)-2-methylpropan-1-ol. The resulting crude product was directly used in the next step.

¹H NMR (400 MHz, CDCl₃) δ 3.77 - 3.69 (m, 1H), 3.68 - 3.49 (m, 3H), 2.82 (brs, 1H), 2.00 - 1.87 (m, 1H), 0.89 (s, 9H), 0.83 (d, *J* = 7.0 Hz, 3H), 0.07 (s, 6H).

### Step 3: preparation of (S)-3-((tert-butyldimethylsilyl)oxy)-2-methylpropyl methanesulfonate

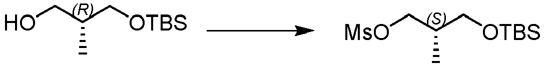

(*R*)-3-((*tert*-Butyldimethylsilyl)oxy)-2-methylpropan-1-ol (35 g, 172 mmol) and triethylamine (34.7 g, 344 mmol) were dissolved in DCM, and the mixture was cooled to 0-5 °C. Methanesulfonyl chloride (23.6 g, 206 mmol) was added, and the mixture was reacted at room temperature for 2 h. An aqueous ammonium chloride solution was added, and the mixture was extracted with DCM. The organic phase was washed with a saturated aqueous sodium chloride solution, dried over anhydrous sodium sulfate, and filtered, and the filtrate was concentrated to give a crude product (*S*)-3-((*tert*-butyldimethylsilyl)oxy)-2-methylpropyl methanesulfonate. The resulting crude product was directly used in the next step.

### Step 4: preparation of (R)-1-benzyl-4-(3-((tert-butyldimethylsilyl)oxy)-2-methylpropyl)piperazine

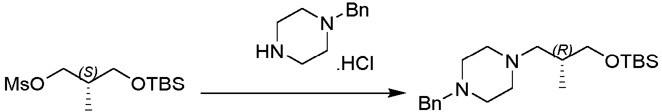

(*S*)-3-((*tert*-Butyldimethylsilyl)oxy)-2-methylpropyl methanesulfonate (48 g, crude) was added to a mixed solution of 1-benzylpiperazine hydrochloride (73 g, 344 mmol) and potassium carbonate (95 g, 688 mmol), and the mixture was heated at 60 °C and reacted for 16 h. The reaction liquid was filtered, and the filtrate was concentrated and purified by column chromatography (PE:EtOAc = 20:1 to 3:1) to give (R)-1-benzyl-4-(3-((*tert*-butyldimethylsilyl)oxy)-2-methylpropyl)piperazine.

LC-MS: (ESI, m/z): [M+H]⁺ = 363.4.

### Step 5: preparation of (R)-3-(4-benzylpiperazin-1-yl)-2-methylpropan-1-ol

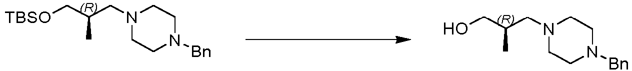

(*R*)-1-Benzyl-4-(3-((*tert*-butyldimethylsilyl)oxy)-2-methylpropyl)piperazine (22 g, 60 mmol) was dissolved in ethanol (300 mL), and the mixture was cooled to 0-5 °C. Concentrated hydrochloric acid (80 mL) was added, and the mixture was reacted at room temperature for 1 h. The reaction liquid was concentrated to remove ethanol, and water (100 mL) was added to the residue. The mixture was then extracted with methyl *tert*-butyl ether (100 mL). The aqueous phase was adjusted to pH = 8-9 with an aqueous sodium carbonate solution, and the mixture was extracted with ethyl acetate (100 mL × 3). The combined ethyl acetate organic phase was dried over anhydrous sodium sulfate and filtered, and the filtrate was concentrated to give (*R*)-3-(4-benzylpiperazin-1-yl)-2-methylpropan-1-ol (*e.e.* = 98.8%).

¹H NMR (400 MHz, CDCl₃) δ 7.34 - 7.28 (m, 4H), 7.27 - 7.22 (m, 1H), 3.70 - 3.59 (m, 1H), 3.54 - 3.39 (m, 3H), 2.86 - 2.25 (m, 10H), 2.18 - 2.10 (m, 1H), 0.73 (d, *J* = 6.8 Hz, 3H).

LC-MS: (ESI, m/z): [M+H]⁺ = 249.2.

### Intermediate 34: (R)-3-(4-benzylpiperazin-1-yl)-2-methoxypropan-1-ol

### Step 1: preparation of (R)-1-(4-benzylpiperazin-1-yl)-3-((tert-butyldimethylsilyl)oxy)propan-2-ol

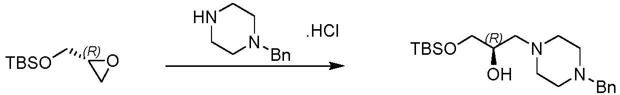

A mixed solution of (*R*)-*tert*-butyldimethyl(oxiran-2-ylmethoxy)silane (20 g, 106 mmol), N-benzylpiperazine hydrochloride (25 g, 117 mmol), and potassium carbonate (29 g, 212 mmol) in THF (15 mL) was heated at reflux for 3 h. After the reaction was completed, the mixture was filtered to remove the solid, and the filtrate was concentrated to give a crude product. The resulting crude product was purified by silica gel column chromatography (PE:EA = 2:1) to give (*R*)-1-(4-benzylpiperazin-1-yl)-3-((*tert*butyldimethylsilyl)oxy)
propan-2-ol.

LC-MS: (ESI, m/z): [M+H]⁺ = 365.3.

### Step 2: preparation of (R)-1-benzyl-4-(3-((tert-butyldimethylsilyl)oxy)-2-methoxypropyl)piperazine

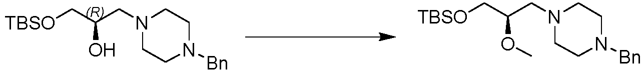

(*R*)-1-(4-Benzylpiperazin-1-yl)-3-((*tert*-butyldimethylsilyl)oxy)propan-2-ol (20.0 g, 55 mmol) was dissolved in THF (200 mL), and NaH (2.6 g, 110 mmol) was added at 0 °C. The mixture was reacted at 25 °C for 30 min and then cooled to 0 °C. Iodomethane (9.7 g, 69 mmol) was added, and the mixture was reacted at room temperature for 3 h. After the reaction was completed, the reaction liquid was slowly poured into a cooled aqueous NH₄Cl solution (200 mL), and then the mixture was extracted with ethyl acetate (200 mL × 3). The organic phases were combined, washed with a saturated NaCl solution, dried over anhydrous Na₂SO₄, and filtered. The filtrate was concentrated, and the resulting crude product was purified by silica gel column chromatography (PE:EA = 5:1) to give (*R*)-1-benzyl-4-(3-((*tert*-butyldimethylsilyl)oxy)-2-methoxypropyl)
piperazine (e.e. = 99.3%).

Chiral resolution: chromatography column: IG 5 µm; column size: 4.6 × 250 mm; mobile phase: Hex:EtOH:DEA = 90:2:0.2; flow rate: 1 mL/min 254 nm; column temperature: 30 °C.

LC-MS: (ESI, m/z): [M+H]⁺ = 379.2.

### Step 3: preparation of (R)-3-(4-benzylpiperazin-1-yl)-2-methoxypropan-1-ol

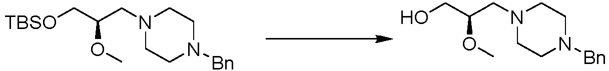

(*R*)-1-Benzyl-4-(3-((*tert*-butyldimethylsilyl)oxy)-2-methoxypropyl)piperazine (15.0 g, 40 mmol) was dissolved in ethanol (100 mL). The mixture was cooled to 0-5 °C, and concentrated hydrochloric acid (40 mL) was added. The mixture was reacted at room temperature for 1 h. After the reaction was completed, the reaction liquid was concentrated to remove ethanol, and water (100 mL) was added to the concentrate. The mixture was extracted with methyl *tert*-butyl ether (100 mL). The aqueous phase was adjusted to pH = 8-9 with an aqueous sodium carbonate solution, and the mixture was extracted with ethyl acetate (100 mL × 3). The combined EA phase was dried over anhydrous sodium sulfate and filtered, and the filtrate was concentrated to give (*R*)-3-(4-benzylpiperazin-1-yl)-2-methoxypropan-1-ol, which was directly used in the next step.

Intermediate 35: 3-(4-benzylpiperazin-1-yl)-2-methylpropan-1-ol

### Step 1: preparation of methyl 3-(4-benzylpiperazin-1-yl)-2-methylpropanoate

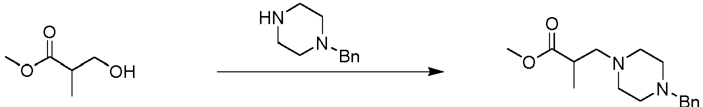

Methyl 3-hydroxy-2-methylpropanoate (3.60 g, 30.47 mmol) was dissolved in dichloromethane (35 mL), and triethylamine (9.25 g, 91.42 mmol) and methanesulfonyl chloride (5.24 g, 45.71 mmol) were added at 0 °C. The mixture was reacted for 2 h. After the reaction was completed, the mixture was warmed to room temperature. Water (50 mL) was added, and the mixture was extracted with ethyl acetate (50 mL × 3). The combined organic phase was washed with a saturated NaCl solution, dried over anhydrous Na₂SO₄, and filtered, and the filtrate was concentrated to give a crude product. The crude product was dissolved in DMF (50 mL), and potassium carbonate (12.64 g, 91.42 mmol) and 1-benzylpiperazine (10.74 g, 60.95 mmol) were added. The reaction liquid was heated to 85 °C and reacted for 16 h. After the reaction was completed, the mixture was cooled to room temperature. Water (200 mL) was added to the reaction liquid, and the mixture was extracted with ethyl acetate (100 mL × 3). The combined organic phase was washed with a saturated NaCl solution, dried over anhydrous Na₂SO₄, and filtered, and the filtrate was concentrated to give a crude product. The resulting crude product was purified by silica gel column chromatography (DCM:MeOH = 20:1) to give methyl 3-(4-benzylpiperazin-1-yl)-2-methylpropanoate.

LC-MS: (ESI, m/z): [M+H]⁺ = 277.3.

### Step 2: preparation of 3-(4-benzylpiperazin-1-yl)-2-methylpropan-1-ol

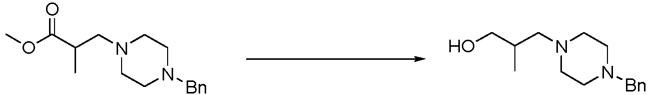

Methyl 3-(4-benzylpiperazin-1-yl)-2-methylpropanoate (1.17 g, 4.23 mmol) was dissolved in THF (10 mL), and DIBAL-H (12.69 mL, 12.69 mmol, 1 M) was added in an ice bath. The mixture was reacted for 3 h. After the reaction was completed, the mixture was warmed to room temperature. A saturated potassium sodium tartrate solution (100 mL) was added to the reaction liquid, and the mixture was extracted with ethyl acetate (50 mL × 3). The combined organic phase was washed with a saturated NaCl solution, dried over anhydrous Na₂SO₄, and filtered, and the filtrate was concentrated to give a crude product. The resulting crude product was purified by silica gel column chromatography (DCM:MeOH = 20:1) to give 3-(4-benzylpiperazin-1-yl)-2-methylpropan-1-ol.

LC-MS: (ESI, m/z): [M+H]⁺ = 249.1.

### Example 1: (R)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

### Step 1: preparation of (R)-1-(2-chloro-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl) dihydropyrimidine-2,4(1H,3H)-dione

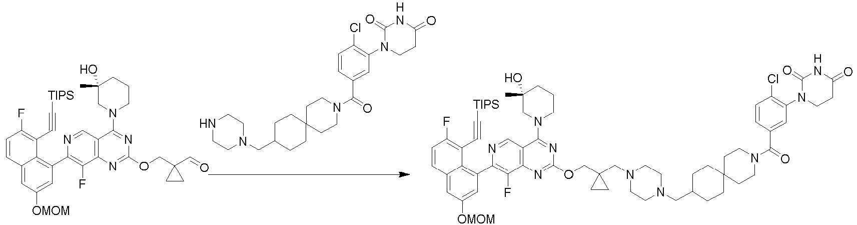

Tetraisopropyl titanate (227 mg, 0.80 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (48 mg, 0.096 mmol) were added to a stirred solution of (*R*)-1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-*d*]pyrimidin-2-yl)oxy)methyl) cyclopropane-1-carbaldehyde (60 mg, 0.08 mmol) in DCM (2 mL), and the mixture was stirred at 30 °C for 2 h. NaBH(OAc)₃ (53 mg, 0.24 mmol) was added to the mixture at room temperature, and the resulting mixture was stirred for 2 h. The mixture was concentrated, and the resulting crude product was purified by silica gel column chromatography (DCM:MeOH = 100:1 to 15:1) to give (*R*)-1-(2-chloro-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]
undecane-3-carbonyl)phenyl)dihydropyrimidine-2,4(1*H*,3*H*)-dione.

LC-MS: (ESI, m/z): [M/2+H]⁺ = 615.9.

### Step 2: preparation of (R)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl) piperazin-1-yl)methyl)-3-azaspiro[5.5]undecane-3-carbonyl)phenyl)dihydropyrimidine-2,4(1H,3H)-dione

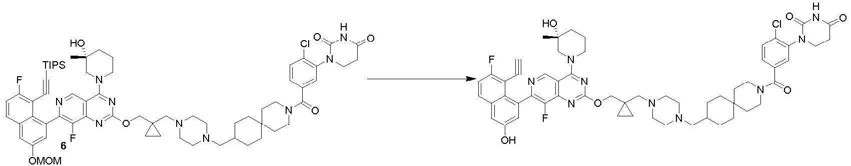

Cesium fluoride (21 mg, 0.14 mmol) was added to a solution of (*R*)-1-(2-chloro-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]
undecane-3-carbonyl)phenyl)dihydropyrimidine-2,4(1*H*,3*H*)-dione (35 mg, 0.028 mmol) in DMF (2 mL), and the mixture was then stirred at room temperature for 30 min. EA and water were added to the reaction liquid, and the organic phase was separated and concentrated under pressure. A mixture of 1,4-dioxane (1 mL) and HCl/1,4-dioxane (6 N, 0.5 mL) was then added to the resulting mixture at room temperature. The mixture was reacted for 0.5 h. The reaction liquid was directly concentrated under reduced pressure, and the resulting crude product was purified by Pre-HPLC to give (*R*)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)
methyl)cyclopropyl)methyl)piperazin-1-yl)methyl)-3-azaspiro[5.5]undecane-3-carbonyl)phenyl) dihydropyrimidine-2,4(1*H*,3*H*)-dione.

LC-MS: (ESI, m/z): [M+H]⁺ = 1030.2.

¹H NMR (400 MHz, DMSO-*d₆*) δ 10.51 (s, 1H), 10.16 (s, 1H), 9.23 - 9.01 (m, 1H), 7.97 (dd, *J* = 9.2, 5.9 Hz, 1H), 7.63 (d, *J =* 8.2 Hz, 1H), 7.54 (d, *J =* 1.9 Hz, 1H), 7.49 - 7.43 (m, 1H), 7.41 - 7.35 (m, 2H), 7.25 - 7.15 (m, 1H), 4.74 (d*, J =* 30.0 Hz, 1H), 4.42 - 4.05 (m, 4H), 3.98 - 3.92 (m, 1H), 3.81-3.69 (m, 1H), 3.66 - 3.49 (m, 4H), 3.29 - 3.18 (m, 2H), 2.78 - 2.65 (m, 2H), 2.45 - 2.16 (m, 9H), 2.13 - 1.90 (m, 4H), 1.76 - 1.59 (m, 5H), 1.57 - 1.38 (m, 5H), 1.34 - 1.21 (m, 3H), 1.20 - 1.12 (m, 3H), 1.11 - 0.92 (m, 4H), 0.66 - 0.57 (m, 2H), 0.45 - 0.40 (m, 2H).

### Example 2: (R)-1-(2-chloro-5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

### Step 1: preparation of (R)-1-(2-chloro-5-(9-((4-((1-(((7-(8-ethyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl) methyl)piperazin-1-yl)methyl)-3-azaspiro[5.5]undecane-3-carbonyl)phenyl)dihydropyrimidine-2,4(1H,3H)-dione

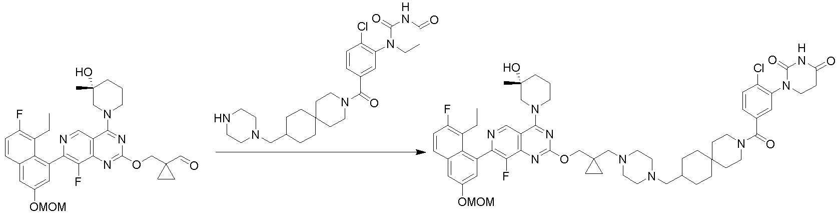

1-(2-Chloro-5-(9-(piperazin-1-ylmethyl)-3-azaspiro[5.5]undecane-3-carbonyl)phenyl)dihydropyrimidine-2,4(1*H*,3*H*)-dione (102 mg, 0.203 mmol) and tetraisopropyl titanate (480 mg, 1.69 mmol) were added to a solution of (*R*)-1-(((7-(8-ethyl-7-fluoro-3-(methoxymethoxy)naphthalen-1-yl)-8-fluoro-4-(3-hydroxy-3-methylpiperidin-1-yl)pyrido [4,3-*d*]pyrimidin-2-yl)oxy)methyl)cyclopropane-1-carbaldehyde (100 mg, 0.169 mmol) in DCM (2 mL), and the mixture was stirred at room temperature for 2 h. NaBH(OAc)₃ (113 mg, 0.507 mmol) was added to the mixture at 0 °C, and the resulting mixture was stirred for 2 h. The mixture was concentrated and purified by silica gel column chromatography (DCM:MeOH = 100:1 to 9:1) to give (*R*)-1-(2-chloro-5-(9-((4-((1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1078.4.

### Step 2: preparation of (R)-1-(2-chloro-5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl) piperazin-1-yl)methyl)-3-azaspiro[5.5]undecane-3-carbonyl)phenyl)dihydropyrimidine-2,4(1H,3H)-dione

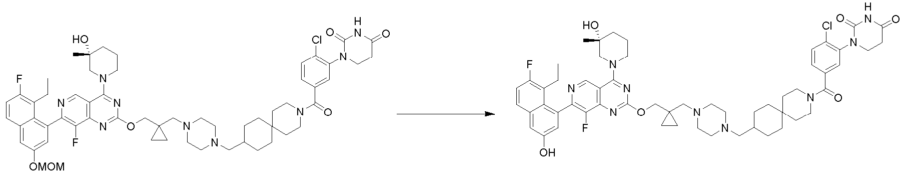

HCl/1,4-dioxane (6 N, 1.0 mL) was added to a solution of (*R*)-1-(2-chloro-5-(9-((4-((1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)phenyl) dihydropyrimidine-2,4(1*H*,3*H*)-dione (100 mg, 0.093 mmol) in 1,4-dioxane (2.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give (*R*)-1-(2-chloro-5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy) methyl)cyclopropyl)methyl)piperazin-1-yl)methyl)-3-azaspiro[5.5]undecane-3-carbonyl)phenyl) dihydropyrimidine-2,4(1*H*,3*H*)-dione.

LC-MS: (ESI, m/z): [M+H]⁺ = 1034.1.
¹H NMR (400 MHz, DMSO-*d₆*) δ10.50 (s, 1H), 9.92 (d, *J* = 3.1 Hz, 1H), 9.21 (s, 1H), 7.79 - 7.73 (m, 1H), 7.63 (d, *J* = 8.2 Hz, 1H), 7.54 (d, *J* = 1.9 Hz, 1H), 7.40 - 7.30 (m, 3H), 7.02 (d, *J* = 2.6 Hz, 1H), 4.73 (d, *J* = 4.6 Hz, 1H), 4.38 - 4.23 (m, 3H), 4.09 - 3.95 (m, 1H), 3.80 - 3.69 (s, 1H), 3.66 - 3.46 (m, 4H), 3.30 - 3.23 (m, 2H), 2.78-2.65 (m, 2H), 2.46 - 2.14 (m, 12H), 2.07 - 1.97 (m, 3H), 1.74 - 1.60 (m, 5H), 1.55 - 1.22 (m, 8H), 1.16 (d, *J =* 9.8 Hz, 3H), 1.12 - 0.93 (m, 4H), 0.77 - 0.69 (m, 3H), 0.63 (s, 2H), 0.40 (s, 2H).

### Example 3: (R)-1-(2-chloro-5-(9-((4-((1-(((7-(8-ethyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

### Step 1: preparation of (R)-1-(7-(8-ethyl-3-(methoxymethoxy)naphthalen-1-yl)-8-fluoro-2-((1-(hydroxymethyl) cyclopropyl)methoxy)pyrido[4,3-d]pyrimidin-4-yl)-3-methylpiperidin-3-ol

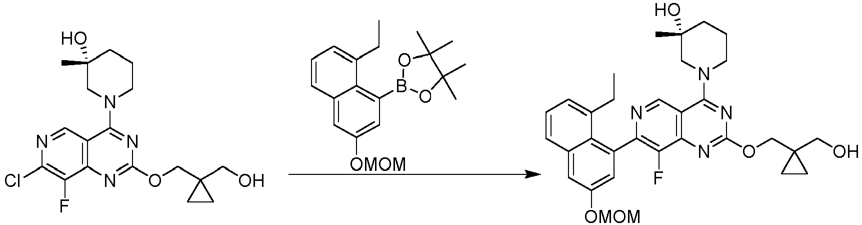

cataCXium A Pd G3 (55 mg, 0.076 mmol) and cesium carbonate (370 mg, 1.134 mmol) were added to a mixed solution of (*R*)-1-(7-chloro-8-fluoro-2-((1-(hydroxymethyl)cyclopropyl)methoxy)pyrido[4,3-*d*]pyrimidin-4-yl)-3-methylpiperidin-3-ol (150 mg, 0.378 mmol) and 2-(8-ethyl-3-(methoxymethoxy)naphthalen-1-yl)-4,4,5,5-tetramethyl-1,3,2-dioxaborolane (155 mg, 0.454 mmol) in 1,4-dioxane/H₂O (2.5 mL/0.5 mL). The mixture was reacted at 80 °C for 16 h under nitrogen atmosphere. Water (10 mL) was added to quench the reaction, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH = 10:1) to give (*R*)-1-(7-(8-ethyl-3-(methoxymethoxy)naphthalen-1-yl)-8-fluoro-2-((1-(hydroxymethyl)cyclopropyl)methoxy)pyrido[4,3-*d*]|pyrimidin-4-y1)-3-methylpiperidin-3-ol.

LC-MS: (ESI, m/z): [M+H]⁺ = 577.2.

### Step 2: preparation of (R)-1-(((7-(8-ethyl-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropane-1-carbaldehyde

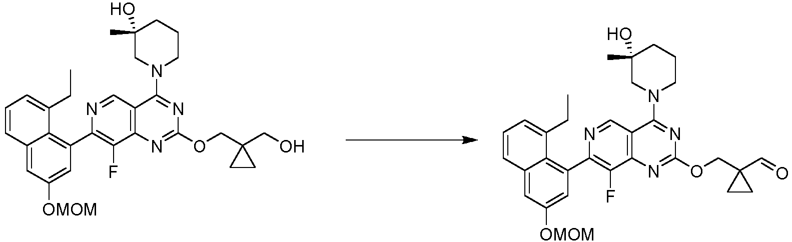

A solution of (*R*)-1-(7-(8-ethyl-3-(methoxymethoxy)naphthalen-1-yl)-8-fluoro-2-((1-(hydroxymethyl) cyclopropyl)methoxy)pyrido[4,3-*d*]pyrimidin-4-yl)-3-methylpiperidin-3-ol (150 mg, 0.26 mmol) in DCM (5 mL) was cooled to 0 °C, and Dess-Martin reagent (221 mg, 0.52 mmol) was added. The mixture was reacted at room temperature for 0.5 h. After the reaction was completed, water (10 mL) was added to quench the reaction, and the mixture was extracted with dichloromethane (30 mL × 3). The organic phases were combined, dried over anhydrous sodium sulfate, and concentrated under reduced pressure. The concentrate was purified by silica gel column chromatography (DCM:MeOH = 10:1) to give (*R*)-1-(((7-(8-ethyl-3-(methoxymethoxy) naphthalen-1-yl)-8-fluoro-4-(3-hydroxy-3-methylpiperidin-1-yl)pyrido[4,3-*d*]pyrimidin-2-yl)oxy)methyl) cyclopropane-1-carbaldehyde.

LC-MS: (ESI, m/z): [M+H]⁺ = 575.2.

### Step 3: preparation of (R)-1-(2-chloro-5-(9-((4-((1-(((7-(8-ethyl-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl)methyl) piperazin-1-yl)methyl)-3-azaspiro[5.5]undecane-3-carbonyl)phenyl)dihydropyrimidine-2,4(1H,3H)-dione

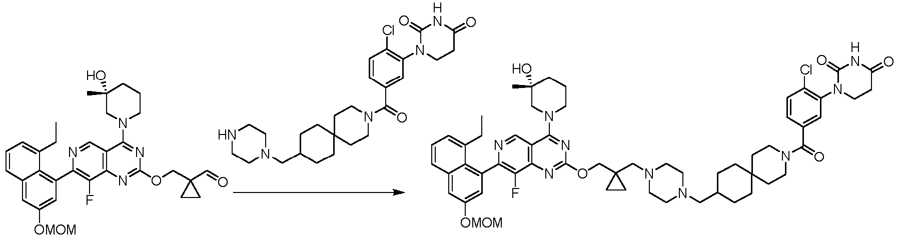

Tetraisopropyl titanate (495 mg, 1.74 mmol) was added to a stirred solution of (*R*)-1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde (100 mg, 0.174 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (105 mg, 0.209 mmol) in DCM (2 mL), and the mixture stirred at 30 °C for 2 h. NaBH(OAc)₃ (116 mg, 0.522 mmol) was added to the mixture at room temperature, and the resulting mixture was stirred for 2 h. The mixture was concentrated and purified by silica gel column chromatography (MeOH:DCM = (0-1):4) to give (*R*)-1-(2-chloro-5-(9-((4-((1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1060.4.

### Step 4: preparation of (R)-1-(2-chloro-5-(9-((4-((1-(((7-(8-ethyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

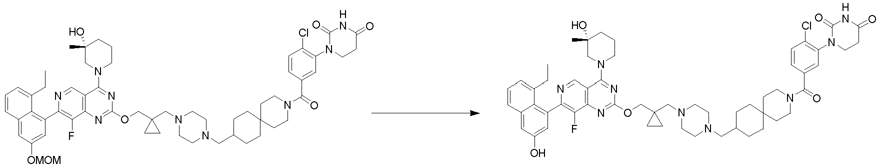

HCl/1,4-dioxane (6 N, 1.0 mL) was added to a solution of (*R*)-1-(2-chloro-5-(9-((4-((1-(((7-(8-ethyl-3-(methoxymethoxy)naphthalen-1-yl)-8-fluoro-4-(3-hydroxy-3-methylpiperidin-1-yl)pyrido [4,3-*d*]pyrimidin-2-yl)oxy)methyl)cyclopropyl)methyl)piperazin-1-yl)methyl)-3-azaspiro[5.5]undecane-3-carbonyl)phenyl) dihydropyrimidine-2,4(1*H*,3*H*)-dione (100 mg, 0.094 mmol) in 1,4-dioxane (2.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give (*R*)-1-(2-chloro-5-(9-((4-((1-(((7-(8-ethyl-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl) methyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1016.2.

¹H NMR (400 MHz, DMSO-*d₆*) δ10.50 (s, 1H), 9.88 (d, *J* = 3.0 Hz, 1H), 9.20 (d, *J* = 6.4 Hz, 1H), 7.69 - 7.56 (m, 2H), 7.54 (d, *J =* 1.9 Hz, 1H), 7.40 - 7.33 (m, 2H), 7.28 (d, *J =* 2.5 Hz, 1H), 7.12 (d, *J =* 7.1 Hz, 1H), 6.97 (d, *J* = 2.6 Hz, 1H), 4.73 (d, *J* = 12.9 Hz, 1H), 4.36 - 4.25 (m, 3H), 4.08 - 3.96 (m, 1H), 3.80 - 3.69 (m, 1H), 3.67 - 3.47 (m, 4H), 3.30 - 3.21 (m, 2H), 2.78 - 2.65 (m, 2H), 2.44 - 2.13 (m, 12H), 2.07 - 1.96 (m, 3H), 1.74 - 1.62 (m, 5H), 1.55 - 1.22 (m, 8H), 1.17 (d, *J=* 11.1 Hz, 3H), 1.11 - 0.93 (m, 4H), 0.85 - 0.78 (m, 3H), 0.63 (s, 2H), 0.40 (s, 2H).

### Example 4: (R)-1-(2-chloro-5-(4-(3-(4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)propyl)piperidine-1-carbonyl)phenyl)dihydropyrimidine-2,4(1H,3H)-dione

### Step 1: preparation of (R)-1-(2-chloro-5-(4-(3-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperazin-1-yl)propyl)piperidine-1-carbonyl)phenyl)dihydropyrimidine-2,4(1H,3H)-dione

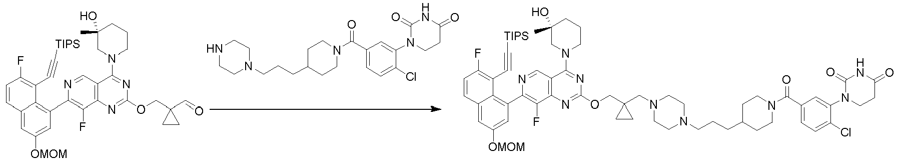

Tetraisopropyl titanate (190 mg, 0.67 mmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropane-1-carbaldehyde (100 mg, 0.134 mmol) and 1-(2-chloro-5-(4-(3-(piperazin-1-yl)propyl)piperidine-1-carbonyl)phenyl)dihydropyrimidine-2,4(1*H*,3*H*)-dione (74 mg, 0.161 mmol) in DCM/AcOH (1.0 mL/0.01 mL), and the mixture was stirred at room temperature for 2 h. NaBH(OAc)₃ (89 mg, 0.402 mmol) was added to the mixture at 0 °C, and the resulting mixture was stirred for 2 h. The mixture was concentrated and purified by silica gel column chromatography (DCM:MeOH = 100:1 to 9:1) to give (*R*)-1-(2-chloro-5-(4-(3-(4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-*d*]pyrimidin-2-yl)oxy)methyl) cyclopropyl)methyl)piperazin-1-yl)propyl)piperidine-1-carbonyl)phenyl)dihydropyrimidine-2,4(1*H*,3*H*)-dione.

LC-MS: (ESI, m/z): [M/2+H]⁺ = 595.9.

### Step 2: preparation of (R)-1-(2-chloro-5-(4-(3-(4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl) piperazin-1-yl)propyl)piperidine-1-carbonyl)phenyl)dihydropyrimidine-2,4(1H,3H)-dione

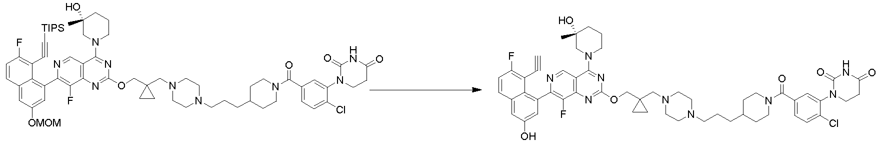

Cesium fluoride (45 mg, 0.295 mmol) was added to a stirred solution of (*R*)-1-(2-chloro-5-(4-(3-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)propyl) piperidine-1-carbonyl)phenyl)dihydropyrimidine-2,4(1*H*,3*H*)-dione (70 mg, 0.059 mmol) in DMF (2 mL), and the mixture was stirred at room temperature for 30 min. The mixture was extracted with ethyl acetate and water. The organic phase was concentrated. A mixture of 1,4-dioxane (1 mL) and HCl/1,4-dioxane (6 N, 0.5 mL) was then added to the residual mixture at room temperature. The mixture was reacted for 0.5 h. An aqueous Na₂CO₃ solution was added to the reaction liquid, and the mixture was adjusted to pH > 7 and extracted with ethyl acetate (10 mL × 3). The organic phase was washed with brine, dried over anhydrous Na₂SO₄, and concentrated. The crude product was purified by high-performance liquid chromatography to give (*R*)-1-(2-chloro-5-(4-(3-(4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)propyl) piperidine-1-carbonyl)phenyl)dihydropyrimidine-2,4(1*H*,3*H*)-dione.

LC-MS: (ESI, m/z): [M/2+H]⁺ = 495.7.

¹H NMR (400 MHz, DMSO-*d₆*) δ 10.49 (s, 1H), 10.15 (s, 1H), 9.12 (d, *J* = 66.1 Hz, 1H), 7.97 (dd, *J* = 9.2, 5.9 Hz, 1H), 7.62 (d, *J* = 8.2 Hz, 1H), 7.54 (d, *J* = 2.0 Hz, 1H), 7.46 (t, *J* = 9.0 Hz, 1H), 7.41 - 7.33 (m, 2H), 7.20 (dd, *J* = 17.2, 2.5 Hz, 1H), 4.73 (d, *J* = 30.7 Hz, 1H), 4.46 - 4.17 (m, 4H), 4.12 - 3.92 (m, 2H), 3.81 - 3.70 (m, 1H), 3.65 - 3.50 (m, 3H), 3.00 (s, 1H), 2.84 - 2.62 (m, 4H), 2.42 - 2.14 (m, 11H), 2.08 - 1.96 (m, 1H), 1.76 - 1.36 (m, 9H), 1.24 - 1.13 (m, 5H), 1.11 - 1.00 (m, 2H), 0.63 (s, 2H), 0.40 (s, 2H).

### Example 5: (R)-1-(2-chloro-5-(4-((2-(1-((1-(((7-(8-ethyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperidin-4-yl)ethoxy)methyl)piperidine-1-carbonyl)phenyl)dihydropyrimidine-2,4(1H,3H)-dione

### Step 1: preparation of (R)-1-(2-chloro-5-(4-((2-(1-((1-(((7-(8-ethyl-7-fluoro-3-(methoxymethoxy)naphthalen-1-yl)-8-fluoro-4-(3-hydroxy-3-methylpiperidin-1-yl)pyrido [4,3-d]pyrimidin-2-yl)oxy)methyl)cyclopropyl) methyl)piperidin-4-yl)ethoxy)methyl)piperidine-1-carbonyl)phenyl)dihydropyrimidine-2,4(1H,3H)-dione

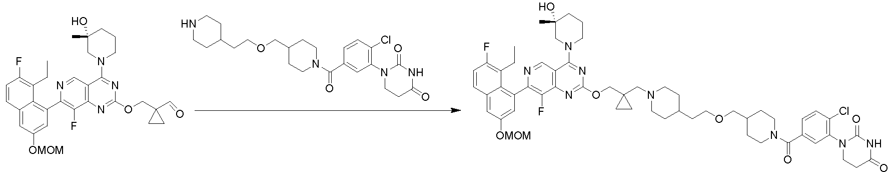

Tetraisopropyl titanate (95 mg, 0.335 mmol) was added to a solution of (*R*)-1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde (40 mg, 0.067 mmol) and 1-(2-chloro-5-(4-((2-(piperidin-4-yl)ethoxy)methyl)piperidine-1-carbonyl)phenyl)dihydropyrimidine-2,4(1*H*,3*H*)-dione (38 mg, 0.08 mmol) in DCM (1 mL), and the mixture was stirred at room temperature for 2 h. NaBH(OAc)₃ (45 mg, 0.201 mmol) was added to the mixture at 0 °C, and the resulting mixture was stirred for 2 h. The mixture was concentrated and purified by silica gel column chromatography (DCM:MeOH = 100:1 to 9:1) to give (*R*)-1-(2-chloro-5-(4-((2-(1-((1-(((7-(8-ethyl-7-fluoro-3-(methoxymethoxy)naphthalen- 1-yl)-8-fluoro-4-(3-hydroxy-3-methylpiperidin-1-yl)pyrido[4,3-*d*]pyrimidin-2-yl)oxy)methyl)cyclopropyl)methyl)piperidin-4-yl)ethoxy)methyl)piperidine-1-carbonyl)phenyl)dihydropyrimidine-2,4(1*H*,3*H*)-dione.

LC-MS: (ESI, m/z): [M+H]⁺ = 1053.5.

### Step 2: preparation of (R)-1-(2-chloro-5-(4-((2-(1-((1-(((7-(8-ethyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl) piperidin-4-yl)ethoxy)methyl)piperidine-1-carbonyl)phenyl)dihydropyrimidine-2,4(1H,3H)-dione

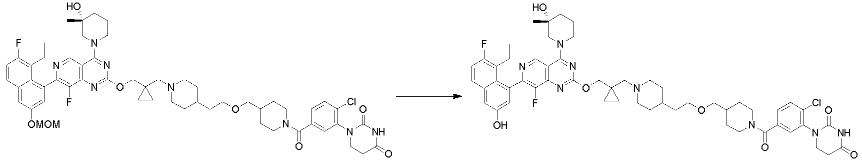

HCl/1,4-dioxane (6 N, 0.5 mL) was added to a stirred solution of (*R*)-1-(2-chloro-5-(4-((2-(1-((1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperidin-4-yl)ethoxy)methyl)piperidine-1-carbonyl) phenyl)dihydropyrimidine-2,4(1*H*,3*H*)-dione (40 mg, 0.038 mmol) in 1,4-dioxane (1.0 mL), and the mixture was reacted at room temperature for 0.5 h. An aqueous Na₂CO₃ solution was added to the reaction liquid, and the mixture was adjusted to pH > 7 and extracted with EtOAc (10 mL × 3). The organic phase was washed with brine, dried over anhydrous Na₂SO₄, and concentrated. The crude product was purified by high-performance liquid chromatography to give (*R*)-1-(2-chloro-5-(4-((2-(1-((1-(((7-(8-ethyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy) methyl)cyclopropyl)methyl)piperidin-4-yl)ethoxy)methyl)piperidine-1-carbonyl)phenyl)dihydropyrimidine-2,4(1*H*,3*H*)-dione.

LC-MS: (ESI, m/z): [M+H]⁺ = 1009.1.

¹H NMR (400 MHz, DMSO-*d₆*) δ 10.51 (s, 1H), 9.93 (s, 1H), 9.21 (s, 1H), 7.76 (dd, *J* = 9.0, 6.0 Hz, 1H), 7.63 (d, *J =* 8.2 Hz, 1H), 7.54 (d, *J* = 1.9 Hz, 1H), 7.38 - 7.31 (m, 3H), 7.02 (d, *J* = 2.5 Hz, 1H), 4.74 (d, *J =* 5.0 Hz, 1H), 4.50 - 4.23 (m, 4H), 4.03 (dd, *J =* 22.0, 13.2 Hz, 1H), 3.79 - 3.71 (m, 1H), 3.66 - 3.48 (m, 3H), 3.43 - 3.34 (m, 3H), 3.20 *(d, J=* 6.1 Hz, 2H), 3.03 (s, 1H), 2.94 - 2.84 (m, 2H), 2.78 - 2.66 (m, 3H), 2.38 - 1.93 (m, 6H), 1.83 - 1.52 (m, 10H), 1.42 - 1.33 (m, 2H), 1.26 - 1.22 (m, 1H), 1.16 (d, *J=* 9.8 Hz, 3H), 1.13 - 1.05 (m, 3H), 0.77 -0.68 (m, 3H), 0.67 - 0.59 (m, 2H), 0.44 - 0.34 (m, 2H).

### Example 6: (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of (R)-1-(5-(9-((4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-d]pyrimidin-2-yl)oxy)methyl) cyclopropyl)methyl)piperazin-1-yl)methyl)-3-azaspiro[5.5]undecane-3-carbonyl)-2-methylphenyl) dihydropyrimidine-2,4(1H,3H)-dione

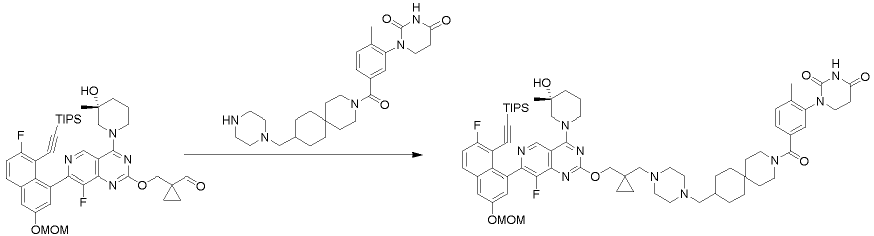

Tetraisopropyl titanate (190 mg, 0.67 mmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropane-1-carbaldehyde (100 mg, 0.134 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (78 mg, 0.161 mmol) in DCM/AcOH (1.0 mL/0.01 mL), and the mixture was stirred at room temperature for 2 h. NaBH(OAc)₃ (89 mg, 0.402 mmol) was added to the mixture at 0 °C, and the resulting mixture was stirred for 2 h. The mixture was concentrated and purified by silica gel column chromatography (DCM:MeOH = 100:1 to 9:1) to give (*R*)-1-(5-(9-((4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-*d*]pyrimidin-2-yl)oxy)methyl) cyclopropyl)methyl)piperazin-1-yl)methyl)-3-azaspiro[5.5]undecane-3-carbonyl)-2-methylphenyl) dihydropyrimidine-2,4(1*H*,3*H*)-dione.

LC-MS: (ESI, m/z): [M/2+H]⁺ = 605.9.

### Step 2: preparation of (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

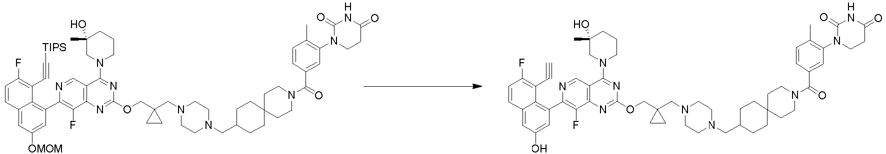

Cesium fluoride (58 mg, 0.385 mmol) was added to a stirred solution of (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1*H*,3*H*)-dione (80 mg, 0.066 mmol) in DMF (2 mL), and the mixture was stirred at room temperature for 30 min and extracted with EA and water. The organic phase was concentrated. A mixture of 1,4-dioxane (1 mL) and HCl/1,4-dioxane (6 N, 0.5 mL) was then added to the residual mixture at room temperature. The mixture was reacted for 0.5 h. An aqueous Na₂CO₃ solution was added to the reaction liquid, and the mixture was adjusted to pH > 7 and extracted with ethyl acetate (10 mL × 3). The organic phase was washed with brine, dried over anhydrous Na₂SO₄, and concentrated. The crude product was purified by high-performance liquid chromatography to give (*R*)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1010.2.

¹H NMR (400 MHz, DMSO-*d₆*) δ 10.39 (s, 1H), 10.18 (s, 1H), 9.12 (d, *J* = 66.8 Hz, 1H), 7.98 (dd, *J* = 9.2, 6.0 Hz, 1H), 7.46 (t, *J* = 9.0 Hz, 1H), 7.39 (s, 1H), 7.35 - 7.28 (m, 2H), 7.26 - 7.17 (m, 2H), 4.76 (d, *J = 29.8* Hz, 1H), 4.40 - 4.21 (m, 3H), 4.12 - 3.92 (m, 2H), 3.85 - 3.76 (m, 1H), 3.65 - 3.42 (m, 4H), 3.32 - 3.21 (m, 3H), 2.82 - 2.65 (m, 2H), 2.48 - 2.22 (m, 9H), 2.21 (s, 3H), 2.09 - 1.97 (m, 3H), 1.74 - 1.58 (m, 5H), 1.55 - 1.37 (m, 5H), 1.27 (d*, J* = 31.2 Hz, 3H), 1.16 (m, 3H), 1.11 - 0.95 (m, 4H), 0.67 - 0.59 (m, 2H), 0.44 - 0.34 (m, 2H).

### Example 7: (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of (R)-1-(5-(9-((4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-d]pyrimidin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1H,3H)-dione

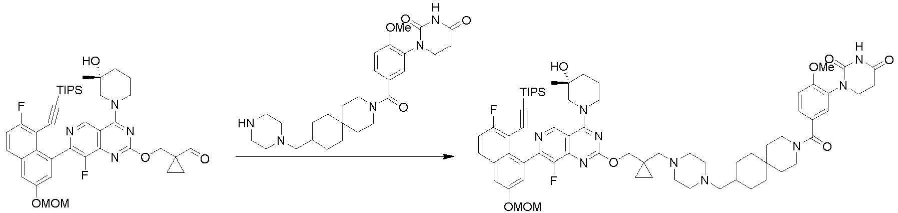

Tetraisopropyl titanate (190 mg, 0.67 mmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropane-1-carbaldehyde (100 mg, 0.134 mmol) and 1-(2-methoxy-5-(9-(piperazin-1-ylmethyl)-3-azaspiro[5.5]undecane-3-carbonyl)phenyl)dihydropyrimidine-2,4(1*H*,3*H-*dione (80 mg, 0.161 mmol) in DCM/AcOH (1.0 mL/0.01 mL), and the mixture was stirred at room temperature for 2 h. NaBH(OAc)₃ (89 mg, 0.402 mmol) was added to the mixture at 0 °C, and the resulting mixture was stirred for 2 h. The mixture was concentrated and purified by silica gel column chromatography (DCM:MeOH = 100:1 to 9:1) to give (*R*)-1-(5-(9-((4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-*d*]pyrimidin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1*H*,3*H*)-dione.

LC-MS: (ESI, m/z): [M/2+H]⁺ = 613.9.

### Step 2: preparation of (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

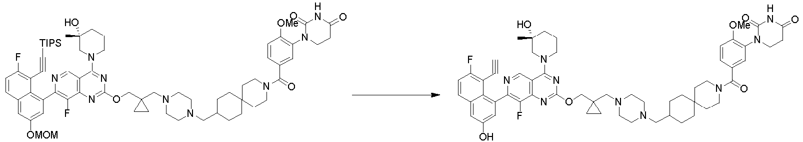

Cesium fluoride (55 mg, 0.365 mmol) was added to a stirred solution of (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1*H*,3*H*)-dione (90 mg, 0.073 mmol) in DMF (2 mL), and the mixture was stirred at room temperature for 30 min and extracted with EA and water. The organic phase was concentrated. A mixture of 1,4-dioxane (1 mL) and HCl/1,4-dioxane (6 N, 0.5 mL) was then added to the residual mixture at room temperature. The mixture was reacted for 0.5 h. An aqueous Na₂CO₃ solution was added to the reaction liquid, and the mixture was adjusted to pH > 7 and extracted with ethyl acetate (10 mL × 2). The organic phase was washed with brine, dried over anhydrous Na₂SO₄, and concentrated. The crude product was purified by high-performance liquid chromatography to give (*R*)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1*H*,3*H*)-dione.

LC-MS: (ESI, m/z): [M+H]⁺ = 1026.2.

¹H NMR (400 MHz, DMSO-*d₆*) δ 10.32 (s, 1H), 10.15 (s, 1H), 9.12 (d, *J* = 65.7 Hz, 1H), 7.97 (dd, *J* = 9.2, 5.9 Hz, 1H), 7.46 (t, *J* = 9.0 Hz, 1H), 7.40 - 7.30 (m, 3H), 7.24 - 7.12 (m, 2H), 4.74 (d, *J* = 30.2 Hz, 1H), 4.40 - 4.22 (m, 3H), 4.12 - 3.91 (m, 2H), 3.84 (s, 3H), 3.65 - 3.36 (m, 7H), 2.68 (t, *J =* 6.5 Hz, 2H), 2.45 - 2.12 (m, 10H), 2.07 - 1.96 (m, 3H), 1.73 - 1.60 (m, 5H), 1.55 - 1.39 (m, 5H), 1.35 - 1.21 (m, 3H), 1.17 (d, *J=* 16.5 Hz, 3H), 1.10 - 0.92 (m, 4H), 0.67 - 0.59 (m, 2H), 0.44 - 0.34 (m, 2H).

### Example 8: (R)-1-(2-chloro-5-(4-((2-(1-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperidin-4-yl)ethoxy)methyl)piperidine-1-carbonyl)phenyl)dihydropyrimidine-2,4(1H,3H)-dione

### Step 1: preparation of (R)-1-(2-chloro-5-(4-((2-(1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperidin-4-yl)ethoxy)methyl)piperidine-1-carbonyl)phenyl)dihydropyrimidine-2,4(1H,3H)-dione

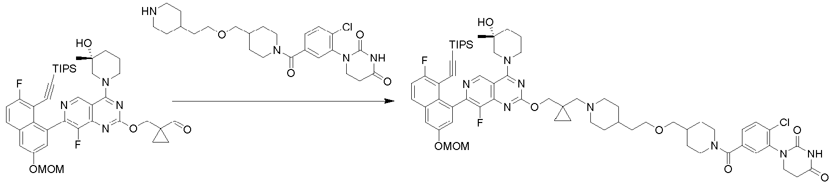

Tetraisopropyl titanate (190 mg, 0.67 mmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde (100 mg, 0.134 mmol) and 1-(2-chloro-5-(4-((2-(piperidin-4-yl)ethoxy)methyl)piperidine-1-carbonyl)phenyl)dihydropyrimidine-2,4(1*H*,3*H*)-dione (77 mg, 0.161 mmol) in DCM/AcOH (1.0 mL/0.01 mL), and the mixture was stirred at room temperature for 2 h. NaBH(OAc)₃ (89 mg, 0.402 mmol) was added to the mixture at 0 °C, and the resulting mixture was stirred for 2 h. The mixture was concentrated and purified by silica gel column chromatography (DCM:MeOH = 100:1 to 9:1) to give (*R*)-1-(2-chloro-5-(4-((2-(1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperidin-4-yl)ethoxy)methyl)piperidine-1-carbonyl)phenyl)dihydropyrimidine-2,4(1*H*,3*H*)-dione.

LC-MS: (ESI, m/z): [M/2+H]⁺ = 603.4.

### Step 2: preparation of (R)-1-(2-chloro-5-(4-((2-(1-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl) piperidin-4-yl)ethoxy)methyl)piperidine-1-carbonyl)phenyl)dihydropyrimidine-2.4(1H,3H)-dione

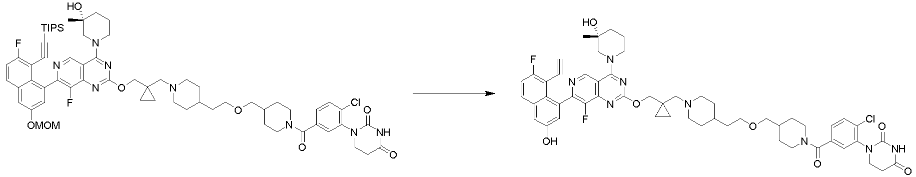

Cesium fluoride (50 mg, 0.33 mmol) was added to a stirred solution of (*R*)-1-(2-chloro-5-(4-((2-(1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperidin-4-yl)ethoxy) methyl)piperidine-1-carbonyl)phenyl)dihydropyrimidine-2,4(1*H*,3*H*)-dione (80 mg, 0.066 mmol) in DMF (2 mL), and the mixture was stirred at room temperature for 30 min. The mixture was extracted with ethyl acetate and water. The organic phase was concentrated. A mixture of 1,4-dioxane (1 mL) and HCl/1,4-dioxane (6 N, 0.5 mL) was then added to the residual mixture at room temperature. The mixture was reacted for 0.5 h. An aqueous Na₂CO₃ solution was added to the reaction liquid, and the mixture was adjusted to pH > 7 and extracted with ethyl acetate (10 mL × 3). The organic phase was washed with brine, dried over anhydrous Na₂SO₄, and concentrated. The crude product was purified by high-performance liquid chromatography to give (*R*)-1-(2-chloro-5-(4-((2-(1-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperidin-4-yl)ethoxy) methyl)piperidine-1-carbonyl)phenyl)dihydropyrimidine-2,4(1*H*,3*H*)-dione.

LC-MS: (ESI, m/z): [M+H]⁺ = 1005.1

¹H NMR (400 MHz, DMSO-*d₆*) δ10.50 (s, 1H), 10.15 (s, 1H), 9.12 (d, *J* = 66.1 Hz, 1H), 7.97 (dd, *J* = 9.2, 5.9 Hz, 1H), 7.63 (d, *J* = 8.2 Hz, 1H), 7.53 (d, *J* = 1.9 Hz, 1H), 7.50 - 7.42 (m, 1H), 7.41 - 7.34 (m, 2H), 7.20 (dd, *J =* 17.0, 2.4 Hz, 1H), 4.74 (d, *J =* 30.2 Hz, 1H), 4.52 - 4.21 (m, 4H), 4.13 - 3.92 (m, 2H), 3.80 - 3.50 (m, 4H), 3.38 - 3.33 (m, 2H), 3.27 - 3.14 (m, 3H), 3.06 - 2.86 (m, 3H), 2.79 - 2.66 (m, 3H), 2.34 - 2.22 (m, 2H), 2.08 - 1.97 (m, 1H), 1.83 - 1.53 (m, 10H), 1.42 - 1.34 (m, 2H), 1.30 - 1.22(m, 2H), 1.20 - 1.13 (m, 3H), 1.12 - 1.05 (m, 3H), 0.67 - 0.59 (m, 2H), 0.44 - 0.34 (m, 2H).

### Example 9: (R)-1-(2-chloro-5-(9-(2-(4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)ethyl)-3-azaspiro[5.5]undecane-3-carbonyl)phenyl)dihydropyrimidine-2,4(1H,3H)-dione

### Step 1: preparation of 2-(3-azaspiro[5.5]undecan-9-yl)acetaldehyde

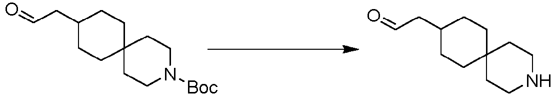

*tert*-Butyl 9-(2-oxoethyl)-3-azaspiro[5.5]undecane-3-carboxylate (250 mg, 0.85 mmol) was dissolved in DCM (2 mL), and then TFA (1 mL) was added. The mixture was stirred at room temperature for 1 h. The reaction liquid was directly concentrated under reduced pressure to give 2-(3-azaspiro[5.5]undecan-9-yl)acetaldehyde. The crude product was directly used in the next step without further purification.

LC-MS: (ESI, m/z): [M+H]⁺ = 196.2.

### Step 2: preparation of 2-(3-(4-chloro-3-(2,4-dioxotetrahydropyrimidin-1(2H)-yl)benzoyl)-3-azaspiro[5.5] undecan-9-yl)acetaldehyde

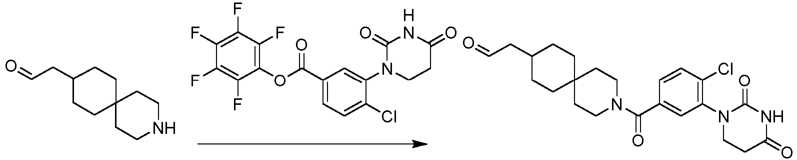

DIEA (496 mg, 3.84 mmol) and pentafluorophenyl 4-chloro-3-(2,4-dioxotetrahydropyrimidin-1(2*H*)-yl)benzoate (334 mg, 0.77 mmol) were added to a solution of 2-(3-azaspiro[5.5]undecan-9-yl)acetaldehyde (150 mg, 0.77 mmol) in DMF (5 mL), and the reaction liquid was stirred at room temperature for 1 h. Water was added to the reaction liquid, and the mixture was extracted with ethyl acetate. The organic phases were combined, dried over anhydrous sodium sulfate, and filtered. The filtrate was concentrated under reduced pressure, and the resulting crude product was purified by silica gel column chromatography (MeOH:DCM = 100:1 to 5:1) to give 2-(3-(4-chloro-3-(2,4-dioxotetrahydropyrimidin-1(2*H*)-yl)benzoyl)-3-azaspiro[5.5]
undecan-9-yl)acetaldehyde.

LC-MS: (ESI, m/z): [M+H]⁺ = 446.2.

### Step 3: preparation of tert-butyl (R)-4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-d]pyrimidin-2-yl)oxy)methyl) cyclopropyl)methyl)piperazine-1-carboxylate

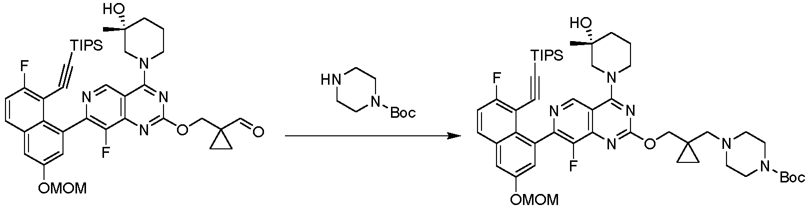

Tetraisopropyl titanate (286 mg, 1.005 mmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde (150 mg, 0.201 mmol) and *tert*-butyl piperazine-1-carboxylate (45 mg, 0.241 mmol) in DCM/AcOH (2.0 mL/0.02 mL), and the mixture was stirred at room temperature for 2 h. NaBH(OAc)₃ (134 mg, 0.603 mmol) was added to the mixture at 0 °C, and the resulting mixture was stirred for 2 h. The mixture was concentrated and purified by silica gel column chromatography (DCM:MeOH = 100:1 to 9:1) to give *tert*-butyl (*R*)-4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropyl)methyl)piperazine-1-carboxylate.

LC-MS: (ESI, m/z): [M+H]⁺ = 915.5.

### Step 4: preparation of (R)-1-(8-fluoro-7-(7-fluoro-3-hydroxy-8-((triisopropylsilyl)ethynyl)naphthalen-1-yl)-2-((1-(piperazin-1-ylmethyl)cyclopropyl)methoxy)pyrido [4,3-d]pyrimidin-4-yl)-3-methylpiperidin-3-ol

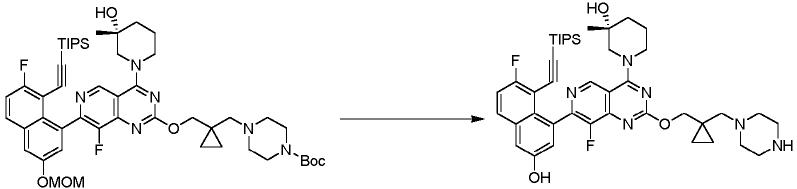

A mixture of HCl/1,4-dioxane (6 N, 1 mL) was added to a solution of *tert*-butyl (*R*)-4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazine-1-carboxylate (120 mg, 0.131 mmol) in 1,4-dioxane (2 mL), and the mixture was reacted at room temperature for 1 h. After the reaction was completed, a saturated sodium carbonate solution was added to adjust the pH >7, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 9:1) to give (*R*)-1-(8-fluoro-7-(7-fluoro-3-hydroxy-8-((triisopropylsilyl)ethynyl)naphthalen-1-yl)-2-((1-(piperazin-1-ylmethyl)cyclopropyl)methoxy)pyrido [4,3-*d*]pyrimidin-4-yl)-3-methylpiperidin-3-ol.

LC-MS: (ESI, m/z): [M+H]⁺ = 771.5.

### Step 5: preparation of (R)-1-(2-chloro-5-(9-(2-(4-((1-(((8-fluoro-7-(7-fluoro-3-hydroxy-8-((triisopropylsilyl) ethynyl)naphthalen-1-yl)-4-(3-hydroxy-3-methylpiperidin-1-yl)pyrido[4,3-d]pyrimidin-2-yl)oxy)methyl) cyclopropyl)methyl)piperazin-1-yl)ethyl)-3-azaspiro[5.5]undecane-3-carbonyl)phenyl)dihydropyrimidine-2,4(1H,3H)-dione

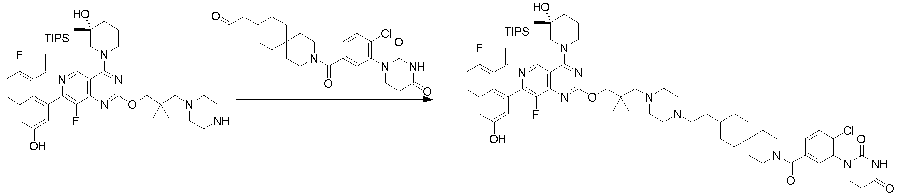

Tetraisopropyl titanate (138 mg, 0.485 mmol) was added to a stirred solution of (*R*)-1-(8-fluoro-7-(7-fluoro-3-hydroxy-8-((triisopropylsilyl)ethynyl)naphthalen-1-yl)-2-((1-(piperazin-1-ylmethyl)cyclopropyl)methoxy) pyrido[4,3-*d*]pyrimidin-4-yl)-3-methylpiperidin-3-ol (75 mg, 0.097 mmol) and 2-(3-(4-chloro-3-(2,4-dioxotetrahydropyrimidin-1(2*H*)-yl)benzoyl)-3-azaspiro[5.5]undecan-9-yl)acetaldehyde (52 mg, 0.1116 mmol) in DCM (1 mL)/AcOH (0.1 mL), and the mixture was stirred at 30 °C for 2 h. NaBH(OAc)₃ (65 mg, 0.291 mmol) was added to the mixture at room temperature, and the resulting mixture was stirred for 2 h. After the reaction was completed, the mixture was concentrated and purified by silica gel column chromatography (DCM:MeOH = 1:0 to 4:1) to give (*R*)-1-(2-chloro-5-(9-(2-(4-((1-(((8-fluoro-7-(7-fluoro-3-hydroxy-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropyl)methyl)piperazin-1-yl)ethyl)-3-azaspiro[5.5]undecane-3-carbonyl)phenyl) dihydropyrimidine-2,4(1*H*,3*H*)-dione.

LC-MS: (ESI, m/z): [1/2M+H]⁺ = 600.9.

### Step 6: preparation of (R)-1-(2-chloro-5-(9-(2-(4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl) methyl)piperazin-1-yl)ethyl)-3-azaspiro[5.5]undecane-3-carbonyl)phenyl)dihydropyrimidine-2,4(1H,3H-dione

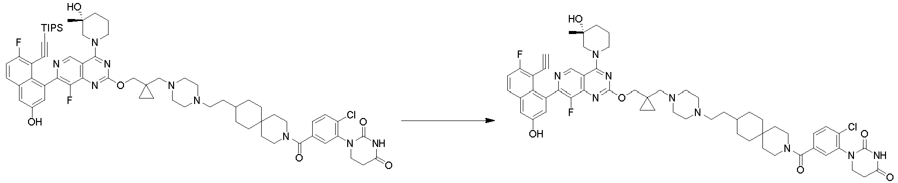

Cesium fluoride (44 mg, 0.29 mmol) was added to a stirred solution of (*R*)-1-(2-chloro-5-(9-(2-(4-((1-(((8-Huoro-7-(7-fluoro-3-hydroxy-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)ethyl)-3-azaspiro[5.5] undecane-3-carbonyl)phenyl)dihydropyrimidine-2,4(1*H*,3*H*)-dione (70 mg, 0.058 mmol) in DMF (2 mL), and the mixture was stirred at 40 °C for 60 min. The mixture was extracted with EA and water. The organic phases were combined and washed with saturated brine. The organic phase was dried over anhydrous sodium sulfate and concentrated to give a crude product. The crude product was purified by high-performance liquid chromatography to give (*R*)-1-(2-chloro-5-(9-(2-(4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl) methyl)piperazin-1-yl)ethyl)-3-azaspiro[5.5]undecane-3-carbonyl)phenyl)dihydropyrimidine-2,4(1*H*,3*H*)-dione.

LC-MS: (ESI, m/z): [M+H]⁺ = 1044.2.

¹H NMR(400 MHz, DMSO-*d₆*) δ10.50 (s, 1H), 10.15 (s, 1H), 9.12 (d, *J* = 65.6 Hz, 1H), 7.97 (dd, *J* = 9.2, 6.0 Hz, 1H), 7.63 (d, *J* = 8.2 Hz, 1H), 7.54 (d, *J* = 1.9 Hz, 1H), 7.49 - 7.42 (m, 1H), 7.41 - 7.35 (m, 2H), 7.20 (dd, *J =* 17.1, 2.4 Hz, 1H), 4.74 (d, *J =* 30.3 Hz, 1H), 4.40 - 4.21 (m, 3H), 4.12 - 3.92 (m, 2H), 3.80 - 3.70 (m, 1H), 3.68 - 3.46 (m, 4H), 3.42 - 3.32 (m, 1H), 3.29 - 3.18 (m, 2H), 2.77 - 2.66 (m, 2H), 2.45 - 2.16 (m, 11H), 2.07 - 1.96 (m, 1H), 1.75 - 1.57 (m, 5H), 1.53 - 1.37 (s, 4H), 1.39 - 1.12 (m, 9H), 1.11 - 0.95 (m, 4H), 0.67 - 0.59 (m, 2H), 0.44 - 0.37 (m, 2H).

### Example 10: (R)-1-(2-chloro-5-(4-(2-((1-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperidin-4-yl)methoxy)ethyl)piperidine-1-carbonyl)phenyl)dihydropyrimidine-2,4(1H,3H)-dione

### Step 1: preparation of tert-butyl 4-(2-((1-benzylpiperidin-4-yl)methoxy)ethyl)piperidine-1-carboxylate

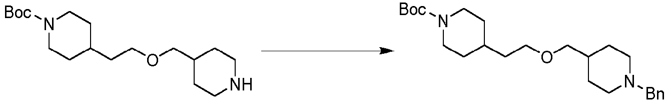

Acetic acid (0.01 mL) was added to a mixed solution of *tert*-butyl 4-(2-(piperidin-4-ylmethoxy)ethyl)
piperidine-1-carboxylate (100 mg, 0.31 mmol) and benzaldehyde (39 mg, 0.37 mmol) in 1,2-dichloroethane/methanol (0.5 mL/0.5 mL). The mixture was stirred at 30 °C for 1 h. NaBH₃CN (39 mg, 0.62 mmol) was added at 0 °C, and the mixture was reacted at room temperature for 2 h. After the reaction was completed, H₂O (10 mL) was added, and the mixture was extracted with ethyl acetate (30 mL × 3). The combined organic phase was dried over anhydrous sodium sulfate, filtered, and concentrated in vacuum. The crude product was purified by silica gel column chromatography (DCM:MeOH = 20:1) to give *tert*-butyl 4-(2-((1-benzylpiperidin-4-yl)methoxy)ethyl)piperidine-1-carboxylate.

LC-MS: (ESI, m/z): [M+H]⁺ = 417.3.

### Step 2: preparation of 1-benzyl-4-((2-(piperidin-4-yl)ethoxy)methyl)piperidine

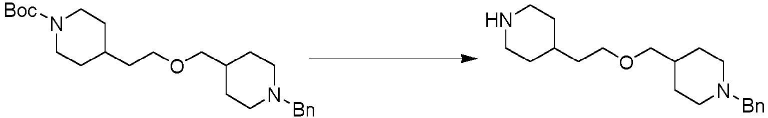

TFA (1 mL) was added to a solution of *tert*-butyl 4-(2-((1-benzylpiperidin-4-yl)methoxy)ethyl)piperidine-1-carboxylate (100 mg, 0.24 mmol) in DCM (2 mL). The mixture was stirred at room temperature for 1 h, and the reaction liquid was directly concentrated under reduced pressure to give 1-benzyl-4-((2-(piperidin-4-yl)ethoxy)methyl)piperidine, which was directly used in the next step.

### Step 3: preparation of 1-(5-(4-(2-((1-benzylpiperidin-4-yl)methoxy)ethyl)piperidine-1-carbonyl)-2-chlorophenyl) dihydropyrimidine-2,4(1H,3H)-dione

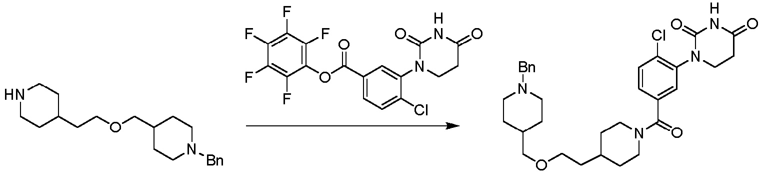

1- Benzyl-4-((2-(piperidin-4-yl)ethoxy)methyl)piperidine (100 mg, 0.32 mmol) was dissolved in DMSO (2 mL), and pentafluorophenyl 4-chloro-3-(2,4-dioxotetrahydropyrimidin-1(2*H*)-yl)benzoate (110 mg, 0.34 mmol) and DIEA (93 mg, 0.96 mmol) were added. The reaction liquid was stirred at room temperature for 2 h. Water (20 mL) was added, and the mixture was extracted with ethyl acetate (20 mL × 2). The organic phases were combined, washed with saturated brine (30 mL × 3), dried over anhydrous sodium sulfate, and filtered. The filtrate was concentrated and purified by silica gel column chromatography (DCM:MeOH = 100:1 to 5:1) to give 1-(5-(4-(2-((1-benzylpiperidin-4-yl)methoxy)ethyl)piperidine-1-carbonyl)-2-chlorophenyl)
dihydropyrimidine-2,4(1*H*,3*H*)-dione.

LC-MS: (ESI, m/z): [M+H]⁺= 567.2.

### Step 4: preparation of 1-(2-chloro-5-(4-(2-(piperidin-4-ylmethoxy)ethyl)piperidine-1-carbonyl)phenyl) dihydropyrimidine-2,4(1H,3H)-dione

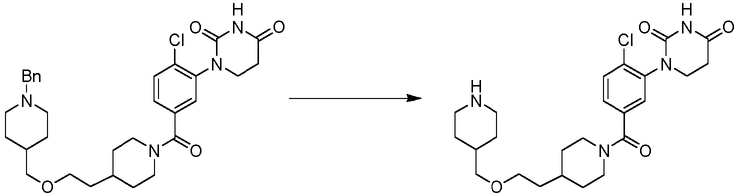

1-1-(5-(4-(2-((1-Benzylpiperidin-4-yl)methoxy)ethyl)piperidine-1-carbonyl)-2-chlorophenyl)dihydropyrimidine-2,4(1*H*,3*H*)-dione (200 mg, 0.35 mmol) was dissolved in DCM (2 mL), and 1-chloroethyl chloroformate (150 mg, 1.05 mmol) and DIEA (90 mg, 0.7 mmol) were added. The mixture was reacted at room temperature for 30 min and concentrated under reduced pressure. MeOH (2 mL) was added to the concentrate for dissolution, and the mixture was heated to 50 °C and reacted for 30 min. After the reaction was completed, the mixture was concentrated and purified by silica gel column chromatography (DCM:MeOH (NH₃) = 100:1 to 9:1) to give 1-(2-chloro-5-(4-(2-(piperidin-4-ylmethoxy)ethyl)piperidine-1-carbonyl)phenyl)dihydropyrimidine-2,4(1*H*,3*H*)-dione.

MS (ESI) m/z: [M+H]⁺ = 477.3.

### Step 5: preparation of (R)-1-(2-chloro-5-(4-(2-((1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperidin-4-yl)methoxy)ethyl)piperidine-1-carbonyl)phenyl) dihydropyrimidine-2,4(1H,3H)-dione

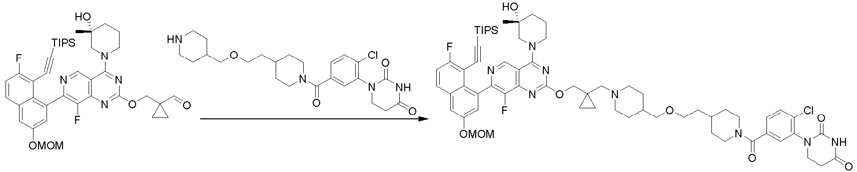

Tetraisopropyl titanate (190 mg, 0.67 mmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde (100 mg, 0.134 mmol) and 1-(2-chloro-5-(4-(2-(piperidin-4-ylmethoxy)ethyl)piperidine-1-carbonyl)phenyl)dihydropyrimidine-2,4(1*H*,3*H*)-dione (77 mg, 0.161 mmol) in DCM/AcOH (1.0 mL/0.01 mL), and the mixture was stirred at room temperature for 2 h. NaBH(OAc)₃ (89 mg, 0.402 mmol) was added to the mixture at 0 °C, and the resulting mixture was stirred for 2 h. The mixture was concentrated and purified by silica gel column chromatography (DCM:MeOH = 100:1 to 9:1) to give (*R*)-1-(2-chloro-5-(4-(2-((1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperidin-4-yl)methoxy)ethyl)piperidine-1-carbonyl)phenyl)
dihydropyrimidine-2,4(1*H*,3*H*)-dione.

LC-MS: (ESI, m/z): [M/2+H]⁺ = 603.4.

### Step 6: preparation of (R)-1-(2-chloro-5-(4-(2-((1-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl) methyl)piperidin-4-yl)methoxy)ethyl)piperidine-1-carbonyl)phenyl)dihydropyrimidine-2,4(1H,3H)-dione

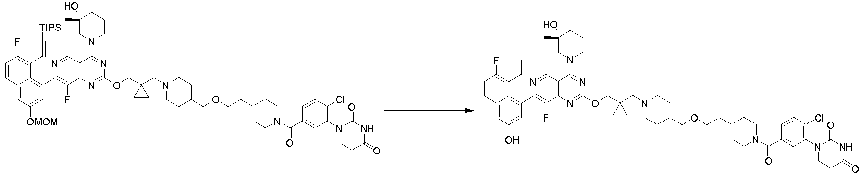

Cesium fluoride (58 mg, 0.385 mmol) was added to a stirred solution of (*R*)-1-(2-chloro-5-(4-(2-((1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperidin-4-yl)methoxy) ethyl)piperidine-1-carbonyl)phenyl)dihydropyrimidine-2,4(1*H*,3*H*)-dione (100 mg, 0.083 mmol) in DMF (2 mL), and the mixture was stirred at room temperature for 30 min. The mixture was extracted with ethyl acetate and water. The organic phase was concentrated. A mixture of 1,4-dioxane (2 mL) and HCl/1,4-dioxane (6 N, 1 mL) was then added to the residual mixture at room temperature. The mixture was reacted for 0.5 h. An aqueous Na₂CO₃ solution was added to the reaction liquid, and the mixture was adjusted to pH > 7 and extracted with ethyl acetate (10 mL × 3). The organic phase was washed with brine, dried over anhydrous Na₂SO₄, and concentrated. The crude product was purified by high-performance liquid chromatography to give (*R*)-1-(2-chloro-5-(4-(2-((1-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperidin-4-yl)methoxy)ethyl)piperidine-1-carbonyl)phenyl)dihydropyrimidine-2,4(1*H*,3*H*)-dione.

LC-MS: (ESI, m/z): [M/2+H]⁺ = 1005.1.

¹H NMR (400 MHz, DMSO-*d₆*) δ10.50 (s, 1H), 10.15 (s, 1H), 9.12 *(d, J =* 66.7 Hz, 1H), 7.97 (dd, *J =* 9.2, 5.9 Hz, 1H), 7.62 (d, *J =* 8.2 Hz, 1H), 7.53 (d, *J =* 1.9 Hz, 1H), 7.49 - 7.43 (t, *J =* 9.1 Hz, 1H), 7.41 - 7.33 (m, 2H), 7.20 (dd, *J =* 17.4, 2.4 Hz, 1H), 4.74 (d, *J =* 31.0 Hz, 1H), 4.49 - 4.20 (m, 4H), 4.13 - 3.92 (m, 2H), 3.81 - 3.70 (m, 1H), 3.68 - 3.43 (m, 3H), 3.38 - 3.33 (m, 2H), 3.32 - 3.23 (m, 2H), 3.14 (d, *J =* 6.1 Hz, 2H), 3.06 - 2.86 (m, 3H), 2.80 - 2.65 (m, 3H), 2.33 - 2.23 (m, 2H), 2.10 - 1.95 (m, 1H), 1.86 - 1.76 (m, 2H), 1.75 - 1.53 (m, 8H), 1.47 - 1.38 (m, 3H), 1.20 - 1.13 (m, 3H), 1.16 (d, *J =* 16.8 Hz, 3H), 0.69 - 0.56 (m, 2H), 0.43 - 0.34 (m, 2H).

### Example 11: (R)-1-(2-chloro-5-(9-((4-((1-(((7-(8-ethynyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

### Step 1: preparation of (R)-1-(8-fluoro-2-((1-(hydroxymethyl)cyclopropyl)methoxy)-7-(3-(methoxymethoxy)-8-((triisopropylsilyl)ethynyl)naphthalen-1-yl)pyrido[4,3-d]pyrimidin-4-yl)-3-methylpiperidin-3-ol

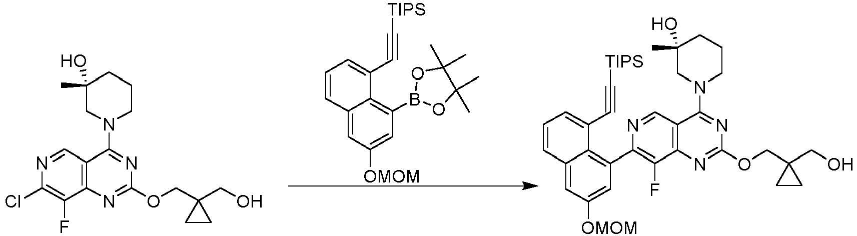

Cesium carbonate (370 mg, 1.134 mmol) and cataXium A Pd G3 (55 mg, 0.076 mmol) were added to a mixed solution of (*R*)-1-(7-chloro-8-fluoro-2-((1-(hydroxymethyl)cyclopropyl)methoxy)pyrido[4,3-*d*]pyrimidin-4-yl)-3-methylpiperidin-3-ol (150 mg, 0.378 mmol) and triisopropyl ((6-(methoxymethoxy)-8-(4,4,5,5-tetramethyl-1,3,2-dioxaborolan-2-yl)naphthalen-1-yl)ethynyl)silane (224 mg, 0.454 mmol) in 1,4-dioxane (3 mL) and H₂O (0.5 mL), and the mixture was reacted at 85 °C overnight under N₂ atmosphere. The mixture was filtered and concentrated, and the resulting crude product was purified by silica gel column chromatography (DCM:MeOH = 100:1 to 10:1) to give (*R*)-1-(8-fluoro-2-((1-(hydroxymethyl)cyclopropyl) methoxy)-7-(3-(methoxymethoxy)-8-((triissopropylsilyl)ethynyl)naphthalen-1-yl)pyrido[4,3-*d*]pyrimidin-4-yl)-3-methylpiperidin-3-ol.

LC-MS: (ESI, m/z): [M+H]⁺ = 729.4.

### Step 2: preparation of (R)-1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-d]pyrimidin-2-yl)oxy)methyl)cyclopropane-1-carbaldehyde

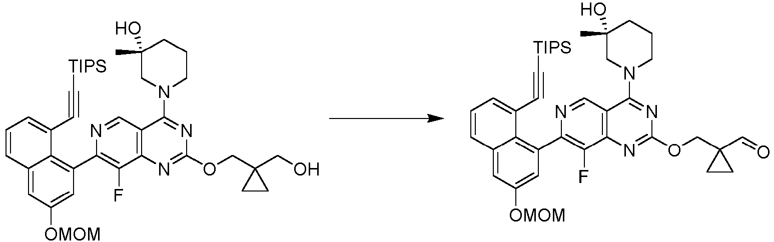

A solution of (*R*)-1-(8-fluoro-2-((1-(hydroxymethyl)cyclopropyl)methoxy)-7-(3-(methoxymethoxy)-8-((triisopropylsilyl)ethynyl)naphthalen-1-yl)pyrido[4,3-*d*]pyrimidin-4-yl)-3-methylpiperidin-3-ol (130 mg, 0.179 mmol) in DCM (3 mL) was cooled to 0 °C, and Dess-Martin reagent (152 mg, 0.358 mmol) was added. The mixture was reacted at room temperature for 0.5 h. After the reaction was completed, a saturated sodium thiosulfate solution (10 mL) was added to quench the reaction, and the mixture was extracted with dichloromethane (10 mL × 3). The organic phases were combined, dried over anhydrous sodium sulfate, and concentrated under reduced pressure. The concentrate was purified by silica gel column chromatography (DCM:MeOH = 10:1) to give (*R*)-1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-*d*]pyrimidin-2-yl)oxy)methyl)cyclopropane-1-carbaldehyde.

LC-MS: (ESI, m/z): [M+H]⁺ = 727.4.

### Step 3: preparation of (R)-1-(2-chloro-5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-d]pyrimidin-2-yl)oxy)methyl) cyclopropyl)methyl)piperazin-1-yl)methyl)-3-azaspiro[5.5]undecane-3-carbonyl)phenyl)dihydropyrimidine-2,4(1H,3H)-dione

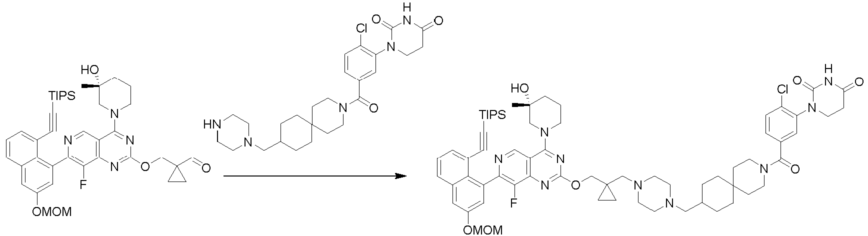

Tetraisopropyl titanate (215 mg, 0.755 mmol) was added to a stirred solution of (*R*)-1-(((8-fluoro-4-(3-hydroxy-3-methylpiperidin-1-yl)-7-(3-(methoxymethoxy)-8-((triisopropylsilyl)ethynyl)naphthalen-1-yl)pyrido [4,3-*d*]pyrimidin-2-yl)oxy)methyl)cyclopropane-1-carbaldehyde (110 mg, 0.151 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (91 mg, 0.181 mmol) in DCM (2 mL), and the mixture was stirred at 30 °C for 2 h. NaBH(OAc)₃ (101 mg, 0.453 mmol) was added to the mixture at room temperature, and the resulting mixture was stirred for 2 h. The mixture was concentrated and purified by silica gel column chromatography (DCM:MeOH = 1:0 to 4:1) to give (*R*)-1-(2-chloro-5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-*d*]pyrimidin-2-yl)oxy)methyl)cyclopropyl)methyl) piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M/2+H]⁺ = 606.9.

### Step 4: preparation of (R)-1-(2-chloro-5-(9-((4-((1-(((7-(8-ethynyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

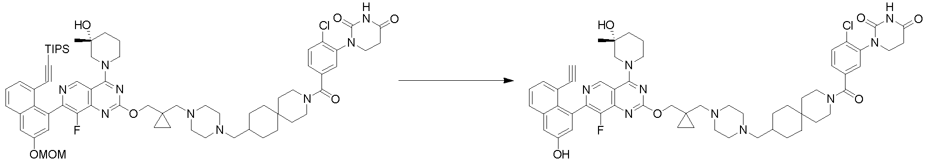

Cesium fluoride (69 mg, 0.455 mmol) was added to a stirred solution of (*R*)-1-(2-chloro-5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)phenyl)dihydropyrimidine-2,4(1*H*,3*H*)-dione (110 mg, 0.091 mmol) in DMF (2 mL), and the mixture was stirred at room temperature for 30 min and extracted with EA and water. The organic phase was concentrated. A mixture of 1,4-dioxane (1 mL) and HCl/1,4-dioxane (6 N, 0.5 mL) was then added to the mixture at room temperature. The mixture was reacted for 0.5 h. The mixture was purified by high-performance liquid chromatography to give (*R*)-1-(2-chloro-5-(9-((4-((1-(((7-(8-ethynyl-3-hydroxynaphthalen-1-yl)-8-fluoro-4-(3-hydroxy-3-methylpiperidin-1-yl)pyrido[4,3-*d*]pyrimidin-2-yl)oxy)methyl)
cyclopropyl)methyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1012.1.

¹H NMR (400 MHz, DMSO-*d₆*) δ 10.50 (s, 1H), 10.13 (s, 1H), 9.11 (d, *J* = 67.2 Hz, 1H), 7.88 (dd, *J =* 8.0, 1.5 Hz, 1H), 7.63 (d, *J =* 8.2 Hz, 1H), 7.54 (d, *J =* 1.9 Hz, 1H), 7.47 - 7.31 (m, 4H), 7.14 (dd, *J =* 16.7, 2.5 Hz, 1H), 4.73 (d, *J* = 37.9 Hz, 1H), 4.41 - 4.20 (m, 3H), 4.02 (dd, *J =* 54.0, 13.3 Hz, 1H), 3.81-3.70 (m, 1H), 3.66 - 3.48 (m, 5H), 3.29 - 3.21 (m, 2H), 2.77 - 2.68 (m, 2H), 2.44 - 2.20 (m, 9H), 2.15 - 1.89 (m, 4H), 1.75 - 1.59 (m, 5H), 1.55 - 1.38 (m, 5H), 1.36 - 1.22 (m, 3H), 1.16 (d, *J =* 17.2 Hz, 3H), 1.11 - 0.92 (m, 4H), 0.69 - 0.56 (m, 2H), 0.43 - 0.34 (m, 2H).

### Example 12: (R)-1-(2-chloro-5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5] undecane-3-carbonyl)phenyl)dihydropyrimidine-2,4(1H,3H)-dione

### Step 1: preparation of (R)-1-(7-bromo-8-fluoro-2-((1-(hydroxymethyl)cyclopropyl)methoxy)quinazolin-4-yl)-3-methylpiperidin-3-ol

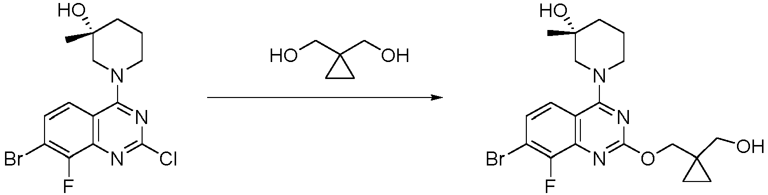

1,4-Diazabicyclo[2.2.2]octane (90 mg, 0.801 mmol) and anhydrous cesium carbonate (783 mg, 2.403 mmol) were added to a solution of (*R*)-1-(7-bromo-2-chloro-8-fluoroquinazolin-4-yl)-3-methylpiperidin-3-ol 1-(7-bromo-2-chloro-8-fluoroquinazolin-4-yl)-3-methylpiperidin-3-ol (600 mg, 1.602 mmol) and cyclopropane-1,1-diyldimethanol (327 mg, 3.203 mmol) in anhydrous acetonitrile (30 mL), and the mixture was stirred at 25 °C for 16 h. After the reaction was completed, the mixture was poured into water (100 mL) and extracted with ethyl acetate (20 mL × 2). The organic phases were combined, washed with saturated sodium chloride (100 mL), dried over anhydrous sodium sulfate, and concentrated under reduced pressure. The resulting crude product was purified by silica gel column chromatography (dichloromethane:methanol = 100:1 to 15:1) to give (*R*)-1-(7-bromo-8-fluoro-2-((1-(hydroxymethyl)cyclopropyl)methoxy)quinazolin-4-yl)-3-methylpiperidin-3-ol.

LC-MS: (ESI, m/z): [M+H]⁺ = 439.8.

### Step 2: preparation of (R)-1-(7-(8-ethyl-3-(methoxymethoxy)naphthalen-1-yl)-8-fluoro-2-((1-(hydroxymethyl) cyclopropyl)methoxy)quinazolin-4-yl)-3-methylpiperidin-3-ol

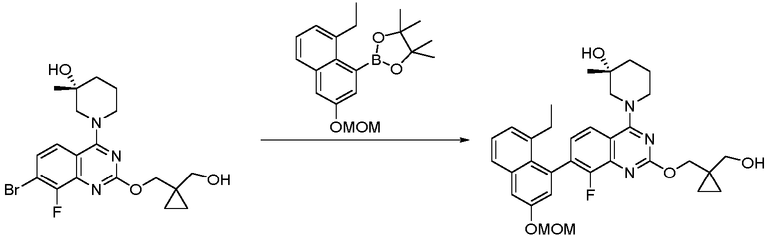

Anhydrous cesium carbonate (314 mg, 0.954 mmol) and cataCXium (R)A Pd G3 (35 mg, 0.048 mmol) were added to a solution of (*R*)-1-(7-bromo-8-fluoro-2-((1-(hydroxymethyl)cyclopropyl)methoxy)quinazolin-4-yl)-3-methylpiperidin-3-ol (140 mg, 0.318 mmol) and 2-(8-ethyl-3-(methoxymethoxy)naphthalen-1-yl)-4,4,5,5-tetramethyl-1,3,2-dioxaborolane (131 mg, 0.382 mmol) in 1,4-dioxane (14 mL) and water (3 mL) at room temperature. After the system was degassed, the mixture was stirred at 85 °C for 16 h under nitrogen atmosphere. After the reaction was completed, the mixture was concentrated under reduced pressure at 40 °C, and the resulting crude product was purified by silica gel thin-layer chromatography (dichloromethane:methanol = 10:1, 0.1% NH₃) to give (*R*)-1-(7-(8-ethyl-3-(methoxymethoxy)naphthalen-1-yl)-8-fluoro-2-((1-(hydroxymethyl)cyclopropyl)methoxy)quinazolin-4-yl)-3-methylpiperidin-3-ol.

LC-MS: (ESI, m/z): [M+H]⁺ = 575.9.

### Step 3: preparation of (R)-1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropane-1-carbaldehyde

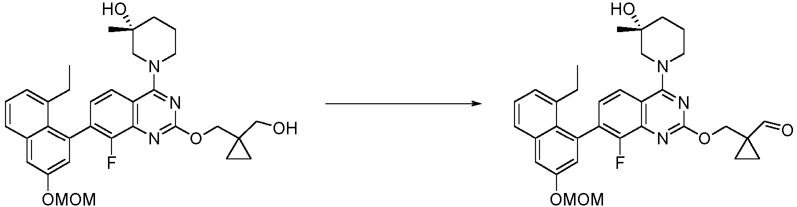

A solution of (*R*)-1-(7-(8-ethyl-3-(methoxymethoxy)naphthalen-1-yl)-8-fluoro-2-((1-(hydroxymethyl)
cyclopropyl)methoxy)quinazolin-4-yl)-3-methylpiperidin-3-ol (77.8 mg, 0.135 mmol) in dichloromethane (5 mL) was cooled to 0 °C from room temperature, and Dess-Martin reagent (75 mg, 0.176 mmol) was added. The mixture was stirred at 25 °C for 4 h. After the reaction was completed, the mixture was poured into water (100 mL) and extracted with dichloromethane (20 mL × 2). The organic phases were combined, washed with saturated sodium chloride (100 mL), dried over anhydrous sodium sulfate, and concentrated under reduced pressure. The resulting crude product was purified by silica gel thin-layer chromatography (petroleum ether:ethyl acetate = 1:1, 0.1% NH₃) to give (*R*)-1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropane-1-carbaldehyde.

LC-MS: (ESI, m/z): [M+H]⁺ = 573.9.

### Step 4: preparation of (R)-1-(2-chloro-5-(9-((4-((1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

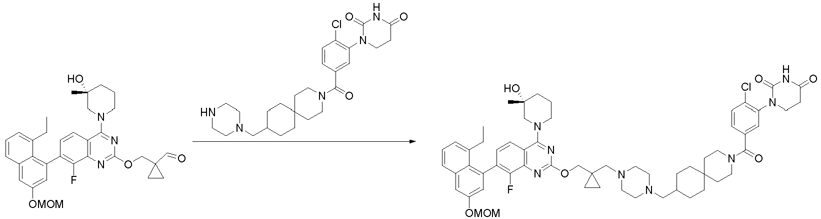

Tetraisopropyl titanate (140 mg, 0.492 mmol) was added to a solution of (*R*)-1-(((7-(8-ethyl-3-(methoxymethoxy)naphthalen-1-yl)-8-fluoro-4-(3-hydroxy-3-methylpiperidin-1-yl)quinazolin-2-yl)oxy) methyl)cyclopropane-1-carbaldehyde (56.4 mg, 0.098 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (52 mg, 0.103 mmol) in dichloromethane (5 mL), and the mixture stirred at 25 °C for 2 h. Sodium triacetoxyborohydride (63 mg, 0.295 mmol) was added to the mixture at room temperature, and the mixture was reacted at 25 °C for another 3 h. Methanol (10 mL) was added to quench the reaction, and the mixture was concentrated under reduced pressure at 40 °C. The resulting crude product was purified by a preparative thin-layer chromatography plate (dichloromethane:methanol = 10:1, 0.1% NH₃) to give (*R*)-1-(2-chloro-5-(9-((4-((1-(((7-(8-ethyl-3-(methoxymethoxy)naphthalen-1-yl)-8-fluoro-4-(3-hydroxy-3-methylpiperidin-1-yl)quinazolin-2-yl)oxy) methyl)cyclopropyl)methyl)piperazin-1-yl)methyl)-3-azaspiro[5.5]undecane-3-carbonyl)phenyl) dihydropyrimidine-2,4(1*H*,3*H*)-dione.

### LC-MS: (ESI, m/z): [M/2+H]⁺ = 530.0.

### Step 5: preparation of (R)-1-(2-chloro-5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

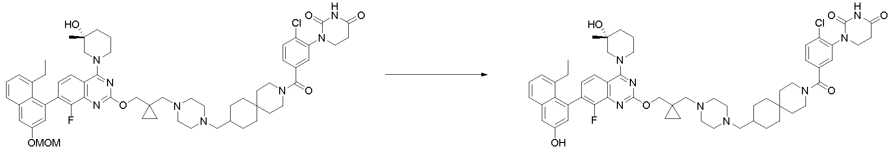

Hydrogen chloride/1,4-dioxane solution (4.0 M, 3 mL) was added to (*R*)-1-(2-chloro-5-(9-((4-((1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)phenyl)
dihydropyrimidine-2,4(1*H*,3*H*)-dione (81.2 mg, 0.077 mmol) at room temperature, and the mixture was reacted at room temperature for 1 h. After the reaction was completed, the mixture was concentrated under reduced pressure at 30 °C, and a solution of ammonia in methanol (1 mL) was then added. Then the mixture was concentrated under reduced pressure at 30 °C. The resulting crude product was purified by a preparative thin-layer chromatography plate (dichloromethane:methanol = 10:1, 0.1% NH₃) and then purified by high-performance liquid chromatography to give (*R*)-1-(2-chloro-5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl)cyclopropyl)methyl) piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1015.2.

¹H NMR (400 MHz, DMSO-*d₆*) δ 10.50 (s, 1H), 9.85 (s, 1H), 7.89 (t, *J =* 9.2 Hz, 1H), 7.69 - 7.59 (m, 2H), 7.54 (*d, J* = 1.9 Hz, 1H), 7.41 - 7.32 (m, 2H), 7.28 - 7.21 (m, 2H), 7.11 (d, *J* = 7.1 Hz, 1H), 6.92 - 6.84 (m, 1H), 4.66 (d, *J =* 13.3 Hz, 1H), 4.31 - 4.17 (m, 2H), 4.06 - 3.93 (m, 1H), 3.87 - 3.68 (m, 2H), 3.62 - 3.38 (m, 4H), 3.29 - 3.23 (m, 2H), 2.78 - 2.67 (m, 2H), 2.45 - 2.17 (m, 12H), 2.06 - 2.01 (m, 2H), 1.75 - 1.19 (m, 14H), 1.16 (d, *J =* 15.4 Hz, 3H), 1.10 - 0.93 (m, 4H), 0.83 - 0.77 (m, 3H), 0.67 - 0.58 (m, 2H), 0.43 - 0.33 (m, 2H).

### Example 13: 2-(2,6-dioxopiperidin-3-yl)-5-(9-((4-((1-(((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl) methyl)piperazin-1-yl)methyl)-3-azaspiro[5.5]undecane-3-carbonyl)isoindoline-1,3-dione

### Step 1: preparation of 2-(2,6-dioxopiperidin-3-yl)-1,3-dioxoisoindoline-5-carboxylic acid

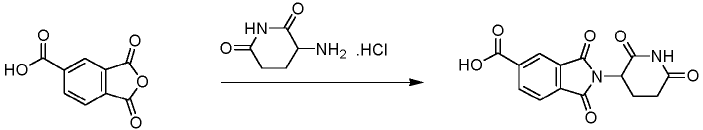

3-Aminopiperidine-2,6-dione hydrochloride (17.5 g, 91.2 mmol) and sodium acetate (6 g, 73 mmol) were added to a solution of 1,3-dioxo-1,3-dihydroisobenzofuran-5-carboxylic acid (10 g, 60.8 mmol) in acetic acid (100 mL), and the mixture was stirred at 120 °C for 6 h. After the reaction was completed, the reaction liquid was quenched with water (100 mL), stirred and filtered. The resulting solid was washed with water (50 mL × 3) and petroleum ether (50 mL × 3) to give 2-(2,6-dioxopiperidin-3-yl)-1,3-dioxoisoindoline-5-carboxylic acid. The crude product was directly used in the next step without further purification.

### Step 2: preparation of pentafluorophenyl 2-(2,6-dioxopiperidin-3-yl)-1,3-dioxoisoindoline-5-carboxylate

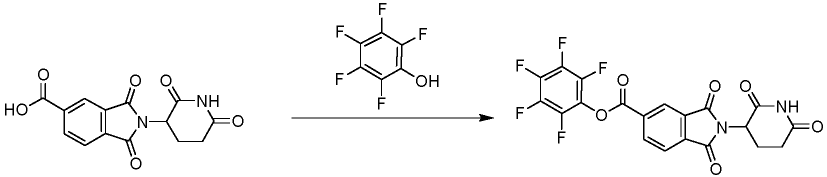

2,3,4,5,6-Pentafluorophenol (1 g, 5.5 mmol) and 4-dimethylaminopyridine (81 mg, 0.66 mmol) were added to a solution of 2-(2,6-dioxopiperidin-3-yl)-1,3-dioxoisoindoline-5-carboxylic acid (2 g, 6.6 mmol) in 1,4-dioxane (20 mL). *N,N*-Dicyclohexylcarbodiimide (1.9 g, 9.2 mmol) was dissolved in 1,4-dioxane (10 mL) solution and added to the mixed solution described above, and the mixture was stirred at 25 °C for 2 h. After the reaction was completed, A HCl (2 M, 20 mL) solution was added to quench the reaction, and the mixture was extracted with ethyl acetate (20 mL × 2). The organic phase was dried over anhydrous Na₂SO₄, filtered, and concentrated in vacuum. The mixture was purified by column chromatography (DCM:MeOH = 20:1) to give pentafluorophenyl 2-(2,6-dioxopiperidin-3-yl)-1,3-dioxoisoindoline-5-carboxylate.

LC-MS: (ESI, m/z): [M+H]⁺ = 469.0.

### Step 3: preparation of tert-butyl (R)-9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carboxylate

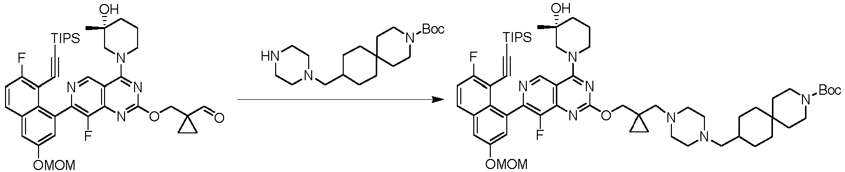

*tert*-Butyl 9-(piperazin-1-ylmethyl)-3-azaspiro[5.5]undecane-3-carboxylate (85 mg, 0.24 mmol) and tetraisopropyl titanate (568 mg, 2.0 mmol) were added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde (150 mg, 0.20 mmol) in dichloromethane (3 mL) and acetic acid (0.2 mL). The mixture was stirred at 25 °C for 2 h and cooled to 0 °C. NaBH(OAc)₃ (127 mg, 0.6 mmol) was added, and the mixture was stirred at 25 °C for another 1 h. After the reaction was completed, H₂O (20 mL) was added, and the mixture was adjusted to pH >7 with an aqueous NaHCO₃ solution and extracted with ethyl acetate (20 mL × 3). The combined organic phase was dried over anhydrous sodium sulfate, filtered, and concentrated in vacuum. The crude product was purified by silica gel column chromatography (DCM:MeOH = 20:1) to give *tert*-butyl (*R*)-9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropyl)methyl)piperazin-1-yl)methyl)-3-azaspiro[5.5]undecane-3-carboxylate.

LC-MS: (ESI, m/z): [(M-tBu)/2+H]⁺ = 512.9.

### Step 4: preparation of (R)-1-(2-((1-((4-((3-azaspiro[5.5]undecan-9-yl)methyl)piperazin-1-yl)methyl) cyclopropyl)methoxy)-7-(8-ethynyl-7-fluoro-3-hydroxynaphthalen-1-yl)-8-fluoropyrido[4,3-d]pyrimidin-4-yl)-3-methylpiperidin-3-ol

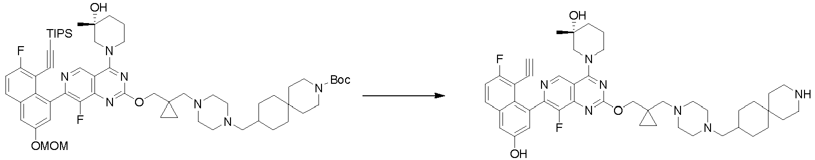

*tert*-Butyl (*R*)-9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl) piperazin-1-yl)methyl)-3-azaspiro[5.5]undecane-3-carboxylate (135 mg, 0.125 mmol) was dissolved in DMF (3 mL). CsF (95 mg, 0.62 mmol) was added, and the mixture was reacted at room temperature for 0.5 h. After the reaction was completed, the mixture was diluted with H₂O (10 mL) and extracted with ethyl acetate (10 mL × 3). The combined organic phase was dried over Na₂SO₄, filtered, and concentrated under reduced pressure. Hydrochloric acid/1,4-dioxane (6 M, 1 mL) was added to the crude product, and the mixture was stirred at room temperature for 0.5 h. After the reaction was completed, the reaction liquid was directly concentrated to give (*R*)-1-(2-((1-((4-((3-azaspiro[5.5]undecan-9-yl)methyl)piperazin-1-yl)methyl)cyclopropyl) methoxy)-7-(8-ethynyl-7-fluoro-3-hydroxynaphthalen-1-yl)-8-fluoropyrido[4,3-*d*]pyrimidin-4-yl)-3-methylpiperidin-3-ol, which was directly used in the next step.

LC-MS: (ESI, m/z): [(M+H]⁺ = 780.5.

### Step 5: preparation of 2-(2,6-dioxopiperidin-3-yl)-5-(9-((4-((1-(((7-(8-ethynyl-7-fluoro-3-hydroxynaphthalen-1-y1)-8-fluoro-4-((R)-3-hydroxy-3-methylpiperidin-1-yl)pyrido[4,3-d]pyrimidin-2-yl)oxy)methyl)cyclopropyl) methyl)piperazin-1-yl)methyl)-3-azaspiro [5. 5]undecane-3-carbonyl)isoindoline-1,3-dione

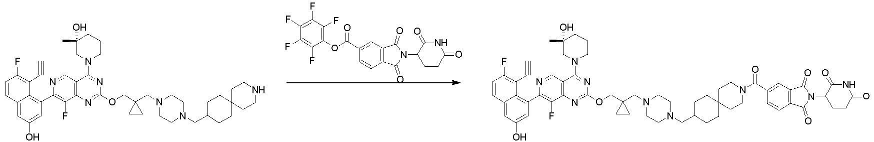

DIEA (52 mg, 0.40 mmol) and pentafluorophenyl 2-(2,6-dioxopiperidin-3-yl)-1,3-dioxoisoindoline-5-carboxylate (34 mg, 0.073 mmol) were added to a solution of (*R*)-1-(2-((1-((4-((3-azaspiro[5.5]undecan-9-yl)methyl)piperazin-1-yl)methyl)cyclopropyl)methoxy)-7-(8-ethynyl-7-fluoro-3-hydroxynaphthalen-1-yl)-8-fluoropyrido[4,3-*d*]pyrimidin-4-yl)-3-methylpiperidin-3-ol (70 mg, 0.086 mmol) in DMF (1.5 mL), and the mixture was reacted at room temperature for 2 h. Water (10 mL) was added, and the mixture was extracted with ethyl acetate (10 mL × 2). The organic phase was washed with saturated brine, dried over anhydrous sodium sulfate, and filtered, and the filtrate was concentrated and purified by high-performance liquid chromatography to give 2-(2,6-dioxopiperidin-3-yl)-5-(9-((4-((1-(((7-(8-ethynyl-7-fluoro-3-hydroxynaphthalen-1-y1)-8-fluoro-4-((*R*)-3-hydroxy-3-methylpiperidin-1-yl)pyrido[4,3-*d*]pyrimidin-2-yl)oxy)methyl)cyclopropyl) methyl)piperazin-1-yl)methyl)-3-azaspiro[5.5]undecane-3-carbonyl)isoindoline-1,3-dione.

LC-MS: (ESI, m/z): [M+H]⁺ = 1064.2.

¹H NMR (400 MHz, DMSO-*d₆*) δ 11.13 (s, 1H), 10.14 (d, *J* = 4.0 Hz, 1H), 9.12 (d, *J* = 65.4 Hz, 1H), 8.01 - 7.93 (m, 2H), 7.90 - 7.79 (m, 2H), 7.49 - 7.42 (m, 1H), 7.39 (s, 1H), 7.20 (dd, *J* = 17.2, 2.2 Hz, 1H), 5.17 (dd, *J* = 12.6, 5.5 Hz, 1H), 4.73 (d, *J* = 30.2 Hz, 1H), 4.41 - 4.19 (m, 3H), 4.14 - 3.89 (m, 2H), 3.70 - 3.48 (m, 3H), 3.25 - 3.16 (m, 2H), 2.95 - 2.84 (m, 1H), 2.70 - 2.56 (m, 2H), 2.41 - 2.17 (m, 9H), 2.13 - 1.95 (m, 5H), 1.76 - 1.62 (m, 5H), 1.57 - 1.46 (m, 3H), 1.45 - 1.34 (m, 3H), 1.29 - 1.21 (m, 2H), 1.17 (d*, J* = 16.4 Hz, 3H), 1.09 - 1.90 (m, 4H), 0.67 - 0.59 (m, 2H), 0.45 - 0.34 (m, 2H).

### Example 14: (R)-1-(5-(9-((4-((3-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)bicyclo[1.1.1]pent-1-yl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 4-(3-(methoxycarbonyl)bicyclo[1.1.1]pentane-1-carbonyl)piperazine-1-carboxylate

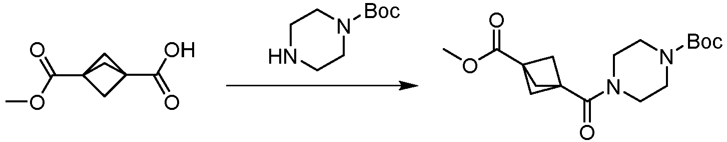

DIEA (4.55 g, 35.25 mmol) and *tert*-butyl piperazine-1-carboxylate (2.63 g, 14.1 mmol) were added to a solution of 3-(methoxycarbonyl)bicyclo[1.1.1]pentane-1-carboxylic acid (2.0 g, 11.75 mmol), EDCI (3.78 g, 17.63 mmol), and HOBT (2.38 g, 17.63 mmol) in DMF (30 mL). The mixture was reacted at room temperature for 3 h. After the reaction was completed, the reaction liquid was slowly poured into stirred water. The mixture was filtered, and the precipitate was washed with water and dried in vacuum to give *tert-*butyl 4-(3-(methoxycarbonyl)bicyclo[1.1.1]pentane-1-carbonyl)piperazine-1-carboxylate, which was directly used in the next step without further purification.

LC-MS: (ESI, m/z): [M-tBu+H]⁺ = 283.2.

### Step 2: preparation of tert-butyl 4-((3-(hydroxymethyl)bicyclo[1.1.1]pent-1-yl)methyl)piperazine-1-carboxylate

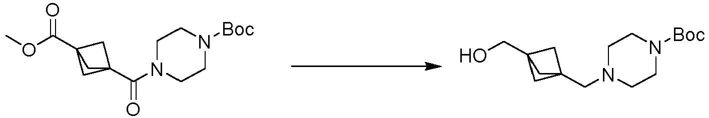

*tert*-Butyl 4-(3-(methoxycarbonyl)bicyclo[1.1.1]pentane-1-carbonyl)piperazine-1-carboxylate (1.5 g, 4.43 mmol) was dissolved in THF (15 mL), and LAH/THF (6.65 mL, 6.65 mmol, 1 M) was added. The reaction liquid was stirred at 0 °C to room temperature for 2 h. Potassium sodium tartrate tetrahydrate was added to quench the reaction, and the mixture was filtered. The filtrate was concentrated and purified by silica gel column chromatography (DCM:MeOH =100:1 to 15:1) to give *tert*-butyl 4-((3-(hydroxymethyl)bicyclo [1.1.1]pent-1-yl)methyl)piperazine-1-carboxylate.

¹H NMR (400 MHz, CDCl₃) δ 3.62 (s, 1H), 3.58 (s, 2H), 3.45 - 3.38 (m, 4H), 2.49 - 2.36 (m, 6H), 1.67 (s, 6H), 1.45 (s, 9H).

LC-MS: (ESI, m/z): [M+H]⁺ = 297.3.

### Step 3: preparation of tert-butyl (R)-4-((3-(((7-chloro-8-fluoro-4-(3-hydroxy-3-methylpiperidin-1-yl)pyrido [4,3-d]pyrimidin-2-yl)oxy)methyl)bicyclo[1.1.1]pent-1-yl)methyl)piperazine-1-carboxylate

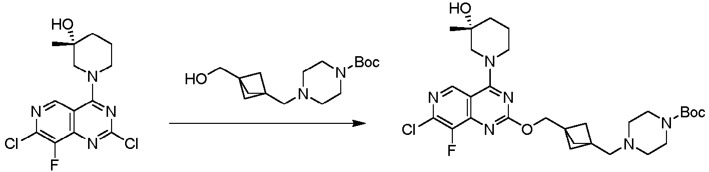

(*R*)-1-(2,7-dichloro-8-fluoropyrido[4,3-*d*]pyrimidin-4-yl)-3-methylpiperidin-3-ol (300 mg, 0.91 mmol) and *tert*-butyl 4-((3-(hydroxymethyl)bicyclo[1.1.1]pent-1-yl)methyl)piperazine-1-carboxylate (322 mg, 1.09 mmol) were added to anhydrous acetonitrile (8 mL), and then cesium carbonate (886 mg, 2.72 mmol) and 1,4-diazabicyclo[2.2.2]octane (51 mg, 0.45 mmol) were added. The mixture was stirred at 25 °C for 1 h. Saturated brine (30 mL) was added to the reaction liquid to quench the reaction, and the mixture was extracted with ethyl acetate (50 mL × 3). The organic phases were combined, dried over anhydrous sodium sulfate, and concentrated under reduced pressure. The concentrate was purified by silica gel column chromatography (DCM:MeOH =10:1) to give *tert*-butyl (*R*)-4-((3-(((7-chloro-8-fluoro-4-(3-hydroxy-3-methylpiperidin-1-yl)pyrido[4,3-*d*]pyrimidin-2-yl)oxy)methyl)bicyclo[1.1.1]pent-1-yl)methyl)piperazine-1-carboxylate.

LC-MS: (ESI, m/z): [M+H]⁺ = 591.3.

### Step 4: preparation of tert-butyl (R)-4-((3-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-d]pyrimidin-2-yl)oxy)methyl) bicyclo[1.1.1]pent-1-yl)methyl)piperazine-1-carboxylate

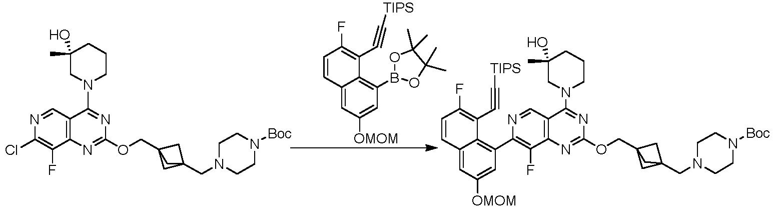

CataCXium A Pd G3 (49 mg, 0.068 mmol) and cesium carbonate (330 mg, 1.01 mmol) were added to a mixed solution of *tert*-butyl (*R*)-4-((3-(((7-chloro-8-fluoro-4-(3-hydroxy-3-methylpiperidin-1-yl)pyrido[4,3-*d*]pyrimidin-2-yl)oxy)methyl)bicyclo[1.1.1]pent-1-yl)methyl)piperazine-1-carboxylate (200 mg, 0.34 mmol) and ((2-fluoro-6-(methoxymethoxy)-8-(4,4,5,5-tetramethyl-1,3,2-dioxaborolan-2-yl)naphthalen-1-yl)ethynyl) triisopropylsilane (208 mg, 0.41 mmol) in 1,4-dioxane (5 mL) and H₂O (0.5 mL). The mixture was reacted at 85 °C for 16 h under nitrogen atmosphere. Water (20 mL) was added to the reaction liquid, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH =10: 1) to give *tert*-butyl (*R*)-4-((3-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido
[4,3-*d*]pyrimidin-2-yl)oxy)methyl)bicyclo[1.1.1]pent-1-yl)methyl)piperazine-1-carboxylate.

LC-MS: (ESI, m/z): [M+H]⁺ = 941.5.

### Step 5: preparation of (R)-1-(8-fluoro-7-(7-fluoro-3-hydroxy-8-((triisopropylsilyl)ethynyl)naphthalen-1-yl)-2-((3-(piperazin-1-ylmethyl)bicyclo[1.1.1]pent-1-yl)methoxy)pyrido[4,3-d]pyrimidin-4-yl)-3-methylpiperidin-3-ol

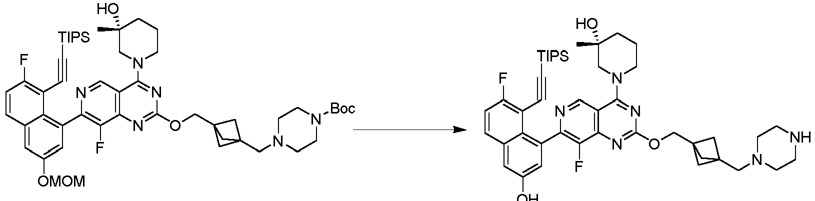

*tert*-Butyl (*R*)-4-((3-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)bicyclo[1.1.1]pent-1-yl)methyl)piperazine-1-carboxylate (93 mg, 0.099 mmol) was dissolved in 1,4-dioxane (1 mL), and HCl/1,4-dioxane (1 mL, 4 M) was added. The reaction liquid was reacted at 25 °C for 1 h. After the reaction was completed, the pH was adjusted to 8 with a saturated Na₂CO₃ solution, and the mixture was diluted with water and extracted with ethyl acetate (15 mL × 3). The organic phases were combined, dried over anhydrous Na₂SO₄, and filtered, and the filtrate was concentrated to give a crude product, which was subjected to silica gel column chromatography (DCM:MeOH (NH₃) = 10:1) to give (*R*)-1-(8-fluoro-7-(7-fluoro-3-hydroxy-8-((triisopropylsilyl)ethynyl)naphthalen-1-yl)-2-((3-(piperazin-1-ylmethyl)bicyclo[1.1.1]pent-1-yl)methoxy) pyrido[4,3-*d*]pyrimidin-4-yl)-3-methylpiperidin-3-ol.

LC-MS: (ESI, m/z): [M+H]⁺ = 797.4.

### Step 6: preparation of (R)-1-(5-(9-((4-((3-(((8-fluoro-7-(7-fluoro-3-hydroxy-8-((triisopropylsilyl)ethynyl) naphthalen-1-yl)-4-(3-hydroxy-3-methylpiperidin-1-yl)pyrido[4,3-d]pyrimidin-2-yl)oxy)methyl)bicyclo [1.1.1]pent-1-yl)methyl)piperazin-1-yl)methyl)-3-azaspiro[5.5]undecane-3-carbonyl)-2-methylphenyl) dihydropyrimidine-2,4(1H,3H)-dione

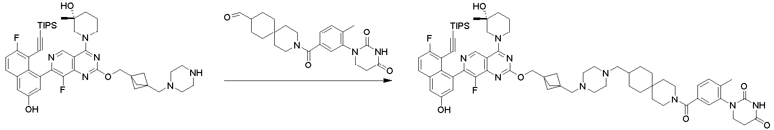

Tetraisopropyl titanate (90 mg, 0.32 mmol) was added to a solution of compound (*R*)-1-(8-fluoro-7-(7-fluoro-3-hydroxy-8-((triisopropylsilyl)ethynyl)naphthalen-1-yl)-2-((3-(piperazin-1-ylmethyl)bicyclo[1.1.1]pent-1-yl)methoxy)pyrido[4,3-*d*]pyrimidin-4-yl)-3-methylpiperidin-3-ol (50 mg, 0.063 mmol) and 3-(3-(2,4-dioxotetrahydropyrimidin-1(2*H*)-yl)-4-methylbenzoyl)-3-azaspiro[5.5]undecane-9-carbaldehyde (31 mg, 0.076 mmol) in DCM/AcOH (1.0 mL/0.01 mL), and the mixed solution was stirred at 30 °C for 2 h. NaBH(OAc)₃ (42 mg, 0.19 mmol) was then added at 0 °C, and the mixture was stirred at room temperature for 2 h. The mixture was concentrated and purified by silica gel column chromatography (MeOH:DCM = 0-3:17) to give (*R*)-1-(5-(9-((4-((3-(((8-fluoro-7-(7-fluoro-3-hydroxy-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)bicyclo[1.1.1]pent-1-yl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 596.9.

### Step 7: preparation of (R)-1-(5-(9-((4-((3-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)bicyclo[1.1.1]pent-1-yl)methyl) piperazin-1-yl)methyl)-3-azaspiro[5.5]undecane-3-carbonyl)-2-methylphenyl)dihydropyrimidine-2,4(1H,3H)-dione

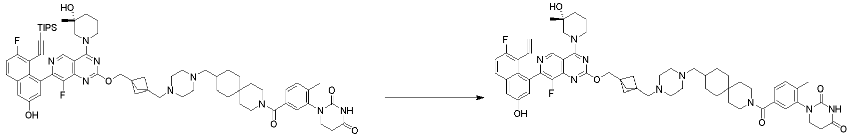

Cesium fluoride (33 mg, 0.22 mmol) was added to a solution of (*R*)-1-(5-(9-((4-((3-(((8-fluoro-7-(7-fluoro-3-hydroxy-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)bicyclo[1.1.1]pent-1-yl)methyl)piperazin-1-yl)methyl)-3-azaspiro[5.5] undecane-3-carbonyl)-2-methylphenyl)dihydropyrimidine-2,4(1*H*,3*H*)-dione (52 mg, 0.044 mmol) in DMF (1 mL), and the mixture was stirred at 40 °C for 2 h. The reaction liquid was diluted with water, and the mixture was extracted with ethyl acetate. The combined organic phase was washed with brine, dried over anhydrous Na₂SO₄, and concentrated. The crude product was purified by preparative high-performance liquid chromatography to give (*R*)-1-(5-(9-((4-((3-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)bicyclo[1.1.1]pent-1-yl)methyl) piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 518.7.

¹H NMR (400 MHz, DMSO-*d₆*) δ 10.38 (s, 1H), 10.17 (d, *J* = 3.8 Hz, 1H), 9.13 (d, *J* = 52.9 Hz, 1H), 7.98 (dd, *J* = 9.2, 6.0 Hz, 1H), 7.49 - 7.43 (m, 1H), 7.39 (s, 1H), 7.36 - 7.29 (m, 2H), 7.26 - 7.16 (m, 2H), 4.74 (d, *J* = 10.1 Hz, 1H), 4.43 - 4.26 (m, 3H), 4.13 - 3.91 (m, 2H), 3.86 - 3.73 (m, 1H), 3.67 - 3.46 (m, 4H), 3.32 - 3.23 (m, 3H), 2.82 - 2.65 (m, 2H), 2.44 - 2.23 (m, 9H), 2.21 (s, 3H), 2.11 - 2.00 (m, 3H), 1.76 - 1.61 (m, 11H), 1.55 - 1.38 (m, 5H), 1.36 - 1.21 (m, 3H), 1.16 (d, *J =* 15.6 Hz, 3H), 1.10 - 0.92 (m, 4H).

### Example 15: (R)-1-(2-chloro-5-(4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazine-1-carbonyl)phenyl)dihydropyrimidine-2,4(1H,3H)-dione

### Step 1: preparation of (R)-1-(2-chloro-5-(4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-d]pyrimidin-2-yl)oxy)methyl) cyclopropyl)methyl)piperazine-1-carbonyl)phenyl)dihydropyrimidine-2,4(1H,3H)-dione

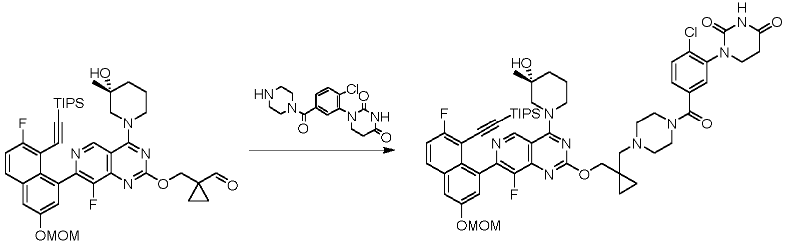

Tetraisopropyl titanate (0.5 mL) was added to a stirred solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropane-1-carbaldehyde (150 mg, 0.20 mmol) and 1-(2-chloro-5-(piperazine-1-carbonyl)phenyl)dihydropyrimidine-2,4(1*H*,3*H*)-dione (81 mg, 0.24 mmol) in DCM/AcOH (1.5 mL/0.1 mL), and the mixture was stirred at 30 °C for 2 h. NaBH(OAc)₃ (128 mg, 0.60 mmol) was added to the mixture in an ice bath, and the resulting mixture was stirred for 2 h. The mixture was concentrated and purified by silica gel column chromatography (MeOH:DCM = (0-1):4) to give (*R*)-1-(2-chloro-5-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazine-1-carbonyl)phenyl)dihydropyrimidine-2,4(1*H*,3*H*)-dione.

LC-MS: (ESI, m/z): [M+H]⁺ = 1065.4.

### Step 2: preparation of (R)-1-(2-chloro-5-(4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazine-1-carbonyl)phenyl)dihydropyrimidine-2,4(1H,3H)-dione

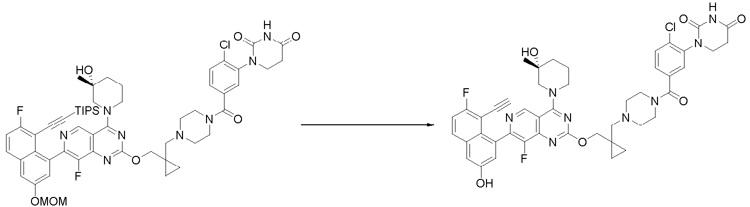

Cesium fluoride (93 mg, 0.61 mmol) was added to a stirred solution of (*R*)-1-(2-chloro-5-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-carbonyl)phenyl)dihydropyrimidine-2,4(1*H*,3*H*)-dione (131 mg, 0.12 mmol) in DMF (1 mL), and the mixture was stirred at room temperature for 30 min. Water (10 mL) was added to the reaction liquid to quench the reaction, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (0.5 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by high-performance liquid chromatography to give (*R*)-1-(2-chloro-5-(4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazine-1-carbonyl)phenyl)dihydropyrimidine-2,4(1*H*,3*H*)-dione.

LC-MS: (ESI, m/z): [M+H]⁺ = 865.0.

¹H NMR (400 MHz, DMSO-*d₆*) δ 10.49 (s, 1H), 10.15 (d, *J =* 4.6 Hz, 1H), 9.13 (d, *J* = 65.1 Hz, 1H), 8.01 - 7.93 (m, 1H), 7.63 (d, *J* = 8.2 Hz, 1H), 7.57 - 7.52 (m, 1H), 7.46 (t, *J* = 9.0 Hz, 1H), 7.41 - 7.34 (m, 2H), 7.24 - 7.15 (m, 1H), 4.73 (d, *J* = 26.6 Hz, 1H), 4.42 - 4.21 (m, 3H), 4.00 - 3.92 (m, 1H), 3.81 - 3.46 (m, 5H), 3.42 - 3.32 (m, 2H), 3.31 - 3.20 (m, 2H), 2.78 - 2.67 (m, 2H), 2.48 - 2.29 (m, 6H), 2.11 - 1.95 (m, 1H), 1.74 - 1.58 (m, 3H), 1.17 (*d, J =* 16.4 Hz, 3H), 0.71 - 0.58 (m, 2H), 0.47 - 0.33 (m, 2H).

### Example 16: (R)-1-(2-chloro-5-(9-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)-3,9-diazaspiro[5.5]undecane-3-carbonyl)phenyl)dihydropyrimidine-2,4(1H,3H)-dione

### Step 1: preparation of (R)-1-(2-chloro-5-(9-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-d]pyrimidin-2-yl)oxy)methyl) cyclopropyl)methyl)-3,9-diazaspiro[5.5]undecane-3-carbonyl)phenyl)dihydropyrimidine-2,4(1H,3H)-dione

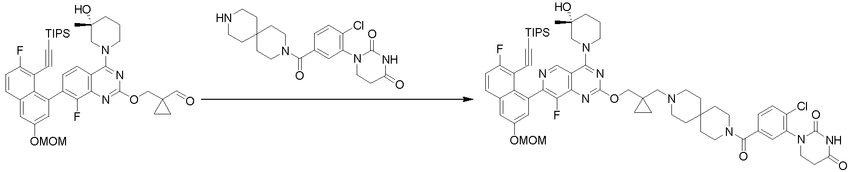

Tetraisopropyl titanate (0.5 mL) was added to a stirred solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl) quinazolin-2-yl)oxy)methyl)cyclopropane-1-carbaldehyde (150 mg, 0.20 mmol) and 1-(2-chloro-5-(3,9-diazaspiro[5.5]undecane-3-carbonyl)phenyl)dihydropyrimidine-2,4(1*H*,3*H*)-dione (122 mg, 0.30 mmol) in DCM/AcOH (1.5 mL/0.1 mL), and the mixture was stirred at 30 °C for 2 h. NaBH(OAc)₃ (128 mg, 0.60 mmol) was added to the reaction mixture in an ice bath, and the mixture was stirred for 2 h. The mixture was concentrated and purified by silica gel column chromatography (MeOH:DCM = (0-1):4) to give (*R*)-1-(2-chloro-5-(9-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)-3,9-diazaspiro[5.5]undecane-3-carbonyl)phenyl)dihydropyrimidine-2,4(1*H*,3*H*)-dione.

LC-MS: (ESI, m/z): [M/2+H]⁺ = 567.4.

### Step 2: preparation of (R)-1-(2-chloro-5-(9-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)-3,9-diazaspiro[5.5]undecane-3-carbonyl)phenyl)dihydropyrimidine-2,4(1H,3H)-dione

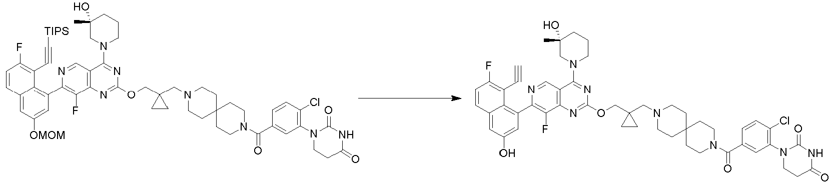

Cesium fluoride (83 mg, 0.55 mmol) was added to a solution of (*R*)-1-(2-chloro-5-(9-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)-3,9-diazaspiro[5.5]undecane-3-carbonyl)
phenyl)dihydropyrimidine-2,4(1*H*,3*H*)-dione (124 mg, 0.11 mmol) in DMF (1 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. 1,4-Dioxane (0.5 mL) was added to the concentrate, and HCl/1,4-dioxane (6 N, 1.0 mL) was then added while stirring. The mixture was reacted at room temperature for 0.5 h. The reaction liquid was adjusted to pH > 7 with an aqueous Na₂CO₃ solution and then extracted with EtOAc (10 mL × 2). The organic phase was washed with brine, dried over anhydrous Na₂SO₄, and concentrated. The crude product was purified by preparative high-performance liquid chromatography to give (*R*)-1-(2-chloro-5-(9-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)-3,9-diazaspiro[5.5] undecane-3-carbonyl)phenyl)dihydropyrimidine-2,4(1*H*,3*H*)-dione.

LC-MS: (ESI, m/z): [M+H]⁺ = 933.1.
¹H NMR (400 MHz, DMSO-*d₆*) δ 10.50 (s, 1H), 10.14 (d, *J* = 4.4 Hz, 1H), 9.12 (d, *J* = 67.1 Hz, 1H), 8.00 - 7.94 (m, 1H), 7.62 (d, *J =* 8.2 Hz, 1H), 7.55 - 7.52 (m, 1H), 7.45 (t, *J =* 9.1 Hz, 1H), 7.40 - 7.35 (m, 2H), 7.24 - 7.15 (m, 1H), 4.73 (d*, J* = 30.8 Hz, 1H), 4.39 - 4.23 (m, 3H), 3.95 - 3.92 (m, 1H), 3.78 - 3.70 (m, 1H), 3.64 - 3.51 (m, 4H), 3.29 - 3.24 (m, 2H), 2.77 - 2.69 (m, 2H), 2.41 - 2.16 (m, 8H), 1.72 - 1.59 (m, 3H), 1.49 - 1.30 (m, 9H), 1.16 (d, *J =* 16.9 Hz, 3H), 0.66 - 0.59 (m, 2H), 0.44 - 0.35 (m, 2H).

### Example 17: (R)-1-(2-chloro-5-(4-(((2-(1-((1-(((7-(8-ethynyl-7-fluoro-3-hydroxynaphthalen-1-yl)-8-fluoro-4-(3-hydroxy-3-methylpiperidin-1-yl)piperidino[4,3-d]pyrimidin-2-yl)oxy)methyl)cyclopropyl) methyl)piperidin-4-yl)ethyl)(methyl)amino)methyl)piperidine-1-carbonyl)phenyl)dihydropyrimidine-2,4(1H,3H)-dione

### Step 1: preparation of tert-butyl (R)-4-(((2-(1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)piperidino[4,3-d]pyrimidin-2-yl)oxy)methyl)cyclopropyl)methyl)piperidin-4-yl)ethyl)(methyl)amino)methyl)piperidine-1-carboxylate

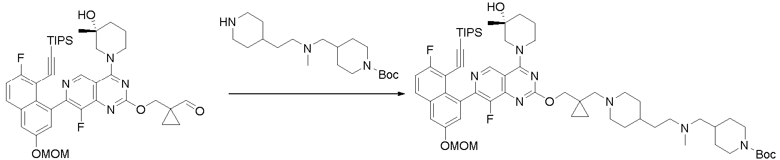

*tert*-Butyl 4-((methyl(2-(piperidin-4-yl)ethyl)amino)methyl)piperidine-1-carboxylate (54 mg, 0.16 mmol) and tetraisopropyl titanate (185 mg, 0.65 mmol) were added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)piperidino [4,3-*d*]pyrimidin-2-yl)oxy)methyl)cyclopropane-1-carbaldehyde (100 mg, 0.13 mmol) in dichloromethane (1 mL) and acetic acid (0.1 mL). The mixture was stirred at 25 °C for 2 h. NaBH(OAc)₃ (83 mg, 0.39 mmol) was added at 0 °C, and the mixture was stirred for 1 h. After the reaction was completed, H₂O (5 mL) was added, and the mixture was extracted with ethyl acetate (5 mL × 3). The combined organic layer was dried over anhydrous sodium sulfate, filtered, and concentrated in vacuum. The crude product was purified by column chromatography (DCM/MeOH = 15/1) to give *tert*-butyl (*R*)-4-(((2-(1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl) piperidino[4,3-*d*]pyrimidin-2-yl)oxy)methyl)cyclopropyl)methyl)piperidin-4-yl)ethyl)(methyl)amino)methyl)piperidine-1-carboxylate.

LC-MS: (ESI, m/z): [M+H]⁺= 1068.9.

### Step 2: preparation of (R)-1-(7-(8-ethynyl-7-fluoro-3-hydroxynaphthalen-1-yl)-8-fluoro-2-((1-((4-(2-(methyl(piperidin-4-ylmethyl)amino)ethyl)piperidin-1-yl)methyl)cyclopropyl)methoxy)piperidino[4,3-d] pyrimidin-4-yl)-3-methylpiperidin-3-ol

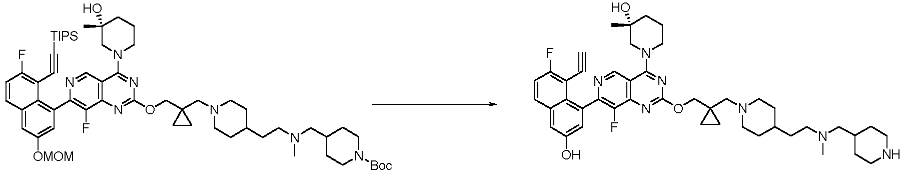

Cesium fluoride (84 mg, 0.55 mmol) was added to a stirred solution of *tert*-butyl (*R*)-4-(((2-(1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)piperidino[4,3-*d*]pyrimidin-2-yl)oxy)methyl)cyclopropyl)methyl)piperidin-4-yl)ethyl)(methyl)amino) methyl)piperidine-1-carboxylate (120 mg, 0.11 mmol) in DMF (1 mL), and the mixture was stirred at 30 °C for 1 h. After the reaction was completed, the mixture was diluted with H₂O (5 mL), and extracted with ethyl acetate (5 mL × 3). The combined organic layer was dried over Na₂SO₄, filtered, and concentrated under reduced pressure. 1,4-Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic layer was dried over Na₂SO₄, filtered, and concentrated under reduced pressure. The crude product was purified by column chromatography (DCM:MeOH (NH₃) = 10:1) to give (*R*)-1-(7-(8-ethynyl-7-fluoro-3-hydroxynaphthalen-1-yl)-8-fluoro-2-((1-((4-(2-(methyl(piperidin-4-ylmethyl)amino)ethyl)piperidin-1-yl)methyl)cyclopropyl)methoxy)piperidino[4,3-*d*]pyrimidin-4-yl)-3-methylpiperidin-3-ol.

LC-MS: (ESI, m/z): [M/2+H]⁺= 384.6.

### Step 3: preparation of (R)-1-(2-chloro-5-(4-(((2-(1-((1-(((7-(8-ethynyl-7-fluoro-3-hydroxynaphthalen-1-yl)-8-fluoro-4-(3-hydroxy-3-methylpiperidin-1-yl)piperidino[4,3-d]pyrimidin-2-yl)oxy)methyl)cyclopropyl) methyl)piperidin-4-yl)ethyl)(methyl)amino)methyl)piperidine-1-carbonyl)phenyl)dihydropyrimidine-2,4(1H,3H)-dione

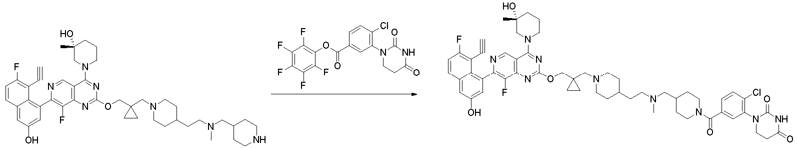

Pentafluorophenyl 4-chloro-3-(2,4-dioxotetrahydropyrimidin-1(2*H*)-yl)benzoate (24 mg, 0.055 mmol) and DIEA (30 mg, 0.23 mmol) were added to a solution of (*R*)-1-(7-(8-ethynyl-7-fluoro-3-hydroxynaphthalen-1-yl)-8-fluoro-2-((1-((4-(2-(methyl(piperidin-4-ylmethyl)amino)ethyl)piperidin-1-yl)methyl)cyclopropyl) methoxy)piperidino[4,3-*d*]pyrimidin-4-yl)-3-methylpiperidin-3-ol (60 mg, 0.078 mmol) in DMF (1 mL). The mixture was stirred at room temperature for 2 h. After the reaction was completed, water (5 mL) was added to quench the reaction, and the mixture was extracted with ethyl acetate (5 mL × 3). The organic phase was dried, concentrated, and purified by silica gel column chromatography (DCM:MeOH = 20:1) to give (*R*)-1-(2-chloro-5-(4-(((2-(1-((1-(((7-(8-ethynyl-7-fluoro-3-hydroxynaphthalen-1-yl)-8-fluoro-4-(3-hydroxy-3-methylpiperidin-1-yl)piperidino[4,3-*d*]pyrimidin-2-yl)oxy)methyl)cyclopropyl)methyl)piperidin-4-yl)ethyl)(methyl)amino) methyl)piperidine-1-carbonyl)phenyl)dihydropyrimidine-2,4(1*H*,3*H*)-dione.

LC-MS: (ESI, m/z): [M+H]⁺ = 1018.2.

¹H NMR (400 MHz, CD₃OD) δ 9.16 (d, *J* = 54.8 Hz, 1H), 8.45 (s, 1H), 7.90 - 7.83 (m, 1H), 7.64 (d, *J=* 8.2 Hz, 1H), 7.52 (d, *J =* 1.8 Hz, 1H), 7.41 (d, *J =* 8.1 Hz, 1H), 7.37 - 7.29 (m, 2H), 7.24 (dd, *J =* 19.2, 2.3 Hz, 1H), 4.69 - 4.54 (m, 3H), 4.40 - 4.27 (m, 2H), 3.83 - 3.74 (m, 3H), 3.70 - 3.56 (m, 3H), 3.45 - 3.36 (m, 1H), 3.21 - 3.05 (m, 3H), 2.93 - 2.77 (m, 5H), 2.66 - 2.57 (m, 2H), 2.49 - 2.42 (m, 2H), 2.39 (s, 3H), 2.26 - 2.10 (m, 1H), 1.99 - 1.64 (m, 9H), 1.63 - 1.45 (m, 5H), 1.27 (d, *J* = 21.5 Hz, 3H), 1.21 - 1.06 (m, 2H), 1.01 - 0.89 (m, 2H), 0.85 - 0.72 (m, 2H).

### Example 18: (R)-1-(2-chloro-5-(4-(2-(((1-((1-(((7-(8-ethynyl-7-fluoro-3-hydroxynaphthalen-1-yl)-8-fluoro-4-(3-hydroxy-3-methylpiperidin-1-yl)piperidino[4,3-d]pyrimidin-2-yl)oxy)methyl)cyclopropyl)methyl) piperidin-4-yl)methyl)(methyl)amino)ethyl)piperidine-1-carbonyl)phenyl)dihydropyrimidine-2,4(1H,3H)-dione

### Step 1: preparation of benzyl 4-(2-oxoethyl)piperidine-1-carboxylate

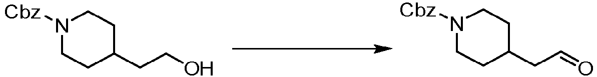

Dess-Martin oxidant (10.5 g, 24.69 mmol) was added to a solution of benzyl 4-(2-hydroxyethyl)piperidine-1-carboxylate (5 g, 18.99 mmol) in DCM (50 mL), and the mixture was stirred at room temperature for 2 h. After the reaction was completed, a sodium thiosulfate solution (50 mL) and a sodium bicarbonate solution (50 mL) were added to quench the reaction, and the mixture was extracted with dichloromethane (50 mL × 3). The organic phases were combined, dried over anhydrous sodium sulfate, and concentrated under reduced pressure to give benzyl 4-(2-oxoethyl)piperidine-1-carboxylate, which was directly used in the next step.

### Step 2: preparation of benzyl 4-(2-(((1-(tert-butoxycarbonyl)piperidin-4-yl)methyl)(methyl)amino)ethyl) piperidine-1-carboxylate

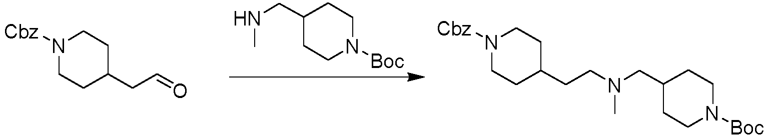

*tert*-Butyl 4-((methylamino)methyl)piperidine-1-carboxylate (2.8 g, 12.3 mmol) and acetic acid (1.5 mL) were added to a solution of benzyl 4-(2-oxoethyl)piperidine-1-carboxylate (2.66 g, 10.2 mmol) in DCM/MeOH (30 mL/15 mL). The mixture was stirred at room temperature for 2 h. NaBH(OAc)₃ (4.3 g, 20.4 mmol) was then added at 0 °C. The mixture was diluted with water (30 mL) and extracted with DCM (30 mL × 3). The combined organic phase was dried over anhydrous sodium sulfate and filtered, and the filtrate was concentrated and purified by silica gel column chromatography (DCM:MeOH = 20:1) to give benzyl 4-(2-(((1-(*tert*-butoxycarbonyl)piperidin-4-yl)methyl)(methyl)amino)ethyl)piperidine-1-carboxylate.

LC-MS: (ESI, m/z): [M+H]⁺= 474.1.

### Step 3: preparation of tert-butyl 4-((methyl(2-(piperidin-4-yl)ethyl)amino)methyl)piperidine-1-carboxylate

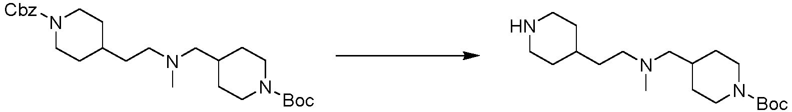

Pd/C (447 mg) was added to a solution of benzyl 4-(2-(((1-(*tert*-butoxycarbonyl)piperidin-4-yl)methyl) (methyl)amino)ethyl)piperidine-1-carboxylate (2 g, 4.2 mmol) in methanol (15 mL), and the mixture was stirred at 30 °C for 16 h under hydrogen atmosphere. The mixture was filtered and concentrated to give *tert-*butyl 4-((methyl(2-(piperidin-4-yl)ethyl)amino)methyl)piperidine-1-carboxylate. The crude product was directly used in the next step without further purification.

LC-MS: (ESI, m/z): [M+H]⁺= 340.3.

### Step 4: preparation of tert-butyl 4-(((2-(1-(4-chloro-3-(2,4-dioxotetrahydropyrimidin-1(2H)-yl)benzoyl) piperidin-4-yl)ethyl)(methyl)amino)methyl)piperidine-1-carboxylate

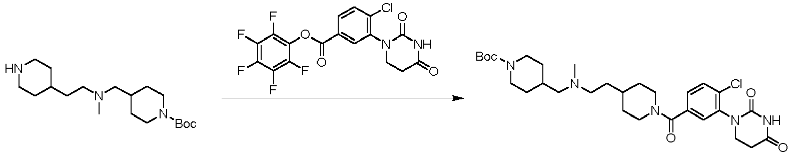

Pentafluorophenyl 4-chloro-3-(2,4-dioxotetrahydropyrimidin-1(2*H*)-yl)benzoate (769 mg, 1.77 mmol) and DIEA (686 mg, 5.31 mmol) were added to a solution of *tert*-butyl 4-((methyl(2-(piperidin-4-yl)ethyl)amino)methyl)piperidine-1-carboxylate (600 mg, crude) in DMF (8 mL). The mixture was stirred at room temperature for 2 h. After the reaction was completed, water (10 mL) was added to quench the reaction, and the mixture was extracted with ethyl acetate (30 mL × 3). The organic phase was dried, concentrated, and purified by silica gel column chromatography (DCM:MeOH = 20:1) to give *tert*-butyl 4-(((2-(1-(4-chloro-3-(2,4-dioxotetrahydropyrimidin-1(2*H*)-yl)benzoyl)piperidin-4-yl)ethyl)(methyl)amino)methyl)piperidine-1-carboxylate.

LC-MS: (ESI, m/z): [M+H]⁺ = 590.3.

### Step 5: preparation of 1-(2-chloro-5-(4-(2-(methyl(piperidin-4-ylmethyl)amino)ethyl)piperidine-1-carbonyl)phenyl) dihydropyrimidine-2,4(1H,3H)-dione

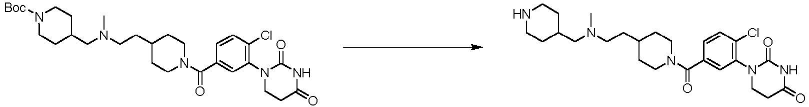

HCl/1,4-dioxane (2 mL) was added to a solution of *tert*-butyl 4-(((2-(1-(4-chloro-3-(2,4-dioxotetrahydropyrimidin-1(2H)-yl)benzoyl)piperidin-4-yl)ethyl)(methyl)amino)methyl)piperidine-1-carboxylate (900 mg, 1.5 mmol) in 1,4-dioxane (3 mL), and the mixture was stirred at 30 °C for 1 h. After the reaction was completed, an aqueous Na₂CO₃ solution (5 mL) was added to adjust the pH > 7, and the mixture was extracted with ethyl acetate (3 × 5 mL). The combined organic phase was dried over Na₂SO₄, filtered, concentrated under reduced pressure, and purified by silica gel column chromatography (DCM:MeOH (NH₃) = 20:1) to give 1-(2-chloro-5-(4-(2-(methyl(piperidin-4-ylmethyl)amino)ethyl)piperidine-1-carbonyl)phenyl) dihydropyrimidine-2,4(1*H*,3*H*)-dione.

LC-MS: (ESI, m/z): [M+H]⁺= 490.3.

### Step 6: preparation of (R)-1-(2-chloro-5-(4-(2-(((1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)piperidino[4,3-d]pyrimidin-2-yl)oxy)methyl)cyclopropyl)methyl)piperidin-4-yl)methyl)(methyl)amino)ethyl)piperidine-1-carbonyl)phenyl)dihydropyrimidine-2,4(1H,3H)-dione

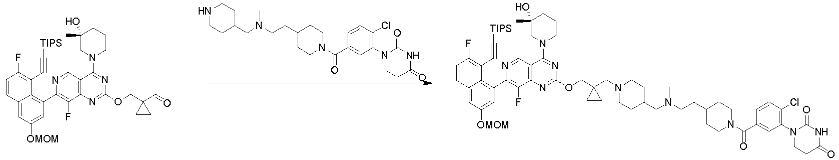

1-(2-Chloro-5-(4-(2-(methyl(piperidin-4-ylmethyl)amino)ethyl)piperidine-1-carbonyl)phenyl)dihydropyrimidine-2,4(1*H*,3*H*)-dione (78 mg, 0.16 mmol) and tetraisopropyl titanate (185 mg, 0.65 mmol) were added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)piperidino[4,3-*d*]pyrimidin-2-yl)oxy)methyl)cyclopropane-1-carbaldehyde (100 mg, 0.13 mmol) in dichloromethane (1 mL) and acetic acid (0.1 mL). The mixture was stirred at 25 °C for 2 h. NaBH(OAc)₃ (83 mg, 0.39 mmol) was added at 0 °C, and the mixture was stirred for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by column chromatography (DCM:MeOH = 15:1) to give (*R*)-1-(2-chloro-5-(4-(2-(((1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)piperidino[4,3-*d*]pyrimidin-2-yl)oxy)methyl)cyclopropyl)methyl)piperidin-4-yl)methyl)(methyl)amino)ethyl)piperidine-1-carbonyl)phenyl)dihydropyrimidine-2,4(1*H*,3*H*)-dione.

LC-MS: (ESI, m/z): [M/2+H]⁺= 609.5.

### Step 7: preparation of (R)-1-(2-chloro-5-(4-(2-(((1-((1-(((7-(8-ethynyl-7-fluoro-3-hydroxynaphthalen-1-yl)-8-fluoro-4-(3-hydroxy-3-methylpiperidin-1-yl)piperidino[4,3-d]pyrimidin-2-yl)oxy)methyl)cyclopropyl) methyl)piperidin-4-yl)methyl)(methyl)amino)ethyl)piperidine-1-carbonyl)phenyl)dihydropyrimidine-2,4(1H,3H)-dione

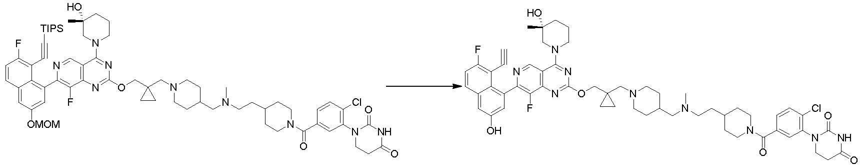

Cesium fluoride (62 mg, 0.41 mmol) was added to a stirred solution of *tert*-butyl (*R*)-1-(2-chloro-5-(4-(2-(((1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)piperidino[4,3-*d*]pyrimidin-2-yl)oxy)methyl)cyclopropyl)methyl)piperidin-4-yl)methyl) (methyl)amino)ethyl)piperidine-1-carbonyl)phenyl)dihydropyrimidine-2,4(1*H*,3*H*)-dione (100 mg, 0.082 mmol) in DMF (1 mL), and the mixture was stirred at 30 °C for 1 h. After the reaction was completed, the mixture was diluted with H₂O (5 mL), and extracted with ethyl acetate (5 mL × 3). The combined organic phase was dried over Na₂SO₄, filtered, and concentrated under reduced pressure. 1,4-Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give (*R*)-1-(2-chloro-5-(4-(2-(((1-((1-(((7-(8-ethynyl-7-fluoro-3-hydroxynaphthalen-1-yl)-8-fluoro-4-(3-hydroxy-3-methylpiperidin-1-yl)piperidino[4,3-*d*]pyrimidin-2-yl)oxy) methyl)cyclopropyl)methyl)piperidin-4-yl)methyl)(methyl)amino)ethyl)piperidine-1-carbonyl)phenyl) dihydropyrimidine-2,4(1*H*,3*H*)-dione.

LC-MS: (ESI, m/z): [M/2+H] ⁺ = 509.8.

¹H NMR (400 MHz, DMSO-*d₆*) δ 10.49 (s, 1H), 10.14 (s, 1H), 9.12 (d, *J* = 66.0 Hz, 1H), 7.97 (dd, *J* = 9.2, 6.0 Hz, 1H), 7.61 (d, *J* = 8.2 Hz, 1H), 7.53 (d, *J* = 1.9 Hz, 1H), 7.49 - 7.42 (m, 1H), 7.40 - 7.34 (m, 2H), 7.20 (dd, *J =* 17.0, 2.4 Hz, 1H), 4.73 (d, *J =* 30.9 Hz, 1H), 4.45 - 4.20 (m, 4H), 4.12 - 3.92 (m, 2H), 3.80 - 3.68 (m, 1H), 3.66 - 3.46 (m, 3H), 3.02 - 2.84 (m, 3H), 2.78 - 2.66 (m, 3H), 2.34 - 2.20 (m, 4H), 2.09 - 1.98 (m, 6H), 1.88 - 1.48 (m, 11H), 1.39 - 1.27 (m, 3H), 1.17 (d, *J =* 16.8 Hz, 3H), 1.10 - 0.94 (m, 4H), 0.67 - 0.59 (m, 2H), 0.43 - 0.35 (m, 2H).

### Example 19: (R)-1-(5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of (R)-1-(5-(9-((4-((1-(((7-(8-ethyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl) methyl)piperazin-1-yl)methyl)-3-azaspiro[5.5]undecane-3-carbonyl)-2-methylphenyl)dihydropyrimidine-2,4(1H,3H)-dione

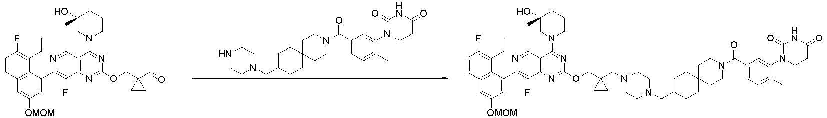

Tetraisopropyl titanate (120 mg, 421.85 µmol) was added to a solution of (*R*)-1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde (50 mg, 84.37 µmol) and 1-(2-methyl-5-(9-(piperazin-1-ylmethyl)-3-azaspiro[5.5]undecane-3-carbonyl)phenyl)dihydropyrimidine-2,4(1*H*,3*H*-dione (61 mg, 126.56 µmol) in dichloromethane (10 mL), and the mixture stirred at 25 °C for 2 h. Sodium triacetoxyborohydride (54 mg, 253.11 µmol) was added to the mixture at room temperature, and the mixture was reacted at 25 °C for another 16 h. The reaction liquid was concentrated under reduced pressure at 40 °C. The resulting crude product was purified by a preparative thin-layer chromatography plate (dichloromethane:methanol = 10:1, 0.1% NH₃) to give (*R*)-1-(5-(9-((4-((1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl) piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1058.5.

### Step 2: preparation of (R)-1-(5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

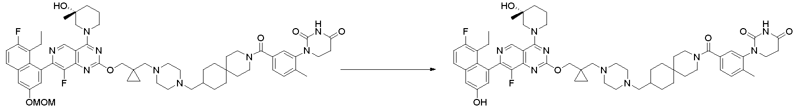

A hydrogen chloride/1,4-dioxane solution (4.0 M, 4 mL) was added to a solution of (*R*)-1-(5-(9-((4-((1-(((7-(8-ethyl-7-fluoro-3-(methoxymethoxy)naphthalen-1-yl)-8-fluoro-4-(3-hydroxy-3-methylpiperidin-1-yl) pyrido[4,3-*d*]pyrimidin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1*H*,3*H*)-dione (43 mg, 40.29 µmol) in 1,4-dioxane (2 mL) at room temperature, and the mixture was reacted at room temperature for 1 h. After the reaction was completed, the mixture was concentrated under reduced pressure at 30 °C, and a solution of ammonia in methanol (1 mL) was then added to the residual liquid. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give (*R*)-1-(5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl) methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 507.8.

¹H NMR (400 MHz, CD₃OD)δ 9.20 (d, *J* = 4.0 Hz, 1H), 7.68 - 7.65 (m, 1H), 7.40 (d, *J* = 8.0 Hz, 1H), 7.33 - 7.29 (m, 3H), 7.27 - 7.20 (m, 1H), 7.05 (s, 1H), 4.56 (s, 1H), 4.51 - 4.48 (m, 2H), 4.41 - 4.37 (m, 1H), 4.24 (t, *J* = 12.0 Hz, 1H), 3.90 - 3.83 (m, 1H), 3.68 - 3.58 (m, 4H), 3.51 - 3.42 (m, 4H), 2.88 - 2.78 (m, 2H), 2.60 - 2.37 (m, 10H), 2.31 (s, 3H), 2.25 - 2.17 (m, 4H), 1.85 - 1.74 (m, 4H), 1.61 - 1.43 (m, 8H), 1.33 - 1.27 (m, 5H), 1.20 - 1.05 (m, 2H), 0.84 - 0.71 (m, 3H), 0.75 - 0.65 (m, 2H), 0.55 - 0.45 (m, 2H).

### Example 20: (R)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperidin-1-yl)methyl)-3-azaspiro[5.5]undecane-3-carbonyl)phenyl)dihydropyrimidine-2,4(1H,3H)-dione

### Step 1: preparation of tert-butyl (R)-4-((1-(((7-bromo-8-fluoro-4-(3-hydroxy-3-methylpiperidin-1-yl)quinazolin-2-yl)oxy)methyl)cyclopropyl)methyl)piperidine-1-carboxylate

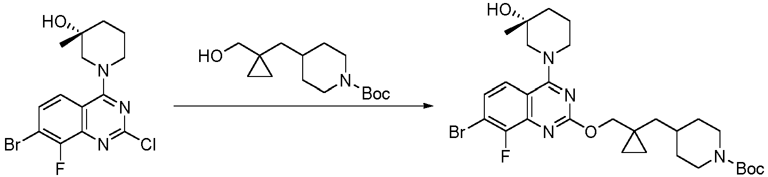

Triethylenediamine (16 mg, 139.21 µmol) and anhydrous cesium carbonate (272 mg, 835.23 µmol) were added to a solution of (*R*)-1-(7-bromo-2-chloro-8-fluoroquinazolin-4-yl)-3-methylpiperidin-3-ol (104 mg, 278.41 µmol) and *tert*-butyl 4-((1-(hydroxymethyl)cyclopropyl)methyl)piperidine-1-carboxylate (150 mg, 556.81 µmol) in anhydrous acetonitrile (10 mL), and the mixture was stirred at 25 °C for 18 h. The reaction liquid was poured into water (100 mL), and the mixture was extracted with ethyl acetate (20 mL × 2). The combined organic phase was washed with saturated sodium chloride (100 mL), dried over anhydrous sodium sulfate, and concentrated under reduced pressure. The resulting crude product was purified by a preparative silica gel thin-layer chromatography plate (dichloromethane:methanol = 15:1) to give *tert*-butyl (*R*)-4-((1-(((7-bromo-8-fluoro-4-(3-hydroxy-3-methylpiperidin-1-yl)quinazolin-2-yl)oxy)methyl)cyclopropyl)methyl)piperidine-1-carboxylate.

LC-MS: (ESI, m/z): [M+H]⁺ = 606.9, 608.9.

### Step 2: preparation of tert-butyl (R)-4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)quinazolin-2-yl)oxy)methyl)cyclopropyl) methyl)piperidine-1-carboxylate

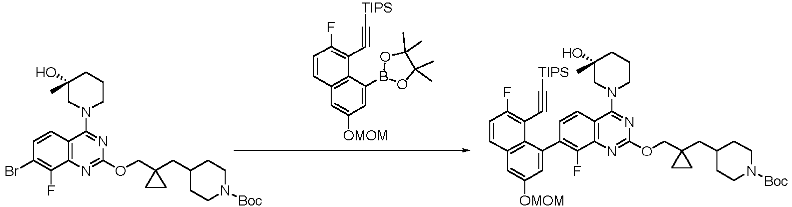

Anhydrous cesium carbonate (272 mg, 835.23 µmol) and cataCXium(R) A Pd G3 (41 mg, 55.68 µmol) were added to a solution of *tert*-butyl (*R*)-4-((1-(((7-bromo-8-fluoro-4-(3-hydroxy-3-methylpiperidin-1-yl)quinazolin-2-yl)oxy)methyl)cyclopropyl)methyl)piperidine-1-carboxylate (167 mg, 275.62 µmol) and ((2-fluoro-6-(methoxymethoxy)-8-(4,4,5,5-tetramethyl-1,3,2-dioxaborolan-2-yl)naphthalen-1-yl)ethynyl) triisopropylsilane (286 mg, 556.82 µmol) in 1,4-dioxane (12 mL) and water (2 mL) at room temperature. After the system was degassed, the mixture was stirred at 75 °C for 18 h under nitrogen atmosphere. The reaction liquid was poured into water (100 mL), and the mixture was extracted with ethyl acetate (20 mL × 2). The combined organic phase was washed with saturated sodium chloride (100 mL), dried over anhydrous sodium sulfate, filtered and concentrated under reduced pressure. The crude product was purified by silica gel thin-layer chromatography (dichloromethane:methanol =10:1, 0.1% NH₃) to give *tert*-butyl (*R*)-4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropyl)methyl)piperidine-1-carboxylate.

LC-MS: (ESI, m/z): [M+H]⁺ = 912.9.

### Step 3: preparation of tert-butyl (R)-4-((1-(((7-(8-ethynyl-7-fluoro-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperidine-1-carboxylate

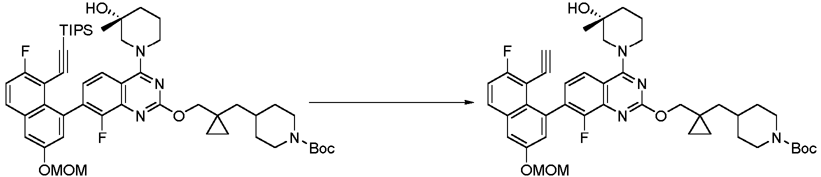

Cesium fluoride (324 mg, 2.132 mmol) was added to a solution of *tert*-butyl (*R*)-4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropyl)methyl)piperidine-1-carboxylate (195 mg, 213.20 µmol) in *N,N*-dimethylformamide (20 mL) at room temperature, and the mixture was reacted at room temperature for 18 h. The reaction liquid was poured into water (100 mL), and the mixture was extracted with ethyl acetate (20 mL × 2). The combined organic phase was washed with saturated sodium chloride (100 mL), dried over anhydrous sodium sulfate, filtered and concentrated under reduced pressure. *tert-Butyl* (*R*)-4-((1-(((7-(8-ethynyl-7-fluoro-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperidine-1-carboxylate was obtained.

LC-MS: (ESI, m/z): [M+H]⁺ = 756.9.

### Step 4: preparation of (R)-1-(7-(8-ethynyl-7-fluoro-3-hydroxynaphthalen-1-yl)-8-fluoro-2-((1-(piperidin-4-ylmethyl)cyclopropyl)methoxy)quinazolin-4-yl)-3-methylpiperidin-3-ol

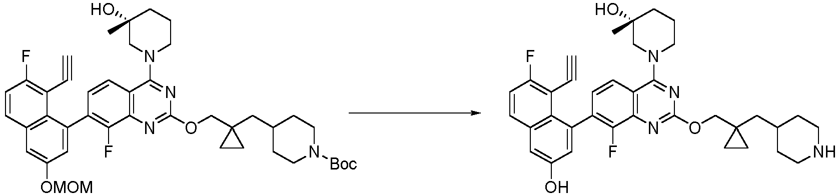

A hydrogen chloride/1,4-dioxane solution (4.0 M, 6 mL) was added to a solution of *tert-Butyl (R)-*4-((1-(((7-(8-ethynyl-7-fluoro-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperidine-1-carboxylate (162 mg, 213.20 µmol) in 1,4-dioxane (3 mL) at room temperature, and the mixture was reacted at room temperature for 1 h. The reaction liquid was concentrated under reduced pressure. A solution of ammonia in methanol (1 mL) was added to the residue, and the mixture was then concentrated under reduced pressure. The resulting crude product was purified by a preparative thin-layer chromatography plate (dichloromethane:methanol = 10:1, 0.1% NH₃) and then purified by preparative high-performance liquid chromatography to give (*R*)-1-(7-(8-ethynyl-7-fluoro-3-hydroxynaphthalen-1-yl)-8-fluoro-2-((1-(piperidin-4-ylmethyl)cyclopropyl)methoxy)quinazolin-4-yl)-3-methylpiperidin-3-ol.

LC-MS: (ESI, m/z): [M+H]⁺ = 612.9.

### Step 5: preparation of (R)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperidin-1-yl)methyl)-3-azaspiro[5.5]undecane-3-carbonyl)phenyl)dihydropyrimidine-2,4(1H,3H)-dione

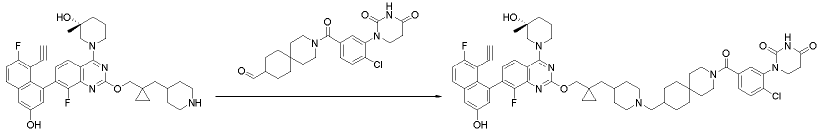

Tetraisopropyl titanate (118 mg, 412.90 µmol) was added to a solution of (*R*)-1-(7-(8-ethynyl-7-fluoro-3-hydroxynaphthalen-1-yl)-8-fluoro-2-((1-(piperidin-4-ylmethyl)cyclopropyl)methoxy)quinazolin-4-yl)-3-methylpiperidin-3-ol (72 mg, 165.16 µmol) in dichloromethane (12 mL), and the mixture was stirred at 25 °C for 16 h. Sodium triacetoxyborohydride (53 mg, 247.74 µmol) was added to the mixture at room temperature, and the mixture was reacted at 25 °C for another 3 h. Methanol (10 mL) was added to the reaction liquid to quench the reaction, and the mixture was concentrated under reduced pressure at 40 °C. The resulting crude product was purified by a preparative thin-layer chromatography plate (dichloromethane:methanol = 10:1, 0.1% NH₃) to give (*R*)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperidin-1-yl)methyl)-3-azaspiro[5.5]undecane-3-carbonyl)phenyl)dihydropyrimidine-2,4(1*H*,3*H*)-dione.

LC-MS: (ESI, m/z): [M/2+H]⁺ = 514.7.

¹H NMR(400 MHz, DMSO-*d*₆)δ 10.51 (s, 1H), 10.13 (s, 1H), 7.98 - 7.94 (m, 1H), 7.87 - 7.76 (m, 1H), 7.63 (d, *J = 8.0* Hz, 1H), 7.55 (d, *J = 4.0* Hz, 1H), 7.48 - 7.42 (m, 1H), 7.39 - 7.36 (m, 1H), 7.36 (d, *J* = 4.0 Hz, 1H), 7.23 - 7.18 (m, 1H), 7.10 - 7.06 (m, 1H), 4.65 (*d, J =* 12.0 Hz, 1H), 4.26 - 3.88 (m, 4H), 3.81 (s, 1H), 3.80 - 3.70 (m, 2H), 3.65 - 3.48 (m, 4H), 3.30 - 3.25 (m, 2H), 2.75 - 2.72 (m, 4H), 2.05 - 1.95 (m, 2H), 1.71 - 1.60 (m, 8H), 1.55- 1.38 (m, 6H), 1.35 - 1.20 (m, 8H), 1.16 (d, *J =* 8.0 Hz, 3H), 1.10 - 1.04 (m, 4H), 0.58 - 0.50 (m, 2H), 0.40 - 0.30 (m, 2H).

### Example 21: (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro [5.5]undecane-3-carbonyl)-2-methoxyphenyl)dihydropyrimidine-2,4(1H,3H)-dione

### Step 1: preparation of (R)-1-(5-(9-((4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)quinazolin-2-yl)oxy)methyl)cyclopropyl) methyl)piperazin-1-yl)methyl)-3-azaspiro[5.5]undecane-3-carbonyl)-2-methoxyphenyl)dihydropyrimidine-2,4(1H,3H)-dione

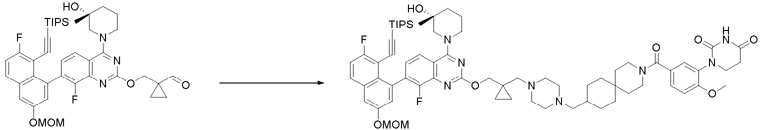

Tetraisopropyl titanate (153 mg, 537.65 µmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropane-1-carbaldehyde (80 mg, 107.53 µmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (70 mg, 139.79 µmol) in dichloromethane (10 mL), and the mixture stirred at 25 °C for 2 h. Sodium triacetoxyborohydride (68 mg, 322.59 µmol) was added to the mixture at room temperature, and the mixture was reacted at 25 °C for another 16 h. Methanol (10 mL) was added to quench the reaction, and the mixture was concentrated under reduced pressure at 40 °C. The resulting crude product was purified by a preparative thin-layer chromatography plate (dichloromethane:methanol = 10:1, 0.1% NH₃) to give (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1*H*,3*H*)-dione.

LC-MS: (ESI, m/z): [M/2+H]⁺ = 613.5.

### Step 2: preparation of (R)-1-(5-(9-((4-((1-(((7-(8-ethynyl-7-fluoro-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxvehenyl)dihydropyrimidine-2,4(1H,3H)-dione

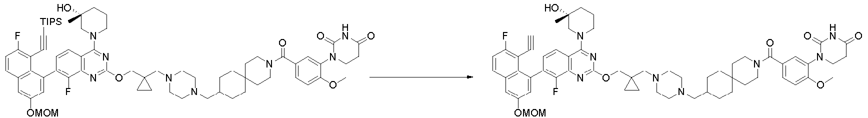

Cesium fluoride (158 mg, 1.036 mmol) was added to a solution of (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione (127 mg, 103.62 µmol) in *N,N*-dimethylformamide (5 mL) at room temperature, and the mixture was reacted at room temperature for 18 h. The reaction liquid was poured into water (100 mL), and the mixture was extracted with ethyl acetate (20 mL × 2). The combined organic phase was washed with saturated sodium chloride (100 mL), dried over anhydrous sodium sulfate, filtered and concentrated under reduced pressure. *(R)-1-(5-*(9-((4-((1-(((7-(8-Ethynyl-7-fluoro-3-(methoxymethoxy)naphthalen-1-yl)-8-fluoro-4-(3-hydroxy-3-methylpiperidin-1-yl)quinazolin-2-yl)oxy) methyl)cyclopropyl)methyl)piperazin-1-yl)methyl)-3 -azaspiro [5.5]undecane-3-carbonyl)-2-methoxyphenyl) dihydropyrimidine-2,4(1*H*,3*H*)-dione was obtained.

LC-MS: (ESI, m/z): [M/2+H]⁺ = 535.0.

### Step 3: preparation of (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

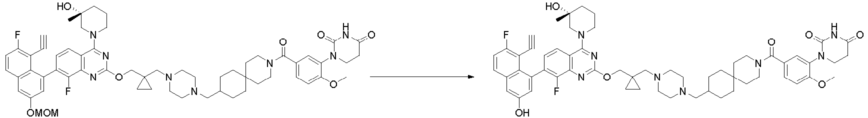

A hydrogen chloride/1,4-dioxane solution (4.0 M, 4 mL) was added to a solution of *(R)-1-(5-(9-*((4-((1-(((7-(8-ethynyl-7-fluoro-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione (94 mg, 87.91 µmol) in 1,4-dioxane (2 mL) at room temperature, and the mixture was reacted at room temperature for 1 h. The reaction liquid was concentrated at 30 °C under reduced pressure. A solution of ammonia in methanol (1 mL) was added to the residue, and the mixture was then concentrated under reduced pressure at 30 °C. The resulting crude product was purified by a preparative thin-layer chromatography plate (dichloromethane:methanol = 10:1, 0.1% NH₃) and then separated by preparative high-performance liquid chromatography to give (*R*)-1-(5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 513.2.

¹H NMR (400 MHz, DMSO-*d*₆) δ 10.33 (s, 1H), 10.08 (br, 1H), 7.96 (dd, *J* = 9.1, 6.0 Hz, 1H), 7.81 (dd, *J* = 34.2, 8.6 Hz, 1H), 7.48 - 7.43 (m, 1H), 7.37 - 7.35 (m, 2H), 7.32 (d, *J* = 4.0 Hz, 1H), 7.22 - 7.17 (m, 1H), 7.14 (d, *J* = 8.0 Hz, 1H), 7.08 (dd, *J* = 12.3, 2.4 Hz, 1H), 4.64 (d, *J* = 24.0 Hz, 1H), 4.28 - 4.15 (m, 2H), 4.15 - 3.95 (m, 1H), 3.90 - 3.73 (m, 1H), 3.84 (s, 3H), 3.63 - 3.55 (m, 2H), 3.53 - 3.36 (m, 4H), 3.29 - 3.25 (m, 2H), 2.70 - 2.65 (m, 2H), 2.45 - 2.15 (m, 10H), 2.15 - 1.90 (m, 4H), 1.75 - 1.60 (m, 4H), 1.55 - 1.25 (m, 8H), 1.16 (d, *J =* 8.0 Hz, 3H), 1.10 - 0.95 (m, 4H), 0.65 - 0.55 (m, 2H), 0.45 - 0.35 (m, 2H).

### Example 22: (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperidin-1-yl)methyl)-3-azaspiro[5.5]undecane-3-carbonyl)-2-methylphenyl)dihydropyrimidine-2,4(1H,3H)-dione

### Step 1: preparation of tert-butyl (R)-4-((1-(((7-chloro-8-fluoro-4-(3-hydroxy-3-methylpiperidin-1-yl)pyrido[4,3-d]pyrimidin-2-yl)oxy)methyl)cyclopropyl)methyl)piperidine-1-carboxylate

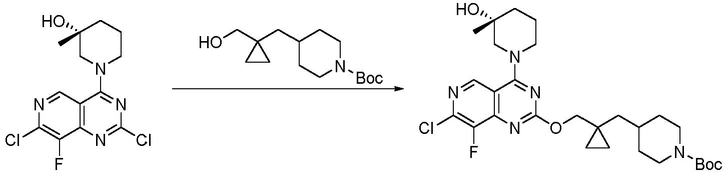

Triethylenediamine (34 mg, 301.96 µmol) and anhydrous cesium carbonate (590 mg, 1.812 mmol) were added to a solution of (*R*)-1-(2,7-dichloro-8-fluoropyrido[4,3-*d*]pyrimidin-4-yl)-3-methylpiperidin-3-ol (200 mg, 603.92 µmol) and *tert-*butyl 4-((1-(hydroxymethyl)cyclopropyl)methyl)piperidine-1-carboxylate (394 mg, 1.463 mmol) in anhydrous acetonitrile (15 mL), and the mixture was stirred at 25 °C for 18 h. The reaction liquid was poured into water (100 mL), and the mixture was extracted with ethyl acetate (20 mL × 2). The combined organic phase was washed with saturated sodium chloride (100 mL), dried over anhydrous sodium sulfate, and concentrated under reduced pressure. The resulting crude product was purified by silica gel column chromatography (dichloromethane:methanol = 100:1 to 15:1) to give *tert-*butyl (*R*)-4-((1-(((7-chloro-8-fluoro-4-(3-hydroxy-3-methylpiperidin-1-yl)pyrido[4,3-*d*]pyrimidin-2-yl)oxy)methyl)cyclopropyl)methyl)piperidine-1-carboxylate.

LC-MS: (ESI, m/z): [M+H]⁺ = 563.9.

### Step 2: preparation of tert-butyl (R)-4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-d]pyrimidin-2-yl)oxy)methyl) cyclopropyl)methyl)piperidine-1-carboxylate

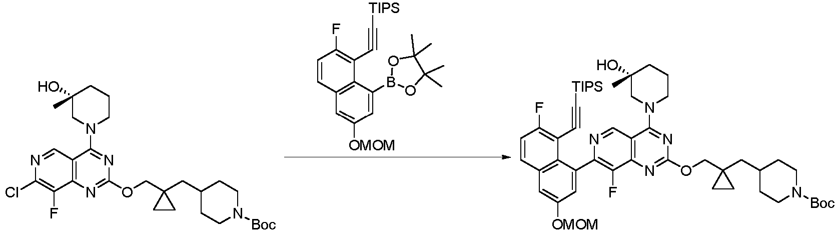

Anhydrous cesium carbonate (205 mg, 630.21 µmol) and cataCXium(R) A Pd G3 (31 mg, 42.01 µmol) were added to a solution of *tert*-butyl (*R*)-4-((1-(((7-chloro-8-fluoro-4-(3-hydroxy-3-methylpiperidin-1-yl)pyrido[4,3-*d*]pyrimidin-2-yl)oxy)methyl)cyclopropyl)methyl)piperidine-1-carboxylate (119 mg, 210.07 µmol) and ((2-fluoro-6-(methoxymethoxy)-8-(4,4,5,5-tetramethyl-1,3,2-dioxaborolan-2-yl)naphthalen-1-yl)ethynyl)triisopropylsilane (216 mg, 420.14 µmol) in 1,4-dioxane (12 mL) and water (3 mL) at room temperature. The mixture was stirred at 75 °C for 18 h under nitrogen atmosphere. The reaction liquid was poured into water (100 mL), and the mixture was extracted with ethyl acetate (20 mL × 2). The combined organic phase was washed with saturated sodium chloride (100 mL), dried over anhydrous sodium sulfate, filtered and concentrated under reduced pressure. The crude product was purified by silica gel thin-layer chromatography (dichloromethane:methanol =10:1, 0.1% NH₃) to give *tert-butyl* (*R*)-4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperidine-1-carboxylate.

LC-MS: (ESI, m/z): [M+H]⁺ = 913.9.

### Step 3: preparation of tert-butyl (R)-4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl)methyl) piperidine-1-carboxylate

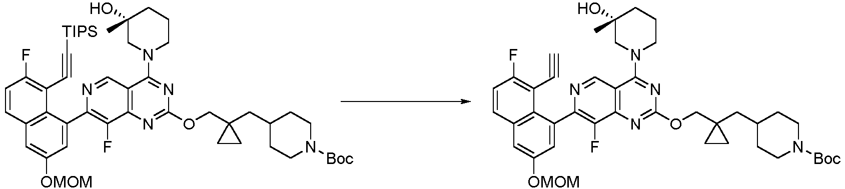

Cesium fluoride (276 mg, 1.881 mmol) was added to a solution of *tert*-butyl (*R*)-4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl) pyrido[4,3-*d*]pyrimidin-2-yl)oxy)methyl)cyclopropyl)methyl)piperidine-1-carboxylate (172 mg, 188.14 µmol) in *N,N-*dimethylformamide (10 mL) at room temperature, and the mixture was reacted at room temperature for 18 h. The reaction liquid was poured into water (100 mL), and the mixture was extracted with ethyl acetate (20 mL × 2). The combined organic phase was washed with saturated sodium chloride (100 mL), dried over anhydrous sodium sulfate, filtered and concentrated under reduced pressure. *tert*-Butyl (*R*)-4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperidine-1-carboxylate was obtained.

LC-MS: (ESI, m/z): [M+H]⁺ = 757.9.

### Step 4: preparation of (R)-1-(7-(8-ethynyl-7-fluoro-3-hydroxynaphthalen-1-yl)-8-fluoro-2-((1-(piperidin-4-ylmethyl)cyclopropyl)methoxy)pyrido[4,3-d]pyrimidin-4-yl)-3-methylpiperidin-3-ol

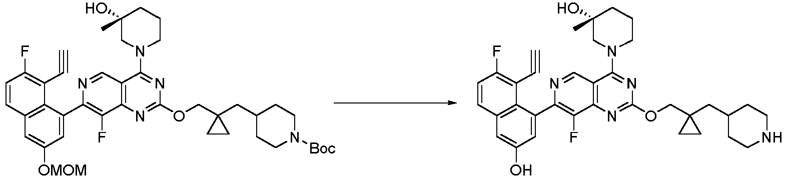

A hydrogen chloride/1,4-dioxane solution (4.0 M, 6 mL) was added to a solution of *tert-Butyl (R)-*4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperidine-1-carboxylate (158 mg, 188.14 µmol) in 1,4-dioxane (2 mL) at room temperature, and the mixture was reacted at room temperature for 1 h. The reaction liquid was concentrated under reduced pressure at 30 °C, and a solution of ammonia in methanol (1 mL) was added to the residue. Then the mixture was concentrated under reduced pressure at 30 °C. The resulting crude product was purified by a preparative thin-layer chromatography plate (dichloromethane:methanol = 10:1, 0.1% NH₃) to give (*R*)-1-(7-(8-ethynyl-7-fluoro-3-hydroxynaphthalen-1-yl)-8-fluoro-2-((1-(piperidin-4-ylmethyl)cyclopropyl)methoxy)pyrido[4,3-*d*]pyrimidin-4-yl)-3-methylpiperidin-3-ol.

LC-MS: (ESI, m/z): [M+H]⁺ = 614.3.

### Step 5: preparation of (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperidin-1-yl)methyl)-3-azaspiro[5.5]undecane-3-carbonyl)-2-methylphenyl)dihydropyrimidine-2,4(1H,3H)-dione

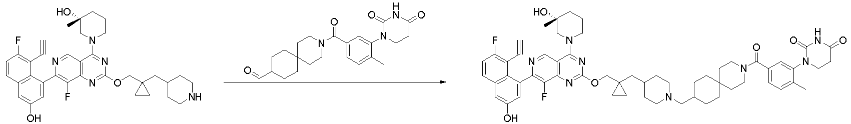

Tetraisopropyl titanate (111 mg, 390.25 µmol) was added to a solution of (*R*)-1-(7-(8-ethynyl-7-fluoro-3-hydroxynaphthalen-1-yl)-8-fluoro-2-((1-(piperidin-4-ylmethyl)cyclopropyl)methoxy)pyrido[4,3-*d*]pyrimidin-4-yl)-3-methylpiperidin-3-ol (48 mg, 64 µmol) and 3-(3-(2,4-dioxotetrahydropyrimidin-1(2*H*)-yl)-4-methylbenzoyl)-3-azaspiro[5.5]undecane-9-carbaldehyde (64 mg, 156.1 µmol) in dichloromethane (5 mL), and the mixture was stirred at 25 °C for 16 h. Sodium triacetoxyborohydride (50 mg, 234.15 µmol) was added to the mixture at room temperature, and the mixture was then reacted at 25 °C for another 3 h. Methanol (10 mL) was added to the reaction liquid to quench the reaction, and the mixture was concentrated under reduced pressure at 40 °C. The resulting crude product was purified by a preparative thin-layer chromatography plate (dichloromethane:methanol = 10:1, 0.1% NH₃) and then purified by Prep-HPLC to give (*R*)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido [4,3-*d*]pyrimidin-2-yl)oxy)methyl)cyclopropyl)methyl)piperidin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1009.2.

¹H NMR (400 MHz, DMSO-*d*₆) δ 10.37 (s, 1H), 10.19 (brs, 1H), 9.13 (d, *J* = 64.0 Hz, 1H), 7.97 (dd, *J=* 9.1, 5.9 Hz, 1H), 7.83 - 7.52 (m, 1H), 7.49 - 7.42 (m, 1H), 7.41 - 7.36 (m, 1H), 7.33 (d, *J=* 8.0 Hz, 1H), 7.30 (s, 1H), 7.25 - 7.18 (m, 2H), 4.75 (d, *J* = 28.0 Hz, 1H), 4.37 - 4.25 (m, 1H), 4.23 - 4.15 (m, 1H), 4.15 - 4.05 (m, 1H), 3.95 - 3.90 (m, 1H), 3.85 - 3.75 (m, 1H), 3.60 - 3.50 (m, 4H), 3.30 - 3.25 (m, 2H), 2.80 - 2.70 (m, 4H), 2.21 (s, 3H), 2.05 - 1.95 (m, 3H), 1.83 - 1.75 (m, 2H), 1.73 - 1.65 (m, 6H), 1.55 - 1.47 (m, 4H), 1.45 - 1.37 (m, 2H), 1.35 - 1.30 (m, 2H), 1.25 - 1.20 (m, 4H), 1.20 - 1.15 (m, 4H), 1.10 - 1.03 (m, 4H), 0.90 - 0.83 (m, 1H), 0.57 - 0.50 (m, 2H), 0.40 - 0.35 (m, 2H).

### Example 23: (R)-1-(5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of (R)-1-(7-(8-ethyl-3-(methoxymethoxy)naphthalen-1-yl)-8-fluoro-2-((1-(hydroxymethyl) cyclopropyl)methoxy)quinazolin-4-yl)-3-methylpiperidin-3-ol

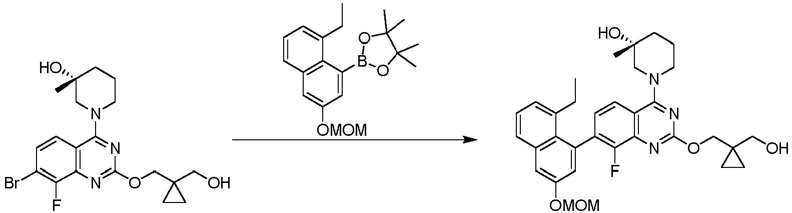

Anhydrous cesium carbonate (336 mg, 1.022 mmol) and cataCXium (R)A Pd G3 (37 mg, 51.10 µmol) were added to a solution of (*R*)-1-(7-bromo-8-fluoro-2-((1-(hydroxymethyl)cyclopropyl)methoxy)quinazolin-4-yl)-3-methylpiperidin-3-ol (150 mg, 340.67 µmol) and 2-(8-ethyl-3-(methoxymethoxy)naphthalen-1-yl)-4,4,5,5-tetramethyl-1,3,2-dioxaborolane (140 mg, 408.80 µmol) in 1,4-dioxane (12 mL) and water (3 mL) at room temperature. The mixture was stirred at 75 °C for 18 h under nitrogen atmosphere. The reaction liquid was poured into water (100 mL), and the mixture was extracted with ethyl acetate (20 mL × 2). The combined organic phase was washed with saturated sodium chloride (100 mL), dried over anhydrous sodium sulfate, filtered and concentrated under reduced pressure. The resulting crude product was purified by silica gel thin-layer chromatography (dichloromethane:methanol = 10:1, 0.1% NH₃) to give (R)-1-(7-(8-ethyl-3-(methoxymethoxy)naphthalen-1-yl)-8-fluoro-2-((1-(hydroxymethyl)cyclopropyl)methoxy)quinazolin-4-yl)-3-methylpiperidin-3-ol.

LC-MS: (ESI, m/z): [M+H]⁺ = 576.3.

### Step 2: preparation of (R)-1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropane-1-carbaldehyde

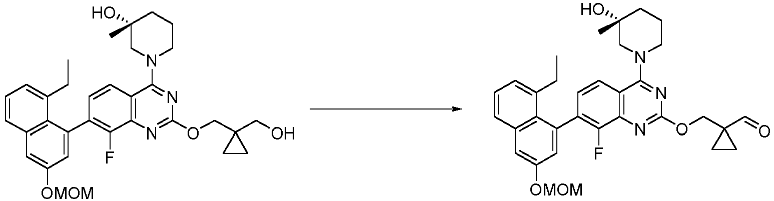

A solution of (*R*)-1-(7-(8-ethyl-3-(methoxymethoxy)naphthalen-1-yl)-8-fluoro-2-((1-(hydroxymethyl) cyclopropyl)methoxy)quinazolin-4-yl)-3-methylpiperidin-3-ol (137 mg, 238.33 µmol) in dichloromethane (15 mL) was cooled to 0 °C from room temperature, and Dess-Martin reagent (132 mg, 309.83 µmol) was added. The mixture was stirred at 25 °C for 2 h. The reaction liquid was poured into a saturated aqueous sodium bicarbonate solution (100 mL), and extracted with dichloromethane (20 mL × 2). The combined organic phase was washed with saturated sodium chloride (100 mL), dried over anhydrous sodium sulfate, filtered and concentrated under reduced pressure. The resulting crude product was purified by silica gel thin-layer chromatography (petroleum ether:ethyl acetate = 1:1, 0.1% NH₃) to give (*R*)-1-(((7-(8-ethyl-3-(methoxymethoxy)naphthalen-1-yl)-8-fluoro-4-(3-hydroxy-3-methylpiperidin-1-yl)quinazolin-2-yl)oxy)methyl) cyclopropane-1-carbaldehyde.

LC-MS: (ESI, m/z): [M+H]⁺ = 574.3.

### Step 3: preparation of (R)-1-(5-(9-((4-((1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

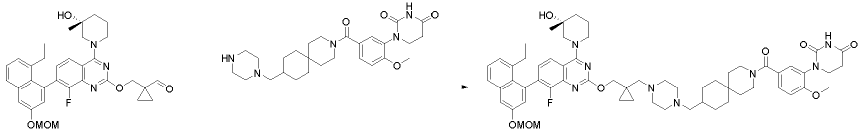

Tetraisopropyl titanate (124 mg, 435.80 µmol) was added to a solution of (*R*)-1-(((7-(8-ethyl-3-(methoxymethoxy)naphthalen-1-yl)-8-fluoro-4-(3-hydroxy-3-methylpiperidin-1-yl)quinazolin-2-yl)oxy) methyl)cyclopropane-1-carbaldehyde (50 mg, 87.16 µmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (46 mg, 91.52 µmol) in dichloromethane (5 mL), and the mixture stirred at 25 °C for 2 h. Sodium triacetoxyborohydride (56 mg, 261.48 µmol) was added to the mixture at room temperature, and the mixture was then reacted at 25 °C for another 3 h. Methanol (10 mL) was added to the reaction liquid to quench the reaction, and the mixture was then concentrated under reduced pressure at 40 °C. The resulting crude product was purified by a preparative thin-layer chromatography plate (dichloromethane:methanol = 10:1, 0.1% NH₃) to give (*R*)-1-(5-(9-((4-((1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 528.4.

### Step 4: preparation of (R)-1-(5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1H,3H)-dione

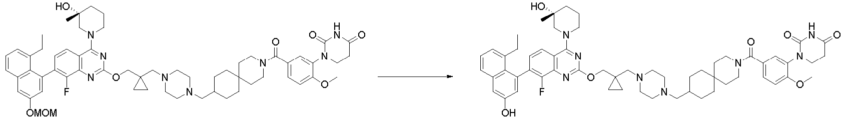

A hydrogen chloride/1,4-dioxane solution (4.0 M, 6 mL) was added to a solution of *(R)-1-(5-(9-*((4-((1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)
dihydropyrimidine-2,4(1*H*,3*H*)-dione (61 mg, 57.23 µmol) in 1,4-dioxane (3 mL) at room temperature, and the mixture was reacted at room temperature for 1 h. The reaction liquid was concentrated under reduced pressure at 30 °C, and a solution of ammonia in methanol (1 mL) was added to the residue. Then the mixture was concentrated under reduced pressure at 30 °C. The resulting crude product was purified by a preparative thin-layer chromatography plate (dichloromethane:methanol = 10:1, 0.1% NH₃) and then separated by preparative high-performance liquid chromatography to give (*R*)-1-(5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1H,3H)-dione.

LC-MS: (ESI, m/z): [M/2+H]⁺ = 506.3.

¹H NMR (400 MHz, CD₃OD) δ 7.91 (dd, *J* = 8.4 Hz, 4.8 Hz, 1H), 7.59 (d, *J* = 8.0 Hz, 1H), 7.43 (dd, *J* = 8.8 Hz, 2.4 Hz, 1H), 7.37 (d, *J* = 2.0 Hz, 1H), 7.35 - 7.28 (m, 1H), 7.25 - 7.18 (m, 2H), 7.17 (d,*J* = 8.4 Hz, 1H), 7.11 (d, *J* = 6.8 Hz, 1H), 6.91 (d, *J* = 2.4 Hz, 1H), 4.47 (d, *J* = 10.8 Hz, 1H), 4.36 (d,*J* = 10.8 Hz, 1H), 4.20 - 4.13 (m, 1H), 4.02 (d, *J* = 13.2 Hz, 1H), 3.91 (s, 3H), 3.80 - 3.60 (m, 4H), 3.60 - 3.45 (m, 4H), 2.80 (t, *J* = 6.8 Hz, 2H), 2.55 - 2.47 (m, 2H), 2.45 - 2.35 (m, 6H), 2.25 - 2.00 (m, 4H), 2.00 - 1.65 (m, 6H), 1.65 - 1.30 (m, 8H), 1.31 - 1.23 (m, 4H), 1.23 - 1.05 (m, 4H), 0.89- 0.84 (m, 3H), 0.75 - 0.65 (m, 2H), 0.50 - 0.42 (m, 2H).

### Example 24: (R)-1-(5-(9-((4-((1-(((7-(8-ethynyl-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1H,3H)-dione

### Step 1: preparation of (R)-1-(8-fluoro-2-((1-(hydroxymethyl)cyclopropyl)methoxy)-7-(3-(methoxymethoxy)-8-((triisopropylsilyl)ethynyl)naphthalen-1-yl)quinazolin-4-yl)-3-methylpiperidin-3-ol

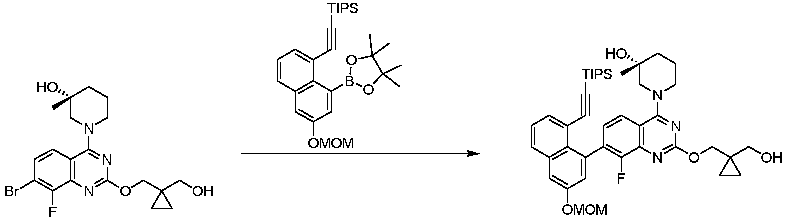

Anhydrous cesium carbonate (269 mg, 817.62 µmol) and cataCXium(R) A Pd G3 (30 mg, 40.88 µmol) were added to a solution of (*R*)-1-(7-bromo-8-fluoro-2-((1-(hydroxymethyl)cyclopropyl)methoxy)quinazolin-4-yl)-3-methylpiperidin-3-ol (120 mg, 272.54 µmol) and triisopropyl ((6-(methoxymethoxy)-8-(4,4,5,5-tetramethyl-1,3,2-dioxaborolan-2-yl)naphthalen-1-yl)ethynyl)silane (162 mg, 327.04 µmol) in 1,4-dioxane (8 mL) and water (2 mL) at room temperature. The mixture was stirred at 75 °C for 18 h under nitrogen atmosphere. After the reaction was completed, the mixture was poured into water (100 mL) and extracted with ethyl acetate (20 mL × 2). The combined organic phase was washed with saturated sodium chloride (100 mL), dried over anhydrous sodium sulfate, filtered and concentrated under reduced pressure. The resulting crude product was purified by silica gel thin-layer chromatography (dichloromethane:methanol = 10:1, 0.1% NH₃) to give (R)-1-(8-fluoro-2-((1-(hydroxymethyl)cyclopropyl)methoxy)-7-(3-(methoxymethoxy)-8-((triisopropylsilyl) ethynyl)naphthalen-1-yl)quinazolin-4-yl)-3-methylpiperidin-3-ol.

LC-MS: (ESI, m/z): [M+H]⁺ = 728.4.

### Step 2: preparation of (R)-1-(((8-fluoro-4-(3-hydroxy-3-methylpiperidin-1-yl)-7-(3-(methoxymethoxy)-8-((triisopropylsilyl)ethynyl)naphthalen-1-yl)quinazolin-2-yl)oxy)methyl)cyclopropane-1-carbaldehyde

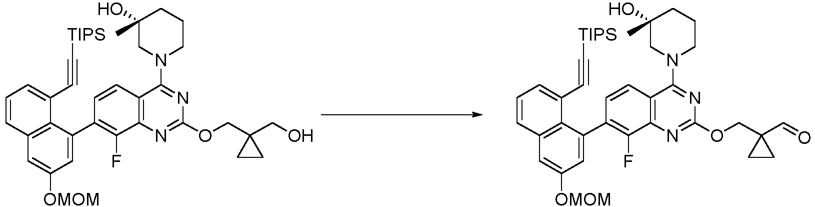

A solution of (*R*)-1-(8-fluoro-2-((1-(hydroxymethyl)cyclopropyl)methoxy)-7-(3-(methoxymethoxy)-8-((triisopropylsilyl)ethynyl)naphthalen-1-yl)quinazolin-4-yl)-3-methylpiperidin-3-ol (100 mg, 137.36 µmol) in dichloromethane (10 mL) was cooled to 0 °C from room temperature, and Dess-Martin reagent (76 mg, 178.57 µmol) was added. The mixture was stirred at 25 °C for 1 h. The reaction liquid was poured into a saturated aqueous sodium bicarbonate solution (100 mL), and extracted with dichloromethane (20 mL × 2). The combined organic phase was washed with saturated sodium chloride (100 mL), dried over anhydrous sodium sulfate, filtered and concentrated under reduced pressure. The resulting crude product was purified by silica gel thin-layer chromatography (petroleum ether:ethyl acetate = 1:1, 0.1% NH₃) to give (R)-1-(((8-fluoro-4-(3-hydroxy-3-methylpiperidin-1-yl)-7-(3-(methoxymethoxy)-8-((triisopropylsilyl)ethynyl)naphthalen-1-yl)quinazolin-2-yl)oxy)methyl)cyclopropane-1-carbaldehyde.

LC-MS: (ESI, m/z): [M+H]⁺ = 726.4.

### Step 3: preparation of (R)-1-(5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)quinazolin-2-yl)oxy)methyl)cyclopropyl) methyl)piperazin-1-yl)methyl)-3-azaspiro[5.5]undecane-3-carbonyl)-2-methoxyphenyl)dihydropyrimidine-2,4(1H,3H)-dione

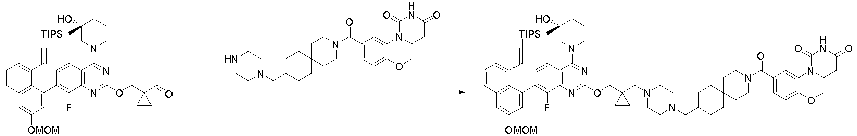

Tetraisopropyl titanate (156 mg, 547.55 µmol) was added to a solution of (*R*)-1-(((8-fluoro-4-(3-hydroxy-3-methylpiperidin-1-yl)-7-(3-(methoxymethoxy)-8-((triisopropylsilyl)ethynyl)naphthalen-1-yl)quinazolin-2-yl)oxy)methyl)cyclopropane-1-carbaldehyde (80 mg, 109.51 µmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (66 mg, 131.41 µmol) in dichloromethane (12 mL), and the mixture stirred at 25 °C for 16 h. Sodium triacetoxyborohydride (170 mg, 802.11 µmol) was added to the mixture at room temperature, and the mixture was then reacted at 25 °C for another 3 h. Methanol (10 mL) was added to the reaction liquid to quench the reaction, and the mixture was then concentrated under reduced pressure at 40 °C. The resulting crude product was purified by a preparative thin-layer chromatography plate (dichloromethane:methanol = 10:1, 0.1% NH₃) to give (*R*)-1-(5-(9-((4-((1-(((8-fluoro-4-(3-hydroxy-3-methylpiperidin-1-yl)-7-(3-(methoxymethoxy)-8-((triisopropylsilyl) ethynyl)naphthalen-1-yl)quinazolin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1*H*,3*H*)-dione.

LC-MS: (ESI, m/z): [M+H]⁺ = 1207.9.

### Step 4: preparation of (R)-1-(5-(9-((4-((1-(((7-(8-ethynyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

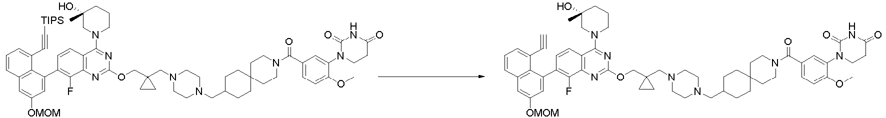

Cesium fluoride (145 mg, 953.90 µmol) was added to a solution of (*R*)-1-(5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione (115 mg, 95.39 µmol) in *N,N-*dimethylformamide (5 mL) at room temperature, and the mixture was reacted at room temperature for 18 h. The reaction liquid was poured into water (100 mL), and the mixture was extracted with ethyl acetate (20 mL × 2). The combined organic phase was washed with saturated sodium chloride (100 mL), dried over anhydrous sodium sulfate, filtered and concentrated under reduced pressure. (*R*)-1-(5-(9-((4-((1-(((7-(8-Ethynyl-3-(methoxymethoxy)naphthalen-1-yl)-8-fluoro-4-(3-hydroxy-3-methylpiperidin-1-yl)quinazolin-2-yl)oxy) methyl)cyclopropyl)methyl)piperazin-1-yl)methyl)-3-azaspiro[5.5]undecane-3-carbonyl)-2-methoxyphenyl) dihydropyrimidine-2,4(1*H*,3*H*)-dione was obtained.

LC-MS: (ESI, m/z): [M+H]⁺ = 1051.9.

### Step 5: preparation of (R)-1-(5-(9-((4-((1-(((7-(8-ethynyl-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1H,3H)-dione

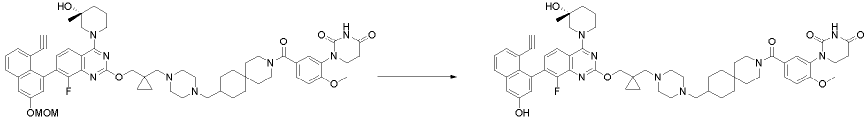

A hydrogen chloride/1,4-dioxane solution (4.0 M, 6 mL) was added to a solution of *(R)-1-(5-(9-*((4-((1-(((7-(8-ethynyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione (61 mg, 58.12 µmol) in 1,4-dioxane (3 mL) at room temperature, and the mixture was reacted at room temperature for 1 h. The reaction liquid was concentrated under reduced pressure at 30 °C, and a solution of ammonia in methanol (1 mL) was added to the residue. Then the mixture was concentrated under reduced pressure at 30 °C. The resulting crude product was purified by a preparative thin-layer chromatography plate (dichloromethane:methanol = 10:1, 0.1% NH₃) and then separated by preparative high-performance liquid chromatography to give (R)-1-(5-(9-((4-((1-(((7-(8-ethynyl-3-hydroxynaphthalen-1-yl)-8-fluoro-4-(3-hydroxy-3-methylpiperidin-1-yl)quinazolin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1H,3H)-dione.

LC-MS: (ESI, m/z): [M/2+H]⁺ = 504.3.

¹H NMR (400 MHz, DMSO-*d*₆) δ 10.33 (s, 1H), 10.1 (br, 1H), 7.90 - 7.72 (m, 2H), 7.45 - 7.38 (m, 2H), 7.38 - 7.35 (m, 1H), 7.32 - 7.30 (m, 2H), 7.20 - 7.17 (m, 1H), 7.14 (d, *J* = 8.0 Hz, 1H), 7.03 (dd,*J* = 11.6 Hz, 2.8 Hz, 1H), 4.64 (d, *J* = 28.0 Hz, 1H), 4.25 - 4.18 (m, 2H), 4.11 - 3.91 (m, 1H), 3.90 - 3.73 (m, 4H), 3.59 (t, *J* = 8.0 Hz, 2H), 3.50 - 3.35 (m, 4H), 3.28 - 3.20 (m, 2H), 2.69 - 2.66 (m, 2H), 2.45 -2.20 (m, 10H), 2.15 - 1.95 (m, 4H), 1.75 - 1.65 (m, 4H), 1.55 - 1.25 (m, 8H), 1.16 (d, *J* = 4.0 Hz, 3H), 1.05 - 0.95 (m, 4H), 0.65 - 0.58 (m, 2H), 0.40- 0.35 (m, 2H).

### Example 25: (R)-1-(5-(9-((4-((1-(((7-(8-ethyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of (R)-1-(5-(9-((4-((1-(((7-(8-ethyl-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

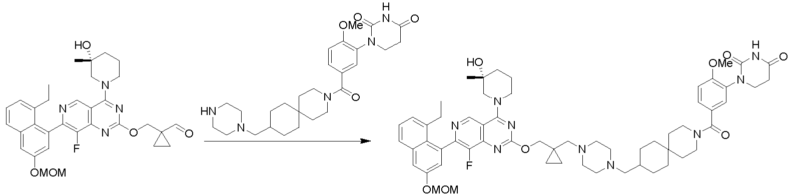

Tetraisopropyl titanate (198 mg, 0.70 mmol) was added to a stirred solution of (*R*)-1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde (80 mg, 0.14 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (83 mg, 0.17 mmol) in DCM/AcOH (2 mL/0.1 mL), and the mixture stirred at 30 °C for 2 h. NaBH(OAc)₃ (93 mg, 0.42 mmol) was added to the mixture at 0 °C, and the resulting mixture was stirred for 2 h. The mixture was concentrated and purified by silica-based column chromatography (DCM:MeOH = 100:1 to 20:1) to give (*R*)-1-(5-(9-((4-((1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1*H*,3*H*)-dione.

LC-MS: (ESI, m/z): [M/2+H]⁺ = 528.8.

### Step 2: preparation of (R)-1-(5-(9-((4-((1-(((7-(8-ethyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

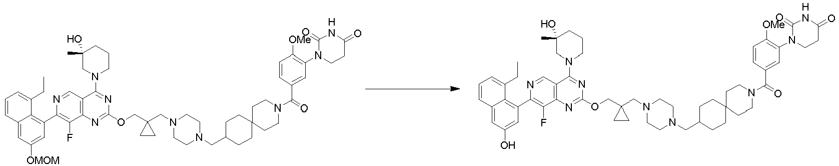

HCl/1,4-dioxane (6 N, 1.0 mL) was added to a stirred solution of (*R*)-1-(5-(9-((4-((1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione (80 mg, 0.077 mmol) in 1,4-dioxane (2.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give (*R*)-1-(5-(9-((4-((1-(((7-(8-ethyl-3-hydroxynaphthalen-1-yl)-8-fluoro-4-(3-hydroxy-3-methylpiperidin-1-yl)pyrido[4,3-*d*]pyrimidin-2-yl)oxy) methyl)cyclopropyl)methyl)piperazin-1-yl)methyl)-3-azaspiro[5.5]undecane-3-carbonyl)-2-methoxyphenyl)
dihydropyrimidine-2,4(1H,3H)-dione.

LC-MS: (ESI, m/z): [M/2+H]⁺ = 506.8.

¹H NMR (400 MHz, CD₃OD) δ 9.18 (s, 1H), 7.62 (d, *J* = 8.2 Hz, 1H), 7.43 (d, *J* = 8.4 Hz, 1H), 7.39 - 7.32 (m, 2H), 7.27 (d, *J* = 2.4 Hz, 1H), 7.19 - 7.14 (m, 2H), 7.01 (d, *J* = 2.3 Hz, 1H), 4.54 - 4.44 (m, 2H), 4.41 - 4.36 (m, 1H), 4.23 *(t, J = 12.3* Hz, 1H), 3.92 (s, 3H), 3.78 - 3.40 (m, 9H), 2.80 (t, *J =* 6.7 Hz, 2H), 2.66 - 2.21 (m, 12H), 2.18 - 2.08 (m, 3H), 1.89 - 1.72 (m, 5H), 1.65 - 1.31 (m, 8H), 1.28 (d, *J* = 11.5 Hz, 3H), 1.22 - 1.05 (m, 4H), 0.94 - 0.87(m, 3H), 0.74 - 0.66 (s, 2H), 0.54 - 0.44 (m, 2H).

### Example 26: (R)-1-(5-(9-((4-((1-(((7-(8-ethyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of (R)-1-(5-(9-((4-((1-(((7-(8-ethyl-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

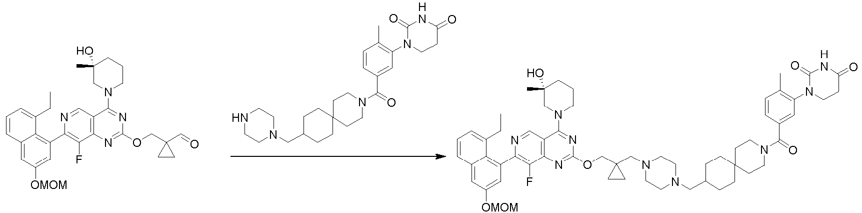

Tetraisopropyl titanate (247 mg, 0.87 mmol) was added to a stirred solution of (*R*)-1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde (100 mg, 0.17 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (101 mg, 0.21 mmol) in DCM/AcOH (2 mL/0.1 mL), and the mixture stirred at 30 °C for 2 h. NaBH(OAc)₃ (116 mg, 0.52 mmol) was added to the mixture at 0 °C, and the mixture was stirred for 2 h. The mixture was concentrated and purified by silica gel column chromatography (DCM/MeOH = 100:1 to 10:1) to give (R)-1-(5-(9-((4-((1-(((7-(8-ethyl-3-(methoxymethoxy)naphthalen-1-yl)-8-fluoro-4-(3-hydroxy-3-methylpiperidin-1-yl)pyrido [4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 520.6.

### Step 2: preparation of (R)-1-(5-(9-((4-((1-(((7-(8-ethyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

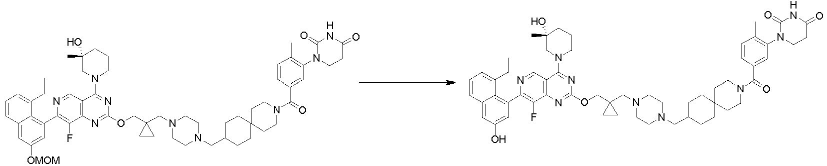

HCl/1,4-dioxane (6 N, 1.0 mL) was added to a stirred solution of (R)-1-(5-(9-((4-((1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione (100 mg, 0.096 mmol) in 1,4-dioxane (2.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give (R)-1-(5-(9-((4-((1-(((7-(8-ethyl-3-hydroxynaphthalen-1-yl)-8-fluoro-4-(3-hydroxy-3-methylpiperidin-1-yl)pyrido[4,3-*d*]pyrimidin-2-yl)oxy) methyl)cyclopropyl)methyl)piperazin-1-yl)methyl)-3-azaspiro[5.5]undecane-3-carbonyl)-2-methylphenyl) dihydropyrimidine-2,4(1H,3H)-dione.

LC-MS: (ESI, m/z): [M/2+H]⁺ = 498.8.

¹H NMR (400 MHz, DMSO-*d*₆) δ 10.36 (s, 1H), 9.88 (s, 1H), 9.20 (d, *J* = 6.2 Hz, 1H), 7.66 (d, *J* = 8.1 Hz, 1H), 7.40 - 7.20 (m, 5H), 7.12 (d, *J =* 6.9 Hz, 1H), 6.97 (d, *J =* 2.5 Hz, 1H), 4.73 (d, *J =* 12.3 Hz, 1H), 4.37 - 4.22 (m, 3H), 4.08 - 3.97 (m, 1H), 3.85 - 3.72 (m, 1H), 3.68 - 3.45 (m, 4H), 3.44 - 3.33 (m, 2H), 2.82 - 2.66 (m, 2H), 2.47 - 2.10 (m, 15H), 2.07 - 1.95 (m, 3H), 1.76 - 1.60 (m, 5H), 1.54 - 1.22 (m, 8H), 1.17 (d, *J =* 11.0 Hz, 3H), 1.10 - 0.92 (m, 4H), 0.87 - 0.78 (m, 3H), 0.67 - 0.58 (m, 2H), 0.45 - 0.35 (m, 2H).

### Example 27: (R)-1-(5-(9-((4-((1-(((7-(8-ethynyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of (R)-1-(5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-d]pyrimidin-2-yl)oxy)methyl) cyclopropyl)methyl)piperazin-1-yl)methyl)-3-azaspiro[5.5]undecane-3-carbonyl)-2-methylphenyl) dihydropyrimidine-2,4(1H,3H)-dione

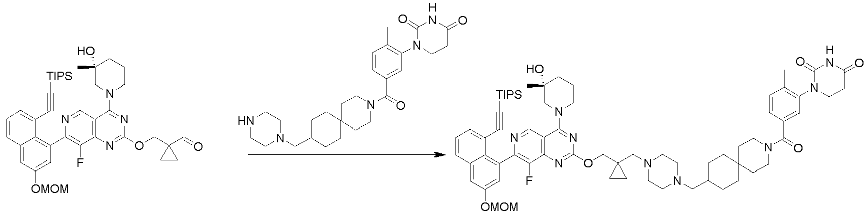

Tetraisopropyl titanate (199 mg, 0.7 mmol) was added to a stirred solution of (*R*)-1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-*d*]pyrimidin-2-yl)oxy)methyl)cyclopropane-1-carbaldehyde (100 mg, 0.14 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (82 mg, 0.17 mmol) in DCM (2 mL), and the mixture was stirred at 30 °C for 2 h. NaBH(OAc)₃ (93 mg, 0.42 mmol) was added to the mixture at room temperature, and the mixture was stirred for 2 h. The mixture was concentrated and purified by silica gel column chromatography (DCM:MeOH = 100:1 to 10:1) to give *(R)-1-(5-(9-((4-((1-(((8-fluoro-4-(3-hydroxy-3-methylpiperidin-1-yl)-7-(3-*(methoxymethoxy)-8-((triisopropylsilyl) ethynyl)naphthalen-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 597.0.

### Step 2: preparation of (R)-1-(5-(9-((4-((1-(((7-(8-ethynyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

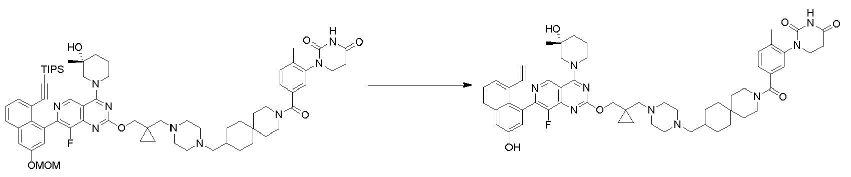

Cesium fluoride (64 mg, 0.42 mmol) was added to a stirred solution of (*R*)-1-(5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1*H*,3*H*)-dione (100 mg, 0.084 mmol) in DMF (2 mL), and the mixture was then stirred at room temperature for 30 min. The mixture was extracted with ethyl acetate and water. The organic phase was concentrated. A mixture of 1,4-dioxane (1 mL) and HCl/1,4-dioxane (6 N, 0.5 mL) was then added to the resulting mixture at room temperature. The mixture was reacted for 0.5 h. The mixture was purified by preparative high-performance liquid chromatography to give (*R*)-1-(5-(9-((4-((1-(((7-(8-ethynyl-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 496.7.

¹H NMR (400 MHz, DMSO-*d*₆) δ 10.37 (s, 1H), 10.14 (s, 1H), 9.11 (d, *J* = 67.7 Hz, 1H), 7.88 (dd, *J=* 8.0, 1.5 Hz, 1H), 7.48 - 7.38 (m, 2H), 7.36 - 7.28 (m, 3H), 7.26 - 7.21 (m, 1H), 7.14 (dd, *J =* 17.0, 2.5 Hz, 1H), 4.74 *(d, J = 37.8* Hz, 1H), 4.42 - 4.17 (m, 3H), 4.02 (dd, *J =* 54.2, 13.7 Hz, 1H), 3.85 - 3.73 (m, 1H), 3.64 - 3.41 (m, 5H), 3.29 - 3.20 (m, 2H), 2.83 - 2.64 (m, 2H), 2.46 - 2.23 (m, 9H), 2.21 (s, 3H), 2.11 - 1.92 (m, 4H), 1.72 - 1.59 (m, 5H), 1.53 - 1.22 (m, 8H), 1.16 (d, *J* = 17.5 Hz, 3H), 1.09-0.92 (m, 4H), 0.67 - 0.59 (m, 2H), 0.44 - 0.36 (m, 2H).

### Example 28: (R)-1-(5-(9-((4-((1-(((7-(8-ethyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-fluorophenyl)dihydropyrimidine-2,4(1H,3H)-dione

### Step 1: preparation of 1-(5-(9-((4-benzylpiperazin-1-yl)methyl)-3-azaspiro[5.5]undecane-3-carbonyl)-2-fluorophenyl)dihydropyrimidine-2,4(1H,3H)-dione

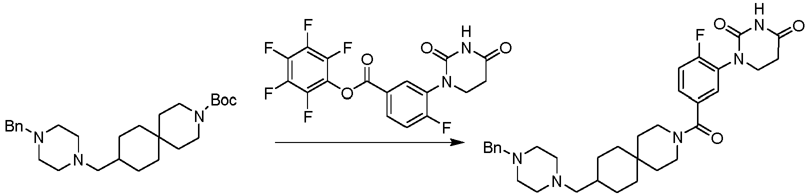

TFA (2.0 mL) was added to a solution of *tert-butyl* 9-((4-benzylpiperazin-1-yl)methyl)-3-azaspiro[5.5]undecane-3-carboxylate (200 mg, 0.45 mmol) in DCM (4.0 mL), and the mixture was stirred at room temperature for 1 h. The resulting solution was concentrated under reduced pressure to give a yellow oil (150 mg), which was dissolved in DMF (4 mL). Pentafluorophenyl 3-(2,4-dioxotetrahydropyrimidin-1(2*H*)-yl)-4-fluorobenzoate (189 mg, 0.45 mmol) and DIEA (467 mg, 3.62 mmol) were then added. The reaction mixture was stirred at room temperature for another 1 h. Water (10 mL) was added to quench the reaction, and the mixture was extracted with ethyl acetate (20 mL × 3). The organic phases were combined, washed with saturated brine, dried over anhydrous sodium sulfate, and concentrated under reduced pressure. The resulting concentrate was purified by silica gel column chromatography (DCM:MeOH = 10:1) to give 1-(5-(9-((4-benzylpiperazin-1-yl)methyl)-3-azaspiro[5.5]undecane-3-carbonyl)-2-fluorophenyl)dihydropyrimidine-2,4(1*H*,3*H*)-dione.

LC-MS: (ESI, m/z): [M+H]⁺ = 576.3.

### Step 2: preparation of 1-(2-fluoro-5-(9-(piperazin-1-ylmethyl)-3-azaspiro[5.5]undecane-3-carbonyl)phenyl) dihydropyrimidine-2,4(1H,3H)-dione

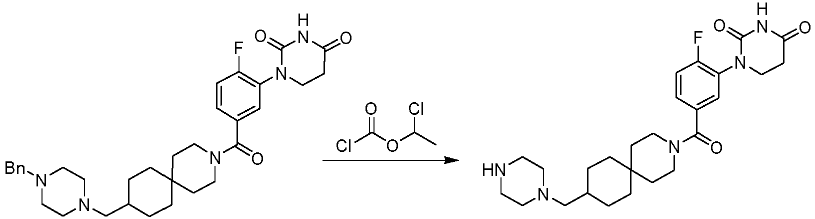

1-(5-(9-((4-Benzylpiperazin-1-yl)methyl)-3-azaspiro[5.5]undecane-3-carbonyl)-2-fluorophenyl) dihydropyrimidine-2,4(1*H*,3*H*)-dione (220 mg, 0.38 mmol) was dissolved in DCM (5 mL), and then methyl 1-chloroethyl chloroformate (164 mg, 1.45 mmol) and DIEA (99 mg, 0.76 mmol) were added. The mixture was reacted at room temperature for 1 h and concentrated under reduced pressure. MeOH (5 mL) was added to the resulting concentrate, and the mixture was then heated to 50 °C and reacted for 30 min. After the reaction was completed, water (10 mL) was added to quench the reaction, and the mixture was extracted with ethyl acetate (30 mL × 3). The organic phases were combined, dried over anhydrous sodium sulfate, and concentrated under reduced pressure. The concentrate was purified by silica gel column chromatography (DCM:MeOH = 10:1) to give 1-(2-fluoro-5-(9-(piperazin-1-ylmethyl)-3-azaspiro[5.5]undecane-3-carbonyl)phenyl)dihydropyrimidine-2,4(1*H*,3*H*)-dione.

LC-MS: (ESI, m/z): [M+H]⁺ = 486.2.

### Step 3: preparation of (R)-1-(5-(9-((4-((1-(((7-(8-ethyl-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-fluorophenyl)dihydropyrimidine-2,4(1H,3H)-dione

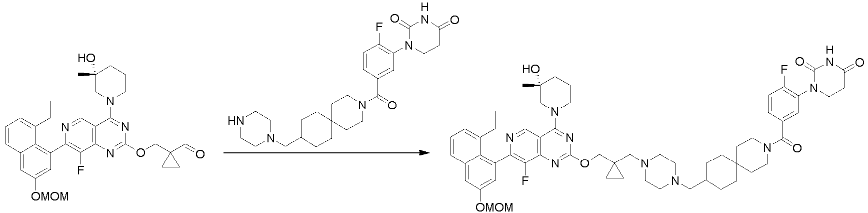

Tetraisopropyl titanate (247 mg, 0.87 mmol) was added to a stirred solution of (*R*)-1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde (100 mg, 0.17 mmol) and 1-(2-fluoro-5-(9-(piperazin-1-ylmethyl)-3-azaspiro[5.5]undecane-3-carbonyl)phenyl)dihydropyrimidine-2,4(1*H*,3*H*)-dione (101 mg, 0.21 mmol) in DCM (2 mL), and the mixture stirred at 25 °C for 2 h. NaBH(OAc)₃ (116 mg, 0.52 mmol) was added to the mixture at 0 °C, and the mixture was stirred for 2 h. The mixture was concentrated and purified by silica gel column chromatography (DCM:MeOH = 100:1 to 10:1) to give *(R)-1-(5-(9-((4-*((1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-fluorophenyl)dihydropyrimidine-2,4(1*H*,3*H*)-dione.

LC-MS: (ESI, m/z): [M/2+H]⁺ = 522.6.

### Step 4: preparation of (R)-1-(5-(9-((4-((1-(((7-(8-ethyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-fluorophenyl)dihydropyrimidine-2,4(1H,3H)-dione

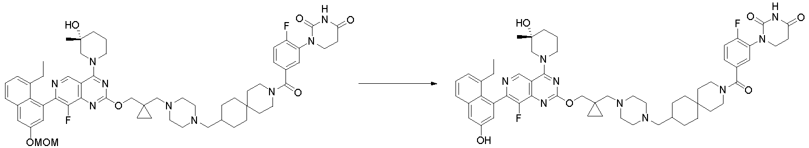

HCl/1,4-dioxane (6 N, 1.0 mL) was added to a stirred solution of (*R*)-1-(5-(9-((4-((1-(((7-(8-ethyl-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-fluorophenyl)dihydropyrimidine-2,4(1*H*,3*H*)-dione (80 mg, 0.077 mmol) in 1,4-dioxane (2.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give (R)-1-(5-(9-((4-((1-(((7-(8-ethyl-3-hydroxynaphthalen-1-yl)-8-fluoro-4-(3-hydroxy-3-methylpiperidin-1-yl)pyrido[4,3-*d*]pyrimidin-2-yl)oxy) methyl)cyclopropyl)methyl)piperazin-1-yl)methyl)-3-azaspiro[5.5]undecane-3-carbonyl)-2-fluorophenyl) dihydropyrimidine-2,4(1H,3H)-dione.

LC-MS: (ESI, m/z): [M/2+H]⁺ = 500.8.

¹H NMR (400 MHz, DMSO-*d*₆) δ 10.53 (s, 1H), 9.89 (s, 1H), 9.20 (d, *J* = 6.5 Hz, 1H), 7.67 (d, *J* = 8.0 Hz, 1H), 7.49 (d, *J* = 8.0 Hz, 1H), 7.40 - 7.32(m, 3H), 7.28 (d, *J =* 2.5 Hz, 1H), 7.12 (d, *J* = 7.0 Hz, 1H), 6.97 (d, *J =* 2.5 Hz, 1H), 4.74 (d, *J =* 13.0 Hz, 1H), 4.36 - 4.23 (m, 3H), 4.09 - 3.96 (m, 1H), 3.75 (t, *J* = 6.6 Hz, 2H), 3.68 - 3.33 (m, 5H), 2.72 (t, *J =* 6.6 Hz, 2H), 2.48 - 2.09(m, 12H), 2.08 - 1.98 (s, 3H), 1.75 - 1.60 (m, 5H), 1.56 - 1.22 (m, 8H), 1.17 (d, *J* = 11.1 Hz, 3H), 0.87 - 0.77 (m, 4H), 0.87 - 0.77 (m, 3H), 0.68 - 0.59 (s, 2H), 0.44 - 0.35(s, 2H).

### Example 29: (R)-1-(5-(9-((4-((1-(((7-(8-ethynyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of (R)-1-(5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-d]pyrimidin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1H,3H)-dione

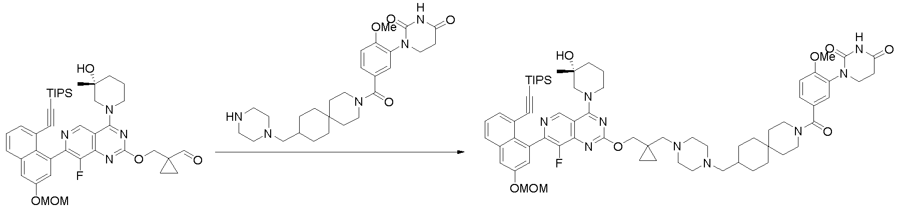

Tetraisopropyl titanate (0.5 mL) was added to a stirred solution of (*R*)-1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-*d*]pyrimidin-2-yl)oxy)methyl)cyclopropane-1-carbaldehyde (150 mg, 0.21 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (154 mg, 0.31 mmol) in DCM/AcOH (1.5 mL/0.1 mL), and the mixture was stirred at 30 °C for 2 h. NaBH(OAc)₃ (131 mg, 0.62 mmol) was added to the mixture in an ice bath, and the resulting mixture was stirred for 2 h. The mixture was concentrated and purified by silica gel column chromatography (DCM:MeOH = 100:1 to 10:1) to give (*R*)-1-(5-(9-((4-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-*d*]pyrimidin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1*H*,3*H*)-dione.

LC-MS: (ESI, m/z): [M/2+H]⁺ = 604.6.

### Step 2: preparation of (R)-1-(5-(9-((4-((1-(((7-(8-ethynyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

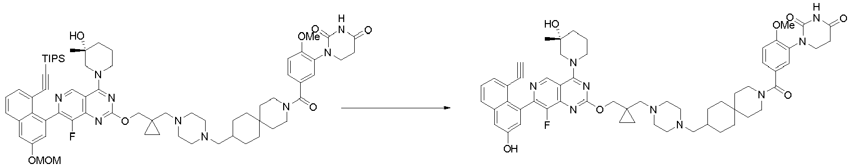

Cesium fluoride (118 mg, 0.78 mmol) was added to a stirred solution of *(R)-1-(5-(9-((4-((1-(((8-*fluoro-4-(3-hydroxy-3-methylpiperidin-1-yl)-7-(3-(methoxymethoxy)-8-((triisopropylsilyl)ethynyl)naphthalen-1-yl)pyrido [4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione (188 mg, 0.16 mmol) in DMF (1 mL), and the mixture was stirred at room temperature for 30 min. Water (10 mL) was added to the reaction liquid to quench the reaction, and the mixture was extracted with ethyl acetate (10 mL × 3). The combined organic phase was dried over anhydrous sodium sulfate and concentrated under reduced pressure. HCl/1,4-dioxane (6 N, 1.0 mL) was added to a stirred solution of the concentrate in 1,4-dioxane (0.5 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give (*R*)-1-(5-(9-((4-((1-(((7-(8-ethynyl-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 504.7.

¹H NMR (400 MHz, DMSO-*d*₆) δ 10.33 (s, 1H), 10.14 (d, *J* = 3.9 Hz, 1H), 9.12 (d, *J* = 67.6 Hz, 1H), 7.88 (d, *J* = 6.6 Hz, 1H), 7.47 - 7.39 (m, 2H), 7.38 - 7.33 (m, 2H), 7.31 (d, *J* = 2.0 Hz, 1H), 7.18 - 7.15 (m, 1H), 7.14 - 7.11 (m, 1H), 4.74 (d, *J* = 37.6 Hz, 1H), 4.39 - 4.18 (m, 3H), 3.84 (s, 3H), 3.64 - 3.55 (m, 4H), 3.31 - 3.22 (m, 2H), 2.72 - 2.63 (m, 2H), 2.45 - 2.20 (m, 10H), 2.12 - 1.90 (m, 4H), 1.75 - 1.61 (m, 5H), 1.60 - 1.36 (m, 6H), 1.36 - 1.20 (m, 3H), 1.16 (d, *J* = 17.4 Hz, 3H), 1.13 - 0.89 (m, 5H), 0.67 - 0.59 (m, 2H), 0.43 - 0.35 (m, 2H).

### Example 30: 1-(5-(9-((4-((1-(((4-(cyclopropyl(methyl)amino)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro [5.5]undecane-3-carbonyl)-2-methoxyphenyl)dihydropyrimidine-2,4(1H,3H)-dione

### Step 1: preparation of 2,7-dichloro-N-cyclopropyl-8-fluoro-N-methylpyrido[4,3-d]pyrimidine-4-amine

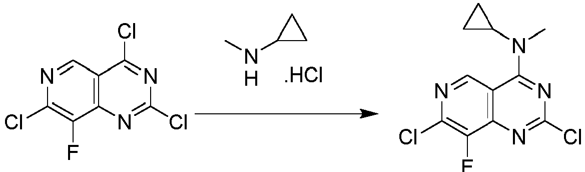

TEA (606 mg, 6.0 mmol) was added to a solution of 2,4,7-trichloro-8-fluoropyrido[4,3-*d*]pyrimidine (500 mg, 2.0 mmol) in THF (5 mL) at -78 °C, and the mixture was stirred for 30 min. N-Methylcyclopropylamine hydrochloride (214 mg, 2.0 mmol) was added, and the mixture was stirred for 4 h. After the reaction was completed, the mixture was naturally warmed to room temperature, H₂O (10 mL) was added, and the mixture was extracted with EtOAc (10 mL × 3). The combined organic phase was dried over anhydrous sodium sulfate and concentrated under reduced pressure, and the concentrate was purified by silica gel column chromatography (PE:EtOAc = 5:1) to give 2,7-dichloro-*N*-cyclopropyl-8-fluoro-N-methylpyrido[4,3-*d*]pyrimidine-4-amine.

LC-MS: (ESI, m/z): [M+H]⁺ = 287.0.

### Step 2: preparation of (1-(((7-chloro-4-(cyclopropyl(methyl)amino)-8-fluoropyrido[4,3-d]pyrimidin-2-yl)oxy)methyl)cyclopropyl)methanol

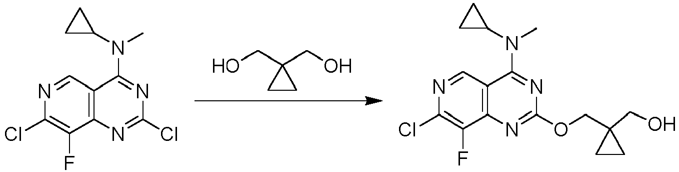

Cs₂CO₃ (1.3 g, 4.2 mmol) and DABCO (78.4 mg, 0.7 mmol) were added to a solution of 2,7-dichloro-*N*-cyclopropyl-8-fluoro-*N*-methylpyrido[4,3-*d*]pyrimidine-4-amine (400 mg, 1.4 mmol) and cyclopropane-1,1-diyldimethanol (286 mg, 2.8 mmol) in acetonitrile (5 mL), and the mixture was stirred at room temperature for 30 min. After the reaction was completed, water (10 mL) was added, and the mixture was extracted with ethyl acetate (10 mL × 3). The combined organic phase was dried over anhydrous sodium sulfate and concentrated under reduced pressure. The concentrate was purified by silica gel column chromatography (DCM:MeOH = 20:1) to give (1-(((7-chloro-4-(cyclopropyl(methyl)amino)-8-fluoropyrido[4,3-*d*]pyrimidin-2-yl)oxy)methyl)cyclopropyl)methanol.

LC-MS: (ESI, m/z): [M+H]⁺ = 353.2.

### Step 3: preparation of (1-(((4-(cyclopropyl(methyl)amino)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropyl)methanol

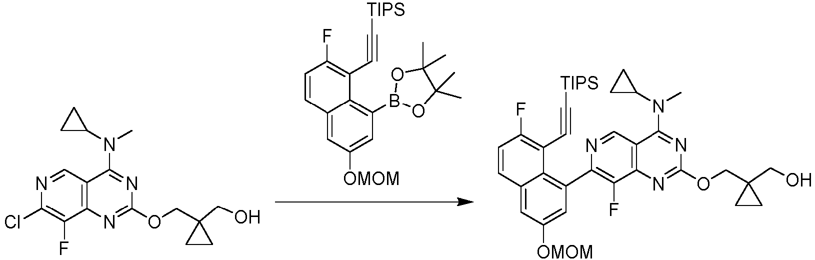

((2-Fluoro-6-(methoxymethoxy)-8-(4,4,5,5-tetramethyl-1,3,2-dioxaborolan-2-yl)naphthalen-1-yl)ethynyl)
triisopropylsilane (430 mg, 0.84 mmol), cataCXium A Pd G3 (81 mg, 0.11 mmol), and cesium carbonate (547 mg, 1.68 mmol) were added to a mixed solution of (1-(((7-chloro-4-(cyclopropyl(methyl)amino)-8-fluoropyrido[4,3-*d*]pyrimidin-2-yl)oxy)methyl)cyclopropyl)methanol (200 mg, 0.56 mmol) in 1,4-dioxane (6 mL) and H₂O (1 mL). The mixture was reacted at 85 °C for 16 h under nitrogen atmosphere. After the reaction was completed, water (10 mL) was added to the reaction liquid, and the mixture was extracted with ethyl acetate (10 mL × 3). The combined organic phase was dried over anhydrous sodium sulfate and concentrated under reduced pressure. The concentrate was purified by silica gel column chromatography (DCM:MeOH = 10:1) to give (1-(((4-(cyclopropyl(methyl)amino)-8-fluoro-7-(7-fluoro-3-(methoxymethoxy)-8-((triisopropylsilyl) ethynyl)naphthalen-1-yl)pyrido[4,3-*d*]pyrimidin-2-yl)oxy)methyl)cyclopropyl)methanol.

LC-MS: (ESI, m/z): [M+H]⁺ = 703.3.

### Step 4: preparation of 1-(((4-(cyclopropyl(methyl)amino)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropane-1-carbaldehyde

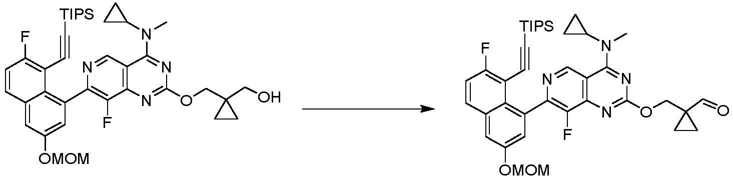

A solution of (1-(((4-(cyclopropyl(methyl)amino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methanol (200 mg, 0.28 mmol) in DCM (5 mL) was cooled to 0 °C, and Dess-Martin reagent (237 mg, 0.56 mmol) was added. The mixture was reacted at room temperature for 1 h. After the reaction was completed, a sodium thiosulfate solution (5 mL) and a sodium bicarbonate solution (5 mL) were added to quench the reaction, and the mixture was extracted with dichloromethane (5 mL × 3). The combined organic phase was dried over anhydrous sodium sulfate and concentrated under reduced pressure. The concentrate was purified by silica gel column chromatography (DCM:MeOH = 20:1) to give 1-(((4-(cyclopropyl(methyl)amino)-8-fluoro-7-(7-fluoro-3-(methoxymethoxy)-8-((triisopropylsilyl)ethynyl)naphthalen-1-yl)pyrido [4,3-*d*]pyrimidin-2-yl)oxy)
methyl)cyclopropane-1-carbaldehyde.

LC-MS: (ESI, m/z): [M+H]⁺ = 701.2.

### Step 5: preparation of 1-(5-(9-((4-((1-(((4-(cyclopropyl(methyl)amino)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1H,3H)-dione

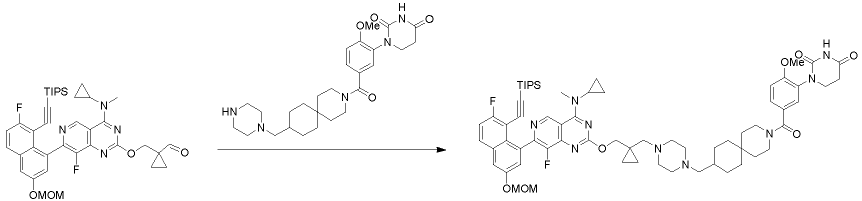

1-(2-Methoxy-5-(9-(piperazin-1-ylmethyl)-3-azaspiro[5.5]undecane-3-carbonyl)phenyl)dihydropyrimidine-2,4(1*H*,3*H*)-dione (149 mg, 0.3 mmol), AcOH (0.1 mL), and tetraisopropyl titanate (284 mg, 1.0 mmol) were added to a solution of 1-(((4-(cyclopropyl(methyl)amino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropane-1-carbaldehyde (140 mg, 0.2 mmol) in DCM (2 mL), and the mixture was stirred at room temperature for 2 h. The reaction liquid was cooled to 0 °C, and NaBH(OAc)₃ (127 mg, 0.6 mmol) was added. The mixture was reacted at room temperature for another 4 h. The mixture was concentrated and purified by silica gel column chromatography (DCM:MeOH = 100:1 to 20:1) to give 1-(5-(9-((4-((1-(((4-(cyclopropyl(methyl)amino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 591.9.

### Step 6: preparation of 1-(5-(9-((4-((1-(((4-(cyclopropyl(methyl)amino)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

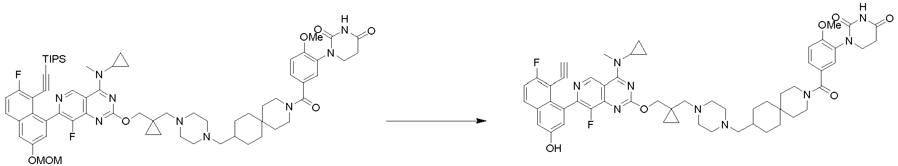

CsF (76 mg, 0.50 mmol) was added to a solution of 1-(5-(9-((4-((1-(((4-(cyclopropyl(methyl)amino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)
dihydropyrimidine-2,4(1*H*,3*H*)-dione (115 mg, 0.10 mmol) in DMF (2 mL), and the mixture was stirred at room temperature for half an hour. Water (10 mL × 3) was added to wash off DMF, and the aqueous phase was extracted with ethyl acetate (10 mL × 3). The combined organic phase was dried over anhydrous sodium sulfate and concentrated under reduced pressure. The concentrate was dissolved in 1,4-dioxane (2 mL), and then HCl/1,4-dioxane (6 N, 1 mL) was added. The mixture was reacted at room temperature for 0.5 h. The reaction liquid was concentrated and purified by preparative high-performance liquid chromatography to give 1-(5-(9-((4-((1-(((4-(cyclopropyl(methyl)amino)-7-(8-ethynyl-7-fluoro-3-hydroxynaphthalen-1-yl)-8-fluoropyrido [4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione.

LC-MS: (ESI, m/z): [M/+H]⁺ = 982.2.

¹H NMR (400 MHz, CD₃OD) δ 9.57 (s, 1H), 7.85 (dd, *J* = 9.1, 5.7 Hz, 1H), 7.43 (dd, *J* = 8.5, 2.1 Hz, 1H), 7.38 (d, *J =* 2.0 Hz, 1H), 7.36 - 7.28 (m, 2H), 7.24 - 7.14 (m, 2H), 4.50 (d, *J* = 11.0 Hz, 1H), 4.33 (*d, J =* 11.0 Hz, 1H), 3.92 (s, 3H), 3.77 - 3.61 (m, 4H), 3.59 - 3.55 (m, 1H), 3.49 - 3.44 (m, 4H), 3.42 (s, 1H), 2.80 (t, *J=* 6.7 Hz, 2H), 2.69 - 2.31 (m, 9H), 2.21 - 2.07 (m, 2H), 1.80 -1.69 (m, 2H), 1.65 - 1.55 (m, 3H), 1.54 - 1.23 (m, 5H), 1.26 - 0.96 (m, 7H), 0.94 - 0.83 (m, 2H), 0.73 - 0.61 (m, 2H), 0.54 - 0.39 (m, 2H).

### Example 31: 1-(5-(9-((4-((1-(((4-(cyclopropylamino)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1H,3H)-dione

### Step 1: preparation of 2,7-dichloro-N-cyclopropyl-8-fluoropyrido[4,3-d]pyrimidine-4-amine

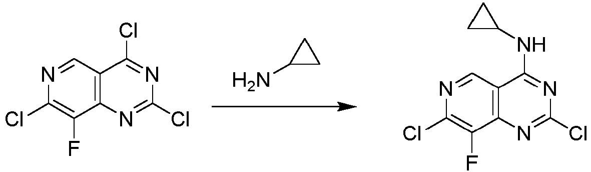

TEA (606 mg, 6.0 mmol) was added to a solution of 2,4,7-trichloro-8-fluoropyrido[4,3-*d*]pyrimidine (500 mg, 2.0 mmol) in THF (5 mL) at -78 °C, and the mixture was stirred for 30 min. Cyclopropylamine (1.6 g, 0.011 mmol) was added, and the mixture was stirred for 4 h. After the reaction was completed, the mixture was naturally warmed to room temperature, H₂O (10 mL) was added, and the mixture was extracted with EtOAc (10 mL × 3). The combined organic phase was dried over anhydrous sodium sulfate and concentrated under reduced pressure, and the concentrate was purified by silica gel column chromatography (PE:EtOAc = 5:1) to give 2,7-dichloro-*N*-cyclopropyl-8-fluoropyrido[4,3-*d*]pyrimidine-4-amine.

LC-MS: (ESI, m/z): [M+H]⁺ = 273.0.

### Step 2: preparation of (1-(((7-chloro-4-(cyclopropylamino)-8-fluoropyrido[4,3-d]pyrimidin-2-yl)oxy)methyl) cyclopropyl)methanol

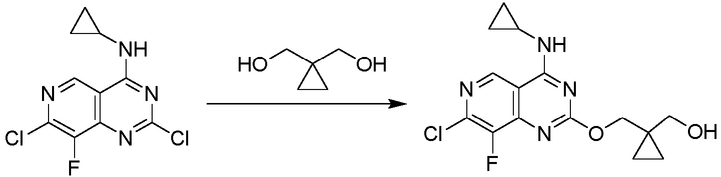

Cs₂CO₃ (1.3 g, 4.2 mmol) and DABCO (78.4 mg, 0.7 mmol) were added to a solution of 2,7-dichloro-*N*-cyclopropyl-8-fluoropyrido[4,3-*d*]pyrimidine-4-amine (380 mg, 1.4 mmol) and cyclopropane-1,1-diyldimethanol (286 mg, 2.8 mmol) in acetonitrile (5 mL), and the mixture was stirred at room temperature for 30 min. After the reaction was completed, water (10 mL) was added, and the mixture was extracted with ethyl acetate (10 mL × 3). The combined organic phase was dried over anhydrous sodium sulfate and concentrated under reduced pressure. The concentrate was purified by silica gel column chromatography (DCM:MeOH = 20:1) to give (1-(((7-chloro-4-(cyclopropylamino)-8-fluoropyrido[4,3-*d*]pyrimidin-2-yl)oxy)methyl)cyclopropyl)methanol.

LC-MS: (ESI, m/z): [M+H]⁺ = 339.1.

### Step 3: preparation of (1-(((4-(cyclopropylamino)-8-fluoro-7-(7-fluoro-3-(methoxymethoxy)-8-((triisopropylsilyl) ethynyl)naphthalen-1-yl)pyrido[4,3-d]pyrimidin-2-yl)oxy)methyl)cyclopropyl)methanol

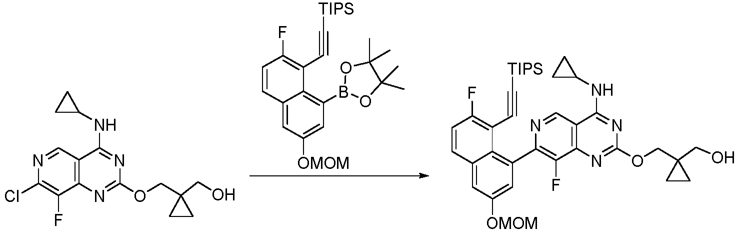

((2-Fluoro-6-(methoxymethoxy)-8-(4,4,5,5-tetramethyl-1,3,2-dioxaborolan-2-yl)naphthalen-1-yl)ethynyl)
triisopropylsilane (184 mg, 0.36 mmol), cataCXium A Pd G3 (35 mg, 0.048 mmol), and cesium carbonate (235 mg, 0.72 mmol) were added to a mixed solution of (1-(((7-chloro-4-(cyclopropylamino)-8-fluoropyrido[4,3-*d*]pyrimidin-2-yl)oxy)methyl)cyclopropyl)methanol (80 mg, 0.24 mmol) in 1,4-dioxane (3 mL) and H₂O (0.5 mL). The mixture was reacted at 85 °C for 16 h under nitrogen atmosphere. After the reaction was completed, water (10 mL) was added, and the mixture was extracted with ethyl acetate (10 mL × 3). The combined organic phase was dried over anhydrous sodium sulfate and concentrated under reduced pressure. The concentrate was purified by silica gel column chromatography (DCM:MeOH = 10:1) to give (1-(((4-(cyclopropylamino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methanol.

LC-MS: (ESI, m/z): [M+H]⁺ = 689.3.

### Step 4: preparation of 1-(((4-(cyclopropylamino)-8-fluoro-7-(7-fluoro-3-(methoxymethoxy)-8-((triisopropylsilyl) ethynyl)naphthalen-1-yl)pyrido[4,3-d]pyrimidin-2-yl)oxy)methyl)cyclopropane-1-carbaldehyde

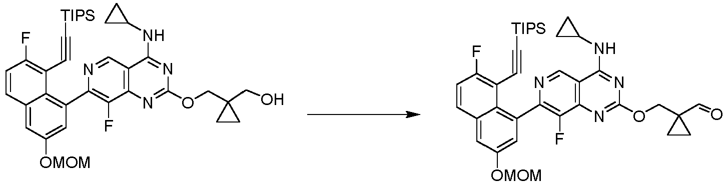

A solution of (1-(((4-(cyclopropylamino)-8-fluoro-7-(7-fluoro-3-(methoxymethoxy)-8-((triisopropylsilyl) ethynyl)naphthalen-1-yl)pyrido[4,3-*d*]pyrimidin-2-yl)oxy)methyl)cyclopropyl)methanol (50 mg, 0.072 mmol) in DCM (2 mL) was cooled to 0 °C, and Dess-Martin reagent (59 mg, 0.14 mmol) was added. The mixture was reacted at room temperature for 1 h. After the reaction was completed, a sodium thiosulfate solution (5 mL) and a sodium bicarbonate solution (5 mL) were added to quench the reaction, and the mixture was then extracted with dichloromethane (5 mL × 3). The combined organic phase was dried over anhydrous sodium sulfate and concentrated under reduced pressure. The concentrate was purified by silica gel column chromatography (DCM:MeOH = 30:1) to give 1-(((4-(cyclopropylamino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl) cyclopropane-1-carbaldehyde.

LC-MS: (ESI, m/z): [M+H]⁺ = 687.2.

### Step 5: preparation of 1-(5-(9-((4-((1-(((4-(cyclopropylamino)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1H,3H)-dione

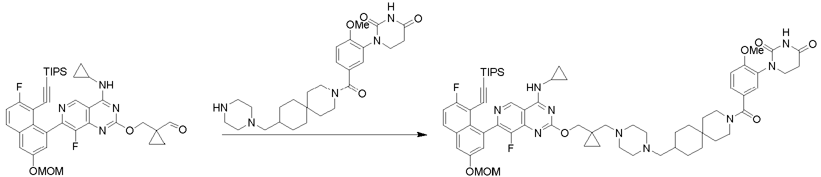

1-(2-Methoxy-5-(9-(piperazin-1-ylmethyl)-3-azaspiro[5.5]undecane-3-carbonyl)phenyl)dihydropyrimidine-2,4(1*H*,3*H*)-dione (30 mg, 0.06 mmol), AcOH (0.1 mL), and tetraisopropyl titanate (57 mg, 0.2 mmol) were added to a solution of 1-(((4-(cyclopropylamino)-8-fluoro-7-(7-fluoro-3-(methoxymethoxy)-8-((triisopropylsilyl) ethynyl)naphthalen-1-yl)pyrido[4,3-*d*]pyrimidin-2-yl)oxy)methyl)cyclopropane-1-carbaldehyde (30 mg, 0.04 mmol) in DCM (1.5 mL), and the mixture was stirred at room temperature for 2 h. The reaction liquid was cooled to 0 °C, and NaBH(OAc)₃ (25 mg, 0.12 mmol) was added. The mixture was reacted at room temperature for another 4 h. The mixture was concentrated and purified by silica gel column chromatography (DCM:MeOH = 100:1 to 20:1) to give 1-(5-(9-((4-((1-(((4-(cyclopropylamino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1168.7.

### Step 6: preparation of 1-(5-(9-((4-((1-(((4-(cyclopropylamino)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro [5.5]undecane-3-carbonyl)-2-methoxyphenyl)dihydropyrimidine-2,4(1H,3H)-dione

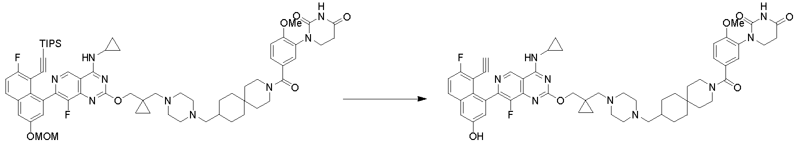

CsF (30 mg, 0.20 mmol) was added to a solution of 1-(5-(9-((4-((1-(((4-(cyclopropylamino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy) methyl)cyclopropyl)methyl)piperazin-1-yl)methyl)-3-azaspiro[5.5]undecane-3-carbonyl)-2-methoxyphenyl)
dihydropyrimidine-2,4(1*H*,3*H*)-dione (50 mg, 0.04 mmol) in DMF (2 mL), and the mixture was stirred at room temperature for half an hour. Water (10 mL × 3) was added to wash off DMF, and the aqueous phase was extracted with ethyl acetate (10 mL × 3). The combined organic phase was dried over anhydrous sodium sulfate and concentrated under reduced pressure. The concentrate was dissolved in dioxane (2 mL), and HCl/dioxane (6 N, 1 mL) was added. The mixture was reacted at room temperature for 0.5 h. The reaction liquid was concentrated and purified by preparative high-performance liquid chromatography to give 1-(5-(9-((4-((1-(((4-(cyclopropylamino)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 968.2.

¹H NMR (400 MHz, CD₃OD) δ 9.07 (s, 1H), 7.85 (dd, *J =* 9.0, 5.8 Hz, 1H), 7.43 (d, *J* = 8.5 Hz, 1H), 7.38 (d, *J =* 2.0 Hz, 1H), 7.35 -7.25 (m, 2H), 7.22 - 7.12 (m, 2H), 4.58 (d, *J* = 11.1 Hz, 1H), 4.41 (d, *J* = 11.2 Hz, 1H), 3.92 (s, 3H), 3.76 - 3.60 (m, 4H), 3.53 - 3.42 (m, 2H), 3.38 (s, 1H), 3.17 - 3.10 (m, 1H), 2.80 (t, *J =* 6.7 Hz, 2H), 2.68 - 2.35 (m, 9H), 2.23 - 2.12 (m, 2H), 1.80 - 1.69 (m, 2H), 1.65 - 1.43 (m, 6H), 1.36 - 1.28 (m, 2H), 1.21 - 1.05 (m, 4H), 0.98 - 0.91 (m, 2H), 0.83 - 0.76 (m, 2H), 0.75 - 0.68 (m, 2H), 0.54 - 0.45 (m, 2H).

### Example 32: 1-(5-(9-((4-((1-(((4-(tert-butylamino)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1H,3H)-dione

### Step 1: preparation of N-(tert-butyl)-2,7-dichloro-8-fluoropyrido[4,3-d]pyrimidine-4-amine

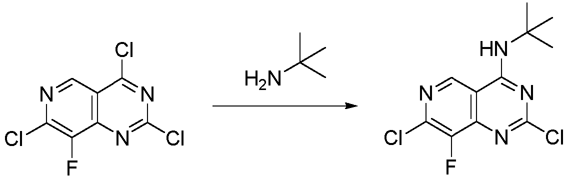

2,4,7-Trichloro-8-fluoropyrido[4,3-*d*]pyrimidine (1.00 g, 3.96 mmol) and triethylamine (1.20 g, 11.88 mmol) were dissolved in dichloromethane (10 mL), and the mixture was cooled to -78 °C. 2-Methylpropan-2-amine (348 mg, 4.75 mmol) was added, and the mixture was reacted at 78 °C for 3 h. After the reaction was completed, the reaction liquid was poured into a saturated aqueous ammonium chloride solution (50 mL), and the mixture was extracted with ethyl acetate (30 mL × 3). The combined organic phase was washed with saturated brine, dried over anhydrous sodium sulfate, and filtered. The filtrate was concentrated, and the resulting crude product was purified by silica gel column chromatography (EA:PE = 3:22) to give *N*-(*tert*-butyl)-2,7-dichloro-8-fluoropyrido[4,3-*d*]pyrimidine-4-amine.

LC-MS: (ESI, m/z): [M+H]⁺ = 288.9.

¹H NMR (400 MHz, CDCl₃) δ 8.68 (s, 1H), 6.12 (s, 1H), 1.62 (s, 9H).

### Step 2: preparation of (1-(((4-(tert-butylamino)-7-chloro-8-fluoropyrido[4,3-d]pyrimidin-2-yl)oxy)methyl) cyclopropyl)methanol

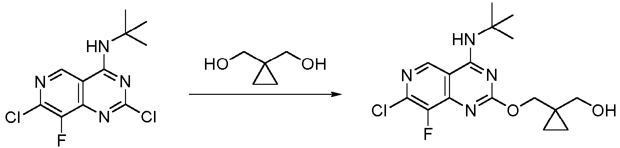

*N*-(*tert*-Butyl)-2,7-dichloro-8-fluoropyrido[4,3-*d*]pyrimidine-4-amine (590 mg, 2.04 mmol) and cyclopropane-1,1-diyldimethanol (417 mg, 4.08 mmol) were added to anhydrous acetonitrile (6 mL), and then cesium carbonate (997 mg, 3.06 mmol) and triethylenediamine (114 mg, 1.02 mmol) were added. The mixture was stirred at 25 °C for 1 h. After the reaction was completed, saturated brine (30 mL) was added to quench the reaction, and then the mixture was extracted with ethyl acetate (50 mL × 3). The combined organic phase was dried over anhydrous sodium sulfate and concentrated under reduced pressure. The concentrate was purified by silica gel column chromatography (PE:EtOAc = 5:1) to give *(1-(((4-(tert-*butylamino)-7-chloro-8-fluoropyrido[4,3-*d*]pyrimidin-2-yl)oxy)methyl)cyclopropyl)methanol.

LC-MS: (ESI, m/z): [M+H]⁺ = 354.9.

### Step 3: preparation of (1-(((4-(tert-butylamino)-8-fluoro-7-(7-fluoro-3-(methoxymethoxy)-8-((triisopropylsilyl) ethynyl)naphthalen-1-yl)pyrido[4,3-d]pyrimidin-2-yl)oxy)methyl)cyclopropyl)methanol

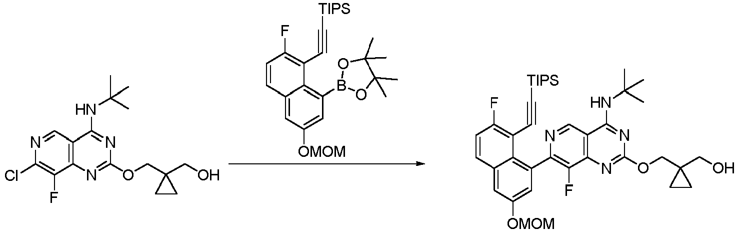

Cesium carbonate (1.38 g, 4.23 mmol) and cataXium APd G3 (102 mg, 0.14 mmol) were added to a mixed solution of (1-(((4-(*tert*-butylamino)-7-chloro-8-fluoropyrido[4,3-*d*]pyrimidin-2-yl)oxy)methyl)cyclopropyl) methanol (500 mg, 1.41 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (867 mg, 01.69 mmol)) in 1,4-dioxane (5 mL) and H₂O (1 mL), and the mixture was reacted at 85 °C overnight under N₂ atmosphere. The reaction mixture was filtered, and the filtrate was concentrated. The resulting crude product was purified by silica gel column chromatography (PE:EtOAc = 5:1) to give (1-(((4-(*tert*-butylamino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl) cyclopropyl)methanol.

LC-MS: (ESI, m/z): [M+H]⁺ = 705.3.

### Step 4: preparation of 1-(((4-(tert-butylamino)-8-fluoro-7-(7-fluoro-3-(methoxymethoxy)-8-((triisopropylsilyl) ethynyl)naphthalen-1-yl)pyrido[4,3-d]pyrimidin-2-yl)oxy)methyl)cyclopropane-1-carbaldehyde

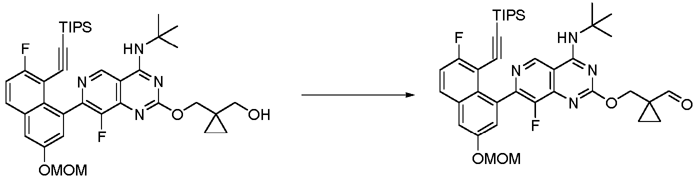

A solution of (1-(((4-(*tert*-butylamino)-8-fluoro-7-(7-fluoro-3-(methoxymethoxy)-8-((triisopropylsilyl) ethynyl)naphthalen-1-yl)pyrido[4,3-*d*]pyrimidin-2-yl)oxy)methyl)cyclopropyl)methanol (278 mg, 0.39 mmol) in DCM (2 mL) was cooled to 0 °C, and Dess-Martin reagent (496 mg, 1.17 mmol) was added. The mixture was reacted at room temperature for 0.5 h. After the reaction was completed, a saturated sodium thiosulfate solution (5 mL) was added to quench the reaction, and the mixture was extracted with dichloromethane (10 mL × 3). The combined organic phase was dried over anhydrous sodium sulfate and concentrated under reduced pressure. The concentrate was purified by silica gel column chromatography (PE:EtOAc = 5:1) to give 1-(((4-(*tert*-butylamino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropane-1-carbaldehyde.

LC-MS: (ESI, m/z): [M+H]⁺ = 703.4.

### Step 5: preparation of 1-(5-(9-((4-((1-(((4-(tert-butylamino)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1H,3H)-dione

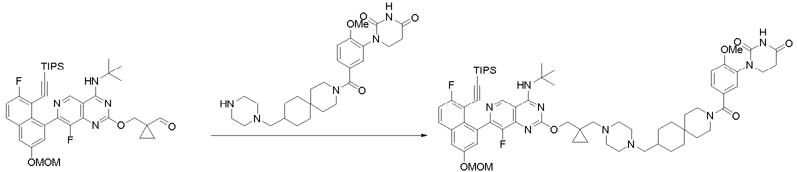

Tetraisopropyl titanate (0.5 mL) was added to a stirred solution of 1-(((4-(*tert*-butylamino)-8-fluoro-7-(7-fluoro-3-(methoxymethoxy)-8-((triisopropylsilyl)ethynyl)naphthalen-1-yl)pyrido [4,3-*d*]pyrimidin-2-yl)oxy) methyl)cyclopropane-1-carbaldehyde (150 mg, 0.21 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (159 mg, 0.32 mmol) in DCM/AcOH (1.5 mL/0.1 mL), and the mixture was stirred at 30 °C for 2 h. NaBH(OAc)₃ (136 mg, 0.64 mmol) was added to the mixture in an ice bath, and the resulting mixture was stirred for 2 h. The mixture was concentrated and purified by silica gel column chromatography (MeOH:DCM = (0-1):4) to give 1-(5-(9-((4-((1-(((4-(*tert*-butylamino)-8-fluoro-7-(7-fluoro-3-(methoxymethoxy)-8-((triisopropylsilyl)ethynyl) naphthalen-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 593.4.

### Step 6: preparation of 1-(5-(9-((4-((1-(((4-(tert-butylamino)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

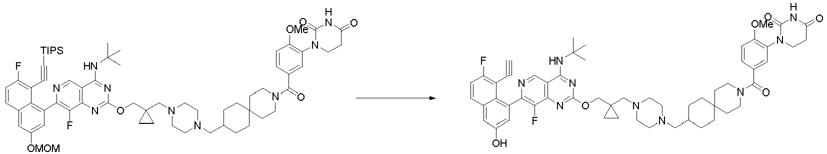

Cesium fluoride (84 mg, 0.55 mmol) was added to a stirred solution of *1-(5-(9-((4-((1-(((4-(tert-*butylamino)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione (131 mg, 0.11 mmol) in DMF (2 mL), and the mixture was stirred at room temperature for 30 min. Water (10 mL) was added to the reaction liquid to quench the reaction, and the mixture was extracted with ethyl acetate (10 mL × 3). The combined organic phase was dried over anhydrous sodium sulfate and concentrated under reduced pressure. HCl/1,4-dioxane (6 N, 1.0 mL) was added to a stirred solution of the concentrate in 1,4-dioxane (0.5 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((1-(((4-(*tert*-butylamino)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 984.2.

¹H NMR (400 MHz, DMSO-*d*₆) δ 10.34 (s, 1H), 10.14 (s, 1H), 9.38 (s, 1H), 8.02 - 7.95 (m, 2H), 7.47 - 7.37 (m, 1H), 7.39 - 7.34 (m, 2H), 7.31 (d, *J=* 2.0 Hz, 1H), 7.14 (d, *J =* 8.6 Hz, 1H), 7.11 (d, *J=* 2.4 Hz, 1H), 4.31 - 4.22 (m, 2H), 3.98 (s, 1H), 3.83 (s, 3H), 3.59 *(t, J=* 6.6 Hz, 2H), 3.32 - 3.29 (m, 1H), 2.70 - 2.64 (m, 2H), 2.47 - 2.17 (m, 10H), 2.07 - 1.98 (m, 2H), 1.71 - 1.63 (m, 2H), 1.58 (s, 9H), 1.54 - 1.37 (m, 6H), 1.36 - 1.15 (m, 3H), 1.14 - 0.82 (m, 5H), 0.66 - 0.59 (m, 2H), 0.44 - 0.36 (m, 2H).

### Example 33: (R)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

### Step 1: preparation of (R)-1-(2-chloro-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl) cyclopropyl)methyl)piperazin-1-yl)methyl)-3-azaspiro[5.5]undecane-3-carbonyl)phenyl)dihydropyrimidine-2,4(1H,3H)-dione

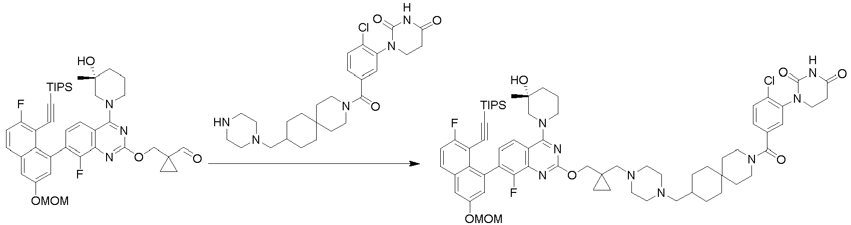

Tetraisopropyl titanate (190 mg, 0.67 mmol) was added to a stirred solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropane-1-carbaldehyde (100 mg, 0.134 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (81 mg, 0.161 mmol) in DCM/AcOH (1 mL/0.1 mL), and the mixture was stirred at 30 °C for 2 h. NaBH(OAc)₃ (89 mg, 0.402 mmol) was added to the mixture at room temperature, and the resulting mixture was stirred for 2 h. The mixture was concentrated and purified by silica gel column chromatography (DCM:MeOH = 100:1 to 20:1) to give (*R*)-1-(2-chloro-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl) cyclopropyl)methyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M/2+H]⁺ = 615.4.

### Step 2: preparation of (R)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

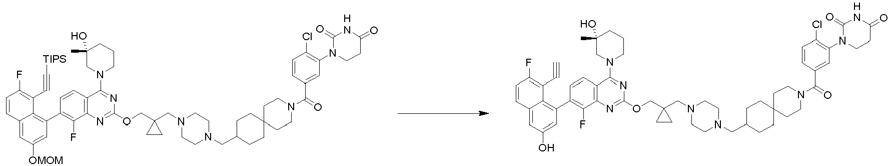

Cesium fluoride (62 mg, 0.41 mmol) was added to a stirred solution of (*R*)-1-(2-chloro-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropyl)methyl)piperazin-1-yl)methyl)-3-azaspiro[5.5] undecane-3-carbonyl)phenyl)dihydropyrimidine-2,4(1*H*,3*H*)-dione (100 mg, 0.081 mmol) in DMF (2 mL), and the mixture was stirred at room temperature for 30 min. Ethyl acetate and water were added to the reaction liquid. The mixture was separated, and the organic phase was concentrated. A mixture of 1,4-dioxane (1 mL) and HCl/1,4-dioxane (6 N, 0.5 mL) was then added to the resulting residue at room temperature. The mixture was reacted for 0.5 h. The mixture was purified by preparative high-performance liquid chromatography to give (R)-1-(2-chloro-5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro [5.5]undecane-3-carbonyl)phenyl)dihydropyrimidine-2,4(1*H*,3*H*)-dione.

LC-MS: (ESI, m/z): [M/2+H]⁺ = 515.2.
¹H NMR (400 MHz, DMSO-*d*₆) δ 10.50 (s, 1H), 10.11 (s, 1H), 7.96 (dd, *J* = 9.2, 6.0 Hz, 1H), 7.81 (dd, *J*= 34.0, 8.5 Hz, 1H), 7.63 (d, *J* = 8.2 Hz, 1H), 7.54 (d, *J* = 1.7 Hz, 1H), 7.49 - 7.42 (m, 1H), 7.40 - 7.33 (m, 2H), 7.23 - 7.15 (m, 1H), 7.08 (dd, *J* = 12.1, 2.3 Hz, 1H), 4.63 (d, *J* = 23.5 Hz, 1H), 4.28 - 4.16 (m, 2H), 4.12 - 3.71 (m, 4H), 3.67 - 3.36 (m, 5H), 3.30 - 3.22 (m, 2H), 2.78 - 2.70 (m, 2H), 2.45 - 2.23 (m, 9H), 2.05 (s, 3H), 1.78 - 1.58 (m, 5H), 1.55 - 1.23 (m, 8H), 1.16 (d, *J* = 7.1 Hz, 3H), 1.12 - 0.92 (m, 4H), 0.68 - *0.58* (m, 2H), 0.43 - 0.35 (m, 2H).

### Example 34: (R)-1-(5-(4-((2-(1-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperidin-4-yl)ethoxy)methyl)piperidine-1-carbonyl)-2-methylphenyl)dihydropyrimidine-2,4(1H,3H)-dione

### Step 1: preparation of tert-butyl 4-(2-((1-(3-(2,4-dioxotetrahydropyrimidin-1(2H)-yl)-4-methylbenzoyl) piperidin-4-yl)methoxy)ethyl)piperidine-1-carboxylate

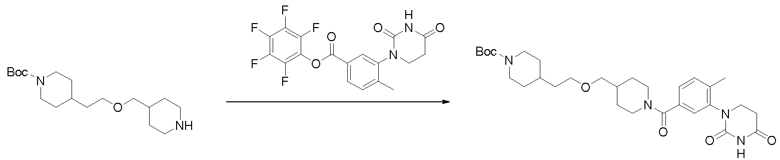

Pentafluorophenyl 3-(2,4-dioxotetrahydropyrimidin-1(2*H*)-yl)-4-methylbenzoate (278 mg, 0.67 mmol) and *N,N*-diisopropylethylamine (790 mg, 6.10 mmol) were added to a solution of *tert-butyl 4-(2-*(piperidin-4-ylmethoxy)ethyl)piperidine-1-carboxylate (200 mg, 0.61 mmol) in *N,N-dimethylformamide* (5 mL), and the mixture was stirred at room temperature for 2 h. After the reaction was completed, water (20 mL) was added, and the mixture was extracted with ethyl acetate (20 mL × 2). The organic phases were combined, washed with saturated sodium chloride (20 mL × 3), dried over anhydrous sodium sulfate, and concentrated under reduced pressure. The resulting crude product was purified by silica gel column chromatography (dichloromethane:methanol = 20:1) to give *tert-butyl* 4-(2-((1-(3-(2,4-dioxotetrahydropyrimidin-1(2*H*)-yl)-4-methylbenzoyl)piperidin-4-yl)methoxy)ethyl)piperidine-1-carboxylate.

LC-MS: (ESI, m/z): [M-Boc+H]⁺ = 457.3.

### Step 2: preparation of 1-(2-methyl-5-(4-((2-(piperidin-4-yl)ethoxy)methyl)piperidine-1-carbonyl)phenyl) dihydropyrimidine-2,4(1H,3H)-dione

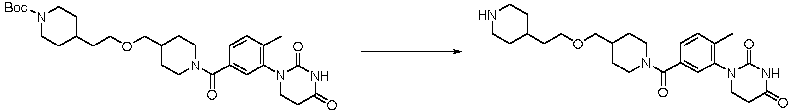

Trifluoroacetic acid (1.5 mL) was added to a solution of *tert-butyl* 4-(2-((1-(3-(2,4-dioxotetrahydropyrimidin-1(2*H*)-yl)-4-methylbenzoyl)piperidin-4-yl)methoxy)ethyl)piperidine-1-carboxylate (280 mg, 0.504 mmol) in dichloromethane (3 mL), and the mixture was stirred at 25 °C for 1 h. After the reaction was completed, an aqueous Na₂CO₃ solution was added, and the mixture was adjusted to pH > 7 and extracted with ethyl acetate (5 mL × 3). The combined organic phase was dried over Na₂SO₄, filtered, and concentrated under reduced pressure, and the resulting crude product was purified by silica gel column chromatography (dichloromethane:methanol = 20:1) to give 1-(2-methyl-5-(4-((2-(piperidin-4-yl)ethoxy)methyl)piperidine-1-carbonyl)phenyl)dihydropyrimidine-2,4(1*H*,3*H*)-dione.

LC-MS: (ESI, m/z): [M+H]⁺ = 457.3.

### Step 3: preparation of (R)-1-(5-(4-((2-(1-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)quinazolin-2-yl)oxy)methyl)cyclopropyl) methyl)piperidin-4-yl)ethoxy)methyl)piperidine-1-carbonyl)-2-methylphenyl)dihydropyrimidine-2,4(1H,3H)-dione

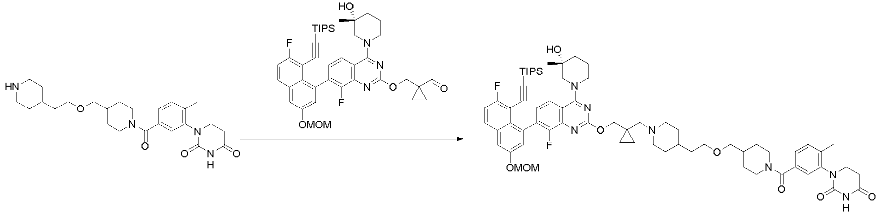

Tetraisopropyl titanate (190 mg, 0.67 mmol) was added to a stirred solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropane-1-carbaldehyde (100 mg, 0.134 mmol) and 1-(2-methyl-5-(4-((2-(piperidin-4-yl)ethoxy)methyl)piperidine-1-carbonyl)phenyl)dihydropyrimidine-2,4(1*H*,3*H*)-dione (81 mg, 0.161 mmol) in DCM/AcOH (1 mL/0.1 mL), and the mixture was stirred at 30 °C for 2 h. NaBH(OAc)₃ (89 mg, 0.402 mmol) was added to the mixture at room temperature, and the resulting mixture was stirred for 2 h. The mixture was concentrated and purified by silica gel column chromatography (MeOH:DCM = (0-1):4) to give (R)-1-(5-(4-((2-(1-((1-(((8-fluoro-7-(7-fluoro-3-(methoxymethoxy)-8-((triisopropylsilyl)ethynyl) naphthalen-1-yl)-4-(3-hydroxy-3-methylpiperidin-1-yl)quinazolin-2-yl)oxy)methyl)cyclopropyl)methyl) piperidin-4-yl)ethoxy)methyl)piperidine-1-carbonyl)-2-methylphenyl)dihydropyrimidine-2,4(1*H*,3*H*)-dione.

LC-MS: (ESI, m/z): [M/2+H]⁺ = 592.9.

### Step 4: preparation of (R)-1-(5-(4-((2-(1-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperidin-4-yl)ethoxy) methyl)piperidine-1-carbonyl)-2-methylphenyl)dihydropyrimidine-2,4(1H,3H)-dione

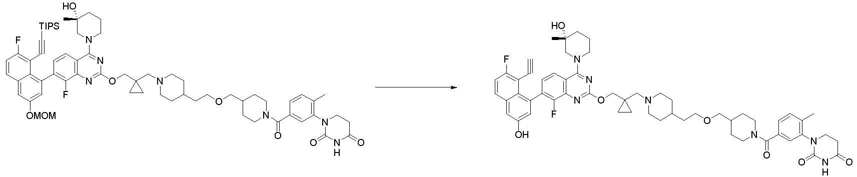

CsF (64 mg, 0.42 mmol) was added to a solution of (*R*)-1-(5-(4-((2-(1-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl) quinazolin-2-yl)oxy)methyl)cyclopropyl)methyl)piperidin-4-yl)ethoxy)methyl)piperidine-1-carbonyl)-2-methylphenyl)dihydropyrimidine-2,4(1*H*,3*H*)-dione (100 mg, 0.085 mmol) in DMF (1 mL), and the mixture was stirred at room temperature for 0.5 h. Water (2 mL) was added to the reaction liquid, and the mixture was then extracted with ethyl acetate (5 mL × 3). The organic phases were combined, dried over anhydrous sodium sulfate, and concentrated under reduced pressure. The intermediate obtained by concentration was dissolved in 1,4-dioxane (2 mL), and HCl/1,4-dioxane (6 N, 1 mL) was then added. The mixture was reacted at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, concentrated under reduced pressure, and purified by preparative high-performance liquid chromatography to give (*R*)-1-(5-(4-((2-(1-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperidin-4-yl)ethoxy) methyl)piperidine-1-carbonyl)-2-methylphenyl)dihydropyrimidine-2,4(1*H*,3*H*)-dione.

LC-MS: (ESI, m/z): [M/2+H]⁺ = 492.8.

¹H NMR (400 MHz, DMSO-*d*₆) δ 10.36 (s, 1H), 10.11 (s, 1H), 7.96 (dd, *J =* 9.1, 5.9 Hz, 1H), 7.81 (dd, *J =* 34.7, 8.7 Hz, 1H), 7.52 - 7.41 (m, 1H), 7.39 - 7.27 (m, 3H), 7.26 - 7.16 (m, 2H), 7.08 (dd, *J =* 12.0, 2.3 Hz, 1H), 4.63 (d, *J* = 24.2 Hz, 1H), 4.53 - 4.30 (m, 1H), 4.28 - 4.14 (m, 2H), 4.11 - 3.93 (m, 1H), 3.91 - 3.71 (m, 3H), 3.67 - 3.33 (m, 6H), 3.30 - 3.24 (m, 1H), 3.20 (d, *J* = 6.1 Hz, 2H), 3.09 - 2.85 (m, 3H), 2.80 - 2.65 (m, 3H), 2.33 - 2.24 (m, 2H), 2.21 (s, 3H), 2.10 - 1.97 (m, 1H), 1.87 - 1.51 (m, 10H), 1.44 - 1.34 (m, 2H), 1.30 - 1.23 (m, 1H), 1.18 - 1.06 (m, 6H), 0.70 - 0.54 (m, 2H), 0.47 - 0.28 (m, 2H).

### Example 35: 3-(6-(9-((4-((1-(((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-1-oxoisoindolin-2-yl)piperidine-2,6-dione

### Step 1: preparation of 2-(2,6-dioxopiperidin-3-yl)-3-oxoisoindoline-5-carboxylic acid

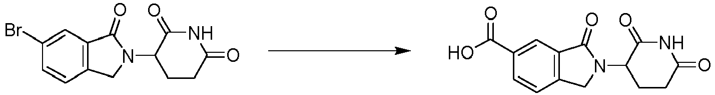

3-(6-Bromo-1-oxoisoindolin-2-yl)piperidine-2,6-dione (500 mg, 1.55 mmol) was dissolved in DMF (10 mL), and then oxalic acid (280 mg, 3.10 mmol), DIEA (600 mg, 4.65 mmol), acetic anhydride (403 mg, 1.27 mmol), palladium acetate (70 mg, 0.31 mmol), and Xantphos (270 mg, 0.47 mmol) were added. The mixture was reacted at 100 °C for 2 h. The reaction liquid was filtered, concentrated, and purified by preparative reversed-phase chromatography to give 2-(2,6-dioxopiperidin-3-yl)-3-oxoisoindoline-5-carboxylic acid.

LC-MS: (ESI, m/z): [M-H]⁻ = 287.1.

### Step 2: preparation of pentafluorophenyl 2-(2,6-dioxopiperidin-3-yl)-3-oxoisoindoline-5-carboxylate

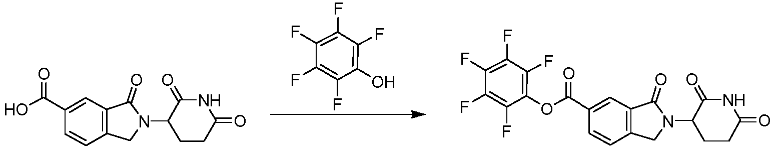

2,3,4,5,6-Pentafluorophenol (64 mg, 0.35 mmol), 4-dimethylaminopyridine (4 mg, 0.035 mmol), and *N,N'*-dicyclohexylcarbodiimide (101 mg, 0.49 mmol) were added to a solution of 2-(2,6-dioxopiperidin-3-yl)-3-oxoisoindoline-5-carboxylic acid (100 mg, 0.35 mmol) in 1,4-dioxane (3 mL). The mixed solution was stirred at 25 °C for 16 h. After the reaction was completed, HCl (2 N, 10 mL) was added, and the mixture was extracted with ethyl acetate (10 mL × 2). The organic phase was dried over anhydrous Na₂SO₄, filtered, and concentrated in vacuum. The concentrate was purified by preparative reversed-phase chromatography to give pentafluorophenyl 2-(2,6-dioxopiperidin-3-yl)-3-oxoisoindoline-5-carboxylate.

LC-MS: (ESI, m/z): [M-H]⁻ = 453.1.

### Step 3: preparation of 3-(6-(9-((4-((1-(((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-1-oxoisoindolin-2-yl)piperidine-2,6-dione

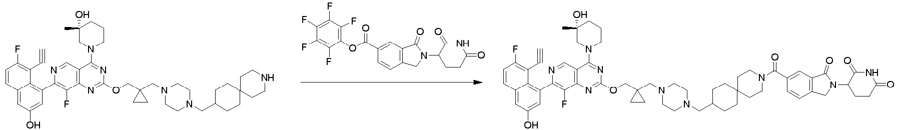

DIEA (10 mg, 0.076 mmol) and pentafluorophenyl 2-(2,6-diox*o*piperidin-3-yl)-3-oxoisoindoline-5-carboxylate (12 mg, 0.027 mmol) were added to a solution of (*R*)-1-(2-((1-((4-((3-azaspiro[5.5]undecan-9-yl)methyl)piperazin-1-yl)methyl)cyclopropyl)methoxy)-7-(8-ethynyl-7-fluoro-3-hydroxynaphthalen-1-yl)-8-fluoropyrido[4,3-*d*]pyrimidin-4-yl)-3-methylpiperidin-3-ol (30 mg, 0.038 mmol) in DMF (1.3 mL), and the mixture was reacted at room temperature for 2 h. Water (10 mL) was added to the reaction liquid, and the mixture was extracted with ethyl acetate (10 mL × 2). The organic phase was washed with saturated brine, dried over anhydrous sodium sulfate, and filtered, and the filtrate was concentrated and purified by preparative high-performance liquid chromatography to give 3-(6-(9-((4-((1-(((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy) methyl)cyclopropyl)methyl)piperazin-1-yl)methyl)-3-azaspiro[5.5]undecane-3-carbonyl)-1-oxoisoindolin-2-yl)piperidine-2,6-dione.

LC-MS: (ESI, m/z): [M/2+H]⁺ = 525.7.

¹H NMR (400 MHz, DMSO-*d*₆) δ 11.00 (s, 1H), 10.21 (brs, 1H), 9.12 (d, *J* = 65.5 Hz, 1H), 8.27 (s, 1H), 7.97 (dd, *J* = 9.1, 6.0 Hz, 1H), 7.72 - 7.57 (m, 3H), 7.50 - 7.41 (m, 1H), 7.39 (s, 1H), 7.21 (d, *J* = 17.2 Hz, 1H), 5.13 (dd, *J=* 13.1, 5.0 Hz, 1H), 4.84 - 4.62 (m, 1H), 4.51 (*d, J =* 17.6 Hz, 1H), 4.43 - 4.20 (m, 4H), 4.09 (d, *J* = 12.9 Hz, 1H), 4.00 - 3.89 (m, 1H), 3.65 - 3.53 (m, 3H), 2.96 - 2.87 (m, 1H), 2.69 - 2.57 (m, 2H), 2.43 - 2.19 (m, 11H), 2.13 - 1.93 (m, 5H), 1.75 - 1.61 (m, 5H), 1.57 - 1.32(m, 7H), 1.27 - 1.22 (m, 1H), 1.17 (d, *J =* 16.5 Hz, 3H), 1.10 - 0.93 (m, 4H), 0.67 - 0.58 (m, 2H), 0.45 - 0.36 (m, 2H).

### Example 36: 1-(5-(9-((4-((1-(((7-(8-ethynyl-7-fluoro-3-hydroxynaphthalen-1-yl)-8-fluoro-4-(1,4-oxazepan-4-yl)pyrido[4,3-d]pyrimidin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1H,3H)-dione

### Step 1: preparation of 4-(2,7-dichloro-8-fluoropyrido[4,3-d]pyrimidin-4-yl)-1,4-oxazepane

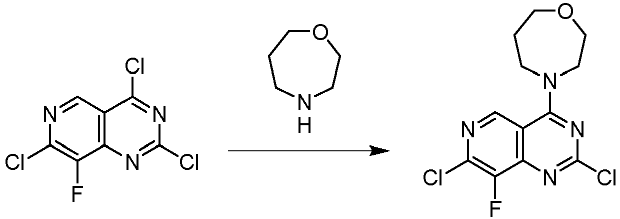

Triethylamine (1.2 g, 11.88 mmol) and 1,4-oxazepane (350 mg, 3.57 mmol) were added to a solution of 2,4,7-trichloro-8-fluoropyrido[4,3-*d*]pyrimidine (1 g, 3.96 mmol) in anhydrous tetrahydrofuran (30 mL) at -50 °C, and the mixture was stirred at -50 °C for 2 h. After the reaction was completed, saturated ammonium chloride (50 mL) was added, and the mixture was extracted with ethyl acetate (50 mL × 2). The organic phases were combined, dried over anhydrous sodium sulfate, and concentrated under reduced pressure. The resulting crude product was purified by silica gel column chromatography (dichloromethane:methanol = 50:1) to give 4-(2,7-dichloro-8-fluoropyrido[4,3-*d*]pyrimidin-4-yl)-1,4-oxazepane.

LC-MS: (ESI, m/z): [M+H]⁺ = 317.1.

### Step 2: preparation of (1-(((7-chloro-8-fluoro-4-(1,4-oxazepan-4-yl)pyrido[4,3-d]pyrimidin-2-yl)oxy)methyl) cyclopropyl)methanol

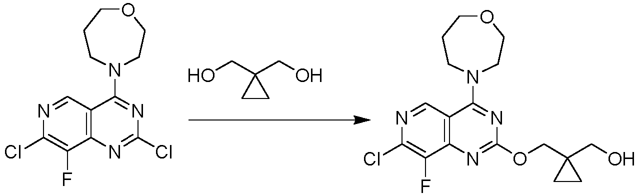

Triethylenediamine (106 mg, 0.079 mmol) and anhydrous cesium carbonate (1850 mg, 5.692 mmol) were added to a solution of 4-(2,7-dichloro-8-fluoropyrido[4,3-*d*]pyrimidin-4-yl)-1,4-oxazepane (600 mg, 1.893 mmol) and cyclopropane-1,1-diyldimethanol (386 mg, 3.784 mmol) in anhydrous acetonitrile (30 mL), and the mixture was stirred at 25 °C for 16 h. The reaction liquid was poured into water (50 mL), and the mixture was extracted with ethyl acetate (20 mL × 2). The organic phases were combined, washed with saturated sodium chloride (50 mL), dried over anhydrous sodium sulfate, and concentrated under reduced pressure. The resulting crude product was purified by silica gel column chromatography (dichloromethane:methanol = 20:1) to give (1-(((7-chloro-8-fluoro-4-(1,4-oxazepan-4-yl)pyrido[4,3-*d*]pyrimidin-2-yl)oxy)methyl)cyclopropyl)
methanol.

LC-MS: (ESI, m/z): [M+H]⁺ = 383.1.

### Step 3: preparation of (1-(((8-fluoro-7-(7-fluoro-3-(methoxymethoxy)-8-((triisopropylsilyl)ethynyl) naphthalen-1-yl)-4-(1,4-oxazepan-4-yl)pyrido[4,3-d]pyrimidin-2-yl)oxy)methyl)cyclopropyl)methanol

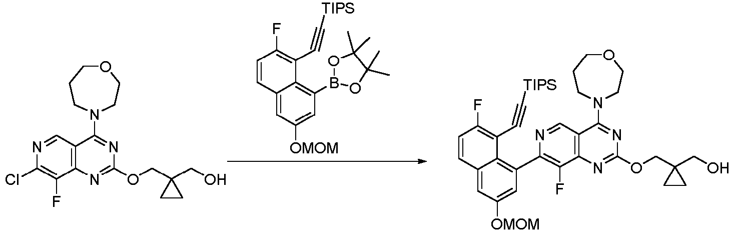

Anhydrous cesium carbonate (1014 mg, 3.120 mmol) and cataCXium(R) A Pd G3 (75 mg, 0.104 mmol) were added to a solution of (1-(((7-chloro-8-fluoro-4-(1,4-oxazepan-4-yl)pyrido[4,3-*d*]pyrimidin-2-yl)oxy)methyl) cyclopropyl)methanol (400 mg, 1.044 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane in 1,4-dioxane (14 mL) and water (3 mL) at room temperature. After the system was degassed, the mixture was stirred at 85 °C for 16 h under nitrogen atmosphere. The reaction liquid was directly concentrated under reduced pressure at 40 °C, and the resulting crude product was purified by silica gel thin-layer chromatography (dichloromethane:methanol = 10:1) to give (1-(((8-fluoro-7-(7-fluoro-3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl)cyclopropyl)methanol.

LC-MS: (ESI, m/z): [M+H]⁺ = 733.3.

### Step 4: preparation of 1-(((8-fluoro-7-(7-fluoro-3-(methoxymethoxy)-8-((triisopropylsilyl)ethynyl) naphthalen-1-yl)-4-(1,4-oxazepan-4-yl)pyrido[4,3-d]pyrimidin-2-yl)oxy)methyl)cyclopropane-1-carbaldehyde

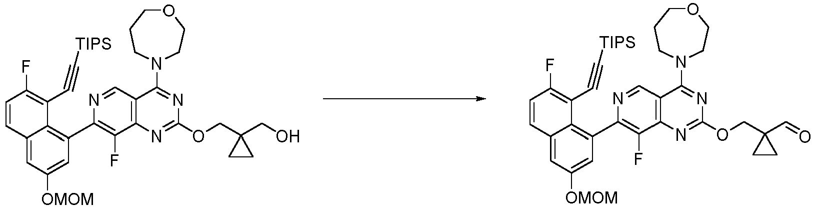

A solution of (1-(((8-fluoro-7-(7-fluoro-3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl)cyclopropyl)methanol (350 mg, 0.546 mmol) in dichloromethane (5 mL) was cooled to 0 °C from room temperature, and Dess-Martin reagent (470 mg, 1.092 mmol) was added. The mixture was stirred at 25 °C for 4 h. The reaction liquid was poured into water (50 mL), and the mixture was then extracted with dichloromethane (20 mL × 2). The organic phases were combined, washed with saturated sodium chloride (50 mL), dried over anhydrous sodium sulfate, and concentrated under reduced pressure. The resulting crude product was purified by silica gel thin-layer chromatography (dichloromethane:methanol = 20:1) to give 1-(((8-fluoro-7-(7-fluoro-3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)
methyl)cyclopropane-1-carbaldehyde.

LC-MS: (ESI, m/z): [M+H]⁺ = 731.3.

### Step 5: preparation of 1-(5-(9-((4-((1-(((8-fluoro-7-(7-fluoro-3-(methoxymethoxy)-8-((triisopropylsilyl) ethynyl)naphthalen-1-yl)-4-(1,4-oxazepan-4-yl)pyrido[4,3-d]pyrimidin-2-yl)oxy)methyl)cyclopropyl) methyl)piperazin-1-yl)methyl)-3-azaspiro[5.5]undecane-3-carbonyl)-2-methylphenyl)dihydropyrimidine-2,4(1H,3H)-dione

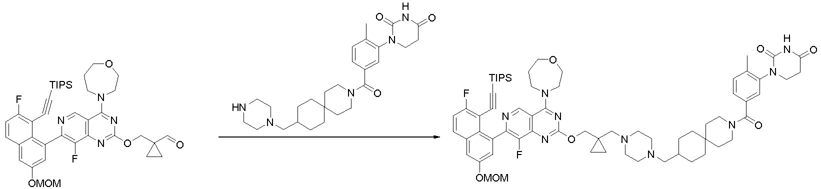

Tetraisopropyl titanate (400 mg, 1.370 mmol) was added to a solution of 1-(((8-fluoro-7-(7-fluoro-3-(methoxymethoxy)-8-((triisopropylsilyl)ethynyl)naphthalen-1-yl)-4-(1,4-oxazepan-4-yl)pyrido[4,3-*d*|pyrimidin-2-yl)oxy)methyl)cyclopropane-1-carbaldehyde (200 mg, 0.274 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (160 mg, 0.333 mmol) in dichloromethane (5 mL) at room temperature, and the mixture was stirred at 25 °C for 16 h. Sodium triacetoxyborohydride (200 mg, 0.822 mmol) was added to the mixture at room temperature, and the mixture was reacted at 25 °C for another 3 h. Methanol (10 mL) was added to the reaction liquid to quench the reaction, and the mixture was concentrated under reduced pressure at 40 °C. The resulting crude product was purified by a preparative thin-layer chromatography plate (dichloromethane:methanol = 10:1, 0.1% NH₃) to give 1-(5-(9-((4-((1-(((8-fluoro-7-(7-fluoro-3-(methoxymethoxy)-8-((triisopropylsilyl)ethynyl) naphthalen-1-yl)-4-(1,4-oxazepan-4-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1196.9.

### Step 6: preparation of 1-(5-(9-((4-((1-(((7-(8-ethynyl-7-fluoro-3-hydroxynaphthalen-1-yl)-8-fluoro-4-(1,4-oxazepan-4-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

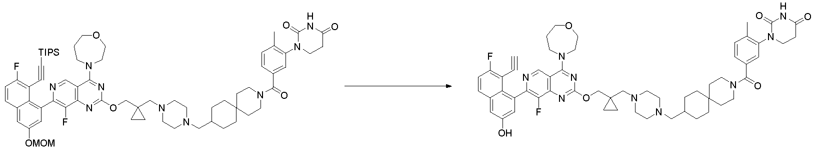

CsF (126 mg, 0.835 mmol) was added to a solution of 1-(5-(9-((4-((1-(((8-fluoro-7-(7-fluoro-3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione (200 mg, 0.167 mmol) in DMF (2 mL), and the mixture was stirred at room temperature for 0.5 h. Water (10 mL) was added to the reaction liquid, and the mixture was then extracted with ethyl acetate (10 mL × 3). The organic phases were combined, dried over anhydrous sodium sulfate, and concentrated under reduced pressure. The intermediate obtained by concentration was dissolved in 1,4-dioxane (2 mL), and HCl/1,4-dioxane (6 N, 1 mL) was then added. The mixture was reacted at room temperature for 0.5 h. The reaction liquid was adjusted to pH > 7 with an aqueous Na₂CO₃ solution and extracted with ethyl acetate (5 mL × 3). The combined organic phase was dried over Na₂SO₄, filtered, concentrated under reduced pressure, and purified by preparative high-performance liquid chromatography to give 1-(5-(9-((4-((1-(((7-(8-ethynyl-7-fluoro-3-hydroxynaphthalen-1-yl)-8-fluoro-4-(1,4-oxazepan-4-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 498.7.

¹H NMR (400 MHz, DMSO-*d*₆) δ 10.36 (s, 1H), 10.14 (s, 1H), 9.09 (s, 1H), 7.97 (dd, *J* = 9.2, 5.9 Hz, 1H), 7.49 - 7.43 (m, 1H), 7.39 (d, *J* = 2.4 Hz, 1H), 7.35 - 7.27 (m, 2H), 7.25-7.19 (m, 1H), 7.17 (d, *J* = 2.4 Hz, 1H), 4.33 - 4.22 (m, 2H), 4.19 - 4.05 (m, 4H), 3.99 (s, 1H), 3.97 - 3.90 (m, 2H), 3.81 - 3.69 (m, 3H), 3.60 - 3.45 (m, 2H), 2.83 - 2.62 (m, 2H), 2.42 - 2.23 (m, 8H), 2.23 - 2.17 (m, 4H), 2.15 - 1.97 (m, 5H), 1.74 - 1.60 (m, 2H), 1.56 - 1.35 (m, 6H), 1.32 - 0.80 (m, 5H), 0.65 - 0.57 (m, 2H), 0.43 - 0.36 (m, 2H).

### Example 37: 1-(5-(9-((4-((1-(((4-(azepan-1-yl)-7-(8-ethynyl-7-fluoro-3-hydroxynaphthalen-1-yl)-8-fluoropyrido [4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of 4-(azepan-1-yl)-2,7-dichloro-8-fluoropyrido[4,3-d]pyrimidine

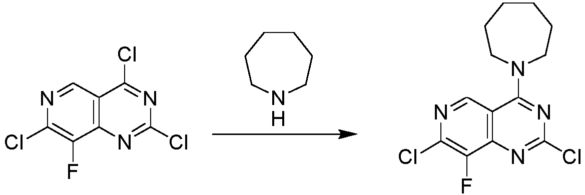

Triethylamine (1.2 g, 11.88 mmol) and azepane (350 mg, 3.57 mmol) were added to a solution of 2,4,7-trichloro-8-fluoropyrido[4,3-*d*]pyrimidine (1 g, 3.96 mmol) in anhydrous tetrahydrofuran (30 mL) at - 50 °C, and the mixture was stirred at -50 °C for 2 h. After the reaction was completed, saturated ammonium chloride (50 mL) was added, and the mixture was extracted with ethyl acetate (50 mL × 2). The organic phases were combined, dried over anhydrous sodium sulfate, and concentrated under reduced pressure. The resulting crude product was purified by silica gel column chromatography (dichloromethane:methanol = 50:1) to give 4-(azepan-1-yl)-2,7-dichloro-8-fluoropyrido[4,3-*d*]pyrimidine.

LC-MS: (ESI, m/z): [M+H]⁺ = 315.0.

### Step 2: preparation of (1-(((4-(azepan-1-yl)-7-chloro-8-fluoropyrido[4,3-d]pyrimidin-2-yl)oxy)methyl) cyclopropyl)methanol

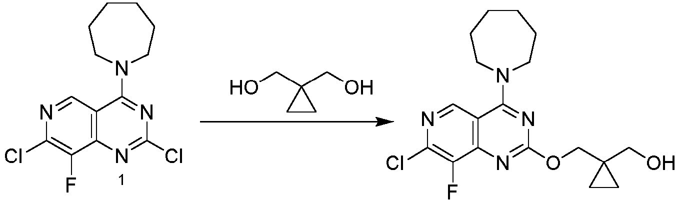

Triethylenediamine (160 mg, 1.428 mmol) and anhydrous cesium carbonate (2.78 g, 8.571 mmol) were added to a solution of 4-(azepan-1-yl)-2,7-dichloro-8-fluoropyrido[4,3-*d*]pyrimidine (900 mg, 2.857 mmol) and cyclopropane-1,1-diyldimethanol (583 mg, 5.714 mmol) in anhydrous acetonitrile (30 mL), and the mixture was stirred at 25 °C for 16 h. The reaction liquid was poured into water (50 mL), and the mixture was extracted with ethyl acetate (30 mL × 2). The organic phases were combined, washed with saturated sodium chloride (50 mL), dried over anhydrous sodium sulfate, and concentrated under reduced pressure. The resulting crude product was purified by silica gel column chromatography (dichloromethane:methanol = 20:1) to give (1-(((4-(azepan-1-yl)-7-chloro-8-fluoropyrido[4,3-*d*]pyrimidin-2-yl)oxy)methyl)cyclopropyl)methanol.

LC-MS: (ESI, m/z): [M+H]⁺ = 381.1.

### Step 3: preparation of (1-(((4-(azepan-1-yl)-8-fluoro-7-(7-fluoro-3-(methoxymethoxy)-8-((triisopropylsilyl) ethynyl)naphthalen-1-yl)pyrido[4,3-d]pyrimidin-2-yl)oxy)methyl)cyclopropyl)methanol

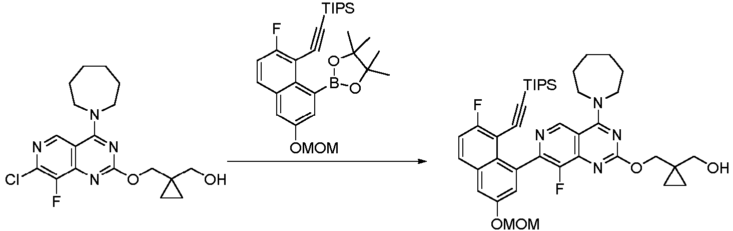

Anhydrous cesium carbonate (1.4 g, 4.341 mmol) and cataCXium(R) A Pd G3 (105 mg, 0.145 mmol) were added to a mixed solution of (1-(((4-(azepan-1-yl)-7-chloro-8-fluoropyrido[4,3-*d*]pyrimidin-2-yl)oxy)methyl) cyclopropyl)methanol (550 mg, 1.447 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (890 mg, 1.736 mmol) in 1,4-dioxane (14 mL) and water (3 mL) at room temperature. After the system was degassed, the mixture was stirred at 85 °C for 16 h under nitrogen atmosphere. The reaction liquid was concentrated under reduced pressure at 40 °C, and the resulting crude product was purified by silica gel thin-layer chromatography (dichloromethane:methanol = 10:1) to give (1-(((4-(azepan-1-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methanol.

LC-MS: (ESI, m/z): [M+H]⁺ = 731.3.

### Step 4: preparation of 1-(((4-(azepan-1-yl)-8-fluoro-7-(7-fluoro-3-(methoxymethoxy)-8-((triisopropylsilyl) ethynyl)naphthalen-1-yl)pyrido[4,3-d]pyrimidin-2-yl)oxy)methyl)cyclopropane-1-carbaldehyde

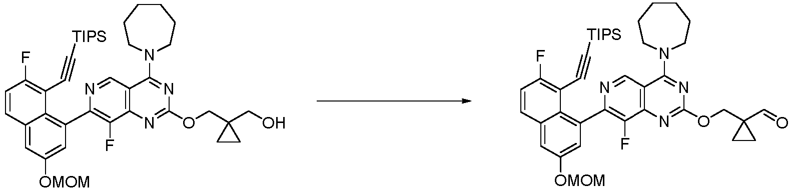

A solution of (1-(((4-(azepan-1-yl)-8-fluoro-7-(7-fluoro-3-(methoxymethoxy)-8-((triisopropylsilyl)ethynyl) naphthalen-1-yl)pyrido[4,3-*d*]pyrimidin-2-yl)oxy)methyl)cyclopropyl)methanol (500 mg, 0.680 mmol) in dichloromethane (8 mL) was cooled to 0 °C from room temperature, and Dess-Martin reagent (850 mg, 1.370 mmol) was added. The mixture was stirred at 25 °C for 4 h. The reaction liquid was poured into water (30 mL), and the mixture was extracted with dichloromethane (20 mL × 2). The organic phases were combined, washed with saturated sodium chloride (30 mL), dried over anhydrous sodium sulfate, and concentrated under reduced pressure. The resulting crude product was purified by silica gel thin-layer chromatography (dichloromethane:methanol = 20:1) to give 1-(((4-(azepan-1-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropane-1-carbaldehyde.

LC-MS: (ESI, m/z): [M+H]⁺ = 729.3.

### Step 5: preparation of 1-(5-(9-((4-((1-(((4-(azepan-1-yl)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropyl)methyl) piperazin-1-yl)methyl)-3-azaspiro[5.5]undecane-3-carbonyl)-2-methylphenyl)dihydropyrimidine-2,4(1H,3H)-dione

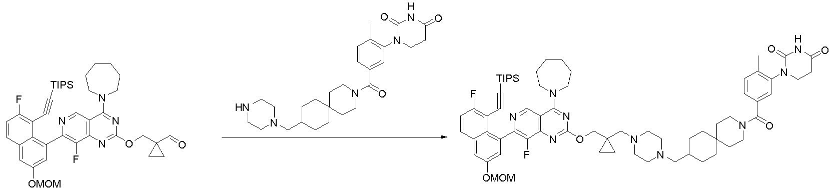

Tetraisopropyl titanate (597 mg, 2.105 mmol) was added to a solution of 1-(((4-(azepan-1-yl)-8-fluoro-7-(7-fluoro-3-(methoxymethoxy)-8-((triisopropylsilyl)ethynyl)naphthalen-1-yl)pyrido [4,3-*d*]pyrimidin-2-yl)oxy)
methyl)cyclopropane-1-carbaldehyde (300 mg, 0.412 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (238 mg, 0.494 mmol) in dichloromethane (5 mL) at room temperature, and the mixture was stirred at 25 °C for 16 h. Sodium triacetoxyborohydride (280 mg, 1.263 mmol) was added to the mixture at room temperature, and the mixture was then reacted at 25 °C for another 3 h. Methanol (10 mL) was added to the reaction liquid to quench the reaction, and the mixture was then concentrated under reduced pressure at 40 °C. The resulting crude product was purified by a preparative thin-layer chromatography plate (dichloromethane:methanol = 10:1, 0.1% NH₃) to give 1-(5-(9-((4-((1-(((4-(azepan-1-yl)-8-fluoro-7-(7-fluoro-3-(methoxymethoxy)-8-((triisopropylsilyl) ethynyl)naphthalen-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 598.4.

### Step 6: preparation of 1-(5-(9-((4-((1-(((4-(azepan-1-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1H,3H)-dione

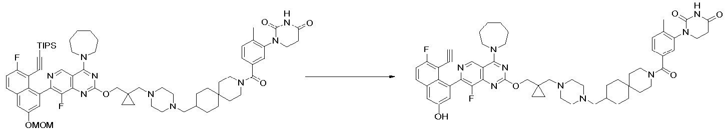

CsF (158 mg, 1.047 mmol) was added to a solution of 1-(5-(9-((4-((1-(((4-(azepan-1-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl) cyclopropyl)methyl)piperazin-1-yl)methyl)-3-azaspiro[5.5]undecane-3-carbonyl)-2-methylphenyl) dihydropyrimidine-2,4(1*H*,3*H*)-dione (250 mg, 0.209 mmol) in DMF (2 mL), and the mixture was stirred at room temperature for 0.5 h. Water (10 mL) was added to the reaction liquid, and the mixture was extracted with ethyl acetate (10 mL × 3). The organic phases were combined, dried over anhydrous sodium sulfate, and concentrated under reduced pressure. The intermediate obtained by concentration was dissolved in dioxane (2 mL), and HCl/dioxane (6 N, 1 mL) was then added. The mixture was reacted at room temperature for 0.5 h. The reaction liquid was adjusted to pH > 7 with an aqueous Na₂CO₃ solution and extracted with ethyl acetate (5 mL × 3). The combined organic phase was dried over Na₂SO₄, filtered, concentrated under reduced pressure, and purified by preparative high-performance liquid chromatography to give 1-(5-(9-((4-((1-(((4-(azepan-1-yl)-7-(8-ethynyl-7-fluoro-3-hydroxynaphthalen-1-yl)-8-fluoropyrido[4,3-*d*]pyrimidin-2-yl)oxy)methyl) cyclopropyl)methyl)piperazin-1-yl)methyl)-3-azaspiro[5.5]undecane-3-carbonyl)-2-methylphenyl) dihydropyrimidine-2,4(1*H*,3*H*)-dione.

LC-MS: (ESI, m/z): [M+H]⁺ = 994.2.

¹H NMR (400 MHz, DMSO-*d*₆) δ 10.38 (s, 1H), 10.15 (s, 1H), 9.07 (s, 1H), 7.97 (dd, *J* = 9.2, 6.0 Hz, 1H), 7.49 - 7.43 (m, 1H), 7.39 (d, *J=* 2.5 Hz, 1H), 7.35 - 7.29 (m, 2H), 7.24 (d, *J=* 7.6 Hz, 1H), 7.17 (d, *J=* 2.4 Hz, 1H), 4.36 - 4.19 (m, 2H), 4.05-3.92 (m, 5H), 3.84 - 3.73 (m, 1H), 3.64 - 3.47 (m, 3H), 3.32 - 3.21 (m, 2H), 2.80 - 2.65 (m, 2H), 2.42 - 2.23 (m, 8H), 2.21 (s, 3H), 2.05-1.90 (m, 6H), 1.72-1.58 (m, 6H), 1.35 - 1.12 (m, 3H), 1.10 -0.92 (m, 4H), 0.66 - 0.59 (m, 2H), 0.43 - 0.36 (m, 2H).

### Example 38: (R)-1-(5-(4-((2-(1-((1-(((7-(8-ethynyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperidin-4-yl)ethoxy)methyl)piperidine-1-carbonyl)-2-methoxyphenyl)dihydropyrimidine-2,4(1H,3H)-dione

### Step 1: preparation of tert-butyl 4-(2-((1-(3-(2,4-dioxotetrahydropyrimidin-1(2H)-yl)-4-methoxybenzoyl) piperidin-4-yl)methoxy)ethyl)piperidine-1-carboxylate

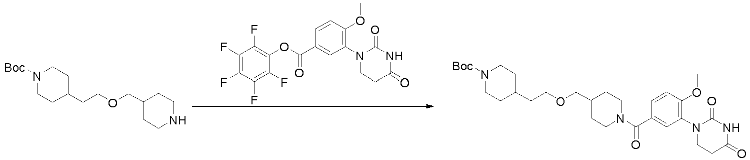

Pentafluorophenyl 3-(2,4-dioxotetrahydropyrimidin-1(2*H*)-yl)-4-methoxybenzoate (290 mg, 0.67 mmol) and *N,N*-diisopropylethylamine (800 mg, 9.20 mmol) were added to a solution of *tert-butyl 4-(2-*(piperidin-4-ylmethoxy)ethyl)piperidine-1-carboxylate (200 mg, 0.61 mmol) in *N,N-dimethylformamide* (6 mL), and the mixture was stirred at room temperature for 2 h. After the reaction was completed, water (20 mL) was added to the reaction liquid, and the mixture was extracted with ethyl acetate (30 mL × 2). The organic phases were combined, washed with saturated sodium chloride (20 mL × 3), dried over anhydrous sodium sulfate, and concentrated under reduced pressure. The resulting crude product was purified by silica gel column chromatography (dichloromethane:methanol = 20:1) to give *tert-butyl* 4-(2-((1-(3-(2,4-dioxotetrahydropyrimidin-1(2*H*)-yl)-4-methoxybenzoyl)piperidin-4-yl)methoxy)ethyl)piperidine-1-carboxylate.

LC-MS: (ESI, m/z): [M+H]⁺ = 573.0.

### Step 2: preparation of 1-(2-methoxy-5-(4-((2-(piperidin-4-yl)ethoxy)methyl)piperidine-1-carbonyl)phenyl) dihydropyrimidine-2,4(1H,3H)-dione

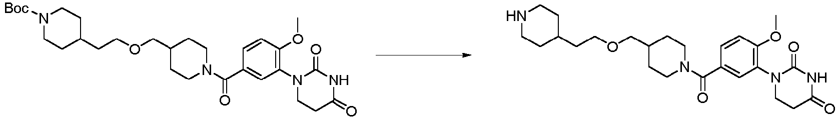

Trifluoroacetic acid (1.5 mL) was added to a solution of *tert-butyl* 4-(2-((1-(3-(2,4-dioxotetrahydropyrimidin-1(2*H*)-yl)-4-methoxybenzoyl)piperidin-4-yl)methoxy)ethyl)piperidine-1-carboxylate (272 mg, 0.476 mmol) in dichloromethane (3 mL), and the mixture was stirred at 25 °C for 1 h. After the reaction was completed, the reaction liquid was adjusted to pH > 7 with an aqueous Na₂CO₃ solution, and the mixture was extracted with ethyl acetate (5 mL × 3). The combined organic phase was dried over Na₂SO₄, filtered, and concentrated under reduced pressure, and the resulting crude product was purified by silica gel column chromatography (dichloromethane:methanol = 20:1) to give 1-(2-methoxy-5-(4-((2-(piperidin-4-yl)ethoxy)methyl)piperidine-1-carbonyl)phenyl)dihydropyrimidine-2,4(1*H*,3*H*)-dione.

LC-MS: (ESI, m/z): [M+H]⁺ = 473.2.

### Step 3: preparation of (R)-1-(5-(4-((2-(1-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-d]pyrimidin-2-yl)oxy)methyl) cyclopropyl)methyl)piperidin-4-yl)ethoxy)methyl)piperidine-1-carbonyl)-2-methoxyphenyl)dihydropyrimidine-2,4(1H,3H)-dione

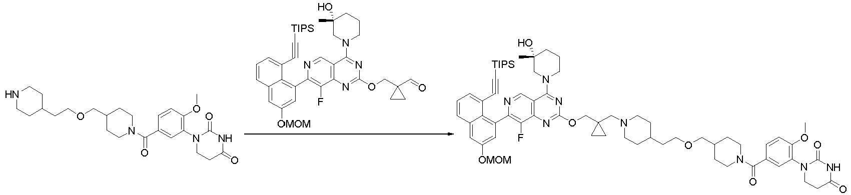

Tetraisopropyl titanate (293 mg, 1.03 mmol) was added to a solution of 1-(2-methoxy-5-(4-((2-(piperidin-4-yl)ethoxy)methyl)piperidine-1-carbonyl)phenyl)dihydropyrimidine-2,4(1*H*,3*H*)-dione (150 mg, 0.25 mmol) and (*R*)-1-(((8-fluoro-4-(3-hydroxy-3-methylpiperidin-1-yl)-7-(3-(methoxymethoxy)-8-((triisopropylsilyl) ethynyl)naphthalen-1-yl)pyrido[4,3-*d*]pyrimidin-2-yl)oxy)methyl)cyclopropane-1-carbaldehyde (117 mg, 0.248 mmol) in dichloromethane (2 mL) at room temperature, and the mixture was stirred at 25 °C for 16 h. Sodium triacetoxyborohydride (137 mg, 0.619 mmol) was added to the mixture at room temperature, and the mixture was then reacted at 25 °C for another 3 h. Methanol (5 mL) was added to the reaction liquid to quench the reaction, and the mixture was concentrated under reduced pressure at 40 °C. The resulting crude product was purified by a preparative thin-layer chromatography plate (dichloromethane:methanol = 10:1, 0.1% NH₃) to give (*R*)*-1-(5-(4-((2-(1-((1-(((8-fluoro-4-(3-hydroxy-3-methylpiperidin-1-yl)-7-(3-*(methoxymethoxy)-8-((triisopropylsilyl)ethynyl)naphthalen-1-yl)pyrido[4,3-*d*]pyrimidin-2-yl)oxy)methyl)cyclopropyl)methyl) piperidin-4-yl)ethoxy)methyl)piperidine-1-carbonyl)-2-methoxyphenyl)dihydropyrimidine-2,4(1*H*,3*H*)-dione.

LC-MS: (ESI, m/z): [M/2+H]⁺ = 592.5.

### Step 4: preparation of (R)-1-(5-(4-((2-(1-((1-(((7-(8-ethynyl-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperidin-4-yl)ethoxy)methyl)piperidine-1-carbonyl)-2-methoxyphenyl)dihydropyrimidine-2,4(1H,3H)-dione

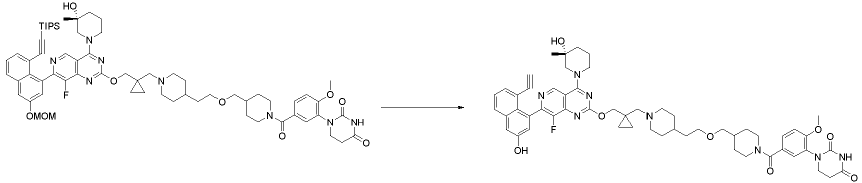

CsF (64 mg, 0.423 mmol) was added to a solution of (*R*)-1-(5-(4-((2-(1-((1-(((8-fluoro-4-(3-hydroxy-3-methylpiperidin-1-yl)-7-(3-(methoxymethoxy)-8-((triisopropylsilyl)ethynyl)naphthalen-1-yl)pyrido[4,3-*d*]pyrimidin-2-yl)oxy)methyl)cyclopropyl)methyl)piperidin-4-yl)ethoxy)methyl)piperidine-1-carbonyl)-2-methoxyphenyl)dihydropyrimidine-2,4(1*H*,3*H*)-dione (100 mg, 0.085 mmol) in DMF (1 mL), and the mixture was stirred at room temperature for 0.5 h. Water (2 mL) was added to the reaction liquid, and the mixture was extracted with ethyl acetate (5 mL × 3). The organic phases were combined, dried over anhydrous sodium sulfate, and concentrated under reduced pressure. The intermediate obtained by concentration was dissolved in 1,4-dioxane (2 mL), and HCl/1,4-dioxane (6 N, 1 mL) was then added. The mixture was reacted at room temperature for 0.5 h. The reaction liquid was adjusted to pH > 7 with an aqueous Na₂CO₃ solution and extracted with ethyl acetate (5 mL × 3). The combined organic phase was dried over Na₂SO₄, filtered, and concentrated under reduced pressure, and the resulting crude product was purified by preparative high-performance liquid chromatography to give (*R*)-1-(5-(4-((2-(1-((1-(((7-(8-ethynyl-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl) methyl)piperidin-4-yl)ethoxy)methyl)piperidine-1-carbonyl)-2-methoxyphenyl)dihydropyrimidine-2,4(1H,3H)-dione.

LC-MS: (ESI, m/z): [M/2+H]⁺ = 492.3.

¹H NMR (400 MHz, DMSO-*d*₆) δ 10.33 (s, 1H), 10.14 (d, *J* = 4.0 Hz, 1H), 9.12 (d, *J* = 67.9 Hz, 1H), 7.88 (*d, J =* 6.6 Hz, 1H), 7.47 - 7.39 (m, 2H), 7.36 - 7.30 (m, 3H), 7.18 - 7.10 (m, 2H), 4.74 (d, *J* = 37.6 Hz, 1H), 4.39 - 4.20 (m, 3H), 4.12 - 3.89 (m, 1H), 3.84 (s, 3H), 3.64 - 3.53 (m, 4H), 3.40 - 3.34 (m, 1H), 3.28 - 3.15 (m, 4H), 3.12 - 2.97 (m, 1H), 2.95 - 2.88 (m, 2H), 2.72 - 2.62 (m, 2H), 2.38 - 2.20 (m, 4H), 1.88 - 1.72 (m, 4H), 1.71 - 1.49 (m, 8H), 1.42 - 1.34 (m, 2H), 1.25 - 1.06 (m, 8H), 0.66 - 0.60 (m, 2H), 0.42 - 0.36 (m, 2H).

### Example 39: 1-(5-(9-((4-((1-(((7-(8-ethynyl-3-hydroxynaphthalen-1-yl)-8-fluoro-4-(1,4-oxazepan-4-yl)pyrido [4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of (1-(((8-fluoro-7-(3-(methoxymethoxy)-8-((triisopropylsilyl)ethynyl)naphthalen-1-yl)-4-(1,4-oxazepan-4-yl)pyrido[4,3-d]pyrimidin-2-yl)oxy)methyl)cyclopropyl)methanol

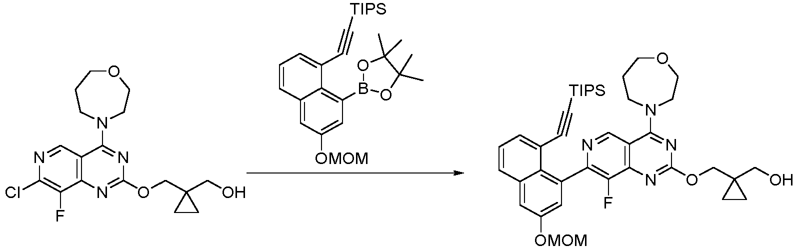

Anhydrous cesium carbonate (980 mg, 3.020 mmol) and cataCXium(R) A Pd G3 (150 mg, 0.204 mmol) were added to a mixed solution of (1-(((7-chloro-8-fluoro-4-(1,4-oxazepan-4-yl)pyrido[4,3-*d*]pyrimidin-2-yl)oxy)methyl)cyclopropyl)methanol (400 mg, 1.044 mmol) and triisopropyl ((6-(methoxymethoxy)-8-(4,4,5,5-tetramethyl-1,3,2-dioxaborolan-2-yl)naphthalen-1-yl)ethynyl)silane (590 mg, 1.20 mmol) in 1,4-dioxane (8 mL) and water (1 mL) at room temperature. After the system was degassed, the mixture was stirred at 85 °C for 16 h under nitrogen atmosphere. After the reaction was completed, the mixture was concentrated under reduced pressure at 40 °C, and the resulting crude product was purified by silica gel thin-layer chromatography (dichloromethane:methanol = 10:1) to give (1-(((8-fluoro-7-(3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl)cyclopropyl)methanol.

LC-MS: (ESI, m/z): [M+H]⁺ = 715.4.

### Step 2: preparation of 1-(((8-fluoro-7-(3-(methoxymethoxy)-8-((triisopropylsilyl)ethynyl)naphthalen-1-yl)-4-(1,4-oxazepan-4-yl)pyrido[4,3-d]pyrimidin-2-yl)oxy)methyl)cyclopropane-1-carbaldehyde

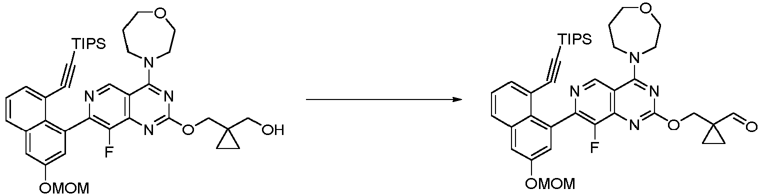

A solution of (1-(((8-fluoro-7-(3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl)cyclopropyl)methanol (510 mg, 0.71 mmol) in dichloromethane (6 mL) was cooled to 0 °C from room temperature, and Dess-Martin reagent (605 mg, 1.43 mmol) was added. The mixture was stirred at 25 °C for 4 h. After the reaction was completed, the mixture was poured into water (20 mL) and extracted with dichloromethane (20 mL × 2). The organic phases were combined, washed with saturated sodium chloride (20 mL), dried over anhydrous sodium sulfate, and concentrated under reduced pressure. The resulting crude product was purified by silica gel thin-layer chromatography (dichloromethane:methanol = 20:1) to give 1-(((8-fluoro-7-(3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl)cyclopropane-1-carbaldehyde.

LC-MS: (ESI, m/z): [M+H]⁺ = 713.4.

### Step 3: preparation of 1-(5-(9-((4-((1-(((8-fluoro-7-(3-(methoxymethoxy)-8-((triisopropylsilyl)ethynyl) naphthalen-1-yl)-4-(1,4-oxazepan-4-yl)pyrido[4,3-d]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1H,3H)-dione

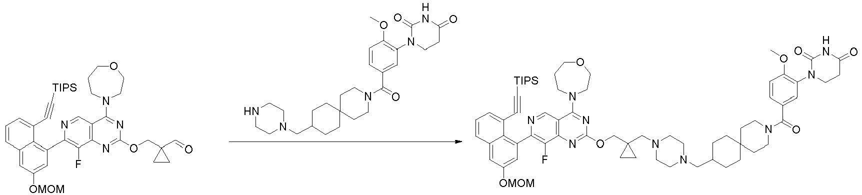

Tetraisopropyl titanate (305 mg, 1.05 mmol) was added to a solution of 1-(((8-fluoro-7-(3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl)
cyclopropane-1-carbaldehyde (150 mg, 0.21 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (125 mg, 0.25 mmol) in dichloromethane (2 mL) at room temperature, and the mixture was stirred at 25 °C for 16 h. Sodium triacetoxyborohydride (140 mg, 0.63 mmol) was added to the mixture at room temperature, and the mixture was reacted at 25 °C for another 3 h. Methanol (5 mL) was added to the reaction liquid to quench the reaction, and the mixture was concentrated under reduced pressure at 40 °C. The resulting crude product was purified by a preparative thin-layer chromatography plate (dichloromethane:methanol = 10:1, 0.1% NH₃) to give 1-(5-(9-((4-((1-(((8-fluoro-7-(3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 598.4.

### Step 4: preparation of 1-(5-(9-((4-((1-(((7-(8-ethynyl-3-hydroxynaphthalen-1-yl)-8-fluoro-4-(1,4-oxazepan-4-yl)pyrido[4,3-d]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1H,3H)-dione

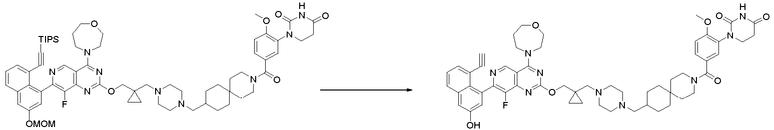

CsF (120 mg, 0.628 mmol) was added to a solution of 1-(5-(9-((4-((1-(((8-fluoro-7-(3-(methoxymethoxy)-8-((triisopropylsilyl)ethynyl)naphthalen-1-yl)-4-(1,4-oxazepan-4-yl)pyrido[4,3-*d*]pyrimidin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1*H*,3*H*)-dione (150 mg, 0.126 mmol) in DMF (2 mL), and the mixture was stirred at room temperature for 0.5 h. Water (2 mL) was added to the reaction liquid, and the mixture was then extracted with ethyl acetate (5 mL × 3). The organic phases were combined, dried over anhydrous sodium sulfate, and concentrated under reduced pressure. The intermediate obtained by concentration was dissolved in 1,4-dioxane (2 mL), and HCl/1,4-dioxane (6 N, 1 mL) was then added. The mixture was reacted at room temperature for 0.5 h. The reaction liquid was adjusted to pH > 7 with an aqueous Na₂CO₃ solution, and the mixture was extracted with ethyl acetate (5 mL × 3). The combined organic phase was dried over Na₂SO₄, filtered, concentrated under reduced pressure, and purified by preparative high-performance liquid chromatography to give 1-(5-(9-((4-((1-(((7-(8-ethynyl-3-hydroxynaphthalen-1-yl)-8-fluoro-4-(1,4-oxazepan-4-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 497.7.

¹H NMR (400 MHz, DMSO-*d*₆) δ 10.33 (s, 1H), 10.13 (s, 1H), 9.08 (s, 1H), 7.88 (d, *J* = 7.9 Hz, 1H), 7.50-7.25 (m, 5H), 7.20-7.05 (m, 2H), 4.27 (s, 2H), 4.17-4.06 (m, 3H), 3.97-3.86 (m, 2H), 3.84 (s, 3H), 3.78-3.71 (m, 2H), 3.65 (s, 1H), 3.59 (t, *J* = 6.5 Hz, 2H), 2.72-2.63 (m, 2H), 2.40-2.20 (m, 11H), 2.15-1.96 (m, 6H), 1.72-1.60 (m, 2H), 1.55-1.35 (m 6H), 1.32-1.20 (m, 2H), 1.15-0.90 (m, 5H), 0.66 - 0.58 (m, 2H), 0.43 - 0.37 (m, 2H).

### Example 40: (R)-1-(5-(9-((4-((1-(((7-(3-chloro-2-cyclopropyl-5-hydroxyphenyl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of (R)-1-(2,7-dichloro-8-fluoropyrido[4,3-d]pyrimidin-4-yl)-3-methylpiperidin-3-ol

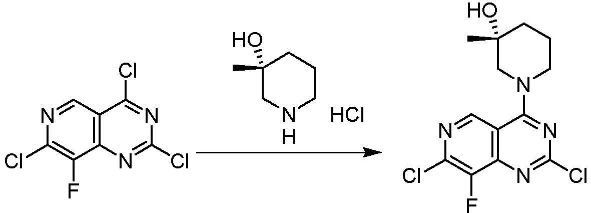

TEA (4.2 g, 0.042 mmol) was added to a solution of 2,4,7-trichloro-8-fluoropyrido[4,3-*d*]pyrimidine (3.6 g, 0.014 mmol) in THF (50 mL) at -78 °C, and the mixture was stirred for 30 min. *(R)-3-*Methylpiperidin-3-ol hydrochloride (1.6 g, 0.011 mmol) was added, and the mixture was stirred for 4 h. After the reaction was completed, the mixture was slowly warmed to room temperature. H₂O (50 mL) was added, and the mixture was extracted with EtOAc (50 mL × 3). The organic phases were combined, dried over anhydrous sodium sulfate, and concentrated under reduced pressure, and the concentrate was purified by silica gel column chromatography (PE:EtOAc = 5:1) to give (*R*)-1-(2,7-dichloro-8-fluoropyrido[4,3-*d*]pyrimidin-4-yl)-3-methylpiperidin-3-ol.

LC-MS: (ESI, m/z): [M+H]⁺ = 330.9.

### Step 2: preparation of (R)-1-(7-chloro-8-fluoro-2-((1-(hydroxymethyl)cyclopropyl)methoxy)pyrido[4,3-d]pyrimidin-4-yl)-3-methylpiperidin-3-ol

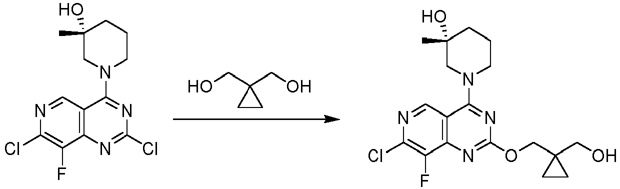

Cs₂CO₃ (9.8 g, 0.03 mmol) and DABCO (0.56 g, 0.005 mmol) were added to a solution of *(R)-1-*(2,7-dichloro-8-fluoropyrido[4,3-*d*]pyrimidin-4-yl)-3-methylpiperidin-3-ol (3.6 g, 0.01 mmol) and cyclopropane-1,1-diyldimethanol (2.0 g, 0.02 mmol) in ACN (50 mL), and the mixture was stirred at room temperature for 30 min. After the reaction was completed, the mixture was filtered and concentrated under reduced pressure, and the concentrate was purified by silica gel column chromatography (DCM:MeOH = 50:1) to give (*R*)-1-(7-chloro-8-fluoro-2-((1-(hydroxymethyl)cyclopropyl)methoxy)pyrido[4,3-*d*]pyrimidin-4-yl)-3-methylpiperidin-3-ol.

LC-MS: (ESI, m/z): [M+H]⁺ = 397.1.

### Step 3: preparation of (R)-1-(7-(3-chloro-2-cyclopropyl-5-hydroxyphenyl)-8-fluoro-2-((1-(hydroxymethyl) cyclopropyl)methoxy)pyrido[4,3-d]pyrimidin-4-yl)-3-methylpiperidin-3-ol

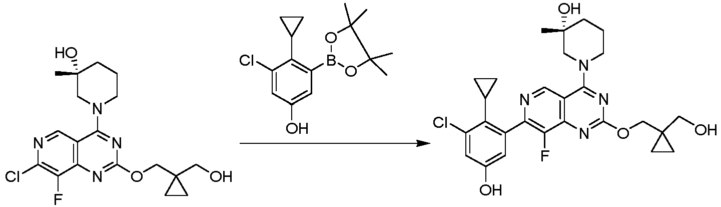

3-Chloro-4-cyclopropyl-5-(4,4,5,5-tetramethyl-1,3,2-dioxaborolan-2-yl)phenol (220 mg, 0.75 mmol), Pd(dppf)Cl₂ (73 mg, 0.10 mmol), and cesium carbonate (489 mg, 1.50 mmol) were added to a mixed solution of (*R*)-1-(7-chloro-8-fluoro-2-((1-(hydroxymethyl)cyclopropyl)methoxy)pyrido[4,3-*d*]pyrimidin-4-yl)-3-methylpiperidin-3-ol (200 mg, 0.50 mmol) in 1,4-dioxane (12 mL) and H₂O (2 mL). The mixture was reacted at 85 °C for 16 h under nitrogen atmosphere. Water (10 mL) was added to the reaction liquid, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 50:1) to give (R)-1-(7-(3-chloro-2-cyclopropyl-5-hydroxyphenyl)-8-fluoro-2-((1-(hydroxymethyl)cyclopropyl)methoxy)pyrido[4,3-*d*]pyrimidin-4-yl)-3-methylpiperidin-3-ol.

LC-MS: (ESI, m/z): [M+H]⁺ = 529.1.

### Step 4: preparation of (R)-1-(((7-(3-chloro-2-cyclopropyl-5-hydroxyphenyl)-8-fluoro-4-(3-hydroxy-3-methylpiperidin-1-yl)pyrido[4,3-d]pyrimidin-2-yl)oxy)methyl)cyclopropane-1-carbaldehyde

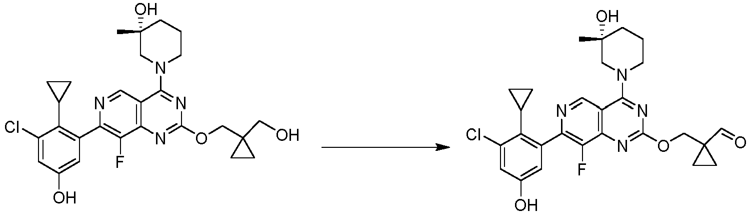

TPAP (3.8 mg, 0.011 mmol) and NMO (28 mg, 0.24 mmol) were added to a solution of (*R*)-1-(7-(3-chloro-2-cyclopropyl-5-hydroxyphenyl)-8-fluoro-2-((1-(hydroxymethyl)cyclopropyl)methoxy)pyrido[4,3-*d*]pyrimidin-4-yl)-3-methylpiperidin-3-ol (60 mg, 0.11 mmol) in DCM (1 mL), and the mixture was stirred at room temperature for 2 h under nitrogen atmosphere. After the reaction was completed, water (10 mL) was added to the reaction liquid, and the mixture was extracted with DCM (10 mL × 3). The organic phases were combined, dried over anhydrous sodium sulfate, and concentrated under reduced pressure. The concentrate was purified by silica gel column chromatography (DCM:MeOH = 50:1) to give (*R*)-1-(((7-(3-chloro-2-cyclopropyl-5-hydroxyphenyl)-8-fluoro-4-(3-hydroxy-3-methylpiperidin-1-yl)pyrido[4,3-*d*]pyrimidin-2-yl)oxy)methyl)cyclopropane-1-carbaldehyde.

LC-MS: (ESI, m/z): [M+H]⁺ = 527.2.

### Step 5: preparation of (R)-1-(5-(9-((4-((1-(((7-(3-chloro-2-cyclopropyl-5-hydroxyphenyl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

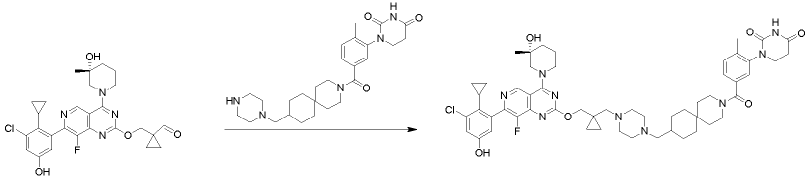

1-(2-Methyl-5-(9-(piperazin-1-ylmethyl)-3-azaspiro[5.5]undecane-3-carbonyl)phenyl)dihydropyrimidine-2,4(1*H*,3*H*)-dione (34 mg, 0.071 mmol), AcOH (0.1 mL), and tetraisopropyl titanate (68 mg, 0.24 mmol) were added to a solution of (*R*)-1-(((7-(3-chloro-2-cyclopropyl-5-hydroxyphenyl)-8-fluoro-4-(3-hydroxy-3-methylpiperidin-1-yl)pyrido[4,3-*d*]pyrimidin-2-yl)oxy)methyl)cyclopropane-1-carbaldehyde (25 mg, 0.047 mmol) in DCM (1 mL), and the mixture was stirred at room temperature for 2 h. The reaction liquid was cooled to 0 °C, and NaBH(OAc)₃ (30 mg, 0.14 mmol) was added. The mixture was reacted at room temperature for another 4 h. The mixture was concentrated and purified by silica gel column chromatography (DCM:MeOH = 100:1 to 20:1) to give (*R*)-1-(5-(9-((4-((1-(((7-(3-chloro-2-cyclopropyl-5-hydroxyphenyl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 496.8.

1H NMR (400 MHz, CD₃OD) δ 9.19 (s, 1H), 7.40 (d, *J* = 7.8 Hz, 1H), 7.35 - 7.26 (m, 2H), 6.95 (d, *J* = 2.5 Hz, 1H), 6.79 (d, *J* = 2.5 Hz, 1H), 4.57 - 4.42 (m, 3H), 4.21 (d*, J =* 13.6 Hz, 1H), 3.92 - 3.77 (m, 1H), 3.75 - 3.56 (m, 4H), 3.48 - 3.41 (m, 2H), 2.91 - 2.78 (m, 2H), 2.59 - 2.37 (m, 7H), 2.31 (s, 3H), 2.23 - 2.07 (m, 4H), 1.90 - 1.71 (m, 6H), 1.62 - 1.43 (m, 6H), 1.34 - 1.27 (m, 9H), 1.18 - 1.12 (m, 2H), 0.73 - 0.67 (m, 2H), 0.67 - 0.58 (m, 2H), 0.52 - 0.47 (m, 2H), 0.15 - 0.02 (m, 2H).

### Example 41: (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of (R)-1-(5-(9-((4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)quinazolin-2-yl)oxy)methyl)cyclopropyl) methyl)piperazin-1-yl)methyl)-3-azaspiro[5.5]undecane-3-carbonyl)-2-methylphenyl)dihydropyrimidine-2,4(1H,3H)-dione

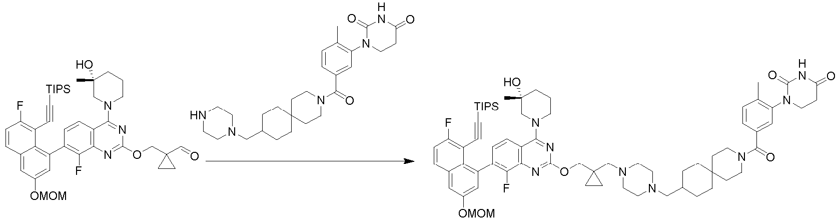

Tetraisopropyl titanate (190 mg, 0.67 mmol) was added to a stirred solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropane-1-carbaldehyde (100 mg, 0.134 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (84 mg, 0.174 mmol) in DCM/AcOH (1 mL/0.1 mL), and the mixture was stirred at 30 °C for 2 h. NaBH(OAc)₃ (89 mg, 0.402 mmol) was added to the mixture at room temperature, and the resulting mixture was stirred for 2 h. The mixture was concentrated and purified by silica gel column chromatography (DCM:MeOH = 100:1 to 15:1) to give (*R*)-1-(5-(9-((4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)quinazolin-2-yl)oxy)methyl)cyclopropyl) methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 605.5.

### Step 2: preparation of (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

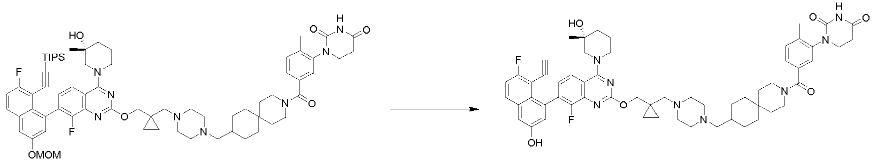

Cesium fluoride (63 mg, 0.415 mmol) was added to a stirred solution of (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H,*3*H*)-dione (100 mg, 0.083 mmol) in DMF (2 mL), and the mixture was stirred at room temperature for 30 min. The mixture was extracted with ethyl acetate and water. The organic phase was concentrated. A mixture of 1,4-dioxane (1 mL) and HCl/1,4-dioxane (6 N, 0.5 mL) was then added to the mixture at room temperature. The mixture was reacted for 0.5 h. The reaction liquid was directly concentrated under reduced pressure, and the resulting crude product was purified by preparative high-performance liquid chromatography to give (*R*)-1-(5-(9-((4-((1-(((7-(8-ethynyl-7-fluoro-3-hydroxynaphthalen-1-yl)-8-fluoro-4-(3-hydroxy-3-methylpiperidin-1-yl)quinazolin-2-yl)oxy)methyl) cyclopropyl)methyl)piperazin-1-yl)methyl)-3-azaspiro[5.5]undecane-3-carbonyl)-2-methylphenyl) dihydropyrimidine-2,4(1*H*,3*H*)-dione.

LC-MS: (ESI, m/z): [M/2+H]⁺ = 505.3.
¹H NMR (400 MHz, DMSO-*d*₆) δ 10.37 (s, 1H), 10.12 (s, 1H), 7.96 (dd, *J* = 9.1, 6.0 Hz, 1H), 7.81 (dd,*J* = 34.1, 8.4 Hz, 1H), 7.49 - 7.42 (m, 1H), 7.37 - 7.29 (m, 3H), 7.26 - 7.16 (m, 2H), 7.08 (dd, *J =* 12.3, 2.4 Hz, 1H), 4.64 (d, *J* = 23.0 Hz, 1H), 4.27 - 4.17 (m, 2H), 4.14 - 3.92 (m, 1H), 3.91 - 3.71 (m, 3H), 3.63 - 3.45 (m, 3H), 3.44 - 3.34 (m, 1H), 3.32 - 3.26 (m, 3H), 2.84 - 2.65 (m, 2H), 2.47 - 2.23 (m, 9H), 2.21 (s, 3H), 2.10 - 1.97 (m, 3H), 1.73 - 1.58 (m, 5H), 1.53 - 1.22 (m, 8H), 1.16 (d, *J =* 7.2 Hz, 3H), 1.12 - 0.93 (m, 4H), 0.66 - 0.58 (m, 2H), 0.44 - 0.34 (m, 2H).

### Example 42: (R)-1-(5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of (R)-1-(7-(8-ethynyl-7-fluoro-3-(methoxymethoxy)naphthalen-1-yl)-8-fluoro-2-((1-(hydroxymethyl)cyclopropyl)methoxy)pyrido[4,3-d]pyrimidin-4-yl)-3-methylpiperidin-3-ol

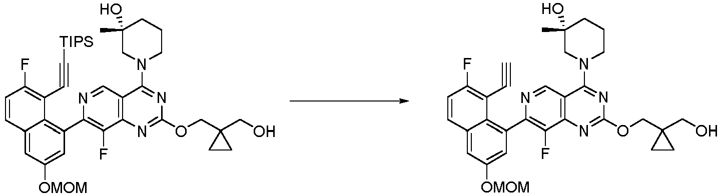

CsF (561 mg, 3.69 mmol) was added to a solution of (*R*)-1-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido[4,3-*d*]pyrimidin-4-yl)-3-methylpiperidin-3-ol (550 mg, 0.738 mmol) in DMF (5 mL), and the mixture was stirred at room temperature for 0.5 h. Water (5 mL) was added to the reaction liquid, and the mixture was extracted with ethyl acetate (20 mL × 3). The organic phases were combined, dried over anhydrous sodium sulfate, and concentrated under reduced pressure. The resulting crude product was purified by silica gel column chromatography (dichloromethane:methanol = 100:1 to 20:1) to give (*R*)-1-(7-(8-ethynyl-7-fluoro-3-(methoxymethoxy)naphthalen-1-yl)-8-fluoro-2-((1-(hydroxymethyl)cyclopropyl)methoxy)pyrido[4,3-*d*]pyrimidin-4-yl)-3-methylpiperidin-3-ol.

LC-MS: (ESI, m/z): [M+H]⁺ = 591.3.

### Step 2: preparation of (R)-1-(7-(8-ethyl-7-fluoro-3-(methoxymethoxy)naphthalen-1-yl)-8-fluoro-2-((1-(hydroxymethyl)cyclopropyl)methoxy)pyrido[4,3-d]pyrimidin-4-yl)-3-methylpiperidin-3-ol

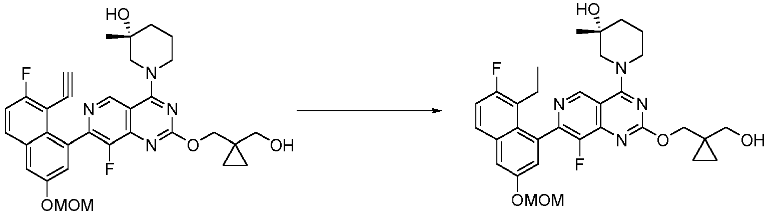

Pd/C (150 mg) was added to a solution of (*R*)-1-(7-(8-ethynyl-7-fluoro-3-(methoxymethoxy)naphthalen-1-yl)-8-fluoro-2-((1-(hydroxymethyl)cyclopropyl)methoxy)pyrido[4,3-*d*]pyrimidin-4-yl)-3-methylpiperidin-3-ol (300 mg, 0.508 mmol) in anhydrous methanol (4 mL), and hydrogen was introduced through. The mixture was stirred at 25 °C for 16 h. After the reaction was completed, the mixture was poured into water (10 mL) and extracted with ethyl acetate (20 mL × 2). The organic phases were combined, washed with saturated sodium chloride (20 mL), dried over anhydrous sodium sulfate, and concentrated under reduced pressure. The resulting crude product was purified by silica gel column chromatography (dichloromethane:methanol = 20:1) to give (R)-1-(7-(8-ethyl-7-fluoro-3-(methoxymethoxy)naphthalen-1-yl)-8-fluoro-2-((1-(hydroxymethyl) cyclopropyl)methoxy)pyrido[4,3-*d*]pyrimidin-4-yl)-3-methylpiperidin-3-ol.

LC-MS: (ESI, m/z): [M+H]⁺ = 595.3.

### Step 3: preparation of (R)-1-(((7-(8-ethyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropane-1-carbaldehyde

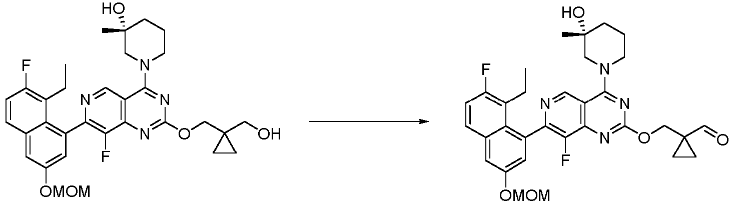

A solution of (*R*)-1-(7-(8-ethyl-7-fluoro-3-(methoxymethoxy)naphthalen-1-yl)-8-fluoro-2-((1-(hydroxymethyl) cyclopropyl)methoxy)pyrido[4,3-*d*]pyrimidin-4-yl)-3-methylpiperidin-3-ol (290 mg, 0.488 mmol) in dichloromethane (4 mL) was cooled to 0 °C from room temperature, and Dess-Martin reagent (430 mg, 1.014 mmol) was added. The mixture was stirred at 25 °C for 4 h. After the reaction was completed, the reaction liquid was poured into water (20 mL) and extracted with dichloromethane (20 mL × 2). The organic phases were combined, washed with saturated sodium chloride (20 mL), dried over anhydrous sodium sulfate, and concentrated under reduced pressure. The resulting crude product was purified by a preparative silica gel thin-layer chromatography plate (dichloromethane:methanol = 20:1) to give (*R*)-1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde.

LC-MS: (ESI, m/z): [M+H]⁺ = 593.3.

### Step 4: preparation of (R)-1-(5-(9-((4-((1-(((7-(8-ethyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1H,3H)-dione

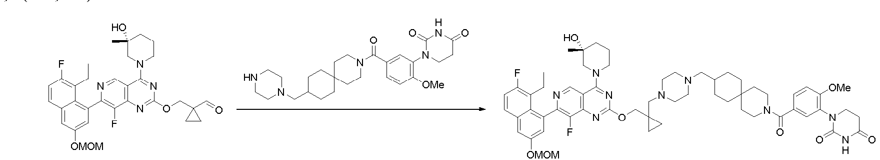

Tetraisopropyl titanate (305 mg, 1.05 mmol) was added to a solution of (*R*)-1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropane-1-carbaldehyde (150 mg, 0.253 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (151 mg, 0.304 mmol) in dichloromethane (2 mL) at room temperature, and the mixture stirred at 25 °C for 16 h. Sodium triacetoxyborohydride (140 mg, 0.63 mmol) was added to the mixture at room temperature, and the mixture was reacted at 25 °C for another 3 h. The reaction liquid was concentrated under reduced pressure at 40 °C. The resulting crude product was purified by a preparative thin-layer chromatography plate (dichloromethane/methanol = 10:1, 0.1% NH₃) to give (*R*)-1-(5-(9-((4-((1-(((7-(8-ethyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yloxy)methyl)cyclopropyl)methyl)piperazin-1-yl)methyl)-3-azaspiro[5.5]undecane-3-carbonyl)-2-methoxyphenyl)
dihydropyrimidine-2,4(1*H*,3*H*)-dione.

LC-MS: (ESI, m/z): [M/2+H]⁺ = 537.9.

### Step 5: preparation of (R)-1-(5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

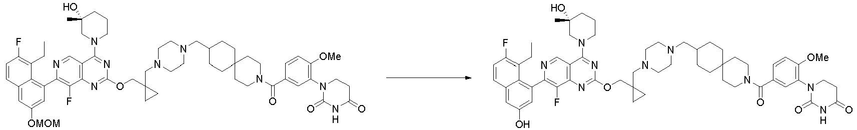

(*R*)-1-(5-(9-((4-((1-(((7-(8-Ethyl-7-fluoro-3-(methoxymethoxy)naphthalen-)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione (130 mg, 0.121 mmol) was dissolved in 1,4-dioxane (2 mL). HCl/1,4-dioxane (6 N, 1 mL) was added, and the mixture was reacted at room temperature for 0.5 h. The reaction liquid was adjusted to pH > 7 with an aqueous Na₂CO₃ solution and extracted with ethyl acetate (15 mL × 3). The combined organic phase was dried over Na₂SO₄, filtered, and concentrated under reduced pressure, and the resulting crude product was purified by preparative high-performance liquid chromatography to give (R)-1-(5-(9-((4-((1-(((7-(8-ethyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy) methyl)cyclopropyl)methyl)piperazin-1-yl)methyl)-3-azaspiro[5.5]undecane-3-carbonyl)-2-methoxyphenyl)
dihydropyrimidine-2,4(1*H*,3*H*)-dione.

LC-MS: (ESI, m/z): [M/2+H]⁺ = 515.8.

¹H NMR (400 MHz, CD₃OD) δ 9.20 (d, *J =* 6.7 Hz, 1H), 7.72 - 7.57 (m, 1H), 7.43 (dd, *J =* 8.5, 2.1 Hz, 1H), 7.38 (d, *J* = 2.1 Hz, 1H), 7.29 (d, *J* = 2.6 Hz, 1H), 7.27 - 7.21 (m, 1H), 7.18 (d, *J* = 8.6 Hz, 1H), 7.07 - 7.03 (m, 1H), 4.54 - 4.44 (m, 2H), 4.42 - 4.34 (m, 1H), 4.30 - 4.18 (m, 1H), 3.92 (s, 3H), 3.77 - 3.56 (m, 5H), 3.55 - 3.41 (m, 3H), 2.80 (t, *J =* 6.7 Hz, 2H), 2.60 - 2.31 (m, 9H), 2.25 - 2.05 (m, 5H), 1.84 - 1.73 (m, 4H), 1.64 - 1.49 (m, 5H), 1.40 - 1.25 (m, 7H), 1.20 - 1.05 (m, 4H), 0.87 - 0.75 (m, 3H), 0.75 - 0.67 (m, 2H), 0.56 - 0.46 (m, 2H).

### Example 43: (R)-1-(2-chloro-5-(4-(4-(4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)but-1-yn-1-yl)piperidine-1-carbonyl)phenyl)dihydropyrimidine-2,4(1H,3H)-dione

### Step 1: preparation of tert-butyl 4-(2,2-dibromovinyl)piperidine-1-carboxylate

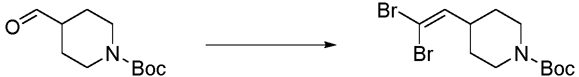

Triphenylphosphine (19.00 g, 72.37 mmol) was added to a solution of *tert*-butyl 4-formylpiperidine-1-carboxylate (12.00 g, 36.185 mmol) in dichloromethane (150 mL) at 0 °C, and the mixture was stirred for 0.5 h under nitrogen atmosphere. Carbon tetrabromide (5.15 g, 24.12 mmol) was then added slowly, and the mixture was stirred at 0 °C for another 1 h. After the reaction was completed, the mixture was filtered to remove the insoluble substances, and the filtrate was concentrated under reduced pressure. The residue was diluted with ethyl acetate (50 mL), poured into water (200 mL), and extracted with ethyl acetate (200 mL × 2). The combined organic phase was washed with saturated brine (100 mL), dried over anhydrous sodium sulfate, filtered, and concentrated under reduced pressure, and the resulting crude product was purified by silica gel column chromatography (petroleum ether:ethyl acetate = 200:1 to 20:1) to give *tert*-butyl 4-(2,2-dibromovinyl)piperidine-1-carboxylate.

LC-MS: (ESI, m/z): [M-56+H]⁺ = 314.0.

### Step 2: preparation of tert-butyl 4-(4-((tetrahydro-2H-pyran-2-yl)oxy)but-1-yn-1-yl)piperidine-1-carboxylate

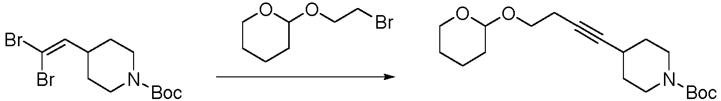

A solution of *n*-butyllithium (2.5 M, 7.5 mL, 18.75 mmol) was added dropwise to a solution of *tert*-butyl 4-(2,2-dibromovinyl)piperidine-1-carboxylate (2.78 g, 7.522 mmol) in anhydrous tetrahydrofuran (100 mL) at -65 °C under nitrogen atmosphere. After the mixture was stirred for 2 h at -65 °C, HMPA (8.09 g, 44.13 mmol) was added, and the mixture was stirred for 10 min. 2-(2-Bromoethoxy)tetrahydro-2*H*-pyran (6.0 g, 28.70 mmol) was then added, and the mixture was reacted for 18 h. After the reaction was completed, the mixture was diluted with petroleum ether (100 mL), poured into water (300 mL), and extracted with ethyl acetate (100 mL × 3). The organic phases were combined, washed with saturated sodium chloride (100 mL), dried over anhydrous sodium sulfate, filtered and concentrated under reduced pressure. The resulting crude product was purified by silica gel column chromatography (petroleum ether:ethyl acetate = 100:1 to 10:1) to give *tert*-butyl 4-(4-((tetrahydro-2*H*-pyran-2-yl)oxy)but-1-yn-1-yl)piperidine-1-carboxylate.

LC-MS: (ESI, m/z): [M+Na]⁺ = 360.3.

### Step 3: preparation of tert-butyl 4-(4-hydroxybut-1-yn-1-yl)piperidine-1-carboxylate

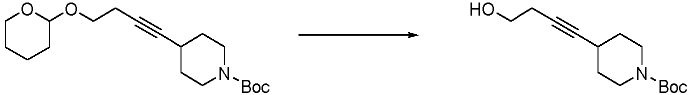

Pyridine 4-methylbenzenesulfonate (0.36 g, 1.43 mmol) was added to a solution of *tert*-butyl 4-(4-((tetrahydro-2*H*-pyran-2-yl)oxy)but-1-yn-1-yl)piperidine-1-carboxylate (1.61 g, 4.77 mmol) in anhydrous methanol (30 mL) at 25 °C, and the mixture was stirred at 40 °C for 18 h. After the reaction was completed, the mixture was concentrated to dryness under reduced pressure. The residue was diluted with ethyl acetate (20 mL), poured into water (100 mL), and extracted with ethyl acetate (100 mL × 3). The organic phases were combined, washed with saturated sodium chloride (100 mL), dried over anhydrous sodium sulfate, filtered and concentrated under reduced pressure. The resulting crude product was purified by silica gel column chromatography (petroleum ether:ethyl acetate = 100:1 to 10:1) to give *tert*-butyl 4-(4-hydroxybut-1-yn-1-yl)piperidine-1-carboxylate.

LC-MS: (ESI, m/z): [M-56+H]⁺ = 198.2

### Step 4: preparation of tert-butyl 4-(4-((methylsulfonyl)oxy)but-1-yn-1-yl)piperidine-1-carboxylate

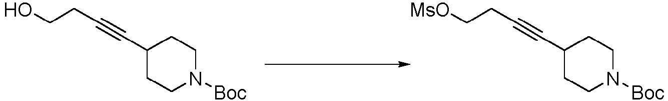

Methanesulfonyl chloride (1.32 g, 11.52 mmol) was added to a solution of *tert*-butyl 4-(4-hydroxybut-1-yn-1-yl)piperidine-1-carboxylate (1.20 g, 4.74 mmol) and triethylamine (2.92 g, 28.85 mmol) in anhydrous dichloromethane (50 mL) at 0 °C under nitrogen atmosphere. The mixture was stirred at 25 °C for 1 h. After the reaction was completed, the mixture was concentrated under reduced pressure to give *tert-*butyl 4-(4-((methylsulfonyl)oxy)but-1-yn-1-yl)piperidine-1-carboxylate.

### Step 5: preparation of tert-butyl 4-(4-(4-benzylpiperazin-1-yl)but-1-yn-1-yl)piperidine-1-carboxylate

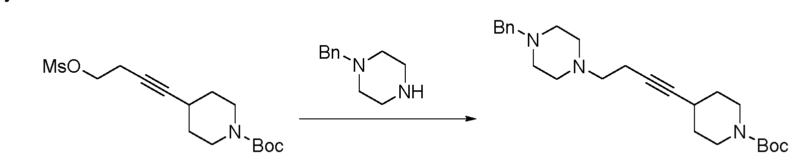

Anhydrous cesium carbonate (5.64 g, 17.307 mmol) was added to a solution of *tert*-butyl 4-(4-((methylsulfonyl)oxy)but-1-yn-1-yl)piperidine-1-carboxylate (1.77 g, 5.34 mmol) and 1-benzylpiperazine (3.05 g, 17.31 mmol) in anhydrous acetonitrile (50 mL), and the mixture was stirred at 60 °C for 18 h. After the reaction was completed, the mixture was poured into water (100 mL) and extracted with ethyl acetate (20 mL × 2). The organic phases were combined, washed with saturated sodium chloride (100 mL), dried over anhydrous sodium sulfate, and concentrated under reduced pressure. The crude product was purified by silica gel column chromatography (petroleum ether:ethyl acetate = 100:1 to 10:1) to give *tert*-butyl 4-(4-(4-benzylpiperazin-1-yl)but-1-yn-1-yl)piperidine-1-carboxylate.

LC-MS: (ESI, m/z): [M+H]⁺ = 412.3

### Step 6: preparation of 1-benzyl-4-(4-(piperidin-4-yl)but-3-yn-1-yl)piperazine

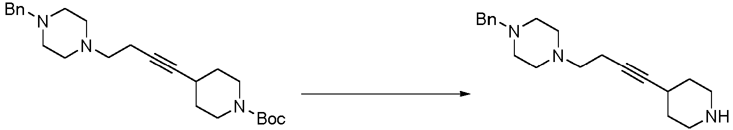

A hydrogen chloride/1,4-dioxane (4.0 M, 9 mL) solution was added to a solution of *tert*-butyl 4-(4-(4-benzylpiperazin-1-yl)but-1-yn-1-yl)piperidine-1-carboxylate (900 mg, 2.19 mmol) in 1,4-dioxane (3 mL) at 25 °C, and the mixture was reacted at 25 °C for 1 h. After the reaction was completed, the mixture was poured into ice water (100 mL) and extracted with ethyl acetate (50 mL), and the aqueous phase was adjusted to pH 9.0 with a 0.1% aqueous sodium hydroxide solution and extracted with dichloromethane (100 mL × 3). The organic phases were combined, washed with saturated sodium chloride (100 mL), dried over anhydrous sodium sulfate, filtered, and concentrated under reduced pressure. A crude product of 1-benzyl-4-(4-(piperidin-4-yl)but-3-yn-1-yl)piperazine was obtained.

LC-MS: (ESI, m/z): [M+H]⁺ = 312.3.

### Step 7: preparation of 1-(5-(4-(4-(4-benzylpiperazin-1-yl)but-1-yn-1-yl)piperidine-1-carbonyl)-2-chlorophenyl)dihydropyrimidine-2,4(1H,3H)-dione

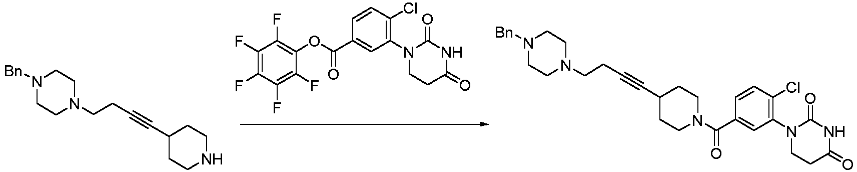

*N,N*-diisopropylethylamine (830 mg, 6421.20 µmol) and pentafluorophenyl 4-chloro-3-(2,4-dioxotetrahydropyrimidin-1(2*H*)-yl)benzoate (293 mg, 674.23 µmol) were added to a solution of 1-benzyl-4-(4-(piperidin-4-yl)but-3-yn-1-yl)piperazine (200 mg, 642.12 µmol) in *N*,*N*-dimethylformamide (10 mL) at 25 °C, and the mixture was reacted at 25 °C for 18 h. After the reaction was completed, the mixture was poured into water (100 mL) and extracted with ethyl acetate (20 mL × 2). The organic phases were combined, washed with saturated sodium chloride (100 mL), dried over anhydrous sodium sulfate, filtered and concentrated under reduced pressure. The resulting crude product was purified by a preparative thin-layer chromatography plate (dichloromethane:methanol = 10:1, 0.1% NH₃) to give 1-(5-(4-(4-(4-benzylpiperazin-1-yl)but-1-yn-1-yl)piperidine-1-carbonyl)-2-chlorophenyl)dihydropyrimidine-2,4(1*H*,3*H*)-dione.

LC-MS: (ESI, m/z): [M+H]⁺ = 562.2.

### Step 8: preparation of 1-(2-chloro-5-(4-(4-(piperazin-1-yl)but-1-yn-1-yl)piperidine-1-carbonyl)phenyl) dihydropyrimidine-2,4(1H,3H)-dione

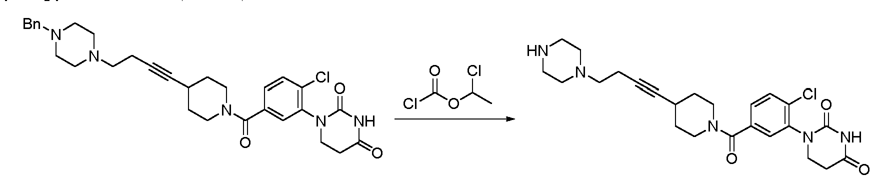

1- Chloroethyl carbonochloridate (270 mg, 1891.98 mmol) was added to a solution of 1-(5-(4-(4-(4-benzylpiperazin-1-yl)but-1-yn-1-yl)piperidine-1-carbonyl)-2-chlorophenyl)dihydropyrimidine-2,4(1*H*,3*H*)-dione (355 mg, 630.66 µmol) in dichloromethane (20 mL) at 25 °C, and the mixture was reacted at 30 °C for 1 h. After the reaction was completed, the mixture was concentrated under reduced pressure at 30 °C, and the resulting crude product was diluted with methanol (30 mL) and stirred at 50 °C for 1 h. After the reaction was completed, the mixture was concentrated under reduced pressure, and the residue was dissolved in water (1 mL), adjusted to pH 9.0 with a 0.1% aqueous sodium hydroxide solution, and concentrated under reduced pressure. The residue was dissolved in dichloromethane/methanol (10/1), the mixture was filtered to remove the insoluble substances, and the filtrate was concentrated under reduced pressure to give 1-(2-chloro-5-(4-(4-(piperazin-1-yl)but-1-yn-1-yl)piperidine-1-carbonyl)phenyl)dihydropyrimidine-2,4(1*H*,3*H*)-dione.

LC-MS: (ESI, m/z): [M+H]⁺ = 472.2.

### Step 9: preparation of (R)-1-(2-chloro-5-(4-(4-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperazin-1-yl)but-1-yn-1-yl)piperidine-1-carbonyl)phenyl)dihydropyrimidine-2,4(1H,3H)-dione

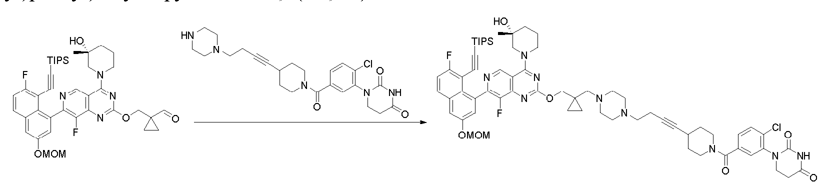

Tetraisopropyl titanate (229 mg, 805.40 µmol) and acetic acid (0.01 mL) were added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde (120 mg, 161.08 µmol) and 1-(2-chloro-5-(4-(4-(piperazin-1-yl)but-1-yn-1-yl)piperidine-1-carbonyl)phenyl)
dihydropyrimidine-2,4(1*H*,3*H*)-dione (91 mg, 193.30 µmol) in dichloromethane (10 mL), and the mixture stirred at 25 °C for 2 h. Sodium triacetoxyborohydride (102 mg, 483.24 µmol) was added to the mixture at 25 °C, and the mixture was then reacted at 25 °C for another 18 h. Methanol (10 mL) was added to the reaction liquid to quench the reaction, and the mixture was concentrated under reduced pressure at 40 °C. The resulting crude product was purified by a preparative thin-layer chromatography plate (dichloromethane:methanol = 10:1, 0.1% NH₃) to give (*R*)-1-(2-chloro-5-(4-(4-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)but-1-yn-1-yl)piperidine-1-carbonyl)phenyl)dihydropyrimidine-2,4(1*H*,3*H*)-dione.

LC-MS: (ESI, m/z): [M+H]⁺ = 1200.6.

### Step 10: preparation of (R)-1-(2-chloro-5-(4-(4-(4-((1-(((7-(8-ethynyl-7-fluoro-3-(methoxymethoxy) naphthalen-1-yl)-8-fluoro-4-(3-hydroxy-3-methylpiperidin-1-yl)pyrido[4,3-d]pyrimidin-2-yl)oxy)methyl) cyclopropyl)methyl)piperazin-1-yl)but-1-yn-1-yl)piperidine-1-carbonyl)phenyl)dihydropyrimidine-2,4(1H,3H)-dione

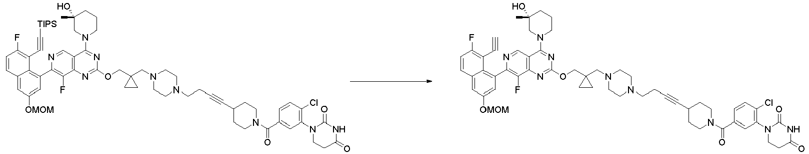

Cesium fluoride (154 mg, 1010.87 µmol) was added to a solution of (*R*)-1-(2-chloro-5-(4-(4-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)but-1-yn-1-yl)piperidine-1-carbonyl)phenyl)dihydropyrimidine-2,4(1*H*,3*H*)-dione (121 mg, 101.09 µmol) in *N,N-*diisopropylethylamine (10 mL), and the mixture was stirred at 25 °C for 1 h. After the reaction was completed, the mixture was poured into water (100 mL) and extracted with ethyl acetate (20 mL × 2). The combined organic phase was sequentially washed with water (100 mL) and saturated sodium chloride (100 mL), dried over anhydrous sodium sulfate, filtered, and concentrated under reduced pressure to give (*R*)-1-(2-chloro-5-(4-(4-(4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)but-1-yn-1-yl)piperidine-1-carbonyl)phenyl)dihydropyrimidine-2,4(1*H*,3*H*)-dione.

LC-MS: (ESI, m/z): [M/2+H]⁺ = 522.8.

### Step 11: preparation of (R)-1-(2-chloro-5-(4-(4-(4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl) methyl)piperazin-1-yl)but-1-yn-1-yl)piperidine-1-carbonyl)phenyl)dihydropyrimidine-2,4(1H,3H)-dione

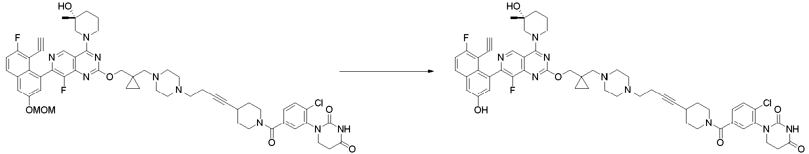

A hydrogen chloride/1,4-dioxane solution (4.0 M, 5 mL) was added to a solution of (*R*)-1-(2-chloro-5-(4-(4-(4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)but-1-yn-1-yl)piperidine-1-carbonyl)phenyl)dihydropyrimidine-2,4(1*H*,3*H*)-dione (100 mg, 96.11 µmol) in 1,4-dioxane (1.5 mL) at 25 °C, and the mixture was reacted at 25 °C for 1 h. After the reaction was completed, the mixture was concentrated under reduced pressure at 30 °C, and a solution of ammonia in methanol (1 mL) was added to the resulting product. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give (R)-1-(2-chloro-5-(4-(4-(4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)but-1-yn-1-yl)piperidine-1-carbonyl)phenyl)dihydropyrimidine-2,4(1*H*,3*H*)-dione.

LC-MS: (ESI, m/z): [M+H]⁺ = 1000.1

¹H NMR (400 MHz, DMSO-*d*₆) δ 10.51 (s, 1H), 10.16 (d, *J =* 4.0 Hz, 1H), 9.15 (d, *J =* 66.0 Hz, 1H), 8.04 - 7.87 (m, 1H), 7.62 (d, *J* = 8.0 Hz, 1H), 7.56 (d, *J* = 2.0 Hz, 1H), 7.49 - 7.43 (m, 1H), 7.41 - 7.36 (m, 2H), 7.21 (dd, *J* = 17.6, 2.4 Hz, 1H), 4.78 (d, *J* = 28.8 Hz, 1H), 4.36 - 3.47 (m, 9H), 3.26 - 3.10 (m, 2H), 2.82 - 2.70 (m, 2H), 2.70 - 2.63 (m, 1H), 2.45 - 2.21 (m, 14H), 2.15 - 1.93 (m, 2H), 1.82 - 1.38 (m, 8H), 1.20 - 1.12 (m, 3H), 0.67 - 0.60 (m, 2H), 0.42 - 0.35 (m, 2H).

### Example 44: 2-(2,6-dioxopiperidin-3-yl)-5-(4-(2-(4-((1-(((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl) methyl)piperazin-1-yl)ethyl)piperidine-1-carbonyl)isoindoline-1,3-dione

### Step 1: preparation of tert-butyl (R)-4-(2-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperazin-1-yl)ethyl)piperidine-1-carboxylate

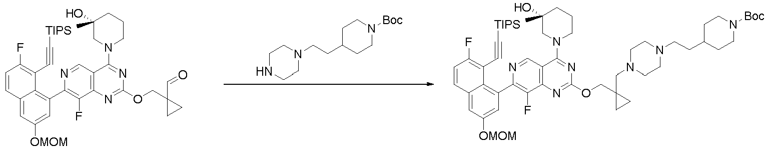

Tetraisopropyl titanate (143 mg, 503.40 µmol) and glacial acetic acid (10 mg, 166.66 µmol) were added to a stirred solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropane-1-carbaldehyde (75 mg, 100.68 µmol) and *tert*-butyl 4-(2-(piperazin-1-yl)ethyl)piperidine-1-carboxylate (33 mg, 110.74 µmol) in dichloromethane (10 mL) at room temperature, and the mixture was stirred at 25 °C for 2 h. Sodium triacetoxyborohydride (64 mg, 302.04 µmol) was then added, and the mixture was then reacted at 25 °C for another 3 h. Methanol (10 mL) was added to the reaction liquid to quench the reaction, and the mixture was concentrated under reduced pressure at 40 °C. The resulting crude product was purified by a preparative thin-layer chromatography plate (dichloromethane:methanol = 10:1) to give *tert*-butyl (*R*)-4-(2-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)ethyl)piperidine-1-carboxylate.

LC-MS: (ESI, m/z): [M+H]⁺ = 1026.9.

### Step 2: preparation of tert-butyl (R)-4-(2-(4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl) methyl)piperazin-1-yl)ethyl)piperidine-1-carboxylate

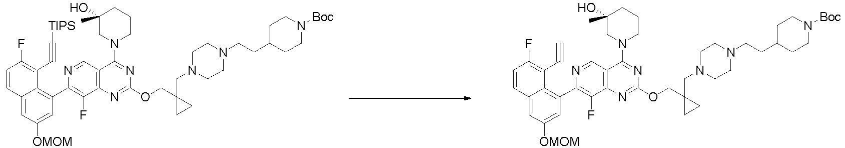

Cesium fluoride (98.4 mg, 647.90 µmol) was added to a solution of *tert*-butyl (*R*)-4-(2-(4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)ethyl)piperidine-1-carboxylate (66.5 mg, 64.79 µmol) in *N*,*N*-dimethylformamide (5 mL) at room temperature, and the mixture was reacted at 25 °C for 1 h under nitrogen atmosphere. After the reaction was completed, the reaction liquid was poured into water (50 mL) and extracted with ethyl acetate (20 mL × 3). The organic phases were combined, washed with saturated brine (50 mL), dried over anhydrous sodium sulfate, filtered, and concentrated under reduced pressure to give *tert*-butyl (R)-4-(2-(4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)ethyl)piperidine-1-carboxylate.

LC-MS: (ESI, m/z): [M+H]⁺ = 869.9.

### Step 3: preparation of (R)-1-(7-(8-ethynyl-7-fluoro-3-hydroxynaphthalen-1-yl)-8-fluoro-2-((1-((4-(2-(piperidin-4-yl)ethyl)piperazin-1-yl)methyl)cyclopropyl)methoxy)pyrido[4,3-d]pyrimidin-4-yl)-3-methylpiperidin-3-ol

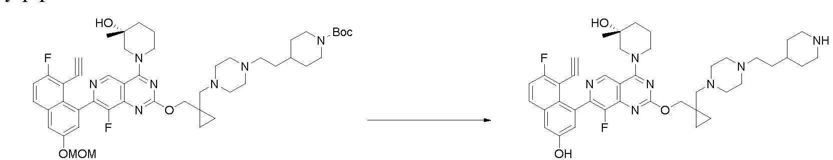

A hydrogen chloride/1,4-dioxane solution (4.0 M, 5 mL) was added to *tert*-butyl (*R*)-4-(2-(4-((1-(((7-(8-ethynyl-7-fluoro-3-(methoxymethoxy)naphthalen-1-yl)-8-fluoro-4-(3-hydroxy-3-methylpiperidin-1-yl)pyrido [4,3-*d*]pyrimidin-2-yl)oxy)methyl)cyclopropyl)methyl)piperazin-1-yl)ethyl)piperidine-1-carboxylate (63.1 mg, 64.79 µmol) at room temperature, and the mixture was reacted at room temperature for 1.5 h. After the reaction was completed, the mixture was concentrated under reduced pressure at 30 °C. A solution of ammonia in methanol (1 mL) was added to the concentrate, and the mixture was concentrated under reduced pressure at 30 °C to give (*R*)-1-(7-(8-ethynyl-7-fluoro-3-hydroxynaphthalen-1-yl)-8-fluoro-2-((1-((4-(2-(piperidin-4-yl)ethyl)piperazin-1-yl)methyl)cyclopropyl)methoxy)pyrido[4,3-*d*]pyrimidin-4-yl)-3-methylpiperidin-3-ol.

LC-MS: (ESI, m/z): [M+H]⁺ = 726.4.

### Step 4: preparation of 2-(2,6-dioxopiperidin-3-yl)-5-(4-(2-(4-((1-(((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin- 2-yl)oxy)methyl)cyclopropyl) methyl)piperazin-1-yl)ethyl)piperidine-1-carbonyl)isoindoline-1,3-dione

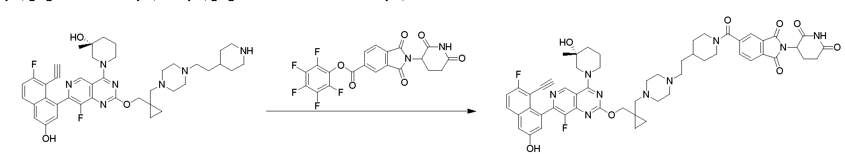

A solution of (*R*)-1-(7-(8-ethynyl-7-fluoro-3-hydroxynaphthalen-1-yl)-8-fluoro-2-((1-((4-(2-(piperidin-4-yl)ethyl)piperazin-1-yl)methyl)cyclopropyl)methoxy)pyrido[4,3-*d*]pyrimidin-4-yl)-3-methylpiperidin-3-ol (50.0 mg, 64.79 µmol) in *N*,*N*-dimethylformamide (5 mL) was cooled to 0 °C from room temperature. *N,N*-Diisopropylethylamine (94 mg, 725.24 µmol) and pentafluorophenyl 2-(2,6-dioxopiperidin-3-yl)-1,3-dioxoisoindoline-5-carboxylate (29 mg, 61.64 µmol) were added, and the mixture was stirred at 25 °C for 1 h. After the reaction was completed, the mixture was poured into water (100 mL) and extracted with ethyl acetate (20 mL × 2). The organic phases were combined, washed with saturated sodium chloride (100 mL), dried over anhydrous sodium sulfate, and concentrated under reduced pressure. The resulting crude product was purified by a preparative silica gel thin-layer chromatography plate (dichloromethane:methanol = 8:1) and then purified by Prep-HPLC to give 2-(2,6-dioxopiperidin-3-yl)-5-(4-(2-(4-((1-(((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)methyl)cyclopropyl) methyl)piperazin-1-yl)ethyl)piperidine-1-carbonyl)isoindoline-1,3-dione.

LC-MS: (ESI, m/z): [M+H]⁺ = 1010.1.

¹H NMR (400 MHz, CD₃OD) δ 9.11 (d, *J =* 56.0 Hz, 1H), 8.49 (s, 1H), 7.98 (d, *J* = 8.0 Hz, 1H), 7.87 - 7.81 (m, 3H), 7.35 - 7.15 (m, 3H), 5.25 - 5.10 (m, 1H), 4.75 - 4.45 (m, 4H), 4.44 - 4.20 (m, 2H), 3.70 - 3.55 (m, 2H), 3.55 - 3.40 (m, 2H), 3.05 - 2.85 (m, 6H), 2.80 - 2.65 (m, 8H), 2.25 - 2.10 (m, 2H), 1.85 - 1.70 (m, 4H), 1.65 - 1.50 (m, 4H), 1.35 - 1.10 (m, 6H), 0.80 - 0.70 (m, 2H), 0.60 - 0.45 (m, 2H).

### Example 45: (R)-1-(5-(9-((4-((1-(((7-(8-ethynyl-7-fluoro-3-hydroxynaphthalen-1-yl)-8-fluoro-4-(3-hydroxypiperidin-1-yl)pyrido[4,3-d]pyrimidin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1H,3H)-dione

### Step 1: preparation of (R)-1-(2,7-dichloro-8-fluoropyrido[4,3-d]pyrimidin-4-yl)piperidin-3-ol

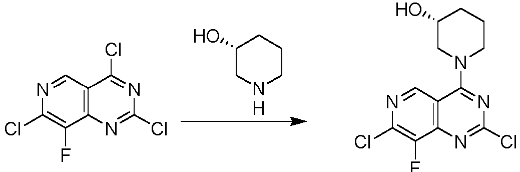

Triethylamine (1.2 g, 11.88 mmol) and (*R*)-piperidin-3-ol (350 mg, 3.57 mmol) were added to a solution of 2,4,7-trichloro-8-fluoropyrido[4,3-*d*]pyrimidine (1 g, 3.96 mmol) in anhydrous tetrahydrofuran (30 mL) at -50 °C, and the mixture was stirred at -50 °C for 2 h. After the reaction was completed, saturated ammonium chloride (50 mL) was added, and the mixture was extracted with ethyl acetate (50 mL × 2). The organic phases were combined, dried over anhydrous sodium sulfate, and concentrated under reduced pressure. The resulting crude product was purified by silica gel column chromatography (dichloromethane:methanol = 50:1) to give (*R*)-1-(2,7-dichloro-8-fluoropyrido[4,3-*d*]pyrimidin-4-yl)piperidin-3-ol.

LC-MS: (ESI, m/z): [M+H]⁺ = 317.1.

### Step 2: preparation of (R)-1-(7-chloro-8-fluoro-2-((1-(hydroxymethyl)cyclopropyl)methoxy)pyrido[4,3-d]pyrimidin-4-yl)piperidin-3-ol

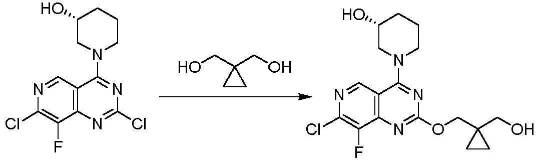

Triethylenediamine (80 mg, 0.71 mmol) and anhydrous cesium carbonate (1.4 g, 4.260 mmol) were added to a solution of (*R*)-1-(2,7-dichloro-8-fluoropyrido[4,3-*d*]pyrimidin-4-yl)piperidin-3-ol (450 mg, 1.420 mmol) and cyclopropane-1,1-diyldimethanol (290 mg, 2.840 mmol) in anhydrous acetonitrile (15 mL), and the mixture was stirred at 25 °C for 16 h. After the reaction was completed, the mixture was poured into water (50 mL) and extracted with ethyl acetate (20 mL × 2). The organic phases were combined, washed with saturated sodium chloride (50 mL), dried over anhydrous sodium sulfate, and concentrated under reduced pressure. The resulting crude product was purified by silica gel column chromatography (dichloromethane:methanol = 20:1) to give (*R*)-1-(7-chloro-8-fluoro-2-((1-(hydroxymethyl)cyclopropyl)methoxy)pyrido[4,3-*d*]pyrimidin-4-yl)piperidin-3-ol.

LC-MS: (ESI, m/z): [M+H]⁺ = 383.8.

### Step 3: preparation of (R)-1-(8-fluoro-7-(7-fluoro-3-(methoxymethoxy)-8-((triisopropylsilyl)ethynyl) naphthalen-1-yl)-2-((1-(hydroxymethyl)cyclopropyl)methoxy)pyrido[4,3-d]pyrimidin-4-yl)piperidin-3-ol

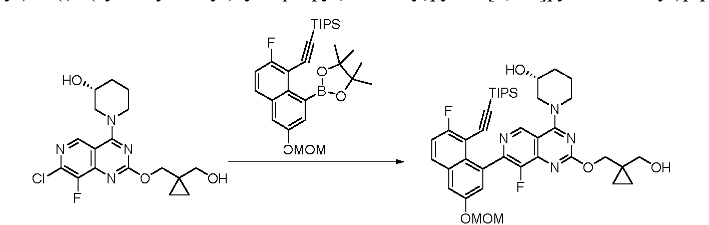

Anhydrous cesium carbonate (900 mg, 2.760 mmol) and cataCXium(R) A Pd G3 (75 mg, 0.104 mmol) were added to a mixed solution of (*R*)-1-(7-chloro-8-fluoro-2-((1-(hydroxymethyl)cyclopropyl)methoxy)pyrido [4,3-*d*]pyrimidin-4-yl)piperidin-3-ol (350 mg, 0.92 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (570 mg, 1.1 mmol) in 1,4-dioxane (8 mL) and water (1 mL) at room temperature. After the system was degassed, the mixture was stirred at 85 °C for 16 h under nitrogen atmosphere. After the reaction was completed, the mixture was concentrated under reduced pressure at 40 °C, and the resulting crude product was purified by silica gel thin-layer chromatography (dichloromethane:methanol = 10:1) to give (*R*)-1-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido[4,3-*d*]pyrimidin-4-yl)piperidin-3-ol.

LC-MS: (ESI, m/z): [M+Na]⁺ = 745.3.

### Step 4: preparation of (R)-1-(((8-fluoro-7-(7-fluoro-3-(methoxymethoxy)-8-((triisopropylsilyl)ethynyl) naphthalen-1-yl)-4-(3-hydroxypiperidin-1-yl)pyrido[4,3-d]pyrimidin-2-yl)oxy)methyl)cyclopropane-1-carbaldehyde

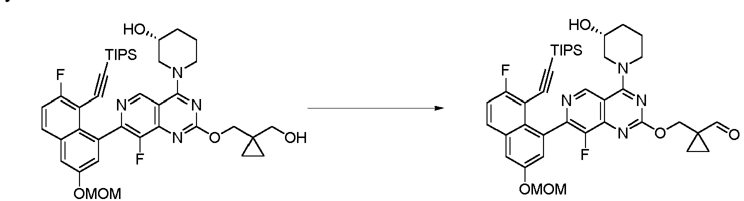

A solution of (*R*)-1-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido [4,3-*d*]pyrimidin-4-yl)piperidin-3-ol (350 mg, 0.546 mmol) in dichloromethane (5 mL) was cooled to 0 °C from room temperature, and Dess-Martin reagent (470 mg, 1.092 mmol) was added. The mixture was stirred at 25 °C for 4 h. After the reaction was completed, the mixture was poured into water (50 mL) and extracted with dichloromethane (20 mL × 2). The organic phases were combined, washed with saturated sodium chloride (50 mL), dried over anhydrous sodium sulfate, and concentrated under reduced pressure. The resulting crude product was purified by silica gel thin-layer chromatography (dichloromethane:methanol = 20:1) to give (*R*)-1-(((8-fluoro-7-(7-fluoro-3-(methoxymethoxy)-8-((triisopropylsilyl)ethynyl)naphthalen-1-yl)-4-(3-hydroxypiperidin-1-yl)pyrido[4,3-*d*]pyrimidin-2-yl)oxy)methyl)cyclopropane-1-carbaldehyde.

LC-MS: (ESI, m/z): [M+H]⁺ = 731.9.

### Step 5: preparation of (R)-1-(5-(9-((4-((1-(((8-fluoro-7-(7-fluoro-3-(methoxymethoxy)-8-((triisopropylsilyl) ethynyl)naphthalen-1-yl)-4-(3-hydroxypiperidin-1-yl)pyrido[4,3-d]pyrimidin-2-yl)oxy)methyl)cyclopropyl) methyl)piperazin-1-yl)methyl)-3-azaspiro[5.5]undecane-3-carbonyl)-2-methylphenyl)dihydropyrimidine-2,4(1H,3H)-dione

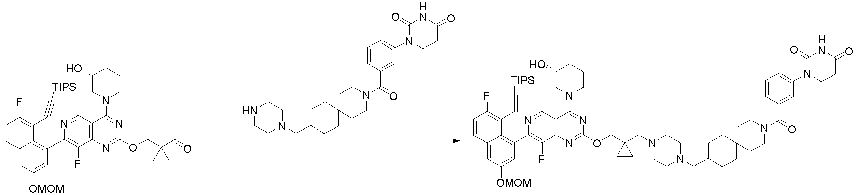

Tetraisopropyl titanate (400 mg, 1.370 mmol) was added to a solution of (*R*)-1-(((8-fluoro-7-(7-fluoro-3-(methoxymethoxy)-8-((triisopropylsilyl)ethynyl)naphthalen-1-yl)-4-(3-hydroxypiperidin-1-yl)pyrido[4,3-*d*]pyrimidin-2-yl)oxy)methyl)cyclopropane-1-carbaldehyde (200 mg, 0.274 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (160 mg, 0.333 mmol) in dichloromethane (5 mL) at room temperature, and the mixture was stirred at 25 °C for 16 h. Sodium triacetoxyborohydride (200 mg, 0.822 mmol) was added to the mixture at room temperature, and the mixture was reacted at 25 °C for another 3 h. Methanol (10 mL) was added to quench the reaction, and the mixture was concentrated under reduced pressure at 40 °C. The resulting crude product was purified by a preparative thin-layer chromatography plate (dichloromethane:methanol = 10:1, 0.1% NH₃) to give (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxypiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1196.9.

### Step 6: preparation of (R)-1-(5-(9-((4-((1-(((7-(8-ethynyl-7-fluoro-3-hydroxynaphthalen-1-yl)-8-fluoro-4-(3-hydroxypiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

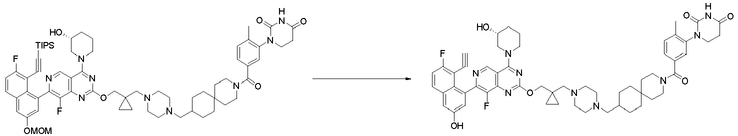

CsF (126 mg, 0.835 mmol) was added to a solution of (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxypiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H,*3*H*)-dione (200 mg, 0.167 mmol) in DMF (2 mL), and the mixture was stirred at room temperature for 0.5 h. Water (10 mL) was added to the reaction liquid, and the mixture was extracted with ethyl acetate (10 mL × 3). The organic phases were combined, dried over anhydrous sodium sulfate, and concentrated under reduced pressure. The intermediate obtained by concentration was dissolved in dioxane (2 mL), and HCl/1,4-dioxane (6 N, 1 mL) was added. The mixture was reacted at room temperature for 0.5 h. The reaction liquid was adjusted to pH > 7 with an aqueous Na₂CO₃ solution and extracted with ethyl acetate (5 mL × 3). The combined organic phase was dried over Na₂SO₄, filtered, concentrated under reduced pressure, and purified by preparative high-performance liquid chromatography to give (*R*)-1-(5-(9-((4-((1-(((7-(8-ethynyl-7-fluoro-3-hydroxynaphthalen-1-yl)-8-fluoro-4-(3-hydroxypiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

MS(ESI) m/z: [M+H]⁺ = 996.2.

¹H NMR (400 MHz, CD₃OD) δ 9.05 (d, *J* = 33.6 Hz, 1H), 7.89 - 7.80 (m, 1H), 7.40 (d, *J* = 7.7 Hz, 1H), 7.36 - 7.27 (m, 4H), 7.22 (dd, *J =* 10.9, 2.2 Hz, 1H), 4.54 - 4.48 (m, 1H), 4.42 - 4.33 (m, 1H), 4.27 - 4.18 (m, 1H), 4.16 - 3.60 (m, 9H), 3.52 - 3.35 (m, 4H), 2.93 - 2.79 (m, 2H), 2.69 - 2.36 (m, 9H), 2.31 (s, 3H), 2.24 - 1.99 (m, 5H), 1.82 - 1.69 (m, 4H), 1.63 - 1.28 (m, 9H), 1.22 - 1.03 (m, 4H), 0.74 - 0.66 (m, 2H), 0.54 - 0.46 (m, 2H).

### Example 46: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

### Step 1: preparation of 1-(5-(9-((4-((R)-3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methoxyphenyl) dihydropyrimidine-2,4(1H,3H)-dione

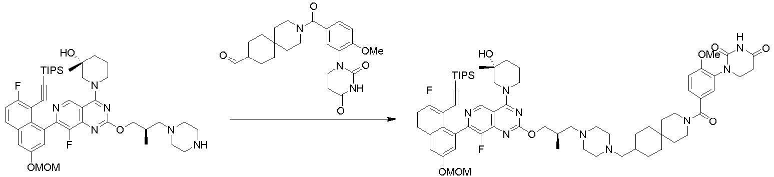

Tetraisopropyl titanate (0.5 mL) was added to a stirred solution of (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy) pyrido[4,3-*d*]pyrimidin-4-yl)-3-methylpiperidin-3-ol (125 mg, 0.16 mmol) and 3-(3-(2,4-dioxotetrahydropyrimidin-1(2*H*)-yl)-4-methoxybenzoyl)-3-azaspiro[5.5]undecane-9-carbaldehyde (100 mg, 0.23 mmol) in DCM/AcOH (1 mL/0.1 mL), and the mixture was stirred at 30 °C for 2 h. NaBH(OAc)₃ (99 mg, 0.47 mmol) was added to the mixture in an ice bath, and the resulting mixture was stirred for 2 h. The mixture was concentrated and purified by silica gel column chromatography (DCM:MeOH = 100:1 to 10:1) to give 1-(5-(9-((4-((*R*)-3-((8-fluoro-7-(7-fluoro-3-(methoxymethoxy)-8-((triisopropylsilyl)ethynyl) naphthalen-1-yl)-4-((*R*)-3-hydroxy-3-methylpiperidin-1-yl)pyrido[4,3-*d*]pyrimidin-2-yl)oxy)-2-methylpropyl) piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 607.9.

### Step 2: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

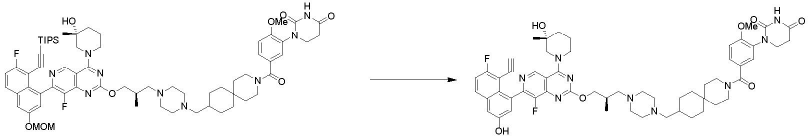

Cesium fluoride (162 mg, 1.07 mmol) was added to a stirred solution of 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione (130 mg, 0.11 mmol) in DMF (1 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,*3*H)-dione.

LC-MS: (ESI, m/z): [M/2+H]⁺ = 508.0.

¹H NMR (400 MHz, DMSO-*d*₆) δ 10.32 (s, 1H), 10.14 (s, 1H), 9.13 (d, *J* = 60.1 Hz, 1H), 7.97 (dd, *J* = 9.2, 5.9 Hz, 1H), 7.50 - 7.42 (m, 1H), 7.40 - 7.34 (m, 2H), 7.33 - 7.30 (m, 1H), 7.25 - 7.18 (m, 1H), 7.14 (d, *J =* 8.6 Hz, 1H), 4.73 (d, *J* = 19.8 Hz, 1H), 4.56 - 4.26 (m, 2H), 4.15 - 3.91 (m, 3H), 3.84 (s, 3H), 3.65 - 3.39 (m, 6H), 2.71 - 2.65 (m, 2H), 2.40 - 1.96 (m, 14H), 1.76 - 1.60 (m, 5H), 1.58 - 1.21 (m, 8H), 1.16 (d, *J* = 15.7 Hz, 3H), 1.13 - 0.80 (m, 8H).

### Example 47: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

### Step 1: preparation of 1-(5-(9-((4-((R)-3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

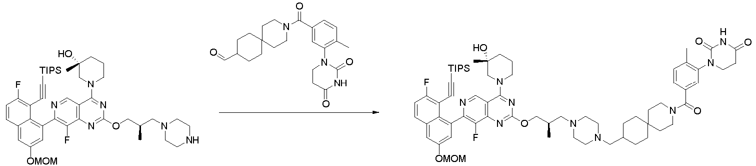

Tetraisopropyl titanate (0.5 mL) was added to a stirred solution of (R)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy) pyrido[4,3-*d*]pyrimidin-4-yl)-3-methylpiperidin-3-ol (150 mg, 0.19 mmol) and 3-(3-(2,4-dioxotetrahydropyrimidin-1(2*H*)-yl)-4-methylbenzoyl)-3-azaspiro[5.5]undecane-9-carbaldehyde (119 mg, 0.29 mmol) in DCM/AcOH (1.5 mL/0.15 mL), and the mixture was stirred at 30 °C for 2 h. NaBH(OAc)₃ (121 mg, 0.57 mmol) was added to the mixture in an ice bath, and the resulting mixture was stirred for 2 h. The reaction liquid was directly concentrated under reduced pressure, and the resulting crude product was purified by silica gel column chromatography (DCM:MeOH = 100:1 to 10:1) to give 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro [5.5]undecane-3-carbonyl)-2-methylphenyl)dihydropyrimidine-2,4(1*H*,3*H*)-dione.

LC-MS: (ESI, m/z): [M/2+H]⁺ = 600.0.

### Step 2: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

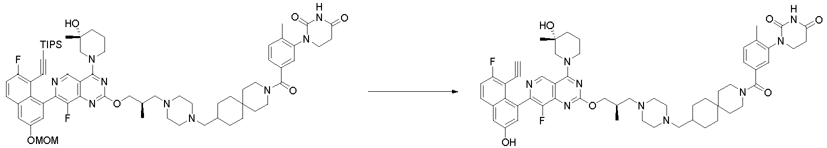

Cesium fluoride (213 mg, 1.40 mmol) was added to a solution of 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1*H,*3*H*)-dione (170 mg, 0.14 mmol) in DMF (2 mL), and the mixture was stirred at room temperature for 30 min. Water (10 mL) was added to the reaction liquid, and the mixture was then extracted with ethyl acetate (10 mL × 3). The organic phases were combined, dried over anhydrous sodium sulfate, and concentrated under reduced pressure. HCl/1,4-dioxane (6 N, 2.0 mL) was added to a stirred solution of the concentrate in 1,4-dioxane (2.0 mL), and the mixture was reacted at room temperature for 0.5 h. The reaction liquid was adjusted to pH > 7 with an aqueous Na₂CO₃ solution and then extracted with EtOAc (10 mL × 2). The organic phase was washed with brine, dried over anhydrous Na₂SO₄, and concentrated. The resulting crude product was purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 998.4.

¹H NMR (400 MHz, DMSO-*d*₆) δ 10.36 (s, 1H), 10.14 (s, 1H), 9.13 (d, *J* = 60.2 Hz, 1H), 7.97 (dd, *J =* 9.2, 5.9 Hz, 1H), 7.48 - 7.43 (m, 1H), 7.41 - 7.37 (m, 1H), 7.36 - 7.29 (m, 2H), 7.28 - 7.16 (m, 2H), 4.73 (d, *J =* 19.9 Hz, 1H), 4.57 - 4.26 (m, 2H), 4.16 - 3.98 (m, 2H), 3.97 - 3.92 (m, 1H), 3.85 - 3.75 (m, 1H), 3.65 - 3.48 (m, 4H), 2.90 - 2.58 (m, 3H), 2.48 - 2.24 (m, 9H), 2.23 - 1.89 (m, 9H), 1.87 - 1.54 (m, 6H), 1.53 - 1.21 (m, 7H), 1.20 - 1.12 (m, 3H), 1.10 - 0.88 (m, 7H).

### Example 48: 1-(2-chloro-5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)phenyl)dihydropyrimidine-2,4(1H,3H)-dione

### Step 1: preparation of 1-(2-chloro-5-(9-((4-((R)-3-((8-fluoro-7-(7-fluoro-3-(methoxymethoxy)-8-((triisopropylsilyl)ethynyl)naphthalen-1-yl)-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)phenyl)dihydropyrimidine-2,4(1H,3H)-dione

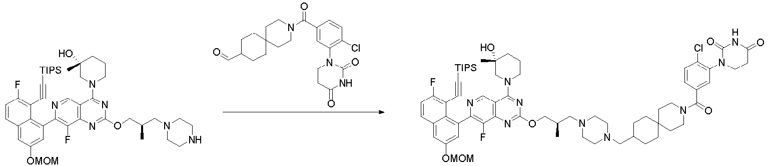

Tetraisopropyl titanate (0.5 mL) was added to a stirred solution of (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy) pyrido[4,3-*d*]pyrimidin-4-yl)-3-methylpiperidin-3-ol (150 mg, 0.19 mmol) and 3-(4-chloro-3-(2,4-dioxotetrahydropyrimidin-1(2*H*)-yl)benzoyl)-3-azaspiro[5.5]undecane-9-carbaldehyde (125 mg, 0.29 mmol) in DCM/AcOH (1.5 mL/0.15 mL), and the mixture was stirred at 30 °C for 2 h. NaBH(OAc)₃ (121 mg, 0.57 mmol) was added to the above mixture in an ice bath, and the mixture was stirred for 2 h. The reaction liquid was directly concentrated under reduced pressure and purified by silica gel column chromatography (DCM:MeOH = 100:1 to 10:1) to give 1-(2-chloro-5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M/2+H]⁺ = 609.9.

### Step 2: preparation of 1-(2-chloro-5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)phenyl)dihydropyrimidine-2,4(1H,3H)-dione

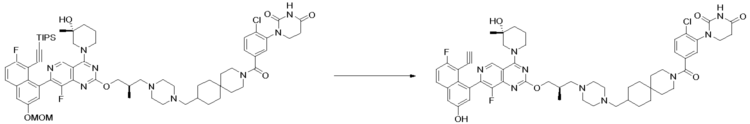

Cesium fluoride (197 mg, 1.30 mmol) was added to a stirred solution of 1-(2-chloro-5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)phenyl)dihydropyrimidine-2,4(1*H*,3*H*)-dione (158 mg, 0.13 mmol) in DMF (1 mL), and the mixture was then stirred at room temperature for 30 min. Water (10 mL) was added to quench the reaction, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(2-chloro-5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1018.4.

¹H NMR (400 MHz, DMSO-*d*₆) δ 10.50 (s, 1H), 10.14 (s, 1H), 9.13 (d, *J* = 60.0 Hz, 1H), 7.97 (dd, *J =* 9.2, 5.9 Hz, 1H), 7.63 (d, *J =* 8.2 Hz, 1H), 7.56 - 7.52 (m, 1H), 7.48 - 7.43 (m, 1H), 7.41 - 7.35 (m, 2H), 7.25 - 7.17 (m, 1H), 4.73 (d*, J =* 19.6 Hz, 1H), 4.55 - 4.26 (m, 2H), 4.14 - 3.92 (m, 3H), 3.80 - 3.71 (m, 1H), 3.66 - 3.51 (m, 4H), 2.78 - 2.69 (m, 2H), 2.45 - 1.90 (m, 16H), 1.79 - 1.57 (m, 6H), 1.55 - 1.39 (m, 5H), 1.37 - 1.21 (m, 3H), 1.20 - 1.12 (m, 3H), 1.11 - 1.01 (m, 3H), 0.99 - 0.95 (m, 3H).

### Example 49: (R)-1-(5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of (R)-1-(5-(9-((4-((1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

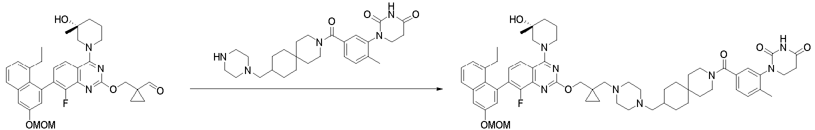

Tetraisopropyl titanate (273 mg, 0.96 mmol) was added to a solution of (*R*)-1-(((7-(8-ethyl-3-(methoxymethoxy)naphthalen-1-yl)-8-fluoro-4-(3-hydroxy-3-methylpiperidin-1-yl)quinazolin-2-yl)oxy) methyl)cyclopropane-1-carbaldehyde (110 mg, 0.192 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (111 mg, 0.23 mmol) in DCM/AcOH (2 mL/0.1 mL), and the mixture stirred at 25 °C for 2 h. Sodium triacetoxyborohydride (128 mg, 0.576 mmol) was added to the mixture at room temperature, and the mixture was reacted at 25 °C for another 3 h. After the reaction was completed, the mixture was concentrated under reduced pressure at 40 °C. The resulting crude product was purified by a preparative thin-layer chromatography plate (dichloromethane:methanol = 10:1) to give (*R*)-1-(5-(9-((4-((1-(((7-(8-ethyl-3-(methoxymethoxy)naphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 520.4.

### Step 2: preparation of (R)-1-(5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1H,3H)-dione

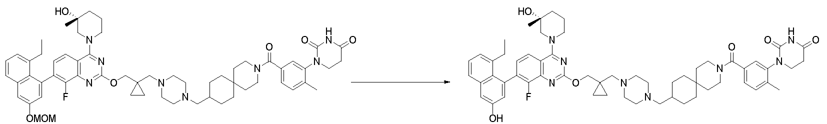

HCl/1,4-dioxane (6 N, 1.0 mL) was added to a stirred solution of (*R*)-1-(5-(9-((4-((1-(((7-(8-ethyl-3-(methoxymethoxy)naphthalen-1-yl)-8-fluoro-4-(3-hydroxy-3-methylpiperidin-1-yl)quinazolin-2-yl)oxy) methyl)cyclopropyl)methyl)piperazin-1-yl)methyl)-3-azaspiro[5.5]undecane-3-carbonyl)-2-methylphenyl) dihydropyrimidine-2,4(1*H*,3*H*)-dione (115 mg, 0.111 mmol) in 1,4-dioxane (2.0 mL) at room temperature, and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give (*R*)-1-(5-(9-((4-((1-(((7-(8-ethyl-3-hydroxynaphthalen-1-yl)-8-fluoro-4-(3-hydroxy-3-methylpiperidin-1-yl)quinazolin-2-yl)oxy)methyl)cyclopropyl) methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 995.2.

¹H NMR (400 MHz, DMSO-*d*₆) δ 10.37 (s, 1H), 9.87 (d, *J =* 2.8 Hz, 1H), 7.93 - 7.85 (m, 1H), 7.66 (d, *J* = 7.8 Hz, 1H), 7.39 - 7.29 (m, 3H), 7.28 - 7.20 (m, 3H), 7.11 (d, *J* = 7.1 Hz, 1H), 6.90 - 6.87 (m, 1H), 4.67 (d, *J* = 13.8 Hz, 1H), 4.31 - 4.18 (m, 2H), 4.05 - 3.95 (m, 1H), 3.87 - 3.74 (m, 2H), 3.63 - 3.34 (m, 5H), 3.32 - 3.22 (m, 2H), 2.83 - 2.64 (m, 2H), 2.49 - 2.22 (m, 11H), 2.21 (s, 3H), 2.10 - 1.90 (m, 3H), 1.78 - 1.57 (m, 5H), 1.56 - 1.20 (m, 8H), 1.16 (d, *J =* 15.4 Hz, 3H), 1.11 - 0.88 (m, 4H), 0.85 - 0.75(m, 3H), 0.66 - 0.58 (m, 2H), 0.42 - 0.35 (m, 2H).

### Example 50: 1-(5-(9-((4-((R)-3-((7-(8-ethynyl-7-fluoro-3-hydroxynaphthalen-1-yl)-8-fluoro-4-(((1S,2S)-2-fluorocyclopropyl)(methyl)amino)pyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

### Step 1: preparation of 2,7-dichloro-8-fluoro-N-((1S,2S)-2-fluorocyclopropyl)pyrido[4,3-d]pyrimidine-4-amine

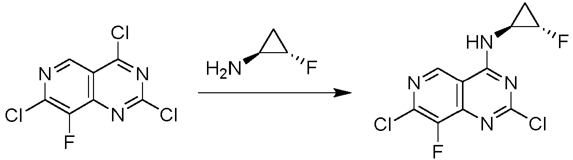

TEA (1.8 g, 5.94 mmol) was added to a solution of 2,4,7-trichloro-8-fluoropyrido[4,3-*d*]pyrimidine (1.5 g, 2.0 mmol) in DCM (30 mL) at -78 °C, and the mixture was stirred for 5 min. (1*S*,2*S*)-2-Fluorocyclopropan-1-amine hydrochloride (596 mg, 5.35 mmol) was then added, and the mixture was stirred for 2 h. After the reaction was completed, the mixture was naturally warmed to room temperature, H₂O (30 mL) was added, and the mixture was extracted with DCM (30 mL × 3). The organic phases were combined, dried over anhydrous sodium sulfate, and concentrated under reduced pressure, and the concentrate was purified by silica gel column chromatography (PE:EtOAc = 5:1) to give 2,7-dichloro-8-fluoro-*N*-((1*S*,2*S*)-2-fluorocyclopropyl)pyrido[4,3-*d*]pyrimidine-4-amine.

LC-MS: (ESI, m/z): [M+H]⁺ = 291.1.

### Step 2: preparation of 2,7-dichloro-8-fluoro-N-((1S,2S)-2-fluorocyclopropyl)-N-methylpyrido[4,3-d]pyrimidine-4-amine

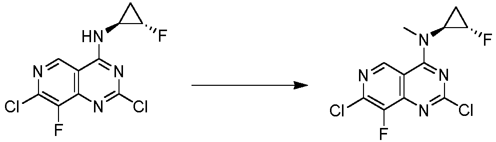

NaH (247.2 mg, 6.18 mmol, 60%) was added to a solution of 2,7-dichloro-8-fluoro-*N*-((1*S*,2*S*)-2-fluorocyclopropyl)pyrido[4,3-*d*]pyrimidine-4-amine (1.2 g, 4.12 mmol) in DMF (15 mL) at 0 °C. The mixture was reacted for 1 h in an ice bath, and iodomethane (1.75 g, 12.36 mmol) was then added. The mixture was reacted at room temperature for 2 h. After the reaction was completed, a saturated aqueous ammonium chloride solution (20 mL) was added, and the mixture was extracted with ethyl acetate (25 mL × 3). The organic phases were combined, washed with saturated brine, dried over anhydrous sodium sulfate, and concentrated under reduced pressure. The concentrate was purified by silica gel column chromatography (PE:EA = 8:1) to give 2,7-dichloro-8-fluoro-*N*-((1*S*,2*S*)-2-fluorocyclopropyl)-*N*-methylpyrido[4,3-*d*]pyrimidine-4-amine.

LC-MS: (ESI, m/z): [M+H]⁺ = 305.1.

### Step 3: preparation of 2-((R)-3-(4-benzylpiperazin-1-yl)-2-methylpropoxy)-7-chloro-8-fluoro-N-((1S,2S)-2-fluorocyclopropyl)-N-methylpyrido[4,3-d]pyrimidine-4-amine

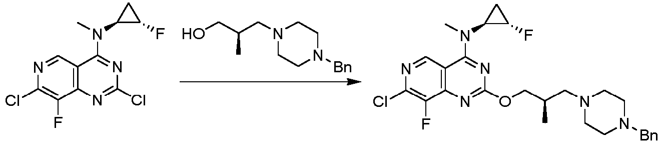

Cs₂CO₃ (2.88 g, 8.85 mmol) was added to a solution of 2,7-dichloro-8-fluoro-*N*-((1*S*,2*S*)-2-fluorocyclopropyl)-*N*-methylpyrido[4,3-*d*]pyrimidine-4-amine (900 mg, 2.95 mmol) and (*R*)-3-(4-benzylpiperazin-1-yl)-2-methylpropan-1-ol (879 mg, 3.54 mmol) in THF (15 mL), and the mixture was stirred at 90 °C for 16 h. After the reaction was completed, water (20 mL) was added, and the mixture was extracted with ethyl acetate (10 mL × 3). The organic phases were combined, dried over anhydrous sodium sulfate, and concentrated under reduced pressure. The concentrate was purified by silica gel column chromatography (PE:EA = 1:1) to give 2-((R)-3-(4-benzylpiperazin-1-yl)-2-methylpropoxy)-7-chloro-8-fluoro-*N*-((1*S*,2*S*)-2-fluorocyclopropyl)-*N*-methylpyrido[4,3-*d*]pyrimidine-4-amine.

LC-MS: (ESI, m/z): [M+H]⁺ = 517.2.

### Step 4: preparation of 2-((R)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)-N-((1S,2S)-2-fluorocyclopropyl)-N-methylpyrido[4,3-d]pyrimidine-4-amine

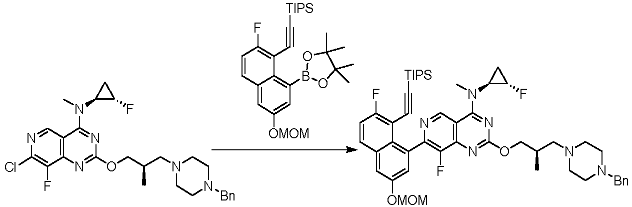

((2-Fluoro-6-(methoxymethoxy)-8-(4,4,5,5-tetramethyl-1,3,2-dioxaborolan-2-yl)naphthalen-1-yl)ethynyl)triisopropylsilane (467 mg, 0.912 mmol), cataCXium A Pd G3 (111 mg, 0.152 mmol), and cesium carbonate (743 mg, 2.28 mmol) were added to a mixed solution of 2-((*R*)-3-(4-benzylpiperazin-1-yl)-2-methylpropoxy)-7-chloro-8-fluoro-*N*-((1*S*,2*S*)-2-fluorocyclopropyl)-*N*-methylpyrido[4,3-*d*]pyrimidine-4-amine (500 mg, 0.76 mmol) in 1,4-dioxane (6 mL) and H₂O (1 mL). The mixture was reacted at 85 °C for 16 h under nitrogen atmosphere. After the reaction was completed, water (10 mL) was added, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 15:1) to give 2-((*R*)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)-*N*-((1*S*,2*S*)-2-fluorocyclopropyl)-*N*-methylpyrido[4,3-*d*]pyrimidine-4-amine.

LC-MS: (ESI, m/z): [M/2+H]⁺ = 434.3.

### Step 5: preparation of 8-fluoro-7-(7-fluoro-3-(methoxymethoxy)-8-((triisopropylsilyl)ethynyl)naphthalen-1-yl)-N-((1S,2S)-2-fluorocyclopropyl)-N-methyl-2-((R)-2-methyl-3-(piperazin-1-yl)propoxy)pyrido[4,3-d]pyrimidine-4-amine

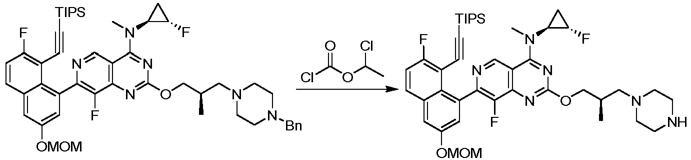

2-((*R*)-3-(4-Benzylpiperazin-1-yl)-2-methylpropoxy)-8-fluoro-7-(7-fluoro-3-(methoxymethoxy)-8-((triisopropylsilyl) ethynyl)naphthalen-1-yl)-*N*-((1*S*,2*S*)-2-fluorocyclopropyl)-*N*-methylpyrido[4,3-*d*]pyrimidine-4-amine (600 mg, 0.69 mmol) was dissolved in DCM (6 mL), and 1-chloroethyl carbonochloridate (296 mg, 2.07 mmol) and DIEA (178 mg, 1.38 mmol) were added. The mixture was reacted at room temperature for 1 h and concentrated under reduced pressure. MeOH (6 mL) was added to the concentrate, and the mixture was heated to 50 °C and reacted for 50 min. After the reaction was completed, the mixture was concentrated under reduced pressure. The concentrate was purified by silica gel column chromatography (DCM:MeOH (NH₃) = 15:1) to give 8-fluoro-7-(7-fluoro-3-(methoxymethoxy)-8-((triisopropylsilyl)ethynyl)naphthalen-1-yl)-*N*-((1*S*,2*S*)-2-fluorocyclopropyl)-*N*-methyl-2-((*R*)-2-methyl-3-(piperazin-1-yl)propoxy)pyrido[4,3-*d*]pyrimidine-4-amine.

LC-MS: (ESI, m/z): [M+H]⁺ = 777.4.

### Step 6: preparation of 1-(5-(9-((4-((R)-3-((8-fluoro-7-(7-fluoro-3-(methoxymethoxy)-8-((triisopropylsilyl) ethynyl)naphthalen-1-yl)-4-(((1S,2S)-2-fluorocyclopropyl)(methyl)amino)pyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

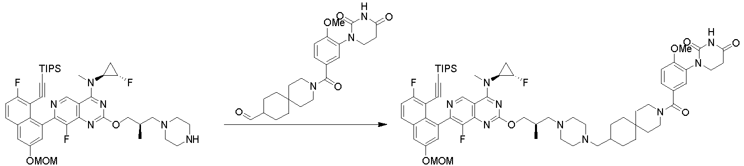

Tetraisopropyl titanate (0.5 mL) was added to a stirred solution of 8-fluoro-7-(7-fluoro-3-(methoxymethoxy)-8-((triisopropylsilyl)ethynyl)naphthalen-1-yl)-*N*-((1*S*,2*S*)-2-fluorocyclopropyl)-*N-*methyl-2-((*R*)-2-methyl-3-(piperazin-1-yl)propoxy)pyrido[4,3-*d*]pyrimidine-4-amine (150 mg, 0.19 mmol) and 3-(3-(2,4-dioxotetrahydropyrimidin-1(2*H*)-yl)-4-methoxybenzoyl)-3 -azaspiro [5. 5]undecane-9-carbaldehyde (124 mg, 0.29 mmol) in DCM/AcOH (2 mL/0.1 mL), and the mixture was stirred at 30 °C for 2 h. NaBH(OAc)₃ (127 mg, 0.57 mmol) was added to the mixture in an ice bath, and the mixture was then stirred at room temperature for 2 h. The reaction liquid was directly concentrated, and the resulting concentrate was purified by silica gel column chromatography (DCM:MeOH =1:0 to 4:1) to give 1-(5-(9-((4-((*R*)-3-((8-fluoro-7-(7-fluoro-3-(methoxymethoxy)-8-((triisopropylsilyl)ethynyl)naphthalen-1-yl)-4-(((1*S*,2*S*)-2-fluorocyclopropyl)(methyl) amino)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 594.9.

### Step 7: preparation of 1-(5-(9-((4-((R)-3-((7-(8-ethynyl-7-fluoro-3-hydroxynaphthalen-1-yl)-8-fluoro-4-(((1S,2S)-2-fluorocyclopropyl)(methyl)amino)pyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

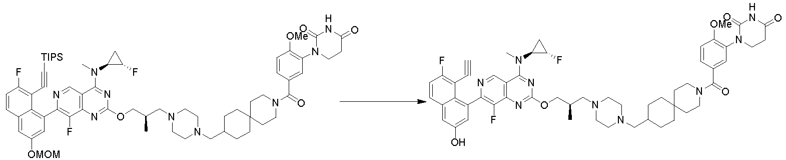

CsF (83.6 mg, 0.55 mmol) was added to a solution of 1-(5-(9-((4-((*R*)-3-((8-fluoro-7-(7-fluoro-3-(methoxymethoxy)-8-((triisopropylsilyl)ethynyl)naphthalen-1-yl)-4-(((1*S*,2*S*)-2-fluorocyclopropyl)(methyl) amino)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione (130 mg, 0.11 mmol) in DMF (2 mL), and the mixture was stirred at room temperature for 0.5 h. Saturated brine (10 mL × 3) was added to wash off DMF, and the mixture was extracted with ethyl acetate (10 mL × 3). The organic phases were combined, dried over anhydrous sodium sulfate, and concentrated under reduced pressure. The resulting concentrate was dissolved in 1,4-dioxane (2 mL), and HCl/1,4-dioxane (6 N, 1 mL) was added. The mixture was reacted at room temperature for 0.5 h. The reaction liquid was concentrated, and the resulting crude product was purified by preparative high-performance liquid chromatography to give 1-(5-(9-((4-((*R*)-3-((7-(8-ethynyl-7-fluoro-3-hydroxynaphthalen-1-yl)-8-fluoro-4-(((1*S*,2*S*)-2-fluorocyclopropyl)(methyl)amino)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)
dihydropyrimidine-2,4(1*H*,3*H*)-dione.

LC-MS: (ESI, m/z): [M+H]⁺ = 988.4.

¹H NMR (400 MHz, DMSO-*d*₆) δ 10.32 (s, 1H), 10.13 (s, 1H), 9.48 (d, *J* = 14.8 Hz, 1H), 7.98 (dd, *J =* 9.2, 6.0 Hz, 1H), 7.46 (t*, J =* 9.0 Hz, 1H), 7.41 - 7.34 (m, 2H), 7.33 - 7.30 (m, 1H), 7.20 (d, *J =* 2.5 Hz, 1H), 7.14 (d, *J =* 8.6 Hz, 1H), 5.13 - 4.80 (m, 1H), 4.52 - 4.36 (m, 1H), 4.17 - 4.04 (m, 2H), 4.02 - 3.93 (m, 1H), 3.84 (s, 3H), 3.63 - 3.55 (m, 2H), 3.54 - 3.35 (m, 3H), 3.29 - 3.24 (m, 2H), 2.73 - 2.68 (m, 2H), 2.44 - 1.95 (m, 14H), 1.73 - 1.56 (m, 3H), 1.55 - 1.37 (m, 5H), 1.34 - 1.16 (m, 4H), 1.12 - 0.90 (m, 7H).

### Example 60: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)quinazolin-2-yl)oxy)-2-methylpropyl)piperazin-1-yl)methyl)-3-azaspiro[5.5] undecane-3-carbonyl)-2-methoxyphenyl)dihydropyrimidine-2,4(1H,3H)-dione

### Step 1: preparation of 1-(5-(9-((4-((R)-3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)quinazolin-2-yl)oxy)-2-methylpropyl) piperazin-1-yl)methyl)-3-azaspiro[5.5]undecane-3-carbonyl)-2-methoxyphenyl)dihydropyrimidine-2,4(1H,3H)-dione

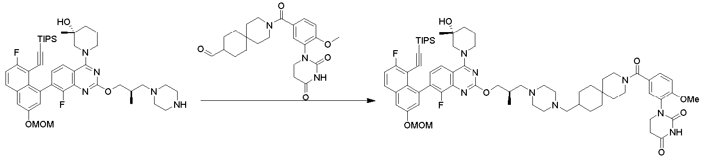

Tetraisopropyl titanate (0.5 mL) was added to a stirred solution of (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy) quinazolin-4-yl)-3-methylpiperidin-3-ol (150 mg, 0.19 mmol) and 3-(3-(2,4-dioxotetrahydropyrimidin-1(2*H*)-yl)-4-methoxybenzoyl)-3-azaspiro[5.5]undecane-9-carbaldehyde (124 mg, 0.29 mmol) in DCM/AcOH (1.5 mL/0.15 mL), and the mixture was stirred at 30 °C for 2 h. NaBH(OAc)₃ (121 mg, 0.57 mmol) was added to the mixture in an ice bath, and the resulting mixture was stirred for 2 h. The reaction mixture was concentrated, and the resulting crude product was purified by silica gel column chromatography (DCM:MeOH = 100:1 to 10:1) to give 1-(5-(9-((4-((*R*)-3-((8-fluoro-7-(7-fluoro-3-(methoxymethoxy)-8-((triisopropylsilyl)ethynyl) naphthalen-1-yl)-4-((*R*)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 607.5.

### Step 2: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

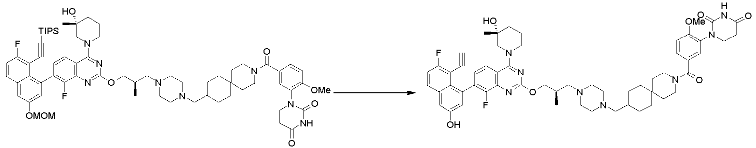

Cesium fluoride (182 mg, 1.20 mmol) was added to a stirred solution of 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione (140 mg, 0.12 mmol) in DMF (1 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1013.4.

¹H NMR (400 MHz, DMSO-*d*₆) δ 10.33 (s, 1H), 10.15 (s, 1H), 7.96 (dd, *J* = 9.1, 6.0 Hz, 1H), 7.81 (dd, *J* = 28.1, 8.5 Hz, 1H), 7.45 (t, *J* = 9.0 Hz, 1H), 7.38 - 7.30 (m, 3H), 7.22 - 7.06 (m, 3H), 4.63 (d, *J =* 12.1 Hz, 1H), 4.50 - 4.36 (m, 1H), 4.15 - 3.95 (m, 2H), 3.91 - 3.76 (m, 5H), 3.59 (t, *J =* 6.6 Hz, 3H), 3.51 - 3.40 (m, 3H), 2.68 (t, *J =* 6.5 Hz, 2H), 2.43 - 1.96 (m, 15H), 1.74 - 1.60 (m, 5H), 1.54 - 1.38 (m, 5H), 1.34 - 1.22 (m, 4H), 1.15 (d, *J =* 7.2 Hz, 3H), 1.07 - 0.95 (m, 6H).

### Example 61: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

### Step 1: preparation of 1-(5-(9-((4-((R)-3-((8-fluoro-7-(7-fluoro-3-(methoxymethoxy)-8-((triisopropylsilyl)ethynyl) naphthalen-1-yl)-4-((R)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

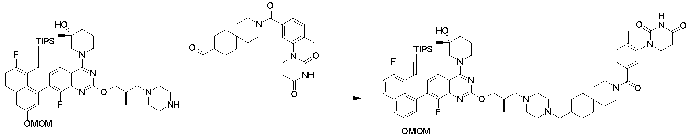

Tetraisopropyl titanate (0.5 mL) was added to a stirred solution of (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)
quinazolin-4-yl)-3-methylpiperidin-3-ol (150 mg, 0.19 mmol) and 3-(3-(2,4-dioxotetrahydropyrimidin-1(2*H*)-yl)-4-methylbenzoyl)-3-azaspiro[5.5]undecane-9-carbaldehyde (119 mg, 0.29 mmol) in DCM/AcOH (*1.5* mL/0.15 mL), and the mixture was stirred at 30 °C for 2 h. NaBH(OAc)₃ (121 mg, 0.57 mmol) was added to the mixture in an ice bath, and the resulting mixture was stirred for 2 h. The reaction mixture was concentrated, and the resulting crude product was purified by silica gel column chromatography (DCM:MeOH = 100:1 to 10:1) to give 1-(5-(9-((4-((*R*)-3-((8-fluoro-7-(7-fluoro-3-(methoxymethoxy)-8-((triisopropylsilyl)ethynyl) naphthalen-1-yl)-4-((*R*)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 599.5.

### Step 2: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)quinazolin-2-yl)oxy)-2-methylpropyl)piperazin-1-yl)methyl)-3-azaspiro [5.5]undecane-3-carbonyl)-2-methylphenyl)dihydropyrimidine-2,4(1H,3H)-dione

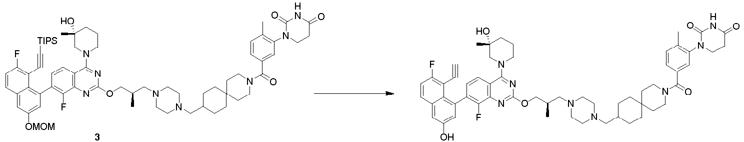

Cesium fluoride (213 mg, 1.40 mmol) was added to a stirred solution of 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione (170 mg, 0.14 mmol) in DMF (1 mL), and the mixture was stirred at room temperature for 30 min. Water (10 mL) was added to the reaction liquid to quench the reaction, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. An aqueous Na₂CO₃ solution was added to the reaction liquid, and the mixture was adjusted to pH > 7, extracted with EtOAc (10 mL × 2), dried over anhydrous Na₂SO₄, and concentrated. The crude product was purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)quinazolin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 499.2.

¹H NMR (400 MHz, DMSO-*d*₆) δ 10.36 (s, 1H), 10.11 (s, 1H), 7.96 (dd, *J* = 9.1, 6.0 Hz, 1H), 7.81 (dd, *J =* 28.6, 8.6 Hz, 1H), 7.45 (t, *J =* 9.0 Hz, 1H), 7.39 - 7.29 (m, 3H), 7.26 - 7.17 (m, 2H), 7.12 - 7.05 (m, 1H), 4.63 (d, *J* = 12.8 Hz, 1H), 4.49 - 4.36 (m, 1H), 4.15 - 3.95 (m, 2H), 3.93 - 3.74 (m, 3H), 3.64 - 3.35 (m, 5H), 2.83 - 2.64 (m, 2H), 2.44 - 1.99 (m, 17H), 1.73 - 1.58 (m, 5H), 1.55 - 1.37 (m, 5H), 1.24 (s, 4H), 1.15 (d, *J =* 7.3 Hz, 3H), 1.10 - 0.91 (m, 7H).

### Example 67: (R)-1-(5-(9-((4-(3-((4-(3,3-difluoropiperidin-1-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5] undecane-3-carbonyl)-2-methoxyphenyl)dihydropyrimidine-2,4(1H,3H)-dione

### Step 1: preparation of (R)-2-(3-(4-benzylpiperazin-1-yl)-2-methylpropoxy)-7-chloro-4-(3,3-difluoropiperidin-1-yl)-8-fluoropyrido[4,3-d]pyrimidine

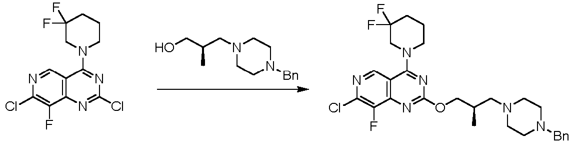

2,7-Dichloro-4-(3,3-difluoropiperidin-1-yl)-8-fluoropyrido[4,3-*d*]pyrimidine (2.2 g, 6.53 mmol) and (*R*)-3-(4-benzylpiperazin-1-yl)-2-methylpropan-1-ol (3.24 g, 13.06 mmol) were dissolved in THF (30 mL), and Cs₂CO₃ (6.38 g, 19.59 mmol) was added. The reaction liquid was heated to 90 °C and reacted for 16 h. After the reaction was completed, the mixture was cooled to room temperature, water (30 mL) was added to the reaction liquid, and the mixture was extracted with ethyl acetate (30 mL × 3). The organic phases were combined, washed with a saturated NaCl solution, dried over anhydrous Na₂SO₄, and filtered, and the filtrate was concentrated. The resulting crude product was subjected to silica gel column chromatography (PE:EtOAc = 1:1) to give (*R*)-2-(3-(4-benzylpiperazin-1-yl)-2-methylpropoxy)-7-chloro-4-(3,3-difluoropiperidin-1-yl)-8-fluoropyrido[4,3-*d*]pyrimidine.

LC-MS: (ESI, m/z): [M+H]⁺ = 549.2.

### Step 2: preparation of (R)-2-(3-(4-benzylpiperazin-1-yl)-2-methylpropoxy)-4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-d]pyrimidine

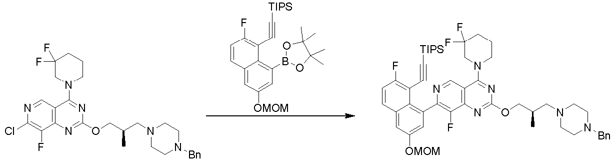

CataCXium A Pd G3 (345 mg, 0.47 mmol) and cesium carbonate (2.32 g, 7.11 mmol) were added to a mixed solution of (*R*)-2-(3-(4-benzylpiperazin-1-yl)-2-methylpropoxy)-7-chloro-4-(3,3-difluoropiperidin-1-yl)-8-fluoropyrido[4,3-*d*]pyrimidine (1.3 g, 2.37 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (1.46 g, 2.84 mmol) in 1,4-dioxane (10 mL) and H₂O (2 mL). The mixture was reacted at 85 °C for 16 h under nitrogen atmosphere. Water (10 mL) was added to the reaction liquid, and the mixture was then extracted with ethyl acetate (10 mL × 3). The organic phases were combined, dried over anhydrous sodium sulfate, and concentrated under reduced pressure. The concentrate was purified by silica gel column chromatography (DCM:MeOH = 20:1) to give (*R*)-2-(3-(4-benzylpiperazin-1-yl)-2-methylpropoxy)-4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-*d*]pyrimidine.

LC-MS: (ESI, m/z): [M/2+H]⁺ = 450.3.

### Step 3: preparation of (R)-4-(3,3-difluoropiperidin-1-yl)-8-fluoro-7-(7-fluoro-3-(methoxymethoxy)-8-((triisopropylsilyl)ethynyl)naphthalen-1-yl)-2-(2-methyl-3-(piperazin-1-yl)propoxy)pyrido[4,3-d]pyrimidine

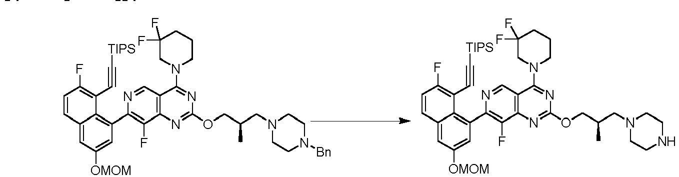

(*R*)-2-(3-(4-Benzylpiperazin-1-yl)-2-methylpropoxy)-4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-*d*]pyrimidine (1.0 g, 1.11 mmol) was dissolved in DCM (5 mL), and 1-chloroethyl chloroformate (476 mg, 3.33 mmol) and DIEA (CAS: 7087-68-5, 430 mg, 3.30 mmol) were added. The mixture was reacted at room temperature for 1 h. The reaction liquid was concentrated under reduced pressure, and the concentrate was dissolved with MeOH (5 mL). The mixture was heated to 50 °C and reacted for 30 min. After the reaction was completed, the mixture was concentrated under reduced pressure, and the concentrate was purified by silica gel column chromatography (DCM:MeOH (NH₃) = 20:1) to give (*R*)-4-(3,3-difluoropiperidin-1-yl)-8-fluoro-7-(7-fluoro-3-(methoxymethoxy)-8-((triisopropylsilyl)ethynyl)naphthalen-1-yl)-2-(2-methyl-3-(piperazin-1-yl)propoxy)
pyrido[4,3-*d*]pyrimidine.

LC-MS: (ESI, m/z): [M+H]⁺ = 809.5.

### Step 4: preparation of (R)-1-(5-(9-((4-(3-((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

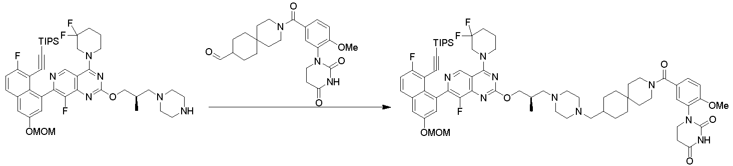

Tetraisopropyl titanate (0.5 mL) was added to a stirred solution of (*R*)-4-(3,3-difluoropiperidin-1-yl)-8-fluoro-7-(7-fluoro-3-(methoxymethoxy)-8-((triisopropylsilyl)ethynyl)naphthalen-1-yl)-2-(2-methyl-3-(piperazin-1-yl)propoxy)pyrido[4,3-*d*]pyrimidine (160 mg, 0.20 mmol) and 3-(3-(2,4-dioxotetrahydropyrimidin-1(2*H*)-yl)-4-methoxybenzoyl)-3-azaspiro[5.5]undecane-9-carbaldehyde (128 mg, 0.30 mmol) in DCM/AcOH (1.5 mL/0.15 mL), and the mixture was stirred at 30 °C for 2 h. NaBH(OAc)₃ (127 mg, 0.60 mmol) was added to the mixture in an ice bath, and the resulting mixture was stirred for 1 h. The mixture was concentrated, and the resulting crude product was purified by silica gel column chromatography (DCM:MeOH = 100:1 to 10:1) to give (*R*)-1-(5-(9-((4-(3-((4-(3,3-difluoropiperidin-1-yl)-8-fluoro-7-(7-fluoro-3-(methoxymethoxy)-8-((triisopropylsilyl) ethynyl)naphthalen-1-yl)pyrido[4,3-*d*]pyrimidin-2-yl)oxy)-2-methylpropyl)piperazin-1-yl)methyl)-3-azaspiro [5.5]undecane-3-carbonyl)-2-methoxyphenyl)dihydropyrimidine-2,4(1*H*,3*H*)-dione.

LC-MS: (ESI, m/z): [M/2+H]⁺ = 611.0.

### Step 5: preparation of (R)-1-(5-(9-((4-(3-((4-(3,3-difluoropiperidin-1-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

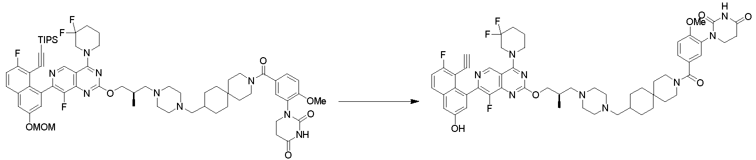

Cesium fluoride (228 mg, 1.50 mmol) was added to a stirred solution of (*R*)-1-(5-(9-((4-(3-((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione (180 mg, 0.15 mmol) in DMF (2 mL), and the mixture was stirred at room temperature for 30 min. Water (10 mL) was added to the reaction liquid, and the mixture was then extracted with ethyl acetate (10 mL × 3). The organic phases were washed with brine, combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phases were dried over anhydrous Na₂SO₄ and concentrated. The resulting crude product was purified by preparative high-performance liquid chromatography to give (*R*)-1-(5-(9-((4-(3-((4-(3,3-difluoropiperidin-1-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1020.1.

¹H NMR (400 MHz, DMSO-*d*₆) δ 10.32 (s, 1H), 10.17 (s, 1H), 9.05 (s, 1H), 7.98 (dd, *J =* 9.2, 6.0 Hz, 1H), 7.46 (t, *J = 9.0* Hz, 1H), 7.42 - 7.30 (m, 3H), 7.23 - 7.19 (m, 1H), 7.17 - 7.12 (m, 1H), 4.56 - 4.44 (m, 1H), 4.31 - 4.08 (m, 3H), 3.93 (s, 3H), 3.84 (s, 3H), 3.68 - 3.36 (m, 6H), 2.75 - 2.66 (m, 2H), 2.40 - 2.11 (m, 12H), 2.07 - 1.96 (m, 4H), 1.71 - 1.63 (m, 2H), 1.54 - 1.38 (m, 5H), 1.33 - 1.21 (m, 3H), 1.11 - 0.94 (m, 7H).

### Example 72: 1-(5-(9-((4-((1-(((4-(3,3-difluoropiperidin-1-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of (1-(((7-chloro-4-(3,3-difluoropiperidin-1-yl)-8-fluoropyrido[4,3-d]pyrimidin-2-yl)oxy)methyl)cyclopropyl)methanol

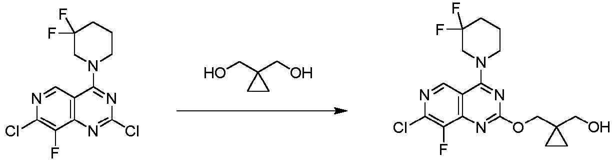

2,7-Dichloro-4-(3,3-difluoropiperidin-1-yl)-8-fluoropyrido[4,3-*d*]pyrimidine (590 mg, 1.75 mmol) and cyclopropane-1,1-diyldimethanol (357 mg, 3.50 mmol) were dissolved in ACN (8 mL), and Cs₂CO₃ (1.71 g, 5.25 mmol) and DABCO (98 mg, 0.875 mmol) were added. The mixture was reacted at room temperature for 1 h. After the reaction was completed, the mixture was filtered to remove the solid, the filter cake was washed twice with dichloromethane, and the filtrate was concentrated to give a crude product. The resulting crude product was purified by silica gel column chromatography (PE:EA = 1:1) to give (1-(((7-chloro-4-(3,3-difluoropiperidin-1-yl)-8-fluoropyrido[4,3-*d*]pyrimidin-2-yl)oxy)methyl)cyclopropyl)methanol.

LC-MS: (ESI, m/z): [M+H]⁺ = 403.1.

### Step 2: preparation of (1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropyl)methanol

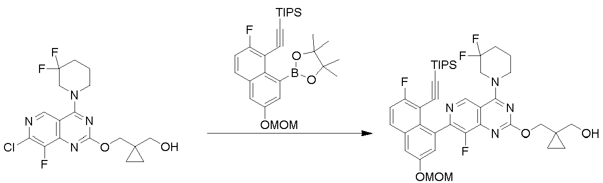

CataCXium A Pd G3 (156 mg, 0.214 mmol) and cesium carbonate (1.05 g, 3.21 mmol) were added to a mixed solution of *tert*-butyl (1-(((7-chloro-4-(3,3-difluoropiperidin-1-yl)-8-fluoropyrido[4,3-*d*]pyrimidin-2-yl)oxy)methyl)cyclopropyl)methanol (430 mg, 1.07 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (658 mg, 1.28 mmol) in 1,4-dioxane (5 mL) and H₂O (1 mL). The mixture was reacted at 85 °C for 16 h under nitrogen atmosphere. Water (10 mL) was added to the reaction liquid, and the mixture was then extracted with ethyl acetate (10 mL × 3). The organic phases were combined, dried over anhydrous sodium sulfate, and concentrated under reduced pressure. The concentrate was purified by silica gel column chromatography (PE:EA = 1:1) to give (1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methanol.

LC-MS: (ESI, m/z): [M+H]⁺ = 753.3.

### Step 3: preparation of 1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropane-1-carbaldehyde

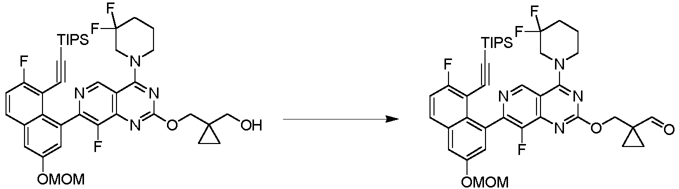

Dess-Martain oxidant (560 mg, 1.32 mmol) was added to a solution of (1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methanol (500 mg, 0.66 mmol) in DCM (3 mL), and the reaction liquid was stirred at room temperature for 1 h. After the reaction was completed, sodium thiosulfate (5 mL) was added to quench the reaction, and the mixture was extracted with ethyl acetate (5 mL × 3). The organic phases were combined, dried over anhydrous sodium sulfate, filtered, and concentrated under reduced pressure, and the concentrate was purified by silica gel column chromatography (PE:EA = 1:1) to give 1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropane-1-carbaldehyde.

LC-MS: (ESI, m/z): [M+H]⁺ = 751.4.

### Step 4: preparation of 1-(5-(9-((4-((1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-d]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1H,3H)-dione

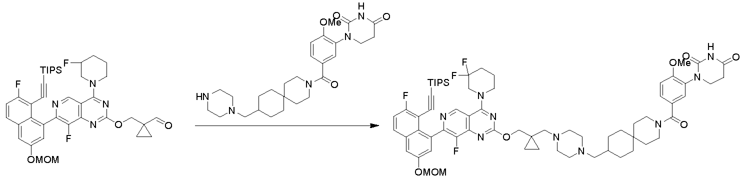

Tetraisopropyl titanate (0.7 mL) was added to a stirred solution of 1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido [4,3-*d*]pyrimidin-2-yl)oxy)methyl)cyclopropane-1-carbaldehyde (200 mg, 0.27 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (204 mg, 0.41 mmol) in DCM/AcOH (2 mL/0.2 mL), and the mixture was stirred at room temperature for 2 h. NaBH(OAc)₃ (172 mg, 0.81 mmol) was added to the mixture in an ice bath, and the resulting mixture was stirred for 1 h. The mixture was concentrated, and the resulting crude product was purified by silica gel column chromatography (DCM:MeOH = 20:1) to give 1-(5-(9-((4-((1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido [4,3-*d*]pyrimidin-2-yl)oxy) methyl)cyclopropyl)methyl)piperazin-1-yl)methyl)-3-azaspiro[5.5]undecane-3-carbonyl)-2-methoxyphenyl)
dihydropyrimidine-2,4(1*H*,3*H*)-dione.

LC-MS: (ESI, m/z): [M/2+H]⁺ = 617.0.

### Step 5: preparation of 1-(5-(9-((4-((1-(((4-(3,3-difluoropiperidin-1-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

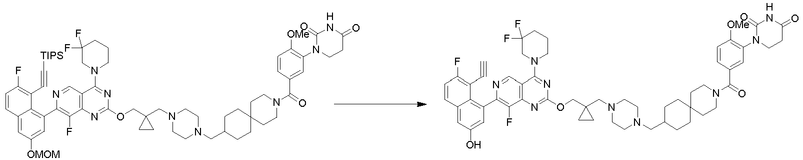

Cesium fluoride (123 mg, 0.81 mmol) was added to a stirred solution of 1-(5-(9-((4-((1-(((4-(3,3-difluoropiperidin-1-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1*H*,3*H*)-dione (100 mg, 0.081 mmol) in DMF (1 mL), and the mixture was stirred at room temperature for 30 min. Water (10 mL) was added to the reaction liquid, and the mixture was then extracted with ethyl acetate (10 mL × 3). The organic phases were combined, dried over anhydrous sodium sulfate, and concentrated under reduced pressure. HCl/1,4-dioxane (6N, 1.0 mL) was added to a stirred solution of the concentrate in 1,4-dioxane (1.0 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((1-(((4-(3,3-difluoropiperidin-1-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1032.0.

¹H NMR (400 MHz, DMSO-*d*₆) δ 10.32 (s, 1H), 10.16 (s, 1H), 9.04 (s, 1H), 7.98 (dd, *J =* 9.2, 5.9 Hz, 1H), 7.47 (t, *J =* 9.0 Hz, 1H), 7.41 - 7.30 (m, 3H), 7.22 - 7.12 (m, 2H), 4.34 - 4.09 (m, 4H), 4.03 - 3.87 (m, 3H), 3.84 (s, 3H), 3.66 - 3.39 (m, 5H), 2.68 (t, *J =* 6.5 Hz, 2H), 2.45 - 1.97 (m, 16H), 1.73 - 1.60 (m, 2H), 1.56 - 1.20 (m, 8H), 1.13 - 0.93 (m, 4H), 0.67 - 0.61 (m, 2H), 0.44 - 0.38 (m, 2H).

### Example 86: 1-(5-(9-((4-(3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro [5.5]undecane-3-carbonyl)-2-methoxyphenyl)dihydropyrimidine-2,4(1H,3H)-dione

### Step 1: preparation of (3R)-1-(2-(3-(4-benzylpiperazin-1-yl)-2-methylpropoxy)-7-chloro-8-fluoropyrido[4,3-d]pyrimidin-4-yl)-3-methylpiperidin-3-ol

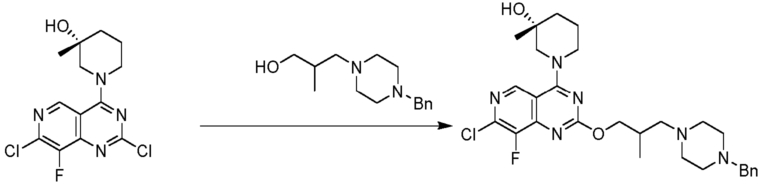

(*R*)-1-(2,7-Dichloro-8-fluoropyrido[4,3-*d*]pyrimidin-4-yl)-3-methylpiperidin-3-ol (2.00 g, 6.04 mmol) and 3-(4-benzylpiperazin-1-yl)-2-methylpropan-1-ol (3.00 g, 12.08 mmol) were dissolved in THF (13 mL) and added to a sealed tube reactor, and Cs₂CO₃ (5.90 g, 18.12 mmol) was added. The mixture was reacted at 90 °C for 16 h. After the reaction was completed, the mixture was cooled to room temperature. Water (30 mL) was added, and the mixture was extracted with ethyl acetate (30 mL × 3). The organic phases were combined, washed with a saturated NaCl solution, dried over anhydrous Na₂SO₄, and filtered, and the filtrate was concentrated. The resulting crude product was subjected to silica gel column chromatography (DCM:MeOH = 20:1) to give (3R)-1-(2-(3-(4-benzylpiperazin-1-yl)-2-methylpropoxy)-7-chloro-8-fluoropyrido[4,3-*d*]pyrimidin-4-yl)-3-methylpiperidin-3-ol.

LC-MS: (ESI, m/z): [M+H]⁺ = 543.3.

### Step 2: preparation of (3R)-1-(2-(3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3-methylpiperidin-3-ol

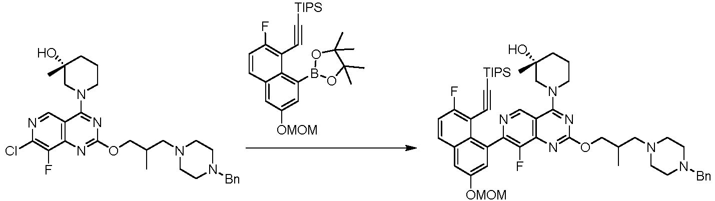

CataCXium A Pd G3 (107 mg, 0.15 mmol) and cesium carbonate (1440 mg, 4.41 mmol) were added to a mixed solution of (3R)-1-(2-(3-(4-benzylpiperazin-1-yl)-2-methylpropoxy)-7-chloro-8-fluoropyrido[4,3-*d*]pyrimidin-4-yl)-3-methylpiperidin-3-ol (800 mg, 1.47 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (906 mg, 1.77 mmol) in 1,4-dioxane (9 mL) and H₂O (1.8 mL). The mixture was reacted at 85 °C for 16 h under nitrogen atmosphere. Water (10 mL) was added to the reaction liquid, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 20:1) to give (3R)-1-(2-(3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3-methylpiperidin-3-ol.

LC-MS: (ESI, m/z): [M+H]⁺ = 893.5.

### Step 3: preparation of (3R)-1-(8-fluoro-7-(7-fluoro-3-(methoxymethoxy)-8-((triisopropylsilyl)ethynyl) naphthalen-1-yl)-2-(2-methyl-3-(piperazin-1-yl)propoxy)pyrido[4,3-d]pyrimidin-4-yl)-3-methylpiperidin-3-ol

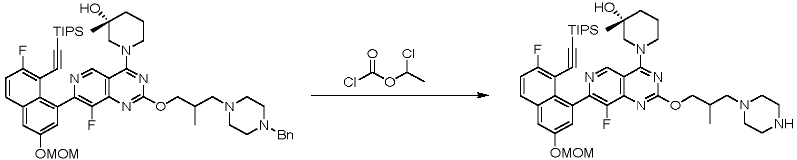

(3*R*)-1-(2-(3-(4-Benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3-methylpiperidin-3-ol (800 mg, 0.90 mmol) was dissolved in DCM (10 mL), and DIEA(232 mg, 1.80 mmol) and 1-chloroethyl chloroformate (193 mg, 1.35 mmol) were added. The mixture was reacted at room temperature for 1 h and concentrated under reduced pressure. MeOH (5 mL) was added to the concentrate, and the mixture was then heated to 50 °C and reacted for 1 h. After the reaction was completed, the mixture was concentrated under reduced pressure, and the concentrate was purified by silica gel column chromatography (DCM:MeOH (NH₃) = 20:1) to give (3*R*)-1-(8-fluoro-7-(7-fluoro-3-(methoxymethoxy)-8-((triisopropylsilyl)ethynyl)naphthalen-1-yl)-2-(2-methyl-3-(piperazin-1-yl)propoxy)pyrido[4,3-*d*]pyrimidin-4-yl)-3-methylpiperidin-3-ol.

LC-MS: (ESI, m/z): [M/2+H]⁺ = 402.3.

### Step 4: preparation of 1-(5-(9-((4-(3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methoxyphenyl) dihydropyrimidine-2,4(1H,3H)-dione

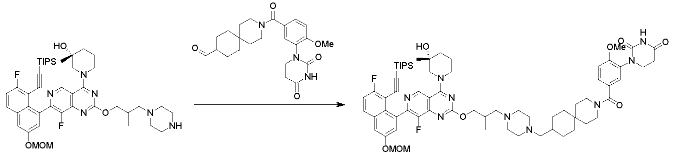

Tetraisopropyl titanate (0.5 mL) was added to a stirred solution of (3*R*)-1-(8-fluoro-7-(7-fluoro-3-(methoxymethoxy)-8-((triisopropylsilyl)ethynyl)naphthalen-1-yl)-2-(2-methyl-3-(piperazin-1-yl)propoxy) pyrido[4,3-*d*]pyrimidin-4-yl)-3-methylpiperidin-3-ol (130 mg, 0.16 mmol) and 3-(3-(2,4-dioxotetrahydropyrimidin-1(2*H*)-yl)-4-methoxybenzoyl)-3 -azaspiro [5.5]undecane-9-carbaldehyde (104 mg, 0.24 mmol) in DCM/AcOH (1.3 mL/0.1 mL), and the mixture was stirred at 30 °C for 2 h. NaBH(OAc)₃ (103 mg, 0.49 mmol) was added to the mixture in an ice bath, and the resulting mixture was stirred for 2 h. The mixture was concentrated and purified by silica gel column chromatography (DCM:MeOH = 100:1 to 10:1) to give 1-(5-(9-((4-(3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido [4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 607.9.

### Step 5: preparation of 1-(5-(9-((4-(3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

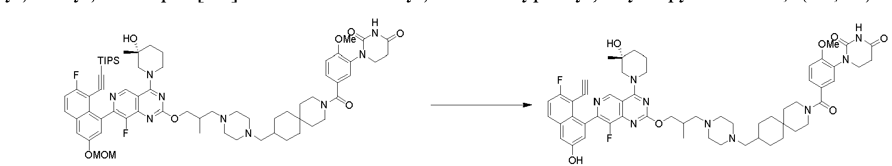

Cesium fluoride (62 mg, 0.41 mmol) was added to a stirred solution of 1-(5-(9-((4-(3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione (100 mg, 0.082 mmol) in DMF (1 mL), and the mixture was stirred at room temperature for 2 h. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. An aqueous Na₂CO₃ solution was added to the reaction liquid to adjust the pH > 7, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(9-((4-(3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1014.4.

¹H NMR (400 MHz, DMSO-*d*₆) δ 10.32 (s, 1H), 10.15 (s, 1H), 9.13 (d, *J* = 60.2 Hz, 1H), 7.97 (dd, *J =* 9.2, 6.0 Hz, 1H), 7.45 (t, *J =* 9.0 Hz, 1H), 7.42 - 7.34 (m, 2H), 7.32 (d, *J =* 2.1 Hz, 1H), 7.21 (dd, *J =* 17.4, 2.5 Hz, 1H), 7.14 (d, *J =* 8.6 Hz, 1H), 4.73 (d, *J =* 20.0 Hz, 1H), 4.53 - 4.24 (m, 2H), 4.17 - 3.92 (m, 3H), 3.84 (s, 3H), 3.68 - 3.36 (m, 7H), 2.68 (t, *J =* 6.6 Hz, 2H), 2.47 - 2.10 (m, 12H), 2.09 - 1.95 (m, 3H), 1.78 - 1.59 (m, 5H), 1.56 - 1.38 (m, 5H), 1.35 - 1.23 (m, 2H), 1.16 *(d, J=* 15.8 Hz, 3H), 1.11 - 0.90 (m, 7H).

### Example 91: (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-4-fluoro-2-methylphenyl)dihydropyrimidine-2,4(1H,3H)-dione

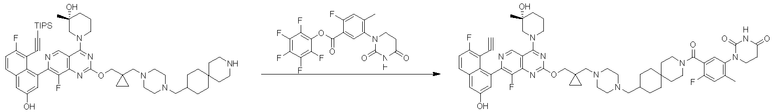

Pentafluorophenyl 5-(2,4-dioxotetrahydropyrimidin-1(2*H*)-yl)-2-fluoro-4-methylbenzoate (43 mg, 99.86 µmol) was added to a solution of the crude product of (*R*)-1-(2-((1-((4-((3-azaspiro[5.5]undecan-9-yl)methyl)piperazin-1-yl)methyl)cyclopropyl)methoxy)-8-fluoro-7-(7-fluoro-3-hydroxy-8-((triisopropylsilyl)
ethynyl)naphthalen-1-yl)pyrido[4,3-*d*]pyrimidin-4-yl)-3-methylpiperidin-3-ol (85 mg, 90.78 µmol) and *N,N*-diisopropylethylamine (59 mg, 453.90 µmol) in N,N-dimethylformamide (2 mL) at room temperature. The mixture was stirred at room temperature for 1 h, cesium fluoride (138 mg, 907.80 µmol) was then added, and the mixture was stirred for another 1 h. After the reaction was completed, the mixture was poured into ice water (60 mL) and extracted with ethyl acetate (20 mL × 3). The organic phases were separately washed with water (60 mL) and saturated sodium chloride (60 mL), dried over anhydrous sodium sulfate, and concentrated under reduced pressure. The resulting crude product was purified by a preparative thin-layer chromatography plate (dichloromethane:methanol = 10:1, 0.1% NH₃) and then purified by preparative high-performance liquid chromatography to give (*R*)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-4-fluoro-2-methylphenyl)dihydropyrimidine-2,4(1*H*,3*H*)-dione.

LC-MS: (ESI, m/z): [M+H]⁺ = 1028.4.

¹H NMR (400 MHz, DMSO-*d*₆) δ 10.41 (s, 1H), 10.21 (br, 1H), 9.15 (d, *J* = 66.0 Hz, 1H), 8.03 - 7.98 (m, 1H), 7.49 (t, *J* = 8.8 Hz, 1H), 7.42 (s, 1H), 7.36 (d, *J* = 5.6 Hz, 1H), 7.28 - 7.21 (m, 2H), 4.82 - 4.75 (m, 1H), 4.40 - 4.20 (m, 3H), 4.20 - 4.10 (m, 1H), 4.10 - 3.95 (m, 1H), 3.85-3.70 (m, 1H), 3.65 - 3.55 (m, 2H), 3.32 - 3.22 (m, 4H), 2.85 - 2.65 (m, 3H), 2.42 - 2.24 (m, 8H), 2.24 - 2.10 (m, 3H), 2.10 - 2.00 (m, 4H), 1.73 - 1.60 (m, 4H), 1.60 - 1.25 (m, 8H), 1.25 - 1.15 (m, 3H), 1.15 - 0.90 (m, 5H), 0.70 - 0.60 (m, 2H), 0.50 - 0.40 (m, 2H).

### Example 92: (R)-1-(2-ethyl-5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

### Step 1: preparation of (R)-1-(2-ethyl-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1H,3H)-dione

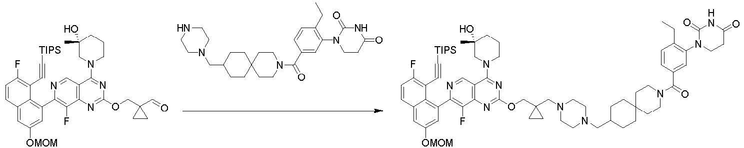

Tetraisopropyl titanate (230 mg, 807.00 µmol) and glacial acetic acid (0.3 mL) were added to a solution of (*R*)-1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropane-1-carbaldehyde (120 mg, 161.40 µmol) and 1-(2-ethyl-5-(9-(piperazin-1-ylmethyl)-3-azaspiro[5.5]undecane-3-carbonyl)phenyl)
dihydropyrimidine-2,4(1*H*,3*H*)-dione (120 mg, 242.10 µmol) in dichloromethane (15 mL) at room temperature, and the mixture stirred at 25 °C for 3 h. Then sodium triacetoxyborohydride (85 mg, 399.90 µmol) was added, and the mixture was reacted at 25 °C for another 16 h. Methanol (5 mL) was added to the reaction liquid, and the mixture was then concentrated under reduced pressure at 40 °C. The resulting crude product was purified by a preparative thin-layer chromatography plate (dichloromethane:methanol = 10:1, a 1% solution of ammonia in methanol) to give (*R*)-1-(2-ethyl-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido [4,3-*d*]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M/2+H]⁺ = 612.9.

### Step 2: preparation of (R)-1-(2-ethyl-5-(9-((4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-d]pyrimidin-2-yl)oxy)methyl)cyclopropyl) methyl)piperazin-1-yl)methyl)-3-azaspiro[5.5]undecane-3-carbonyl)phenyl)dihydropyrimidine-2,4(1H,3H)-dione

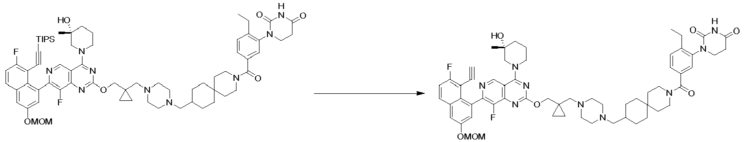

Cesium fluoride (118 mg, 779.02 µmol) was added to a solution of (*R*)-1-(2-ethyl-5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)phenyl)dihydropyrimidine-2,4(1*H*,3*H*)-dione (95.4 mg, 77.90 µmol) in *N,N-*dimethylformamide (10 mL) at room temperature, and the mixture was reacted at 25 °C for 2 h under nitrogen atmosphere. After the reaction was completed, the reaction liquid was poured into water (60 mL), and the mixture was extracted with ethyl acetate (20 mL × 3). The organic phases were separately washed with water (60 mL) and saturated brine (60 mL), dried over anhydrous sodium sulfate, filtered, and concentrated under reduced pressure to give (R)-1-(2-ethyl-5-(9-((4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl) methyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1068.5.

### Step 3: preparation of (R)-1-(2-ethyl-5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl) piperazin-1-yl)methyl)-3-azaspiro[5.5]undecane-3-carbonyl)phenyl)dihydropyrimidine-2,4(1H,3H)-dione

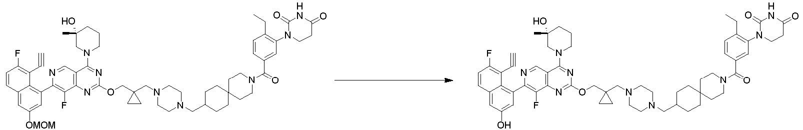

A hydrogen chloride/1,4-dioxane solution (4.0 M, 3 mL) was added to a solution of the crude product of (*R*)-1-(2-ethyl-5-(9-((4-((1-(((7-(8-ethynyl-7-fluoro-3-(methoxymethoxy)naphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)phenyl)dihydropyrimidine-2,4(1*H*,3*H*)-dione (55.3 mg, 51.76 µmol) in 1,4-dioxane (2 mL) at room temperature, and the mixture was reacted at room temperature for 1 h. After the reaction was completed, the mixture was concentrated under reduced pressure at 30 °C, and a solution of ammonia in methanol (5 mL) was added to the concentrate. Then the mixture was concentrated to dryness under reduced pressure at 30 °C. The resulting crude product was purified by a preparative thin-layer chromatography plate (dichloromethane:methanol = 10:1, 0.1% NH₃) and then separated by preparative high-performance liquid chromatography to give (*R*)-1-(2-ethyl-5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)
methyl)cyclopropyl)methyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1024.4.

¹H NMR (400 MHz, DMSO-*d*₆) 10.36 (s, 1H), 10.17 (br, 1H), 9.12 (d, *J =* 65.2 Hz, 1H), 7.99 - 7.95 (m, 1H), 7.46 (t, *J* = 8.8 Hz, 1H), 7.38 - 7.36 (m, 2H), 7.30 - 7.28 (m, 2H), 7.22 - 7.18 (m, 1H), 4.78 - 4.70 (m, 1H), 4.40 - 4.23 (m, 3H), 4.10 - 3.94 (m, 2H), 3.85-3.70 (m, 1H), 3.55 - 3.45 (m, 2H), 2.81 - 2.77 (m, 3H), 2.40 - 2.20 (m, 8H), 2.20 - 1.90 (m, 4H), 1.80 - 1.60 (m, 6H), 1.60 - 1.35 (m, 6H), 1.35 - 1.20 (m, 5H), 1.20 - 1.15 (m, 2H), 1.15 - 1.10 (m, 4H), 1.10 - 0.80 (m, 6H), 0.65 - 0.55 (m, 2H), 0.45 - 0.35 (m, 2H).

### Example 111: Preparation of (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl) piperazin-1-yl)methyl)-3-azaspiro[5.5]undecane-3-carbonyl)-2-(methyl-d3)phenyl)dihydropyrimidine-2,4(1H,3H)-dione

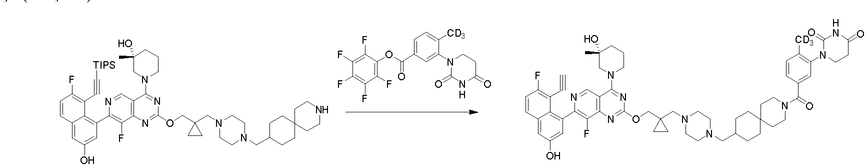

Pentafluorophenyl 3-(2,4-dioxotetrahydropyrimidin-1(2*H*)-yl)-4-(methyl-d3)benzoate (63 mg, 149.79 µmol) was added to a solution of (*R*)-1-(2-((1-((4-((3-azaspiro[5.5]undecan-9-yl)methyl)piperazin-1-yl)methyl)cyclopropyl)methoxy)-8-fluoro-7-(7-fluoro-3-hydroxy-8-((triisopropylsilyl)ethynyl)naphthalen-1-yl)pyrido[4,3-*d*]pyrimidin-4-yl)-3-methylpiperidin-3-ol (150 mg, 136.17 µmol) and *N*,*N*-diisopropylethylamine (176 mg, 1316.71 µmol) in *N*,*N*-dimethylformamide (5 mL) at room temperature. The mixture was stirred at room temperature for 1 h, cesium fluoride (208 mg, 1361.70 µmol) was then added, and the mixture was stirred for another 1 h. After the reaction was completed, the reaction liquid was poured into water (30 mL) and extracted with ethyl acetate (20 mL × 3). The organic phases were separately washed with water (60 mL) and saturated sodium chloride (60 mL), dried over anhydrous sodium sulfate, and concentrated under reduced pressure. The resulting crude product was purified by a preparative thin-layer chromatography plate (dichloromethane:methanol = 10:1, 0.1% NH₃) and then purified by preparative high-performance liquid chromatography to give (*R*)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5]undecane-3-carbonyl)-2-(methyl-d3)phenyl)dihydropyrimidine-2,4(1*H*,3*H*)-dione.

LC-MS: (ESI, m/z): [M+H]⁺ = 1013.4.

¹H NMR (400 MHz, DMSO-*d*₆) 10.36 (s, 1H), 10.17 (br, 1H), 9.12 (d, *J =* 66 Hz, 1H), 7.99 - 7.95 (m, 1H), 7.46 (t, *J* = 8.8 Hz, 1H), 7.39 (s, 1H), 7.33 (d, J = 7.6 Hz, 1H), 7.30 (s, 1H), 7.24 - 7.18 (m, 2H), 4.78 - 4.70 (m, 1H), 4.40 - 4.23 (m, 3H), 4.10 - 3.93 (m, 1H), 3.93 - 3.90 (m, 1H), 3.83-3.76 (m, 1H), 3.57 - 3.54 (m, 2H), 2.81 - 2.67 (m, 3H), 2.40 - 2.18 (m, 10H), 2.18 - 1.95 (m, 4H), 1.85 - 1.20 (m, 15H), 1.20 - 1.15 (m, 3H), 1.08 - 0.85 (m, 4H), 0.70 - 0.55 (m, 2H), 0.45 - 0.35 (m, 2H).

### Example 132: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-(methyl-d3)phenyl)dihydropyrimidine-2,4(1H,3H)-dione

### Step 1: preparation of 1-(5-(9-((4-((R)-3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-(methyl-d3)phenyl) dihydropyrimidine-2,4(1H,3H)-dione

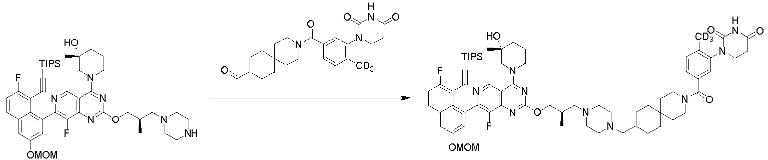

Tetraisopropyl titanate (212 mg, 747.10 µmol) and glacial acetic acid (0.05 mL) were added to a solution of (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3-methylpiperidin-3-ol (120 mg, 149.42 µmol) and 3-(3-(2,4-dioxotetrahydropyrimidin-1(2*H*)-yl)-4-(methyl-d3)benzoyl)-3-azaspiro[5.5]undecane-9-carbaldehyde (93 mg, 224.14 µmol) in dichloromethane (15 mL) at room temperature, and the mixture was stirred at 25 °C for 2 h. Then sodium triacetoxyborohydride (63 mg, 298.84 µmol) was added, and the mixture was reacted at 25 °C for another 16 h. The reaction liquid was concentrated under reduced pressure at 40 °C, and the resulting crude product was purified by a preparative thin-layer chromatography plate (dichloromethane:methanol = 10:1, a 1% solution of ammonia in methanol) to give 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro
undecane-3-carbonyl)-2-(methyl-d3)phenyl)dihydropyrimidine-2,4(1*H*,3*H*)-dione.

LC-MS: (ESI, m/z): [M/2+H]⁺ = 601.4.

### Step 2: preparation of 1-(5-(9-((4-((R)-3-((7-(8-ethynyl-7-fluoro-3-(methoxymethoxy)naphthalen-1-yl)-8-fluoro-4-((R)-3-hydroxy-3-methylpiperidin-1-yl)pyrido[4,3-d]pyrimidin-2-yl)oxy)-2-methylpropyl) piperazin-1-yl)methyl)-3-azaspiro[5.5]undecane-3-carbonyl)-2-(methyl-d3)phenyl)dihydropyrimidine-2,4(1H,3H)-dione

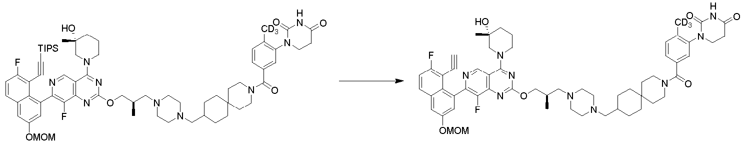

Cesium fluoride (168 mg, 1108.51 µmol) was added to a solution of 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-(methyl-d3)phenyl)dihydropyrimidine-2,4(1*H*,3*H*)-dione (133.2 mg, 110.85 µmol) in *N,N*-dimethylformamide (5 mL) at room temperature, and the mixture was reacted at 25 °C for 2 h under nitrogen atmosphere. After the reaction was completed, the reaction liquid was poured into water (40 mL), and the mixture was extracted with ethyl acetate (20 mL × 3). The organic phases were separately washed with water (60 mL) and saturated brine (60 mL), dried over anhydrous sodium sulfate, filtered, and concentrated under reduced pressure to give 1-(5-(9-((4-((*R*)-3-((7-(8-ethynyl-7-fluoro-3-(methoxymethoxy)naphthalen-1-yl)-8-fluoro-4-((*R*)-3-hydroxy-3-methylpiperidin-1-yl)pyrido[4,3-*d*]pyrimidin-2-yl)oxy)-2-methylpropyl) piperazin-1-yl)methyl)-3-azaspiro[5.5]undecane-3-carbonyl)-2-(methyl-d3)phenyl)dihydropyrimidine-2,4(1*H*,3*H*)-dione.

LC-MS: (ESI, m/z): [M+H]⁺ = 1045.5.

### Step 3: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-(methyl-d3)phenyl)dihydropyrimidine-2,4(1H,3H)-dione

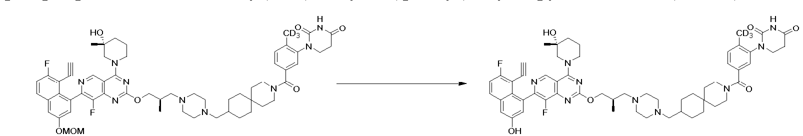

A hydrogen chloride/1,4-dioxane solution (4.0 M, 5 mL) was added to a solution of the crude product of 1-(5-(9-((4-((*R*)-3-((7-(8-ethynyl-7-fluoro-3-(methoxymethoxy)naphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro [5.5]undecane-3-carbonyl)-2-(methyl-d3)phenyl)dihydropyrimidine-2,4(1*H*,3*H*)-dione (144.4 mg, 110.85 µmol) in 1,4-dioxane (2.5 mL) at 0 °C, and the mixture was reacted at room temperature for 1 h. After the reaction was completed, the mixture was concentrated under reduced pressure at 30 °C, and a solution of ammonia in methanol (5 mL) was added to the concentrate. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-(methyl-d3)phenyl)dihydropyrimidine-2,4(1*H*,3*H*)-dione.

LC-MS: (ESI, m/z): [M+H]⁺ = 1001.4.

¹H NMR (400 MHz, DMSO-*d*₆) δ 10.37 (s, 1H), 10.19 (br, 1H), 9.13 (d, *J =* 60.8 Hz, 1H), 7.99 - 7.95 (m, 1H), 7.46 (t, *J =* 8.8 Hz, 1H), 7.39 (s, 1H), 7.33 (d, *J =* 8.0 Hz, 1H), 7.30 (s, 1H), 7.25 - 7.23 (m, 2H), 4.78 - 4.73 (m, 1H), 4.50 - 4.44 (m, 1H), 4.40 - 4.25 (m, 1H), 4.18 - 3.95 (m, 2H), 3.95 - 3.90 (m, 1H), 3.90 - 3.83 (m, 1H), 3.60 - 3.54 (m, 2H), 2.80 - 2.62 (m, 3H), 2.40 - 1.90 (m, 15H), 1.80 - 1.55 (m, 6H), 1.55 - 1.20 (m, 8H), 1.19 - 1.15 (m, 3H), 1.15 - 0.70 (m, 8H).

### Example 133: 1-(2-ethyl-5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)phenyl)dihydropyrimidine-2,4(1H,3H)-dione

### Step 1: preparation of 1-(2-ethyl-5-(9-((4-((R)-3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)phenyl)dihydropyrimidine-2,4(1H,3H)-dione

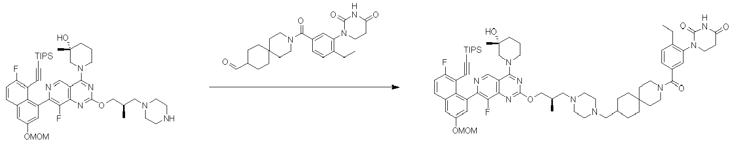

Tetraisopropyl titanate (142 mg, 498.10 µmol) and glacial acetic acid (0.1 mL) were added to a stirred solution of (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3-methylpiperidin-3-ol (80 mg, 99.62 µmol) and 3-(3-(2,4-dioxotetrahydropyrimidin-1(2*H*)-yl)-4-ethylbenzoyl)-3-azaspiro[5.5]undecane-9-carbaldehyde (77 mg, 179.31 µmol) in dichloromethane (2 mL) at room temperature, and the mixture was stirred at 25 °C for 2 h. Then sodium triacetoxyborohydride (43 mg, 199.24 µmol) was added, and the mixture was reacted at 25 °C for another 16 h. The reaction liquid was concentrated under reduced pressure at 40 °C, and the resulting crude product was purified by a preparative thin-layer chromatography plate (dichloromethane:methanol = 10:1, a 1% solution of ammonia in methanol) to give 1-(2-ethyl-5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1212.6.

### Step 2: preparation of 1-(2-ethyl-5-(9-((4-((R)-3-((7-(8-ethynyl-7-fluoro-3-(methoxymethoxy)naphthalen-1-yl)-8-fluoro-4-((R)-3-hydroxy-3-methylpiperidin-1-yl)pyrido[4,3-d]pyrimidin-2-yl)oxy)-2-methylpropyl) piperazin-1-yl)methyl)-3-azaspiro[5.5]undecane-3-carbonyl)phenyl)dihydropyrimidine-2,4(1H,3H)-dione

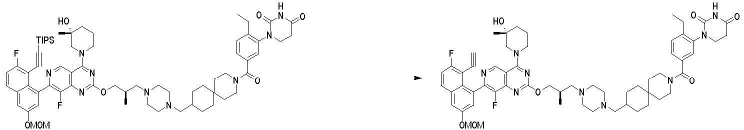

Cesium fluoride (119 mg, 781.78 µmol) was added to a solution of 1-(2-ethyl-5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)phenyl)dihydropyrimidine-2,4(1*H*,3*H*)-dione (94.8 mg, 78.18 µmol) in *N*,*N*-dimethylformamide (10 mL) at room temperature, and the mixture was reacted at 25 °C for 1 h under nitrogen atmosphere. After the reaction was completed, the reaction liquid was poured into water (60 mL), and the mixture was extracted with ethyl acetate (20 mL × 3). The organic phases were separately washed with water (60 mL) and saturated brine (60 mL), dried over anhydrous sodium sulfate, filtered, and concentrated under reduced pressure to give 1-(2-ethyl-5-(9-((4-((*R*)-3-((7-(8-ethynyl-7-fluoro-3-(methoxymethoxy)naphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1056.5.

### Step 3: preparation of 1-(2-ethyl-5-(9-((4-((R)-3-((7-(8-ethynyl-7-fluoro-3-hydroxynaphthalen-1-yl)-8-fluoro-4-((R)-3-hydroxy-3-methylpiperindin-1-yl)pyrido[4,3-d]pyrimidin-2-yl)oxy)-2-methylpropyl)piperazin-1-yl)methyl)-3-azaspiro[5.5]undecane-3-carbonyl)phenyl)dihydropyrimidine-2,4(1H,3H)-dione

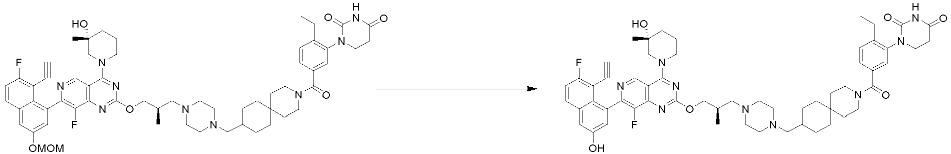

A hydrogen chloride/1,4-dioxane solution (4.0 M, 5 mL) was added to a solution of the crude product of 1-(2-ethyl-5-(9-((4-((*R*)-3-((7-(8-ethynyl-7-fluoro-3-(methoxymethoxy)naphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)phenyl)dihydropyrimidine-2,4(1*H*,3*H*)-dione (104.2 mg, 87.74 µmol) in 1,4-dioxane (2.5 mL) at 0 °C, and the mixture was reacted at room temperature for 1 h. After the reaction was completed, the mixture was concentrated under reduced pressure at 30 °C, and a solution of ammonia in methanol (5 mL) was added to the concentrate. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(2-ethyl-5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺ = 1012.4.

¹H NMR (400 MHz, DMSO-*d*₆) δ 10.37 (s, 1H), 10.18 (br, 1H), 9.13 (d, *J =* 60.0 Hz, 1H), 7.99 - 7.95 (m, 1H), 7.46 (t*, J =* 8.8 Hz, 1H), 7.39 - 7.36 (m, 2H), 7.30 - 7.24 (m, 2H), 7.23 - 7.19 (m, 1H), 4.78 - 4.73 (m, 1H), 4.50 - 4.47 (m, 1H), 4.44 - 4.29 (m, 1H), 4.11 - 3.77 (m, 5H), 3.58 - 3.50 (m, 2H), 2.81 - 2.65 (m, 3H), 2.41 - 1.99 (m, 15H), 1.90 - 1.58 (m, 6H), 1.58 - 1.25 (m, 9H), 1.25 - 1.15 (m, 6H), 1.15 - 0.95 (m, 8H).

### Example 162: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-4-fluoro-2-methylphenyl)dihydropyrimidine-2,4(1H,3H)-dione

### Step 1: preparation of 1-(4-fluoro-5-(9-((4-((R)-3-((8-fluoro-7-(7-fluoro-3-(methoxymethoxy)-8-((triisopropylsilyl)ethynyl)naphthalen-1-yl)-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

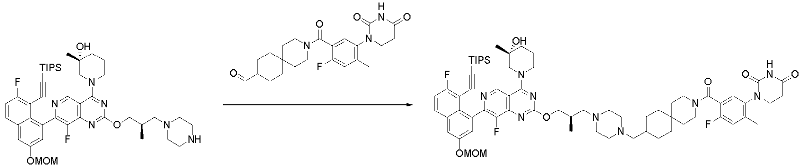

Tetraisopropyl titanate (212 mg, 747.10 µmol) and glacial acetic acid (0.3 mL) were added to a stirred solution of (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3-methylpiperidin-3-ol (120 mg, 149.42 µmol) and 3-(5-(2,4-dioxotetrahydropyrimidin-1(2*H*-yl)-2-fluoro-4-methylbenzoyl)-3-azaspiro[5.5]undecane-9-carbaldehyde (116 mg, 268.96 µmol) in dichloromethane (10 mL) at room temperature, and the mixture was stirred at 25 °C for 2 h. Then sodium triacetoxyborohydride (95 mg, 448.26 µmol) was added, and the mixture was reacted at 25 °C for another 16 h. The mixture was concentrated under reduced pressure at 40 °C, and the resulting crude product was purified by a preparative thin-layer chromatography plate (dichloromethane:methanol = 10:1, a 1% solution of ammonia in methanol) to give 1-(4-fluoro-5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1216.6.

### Step 2: preparation of 1-(5-(9-((4-((R)-3-((7-(8-ethynyl-7-fluoro-3-(methoxymethoxy)naphthalen-1-yl)-8-fluoro-4-((R)-3-hydroxy-3-methylpiperidin-1-yl)pyrido[4,3-d]pyrimidin-2-yl)oxy)-2-methylpropyl) piperazin-1-yl)methyl)-3-azaspiro[5.5]undecane-3-carbonyl)-4-fluoro-2-methylphenyl)dihydropyrimidine-2,4(1H,3H)-dione

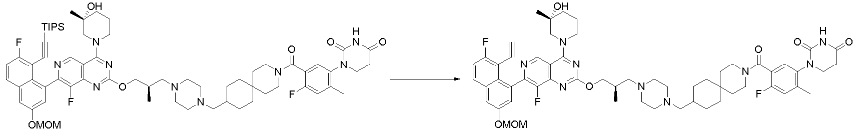

Cesium fluoride (79 mg, 517.85 µmol) was added to a solution of 1-(4-fluoro-5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione (63.0 mg, 51.78 µmol) in *N,N-*dimethylformamide (5 mL) at room temperature, and the mixture was reacted at 25 °C for 1 h under nitrogen atmosphere. After the reaction was completed, the reaction liquid was poured into water (60 mL), and the mixture was extracted with ethyl acetate (20 mL × 3). The organic phases were separately washed with water (60 mL) and saturated brine (60 mL), dried over anhydrous sodium sulfate, filtered, and concentrated under reduced pressure to give 1-(5-(9-((4-((*R*)-3-((7-(8-ethynyl-7-fluoro-3-(methoxymethoxy)naphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-4-fluoro-2-methylphenyl)dihydropyrimidine-2,4(1*H*,3*H*)-dione.

LC-MS: (ESI, m/z): [M+H]⁺ = 1060.5.

### Step 3: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-4-fluoro-2-methylphenyl)dihydropyrimidine-2,4(1H,3H)-dione

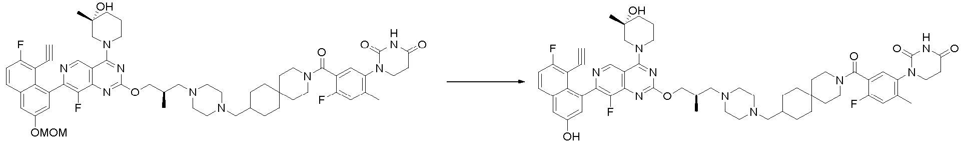

A hydrogen chloride/1,4-dioxane solution (4.0 M, 5 mL) was added to a solution of the crude product of 1-(5-(9-((4-((*R*)-3-((7-(8-ethynyl-7-fluoro-3-(methoxymethoxy)naphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro [5.5]undecane-3-carbonyl)-4-fluoro-2-methylphenyl)dihydropyrimidine-2,4(1*H*,3*H*)-dione (67.8 mg, 51.78 µmol) in 1,4-dioxane (2.5 mL) at 0 °C, and the mixture was reacted at room temperature for 1 h. After the reaction was completed, the mixture was concentrated under reduced pressure at 30 °C, and a solution of ammonia in methanol (5 mL) was added to the concentrate. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-4-fluoro-2-methylphenyl)dihydropyrimidine-2,4(1*H*,3*H*)-dione.

LC-MS: (ESI, m/z): [M+H]⁺ = 1016.4.

¹H NMR (400 MHz, CD₃OD) δ 9.10 (d, *J =* 54.8 Hz, 1H), 7.86 - 7.82 (m, 1H), 7.34 - 7.28 (m, 3H), 7.25 - 7.20 (m, 1H), 7.17 (d, *J* = 9.2 Hz, 1H), 4.63 - 4.48 (m, 2H), 4.32 - 4.18 (m, 2H), 3.87 - 3.80 (m, 1H), 3.80 - 3.48 (m, 5H), 3.48 - 3.38 (m, 1H), 3.38 - 3.34 (m, 2H), 2.91 - 2.76 (m, 2H), 2.50 - 2.30 (m, 8H), 2.30 - 2.15 (m, 3H), 2.15 - 2.10 (m, 4H), 1.90 - 1.30 (m, 14H), 1.30 - 1.20 (m, 3H), 1.20 - 1.10 (m, 4H), 1.10 - 1.00 (m, 3H).

### Example 163: 1-(5-(9-((4-((1-(((4-(3-amino-3-methylpiperidin-1-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro [5.5]undecane-3-carbonyl)-2-methoxyphenyl)dihydropyrimidine-2,4(1H,3H)-dione

### Step 1: preparation of tert-butyl (1-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3-methylpiperidin-3-yl)carboxylate

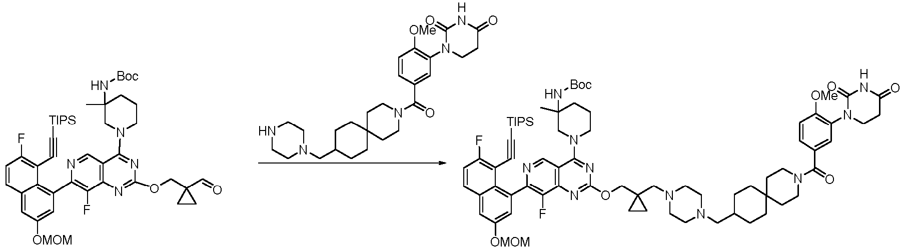

Tetraisopropyl titanate (252 mg, 0.899 mmol) was added to a solution of *tert*-butyl (1-(8-fluoro-7-(7-fluoro-3-(methoxymethoxy)-8-((triisopropylsilyl)ethynyl)naphthalen-1-yl)-2-((1-formylcyclopropyl)methoxy)pyrido [4,3-*d*]pyrimidin-4-yl)-3-methylpiperidin-3-yl)carboxylate (150 mg, 0.178 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (106 mg, 0.213 mmol) in dichloromethane (2 mL) at room temperature, and the mixture was stirred at 25 °C for 16 h. Sodium triacetoxyborohydride (118 mg, 0.533 mmol) was added to the mixture at 0 °C, and the mixture was reacted at 25 °C for another 3 h. Methanol (5 mL) was added to quench the reaction, and the mixture was concentrated under reduced pressure at 40 °C. The resulting crude product was purified by a preparative thin-layer chromatography plate (dichloromethane:methanol = 10:1, 0.1% NH₃) to give *tert-*butyl (1-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3-methylpiperidin-3-yl)carboxylate.

LC-MS: (ESI, m/z): [M+H]⁺ = 1325.8.

### Step 2: preparation of 1-(5-(9-((4-((1-(((4-(3-amino-3-methylpiperidin-1-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

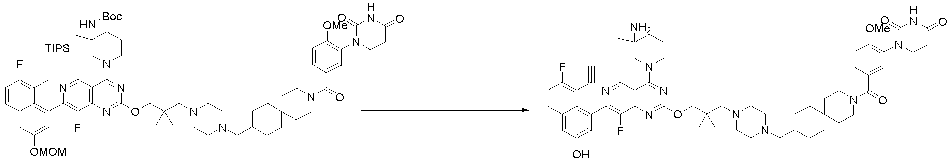

CsF (83 mg, 0.547 mmol) was added to a solution of *tert*-butyl (1-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3-methylpiperidin-3-yl)carboxylate (145 mg, 0.109 mmol) in DMF (2 mL), and the mixture was stirred at room temperature for half an hour. Water (2 mL) was added, and the mixture was extracted with ethyl acetate (5 mL × 3). The organic phases were combined, dried over anhydrous sodium sulfate, and concentrated under reduced pressure. The intermediate obtained by concentration was dissolved in 1,4-dioxane (2 mL), and HCl/1,4-dioxane (6 N, 1 mL) was added. The mixture was reacted at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure, and the resulting crude product was purified by preparative high-performance liquid chromatography to give 1-(5-(9-((4-((1-(((4-(3-amino-3-methylpiperidin-1-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 513.3.

¹H NMR (400 MHz, DMSO-*d*₆) δ 10.32 (s, 1H), 9.22 (d, *J =* 54.4 Hz, 1H), 7.97 (dd, *J =* 8.8 Hz, 6.0 Hz, 1H), 7.46 (t, *J* = 8.8 Hz, 1H), 7.39-7.35 (m, 2H), 7.31-7.30 (m, 1H), 7.20-7.19 (m, 1H), 7.14 (d,*J* = 8.4 Hz, 1H), 4.30 - 4.24 (m, 2H), 4.11 - 4.06 (m, 1H), 3.95 -3.93 (m, 1H), 3.87 - 3.80 (m, 4H), 3.67 (s, 1H), 3.61 - 3.54 (m, 6H), 2.69 - 2.66 (m, 3H), 2.38 - 2.28 (m, 9H), 2.05 - 1.99 (m, 3H), 1.70 - 1.35 (m 15H), 1.29 - 0.90 (m, 8H), 0.66 - 0.58 (m, 2H), 0.43 - 0.37 (m, 2H).

### Example 164: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-(methoxy-d3)phenyl)dihydropyrimidine-2,4(1H,3H)-dione

### Step 1: preparation of 1-(5-(9-((4-((R)-3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-(methoxy-d3)phenyl) dihydropyrimidine-2,4(1H,3H)-dione

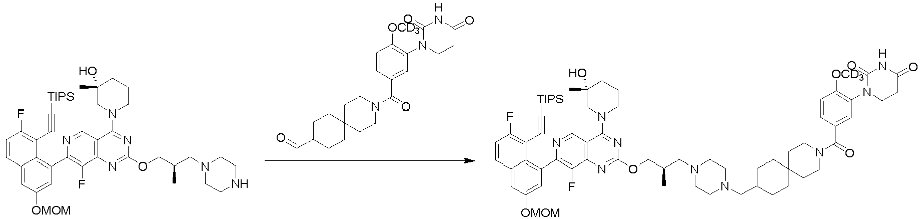

Tetraisopropyl titanate (0.2 mL) was added to a solution of (*R*)-1-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3-methylpiperidin-3-ol (90 mg, 0.11 mmol) and 3-(3-(2,4-dioxotetrahydropyrimidin-1(2*H*)-yl)-4-(methoxy-d3)benzoyl)-3-azaspiro[5.5]undecane-9-carbaldehyde (72 mg, 0.17 mmol) in DCM/AcOH (1.5 mL/0.15 mL), and the mixture was stirred at 30 °C for 2 h. NaBH(OAc)₃ (72 mg, 0.33 mmol) was added to the mixture in an ice bath, and the resulting mixture was stirred for 2 h. The mixture was concentrated and purified by silica gel column chromatography (DCM:MeOH = 10:1) to give 1-(5-(9-((4-((*R*)-3-((8-fluoro-7-(7-fluoro-3-(methoxymethoxy)-8-((triisopropylsilyl)ethynyl)naphthalen-1-yl)-4-((R)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-(methoxy-d3)phenyl)dihydropyrimidine-2,4(1*H*,3*H*)-dione.

LC-MS: (ESI, m/z): [M/2+H]⁺ = 609.9.

### Step 2: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methylpropyl)piperazin-1-yl)methyl)-3-azaspiro[5.5]undecane-3-carbonyl)-2-(methoxy-d3)phenyl)dihydropyrimidine-2,4(1H,3H)-dione

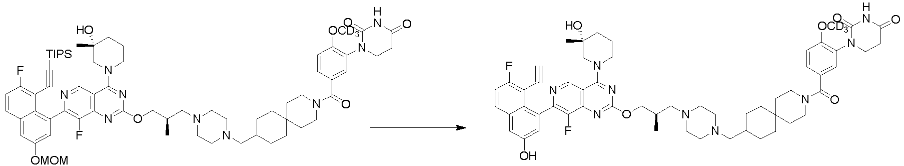

Cesium fluoride (56 mg, 0.34 mmol) was added to a solution of 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]|pyrimidin-2-yl)oxy)-2-methylpropyl)piperazin-1-yl)methyl)-3-azaspiro[5.5]undecane-3-carbonyl)-2-(methoxy-d3)phenyl)dihydropyrimidine-2,4(1*H*,3*H*)-dione (90 mg, 0.074 mmol) in DMF (1 mL), and the mixture was stirred at room temperature for 30 min. Water (20 mL) was added to the reaction liquid, and the mixture was extracted with ethyl acetate (20 mL × 3). The organic phases were dried over anhydrous sodium sulfate and concentrated under reduced pressure. HCl/1,4-dioxane (6 N, 1.0 mL) was added to a stirred solution of the concentrate in 1,4-dioxane (2 mL), and the mixture was reacted at room temperature for 0.5 h. The reaction liquid was adjusted to pH > 7 with an aqueous Na₂CO₃ solution, and the mixture was extracted with EtOAc (20 mL × 2). The organic phase was washed with brine, dried over anhydrous Na₂SO₄, and concentrated. The crude product was purified by preparative high-performance liquid chromatography to give 1-(5-(9-((4-((*R*)-3-((7-(8-cthynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-(methoxyd3)phenyl)dihydropyrimidine-2,4(1*H*,3*H*)-dione.

LC-MS: (ESI, m/z): [M+H]⁺ = 1017.4.

¹H NMR (400 MHz, DMSO-*d*₆) δ 10.32 (s, 1H), 10.14 (d, *J* = 4.1 Hz, 1H), 9.13 (d, *J* = 59.9 Hz, 1H), 7.97 (dd, *J* = 9.2, 5.9 Hz, 1H), 7.46 (t, *J* = 9.0 Hz, 1H), 7.41 - 7.33 (m, 2H), 7.31 (d, *J* = 1.9 Hz, 1H), 7.21 (dd, *J* = 17.9, 2.4 Hz, 1H), 7.14 (d, *J* = 8.5 Hz, 1H), 4.73 (d, *J* = 19.8 Hz, 1H), 4.54 - 4.42 (m, 1H), 4.40 - 4.24 (m, 1H), 4.16 - 3.90 (m, 3H), 3.64 - 3.39 (m, 6H), 2.68 (t, *J* = 6.4 Hz, 2H), 2.40 - 1.98 (m, 14H), 1.73 - 1.60 (m, 5H), 1.55 - 1.23 (m, 8H), 1.16 (d, *J* = 15.6 Hz, 3H), 1.12 - 0.88 (m, 8H).

### Example 165: 1-(5-(9-((4-((1-(((7-(8-ethynyl-7-fluoro-3-hydroxynaphthalen-1-yl)-8-fluoro-4-(6-hydroxy-6-methyl-1,4-oxazepan-4-yl)pyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of 4-(2,7-dichloro-8-fluoropyrido[4,3-d]pyrimidin-4-yl)-6-methyl-1,4-oxazepan-6-ol

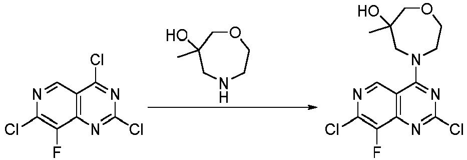

Triethylamine (362 mg, 3.579 mmol) and 6-methyl-1,4-oxazepan-6-ol (200 mg, 1.193 mmol) were added to a solution of 2,4,7-trichloro-8-fluoropyrido[4,3-*d*]pyrimidine (300 mg, 1.193 mmol) in anhydrous dichloromethane (30 mL) at -50 °C, and the mixture was stirred at -50 °C for 5 h. After the reaction was completed, the mixture was poured into water (20 mL) and extracted with ethyl acetate (20 mL × 2). The organic phases were combined, washed with saturated sodium chloride (30 mL), dried over anhydrous sodium sulfate, and concentrated under reduced pressure. 4-(2,7-Dichloro-8-fluoropyrido[4,3-*d*]pyrimidin-4-yl)-6-methyl-1,4-oxazepan-6-ol was obtained.

LC-MS: (ESI, m/z): [M+H]⁺ = 346.9.

### Step 2: preparation of 4-(7-chloro-8-fluoro-2-((1-(hydroxymethyl)cyclopropyl)methoxy)pyrido[4,3-d]pyrimidin-4-yl)-6-methyl-1,4-oxazepan-6-ol

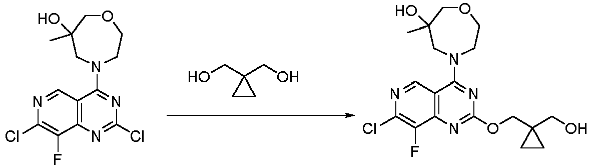

Triethylenediamine (67 mg, 0.597 mmol) and anhydrous cesium carbonate (777 mg, 2.386 mmol) were added to a solution of 4-(2,7-dichloro-8-fluoropyrido[4,3-*d*]pyrimidin-4-yl)-6-methyl-1,4-oxazepan-6-ol (427.6 mg, 1.193 mmol) and cyclopropane-1,1-dimethyldimethanol (244 mg, 2.386 mmol) in anhydrous acetonitrile (20 mL), and the mixture was stirred at 25 °C for 18 h. After the reaction was completed, the mixture was poured into water (100 mL) and extracted with ethyl acetate (20 mL × 2). The organic phases were combined, washed with saturated sodium chloride (100 mL), dried over anhydrous sodium sulfate, and concentrated under reduced pressure to give 4-(7-chloro-8-fluoro-2-((1-(hydroxymethyl)cyclopropyl)methoxy)pyrido[4,3-*d*]pyrimidin-4-yl)-6-methyl-1,4-oxazepan-6-ol.

LC-MS: (ESI, m/z): [M+H]⁺ = 412.9.

### Step 3: preparation of 4-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido [4,3-d]pyrimidin-4-yl)-6-methyl-1,4-oxazepan-6-ol

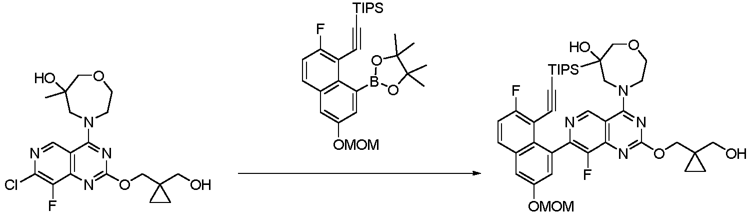

Anhydrous cesium carbonate (237 mg, 726.66 µmol) and cataCXium(R) A Pd G3 (35 mg, 48.44 µmol) were added to a solution of 4-(7-chloro-8-fluoro-2-((1-(hydroxymethyl)cyclopropyl)methoxy)pyrido[4,3-*d*]pyrimidin-4-yl)-6-methyl-1,4-oxazepan-6-ol (100 mg, 242.22 µmol) and ((2-fluoro-6-(methoxymethoxy)-8-(4,4,5,5-tetramethyl-1,3,2-dioxaborolan-2-yl)naphthalen-1-yl)ethynyl)triisopropylsilane (248 mg, 484.44 µmol) in 1,4-dioxane (8 mL) and water (2 mL) at room temperature. After the system was purged with nitrogen, the mixture was stirred at 75 °C for 18 h under nitrogen atmosphere. After the reaction was completed, the mixture was poured into water (100 mL) and extracted with ethyl acetate (20 mL × 2). The organic phases were combined, washed with saturated sodium chloride (100 mL), dried over anhydrous sodium sulfate, filtered and concentrated under reduced pressure. The crude product was purified by silica gel thin-layer chromatography (dichloromethane:methanol = 10:1, 0.1% NH₃) to give 4-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido [4,3-*d*]pyrimidin-4-yl)-6-methyl-1,4-oxazepan-6-ol.

LC-MS: (ESI, m/z): [M+H]⁺ = 763.4.

### Step 4: preparation of 1-(((8-fluoro-7-(7-fluoro-3-(methoxymethoxy)-8-((triisopropylsilyl)ethynyl)naphthalen-1-yl)-4-(6-hydroxy-6-methyl-1,4-oxazepan-4-yl)pyrido [4,3-d]pyrimidin-2-yl)oxy)methyl)cyclopropane-1-carbaldehyde

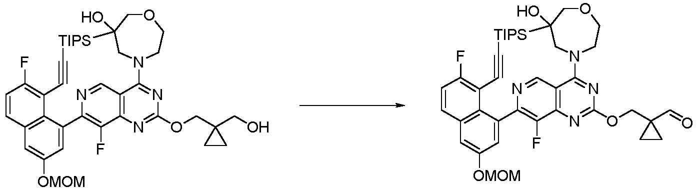

A solution of 4-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido [4,3-*d*]pyrimidin-4-yl)-6-methyl-1,4-oxazepan-6-ol (146 mg, 191.10 µmol) in dichloromethane (15 mL) was cooled to 0 °C from room temperature, and Dess-Martin reagent (105 mg, 248.43 µmol) was added. The mixture was stirred at 25 °C for 2 h. After the reaction was completed, the mixture was poured into a saturated aqueous sodium bicarbonate solution (100 mL), and extracted with dichloromethane (20 mL × 2). The organic phases were combined, washed with saturated sodium chloride (100 mL), dried over anhydrous sodium sulfate, filtered and concentrated under reduced pressure to give 1-(((8-fluoro-7-(7-fluoro-3-(methoxymethoxy)-8-((triisopropylsilyl)ethynyl)naphthalen-1-yl)-4-(6-hydroxy-6-methyl-1,4-oxazepan-4-yl)pyrido[4,3-*d*]pyrimidin-2-yl)oxy)methyl)cyclopropane-1-carbaldehyde.

LC-MS: (ESI, m/z): [M+H]⁺ = 761.3.

### Step 5: preparation of 1-(5-(9-((4-((1-(((8-fluoro-7-(7-fluoro-3-(methoxymethoxy)-8-((triisopropylsilyl)ethynyl) naphthalen-1-yl)-4-(6-hydroxy-6-methyl-1,4-oxazepan-4-yl)pyrido[4,3-d]pyrimidin-2-yl)oxy)methyl)cyclopropyl) methyl)piperazin-1-yl)methyl)-3-azaspiro[5.5]undecane-3-carbonyl)-2-methoxyphenyl)dihydropyrimidine-2,4(1H,3H)-dione

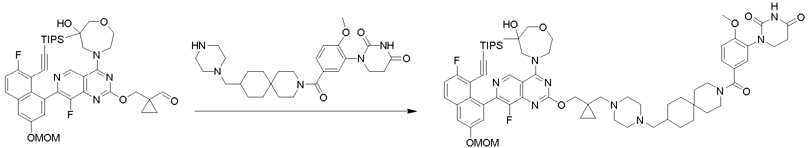

Tetraisopropyl titanate (168 mg, 591.35 µmol) and glacial acetic acid (0.3 mL) were added to a stirred solution of 1-(((8-fluoro-7-(7-fluoro-3-(methoxymethoxy)-8-((triisopropylsilyl)ethynyl)naphthalen-1-yl)-4-(6-hydroxy-6-methyl-1,4-oxazepan-4-yl)pyrido[4,3-*d*]pyrimidin-2-yl)oxy)methyl)cyclopropane-1-carbaldehyde (90 mg, 118.27 µmol) and 1-(2-methoxy-5-(9-(piperazin-1-ylmethyl)-3-azaspiro[5.5]undecane-3-carbonyl)phenyl) dihydropyrimidine-2,4(1*H*,3*H*)-dione (71 mg, 141.92 µmol) in dichloromethane (10 mL) at room temperature, and the mixture stirred at 25 °C for 2 h. Then sodium triacetoxyborohydride (75 mg, 354.81 µmol) was added, and the mixture was reacted at 25 °C for another 16 h. Methanol (5 mL) was added to quench the reaction, and the mixture was concentrated under reduced pressure at 40 °C. The resulting crude product was purified by a preparative thin-layer chromatography plate (dichloromethane:methanol = 10:1, a 1% solution of ammonia in methanol) to give 1-(5-(9-((4-((1-(((8-fluoro-7-(7-fluoro-3-(methoxymethoxy)-8-((triisopropylsilyl)ethynyl) naphthalen-1-yl)-4-(6-hydroxy-6-methyl-1,4-oxazepan-4-yl)pyrido[4,3-*d*]pyrimidin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1*H*,3*H*)-dione.

LC-MS: (ESI, m/z): [M+H]⁺ = 1242.8.

### Step 6: preparation of 1-(5-(9-((4-((1-(((7-(8-ethynyl-7-fluoro-3-hydroxynaphthalen-1-yl)-8-fluoro-4-(6-hydroxy-6-methyl-1,4-oxazepan-4-yl)pyrido[4,3-d]pyrimidin-2-yl)oxy)methyl)cyclopropyl)methyl) piperazin-1-yl)methyl)-3-azaspiro[5.5]undecane-3-carbonyl)-2-methoxyphenyl)dihydropyrimidine-2,4(1H,3H)-dione

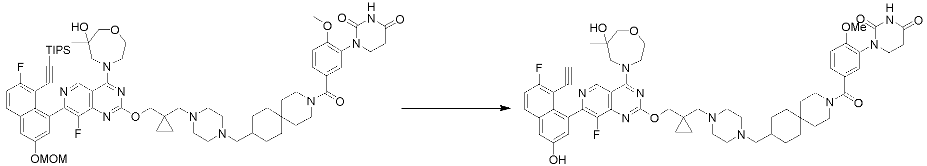

Cesium fluoride (165 mg, 1085.63 µmol) was added to a solution of 1-(5-(9-((4-((1-(((8-fluoro-7-(7-fluoro-3-(methoxymethoxy)-8-((triisopropylsilyl)ethynyl)naphthalen-1-yl)-4-(6-hydroxy-6-methyl-1,4-oxazepan-4-yl)pyrido[4,3-*d*]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1*H*,3*H*)-dione (134.9 mg, 108.56 µmol) in *N,N*-dimethylformamide (10 mL) at room temperature, and the mixture was reacted at 25 °C for 2 h under nitrogen atmosphere. After the reaction was completed, the reaction liquid was poured into water (60 mL) and extracted with ethyl acetate (20 mL × 3). The organic phases were separately washed with water (60 mL) and saturated brine (60 mL), and the combined organic phase was dried over anhydrous sodium sulfate, filtered, and concentrated under reduced pressure. After the concentrate was dissolved in a 1,4-dioxane solution (2.5 mL), a hydrogen chloride/1,4-dioxane solution (4.0 M, 5 mL) was added, and the mixture was reacted at room temperature for 1 h. After the reaction was completed, the mixture was concentrated under reduced pressure at 30 °C, and a solution of ammonia in methanol (5 mL) was added. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((1-(((7-(8-ethynyl-7-fluoro-3-hydroxynaphthalen-1-yl)-8-fluoro-4-(6-hydroxy-6-methyl-1,4-oxazepan-4-yl)pyrido[4,3-*d*]pyrimidin-2-yl)oxy)methyl)cyclopropyl) methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1042.2.

¹H NMR (400 MHz, DMSO-*d*₆) δ 10.32 (s, 1H), 10.16 (s, 1H), 9.36 (d, *J* = 50.8 Hz, 1H), 7.99 - 7.95 (m, 1H), 7.46 (t, *J* = 8.8 Hz, 1H), 7.39 (d, *J* = 1.2 Hz, 1H), 7.37 - 7.35 (m, 1H), 7.31 (d, *J* = 2.0 Hz, 1H), 7.20 - 7.17 (m, 1H), 7.46 (d, *J* = 8.8 Hz, 1H), 5.15 (d, *J* = 72.0 Hz, 1H), 4.60 - 4.20 (m, 4H), 4.20 - 3.90 (m, 6H), 3.84 (s, 3H), 3.63 - 3.58 (m, 4H), 2.69 - 2.66 (m, 2H), 2.43 - 2.20 (m, 10H), 2.10 - 2.00 (m, 2H), 1.68 - 1.64 (m, 2H), 1.56 - 1.38 (m, 6H), 1.38 - 1.20 (m, 4H), 1.17 - 1.15 (m, 3H), 1.10 - 0.90 (m, 4H), 0.65 - 0.60 (m, 2H), 0.45 - 0.35 (m, 2H).

### Example 166: 1-(5-(9-((4-((1-(((4-(3-amino-3-methylpiperidin-1-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1H,3H)-dione

### Step 1: preparation of tert-butyl (1-(2-((1-((4-((3-(3-(2,4-dioxotetrahydropyrimidin-1(2H)-yl)-4-methylbenzoyl)-3-azaspiro [5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3-methylpiperidin-3-yl)carboxylate

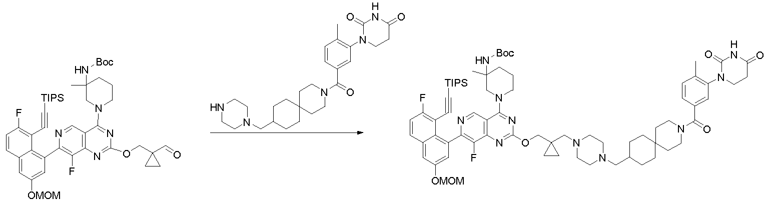

Tetraisopropyl titanate (203 mg, 0.715 mmol) was added to a solution of *tert*-butyl (1-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3-methylpiperidin-3-yl)carboxylate (120 mg, 0.143 mmol) and 1-(2-methyl-5-(9-(piperazin-1-ylmethyl)-3-azaspiro[5.5]undecane-3-carbonyl)phenyl) dihydropyrimidine-2,4(1*H*,3*H*)-dione (105 mg, 0.218 mmol) in dichloromethane (2 mL) at room temperature, and the mixture was stirred at 25 °C for 16 h. Sodium triacetoxyborohydride (118 mg, 0.533 mmol) was added to the mixture at 0 °C, and the mixture was reacted at 25 °C for another 3 h. Methanol (5 mL) was added to quench the reaction, and the mixture was concentrated under reduced pressure at 40 °C. The resulting crude product was purified by a preparative thin-layer chromatography plate (dichloromethane:methanol = 10:1, 0.1% NH₃) to give *tert*-butyl (1-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3-methylpiperidin-3-yl)carboxylate.

MS (ESI) *m*/*z*: [M/2+H]⁺ = 655.6.

### Step 2: preparation of 1-(5-(9-((4-((1-(((4-(3-amino-3-methylpiperidin-1-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

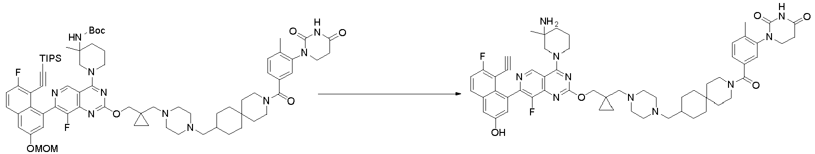

CsF (83 mg, 0.547 mmol) was added to a solution of *tert*-butyl (1-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3-methylpiperidin-3-yl)carboxylate (145 mg, 0.109 mmol) in DMF (2 mL), and the mixture was stirred at room temperature for half an hour. Water (2 mL) was added, and the mixture was extracted with ethyl acetate (5 mL × 3). The organic phases were combined, dried over anhydrous sodium sulfate, and concentrated under reduced pressure. The intermediate obtained by concentration was dissolved in 1,4-dioxane (2 mL), and HCl/1,4-dioxane (6 N, 1 mL) was added. The mixture was reacted at room temperature for 0.5 h. The reaction liquid was adjusted to pH > 7 with an aqueous Na₂CO₃ solution and extracted with ethyl acetate (5 mL × 3). The combined organic phase was dried over Na₂SO₄, filtered, and concentrated under reduced pressure, and the crude product was purified by preparative high-performance liquid chromatography to give 1-(5-(9-((4-((1-(((4-(3-amino-3-methylpiperidin-1-y1)-7-(8-ethynyl-7-fluoro-3-hydroxynaphthalen-1-yl)-8-fluoropyrido[4,3-*d*]pyrimidin-2-yl)oxy)methyl)cyclopropyl) methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 505.3.

¹H NMR (400 MHz, DMSO-*d*₆) δ 10.36 (s, 1H), 10.14 (s, 1H), 9.16 (d, *J* = 56.0 Hz, 1H), 7.97 (dd, *J*= 9.1, 5.9 Hz, 1H), 7.46 (t, *J* = 8.9 Hz, 1H), 7.39 (d, *J* = 2.5 Hz, 1H), 7.33 (d, *J*= 7.9 Hz, 1H), 7.30 (s, 1H), 7.23 (d, *J=* 7.7 Hz, 1H), 7.19 (dd, *J=* 5.0, 2.5 Hz, 1H), 4.36 - 4.19 (m, 2H), 4.16 - 4.05 (m, 1H), 4.03 - 3.90 (m, 1H), 3.88 - 3.65 (m, 2H), 3.64 - 3.42 (m, 4H), 2.84 - 2.61 (m, 2H), 2.46 - 2.17 (m, 17H), 2.07 - 1.92 (m, 13.9 Hz, 4H), 1.69 - 1.39 (m, 10H), 1.30 - 1.20 (m, 3H), 1.11 - 0.96 (m, 7H), 0.65 - 0.59 (m, 2H), 0.43 - 0.36 (m, 2H).

### Example 167: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methoxypropyl)piperazin-1-yl)methyl)-3-azaspiro [5.5]undecane-3-carbonyl)-2-methoxyphenyl)dihydropyrimidine-2,4(1H,3H)-dione

### Step 1: preparation of 1-(5-(9-((4-((R)-3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)pyrido[4,3-d]pyrimidin-2-yl)oxy)-2-methoxypropyl)piperazin-1-yl)methyl)-3-azaspiro[5.5]undecane-3-carbonyl)-2-methoxyphenyl) dihydropyrimidine-2,4(1H,3H)-dione

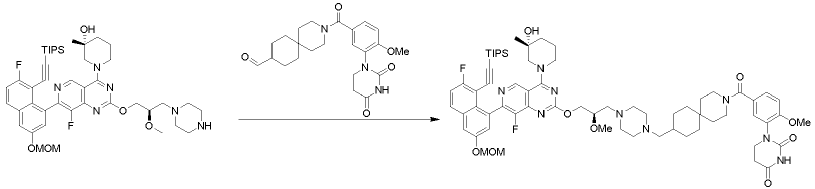

Tetraisopropyl titanate (208 mg, 732.55 µmol) and glacial acetic acid (0.3 mL) were added to a stirred solution of the crude product of (*R*)-1-(8-fluoro-7-(7-fluoro-3-(methoxymethoxy)-8-((triisopropylsilyl)ethynyl) naphthalen-1-yl)-2-((*R*)-2-methoxy-3-(piperazin-1-yl)propoxy)pyrido[4,3-d]pyrimidin-4-yl)-3-methylpiperidin-3-ol (60.7%, 200 mg, 146.51 µmol) and 3-(3-(2,4-dioxotetrahydropyrimidin-1(2*H*)-yl)-4-methoxybenzoyl)-3-azaspiro[5.5]undecane-9-carbaldehyde (94 mg, 219.76 µmol) in dichloromethane (10 mL) at room temperature, and the mixture was stirred at 25 °C for 2 h. Then sodium triacetoxyborohydride (93 mg, 439.53 µmol) was added, and the mixture was reacted at 25 °C for another 16 h. Methanol (5 mL) was added to quench the reaction, and the mixture was concentrated under reduced pressure at 40 °C, and the resulting crude product was purified by a preparative thin-layer chromatography plate (dichloromethane:methanol = 10:1, a 1% solution of ammonia in methanol) to give 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methoxypropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1230.5.

### Step 5: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methoxypropyl)piperazin-1-yl)methyl)-3-azaspiro[5.5]undecane-3-carbonyl)-2-methoxyphenyl)dihydropyrimidine-2,4(1H,3H)-dione

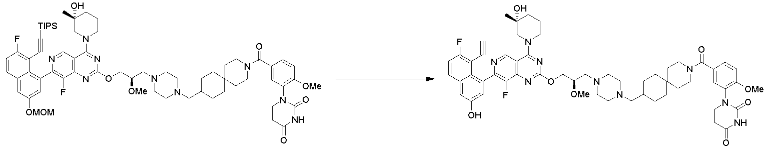

Cesium fluoride (110 mg, 724.90 µmol) was added to a solution of 1-*(*5-(9-((4-((R)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methoxypropyl)piperazin-1-yl)methyl)-3-azaspiro[5.5 Jundecane-3-carbonyl)-2-methoxyphenyl)dihydropyrimidine-2,4(1*H*,3*H*)-dione (89.2 mg, 72.49 µmol) in *N*,*N*-dimethylformamide (8 mL) at room temperature, and the mixture was reacted at 25 °C for 2 h under nitrogen atmosphere. After the reaction was completed, the reaction liquid was poured into water (60 mL) and extracted with ethyl acetate (20 mL × 3). The organic phases were separately washed with water (60 mL) and saturated brine (60 mL), and the combined organic phase was dried over anhydrous sodium sulfate, filtered, and concentrated under reduced pressure. After the concentrate was dissolved in a 1,4-dioxane solution (2.5 mL), a hydrogen chloride/1,4-dioxane solution (4.0 M, 5 mL) was added, and the mixture was reacted at room temperature for 1 h. After the reaction was completed, the mixture was concentrated under reduced pressure at 30 °C, and a solution of ammonia in methanol (5 mL) was added. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methoxypropyl) piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1030.1.

¹H NMR (400 MHz, DMSO-*d*₆) δ 10.33 (s, 1H), 10.17 (s, 1H), 9.14 (d, *J* = 59.2 Hz, 1H), 8.00 - 7.95 (m, 1H), 7.46 (t, *J* = 9.2 Hz, 1H), 7.39 - 7.34 (m, 2H), 7.32 - 7.30 (m, 1H), 7.24 - 7.18 (m, 1H), 7.16 - 7.13(m, 1H), 4.77 - 4.70 (m, 1H), 4.60 - 4.50 (m, 1H), 4.40 - 4.25 (m, 2H), 4.15 - 3.97 (m, 1H), 3.97 - 3.93 (m, 1H), 3.85 (s, 3H), 3.73 - 3.67 (m, 1H), 3.63 - 3.50 (m, 4H), 3.37 - 3.33 (m, 3H), 3.33 - 3.25 (m, 2H), 2.73 - 2.63(m, 2H), 2.47 - 2.23 (m, 10H), 2.13 - 1.93 (m, 4H), 1.73 - 1.60 (m, 5H), 1.53 - 1.23 (m, 8H), 1.20 - 1.13 (m, 3H), 1.10 - 0.93 (m, 4H).

### Example 168: 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methoxypropyl)piperazin-1-yl)methyl)-3-azaspiro[5.5]undecane-3-carbonyl)-2-methylphenyl)dihydropyrimidine-2,4(1H,3H)-dione

### Step 1: preparation of 1-(5-(9-((4-((R)-3-((8-fluoro-7-(7-fluoro-3-(methoxymethoxy)-8-((triisopropylsilyl) ethynyl)naphthalen-1-yl)-4-((R)-3-hydroxy-3-methylpiperidin-1-yl)pyrido[4,3-d]pyrimidin-2-yl)oxy)-2-methoxypropyl)piperazin-1-yl)methyl)-3-azaspiro[5.5]undecane-3-carbonyl)-2-methylphenyl)dihydropyrimidine-2,4(1H,3H)-dione

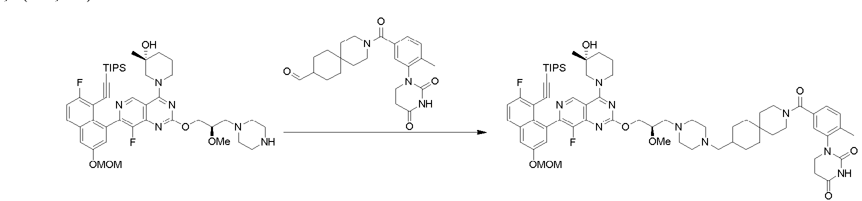

Tetraisopropyl titanate (208 mg, 732.55 µmol) and glacial acetic acid (0.3 mL) were added to a stirred solution of the crude product of (*R*)-1-(8-fluoro-7-(7-fluoro-3-(methoxymethoxy)-8-((triisopropylsilyl)ethynyl)naphthalen-1-yl)-2-((*R*)-2-methoxy-3-(piperazin-1-yl)propoxy)pyrido[4,3-*d*]pyrimidin-4-yl)-3-methylpiperidin-3-ol (60.7%, 200 mg, 146.51 µmol) and 3-(3-(2,4-dioxotetrahydropyrimidin-1(2*H*)-yl)-4-methylbenzoyl)-3-azaspiro[5.5]undecane-9-carbaldehyde (91 mg, 219.76 µmol) in dichloromethane (10 mL) at room temperature, and the mixture was stirred at 25 °C for 2 h. Then sodium triacetoxyborohydride (93 mg, 439.53 µmol) was added, and the mixture was reacted at 25 °C for another 16 h. Methanol (5 mL) was added, and the mixture was concentrated under reduced pressure at 40 °C, and the resulting crude product was purified by a preparative thin-layer chromatography plate (dichloromethane:methanol = 10:1, a 1% solution of ammonia in methanol) to give 1-(5-(9-((4-((*R*)-3-((8-fluoro-7-(7-fluoro-3-(methoxymethoxy)-8-((triisopropylsilyl)ethynyl) naphthalen-1-yl)-4-((*R*)-3-hydroxy-3-methylpiperidin-1-yl)pyrido[4,3-*d*]pyrimidin-2-yl)oxy)-2-methoxypropyl) piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1214.6.

### Step 2: preparation of 1-(5-(9-((4-((R)-3-((7-(8-ethynyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)-2-methoxypropyl)piperazin-1-yl)methyl)-3-azaspiro[5.5]undecane-3-carbonyl)-2-methylphenyl)dihydropyrimidine-2,4(1H,3H)-dione

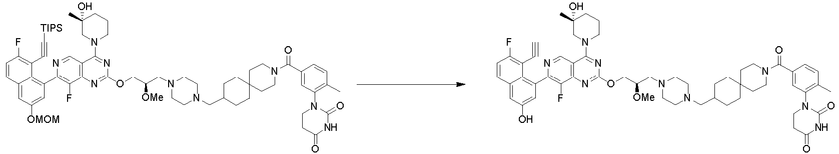

Cesium fluoride (107 mg, 704.80 µmol) was added to a solution of 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-hydroxy-3-methylpiperidin-1-yl)pyrido[4,3-*d*]pyrimidin-2-yl)oxy)-2-methoxypropyl)piperazin-1-yl)methyl)-3-azaspiro[5.5]undecane-3-carbonyl)-2-methylphenyl)dihydropyrimidine-2,4(1*H*,3*H*)-dione (85.6 mg, 70.48 µmol) in *N*,*N*-dimethylformamide (8 mL) at room temperature, and the mixture was reacted at 25 °C for 2 h under nitrogen atmosphere. After the reaction was completed, the reaction liquid was poured into water (60 mL) and extracted 3 times with ethyl acetate (20 mL × 3). The organic phases were separately washed with water (60 mL) and saturated brine (60 mL), and the combined organic phase was dried over anhydrous sodium sulfate, filtered, and concentrated under reduced pressure. After the concentrate was dissolved in a 1,4-dioxane solution (2.5 mL), a hydrogen chloride/1,4-dioxane solution (4.0 M, 5 mL) was added, and the mixture was reacted at room temperature for 1 h. After the reaction was completed, the mixture was concentrated under reduced pressure at 30 °C, and a solution of ammonia in methanol (5 mL) was added. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)-2-methoxypropyl) piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1014.1.

¹H NMR (400 MHz, DMSO-*d*₆) δ 10.38 (s, 1H), 10.16 (s, 1H), 9.14 (d, *J* = 59.2 Hz, 1H), 8.00 - 7.95 (m, 1H), 7.46 (t, *J* = 9.2 Hz, 1H), 7.39 - 7.38 (m, 1H), 7.34 - 7.30 (m, 2H), 7.24 - 7.18 (m, 2H), 4.76 - 4.72 (m, 1H), 4.60 - 4.50 (m, 1H), 4.40 - 4.25 (m, 2H), 4.15 - 3.97 (m, 1H), 3.97 - 3.93 (m, 1H), 3.87 - 3.65 (m, 2H), 3.65 - 3.50 (m, 3H), 3.37 - 3.33 (m, 3H), 3.30 - 3.23 (m, 2H), 2.83 - 2.63 (m, 2H), 2.47 - 2.23 (m, 10H), 2.21 (s, 3H), 2.13 - 1.93 (m, 4H), 1.73 - 1.60 (m, 5H), 1.53 - 1.23 (m, 8H), 1.20 - 1.13 (m, 3H), 1.10 - 0.93 (m, 4H).

### Example 169: 1-(5-(9-((4-((R)-3-((7-(8-ethynyl-7-fluoro-3-hydroxynaphthalen-1-yl)-8-fluoro-4-((R)-3-fluoropiperidin-1-yl)pyrido[4,3-d]pyrimidin-2-yl)oxy)-2-methylpropyl)piperazin-1-yl)methyl)-3-azaspiro [5.5]undecane-3-carbonyl)-2-methoxyphenyl)dihydropyrimidine-2,4(1H,3H)-dione

### Step 1: preparation of (R)-2,7-dichloro-8-fluoro-4-(3-fluoropiperidin-1-yl)pyrido[4,3-d]pyrimidine

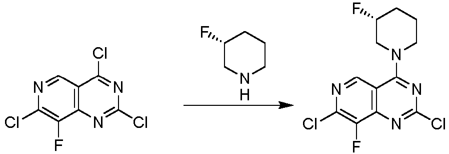

2,4,7-Trichloro-8-fluoropyrido[4,3-*d*]pyrimidine (1.5 g, 5.94 mmol) and triethylamine (1.80 g, 17.82 mmol) were dissolved in tetrahydrofuran (20 mL), and the mixture was cooled to -50 °C. (*R*)-3-Fluoropiperidine (746 g, 5.35 mmol) was added, and the mixture was reacted at -50 °C for 3 h. After the reaction was completed, the reaction liquid was poured into a saturated aqueous ammonium chloride solution (20 mL), and the mixture was extracted with ethyl acetate (20 mL × 3). The organic phases were combined, washed with saturated brine, dried over anhydrous sodium sulfate, and filtered. The filtrate was concentrated, and the resulting crude product was subjected to silica gel column chromatography (PE:EA = 3:1) to give (*R*)-2,7-dichloro-8-fluoro-4-(3-fluoropiperidin-1-yl)pyrido[4,3-*d*]pyrimidine.

LC-MS: (ESI, m/z): [M+H]⁺ = 319.1.

### Step 2: preparation of 2-((R)-3-(4-benzylpiperazin-1-yl)-2-methylpropoxy)-7-chloro-8-fluoro-4-((R)-3-fluoropiperidin-1-yl)pyrido[4,3-d]pyrimidine

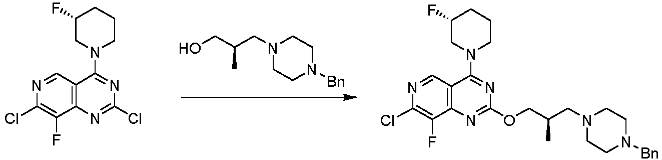

(*R*)-2,7-Dichloro-8-fluoro-4-(3-fluoropiperidin-1-yl)pyrido[4,3-*d*]pyrimidine (1.5 g, 4.70 mmol) and (*R*)-3-(4-benzylpiperazin-1-yl)-2-methylpropan-1-ol (1.52 g, 6.11 mmol) were dissolved in THF (20 mL), and Cs₂CO₃ (4.60 g, 19.59 mmol) was added. The reaction liquid was heated to 90 °C and reacted for 16 h. After the reaction was completed, the mixture was cooled to room temperature. Water (30 mL) was added, and the mixture was extracted with ethyl acetate (30 mL × 3). The organic phases were combined, washed with a saturated NaCl solution, dried over anhydrous Na₂SO₄, and filtered, and the filtrate was concentrated. The resulting crude product was subjected to silica gel column chromatography (PE:EtOAc = 2:1) to give 2-((*R*)-3-(4-benzylpiperazin-1-yl)-2-methylpropoxy)-7-chloro-8-fluoro-4-((R)-3-fluoropiperidin-1-yl)pyrido[4,3-*d*]pyrimidine.

LC-MS: (ESI, m/z): [M+H]⁺ = 531.2.

### Step 3: preparation of 2-((R)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)-4-((R)-3-fluoropiperidin-1-yl)pyrido[4,3-d]pyrimidine

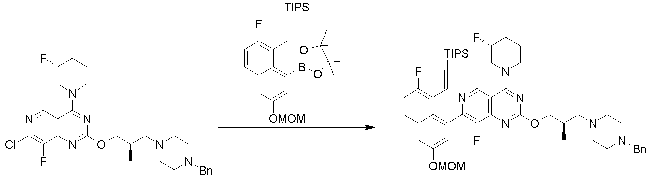

CataCXium A Pd G3 (137 mg, 0.19 mmol) and cesium carbonate (919 mg, 2.82 mmol) were added to a mixed solution of 2-((*R*)-3-(4-benzylpiperazin-1-yl)-2-methylpropoxy)-7-chloro-8-fluoro-4-((R)-3-fluoropiperidin-1-yl)pyrido[4,3-*d*]pyrimidine (500 mg, 0.94 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (578 g, 1.13 mmol) in 1,4-dioxane (5 mL) and H₂O (1 mL). The mixture was reacted at 85 °C for 16 h under nitrogen atmosphere. Water (10 mL) was added to quench the reaction, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 20:1) to give 2-((*R*)-3-(4-benzylpiperazin-1-yl)-2-methylpropoxy)-8-fluoro-7-(7-fluoro-3-(methoxymethoxy)-8-((triisopropylsilyl)
ethynyl)naphthalen-1-yl)-4-((*R*)-3-fluoropiperidin-1-yl)pyrido[4,3-*d*]pyrimidine.

LC-MS: (ESI, m/z): [M/2+H]⁺ = 441.3.

### Step 4: preparation of 8-fluoro-7-(7-fluoro-3-(methoxymethoxy)-8-((triisopropylsilyl)ethynyl)naphthalen-1-yl)-4-((R)-3-fluoropiperidin-1-yl)-2-((R)-2-methyl-3-(piperazin-1-yl)propoxy)pyrido[4,3-d]pyrimidine

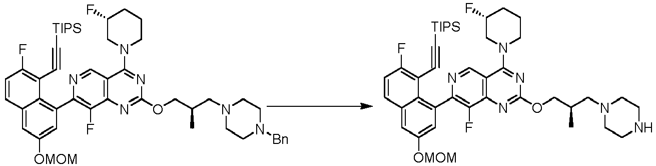

2-((*R*)-3-(4-Benzylpiperazin-1-yl)-2-methylpropoxy)-8-fluoro-7-(7-fluoro-3-(methoxymethoxy)-8-((triisopropylsilyl)ethynyl)naphthalen-1-yl)-4-((*R*)-3-fluoropiperidin-1-yl)pyrido[4,3-*d*]pyrimidine (380 mg, 0.43 mmol) was dissolved in DCM (4 mL), and DIEA (111 mg, 0.86 mmol) and 1-chloroethyl chloroformate (476 mg, 3.33 mmol) were added. The mixture was reacted at room temperature for 1 h and concentrated under reduced pressure. The concentrate was dissolved in MeOH (4 mL). The mixture was heated to 50 °C and reacted for 30 min. After the reaction was completed, the mixture was concentrated under reduced pressure, and the concentrate was purified by silica gel column chromatography (DCM:MeOH (NH₃) = 15:1) to give 8-fluoro-7-(7-fluoro-3-(methoxymethoxy)-8-((triisopropylsilyl)ethynyl)naphthalen-1-yl)-4-((*R*)-3-fluoropiperidin-1-yl)-2-((*R*)-2-methyl-3-(piperazin-1-yl)propoxy)pyrido [4,3-*d*]pyrimidine.

LC-MS: (ESI, m/z): [M+H]⁺ = 791.4.

### Step 5: preparation of 1-(5-(9-((4-((R)-3-((8-fluoro-7-(7-fluoro-3-(methoxymethoxy)-8-((triisopropylsilyl) ethynyl)naphthalen-1-yl)-4-((R)-3-fluoropiperidin-1-yl)pyrido[4,3-d]pyrimidin-2-yl)oxy)-2-methylpropyl) piperazin-1-yl)methyl)-3-azaspiro[5.5]undecane-3-carbonyl)-2-methoxyphenyl)dihydropyrimidine-2,4(1H,3H)-dione

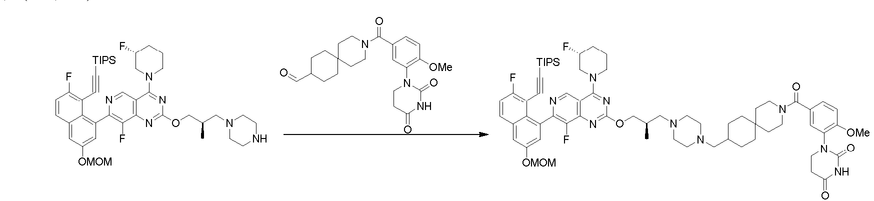

Tetraisopropyl titanate (0.5 mL) was added to a stirred solution of 8-fluoro-7-(7-fluoro-3-(methoxymethoxy)-8-((triisopropylsilyl)ethynyl)naphthalen-1-yl)-4-((*R*)-3-fluoropiperidin-1-yl)-2-((R)-2-methyl-3-(piperazin-1-yl)propoxy)pyrido[4,3-*d*]pyrimidine (110 mg, 0.14 mmol) and 3-(3-(2,4-dioxotetrahydropyrimidin-1(2*H*)-yl)-4-methoxybenzoyl)-3-azaspiro[5.5]undecane-9-carbaldehyde (90 mg, 0.21 mmol) in DCM/AcOH (1 mL/0.05 mL), and the mixture was stirred at 30 °C for 2 h. NaBH(OAc)₃ (93 mg, 0.42 mmol) was added to the mixture in an ice bath, and the resulting mixture was stirred for 1 h. The mixture was concentrated and purified by silica gel column chromatography (DCM:MeOH = 100:1 to 10:1) to give 1-(5-(9-((4-((*R*)-3-((8-fluoro-7-(7-fluoro-3-(methoxymethoxy)-8-((triisopropylsilyl)ethynyl)naphthalen-1-yl)-4-((*R*)-3-fluoropiperidin-1-yl)pyrido [4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione.

LC-MS: (ESI, m/z): [M/2+H]⁺ = 601.9.

### Step 6: preparation of 1-(5-(9-((4-((R)-3-((7-(8-ethynyl-7-fluoro-3-hydroxynaphthalen-1-yl)-8-fluoro-4-((R)-3-fluoropiperidin-1-yl)pyrido[4,3-d]pyrimidin-2-yl)oxy)-2-methylpropyl)piperazin-1-yl)methyl)-3-azaspiro [5.5]undecane-3-carbonyl)-2-methoxyphenyl)dihydropyrimidine-2,4(1H,3H)-dione

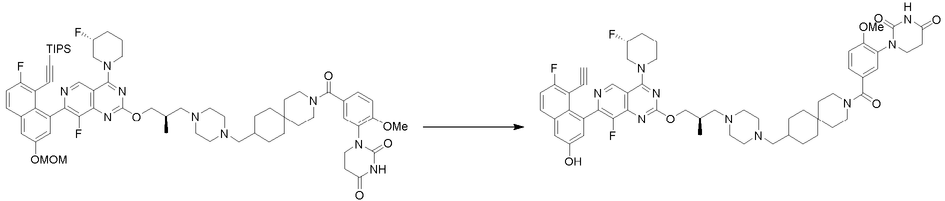

Cesium fluoride (5 mg, 0.375 mmol) was added to a stirred solution of 1-(5-(9-((4-((R)-3-((8-fluoro-7-(7-fluoro-3-(methoxymethoxy)-8-((triisopropylsilyl)ethynyl)naphthalen-1-yl)-4-((*R*)-3-fluoropiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione (90 mg, 0.075 mmol) in DMF (1 mL), and the mixture was stirred at room temperature for 30 min. Water (5 mL) was added to the reaction liquid to quench the reaction, and the mixture was extracted with ethyl acetate (5 mL × 3). The organic phases were washed with brine, combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. The reaction liquid was adjusted to pH > 7 with an aqueous Na₂CO₃ solution, and the mixture was extracted with EtOAc (10 mL × 2), dried over anhydrous Na₂SO₄, and concentrated. The crude product was purified by preparative high-performance liquid chromatography to give 1-(5-(9-((4-((*R*)-3-((7-(8-ethynyl-7-fluoro-3-hydroxynaphthalen-1-yl)-8-fluoro-4-((*R*)-3-fluoropiperidin-1-yl)pyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1002.4.

¹H NMR (400 MHz, DMSO-*d*₆) δ 10.32 (s, 1H), 10.12 (s, 1H), 8.87 (d, *J* = 1.3 Hz, 1H), 8.03 - 7.89 (m, 1H), 7.45 (t, *J =* 9.0 Hz, 1H), 7.39 - 7.29 (m, 3H), 7.20 - 7.08 (m, 2H), 5.01 - 4.20 (m, 5H), 4.04 - 3.89 (m, 1H), 3.84 (s, 3H), 3.63 - 3.33 (m, 6H), 2.68 (t, *J =* 6.6 Hz, 2H), 2.49 - 2.14 (m, 12H), 2.12 - 2.03 (m, 2H), 1.99 - 1.74 (m, 3H), 1.73 - 1.38 (m, 8H), 1.37 - 1.16 (m, 3H), 1.14 - 0.92 (m, 7H).

### Example 170: (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-(methoxy-d3)phenyl)dihydropyrimidine-2,4(1H,3H)-dione

### Step 1: preparation of (R)-1-(5-(9-((4-((1-(((8-fluoro-7-(7-fluoro-3-(methoxymethoxy)-8-((triisopropylsilyl) ethynyl)naphthalen-1-yl)-4-(3-hydroxy-3-methylpiperidin-1-yl)pyrido[4,3-d]pyrimidin-2-yl)oxy)methyl) cyclopropyl)methyl)piperazin-1-yl)methyl)-3-azaspiro[5.5]undecane-3-carbonyl)-2-(methoxy-d3)phenyl) dihydropyrimidine-2,4(1H,3H)-dione

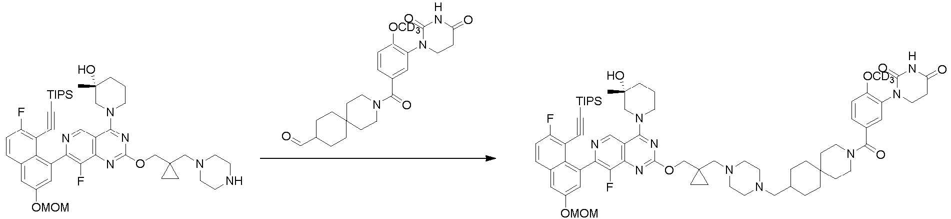

Tetraisopropyl titanate (235 mg, 828.55 µmol) and glacial acetic acid (0.3 mL) were added to a solution of the crude product of (*R*)-1-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido [4,3-d]pyrimidin-4-yl)-3-methylpiperidin-3-ol (135 mg, 165.71 µmol) and 3-(3-(2,4-dioxotetrahydropyrimidin-1(2*H*)-yl)-4-(methoxy-d3)benzoyl)-3-azaspiro[5.5]undecane-9-carbaldehyde (107 mg, 248.57 µmol) in dichloromethane (10 mL) at room temperature, and the mixture was stirred at 25 °C for 2 h. Then sodium triacetoxyborohydride (70 mg, 331.42 µmol) was added, and the mixture was reacted at 25 °C for another 16 h. Methanol (5 mL) was added to quench the reaction, and the mixture was concentrated under reduced pressure at 40 °C. The resulting crude product was purified by a preparative thin-layer chromatography plate (dichloromethane:methanol = 10:1, a 1% solution of ammonia in methanol) to give (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-(methoxy-d3)phenyl)dihydropyrimidine-2,4(1*H*,3*H*)-dione.

LC-MS: (ESI, m/z): [M+H]⁺ = 1229.7.

### Step 2: preparation of (R)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-d]pyrimidin-2-yl)oxy)methyl)cyclopropyl)methyl)piperazin-1-yl)methyl)-3-azaspiro[5.5]undecane-3-carbonyl)-2-(methoxy-d3)phenyl)dihydropyrimidine-2,4(1H,3H)-dione

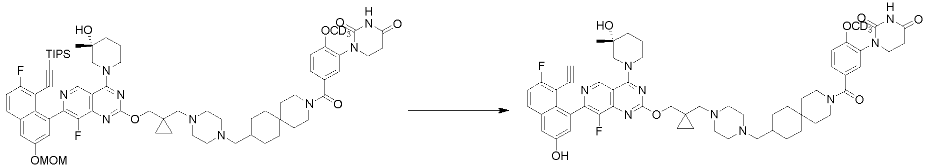

Cesium fluoride (107 mg, 704.80 µmol) was added to a solution of (*R*)-1-(5-(9-((4-((1-(((8-fluoro-7-(7-fluoro-3-(methoxymethoxy)-8-((triisopropylsilyl)ethynyl)naphthalen-1-yl)-4-(3-hydroxy-3-methylpiperidin-1-yl)pyrido[4,3-*d*]pyrimidin-2-yl)oxy)methyl)cyclopropyl)methyl)piperazin-1-yl)methyl)-3-azaspiro[5.5] undecane-3-carbonyl)-2-(methoxy-d3)phenyl)dihydropyrimidine-2,4(1*H*,3*H*)-dione (86.3 mg, 70.14 µmol) in *N*,*N*-dimethylformamide (8 mL) at room temperature, and the mixture was reacted at 25 °C for 2 h under nitrogen atmosphere. After the reaction was completed, the reaction liquid was poured into water (60 mL) and extracted with ethyl acetate (20 mL × 3). The organic phases were separately washed with water (60 mL) and saturated brine (60 mL), and the combined organic phase was dried over anhydrous sodium sulfate, filtered, and concentrated under reduced pressure. After the concentrate was dissolved in a 1,4-dioxane solution (2.5 mL), a hydrogen chloride/1,4-dioxane solution (4.0 M, 5 mL) was added, and the mixture was reacted at room temperature for 1 h. After the reaction was completed, the mixture was concentrated under reduced pressure at 30 °C, and a solution of ammonia in methanol (5 mL) was then added. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give (*R*)-1-(5-(9-((4-((1-(((7-(8-ethynyl-7-fluoro-3-hydroxynaphthalen-1-yl)-8-fluoro-4-(3-hydroxy-3-methylpiperidin-1-yl)pyrido[4,3-*d*]pyrimidin-2-yl)oxy) methyl)cyclopropyl)methyl)piperazin-1-yl)methyl)-3-azaspiro[5.5]undecane-3-carbonyl)-2-(methoxy-d3) phenyl)dihydropyrimidine-2,4(1*H*,3*H*)-dione.

LC-MS: (ESI, m/z): [M+H]⁺ = 1029.5.

¹H NMR (400 MHz, DMSO-*d*₆) δ 10.32 (s, 1H), 10.14 (br, 1H), 9.12 (d, *J* = 65.6 Hz, 1H), 7.99 - 7.95 (m, 1H), 7.48 - 7.43 (m, 1H), 7.39 - 7.38 (m, 1H), 7.37 - 7.34 (m, 1H), 7.31 - 7.30 (m, 1H), 7.22 - 7.18 (m, 1H), 7.13 (d, *J* = 8.0 Hz, 1H), 4.78 - 4.70 (m, 1H), 4.38 - 4.23 (m, 3H), 4.10 - 3.93 (m, 2H), 3.61 - 3.55 (m, 4H), 3.30 - 3.25 (m, 2H), 2.69 - 2.66 (m, 2H), 2.45 - 2.38 (m, 3H), 2.35 - 2.20 (m, 6H), 2.30 - 1.90 (m, 4H), 1.72 - 1.20 (m, 14H), 1.19 - 1.10 (m, 3H), 1.10 - 0.96 (m, 4H), 0.68 - 0.55 (m, 2H), 0.45 - 0.30 (m, 2H).

The compounds in Table 1 and Table 2 were synthesized using methods similar to those shown in the preceding examples.

**Table 1**

| Example | Compound structure | LC-MS: (ESI, m/z): [M+H]⁺ |
|---|---|---|
| 51 | 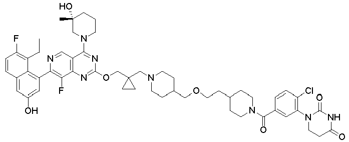 | 1009 |
| 52 | 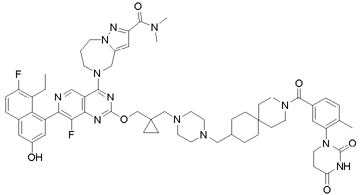 | 1107 |
| 53 | 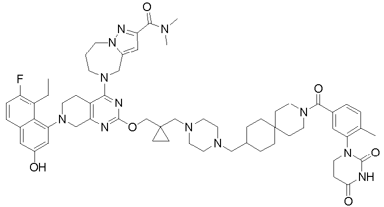 | 1093 |
| 54 | 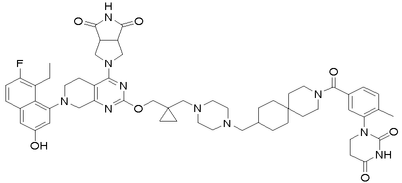 | 1025 |
| 55 | 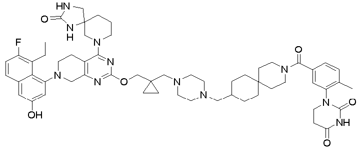 | 1040 |
| 56 | 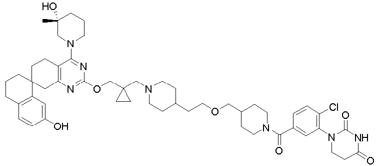 | 938 |
| 57 | 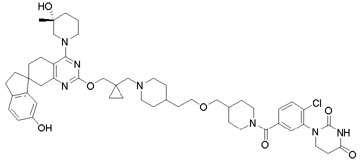 | 924 |
| 58 | 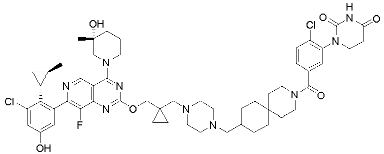 | 1026 |
| 59 | 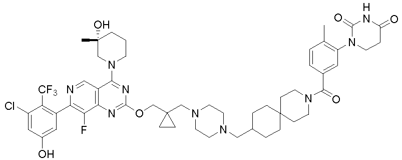 | 1020 |
| 62 | 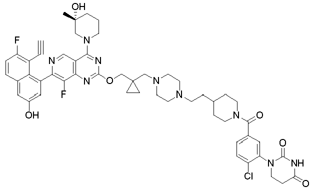 | 976 |
| 63 | 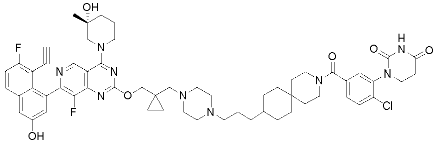 | 1058 |
| 65 | 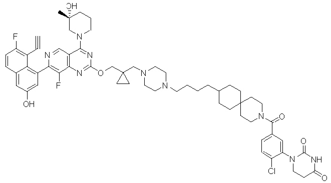 | 1072 |
| 66 | 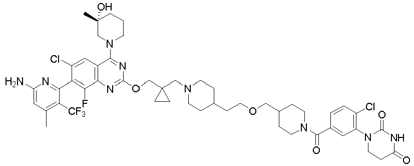 | 1028 |
| 68 | 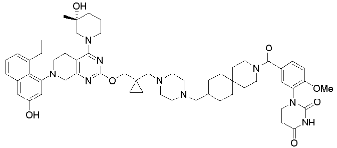 | 998 |
| 69 | 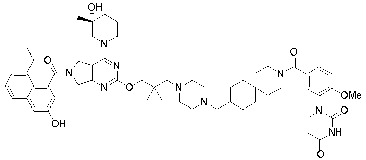 | 1012 |
| 70 | 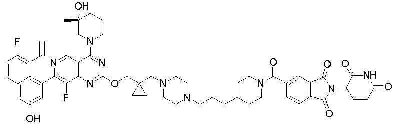 | 1024 |
| 71 | 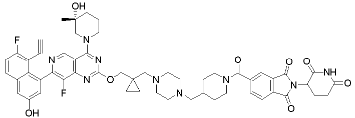 | 996 |
| 73 | 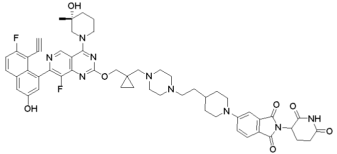 | 982 |
| 74 | 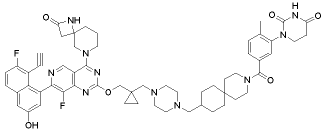 | 1035 |
| 75 | 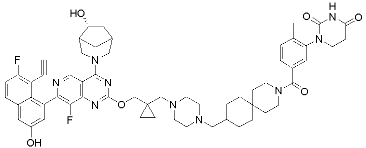 | 1022 |
| 76 | 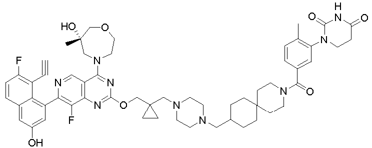 339 | 1026 |
| 77 | 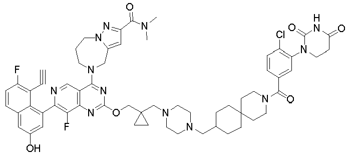 | 1123 |
| 78 | 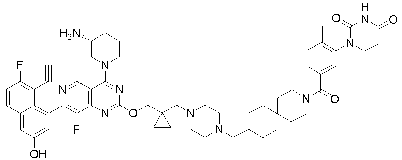 | 995 |
| 79 | 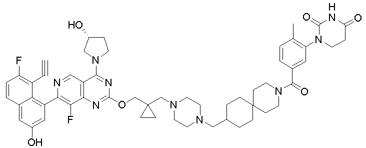 | 982 |
| 80 | 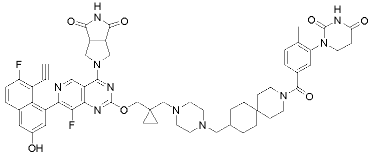 | 1035 |
| 81 | 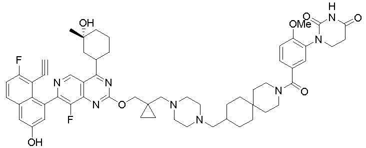 | 1025 |
| 82 | 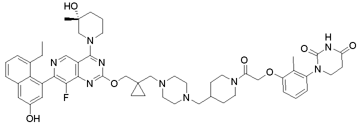 | 958 |
| 83 | 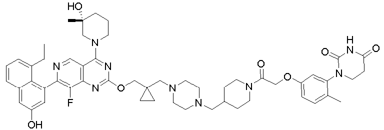 | 958 |
| 84 | 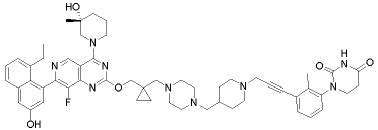 | 938 |
| 85 | 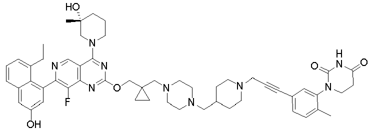 | 938 |
| 87 | 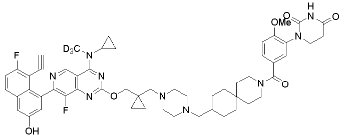 | 985 |
| 88 | 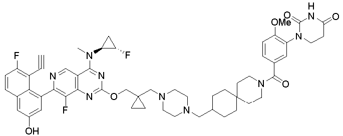 | 1000 |
| 89 | 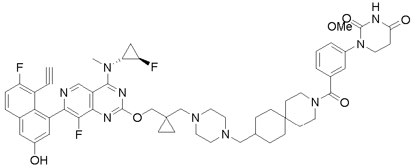 | 1000 |
| 90 | 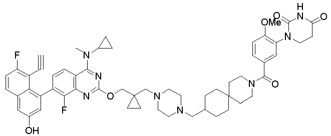 | 981 |
| 93 | 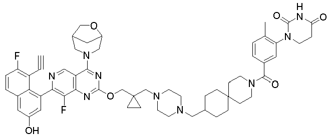 | 1008 |
| 94 | 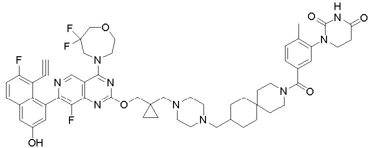 | 1032 |
| 95 | 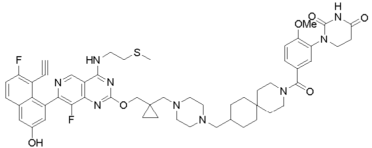 | 1002 |
| 96 | 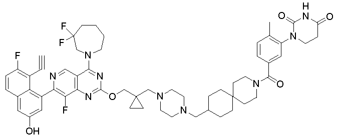 | 1030 |
| 97 | 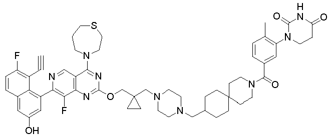 | 1012 |
| 98 | 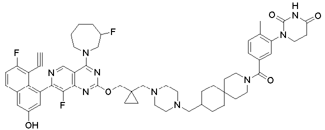 | 1012 |
| 99 | 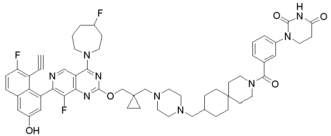 | 1012 |
| 100 | 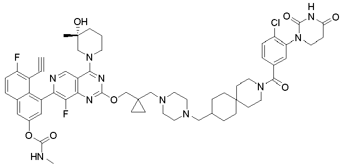 | 1087 |
| 101 | 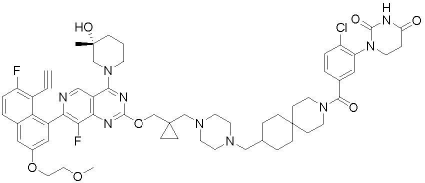 | 1088 |

**Table 2**

| Example | Compound structure | LC-MS: (ESI, m/z): [M+H]⁺ |
|---|---|---|
| 102 | 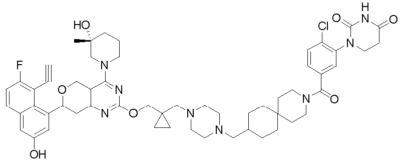 | 1019 |
| 103 | 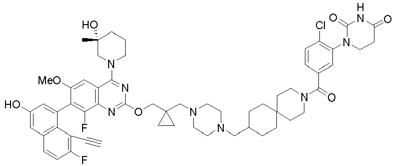 | 1059 |
| 104 | 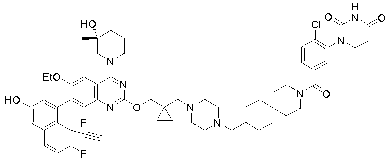 | 1073 |
| 105 | 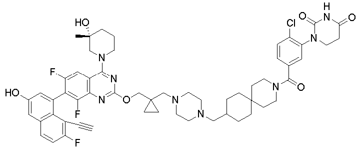 | 1047 |
| 106 | 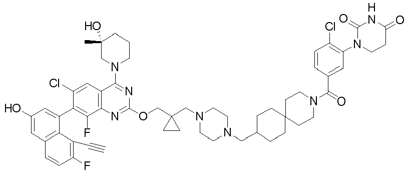 | 1063 |
| 107 | 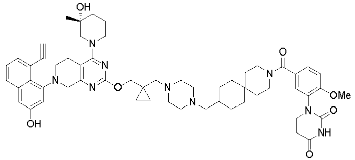 | 994 |
| 108 | 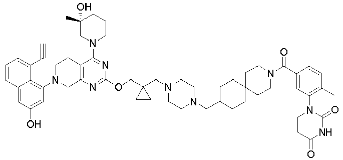 | 978 |
| 109 | 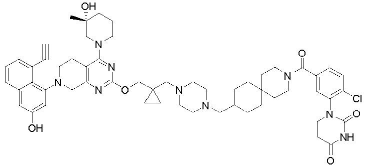 | 998 |
| 110 | 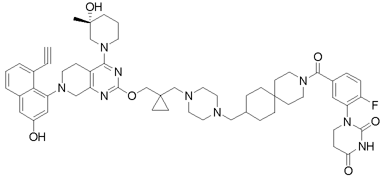 | 982 |
| 112 | 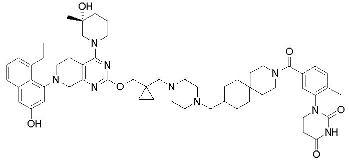 | 982 |
| 113 | 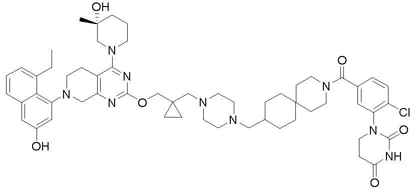 | 1002 |
| 114 | 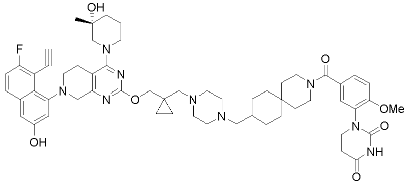 | 1012 |
| 115 | 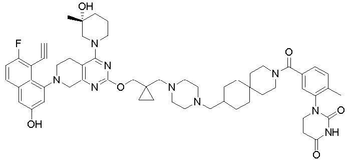 | 996 |
| 116 | 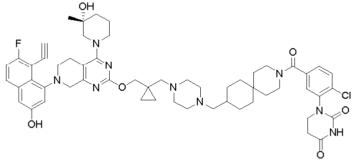 | 1016 |
| 117 | 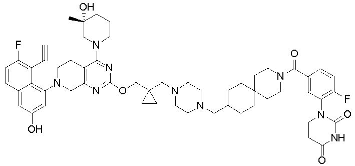 | 1000 |
| 118 | 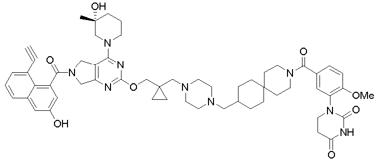 | 1008 |
| 119 | 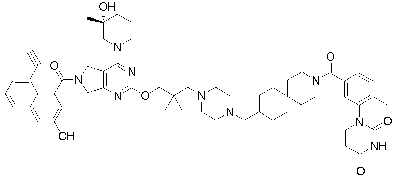 | 992 |
| 120 | 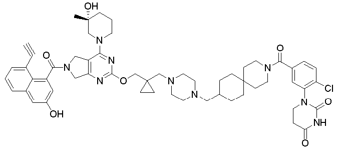 | 1012 |
| 121 | 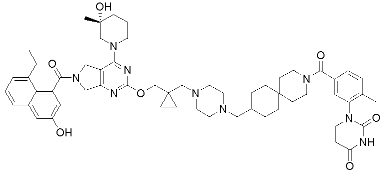 | 996 |
| 122 | 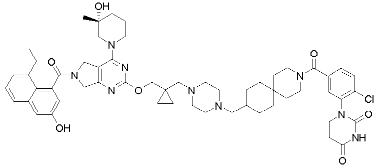 | 1016 |
| 123 | 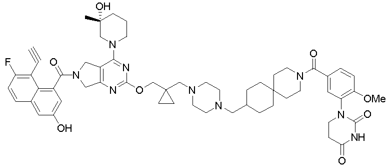 | 1026 |
| 124 | 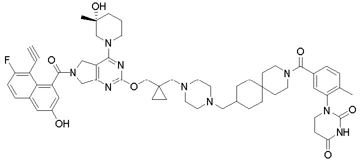 | 1010 |
| 125 | 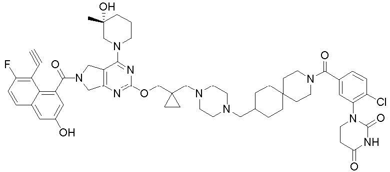 | 1030 |
| 126 | 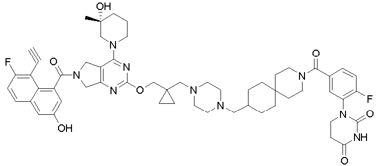 | 1014 |
| 127 | 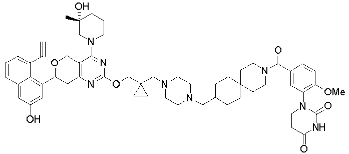 | 995 |
| 128 | 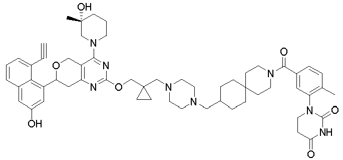 | 979 |
| 129 | 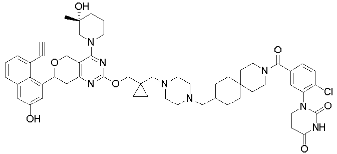 | 999 |
| 130 | 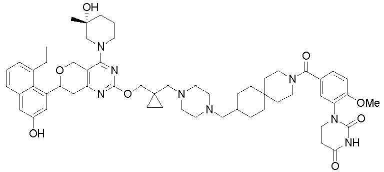 | 999 |
| 131 | 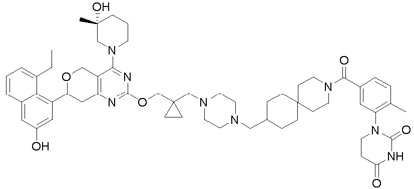 | 983 |
| 134 | 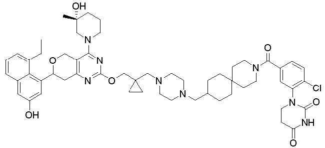 | 1003 |
| 135 | 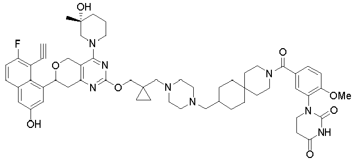 | 1013 |
| 136 | 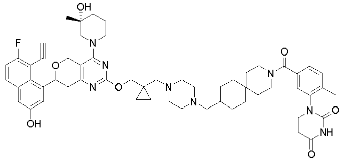 | 997 |
| 137 | 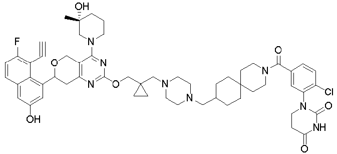 | 1017 |
| 138 | 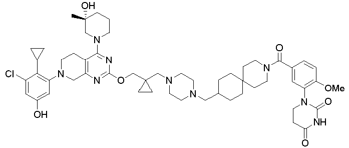 | 994 |
| 139 | 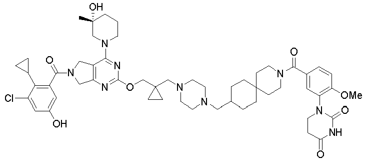 | 1008 |
| 140 | 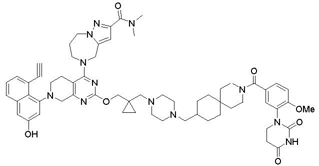 | 1087 |
| 141 | 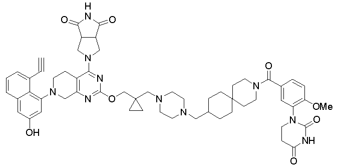 | 1019 |
| 142 | 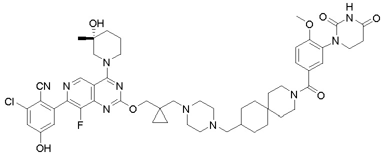 | 993 |
| 143 | 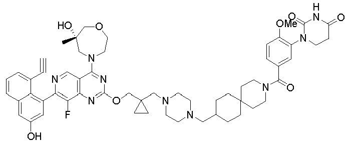 | 1024 |
| 144 | 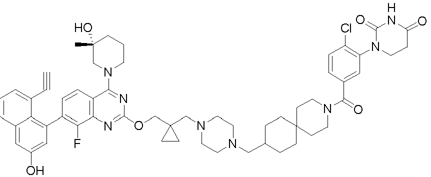 | 1011 |
| 145 | 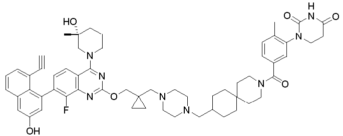 | 991 |
| 146 | 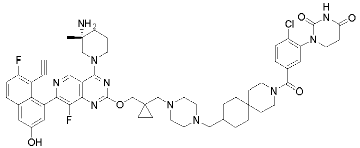 | 1029 |
| 147 | 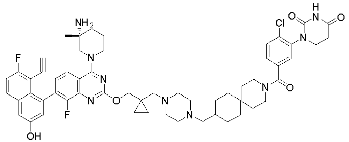 | 1028 |
| 148 | 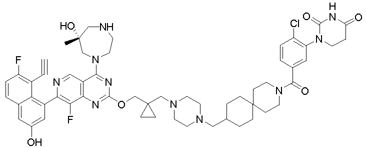 | 1045 |
| 149 | 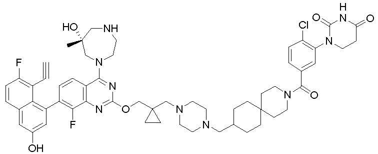 | 1044 |
| 150 | 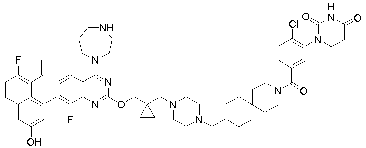 | 1014 |
| 151 | 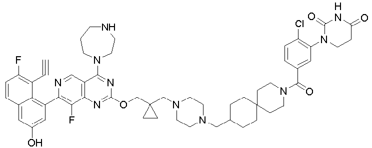 | 1015 |
| 152 | 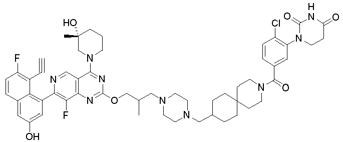 | 1018 |
| 153 | 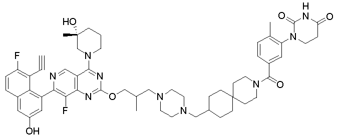 | 998 |
| 154 | 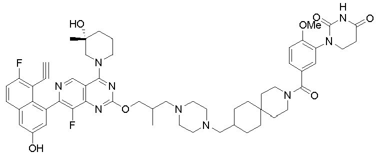 | 1014 |
| 155 | 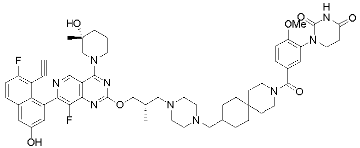 | 1014 |
| 156 | 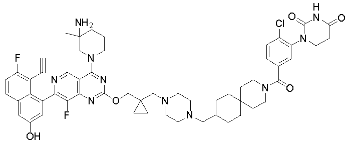 | 1029 |
| 157 | 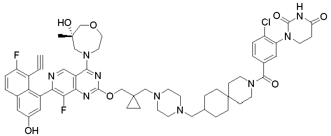 | 1046 |
| 158 | 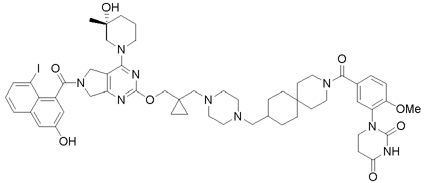 | 1110 |
| 159 | 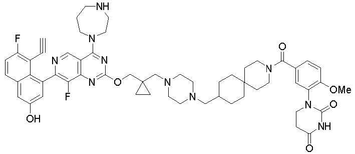 | 1011 |
| 160 | 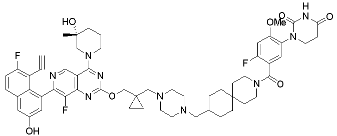 | 1044 |
| 161 | 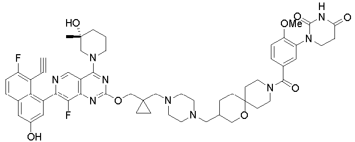 | 1028 |
| 171 | 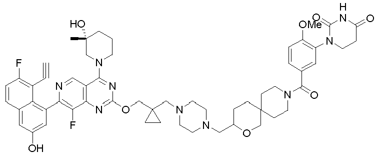 | 1028 |
| 172 | 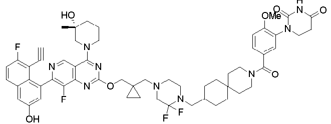 | 1062 |
| 173 | 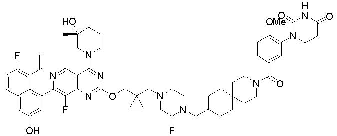 | 1044 |
| 174 | 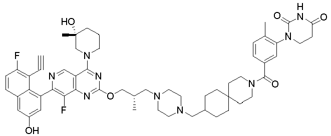 | 998 |
| 175 | 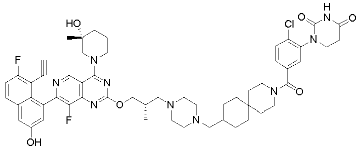 | 1018 |

### II. Biological Activity Test Examples

### Test Example 1: KRAS G12D Binding Assay

In this method, the inhibitory effects of the compounds on the binding of KRAS G12D to GTP were assayed by a homogeneous time-resolved fluorescence (HTRF) technique using a KRAS-G12D/GTP binding assay kit (PerkinElmer).

The experimental procedures were as follows (refer to the kit instructions for detailed test methods): the compounds of the present disclosure were each firstly dissolved in DMSO at a concentration of 20 mM, and then diluted in an equal gradient with a buffer in the kit, so that the final concentrations of the test compounds in the reaction system were in a range of 0.02-5000 nM, and the final concentration of DMSO was 0.5%. 5 µL of each compound was incubated with 5 µL of 1× Human KRAS G12D 6His-tagged (PerkinElmer), 5 µL of 1× 6His Eu Cryptate Antibody (PerkinElmer), and 5 µL of 1× GTP Red reagent (PerkinElmer) in a 384-well plate (Greiner) at 25 °C for 1 h. After the incubation was completed, the fluorescence intensity of each well was measured at the excitation wavelength of 337 nm and the emission wavelengths of 620 nm and 665 nm with an EnVision microplate reader (PerkinElmer) in the HTRF mode, and Ratio values were calculated using the formula: Ratio = (665 nm/620 nm) × 10⁴. The inhibition rates of the compounds at each concentration were calculated by comparing the fluorescence intensity ratio with that of the control group. Then non-linear curve fittings for logarithmic concentration-inhibition rate were performed using the GraphPad Prism 8 analysis software, and the IC₅₀ values of the compounds were obtained (Table 3).

### Test Example 2: KRAS G12V Binding Assay

In this method, the inhibitory effects of the compounds on the binding of KRAS G12V to GTP were assayed by a homogeneous time-resolved fluorescence (HTRF) technique using a KRAS-G12V/GTP binding assay kit (PerkinElmer).

The experimental procedures were as follows (refer to the kit instructions for detailed test methods): the compounds of the present disclosure were each firstly dissolved in DMSO at a concentration of 20 mM, and then diluted in an equal gradient with a buffer in the kit, so that the final concentrations of the test compounds in the reaction system were in a range of 0.02-5000 nM, and the final concentration of DMSO was 0.5%. 5 µL of each compound was incubated with 5 µL of 1× Human KRAS G12V 6His-tagged (PerkinElmer), 5 µL of 1× 6His Eu Cryptate Antibody (PerkinElmer), and 5 µL of 1× GTP Red reagent (PerkinElmer) in a 384-well plate (Greiner) at 25 °C for 1 h. After the incubation was completed, the fluorescence intensity of each well was measured at the excitation wavelength of 337 nm and the emission wavelengths of 620 nm and 665 nm with an EnVision microplate reader (PerkinElmer) in the HTRF mode, and Ratio values were calculated using the formula: Ratio = (665 nm/620 nm) × 10⁴. The inhibition rates of the compounds at each concentration were calculated by comparing the fluorescence intensity ratio with that of the control group. Then non-linear curve fittings for logarithmic concentration-inhibition rate were performed using the GraphPad Prism 8 analysis software, and the IC₅₀ values of the compounds were obtained (Table 3).

### Test Example 3: KRAS WT Binding Assay

In this method, the inhibitory effects of the compounds on the binding of KRAS WT to GTP were assayed by a homogeneous time-resolved fluorescence (HTRF) technique using a KRAS WT:GTP binding assay kit (PerkinElmer).

The experimental procedures were as follows (refer to the kit instructions for detailed test methods): the compounds of the present disclosure were each firstly dissolved in DMSO at a stock concentration of 20 mM. The stock solutions of the compounds were diluted in an equal gradient with DMSO, and then were subjected to second dilution with a buffer in the kit, so that the final concentrations of the test compounds in the reaction system were in a range of 0.02-5000 nM, and the final concentration of DMSO was 0.5%. 5 µL of each compound solution that had been subjected to second dilution was incubated with 5 µL of 1× Human KRAS WT 6His-tagged (PerkinElmer), 5 µL of 1× 6His Eu Cryptate Antibody (PerkinElmer), and 5 µL of 1× GTP Red reagent (PerkinElmer) in a 384-well plate (Greiner) at 25 °C for 1 h. After the incubation was completed, the fluorescence intensity of each well was measured at the excitation wavelength of 337 nm and the emission wavelengths of 620 nm and 665 nm with an EnVision microplate reader (PerkinElmer) in the HTRF mode, and Ratio values were calculated using the formula: Ratio = (665 nm/620 nm) × 104. The inhibition rates of the compounds at each concentration were calculated by comparing the fluorescence intensity ratio with that of the control group. Then non-linear curve fittings for logarithmic concentration-inhibition rate were performed using the GraphPad Prism 8 analysis software, and the IC₅₀ values of the compounds were obtained (Table 3).

### Test Example 4: KRAS G12C Binding Assay

In this method, the inhibitory effects of the compounds on the binding of KRAS G12C to GTP were assayed by a homogeneous time-resolved fluorescence (HTRF) technique using a KRAS G12C:GTP binding assay kit (PerkinElmer).

The experimental procedures were as follows (refer to the kit instructions for detailed test methods): the compounds of the present disclosure were each firstly dissolved in DMSO at a stock concentration of 20 mM. The stock solutions of the compounds were diluted in an equal gradient with DMSO, and then were subjected to second dilution with a buffer in the kit, so that the final concentrations of the test compounds in the reaction system were in a range of 0.02-5000 nM, and the final concentration of DMSO was 0.5%. 5 µL of each compound solution that had been subjected to second dilution was incubated with 5 µL of 1× Human KRAS G12C 6His-tagged (PerkinElmer), 5 µL of 1× 6His Eu Cryptate Antibody (PerkinElmer), and 5 µL of 1× GTP Red reagent (PerkinElmer) in a 384-well plate (Greiner) at 25 °C for 1 h. After the incubation was completed, the fluorescence intensity of each well was measured at the excitation wavelength of 337 nm and the emission wavelengths of 620 nm and 665 nm with an EnVision microplate reader (PerkinElmer) in the HTRF mode, and Ratio values were calculated using the formula: Ratio = (665 nm/620 nm) × 104. The inhibition rates of the compounds at each concentration were calculated by comparing the fluorescence intensity ratio with that of the control group. Then non-linear curve fittings for logarithmic concentration-inhibition rate were performed using the GraphPad Prism 8 analysis software, and the IC₅₀ values of the compounds were obtained (Table 3).

### Test Example 5: KRAS G12C, KRAS G12D, KRAS G12V, KRAS G13D, and KRAS WT (Expanded) Degradation Assays

The degradation effect of the compounds on KRAS was investigated on KRAS G12C mutant human non-small cell lung cancer cell NCI-H358, KRAS G12D mutant human metastatic pancreatic cancer cell AsPc-1, KRAS G12D mutant human lung cancer cell A427, KRAS G12V mutant human colorectal cancer cell SW620, KRAS G12V mutant human lung cancer cell SW900, KRAS G13D mutant human colorectal cancer epithelial cell DLD-1, and KRAS WT human gastric cancer cell MKN-1. The specific procedures were as follows: 0.95 mL of cells were seeded into each well of a 24-well cell culture plate to achieve a cell density of 5 × 10⁵ cells/well; and the cell plate was incubated in an incubator at 37 °C with 5% CO₂ overnight. Then 50 µL of the compound solution after dilution was added to each of the wells with the corresponding cells, so that the final concentration of the compound was in a range of 0.03-3000 nM. The final concentration of DMSO was 0.25%. After the compounds were added, the cell plate was incubated in an incubator at 37 °C with 5% CO₂ for 24 h. The culture medium in the 24-well cell culture plate was then removed, and the plate was washed twice with 1× PBS (KeyGEN). 120 µL of RIPA (strong) lysis buffer (Beyotime) (supplemented with 1 mM phenylmethylsulfonyl fluoride, a protease inhibitor mixture (Beyotime), and a protease phosphatase inhibitor mixture (Beyotime)) was added to lyse adherent SW900 cells at the bottom of the cell culture plate. After the plate was left to stand on ice for 30 min, the protein lysate in each well was transferred to a 1.5 mL centrifuge tube and centrifuged at 15000 g for 20 min at 4 °C. The cell lysate supernatants after centrifugation were cryopreserved in a refrigerator at -80 °C for later use.

After the samples of the cell lysate supernatants that had been prepared were thawed from the refrigerator at -80 °C, the total protein concentrations of the cell lysates were determined using a BCA protein quantification kit (Tiangen). The total protein concentrations of the samples were then adjusted to 0.5 µg/µL with PBS and 5× SDS-PAGE protein loading buffer (Beyotime), and the samples were incubated in a water bath at 100 °C for 15 min. The samples were then incubated in an ice bath for 5 min and centrifuged at 14000 g for 1 min at 4 °C, and the supernatants were mixed well as loading samples for the Western Blot assay. The samples were loaded into wells of a 10% precast gel (KeyGEN) with a loading volume of 10 µL (total protein: 5 µg), a sufficient Tris-MOPS-SDS running buffer (Adamas) was added, and then electrophoresis was performed at a constant voltage of 120 V for 55 min. After the electrophoresis, the proteins on the gel were transferred to PVDF membranes at a constant current of 250 mA for 55 min. After the membrane transfer, the PVDF membranes were incubated in 1× Quick Block blocking buffer (Beyotime) at room temperature for 30 min. After the blocking, the PVDF membranes were separately incubated at 4 °C overnight with the KRAS antibody (Abcam) diluted in a ratio of 1:1000 and the β-actin antibody (Abcam) diluted in a ratio of 1:2000 in 5% BSA as the diluent, and then washed with 1× TBST buffer (2.4 g of Tris, 8.8 g of NaCl, 1.5 mL of Tween 20, with pH adjusted to 7.4, with the volume brought to 1 L) 3 times for 10 min/time. The membranes were incubated with a secondary antibody (Abcam) diluted in 5% BSA at room temperature for 2 h, and then washed with 1× TBST buffer 3 times for 10 min/time. Finally, the membranes were incubated with Clarity Western ECL Substrate (BIO-RAD) for 5 min for luminescence and color development, and a ChemiScope 6200 Touch chemiluminescence imaging system (Clinx) was used for color development and protein mapping. Gray value analysis of the protein maps was performed using the chemiluminescence analysis software (Clinx). The gray correction value for each sample was calculated using the formula: gray correction value = (gray value of target protein/gray value of corresponding internal reference) × 10³. Then degradation rates were calculated by comparison with the gray correction value of the control group. Then non-linear curve fittings for logarithmic concentration-inhibition rate were performed using the GraphPad Prism 8 analysis software, and the DC₅₀ and Dₘₐₓ values of the compounds were obtained (Tables 4 and 5).

### Test Example 6: 3D Cell Proliferation Inhibition Assay

The inhibitory effects of the compounds on 3D cell proliferation were investigated on KRAS G12C mutant human non-small cell lung cancer cell NCI-H358, KRAS G12D mutant human lung cancer cell A427, KRAS G12D mutant human gastric cancer cell AGS, KRAS G12D mutant human metastatic pancreatic cancer cell AsPc-1, KRAS G12V mutant human lung squamous carcinoma cell SW900, KRAS G12V mutant human colorectal cancer cell SW620, KRAS G13D mutant human colorectal cancer epithelial cell DLD-1, and KRAS WT human gastric cancer cell MKN1. The specific procedures were as follows:

10 µL of each compound solution diluted in a gradient was added to corresponding wells of a 96-well low-adsorption microplate (PerkinElmer), so that the final concentrations of the test compounds in the reaction system were in a range of 10000-0.026 nM. The final concentration of DMSO was 0.2%. Then 90 µL of cells were correspondingly seeded into each well to achieve a cell density of 5 × 10² cells/well (the cell density of SW900 was 5 × 10³ cells/well, and the cell density of MKN1 was 1 × 10³ cells/well). In addition to test wells for the test compounds, both a DMSO control well and a medium control well were set, with the DMSO control well containing DMSO and cells and the medium well containing only a medium. After the sample loading, the 96-well low-adsorption microplate was incubated in an incubator at 37 °C with 5% carbon dioxide for 7 days. After 7 days, the microplate was taken out, and 100 µL of CTG reagent (Promega) was added to each well. The plate was incubated at room temperature for 60 min and read on a microplate reader EnVision by the chemiluminescence program. The inhibition rates of the compounds at each concentration were calculated using the formula for the percentage inhibition of cell proliferation: inhibition rate % = (mean value of DMSO control group - single concentration reading value of compound)/(mean value of DMSO control group - mean value of medium control group) × 100. Then non-linear curve fittings for logarithmic concentration-inhibition rate were performed by GraphPad Prism 8, and the IC₅₀ values of the compounds were obtained (Table 6).

**Table 3**

| Example | KRAS WT:GTP IC₅₀ (nM) | KRAS G12C:GTP IC₅₀ (nM) | KRAS G12D:GTP IC₅₀ (nM) | KRAS G12V:GTP IC₅₀ (nM) |
|---|---|---|---|---|
| Example 1 | C | B | A | A |
| Example 2 | - | - | - | A |
| Example 3 | B | A | A | A |
| Example 6 | - | - | - | A |
| Example 7 | - | - | - | A |
| Example 11 | - | - | - | B |
| Example 14 | - | - | - | A |
| Example 15 | - | - | - | A |
| Example 16 | - | - | - | A |
| Example 29 | - | - | - | A |
| Example 30 | - | - | - | B |
| Example 40 | - | - | - | B |

In Table 3: "-" represents undetectable;
when IC₅₀ ≤ 500 nM, the level was A; when 500 nM < IC₅₀ ≤ 1000 nM, the level was B; when 1000 nM < IC₅₀ ≤ 10 µM, the level was C; when 10 µM < IC₅₀, the level was D.

**Table 4**

| Example | A427 (KRAS G12D) | | SW900 (KRAS G12V) | | SW620 (KRAS G12V) | | MKN1 (KRAS WT) | |
|---|---|---|---|---|---|---|---|---|
| | DC₅₀ (nM) | Dmax (%) | DC₅₀ (nM) | Dmax (%) | DC₅₀ (nM) | Dmax (%) | DC₅₀ (nM) | Dmax (%) |
| Example 1 | A | A | A | A | 2.67 | 100 | A | A |
| Example 2 | - | - | A | A | - | - | - | - |
| Example 3 | A | A | A | A | - | - | A | A |
| Example 4 | - | - | A | A | - | - | - | - |
| Example 5 | - | - | A | A | - | - | - | - |
| Example 6 | A | A | A | A | 3.56 | 99.07 | A | A |
| Example 7 | A | A | A | A | 0.62 | 100 | A | A |
| Example 8 | - | - | A | A | - | - | - | - |
| Example 9 | - | - | A | A | - | - | - | - |
| Example 10 | - | - | A | A | - | - | - | - |
| Example 11 | - | - | A | A | - | - | - | - |
| Example 12 | - | - | A | A | - | - | - | - |
| Example 14 | - | - | A | B | - | - | - | - |
| Example 18 | - | - | C | B | - | - | - | - |
| Example 19 | - | - | A | A | - | - | - | - |
| Example 21 | - | - | A | A | - | - | - | - |
| Example 23 | - | - | A | A | - | - | - | - |
| Example 24 | - | - | A | A | - | - | - | - |
| Example 25 | - | - | A | A | - | - | - | - |
| Example 26 | - | - | A | A | - | - | - | - |
| Example 27 | - | - | A | A | - | - | - | - |
| Example 28 | - | - | A | A | - | - | - | - |
| Example 29 | - | - | A | A | - | - | - | - |
| Example 30 | - | - | A | B | - | - | - | - |
| Example 31 | - | - | B | B | - | - | - | - |
| Example 32 | - | - | A | B | - | - | - | - |
| Example 33 | - | - | A | A | - | - | - | - |
| Example 34 | - | - | A | A | - | - | - | - |
| Example 35 | - | - | A | A | - | - | - | - |
| Example 36 | - | - | A | A | - | - | - | - |
| Example 38 | - | - | A | A | - | - | - | - |
| Example 39 | - | - | A | A | - | - | - | - |
| Example 40 | - | - | A | A | - | - | - | - |
| Example 41 | - | - | A | A | - | - | - | - |
| Example 42 | - | - | A | A | - | - | - | - |
| Example 43 | - | - | A | A | - | - | - | - |
| Example 45 | - | - | A | A | - | - | - | - |
| Example 46 | - | - | - | - | 0.87 | 100 | 1.26 | 100 |
| Example 47 | - | - | - | - | 0.99 | 100 | 0.28 | 97.41 |
| Example 48 | - | - | - | - | 1.02 | 100 | 2.33 | 100 |
| Example 49 | - | - | 62.95 | 75.26 | - | - | - | - |
| Example 50 | - | - | - | - | 3.22 | 99.37 | - | - |
| Example 60 | - | - | - | - | 4.25 | 99.07 | - | - |
| Example 61 | - | - | - | - | 18.86 | 99.31 | - | - |
| Example 67 | - | - | - | - | 33.58 | 93.13 | - | - |
| Example 72 | - | - | - | - | 11.81 | 95.23 | - | - |
| Example 86 | - | - | - | - | 14.24 | 89.18 | - | - |
| Example 91 | - | - | - | - | 5.16 | 100 | 1.15 | 98.94 |
| Example 92 | - | - | - | - | 1.34 | 100 | 0.42 | 99.02 |
| Example 111 | - | - | - | - | 1.65 | 100 | 0.69 | 99.86 |
| Example 132 | - | - | - | - | 2.42 | 100 | - | - |
| Example 133 | - | - | - | - | 6.73 | 98.88 | - | - |
| Example 162 | - | - | - | - | 21.7 | 100 | - | - |
| Example 163 | - | - | 251.19 | 70.95 | - | - | - | - |
| Example 164 | - | - | - | - | 7.48 | 99.45 | 1.25 | 99.5 |
| Example 165 | - | - | 5.9 | 100 | 7.6 | 100 | - | - |
| Example 170 | - | - | - | - | 2.35 | 97.72 | 0.76 | 99.35 |

**Table 5**

| Example | NCI-H358 (KRAS G12C) | | DLD-1 (KRAS G13D) | | AsPc-1 (KRAS G12D) | |
|---|---|---|---|---|---|---|
| | DC₅₀ (nM) | Dmax (%) | DC₅₀ (nM) | Dmax (%) | DC₅₀ (nM) | Dmax (%) |
| Example 1 | A | A | A | A | A | A |
| Example 3 | A | A | A | A | A | A |
| Example 6 | A | A | A | A | A | A |
| Example 7 | A | A | A | A | A | A |
| Example 39 | - | - | - | - | A | B |
| Example 42 | - | - | A | A | - | - |
| Example 46 | 3.14 | 86.73 | 2.36 | 100 | - | - |
| Example 47 | 1.7 | 88.91 | 2.05 | 100 | 18.68 | 93.3 |
| Example 48 | 17.2 | 100 | 1.34 | 100 | - | - |
| Example 49 | - | - | - | - | 822.24 | 57 |
| Example 50 | 21.59 | 96.3 | 3.98 | 98 | 167.1 | 77.08 |
| Example 91 | 6.04 | 92.7 | 8.8 | 100 | - | - |
| Example 111 | - | - | 6.78 | 99.3 | - | - |
| Example 132 | 1.37 | 100 | 5.6 | 100 | 25.23 | 95.3 |
| Example 133 | - | - | 7.28 | 100 | - | - |
| Example 163 | - | - | - | - | 185.24 | 89.02 |
| Example 164 | 1.11 | 98.2 | 4.39 | 100 | 5.91 | 99.56 |
| Example 165 | - | - | - | - | 13.35 | 100 |
| Example 170 | 2.65 | 96.06 | 1.96 | 99.94 | 5.53 | 100 |

In Tables 4 and 5: "-" represents undetectable;
when DC₅₀ ≤ 50 nM, the level was A; when 50 nM < DC₅₀ ≤ 100 nM, the level was B; when 100 nM < DC₅₀ ≤ 500 nM, the level was C; when 500 nM < DC₅₀ ≤ 1000 nM, the level was D; when 1000 nM < DC₅₀ ≤ 10 µM, the level was E;
when 80 ≤ Dₘₐₓ (%) ≤ 100, the level was A; when 50 ≤ Dₘₐₓ (%) < 80, the level was B; when 30 ≤ Dₘₐₓ (%) < 50, the level was C; when Dₘₐₓ (%) < 30, the level was D.

**Table 6**

| Example | 3D Cell proliferation IC₅₀ (nM) | | | | | | | |
|---|---|---|---|---|---|---|---|---|
| | A427 (KRAS G12D) | AGS (KRAS G12D) | AsPc-1 (KRAS G12D) | NCI-H358 (KRAS G12C) | SW900 (KRAS G12V) | SW620 (KRAS G12V) | DLD-1 (KRAS G13D) | MKN1 (KRAS WT) |
| Example 1 | A | A | A | A | A | 1.41 | A | A |
| Example 2 | A | A | A | A | A | 1.85 | A | A |
| Example 3 | A | A | A | A | C | 2.52 | A | A |
| Example 4 | C | A | B | A | A | - | - | A |
| Example 5 | A | A | A | A | B | - | A | A |
| Example 6 | A | A | A | A | A | 1.28 | A | A |
| Example 7 | A | A | A | A | A | 1.81 | A | A |
| Example 8 | B | A | A | A | A | - | A | A |
| Example 9 | A | A | B | A | A | - | A | A |
| Example 10 | B | A | B | A | A | - | B | A |
| Example 11 | B | A | A | A | A | 5.43 | A | A |
| Example 12 | C | A | C | A | A | 15.35 | - | A |
| Example 13 | D | D | - | D | A | - | - | B |
| Example 19 | C | A | A | A | A | 0.82 | A | A |
| Example 21 | B | A | A | C | A | 5.54 | - | A |
| Example 23 | - | 69.49 | 64.22 | 23.55 | 46.81 | 12.33 | 63.53 | 5.55 |
| Example 24 | C | B | A | C | A | 9.33 | - | A |
| Example 25 | C | A | A | C | A | 2.45 | A | A |
| Example 26 | C | A | A | C | A | 2.11 | A | A |
| Example 27 | - | - | A | A | A | 3.47 | - | A |
| Example 28 | C | A | A | A | A | - | A | A |
| Example 29 | - | - | A | - | A | 4.46 | - | A |
| Example 33 | A | A | A | B | B | 7.71 | A | B |
| Example 34 | C | A | A | B | A | - | - | A |
| Example 35 | D | A | A | A | C | - | - | A |
| Example 36 | B | A | A | B | A | 3.36 | A | A |
| Example 37 | - | - | C | - | A | - | - | A |
| Example 38 | - | - | B | - | C | - | - | A |
| Example 39 | - | - | - | 11.63 | - | 35.61 | 86.6 | - |
| Example 41 | - | - | B | - | A | 9.08 | - | A |
| Example 42 | C | A | A | A | A | 2.15 | A | A |
| Example | - | - | B | - | A | - | - | B |
| 43 | | | | | | | | |
| Example 45 | B | A | A | C | A | - | A | B |
| Example 46 | - | - | 9.03 | 18.22 | - | 2.81 | 5.49 | 2.01 |
| Example 47 | - | - | 6.48 | 2.33 | - | 1.92 | 3.55 | 2.33 |
| Example 48 | - | - | 23.24 | 85.51 | - | 5.17 | 2.61 | 21.17 |
| Example 49 | - | - | - | 67.39 | 527.3 | 27.38 | 46.13 | 39.21 |
| Example 50 | - | - | 157.4 | 46.72 | - | 3.73 | 90.92 | 23.68 |
| Example 60 | - | - | 60.8 | 24.02 | - | 15.39 | 57.52 | - |
| Example 61 | - | - | 68.22 | 18.9 | - | 13.3 | 39.94 | - |
| Example 67 | - | - | 416.6 | 535.2 | - | 32.85 | 70.2 | - |
| Example 72 | - | - | 175.7 | 32.89 | - | 21.96 | 54.29 | 102.2 |
| Example 86 | - | - | 21.18 | 6.63 | - | 7.01 | 9.98 | 5.23 |
| Example 91 | - | - | 4.26 | 2.13 | - | 1.92 | 8.33 | 5.41 |
| Example 92 | - | - | 6.75 | 19.29 | - | 1.31 | 2.26 | 447.4 |
| Example 111 | - | - | 4.68 | 2.50 | - | 1.12 | 5.88 | 2.15 |
| Example 132 | - | - | 9.75 | 6.20 | - | 0.43 | 7.7 | 4.31 |
| Example 133 | - | - | 24.0 | 5.58 | - | 2.93 | 5.68 | 9.19 |
| Example 162 | - | - | 19.9 | 3.83 | - | 4.85 | 12.84 | - |
| Example 163 | 563.5 | 587.8 | 368.3 | 420.1 | 235 | 285.3 | - | 367.4 |
| Example 164 | - | - | 12.11 | 4.66 | - | 3.76 | 3.9 | 22.58 |
| Example 165 | 108.4 | 27.82 | 18.63 | 7.23 | 15.58 | 4.58 | 18.85 | 52.51 |
| Example 166 | - | - | 523.3 | - | - | - | - | - |
| Example 170 | - | - | 6.31 | 2.49 | - | 2.59 | 6.28 | 6.7 |

In Table 6: "-" represents undetectable;
when IC₅₀ ≤ 50 nM, the level was A; when 50 nM < IC₅₀ ≤ 100 nM, the level was B; when 100 nM < IC₅₀ ≤ 500 nM, the level was C; when 500 nM < IC₅₀ ≤ 1000 nM, the level was D; when 1000 nM < IC₅₀ ≤ 10 µM, the level was E.

Experimental conclusion: the compounds of the present disclosure are able to effectively bind to the pan-KRAS target protein or have an inhibitory effect, and the compounds of the present disclosure are able to effectively and specifically degrade the pan-KRAS protein. The compounds of the present disclosure are able to effectively inhibit 3D cell proliferation.

## Claims

1. A compound of formula I or I', and/or a stereoisomer, an enantiomer, a diastereomer, an atropisomer, a deuteride, a hydrate, a solvate or a prodrug thereof and/or a pharmaceutically acceptable salt thereof:
K-L-E I
K'-L-E I'
wherein:
K is K1: 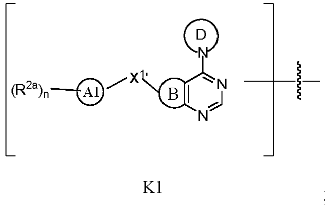
K' is K2: 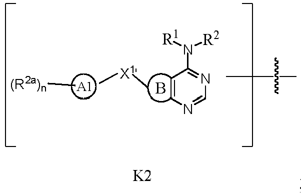
each R^{2a} is independently halogen, deuterium, cyano, amino, hydroxy, C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, triazolyl, -O-C₁-C₆ alkyl, -O-C₃-C₈ cycloalkyl, -CH₂C(=O)N(R^{3a})₂, N(R^{3a})₂, C₁-C₃ alkyl-O-C₁-C₃ alkyl-, HC(=O)-, - CO₂R^{3a}, -CON(R^{3a})₂, or 5- to 6-membered heteroaryl, wherein the C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, -O-C₁-C₆ alkyl, (NR^{3a})₂, -O-C₃-C₈ cycloalkyl, or 5- to 6-membered heteroaryl is optionally substituted with 1 or more deuterium, halogen, hydroxy, cyano, amino, nitro, C₁-C₃ alkoxy, or C₁-C₃ alkyl;
each R^{3a} is independently selected from H, C₁-C₆ alkyl, C₃-C₈ cycloalkyl, C₃-C₆ alkenyl, and C₃-C₆ alkynyl; or two R^{3a}, together with the atom linked thereto, form 4- to 12-membered heterocycloalkyl, wherein the 4-to 12-membered heterocycloalkyl is optionally substituted with 1 or more deuterium, halogen, hydroxy, cyano, amino, C₁-C₃ alkoxy, or C₁-C₃ alkyl;
each ring A1 is independently absent, C₆-C₁₀ aryl, 5- to 10-membered heteroaryl, or 5- to 10-membered heterocycloalkyl;
each ring B is independently C₆-C₁₅ aryl, 5- to 15-membered heteroaryl, C₅-C₁₅ cycloalkyl, or 5- to 16-membered heterocycloalkyl, wherein the C₆-C₁₅ aryl, 5- to 15-membered heteroaryl, C₅-C₁₅ cycloalkyl, or 5- to 16-membered heterocycloalkyl is optionally substituted with 1 or more R^{a};
ring D is 4- to 12-membered heterocycloalkyl, wherein the 4- to 12-membered heterocycloalkyl is optionally substituted with 1 or more R^{a}, and the 4- to 12-membered heterocycloalkyl contains at least one heteroatom or heteroatom group selected from N, S, and O;
each R^{a} is independently selected from hydrogen, hydroxy, halogen, cyano, -N(R^{3a})₁₋₂, -CH₂N(R^{3a})₁₋₂, 3- to 8-membered heterocycloalkyl, C₁-C₆ alkyl, HC(=O)-, -CO₂R^{4a}, -CON(R^{4a})₁₋₂, C₁-C₃ alkoxy, (C₁-C₃ alkoxy)-C₁-C₃ alkyl-, C₁-C₃ alkyl-N(R^{4a})₁₋₂, C₂-C₄ alkenyl, C₂-C₆ alkynyl, C₃-C₈ cycloalkyl, 3- to 8-membered heterocycloalkyl, and 5- to 6-membered heteroaryl, wherein the C₁-C₆ alkyl, C₁-C₆ heteroalkyl, -O(C₁-C₆ alkyl), -OC₁-C₆ heteroalkyl, -O(C₃-C₈ cycloalkyl), -O(3- to 8-membered heterocycloalkyl), -S(C₁-C₆ alkyl), -S(C₃-C₈ cycloalkyl), -O-phenyl, -O-pyridyl, C₁-C₃ alkoxy, C₂-C₄ alkenyl, C₂-C₆ alkynyl, C₃-C₈ cycloalkyl, 3- to 8-membered heterocycloalkyl, or 5- to 6-membered heteroaryl is optionally substituted with 1 or more deuterium, halogen, cyano, hydroxy, amino, nitro, C₁-C₃ alkyl, or C₁-C₃ alkoxy;
each R^{4a} is independently selected from hydrogen, C₁-C₄ alkyl, and C₁-C₃ hydroxyalkyl;
or two R^{4a}, together with the atom linked thereto, form heterocycloalkyl, wherein the heterocycloalkyl is optionally substituted with 1 or more deuterium, -OH, -NH₂, C₁-C₃ alkyl, or C₁-C₃ alkoxy;
each X¹' is independently a bond or -C(O)-;
each R¹ and each R² are independently hydrogen, C₁-C₆ alkyl, C₁-C₆ alkoxy, C₃-C₈ cycloalkyl, or 3- to 8-membered heterocycloalkyl, wherein the C₁-C₆ alkyl, C₃-C₈ cycloalkyl, or 3- to 8-membered heterocycloalkyl is optionally substituted with one or more deuterium, halogen, hydroxy, amino, -C₁-C₃ alkyl, -S-C₁-C₃ alkyl, -O-C₁-C₃ alkyl, or -NH-C₁-C₃ alkyl;
n is 0, 1, 2, 3, or 4;
L is a linking chain, which links K and E by means of covalent bonds;
L is a linking chain, which links K' and E by means of covalent bonds;
each E is independently E1, E2, or E3: 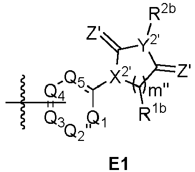
wherein in E1:
Z' is O, S, or CH₂;
X²' is CH or N;
Y²' is CH, N, O, or S;
Q₁, Q₂, Q₃, Q₄, and Q₅ are each independently CR^{3b} or N;
each R^{3b} is independently hydrogen, deuterium, hydroxy, amino, cyano, halogen, nitro, sulfhydryl, C₁-C₆ alkyl, C₁-C₆ heteroalkyl, C₂-C₆ alkenyl, C₂-C₆ alkynyl, C₃-C₈ cycloalkyl, 3- to 8-membered heterocycloalkyl, C₆-C₁₀ aryl, 5- to 10-membered heteroaryl, -O-(C₁-C₆ alkyl), -O-(C₁-C₆ heteroalkyl), -O-(C₃-C₈ cycloalkyl), -O-(3- to 8-membered heterocycloalkyl), -S-(C₁-C₆ alkyl), -S-(C₁-C₆ heteroalkyl), -S-(C₃-C₈ cycloalkyl), -S-(3- to 8-membered heterocycloalkyl), -N(C₁-C₆ alkyl)₁₋₂, -N(C₁-C₆ heteroalkyl)₁₋₂, - N(C₃-C₈ cycloalkyl)₁₋₂, -N(3- to 8-membered heterocycloalkyl)₁₋₂, -O-(C₆-C₁₀ aryl), or -O-(5- to 10-membered heteroaryl); the C₁-C₆ alkyl, C₁-C₆ heteroalkyl, C₂-C₆ alkenyl, C₂-C₆ alkynyl, C₃-C₈ cycloalkyl, 3- to 8-membered heterocycloalkyl, C₆-C₁₀ aryl, or 5- to 10-membered heteroaryl is optionally substituted with 1-3 groups independently selected from deuterium, hydroxy, halogen, cyano, amino, O(C₁-C₆ alkyl), O-(C₃-C₈ cycloalkyl), -O-(3- to 8-membered heterocycloalkyl), N(C₁-C₆ alkyl)₁₋₂, -NH(C₃-C₈ cycloalkyl), - NH(3- to 8-membered heterocycloalkyl), -O-(C₆-C₁₀ aryl), and -O-(5- to 10-membered heteroaryl); or R^{3b}, together with the atom linked thereto, form cycloalkyl, heterocycloalkyl, heteroaryl, or aryl;
m" is 1, 2, or 3;
each R^{1b} is independently hydrogen, deuterium, hydroxy, amino, cyano, halogen, C₁-C₆ alkyl, C₁-C₆ heteroalkyl, C₃-C₈ cycloalkyl, C₃-C₈ heterocycloalkyl, C₆-C₁₀ aryl, 5- to 10-membered heteroaryl, -O(C₁-C₆ alkyl), -O-(C₃-C₈ cycloalkyl), -O-(3- to 8-membered heterocycloalkyl), -N(C₁-C₆ alkyl)₁₋₂, -NH(C₃-C₈ cycloalkyl), -NH(3- to 8-membered heterocycloalkyl), -O-(C₆-C₁₀ aryl), or -O-(5- to 10-membered heteroaryl), wherein the alkyl, cycloalkyl, heterocycloalkyl, aryl, or heteroaryl is optionally substituted with 1-3 groups independently selected from hydroxy, halogen, cyano, hydroxy, and amino;
R^{2b} is absent, hydrogen, deuterium, C₁-C₆ alkyl, or C₃-C₆ cycloalkyl, wherein the C₁-C₆ alkyl and C₃-C₆ cycloalkyl are optionally substituted with 1-3 groups independently selected from hydroxy, halogen, cyano, hydroxy, amino, and -OC(O)(C₁-C₆ alkyl); 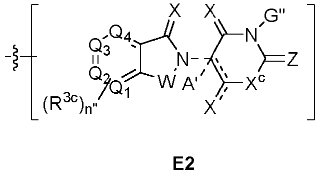
wherein in E2:
Q₁, Q₂, Q₃, and Q₄ are each independently CR^{3b} or N;
W is CR^{1c}R^{2c}, C(S), C(O), SO₂, -OC=R^{4c}-, -SC=R^{4c}-, -C=R^{4c}NR^{5c}-, or -N=CA'-;
X is CH₂, O, or S;
X^{c}is -CH₂- or -NG'-;
Z is CH₂, O, or S;
G' and G" are each independently selected from hydrogen, deuterium, C₁-C₆ alkyl, OH, C₃-C₆ cycloalkyl, - CH₂-heterocycloalkyl, and -CH₂-phenyl, wherein the C₁-C₆ alkyl, C₃-C₆ cycloalkyl, -CH₂-heterocycloalkyl, or -CH₂-phenyl is optionally substituted with 1 or more hydroxy, halogen, cyano, and amino;
A' is hydrogen, deuterium, C₁-C₆ alkyl, C₃-C₈ cycloalkyl, or halogen;
R^{1c}, R^{2c}, and R^{3c} are each independently selected from hydrogen, deuterium, hydroxy, halogen, -NH₂, -N(C₁-C₆ alkyl)₁₋₂, C₁-C₆ alkyl, C₁-C₆ alkoxy, C₁-C₆ haloalkyl, -CONR_{'}R_{"} -OR_{'}, -NR_{'}R_{"}, -SR_{'}, -SO₂R_{'}, -SO₂NR_{'}R_{"}, -CR_{'}R_{"}, -CR_{'}NR_{'}R_{"}, C₆-C₁₀ aryl, 5- to 10-membered heteroaryl, C₃-C₈ cycloalkyl, 3- to 8-membered heterocycloalkyl, -P(O)(OR_{'})R_{"}, -P(O)R_{'}R_{"}, -OP(O)(OR_{'})R_{"}, -CN, -NR_{'}SO₂NR_{'}R_{"}, -NR_{'}C(O)NR_{'}R_{"}, - C(O)NR_{'}C(O)R_{"}, -NR_{'}C(=N-CN)NR_{'}R_{"}, -C(=N-CN)NR_{'}R_{"}, -NR_{'}C(=N-CN)R_{"}, -NR_{'}C(=C-NO₂)NR_{'}R_{"}, - SO₂NR_{'}COR_{"}, -NO₂, -COR_{'}, -C(C=N-OR_{'})R_{"}, -CR_{'}=CR_{'}R_{"}, -CCR', -S(C=O)(C=N-R_{'})R_{"}, -SF₅ and -OCF₃;
R^{4c} is O or S;
R^{5c} is H, C₁-C₆ alkyl, C₆-C₁₀ aryl, 5- to 12-membered heteroaryl, C₃-C₈ cycloalkyl, or 3- to 8-membered heterocycloalkyl;
R_{'} and R_{"} are each independently selected from a bond, hydrogen, deuterium, C₁-C₆ alkyl, C₃-C₈ cycloalkyl, C₆-C₁₀ aryl, 5- to 10-membered heteroaryl, and 3- to 8-membered heterocycloalkyl;
n" is 0, 1, 2, 3, or 4;
-̅ -̅ -̅ is a single bond or a double bond;
----- is a bond, which may be an R stereoisomer, an S stereoisomer, or a non-stereoisomer; 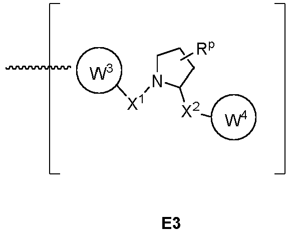
wherein in E3:
X¹ and X² are each independently a bond, O, C(O), C(S), NR^{1d}, or CR^{1d}R^{2d};
R^{1d} and R^{2d} are each independently selected from H, deuterium, and C₁-C₆ alkyl, wherein the C₁-C₆ alkyl is optionally substituted with 1 or more halogen or C₁-C₆ alkoxy;
R^{P} is independently selected from H, deuterium, halogen, -OH, and C₁-C₃ alkyl, wherein the C₁-C₃ alkyl is optionally substituted with 1 or more halogen, hydroxy, or C₁-C₃ alkoxy;
W³ is C₁-C₆ alkyl, -T-N(R^{3d}R^{4d}), -T-N(R^{3d}R^{4d})X³, -T-(C₆-C₁₀) aryl, -T-(5- to 10-membered)heteroaryl, -T-(4-to 12-membered) heterocycloalkyl, -NR^{5d}-T-(C₆-C₁₀) aryl, -NR^{5d}-T-(5- to 10-membered)heteroaryl, or - NR^{5d}-T-(4- to 12-membered) heterocycloalkyl, wherein the C₁-C₆ alkyl, -T-N(R^{3d}R^{4d}), -T-N(R^{3d}R^{4d})X³, -T-(C₆-C₁₀) aryl, -T-(5- to 10-membered)heteroaryl, -T-(4- to 12-membered)heterocycloalkyl, -NR^{5d}-T-(C₆-C₁₀) aryl, -NR^{5d}-T-(5- to 10-membered)heteroaryl, or -NR^{5d}-T-(4- to 12-membered)heterocycloalkyl is optionally substituted;
X³ is C(O), R^{3d}, R^{4d}, or R^{5d};
R^{3d}, R^{4d}, or R^{5d} is each independently selected from H, deuterium, and C₁-C₆ alkyl, wherein the C₁-C₆ alkyl is optionally substituted with 1 or more halogen, -OH, R^{1d}C(O), R^{1d}C(S), R^{1d}SO, R^{1d}SO₂, NR^{1d}R^{2d}C(O), NR^{1d}R^{2d}C(S), NR^{1d}R^{2d}SO, or NR^{1d}R^{2d}SO₂;
T is C₁-C₆ alkyl or -(CH₂)_{n'}-, wherein 1 or more methylene groups in the -(CH₂)_{n'}- are optionally substituted with groups selected from deuterium, halogen, and C₁-C₆ alkyl, and the C₁-C₆ alkyl is optionally substituted with halogen, -OH, or amino;
n' is 0, 1, 2, 3, 4, 5, or 6;
W⁴ is 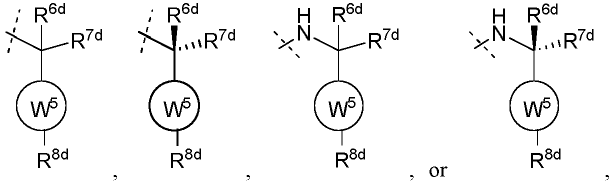 wherein the 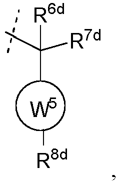 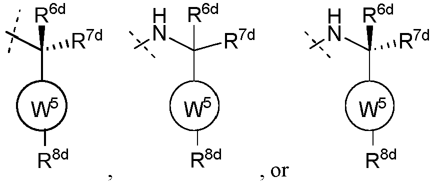 is optionally substituted;
R^{6d} and R^{7d} are each independently H, deuterium, C₃-C₈ cycloalkyl, or C₁-C₆ alkyl, wherein the C₃-C₈ cycloalkyl or C₁-C₆ alkyl is optionally substituted with halogen, -OH, CN, NO₂, or amino;
W⁵ is 6- to 10-membered aryl or 5- to 10-membered heteroaryl;
R^{8d} is H, deuterium, halogen, CN, OH, NO₂, NR^{6d}R^{7d}, OR^{6d}, COR^{6d}R^{7d}, NR^{6d}COR^{7d}, SO₂R^{6d}R^{7d}, R^{6d}SO₂R^{7d}, C₁-C₆ alkyl, C₁-C₆ alkoxy, C₆-C₁₀ aryl, 5- to 10-membered heteroaryl, C₃-C₈ cycloalkyl, or 3- to 8-membered heterocycloalkyl, wherein the C₁-C₆ alkyl or C₁-C₆ alkoxy is optionally substituted with deuterium, halogen, -OH, CN, NO₂, or amino.

2. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1, wherein
in formula I or I', the definitions of one or more of the groups R^{2a}, R^{3a}, R^{a}, and R^{4a} are also replaced by the following definitions, provided that L is linked to E via -C(O)-;
R^{2a} is sulfhydryl, (C₁-C₃ alkoxy)C₁-C₃ alkyl-, -O-C₁-C₆ heteroalkyl, -OCOR^{3a}, -NH(C₁-C₆ heteroalkyl), - N(C₁-C₆ heteroalkyl)(C₁-C₆ heteroalkyl), or -C₃-C₄ alkynyl-N(R^{5a'})₂;
in R^{2a}, the hydroxy is independently optionally substituted with 1 or more deuterium C₁-C₃ alkoxy, C₁-C₃ alkyl, C₃-C₈ cycloalkyl, -Si-C₁-C₃ alkyl, 3- to 8-membered heterocycloalkyl, or -CON(R^{3a})₂;
in R^{2a}, the C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, -O-C₁-C₆ heteroalkyl, -O-C₃-C₈ cycloalkyl, and 5- to 6-membered heteroaryl are independently optionally substituted with 1 or more C₃-C₈ cycloalkyl, -Si-C₁-C₃ alkyl, 3- to 8-membered heterocycloalkyl, or -CON(R^{3a})₂;
each R^{3a} is deuterium or -NH-C₁-C₃ alkyl;
R^{a} is independently a bond, deuterium, oxo, cyanomethyl, C₁-C₆ heteroalkyl, -O(C₁-C₆ alkyl), -O(C₃-C₈ cycloalkyl), -O(C₁-C₆ heteroalkyl), -O(3- to 8-membered heterocycloalkyl), -S(C₁-C₆ alkyl), -S(C₃-C₈ cycloalkyl), -O-phenyl, -O-pyridyl, triazolyl, -CH₂C(=O)N(R^{3a})₂, or -C₃-C₄ alkynyl-N(R^{3a})₂;
or two adjacent R^{a}, together with the atoms linked thereto, form 6- to 10-membered aryl, 5- to 10-membered heteroaryl, 5- to 8-membered cycloalkyl, or 5- to 8-membered heterocycloalkyl, wherein the 6- to 10-membered aryl, 5- to 10-membered heteroaryl, 5- to 8-membered cycloalkyl, or 5- to 8-membered heterocycloalkyl is optionally substituted with 1 or more hydroxy, amino, halogen, cyano, or nitro;
in R^{a}, the C₁-C₆ alkyl, C₁-C₃ alkoxy, C₂-C₄ alkenyl, C₃-C₈ cycloalkyl, 3- to 8-membered heterocycloalkyl, or 5- to 6-membered heteroaryl is optionally substituted with 1 or more C₁-C₃ haloalkyl;
in R^{a}, the C₁-C₆ heteroalkyl, phenyl, pyridyl, C₂-C₄ alkynyl, triazolyl, -CH₂C(=O)N(R^{3a})₁₋₂, or -C₃-C₄ alkynyl-N(R^{3a})₂ is optionally substituted with 1 or more deuterium, halogen, cyano, hydroxy, amino, nitro, C₁-C₃ haloalkyl, C₁-C₃ alkyl, or C₁-C₃ alkoxy;
each R^{4a} is independently C₁-C₆ alkyl.

3. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein K1 is K1-I or K1-II, and K2 is K2-I: 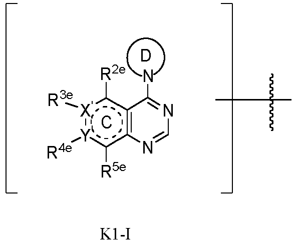 wherein in K1-I:
ring C is C₆-C₁₅ aryl, 5- to 10-membered heteroaryl, C₆-C₁₅ cycloalkyl, or 5- to 10-membered heterocycloalkyl;
X' is a bond, C, O, or N;
Y' is C or N; 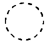
is one or more double bonds that are optionally present;
R^{2e} is H, deuterium, halogen, hydroxy, amino, C₁-C₆ alkyl, C₃-C₈ cycloalkyl, -O(C₁-C₆ alkyl), or -O(C₃-C₈ cycloalkyl);
R^{3e} is a bond, H, deuterium, halogen, cyano, oxo, -O(C₁-C₆ alkyl), -O(C₁-C₆ heteroalkyl), -O(C₃-C₈ cycloalkyl), -O(3- to 8-membered heterocycloalkyl), -S(C₁-C₆ alkyl), -S(C₃-C₈ cycloalkyl), -O-phenyl, or - O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
or R^{4e}, together with the carbon atom linked thereto, forms C₆-C₁₂ aryl or 5- to 12-membered heteroaryl, wherein the C₆-C₁₂ aryl or 5- to 12-membered heteroaryl is optionally substituted with H, deuterium, halogen, hydroxy, amino, C₁-C₆ alkyl, S(C₁-C₆ alkyl), or O(C₁-C₆ alkyl);
R^{5e} is hydrogen, deuterium, halogen, cyano, C₁-C₆ alkyl, C₃-C₈ cycloalkyl, -O(C₁-C₆ alkyl), or -O(C₃-C₈ cycloalkyl);
R^{a'} is halogen, cyano, hydroxy, C₁-C₆ alkyl, C₁-C₆ heteroalkyl, -S-C₁-C₆ alkyl, C₂-C₆ alkenyl, C₂-C₆ alkynyl, triazolyl, -O-C₁-C₆ alkyl, -O-C₁-C₆ heteroalkyl, -N(C₁-C₆ alkyl)₁₋₂, -N(C₁-C₆ heteroalkyl)₁₋₂, - CH₂C(=O)N(R^{5a'})₂, -C₃-C₄ alkynyl(NR^{5a'})₂, -N(R^{5a'})₂, (C₁-C₃ alkoxy)C₁-C₃ alkyl-, C₃-C₆ cycloalkyl, or 3- to 6-membered heterocycloalkyl, wherein the hydroxy, C₁-C₆ alkyl, C₁-C₆ heteroalkyl, -S-C₁-C₆ alkyl, C₂-C₆ alkenyl, C₂-C₆ alkynyl, -O-C₁-C₆ alkyl, -O-C₁-C₆ heteroalkyl, -N(C₁-C₆ alkyl)₁₋₂, -N(C₁-C₆ heteroalkyl)₁₋₂, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-(C₁-C₃ alkyl)₃;
each R^{5a'} is independently H, deuterium, or C₁-C₆ alkyl;
ring D is as defined and described in claim 1 or 2; 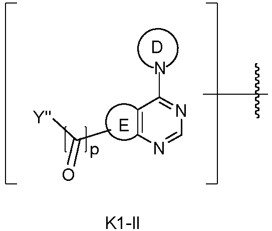
wherein in K1-II:
ring E is C₅-C₇ cycloalkyl, 5- to 7-membered heterocycloalkyl, C₆-C₁₀ aryl, 5- to 10-membered heteroaryl, C₅-C₇ cycloalkenyl, or an 8- to 10-membered spiro ring, and a fused ring is formed between ring E and a pyrimidine ring, wherein the C₅-C₇ cycloalkyl, 5- to 7-membered heterocycloalkyl, C₆-C₁₀ aryl, 5- to 10-membered heteroaryl, C₅-C₇ cycloalkenyl, or 8- to 10-membered spiro ring is optionally substituted with R^{b"}, and the heterocycloalkyl contains at least one heteroatom of N, S, or O;
Y" is 6- to 10-membered heterocycloalkyl, C₆-C₁₀ aryl, or 5- to 10-membered heteroaryl, wherein the 6- to 10-membered heterocycloalkyl or 5- to 10-membered heteroaryl contains at least one heteroatom of N, S, or O, and is optionally substituted with R^{b‴};
R^{b"} is hydrogen, oxo, halogen, deuterium, cyano, hydroxy, amino, C₁-C₆ alkyl, C₁-C₆ heteroalkyl, C₃-C₆ cycloalkyl, -S-C₁-C₃ alkyl, C₂-C₄ alkenyl, C₂-C₄ alkynyl, triazolyl, -O-C₁-C₆ alkyl, -CH₂C(=O)N(R^{x'})₂, -C₃-C₄ alkynyl(NR^{x'})₂, -N(R^{x'})₂, or C₁-C₃ alkoxy-C₁-C₃ alkyl-, wherein the C₁-C₆ alkyl, C₁-C₆ heteroalkyl, C₃-C₆ cycloalkyl, -O-C₁-C₆ alkyl, -S-C₁-C₃ alkyl, C₂-C₄ alkenyl, or C₂-C₄ alkynyl is optionally substituted with deuterium, halogen, hydroxy, cyano, amino, nitro, C₁-C₃ alkoxy, or C₁-C₃ alkyl;
R^{b‴} is hydrogen, hydroxy, halogen, cyano, C₁-C₆ alkyl, C₂-C₄ alkynyl, C₂-C₄ alkenyl, -O(C₁-C₆ alkyl), HC(=O)-, -CO₂R^{x'}, -CO₂N(R^{x'})₂, or 5- to 6-membered heteroaryl, wherein the C₁-C₆ alkyl is optionally substituted with halogen, hydroxy, cyano, or amino;
each R^{x'} is independently H or C₁-C₃ alkyl;
p is 0 or 1; and
ring D is as defined and described in claim 1 or 2; 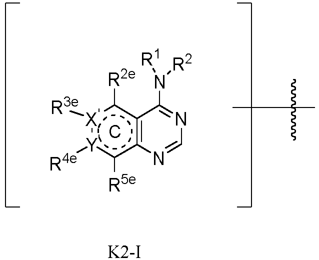
wherein in K2-I:
ring C is C₆-C₁₀ aryl, 5- to 10-membered heteroaryl, C₆-C₁₀ cycloalkyl, or 5- to 10-membered heterocycloalkyl;
X' is a bond, C, O, or N;
Y' is C or N; 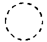
is one or more double bonds that are optionally present;
R^{2e} is H, deuterium, halogen, hydroxy, amino, C₁-C₆ alkyl, C₃-C₈ cycloalkyl, -O(C₁-C₆ alkyl), or -O(C₃-C₈ cycloalkyl);
R^{3e} is a bond, H, deuterium, halogen, cyano, oxo, -O(C₁-C₆ alkyl), -O(C₁-C₆ heteroalkyl), -O(C₃-C₈ cycloalkyl), -O(3- to 8-membered heterocycloalkyl), -S(C₁-C₆ alkyl), -S(C₃-C₈ cycloalkyl), -O-phenyl, or - O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
or R^{4e}, together with the carbon atom linked thereto, forms C₆-C₁₂ aryl or 5- to 12-membered heteroaryl, wherein the C₆-C₁₂ aryl or 5- to 12-membered heteroaryl is optionally substituted with H, deuterium, halogen, hydroxy, amino, C₁-C₆ alkyl, -S(C₁-C₆ alkyl), or -O(C₁-C₆ alkyl);
R^{5e} is hydrogen, deuterium, halogen, cyano, C₁-C₆ alkyl, C₃-C₈ cycloalkyl, -O(C₁-C₆ alkyl), or -O(C₃-C₈ cycloalkyl);
R^{a'} is halogen, cyano, hydroxy, amino, sulfhydryl, C₁-C₆ alkyl, C₁-C₆ heteroalkyl, -S-C₁-C₆ alkyl, C₂-C₆ alkenyl, C₂-C₆ alkynyl, triazolyl, -O-C₁-C₆ alkyl, -O-C₁-C₆ heteroalkyl, -N(C₁-C₆ alkyl)₁₋₂, -N(C₁-C₆ heteroalkyl)₁₋₂, -CH₂C(=O)N(R^{5a'})₂, -C₃-C₄ alkynyl(NR^{5a})₂, -N(R^{5a})₂, (C₁-C₃ alkoxy)C₁-C₃ alkyl-, C₃-C₆ cycloalkyl, or 3- to 6-membered heterocycloalkyl, wherein the hydroxy, C₁-C₆ alkyl, C₁-C₆ heteroalkyl, -S-C₁-C₆ alkyl, C₂-C₆ alkenyl, C₂-C₆ alkynyl, -O-C₁-C₆ alkyl, -O-C₁-C₆ heteroalkyl, -N(C₁-C₆ alkyl)₁₋₂, -N(C₁-C₆ heteroalkyl)₁₋₂, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-(C₁-C₃ alkyl)₃;
each R^{5a'} is independently H, deuterium, or C₁-C₆ alkyl;
R¹ and R² are as defined and described in claim 1.

4. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein K1 is K1-I-a or K1-II-a, or K2 is K2-I-a: 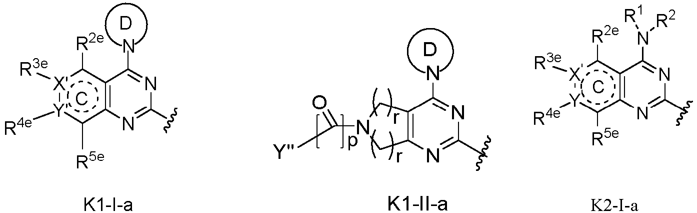 wherein:
r is 1 or 2;
wherein ring C, ring D, R^{2e}, R^{3e}, R^{4e}, R^{5e}, X', Y', R¹, R², Y", and p in K1-I-a or K1-II-a are as defined and described in claim 2.

5. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 4, wherein K1 is K1-I-a1, K1-I-a2, K1-I-a3, K1-I-a4, K1-I-a5, K1-I-a6, K1-I-a7, K1-II-a1, K1-II-a2, K1-II-a3, K1-II-a4, or K1-II-a5, and K2 is K2-I-a1: 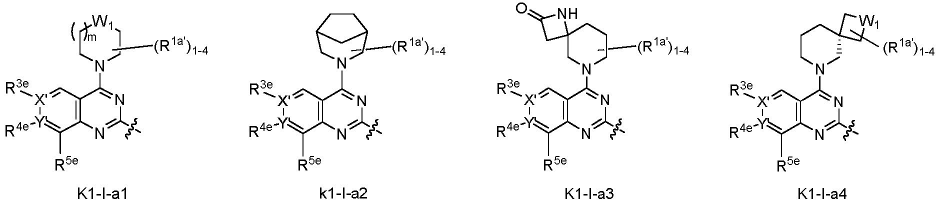 wherein in K1-I-a1, K1-I-a2, K1-I-a3, K1-I-a4, K1-I-a5, K1-I-a6, or K1-I-a7:
R^{1a'} is hydrogen, hydroxy, halogen, cyano, amino, C₁-C₆ alkyl, C₃-C₈ cycloalkyl, HC(=O)-, -CO₂R^{5a'}, - CON(R^{5a'})₁₋₂, or 5- to 6-membered heteroaryl, wherein the C₁-C₆ alkyl or C₃-C₈ cycloalkyl is optionally substituted with halogen, hydroxy, cyano, or amino;
each R^{5a'} is independently hydrogen or C₁-C₆ alkyl;
or two R^{5a'}, together with the N atom linked thereto, form 4- to 8-membered heterocycloalkyl, wherein the 4- to 8-membered heterocycloalkyl contains 1 or more N, O, or S heteroatoms or heteroatom groups;
W₁ is CH₂, O, NH, or S;
m is 0, 1, 2, or 3;
R^{3e}, R^{4e}, R^{5e}, X', and Y' are as defined and described in K1-I-a in claim 3; 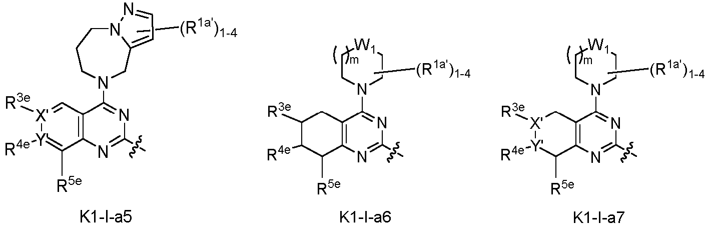 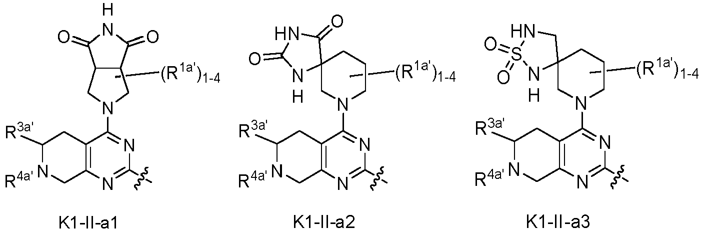 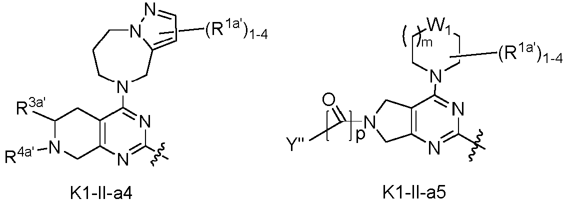
wherein in K1-II-a1, K1-II-a2, K1-II-a3, K1-II-a4, or K1-II-a5:
R^{1a'} is hydrogen, hydroxy, halogen, cyano, C₁-C₆ alkyl, C₃-C₈ cycloalkyl, HC(=O)-, -CO₂R^{5a'}, -CON(R^{5a})₁₋₂, or 5- to 6-membered heteroaryl, wherein the C₁-C₆ alkyl or C₃-C₈ cycloalkyl is optionally substituted with halogen, hydroxy, cyano, or amino;
each R^{5a'} is independently hydrogen or C₁-C₆ alkyl;
or two R^{5a'}, together with the N atom linked thereto, form 4- to 8-membered heterocycloalkyl, wherein the 4- to 8-membered heterocycloalkyl contains 1 or more N, O, or S heteroatoms or heteroatom groups;
R^{4a'} is 6- to 10-membered aryl or 5- to 10-membered heteroaryl, wherein the 6- to 10-membered aryl or 5-to 10-membered heteroaryl may be optionally substituted with one or more R^{8a'};
each R^{8a'} is independently halogen, deuterium, cyano, hydroxy, amino, C₁-C₆ alkyl, C₁-C₆ heteroalkyl, C₃-C₆ cycloalkyl, -S-C₁-C₃ alkyl, C₂-C₄ alkenyl, C₂-C₄ alkynyl, triazolyl, N(R^{5a'})₁₋₂, or -O-C₁-C₆ alkyl, wherein the C₁-C₆ alkyl, C₁-C₆ heteroalkyl, C₃-C₆ cycloalkyl, -S-C₁-C₃ alkyl, C₂-C₄ alkenyl, C₂-C₄ alkynyl, or -O-C₁-C₆ alkyl is optionally substituted with halogen, hydroxy, cyano, or amino;
R^{3a'} is hydrogen or oxo;
W₁ is CH₂, O, NH, or S;
m is 0, 1, 2, or 3;
Y" is as defined and described in claim 3; 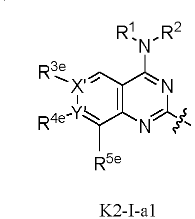
wherein in K2-I-a1:
R^{3e}, R^{4e}, R^{5e}*,* X', Y', R¹, and R² are as defined and described in claim 3.

6. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 5, wherein K1 is K1-I-a1, or K2 is K2-I-a1; 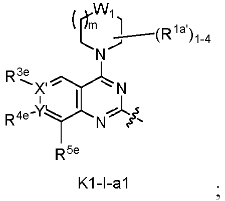
wherein in K1-I-a1, X' is C or N; Y' is C;
R^{3e} is H or absent; R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R^{1a'} is hydrogen, hydroxy, NH₂, halogen, or C₁-C₆ alkyl;
W₁ is CH₂ or O;
m is 0, 1, 2, or 3; 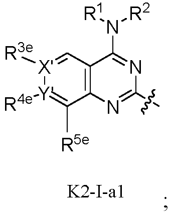
wherein in K2-I-a1, X' is C or N; Y' is C;
R^{3e} is H or absent; R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R¹ and R² are independently hydrogen, C₁-C₆ alkyl, or C₃-C₈ cycloalkyl, wherein the C₁-C₆ alkyl or C₃-C₈ cycloalkyl is optionally substituted with one or more halogen, hydroxy, or amino.

7. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein the structural unit 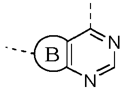 is 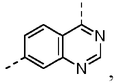 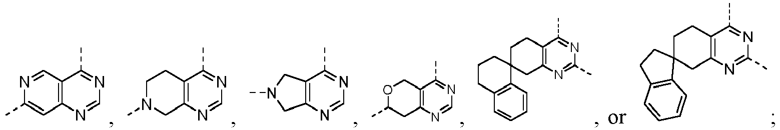 the structural unit 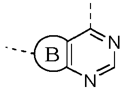 is optionally substituted with F, Cl, Br, I, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -(3- to 8-membered heterocycloalkyl), -O(C₁-C₆ alkyl), -O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂.

8. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein ring A1 is C₆-C₁₅ aryl or 5- to 15-membered heteroaryl, wherein the C₆-C₁₅ aryl or 5- to 15-membered heteroaryl is optionally substituted with -OH, F, Cl, Br, I, CN, -NH₂, -CH₃, -CH₂CH₃, -CH₂CH₂CH₃, -CH(CH₃)₂-, -OCH₃, OCH₂CH₃, -C≡CH , 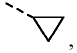 or -SCH₃, the -CH₃, -CH₂CH₃, -CH₂CH₂CH₃, -CH(CH₃)₂, 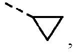 or -SCH₃ being optionally substituted with halogen, CN, -OH, -NH₂, or C₁-C₃ alkyl.

9. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein the structural unit 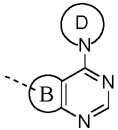 is 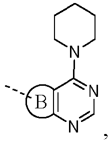 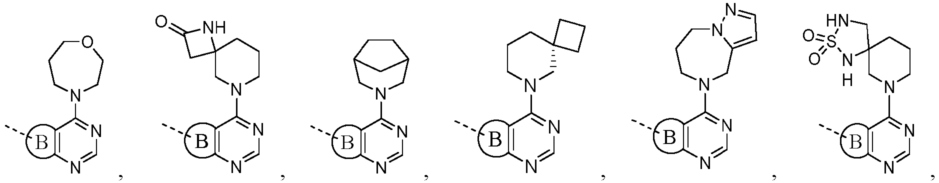 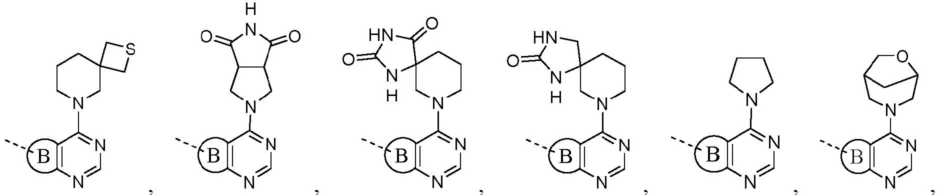 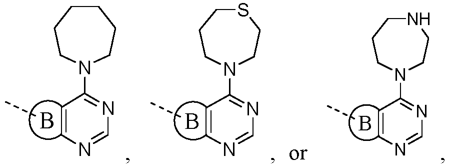 and the structural unit 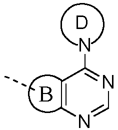 is optionally substituted with 1, 2, or 3 R^{a}.

10. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 9, wherein the structural unit 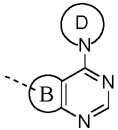 is 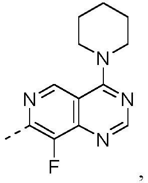 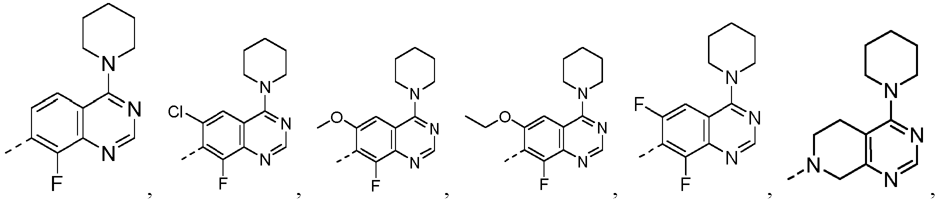 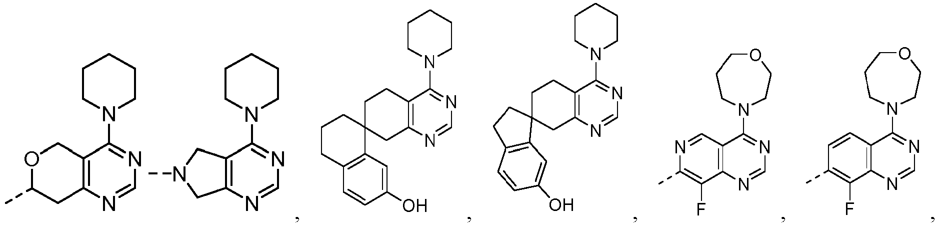 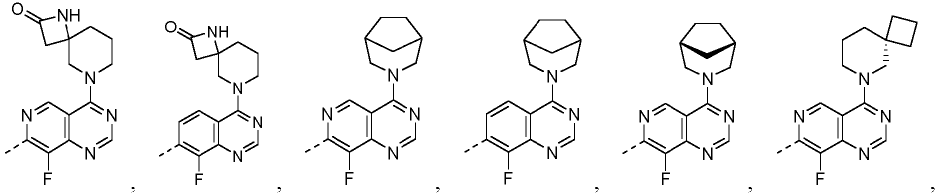 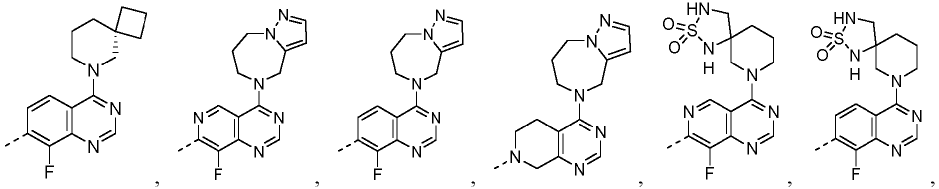 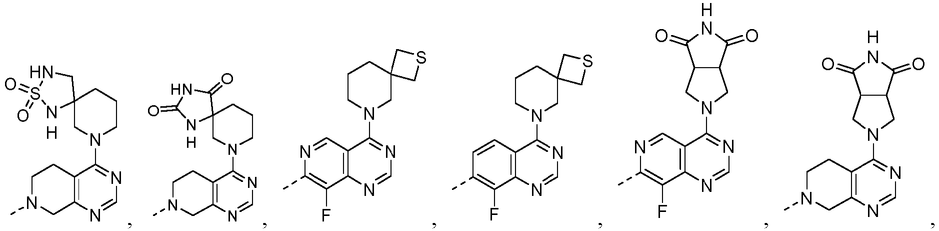 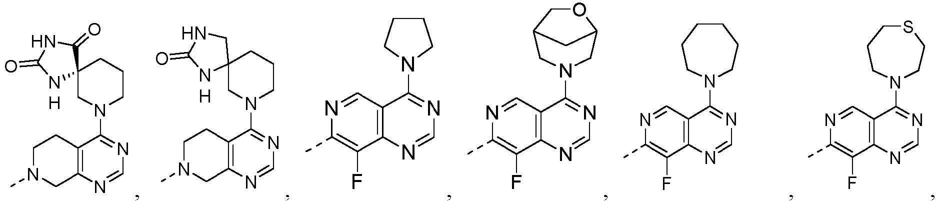 or 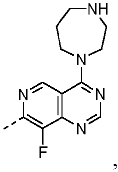 and the structural unit 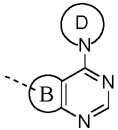 is optionally substituted with 1, 2, or 3 R^{a}.

11. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein the structural unit 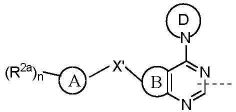 is 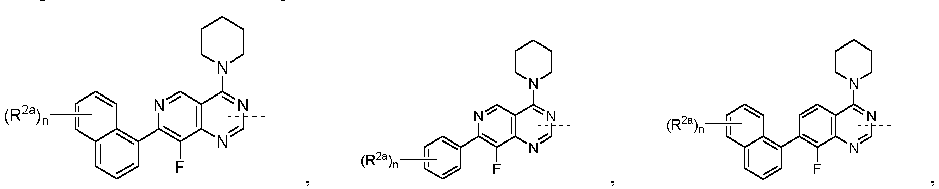 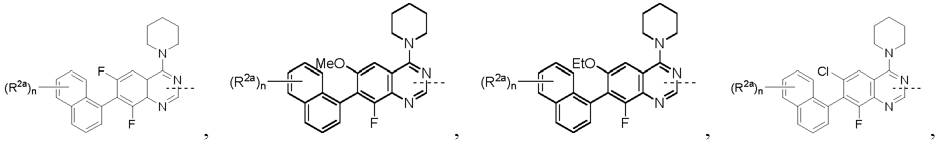 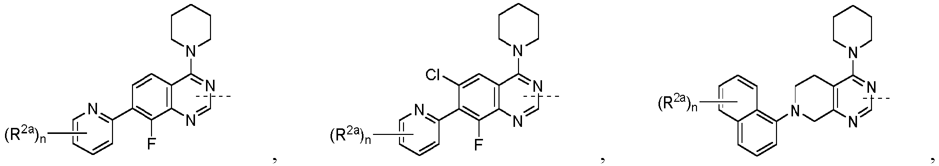 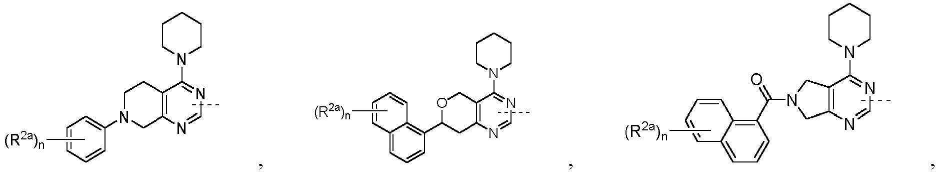 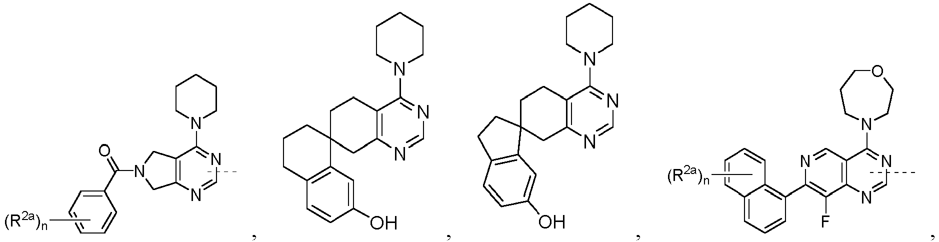 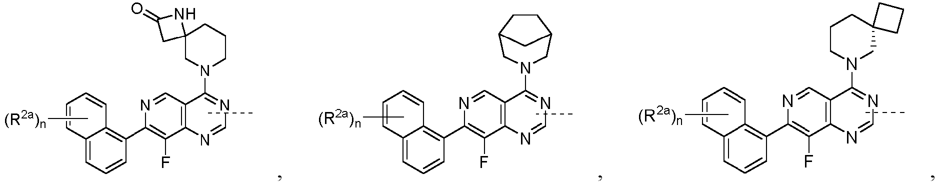 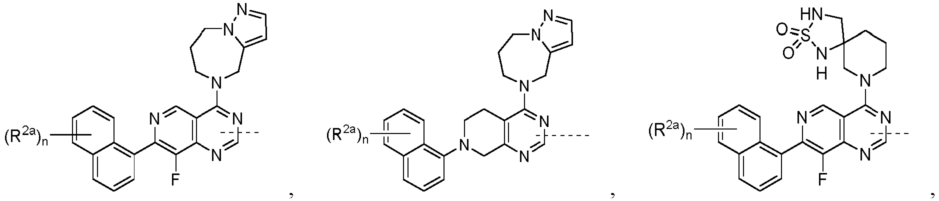 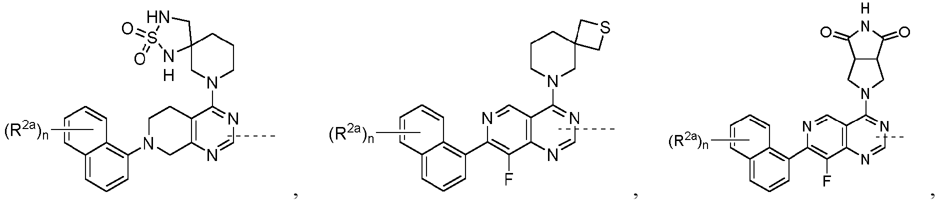 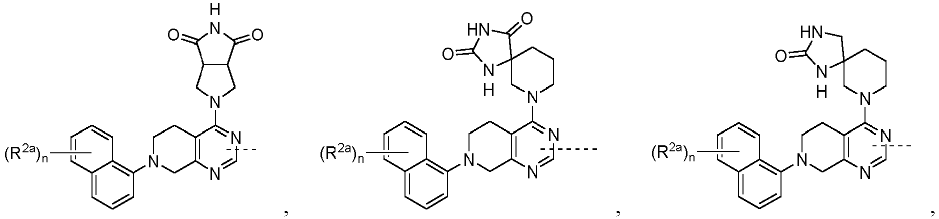 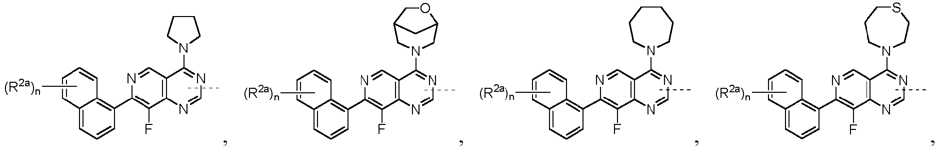 or 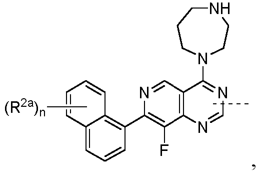 and the structural unit 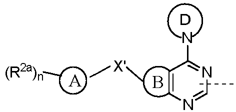 is optionally substituted with 1, 2, or 3 R^{a}.

12. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 11, wherein the structural unit 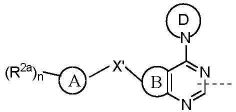 is: 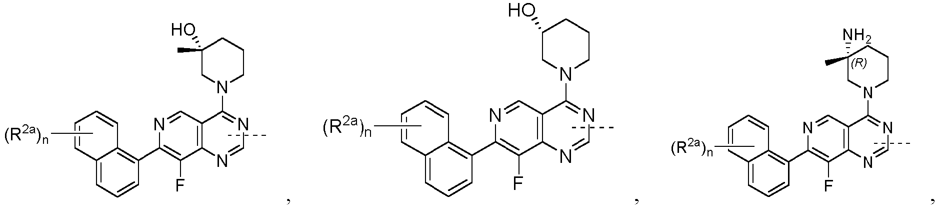 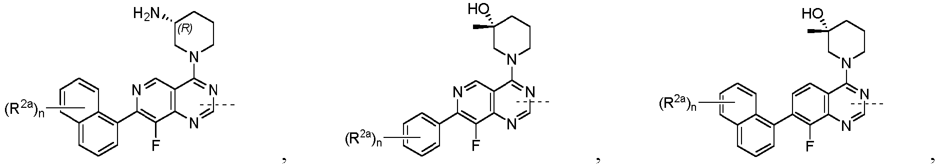 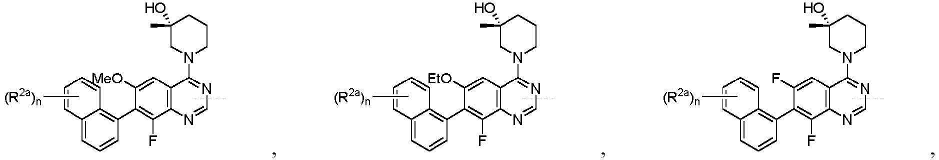 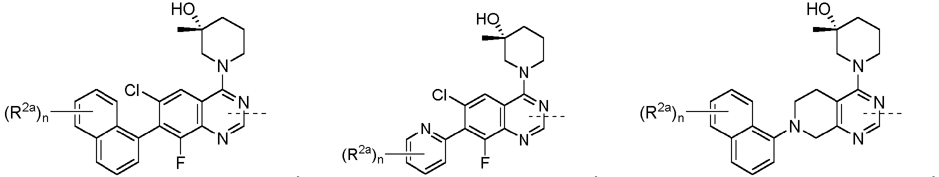 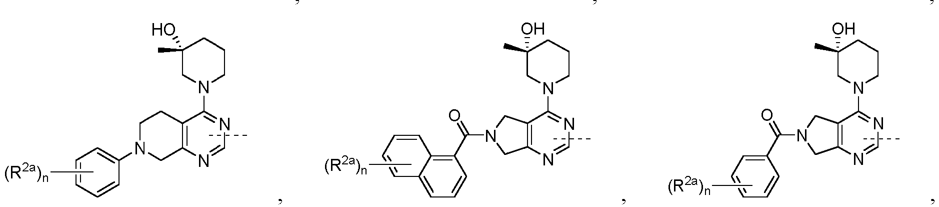 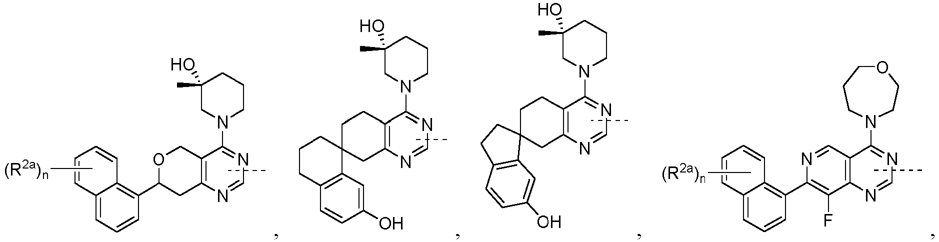 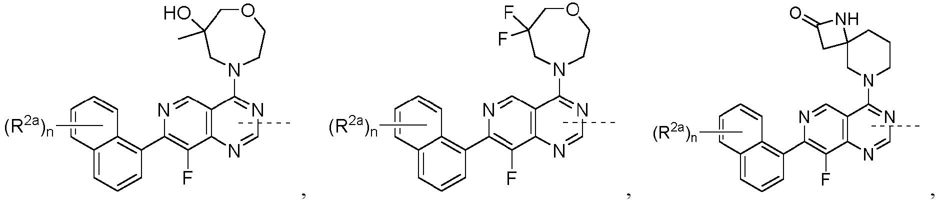 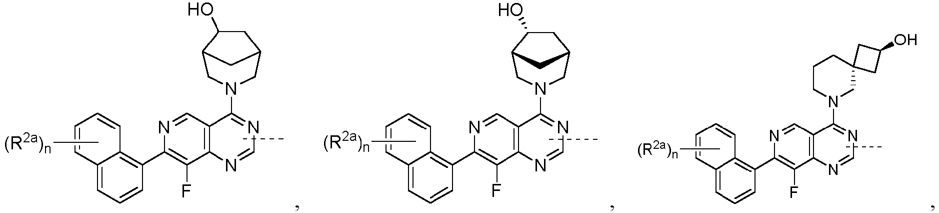 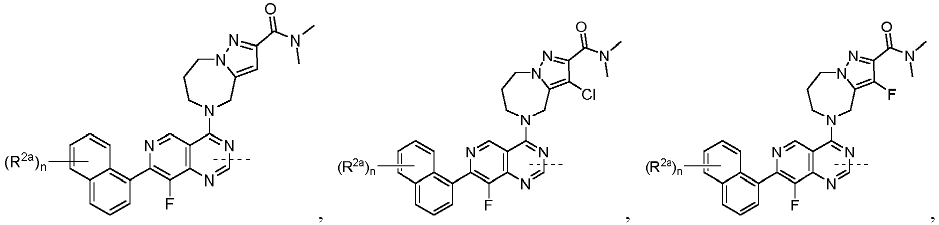 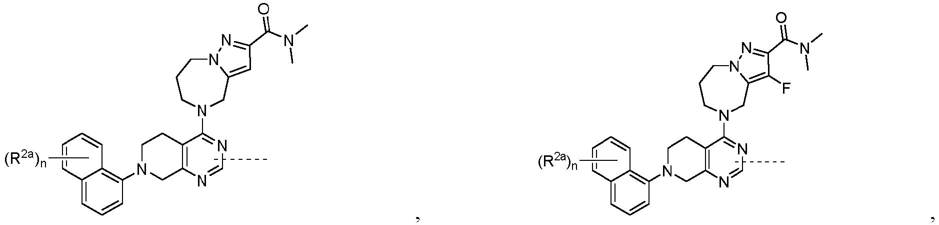 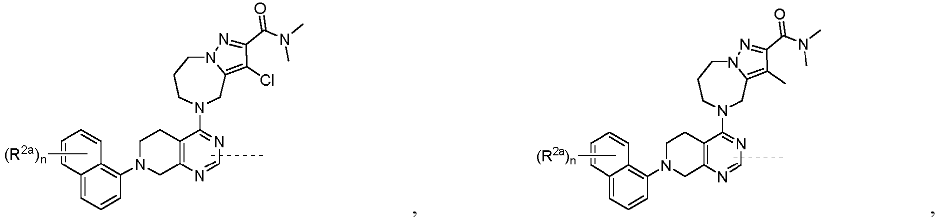 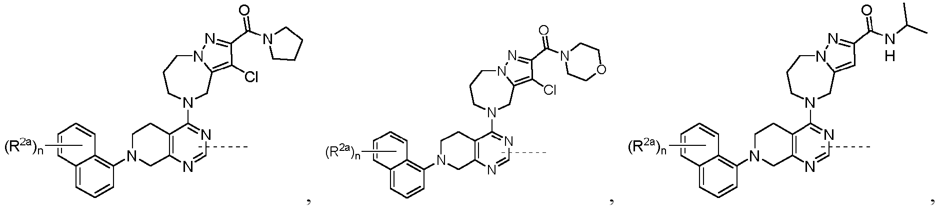 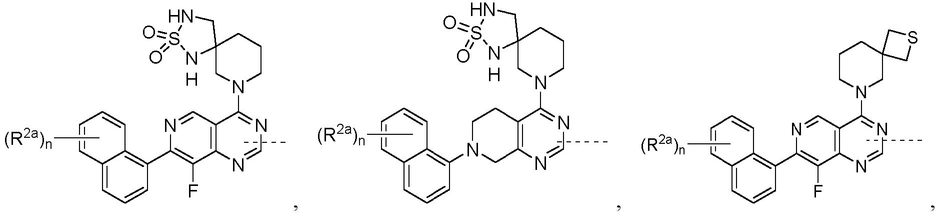 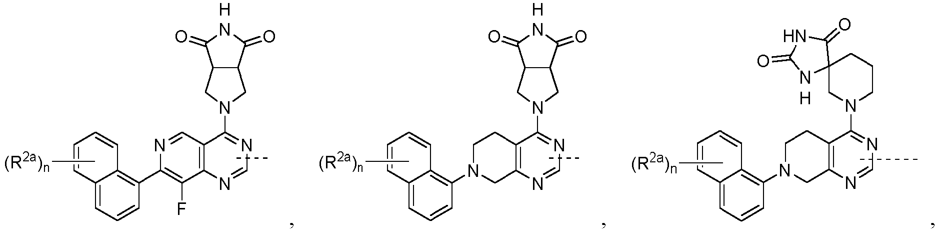 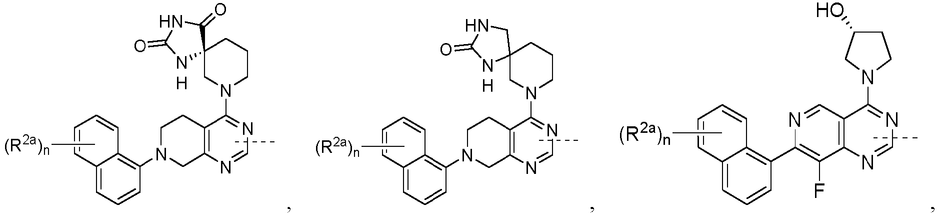 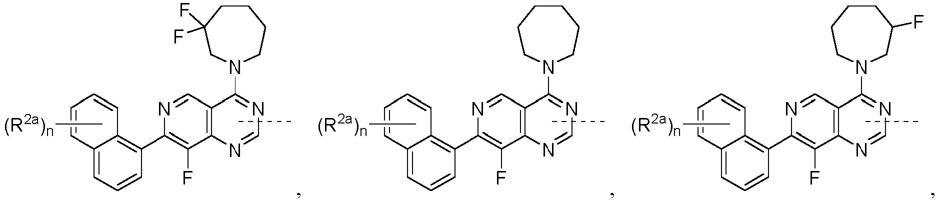 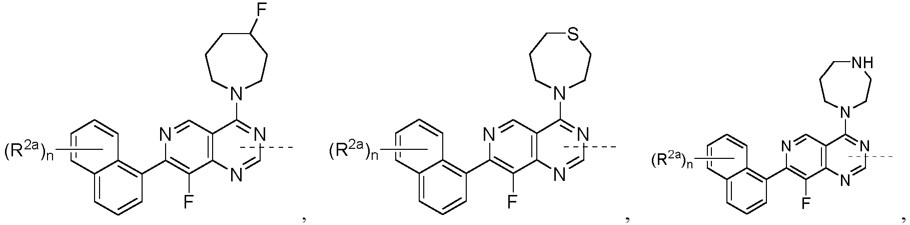 or

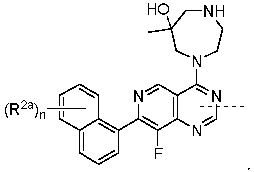

13. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein the structural unit 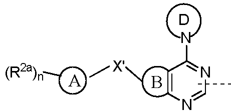 is

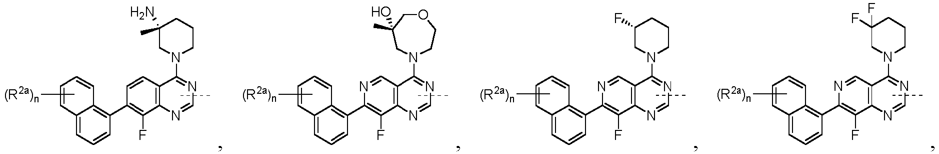

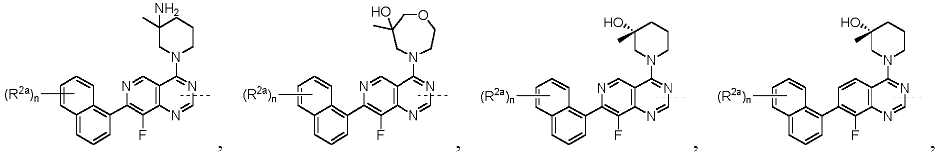

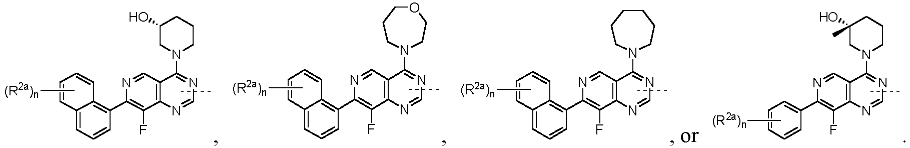

14. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 12, wherein the structural unit 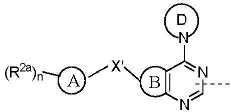 is

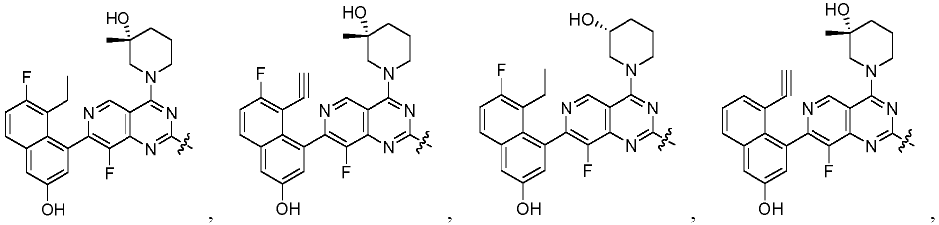

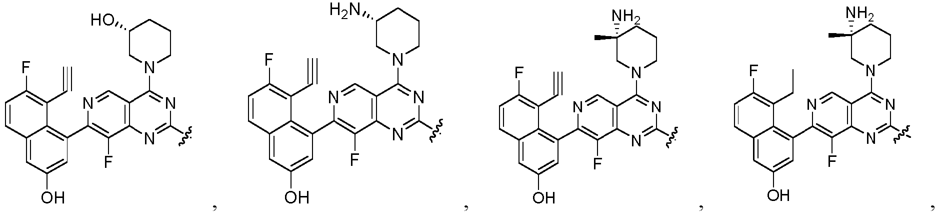

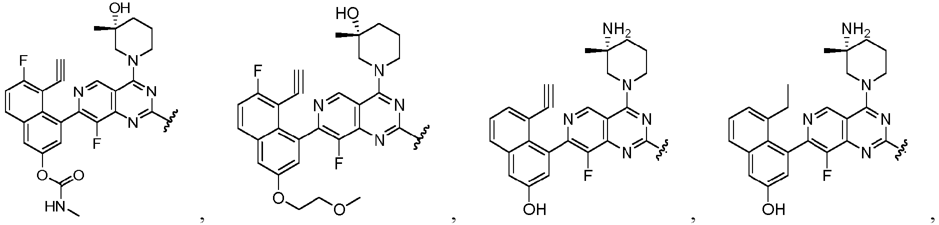

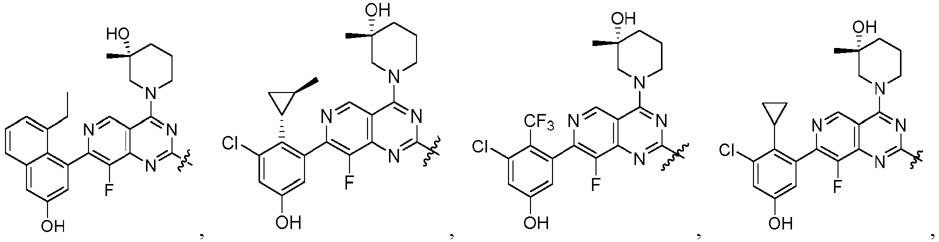

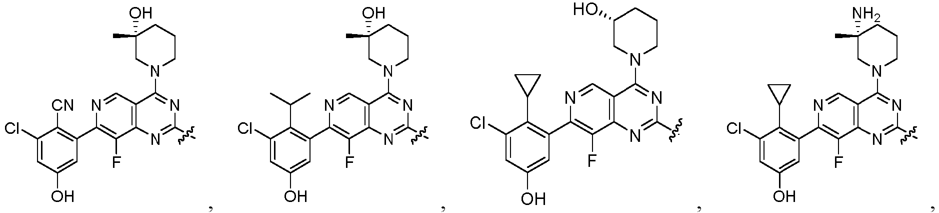

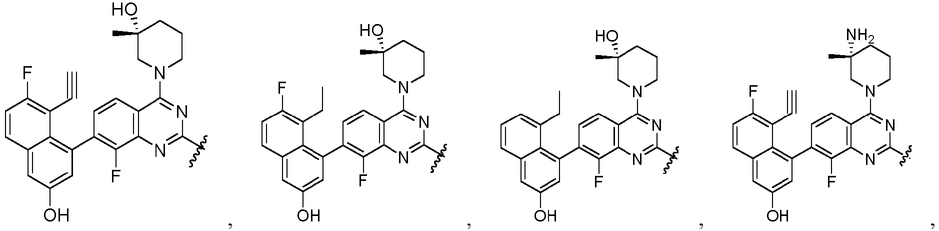

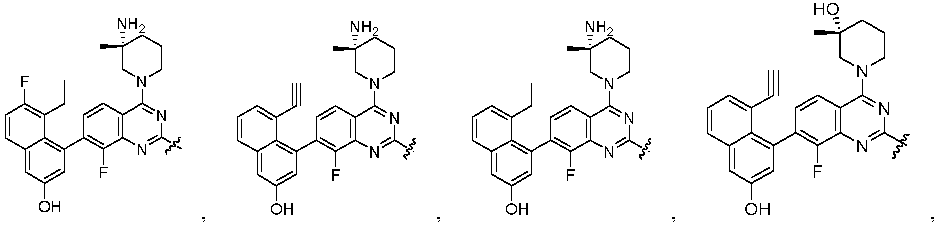

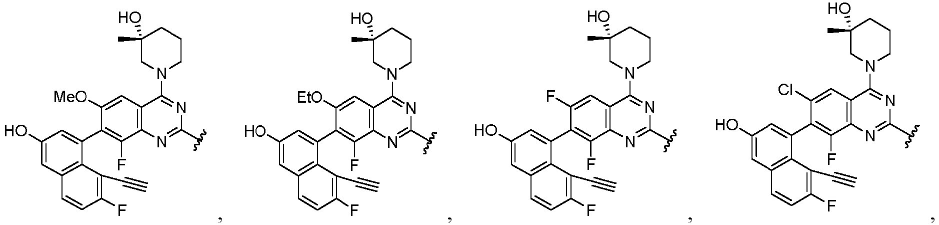

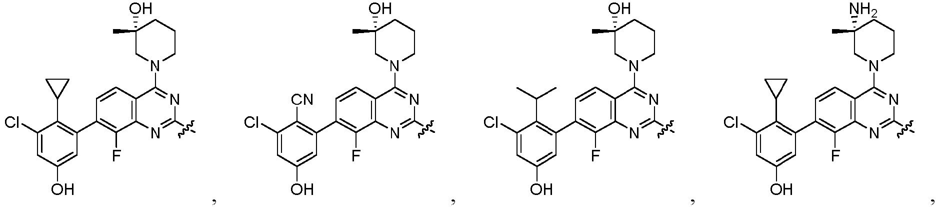

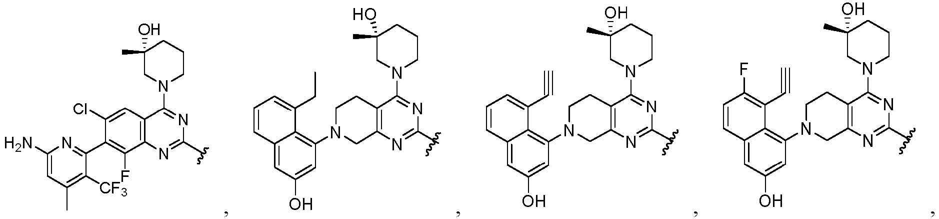

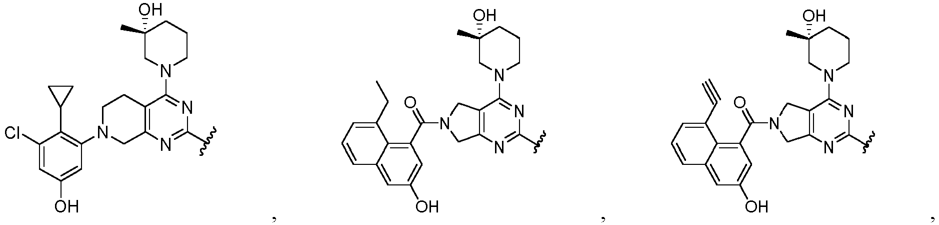

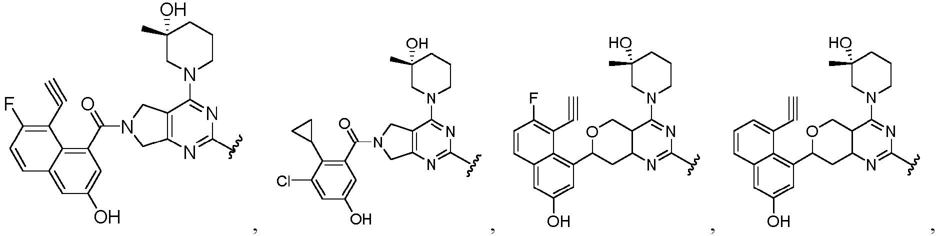

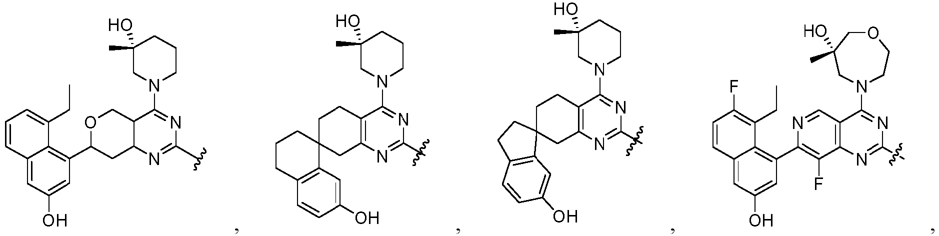

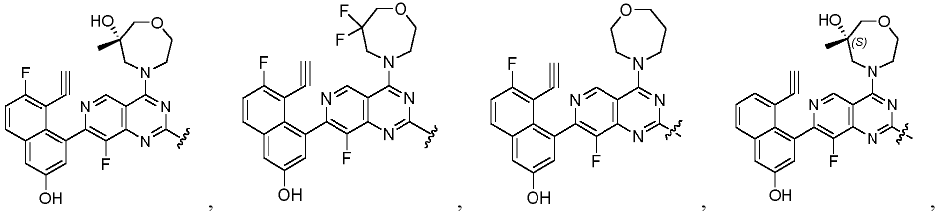

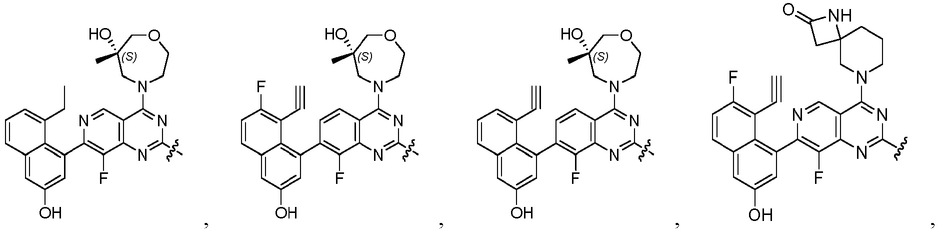

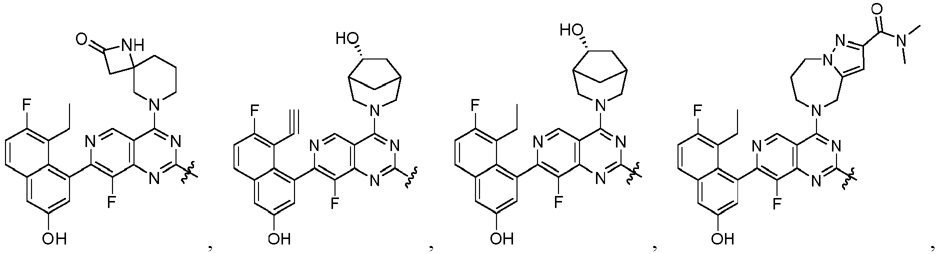

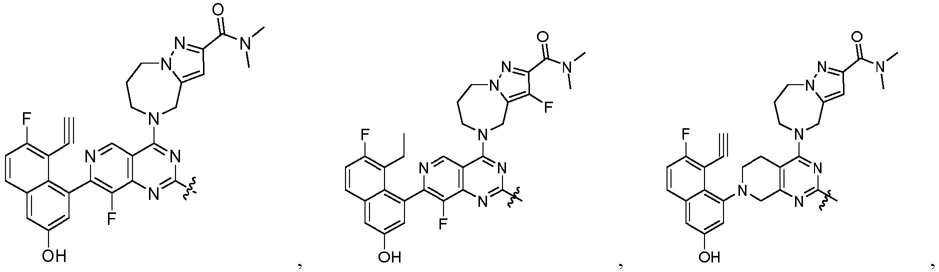

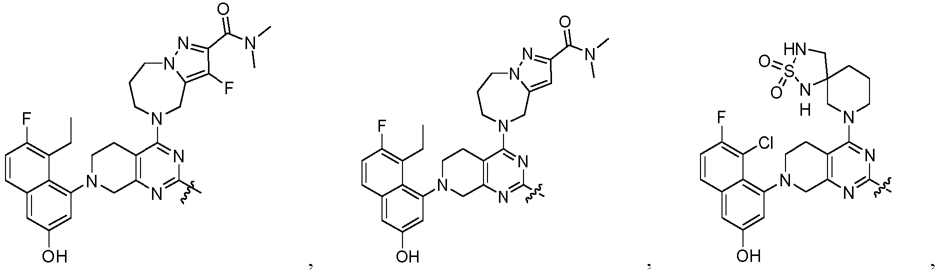

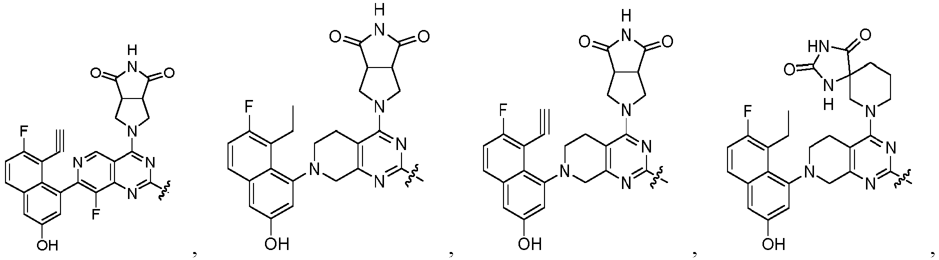

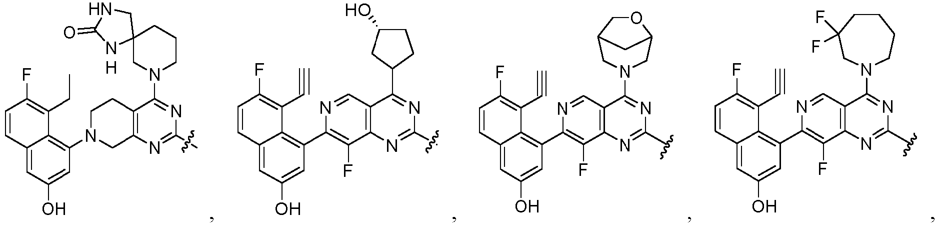

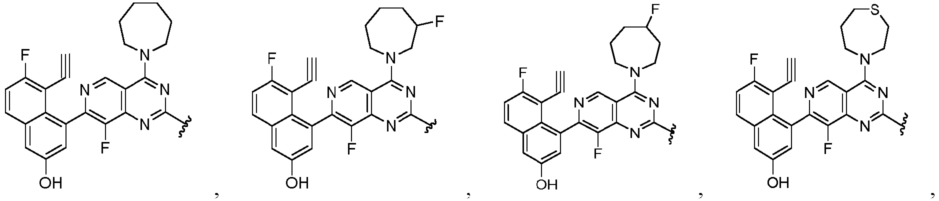

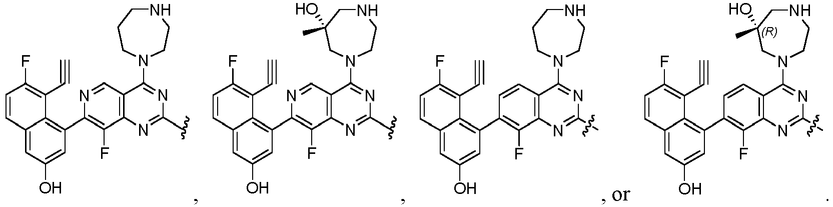

15. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein the structural unit 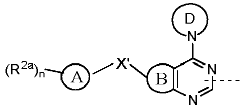 is

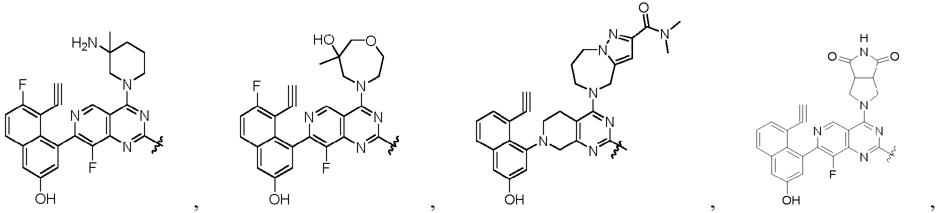

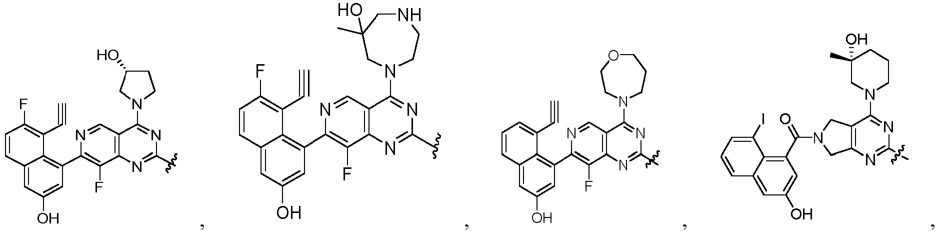

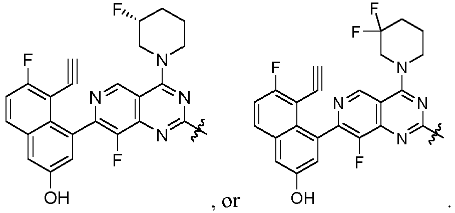

16. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein the structural unit 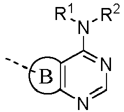 is 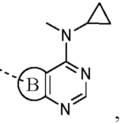 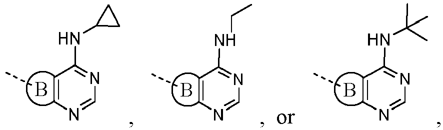 and the structural unit 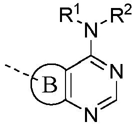 is optionally substituted with one or more deuterium, F, Cl, Br, hydroxy, amino, -CH₃, or -S-CH₃.

17. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 16, wherein the structural unit 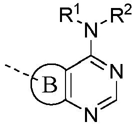 is

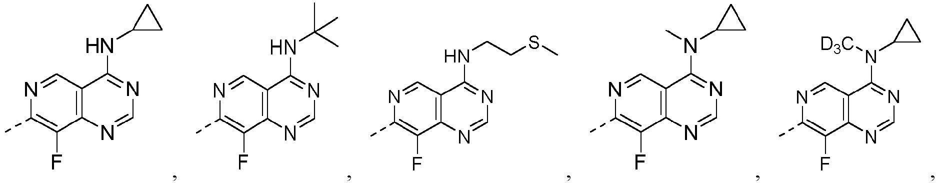

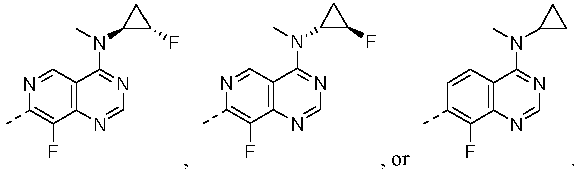

18. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein the structural unit 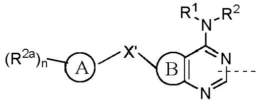 is 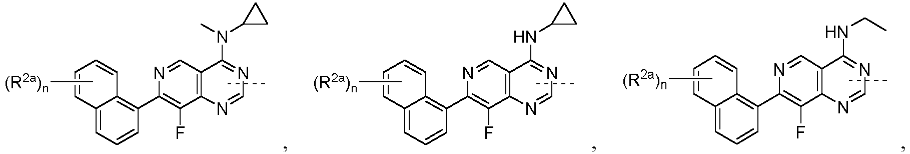 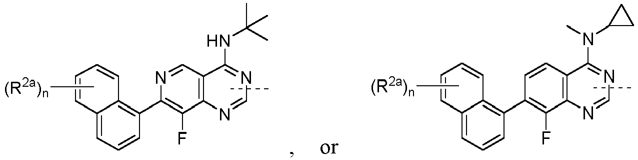 , and the structural unit 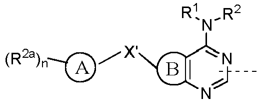 is optionally substituted with one or more deuterium, F, Cl, Br, hydroxy, amino, -CH₃, or -S-CH₃.

19. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 18, wherein the structural unit 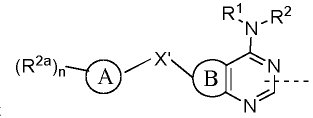 is

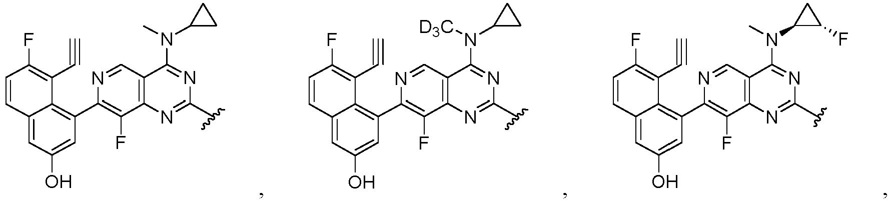

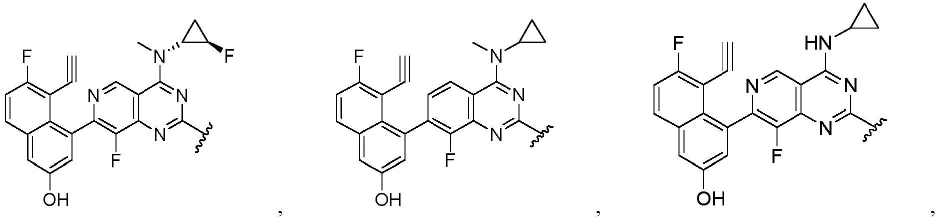

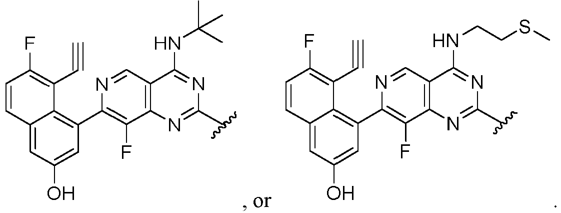

20. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to any one of claims 1-19, wherein L is -(CH₂)ⱼ-, wherein 1 or more methylene groups in the -(CH₂)ⱼ- are optionally replaced by a group of -NR^{3'}-, -O-, -CR^{1'}R^{2'}-, -C(O)-, -S(O)-, -S(O)₂-, -C(O)O-, -OC(O)-, -C(O)NR^{3'}-, -NR^{3'}C(O)-, -S(O)₂NR^{3'}-, -NR^{3'}S(O)₂-, ethenylene, ethynylene, phenyl, 8- to 10-membered bicyclic arylene, saturated or partially unsaturated 3- to 7-membered cycloalkylene, saturated or partially unsaturated 5- to 11-membered spirocycloalkylene, saturated or partially unsaturated 5- to 11-membered fused cycloalkylene, saturated or partially unsaturated 8- to 10-membered bicyclic cycloalkylene, saturated or partially unsaturated 4- to 7-membered heterocycloalkylene having 1-2 heteroatoms independently being nitrogen, oxygen, and sulfur, saturated or partially unsaturated 5- to 11-membered spiroheterocycloalkylene having 1-2 heteroatoms independently being nitrogen, oxygen, and sulfur, saturated or partially unsaturated 5- to 11-membered fused heterocycloalkylene having 1-2 heteroatoms independently being nitrogen, oxygen, and sulfur, saturated or partially unsaturated 8- to 10-membered bicyclic heterocycloalkylene having 1-2 heteroatoms independently being nitrogen, oxygen, and sulfur, 5- to 6-membered heteroarylene having 1-4 heteroatoms independently being nitrogen, oxygen, and sulfur, and 8- to 10-membered bicyclic heteroaryl having 1-5 heteroatoms being nitrogen, oxygen, and sulfur, wherein the ethenylene, ethynylene, cycloalkylene, heterocycloalkylene, phenyl, spiroheterocycloalkylene, fused heterocycloalkylene, spirocycloalkylene, fused cycloalkylene, and heteroarylene are each independently optionally substituted with 1 or more substituents being halogen, oxo, -NR^{3'}R^{4'}, -OR^{3'}, nitro, -CN, C₁-C₆ alkyl, C₃-C₁₀ cycloalkyl, and C₃-C₁₀ heterocycloalkyl, the alkyl, cycloalkyl, or heterocycloalkyl being optionally substituted with 1 or more substituents being halogen, -OH, -NH₂, -CN, C₁-C₄ alkyl, and C₃-C₆ cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl), -NH(C₁-C₄ alkyl), -NH(C₁-C₄ alkyl), C₃-C₆ cycloalkyl, -O(C₃-C₆ cycloalkyl), -NH(C₃-C₆ cycloalkyl), C₃-C₆ heterocycloalkyl, -O(C₃-C₆ heterocycloalkyl), or -NH(C₃-C₆ cycloalkyl); R^{3'} and R^{4'} are each independently hydrogen, deuterium, C₁-C₄ alkyl, C₃-C₆ cycloalkyl, or C₃-C₆ heterocycloalkyl; j is 2, 3, 4, 5, 6, 7, 8, 9, 10, 11, or 12.

21. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 20, wherein L is: 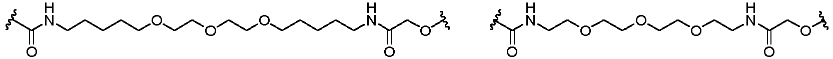 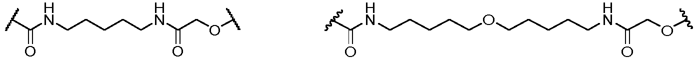 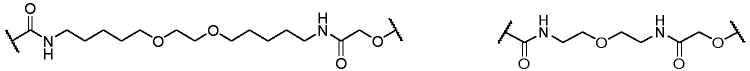 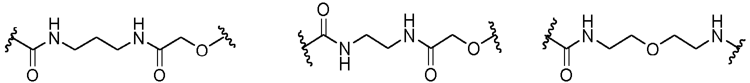 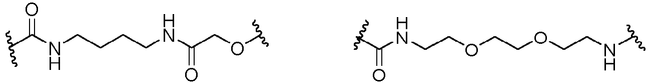 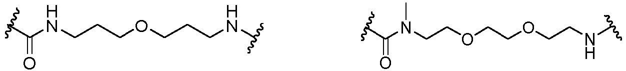 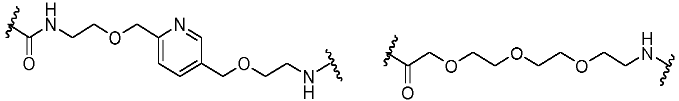 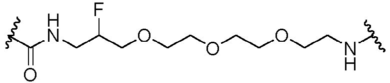 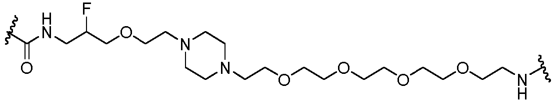 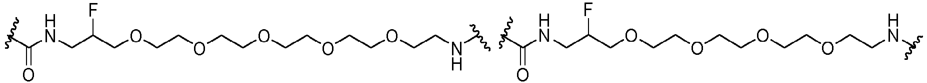 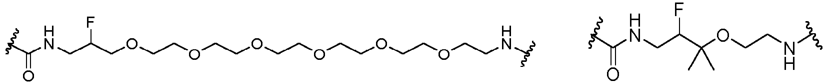 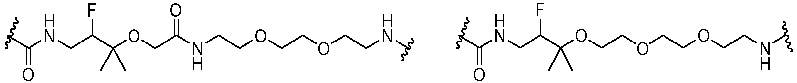 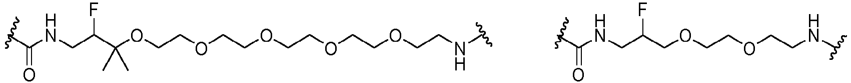 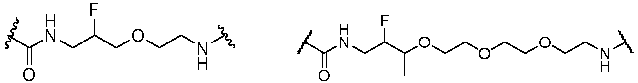 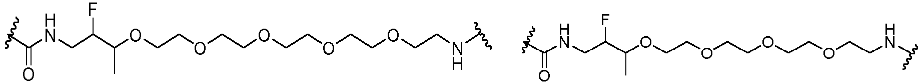 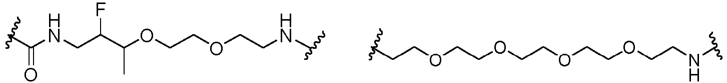 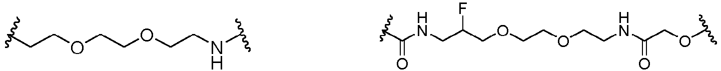 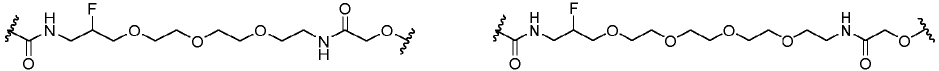 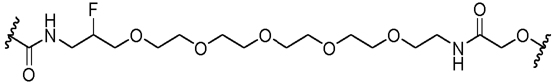 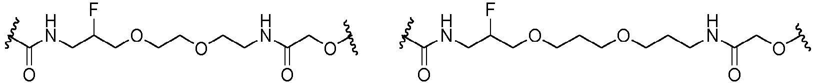 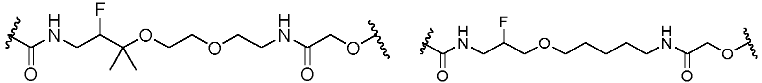 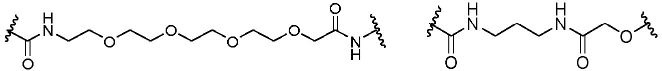 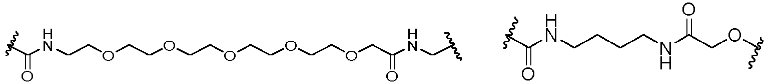 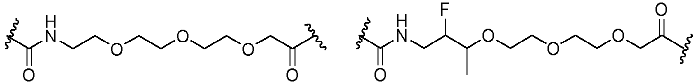 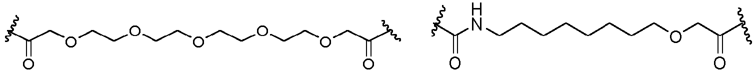 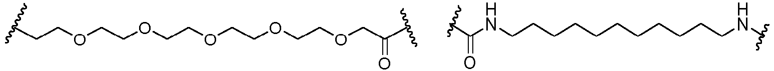 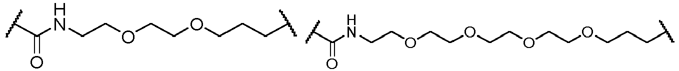 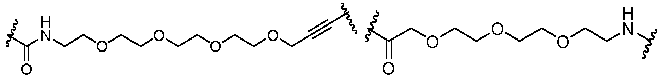 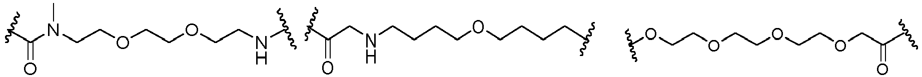 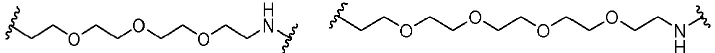 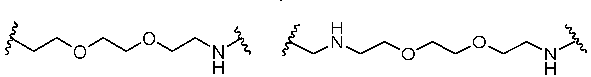 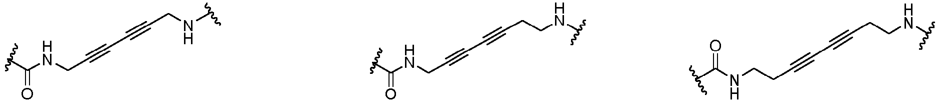 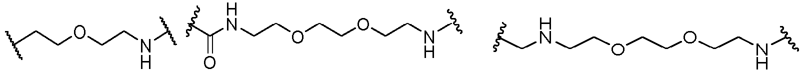 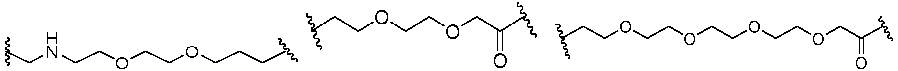 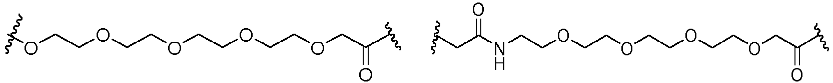 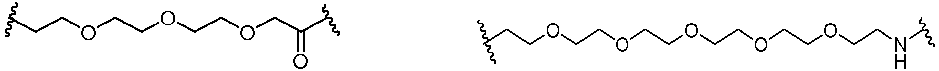 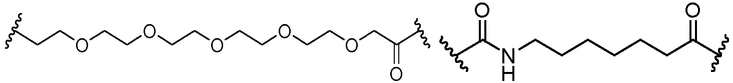 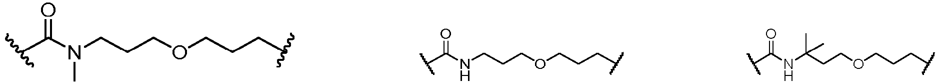 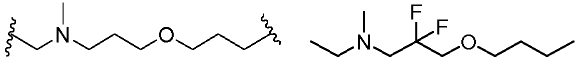 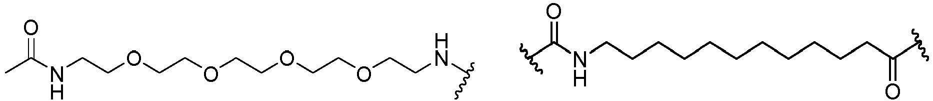 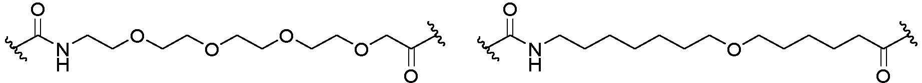 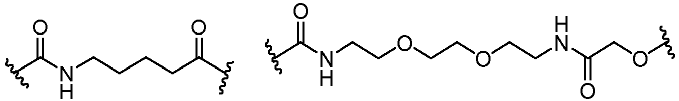 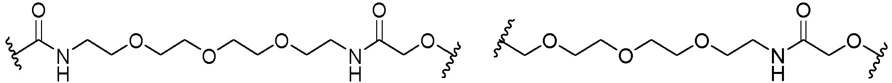 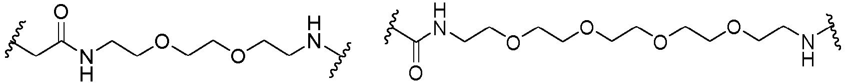 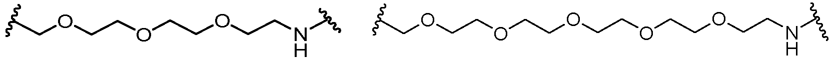 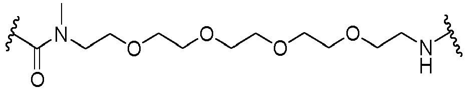 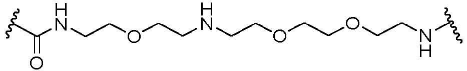 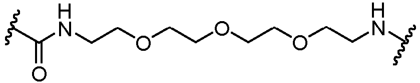 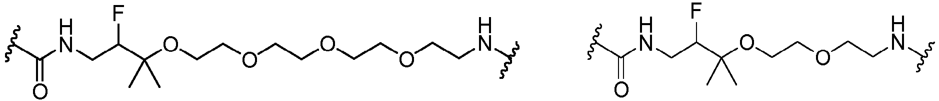 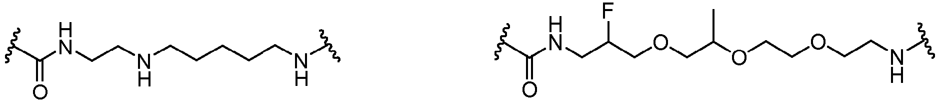 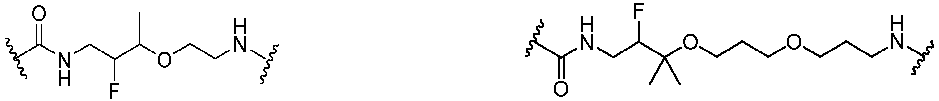 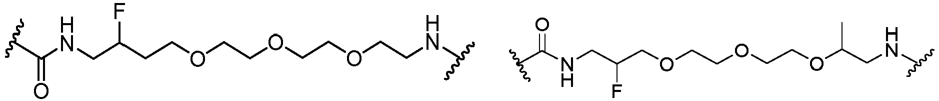 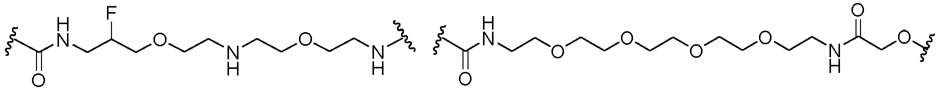 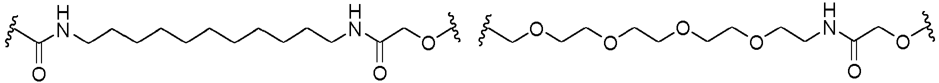 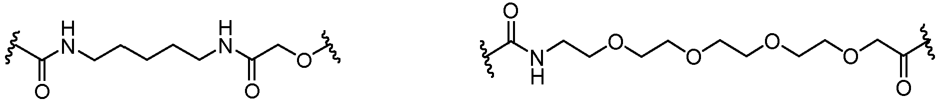 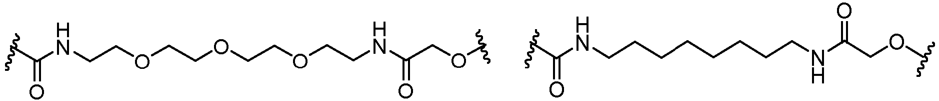 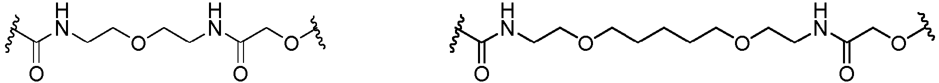 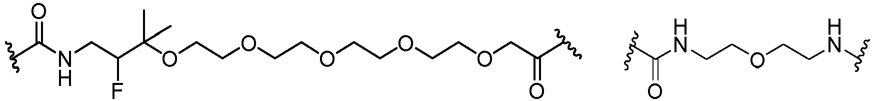 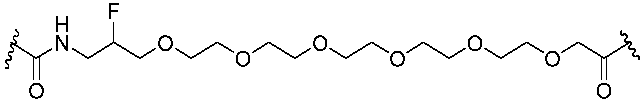 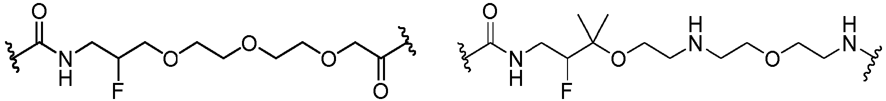 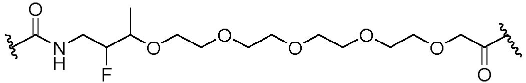 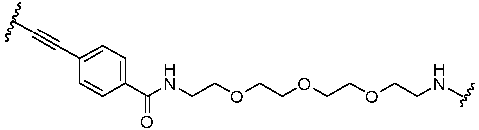 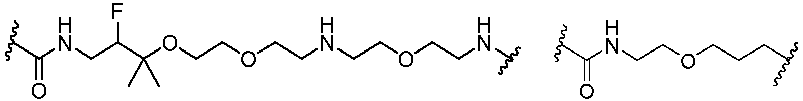 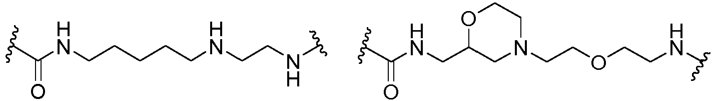 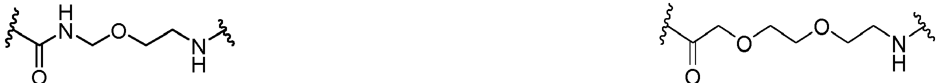 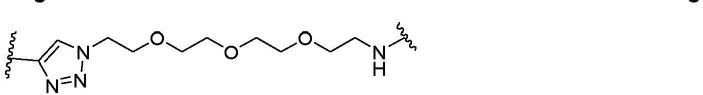 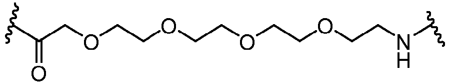 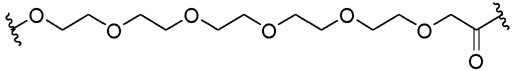 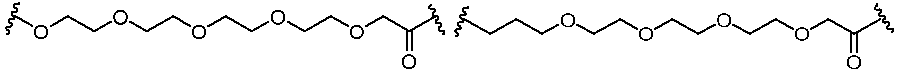 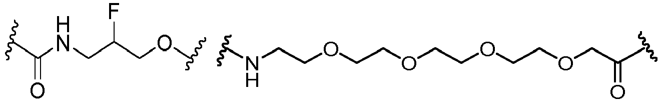 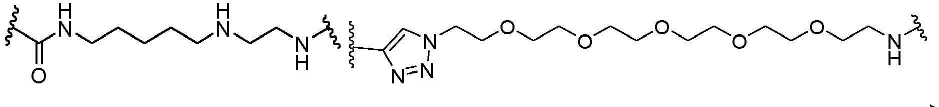 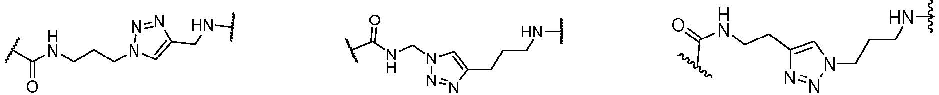 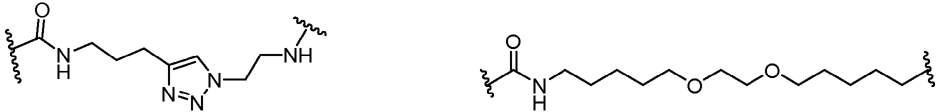 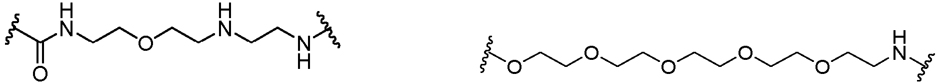 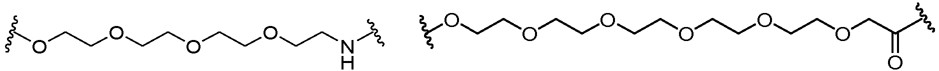 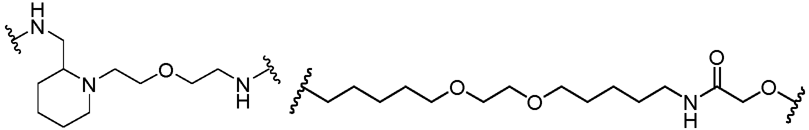 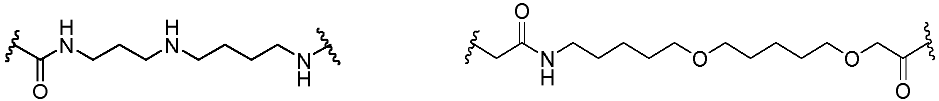 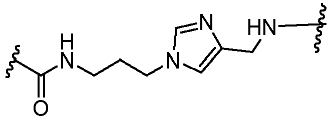 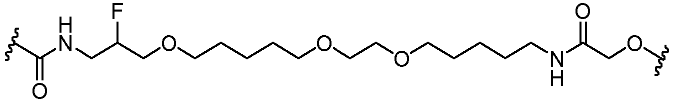 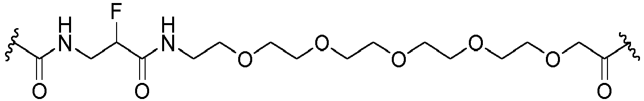 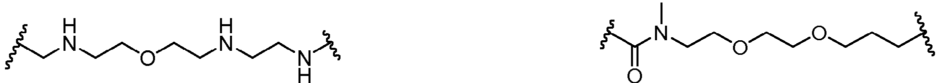 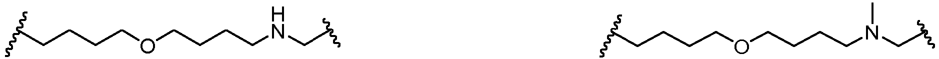 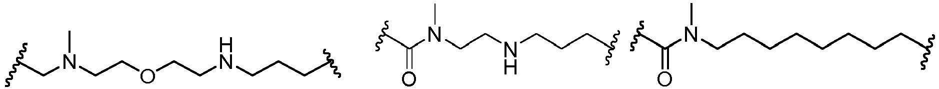 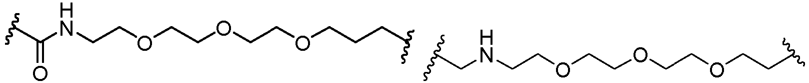 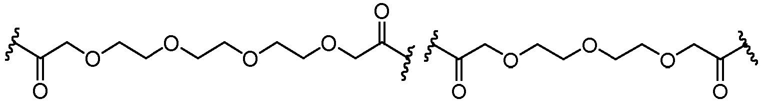 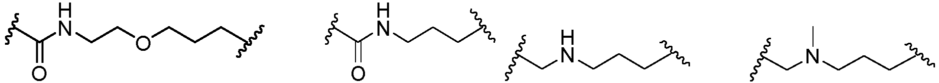 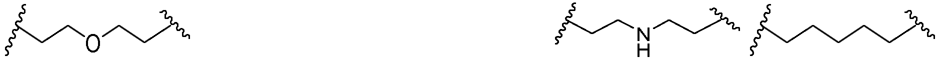 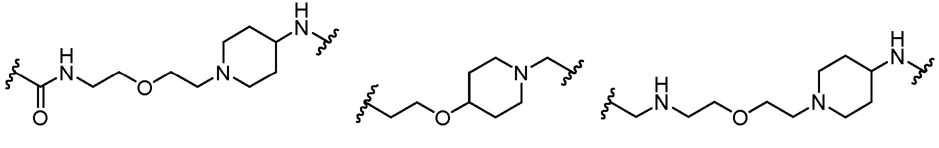 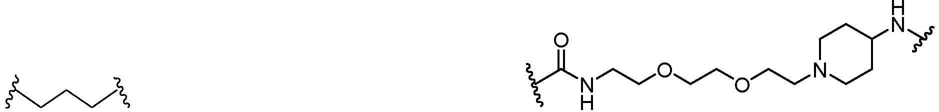 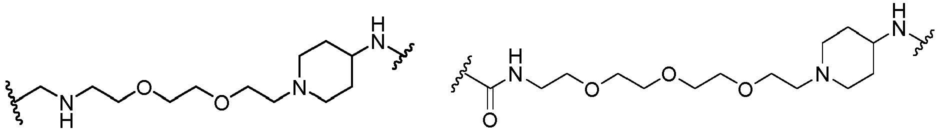 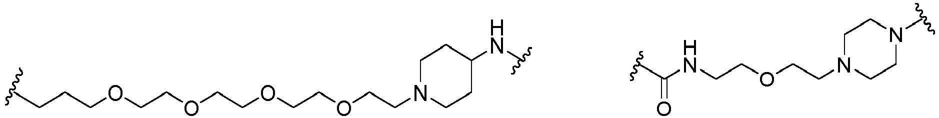 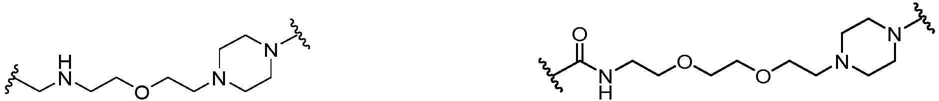 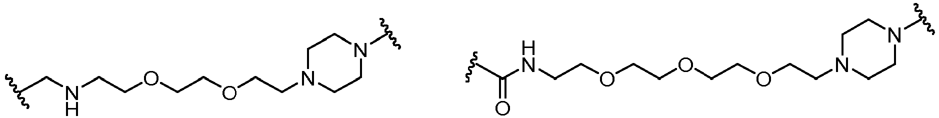 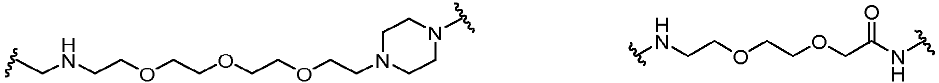 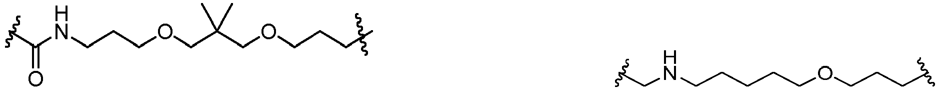 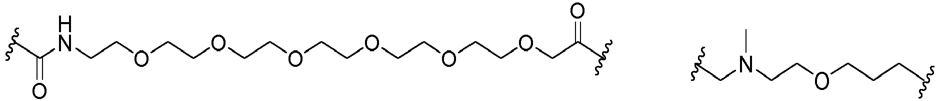 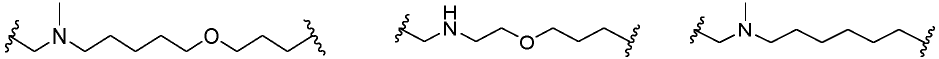 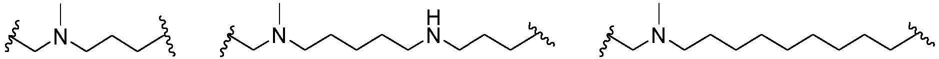 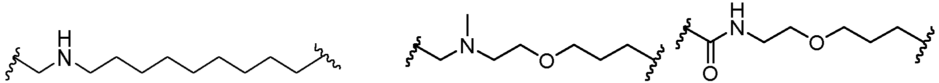 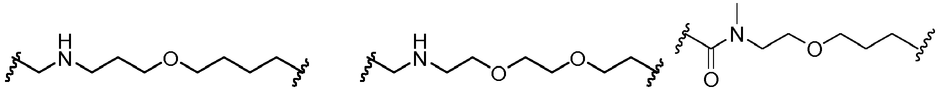 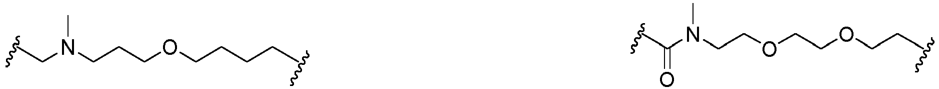 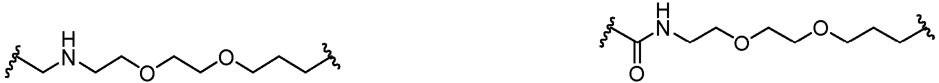 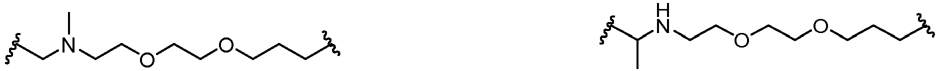 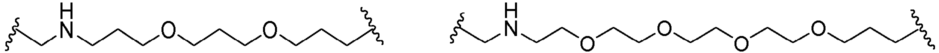 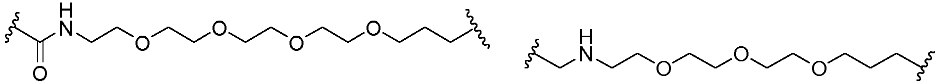 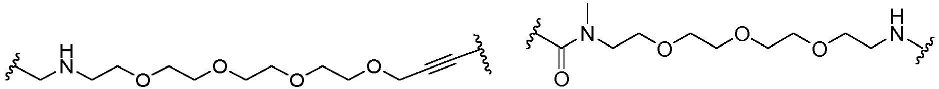 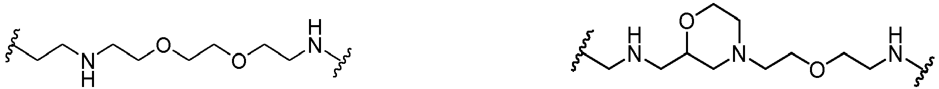 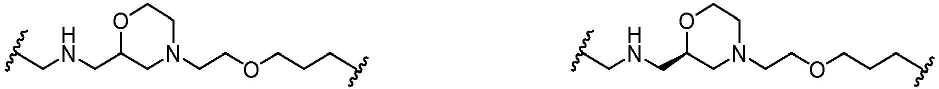 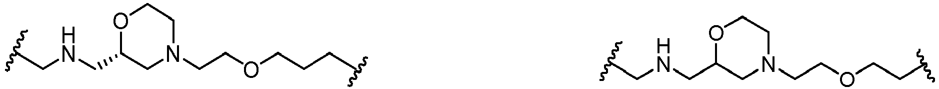 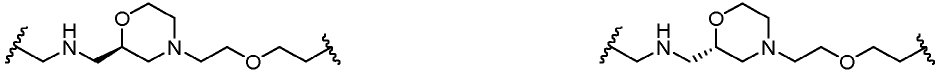 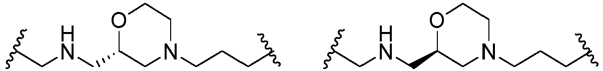 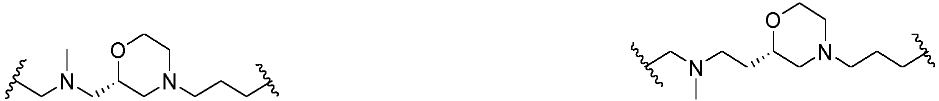 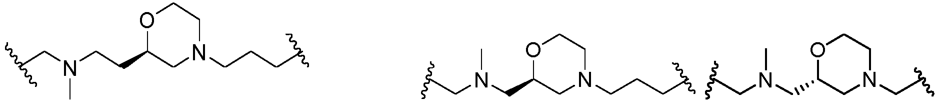 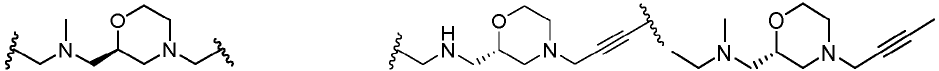 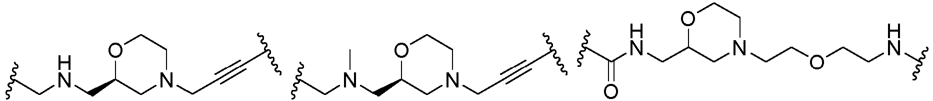 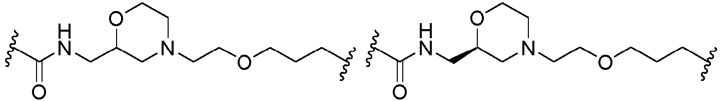 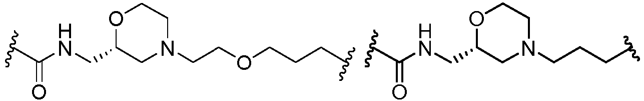 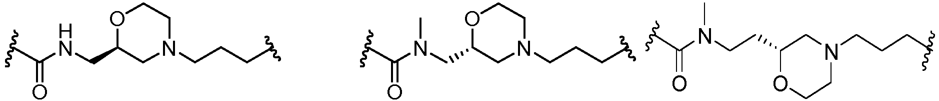 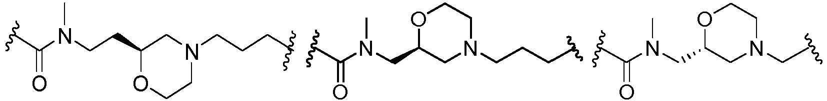 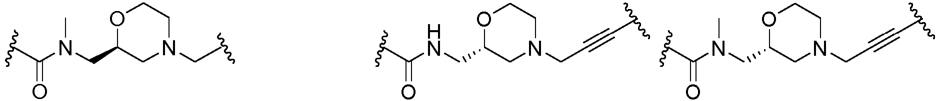 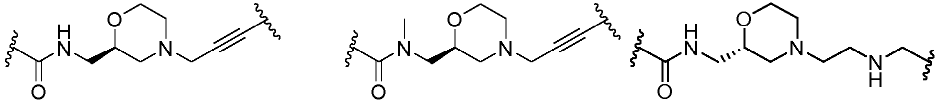 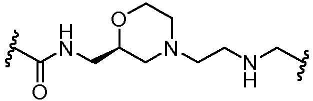 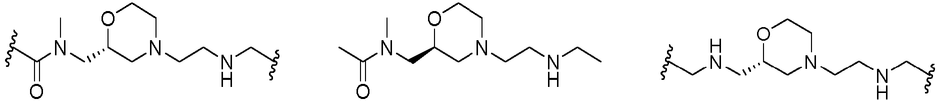 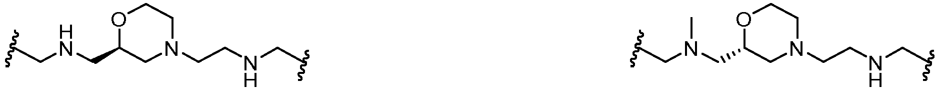 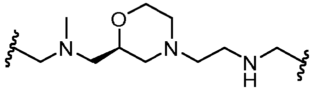 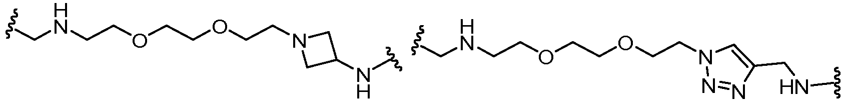 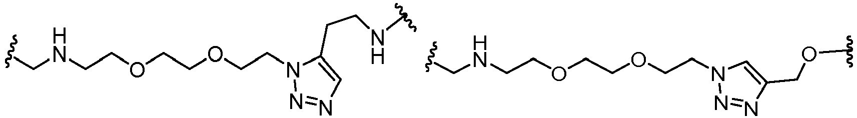 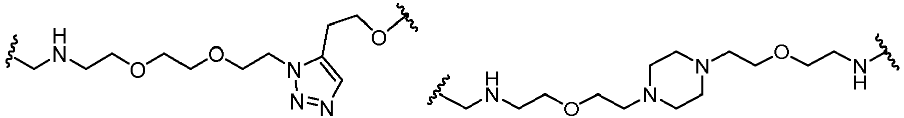 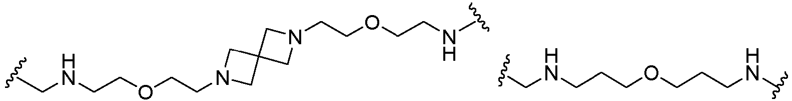 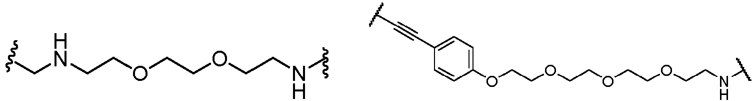 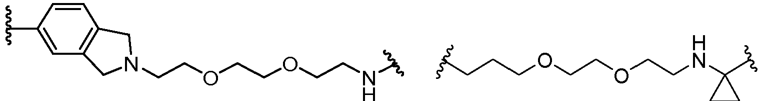 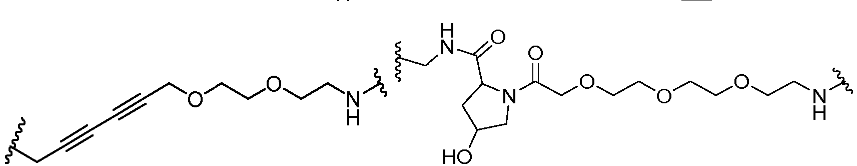 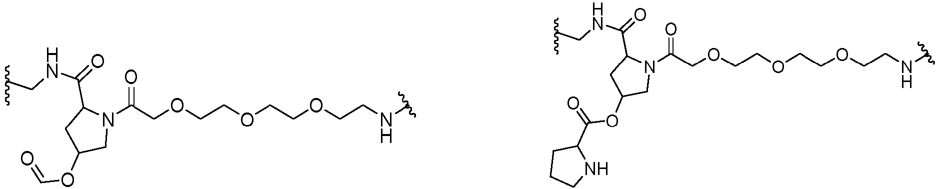 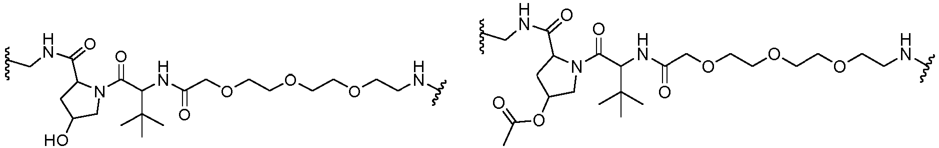 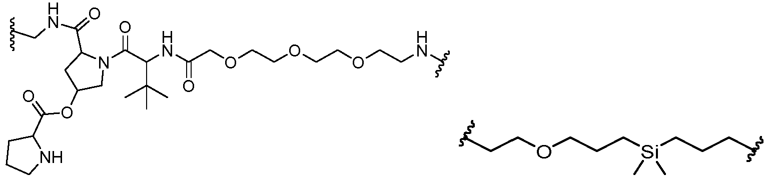 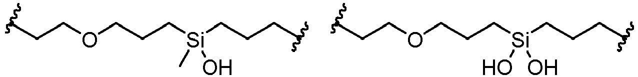 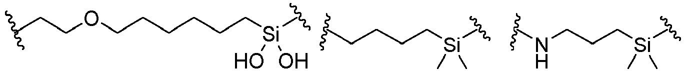 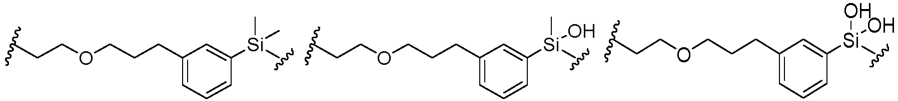 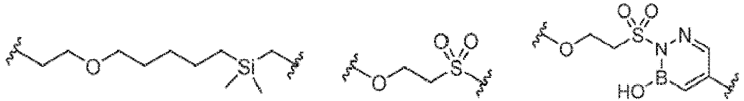 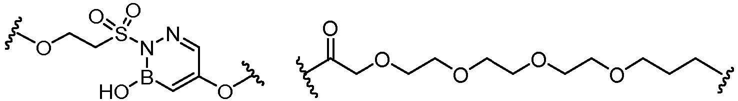 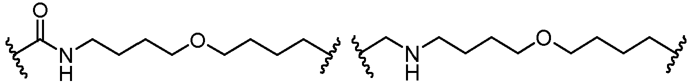 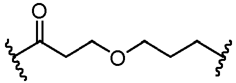 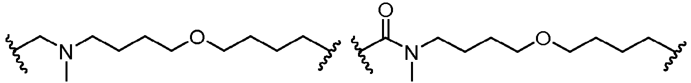 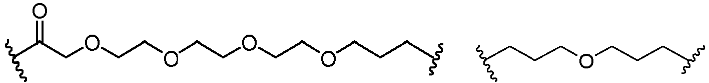 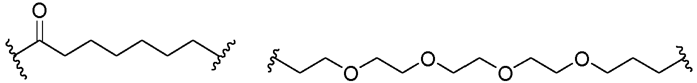 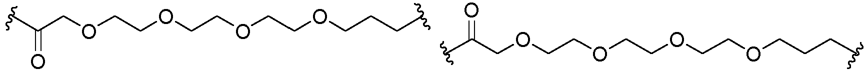 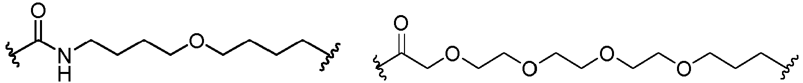 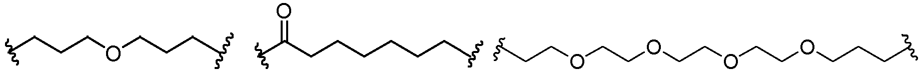 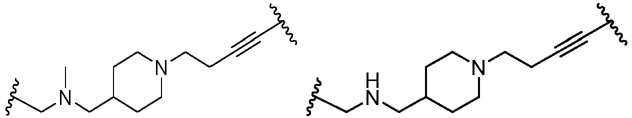 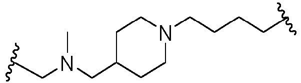 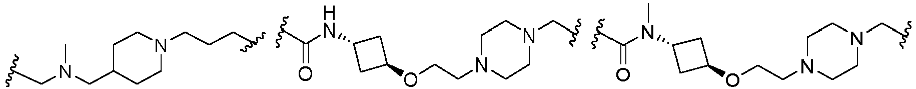 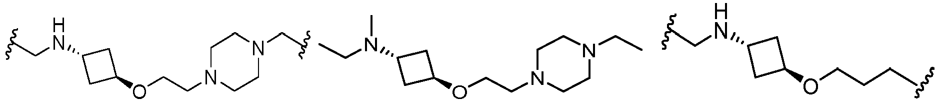 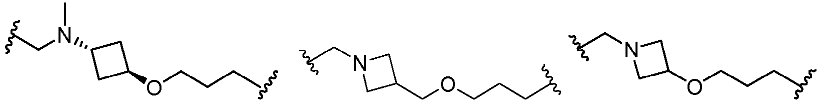 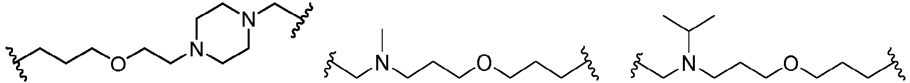 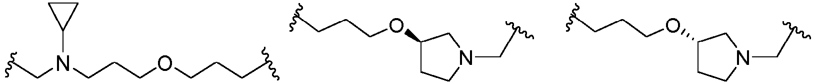 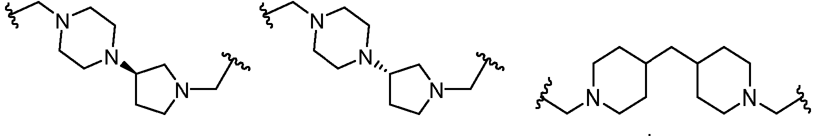 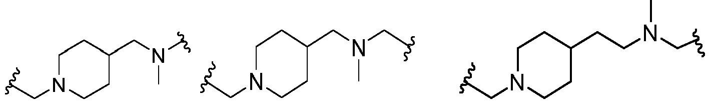 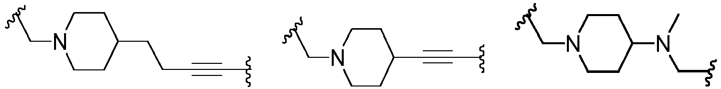 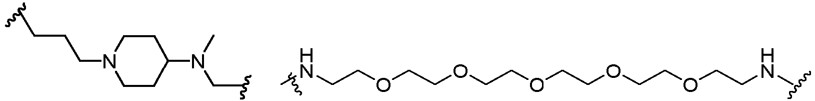 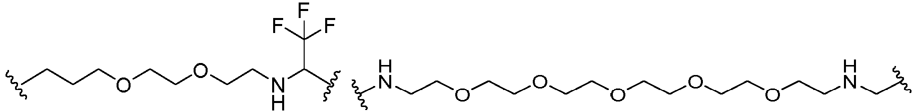 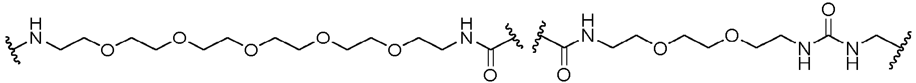 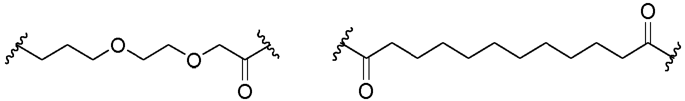 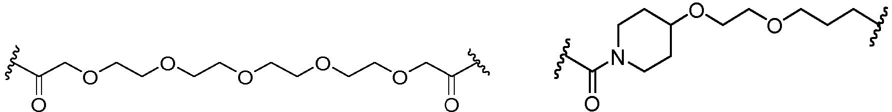 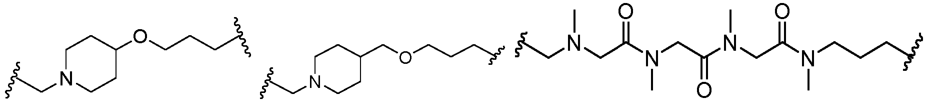 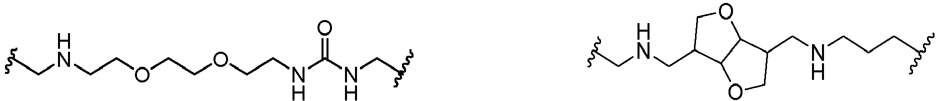 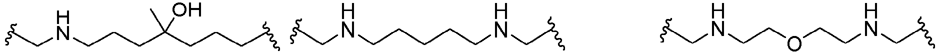 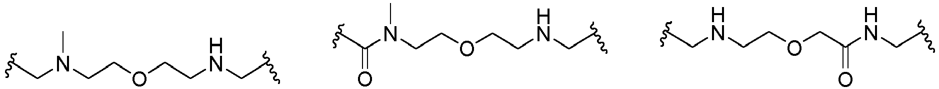 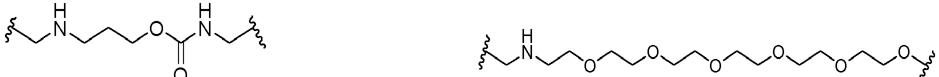 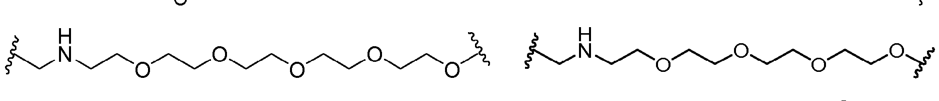 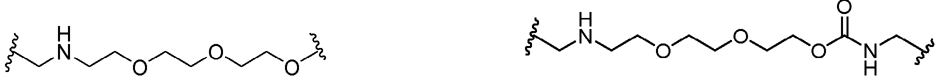 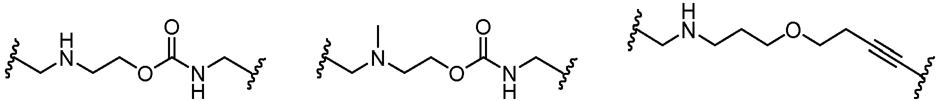 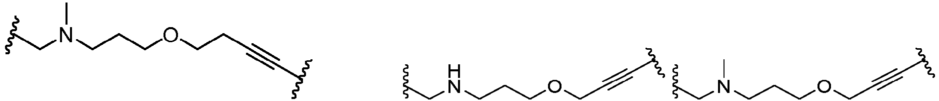 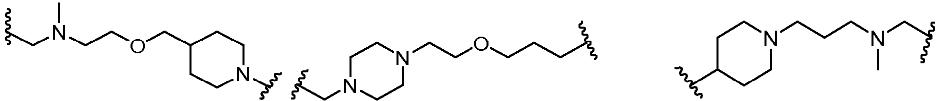 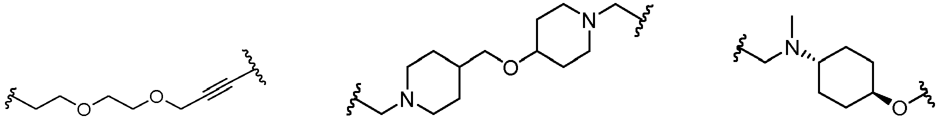 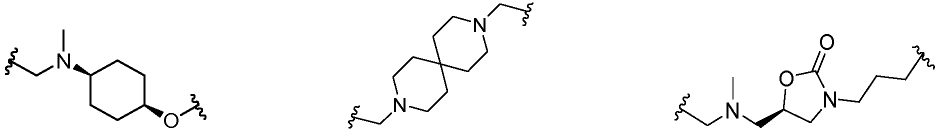 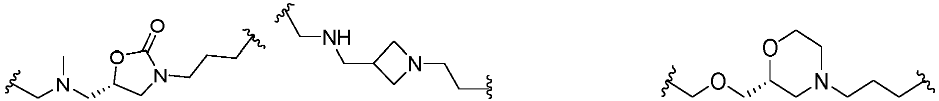 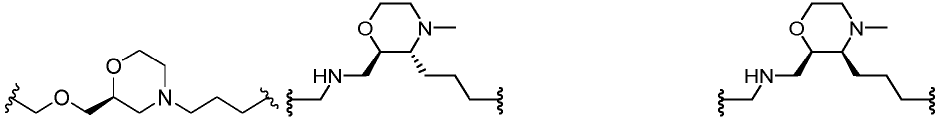 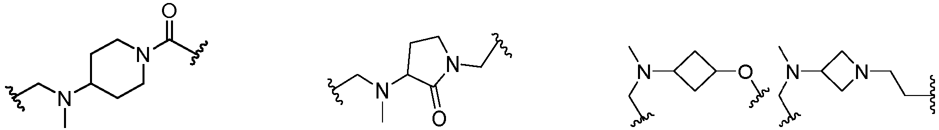 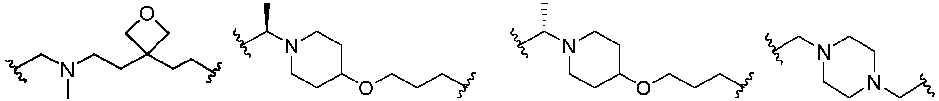 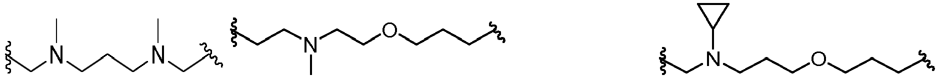 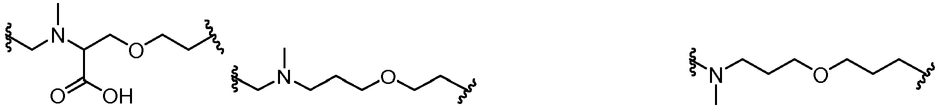 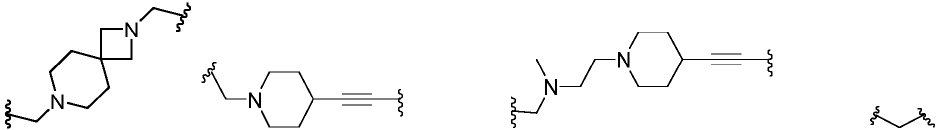 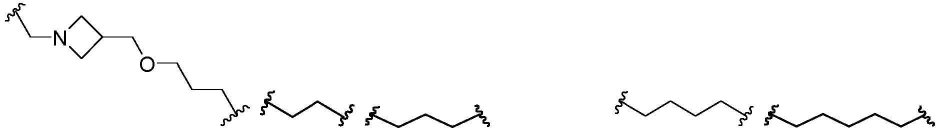 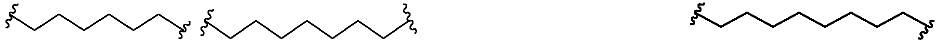 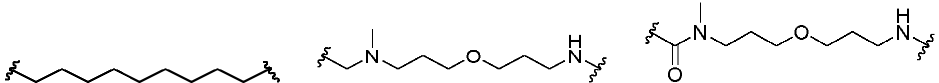 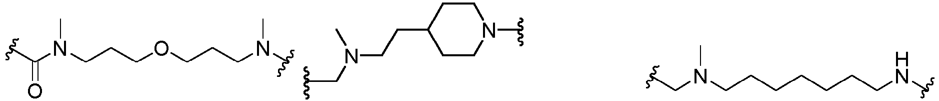 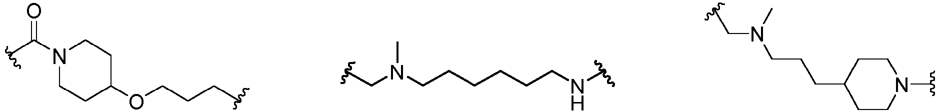 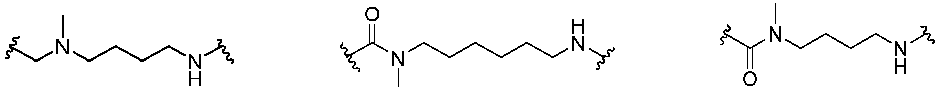 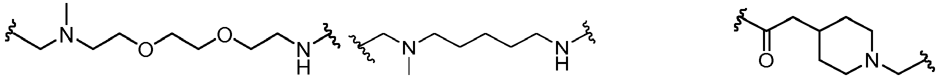 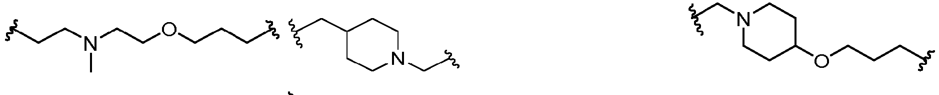 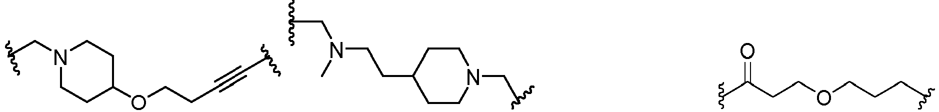 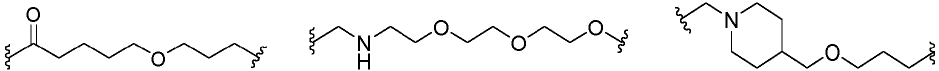 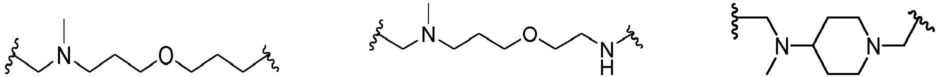 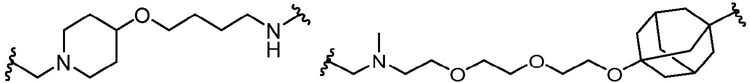 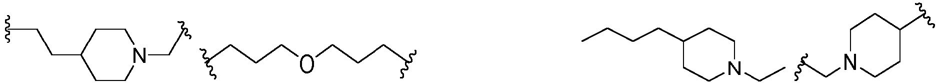 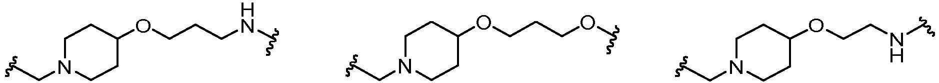 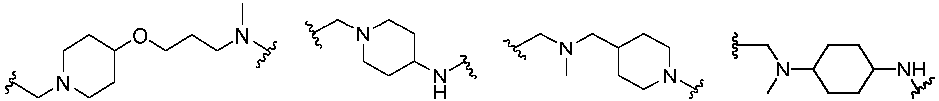 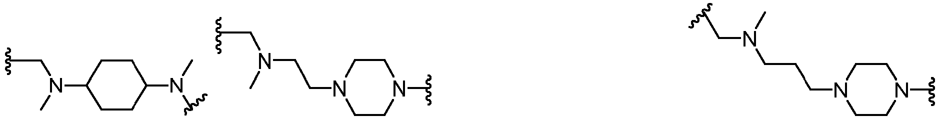 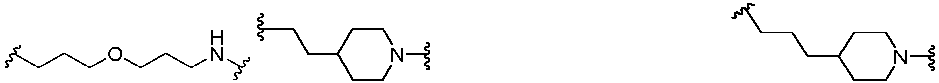 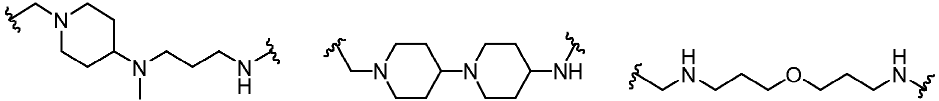 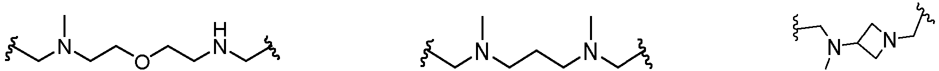 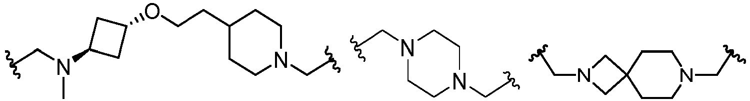 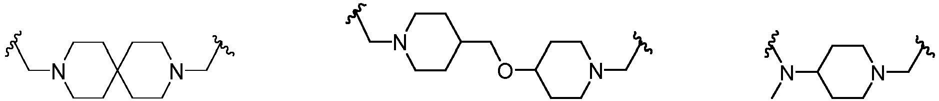 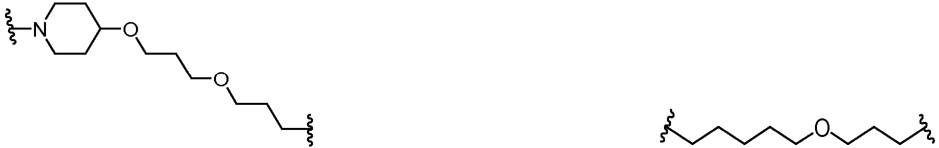 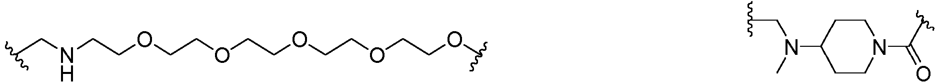 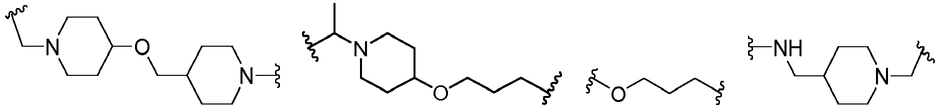 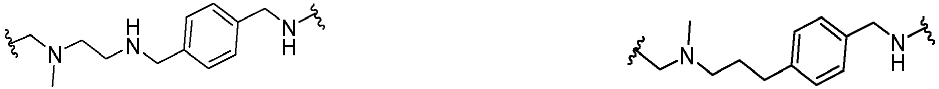 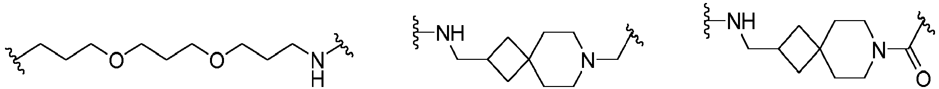 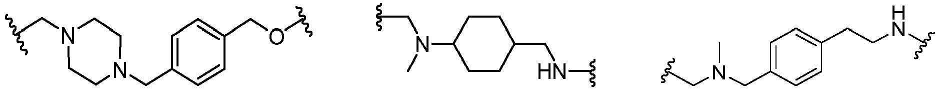 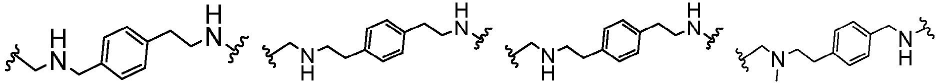 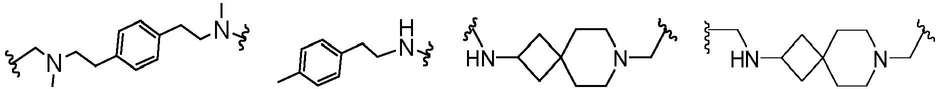 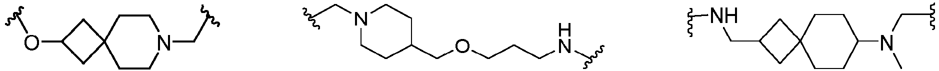 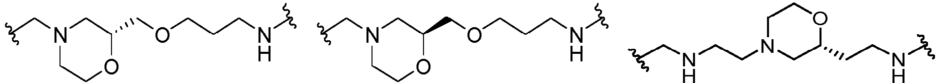 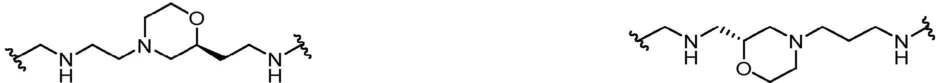 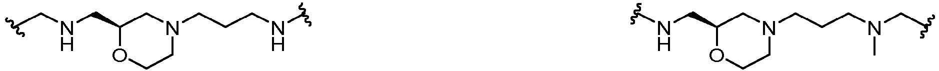 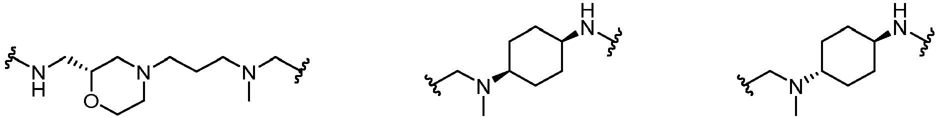 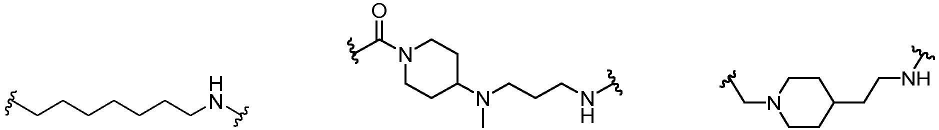 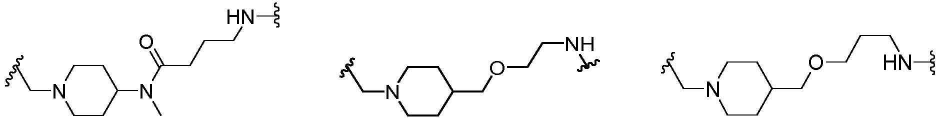 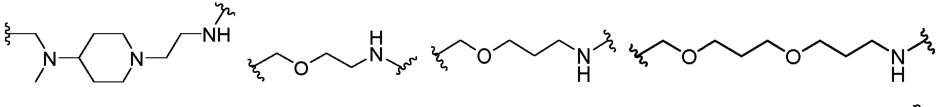 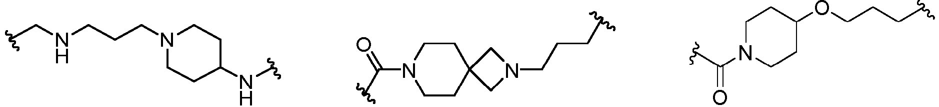 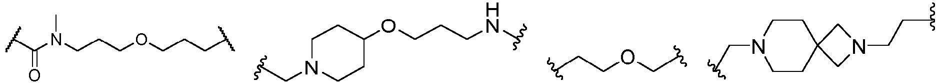 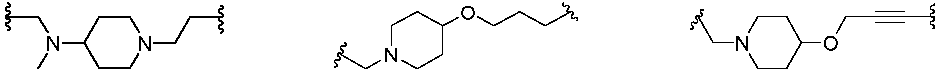 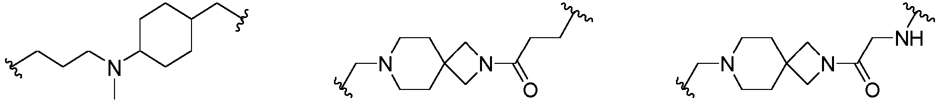 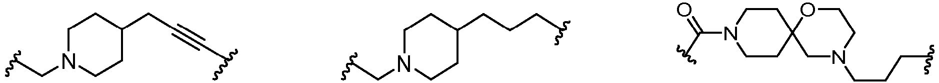 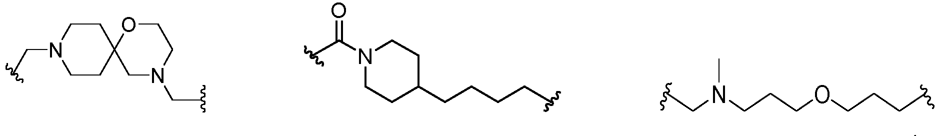 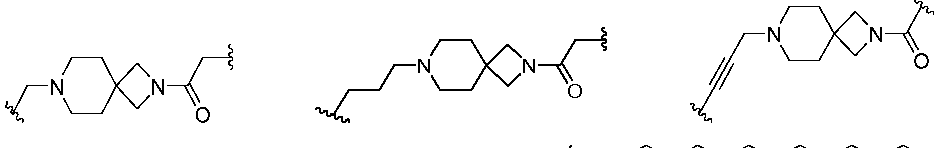 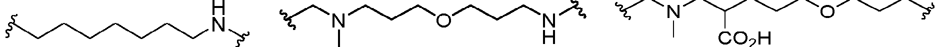 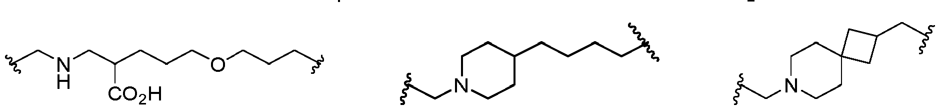 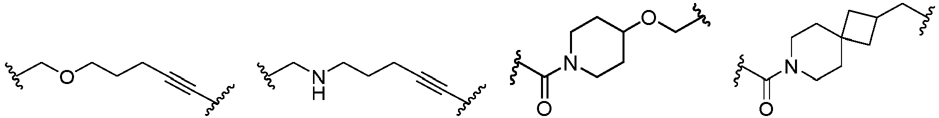 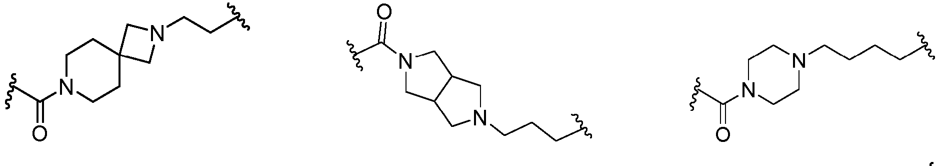 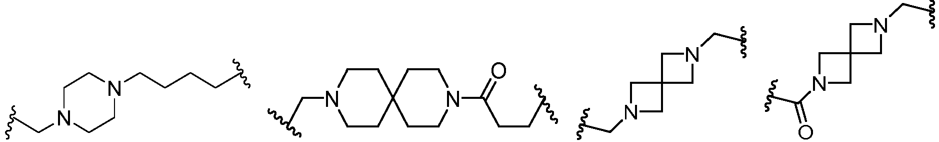 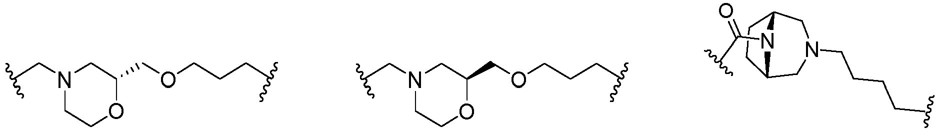 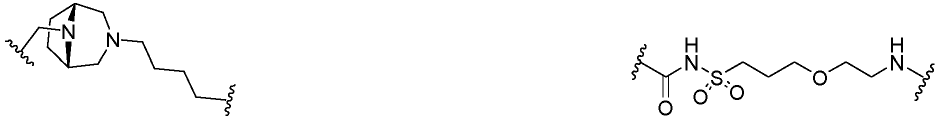 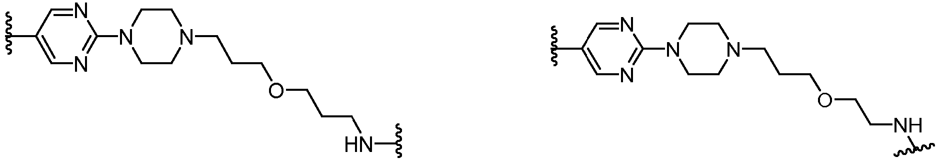 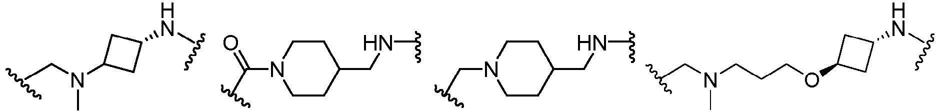 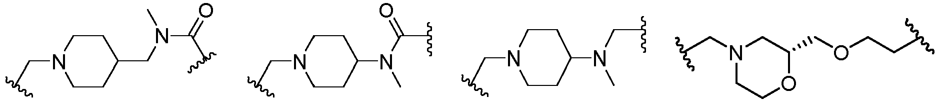 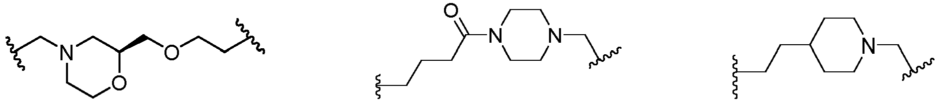 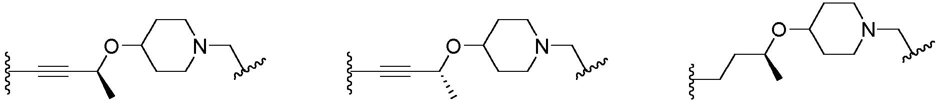 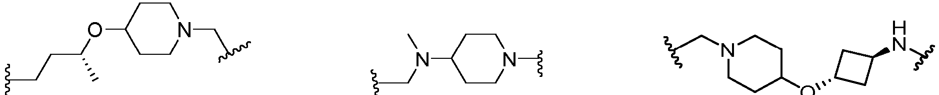 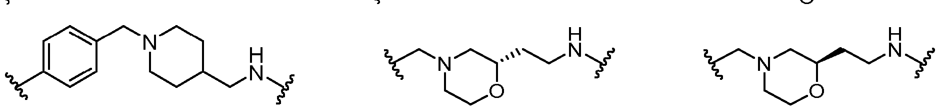 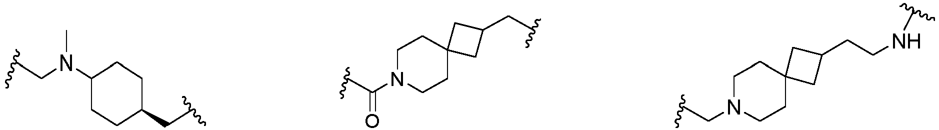 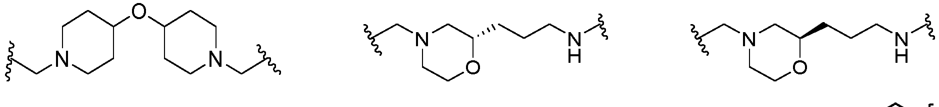 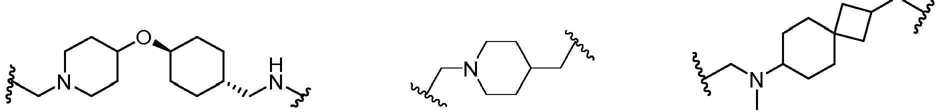 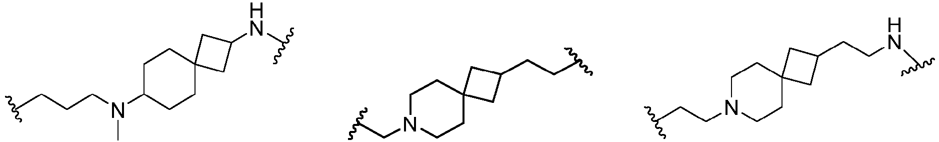 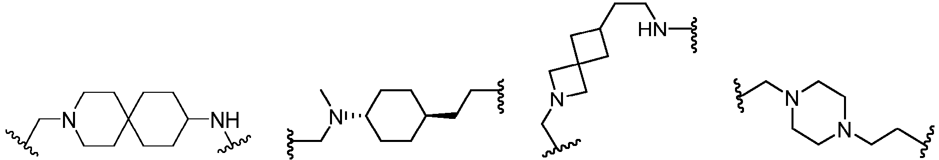 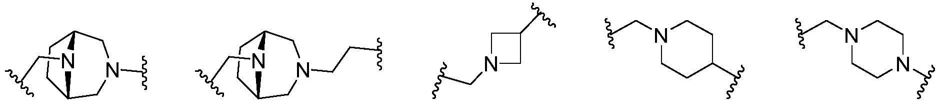 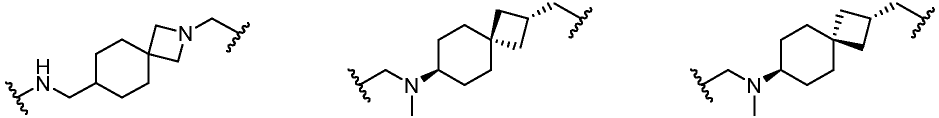 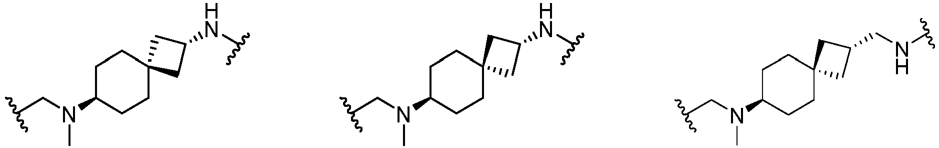 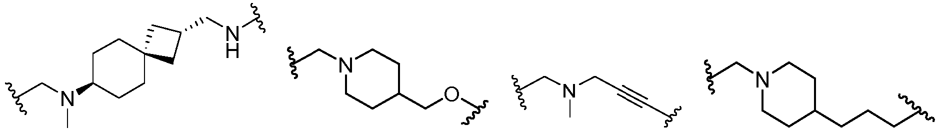 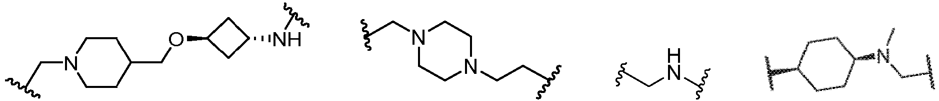 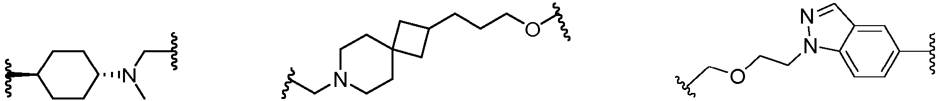 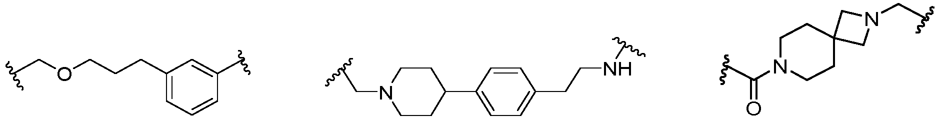 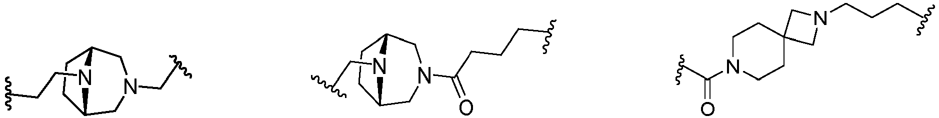 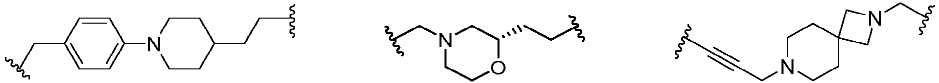 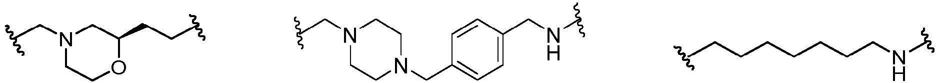 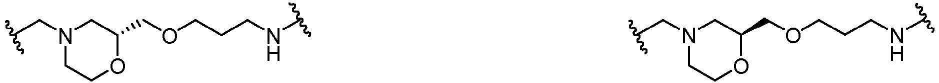 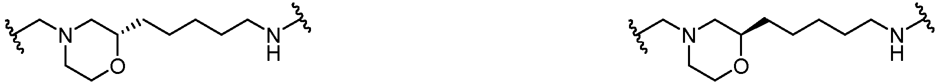 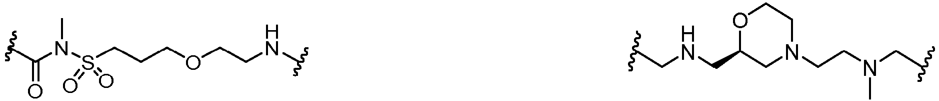 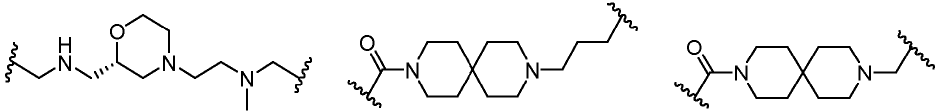 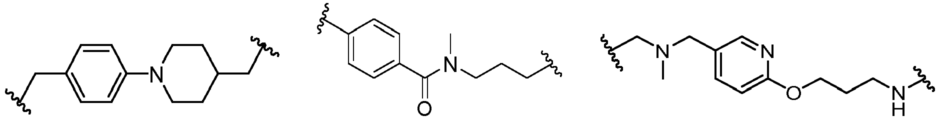 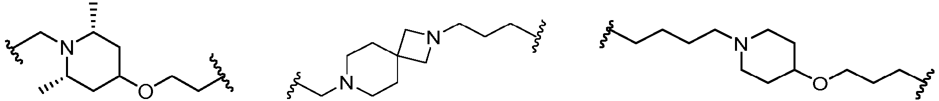 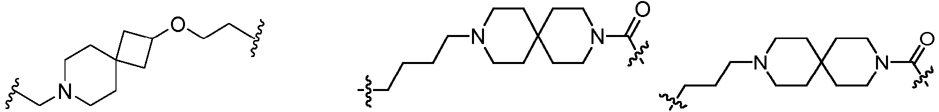 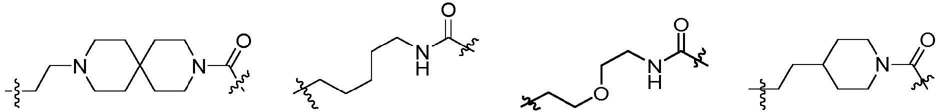 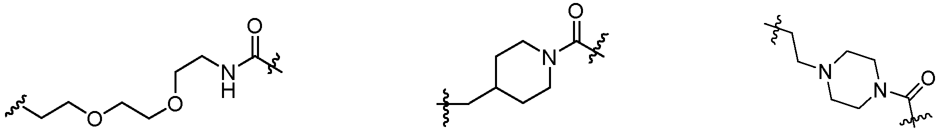 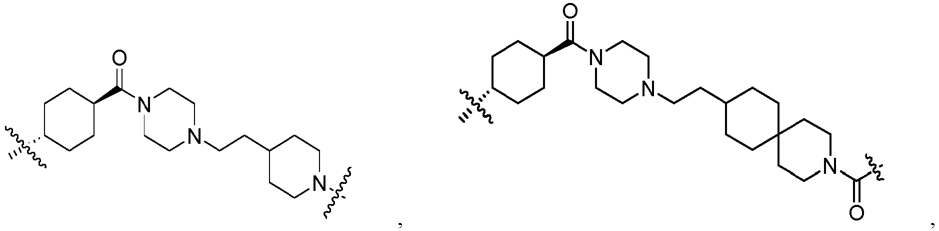 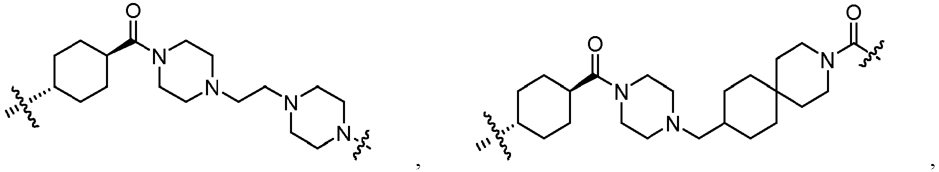 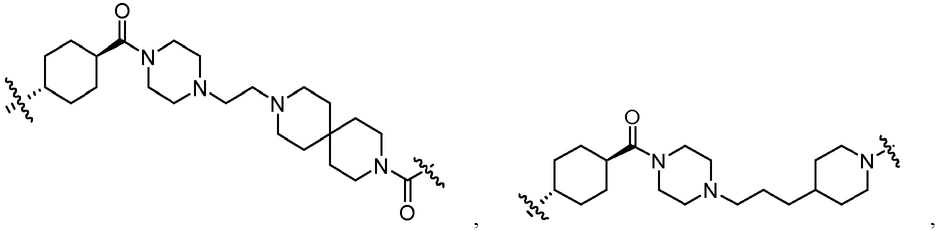 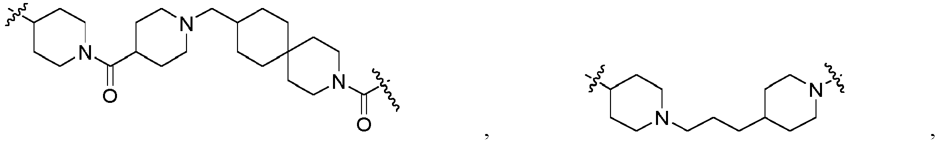 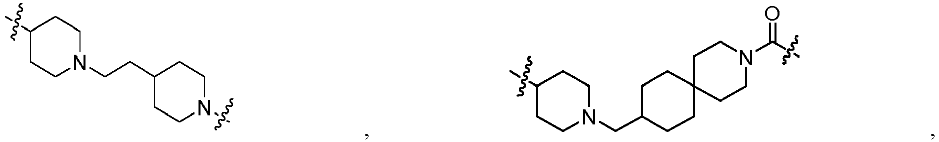 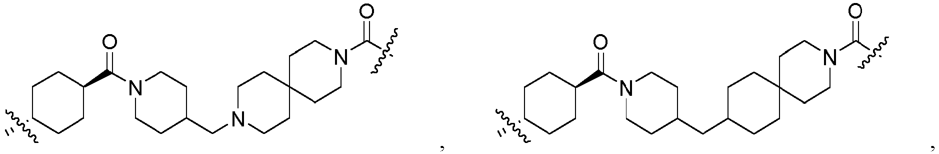 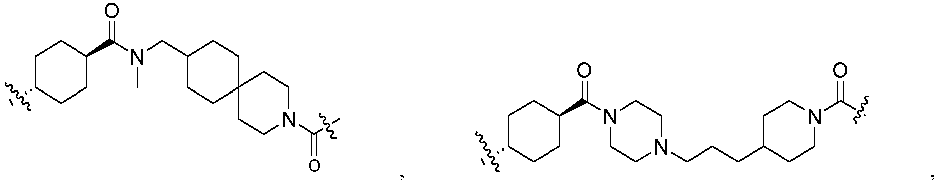 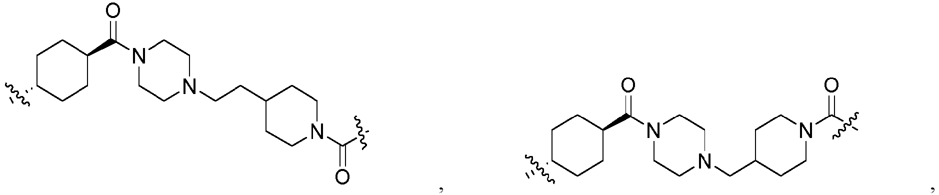 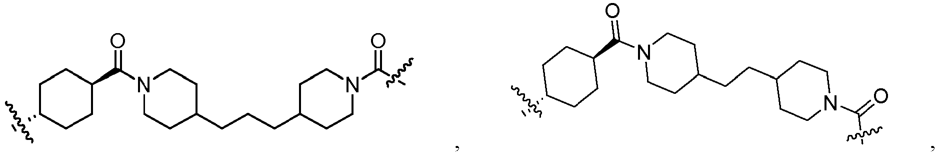 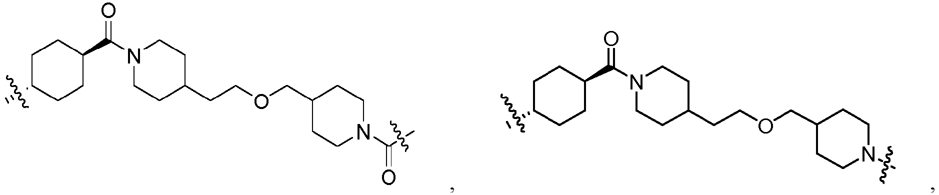 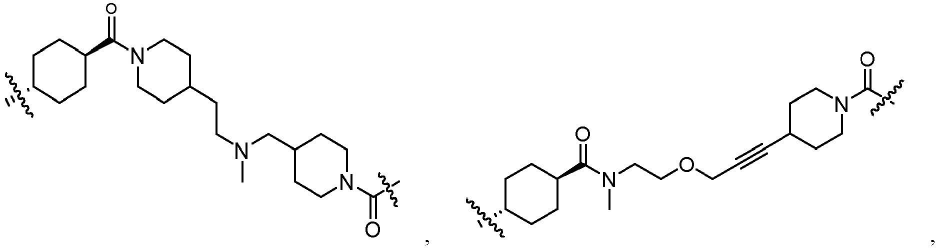 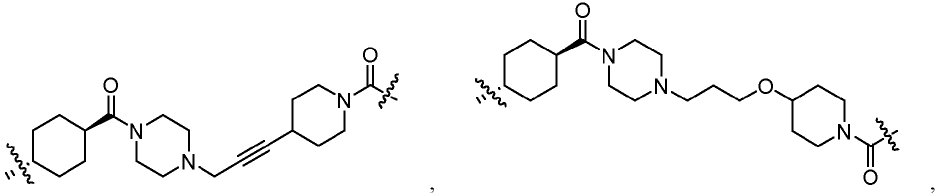 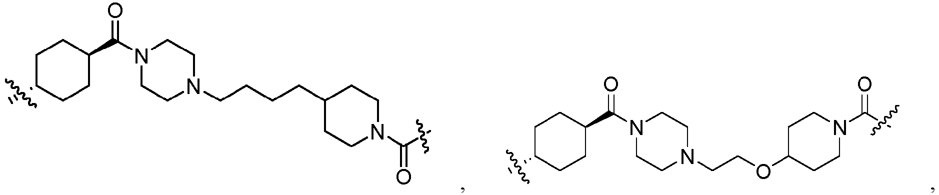 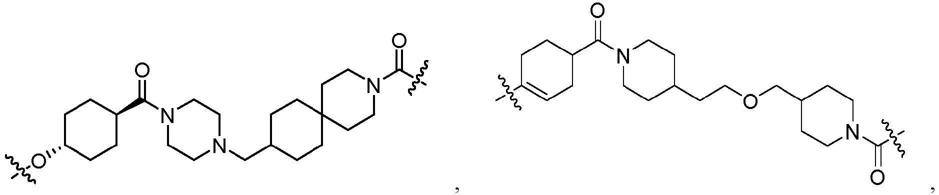 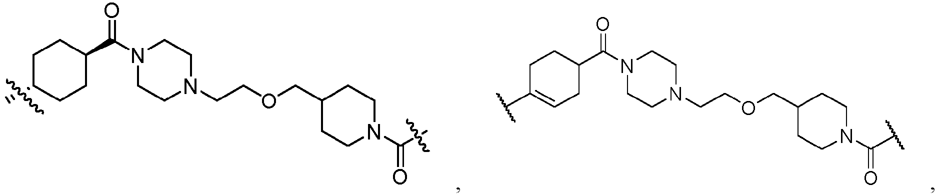 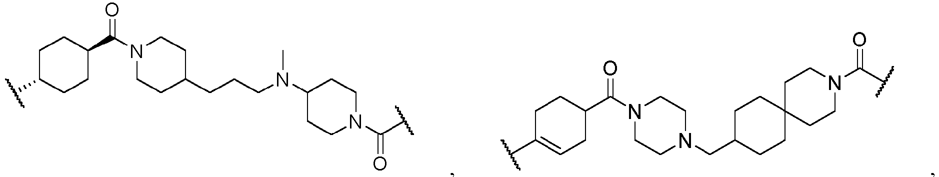 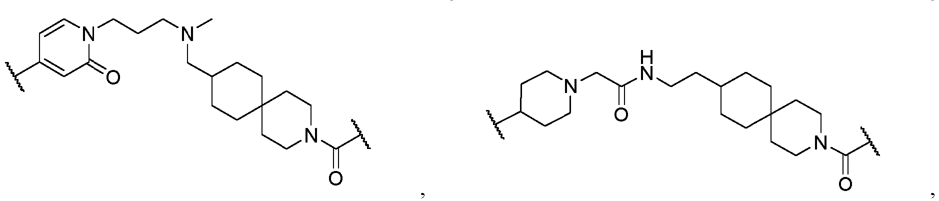 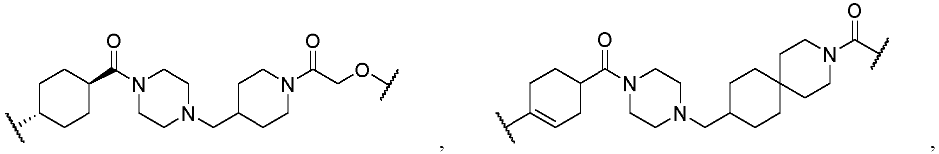 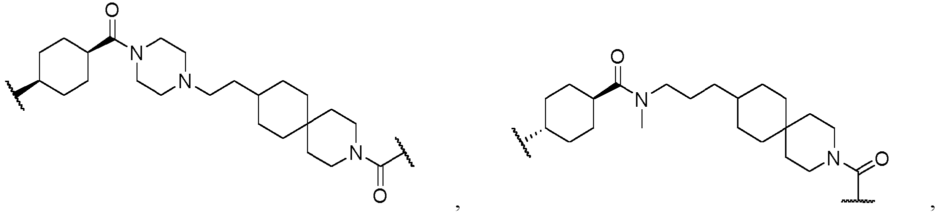 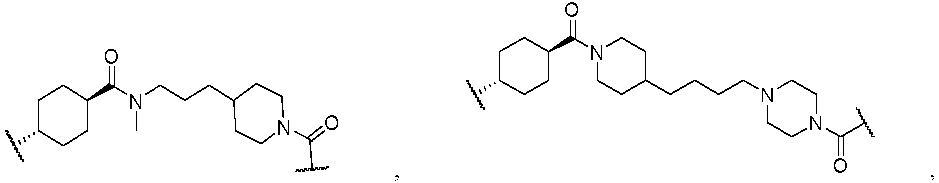 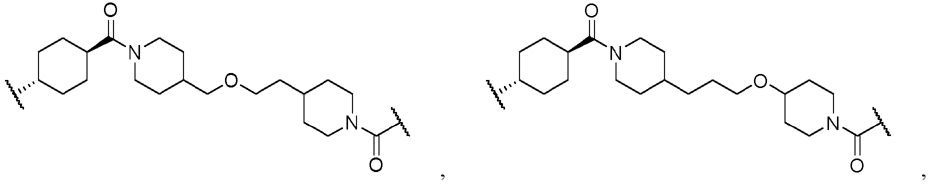 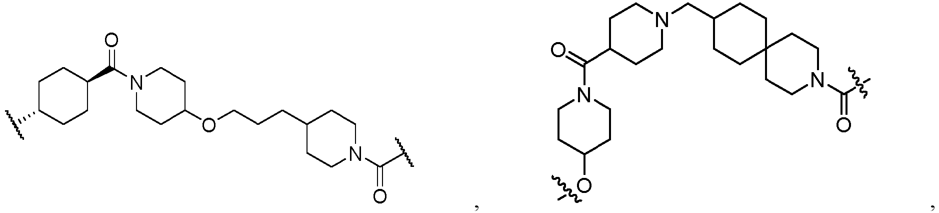 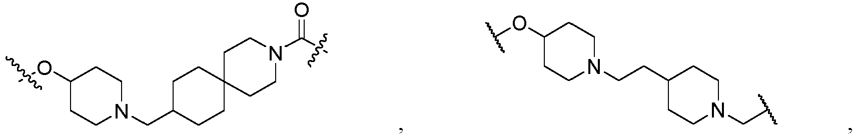 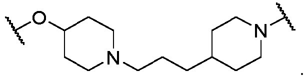 or

22. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to any one of claims 1-19, wherein L is LA: 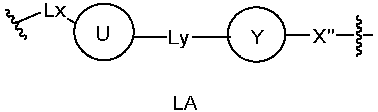 wherein LA satisfies any one of the following conditions:
(1) ring U is C₃-C₁₂ cycloalkylene or 3- to 12-membered heterocycloalkylene containing 1-2 heteroatoms of N, O, or S, wherein the cycloalkylene and heterocycloalkylene are optionally substituted with substituents of halogen, oxo, cyano, amino, hydroxy, C₁-C₆ alkyl, C₁-C₆ haloalkyl, C₁-C₆ hydroxyalkyl, or -O-(C₁-C₆ alkyl);
ring Y is a bond, C₃-C₁₂ cycloalkylene, or 3- to 12-membered heterocycloalkylene containing 1-2 heteroatoms of N, O, or S, wherein the cycloalkylene and heterocycloalkylene are optionally substituted with substituents of halogen, oxo, cyano, amino, hydroxy, C₁-C₆ alkyl, C₁-C₆ haloalkyl, C₁-C₆ hydroxyalkyl, or - O-(C₁-C₆ alkyl);
X" is a bond, -C(O)-, -NH-, -NCH₃-, -O-, -C(CH₃)₂-, -S-, -C=C-, CHF-, -CHCF₃-, -(CH₂)_{q}C(O)-, -S(O)-, - S(O)₂-, -C(O)O-, -OC(O)-, -(CH₂)_{q}C(O)NH-, -C(O)NCH₃-, -NHC(O)-, -NCH₃C(O)-, or -C(O)CH₂O-;
q is 1 or 2;
Lx is -(CH₂)ᵥ-, wherein one or two methylene groups in Lx are optionally replaced by a group of -O-, -S-, - NH-, -C≡C-, -N(C₁-C₆ alkyl)-, -N(C₁-C₆ haloalkyl)-, -C(O)-, -N(C₁-C₆ hydroxyalkyl)-, -N(C₃-C₈ cycloalkyl)-, or -CR_{d}Rₑ-; v is 1, 2, 3, 4, 5, 6, or 7;
R_{d} and Rₑ are each independently H, -OH, C₁-C₆ alkyl, or C₁-C₆ alkoxy;
or R_{d} and Rₑ, together with the C atom linked thereto, form C₃-C₈ cycloalkyl or 3- to 8-membered heterocycloalkyl;
Ly is -(CH₂)ₖ-, wherein one or two methylene groups in Ly are optionally replaced by a group of -O-, -NH-, -C≡C-, -N(C₁-C₆ alkyl)-, -N(C₁-C₆ haloalkyl)-, -C(O)-, -N(C₁-C₆ hydroxyalkyl)-, or -N(C₃-C₈ cycloalkyl)-; k is 1, 2, 3, 4, 5, 6, 7, or 8;
(2) ring U is C₃-C₁₂ cycloalkylene or 3- to 12-membered heterocycloalkylene containing 1-3 heteroatoms of N, O, or S, wherein the cycloalkylene and heterocycloalkylene are optionally substituted with substituents of halogen, oxo, cyano, amino, hydroxy, C₁-C₆ alkyl, C₁-C₆ haloalkyl, C₁-C₆ hydroxyalkyl, or -O-(C₁-C₆ alkyl);
ring Y is a bond, C₃-C₁₂ cycloalkylene, or 3- to 12-membered heterocycloalkylene containing 1-2 heteroatoms of N, O, or S, wherein the cycloalkylene and heterocycloalkylene are optionally substituted with substituents of halogen, oxo, cyano, amino, hydroxy, C₁-C₆ alkyl, C₁-C₆ haloalkyl, C₁-C₆ hydroxyalkyl, or - O-(C₁-C₆ alkyl);
X" is a bond, -C(O)-, -NH-, -NCH₃-, -O-, -C(CH₃)₂-, -S-, -C=C-, CHF-, -CHCF₃-, -(CH₂)_{q}C(O)-, -S(O)-, - S(O)₂-, -C(O)O-, -OC(O)-, -(CH₂)_{q}C(O)NH-, -C(O)NCH₃-, -NHC(O)-, -NCH₃C(O)-, or -C(O)CH₂O-;
q is 1 or 2;
Lx is -(CH₂)ᵥ-, wherein one or two methylene groups in Lx are optionally replaced by a group of -O-, -S-, - NH-, -C≡C-, -N(C₁-C₆ alkyl)-, -N(C₁-C₆ haloalkyl)-, -C(O)-, -N(C₁-C₆ hydroxyalkyl)-, -N(C₃-C₈ cycloalkyl)-, or -CR_{d}Rₑ-; v is 1, 2, 3, 4, 5, 6, or 7;
R_{d} and Rₑ are each independently H, -OH, -NH₂, C₁-C₆ haloalkyl, C₁-C₆ alkyl, or C₁-C₆ alkoxy;
or R_{d} and Rₑ, together with the C atom linked thereto, form C₃-C₈ cycloalkyl or 3- to 8-membered heterocycloalkyl;
Ly is -(CH₂)ₖ-, wherein one or two methylene groups in Ly are optionally replaced by a group of -O-, -NH-, -C≡C-, -N(C₁-C₆ alkyl)-, -N(C₁-C₆ haloalkyl)-, -C(O)-, -N(C₁-C₆ hydroxyalkyl)-, or -N(C₃-C₈ cycloalkyl)-; k is 1, 2, 3, 4, 5, 6, 7, or 8.

23. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 22, wherein ring U in LA is 4- to 8-membered saturated monocyclic heterocycloalkylene, 6- to 10-membered fused heterocycloalkylene, or 7- to 11-membered spiroheterocycloalkylene containing 1 or 2 nitrogen heteroatoms; ring Y is 6- to 8-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond, -C(O)CH₂O-, or -C(O)-; Lx is -(CH₂)ᵥ-, and v is 1, 2, 3, 4, or 5; Ly is -(CH₂)ₖ-, and k is 1, 2, 3, 4, or 5.

24. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 22, wherein LA is LA-1 or LA-2: 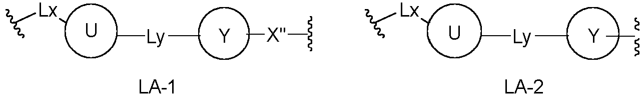
X" is -C(O)-, -C(O)NH-, or -C(O)CH₂O-;
wherein in LA-1 and LA-2, ring U, ring Y, Lx, Ly, and q are as defined and described in claim 22.

25. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 22, wherein LA is LA-3, LA-4, LA-5, or LA-6: 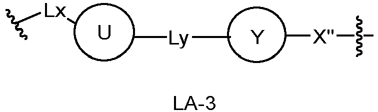 wherein in LA-3:
Lx is a bond;
Lx is -(CH₂)ᵥ-, wherein one or two CH₂ contained in Lx are each independently optionally replaced by -O-, -S-, -NH-, -NMe-, or -CR_{d}Rₑ-; -CR_{d}Rₑ- is 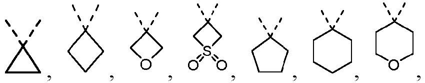 -C(CH₃)₂-, - CH(CH₃)-, -CH(OH)-, -CH(OCH₃)-, or C(CH₃)(OH)-; v is 1, 2, 3, 4, 5, or 6;
ring U is 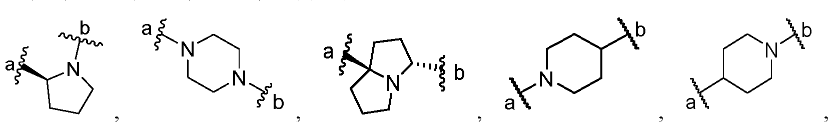 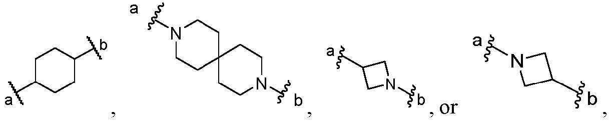 wherein end a is linked to Lx, and end b is linked to Ly; 1, 2, 3, or 4 hydrogen atoms in ring U are optionally substituted with F;
ring Y is a bond, 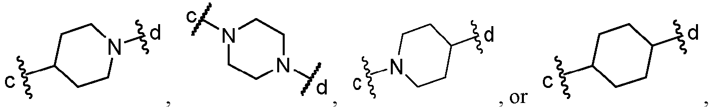 wherein end c is linked to Ly, and end d is linked to X"; 1, 2, 3, or 4 hydrogen atoms in ring Y are optionally substituted with F;
Ly is -(CH₂)ₖ-, wherein one or two CH₂ contained in Ly are each independently optionally replaced by -O-, -C≡C, or -N(C₁-C₆ alkyl)-; k is 1, 2, 3, 4, 5, or 6;
X" is a bond, -C(O)-, -(CH₂)₁₋₂C(O)-, or -(CH₂)₁₋₂C(O)NH-; 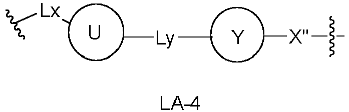
wherein in LA-4:
Lx is -(CH₂)ᵥ-, wherein one or two CH₂ contained in Lx are each independently optionally replaced by -O-, -S-, -NH-, or -CR_{d}Rₑ-; -CR_{d}Rₑ- is 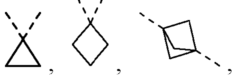 -C(CH₃)₂-, -CH(CH₃)-, -CH(OCH₃)-, - CH(OH)-, or -C(CH₃)(OH)-; v is 1, 2, 3, 4, 5, or 6;
ring U is 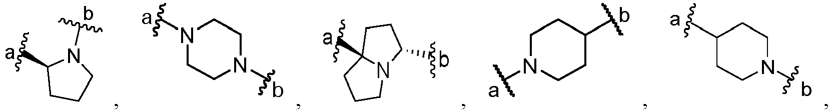 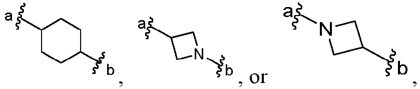 wherein end a is linked to Lx, and end b is linked to Ly; 1, 2, 3, or 4 hydrogen atoms in ring U are optionally substituted with F;
ring Y is 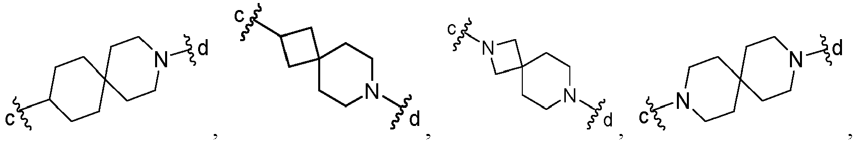 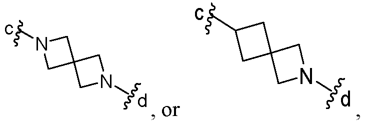 wherein end c is linked to Ly, and end d is linked to X"; 1, 2, 3, or 4 hydrogen atoms in ring Y are optionally substituted with F;
Ly is -(CH₂)ₖ-, wherein one or two CH₂ contained in Ly are each independently optionally replaced by -O- or -N(C₁-C₆ alkyl)-; k is 1, 2, 3, 4, 5, or 6;
X" is -C(O)-; 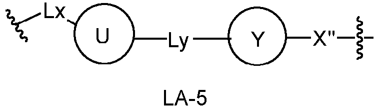
wherein in LA-5:
Lx is -(CH₂)ᵥ-, wherein one or two CH₂ contained in Lx are each independently optionally replaced by -O-;
v is 1, 2, 3, or 4;
ring U is 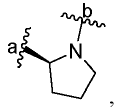 wherein end a is linked to Lx, and end b is linked to Ly; 1, 2, 3, or 4 hydrogen atoms in ring U are optionally substituted with F;
ring Y is a bond;
Ly is -(CH₂)ₖ-, wherein one or two CH₂ contained in Ly are each independently optionally replaced by -O-, -C(O)-, or -NH-; k is 1, 2, 3, 4, 5, 6, 7, or 8;
X" is -C(O)NH-; 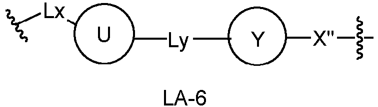
wherein in LA-6:
Lx is -(CH₂)ᵥ-, wherein one or two CH₂ contained in Lx are each independently optionally replaced by -O- or -CR_{d}Rₑ-; -CR_{d}Rₑ- is 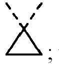 v is 1, 2, 3, or 4;
ring U is 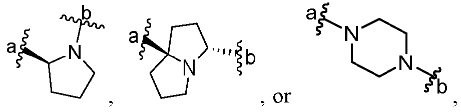
wherein end a is linked to Lx, and end b is linked to Ly;
ring Y is 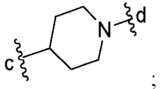
Ly is -(CH₂)ₖ-, wherein one or two CH₂ contained in Ly are each independently optionally replaced by -O-, -C(O)-, or -NH-; k is 1, 2, 3, 4, 5, or 6;
X" is a bond.

26. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 22, wherein LA is LA-7, LA-8, LA-9, LA-10, LA-11, or LA-12: 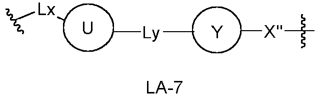 wherein in LA-7:
Lx is -(CH₂)ᵥ-, wherein one or two CH₂ contained in Lx are each independently optionally replaced by -O-, -S-, -NH-, -NMe-, or -CR_{d}Rₑ-; -CR_{d}Rₑ- is 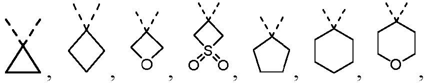 -C(CH₃)₂-, - CH(CH₃)-, -CH(OH)-, -CH(OCH₃)-, or C(CH₃)(OH)-; v is 1, 2, 3, 4, 5, or 6;
ring U is 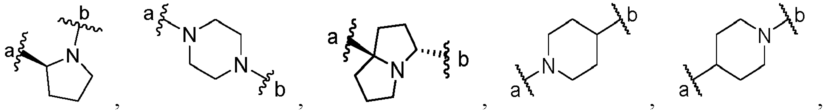 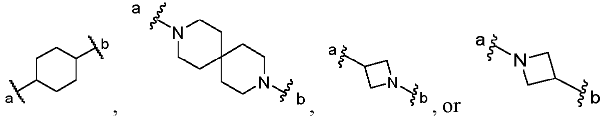 , wherein end a is linked to Lx, and end b is linked to Ly; 1, 2, 3, or 4 hydrogen atoms in ring U are optionally substituted with F;
ring Y is a bond, 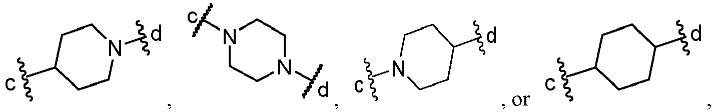 wherein end c is linked to Ly, and end d is linked to X"; 1, 2, 3, or 4 hydrogen atoms in ring Y are optionally substituted with F;
Ly is -(CH₂)ₖ-, wherein one or two CH₂ contained in Ly are each independently optionally replaced by -O-, -C≡C-, -C(O)-, or -N(C₁-C₆ alkyl)-; k is 1, 2, 3, 4, 5, or 6;
X" is a bond, -C(O)-, -(CH₂)₁₋₂C(O)-, -CH₂-C≡C-, -C(O)CH₂O-, or -(CH₂)₁₋₂C(O)NH-; 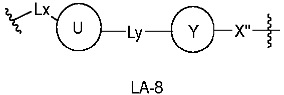
wherein in LA-8:
Lx is -(CH₂)ᵥ-, wherein one or two CH₂ contained in Lx are each independently optionally replaced by -O-, -S-, -NH-, or -CR_{d}Rₑ-; -CR_{d}Rₑ- is 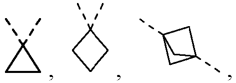 -C(CH₃)₂-, -CH(CH₃)-, -CH(OCH₃)-, - CH(OH)-, -C(CH₃)(OH)-, -CH(CHF₂)-, -CH(CH(CH₃)₂)-, -CH(CH₂CF₃)-, or -CH(NH₂)-; v is 1, 2, 3, 4, 5, or 6;
ring U is 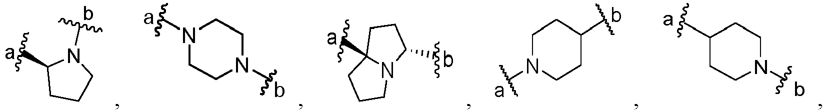 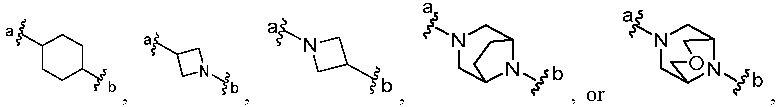 wherein end a is linked to Lx, and end b is linked to Ly; 1, 2, 3, or 4 hydrogen atoms in ring U are optionally substituted with F, cyano, C₁₋₆ alkyl, or C₁-C₆ hydroxyalkyl;
ring Y is 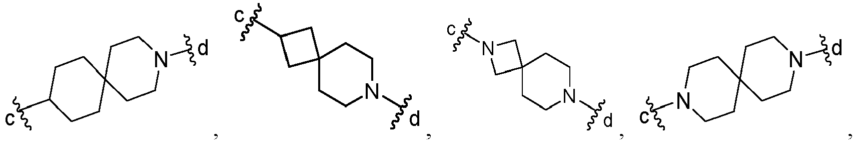 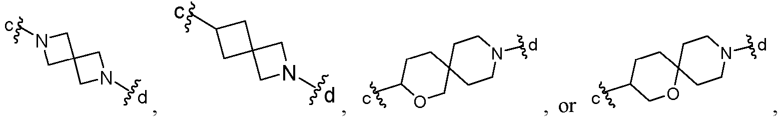 wherein end c is linked to Ly, and end d is linked to X"; 1, 2, 3, or 4 hydrogen atoms in ring Y are optionally substituted with F;
Ly is -(CH₂)ₖ-, wherein one or two CH₂ contained in Ly are each independently optionally replaced by -O- or -N(C₁-C₆ alkyl)-; k is 1, 2, 3, 4, 5, or 6;
X" is -C(O)-; 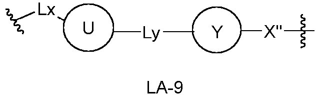
wherein in LA-9:
Lx is -(CH₂)ᵥ-, wherein one or two CH₂ contained in Lx are each independently optionally replaced by -O- or -CR_{d}Rₑ-; -CR_{d}Rₑ- is 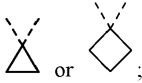 v is 1, 2, 3, 4, 5, or 6;
ring U is 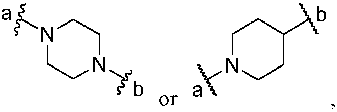 wherein end a is linked to Lx, and end b is linked to Ly;
ring Y is 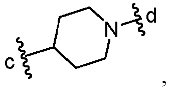 wherein end c is linked to Ly, and end d is linked to X";
Ly is -(CH₂)ₖ-, wherein one or two CH₂ contained in Ly are each independently optionally replaced by -O-, -C≡C-, or -N(C₁-C₆ alkyl)-; k is 1, 2, 3, 4, 5, or 6;
X" is -C(O)-; 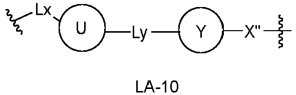
wherein in LA-10:
Lx is -(CH₂)ᵥ-, wherein one or two CH₂ contained in Lx are each independently optionally replaced by -O- or -CR_{d}Rₑ-; -CR_{d}Rₑ- is 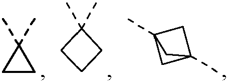 -CH(CH₃)-, -CH(OCH₃)-, -CH(OH)-, or -CH(NH₂)-; v is 1, 2, 3, 4, 5, or 6;
ring U is 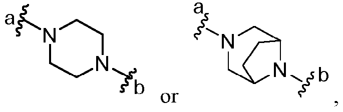 wherein end a is linked to Lx, and end b is linked to Ly;
ring Y is 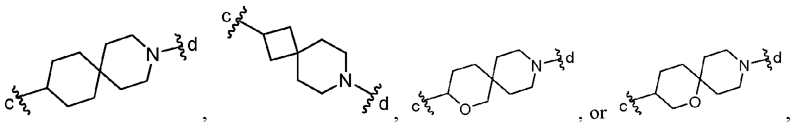 wherein end c is linked to Ly, and end d is linked to X";
Ly is -(CH₂)ₖ-, and k is 1, 2, 3, 4, 5, or 6;
X" is -C(O)-; 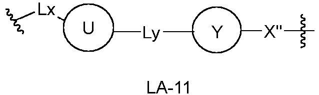
wherein in LA-11:
Lx is -(CH₂)ᵥ-, wherein one or two CH₂ contained in Lx are each independently optionally replaced by -O- or -CR_{d}Rₑ-; -CR_{d}Rₑ- is 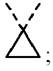 v is 1, 2, 3, 4, 5, or 6;
ring U is 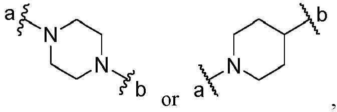 wherein end a is linked to Lx, and end b is linked to Ly;
ring Y is 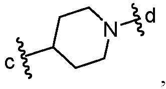 wherein end c is linked to Ly, and end d is linked to X";
Ly is -(CH₂)ₖ-, wherein one or two CH₂ contained in Ly are each independently optionally replaced by -O- or -C≡C-; k is 1, 2, 3, 4, 5, or 6;
X" is -C(O)-; 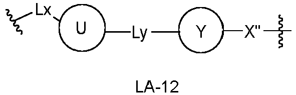
wherein in LA-12:
Lx is -(CH₂)ᵥ-, wherein one or two CH₂ contained in Lx are each independently optionally replaced by -O- or -CR_{d}Rₑ-; -CR_{d}Rₑ- is 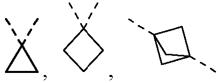 , or -CH(CH₃)-; v is 1, 2, 3, 4, 5, or 6;
ring U is 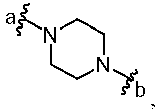 wherein end a is linked to Lx, and end b is linked to Ly;
ring Y is 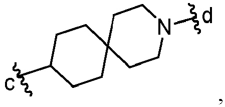 wherein end c is linked to Ly, and end d is linked to X";
Ly is -(CH₂)ₖ-, and k is 1, 2, 3, 4, 5, or 6;
X" is -C(O)-.

27. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 22 or 25, wherein LA is of any one of the following structures:

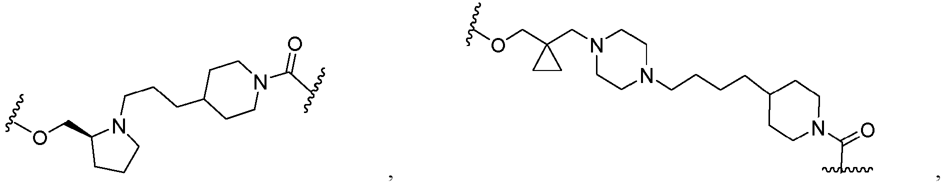

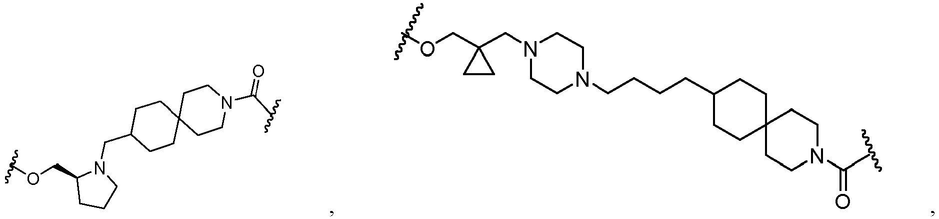

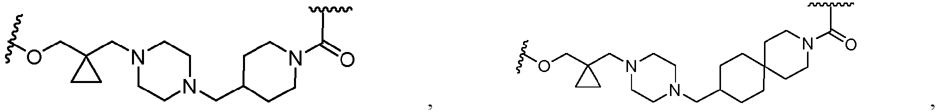

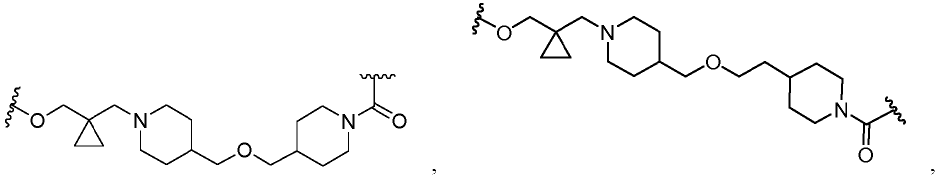

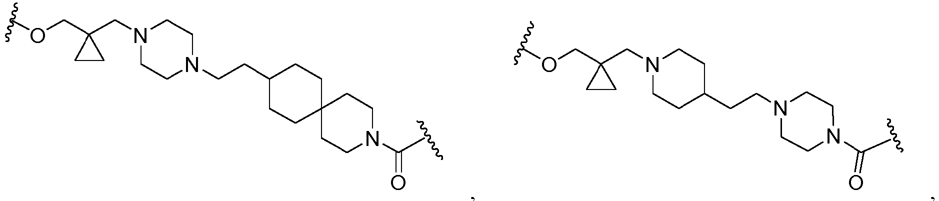

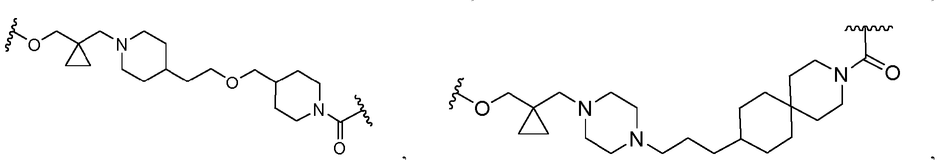

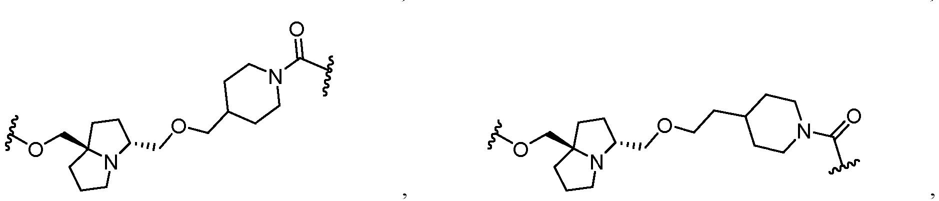

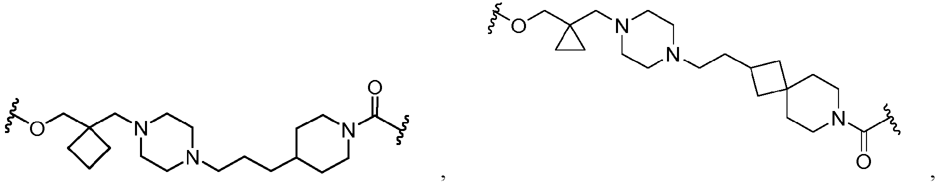

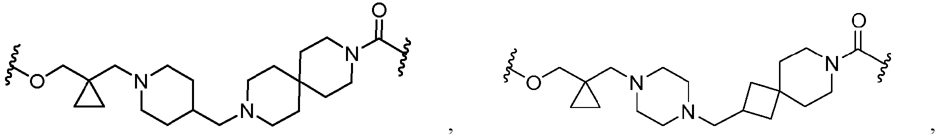

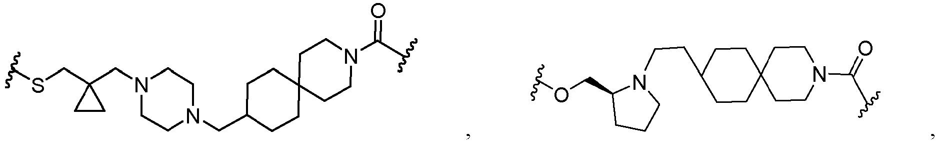

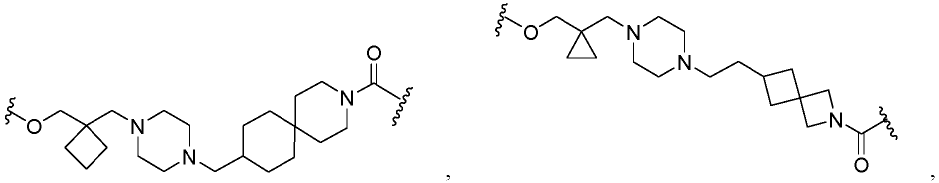

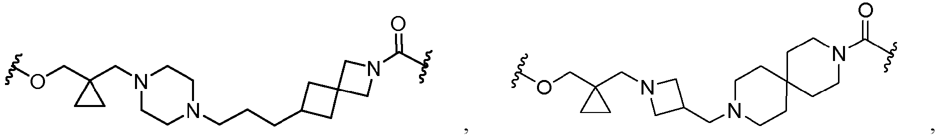

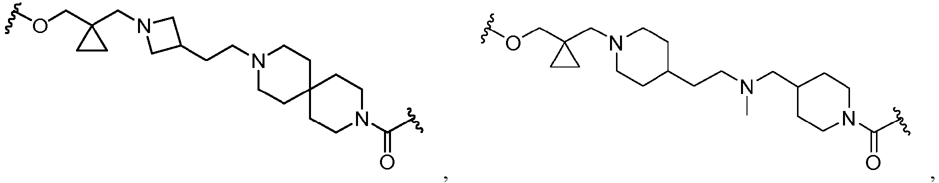

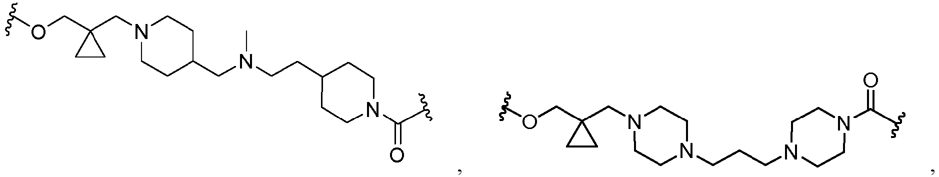

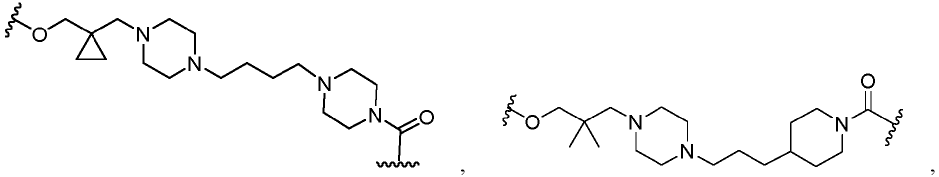

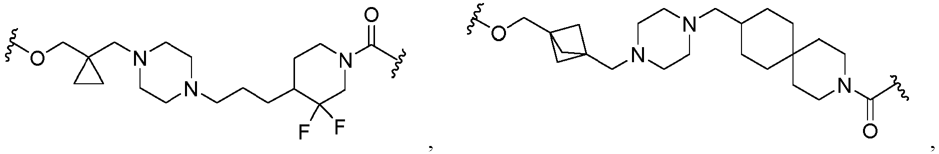

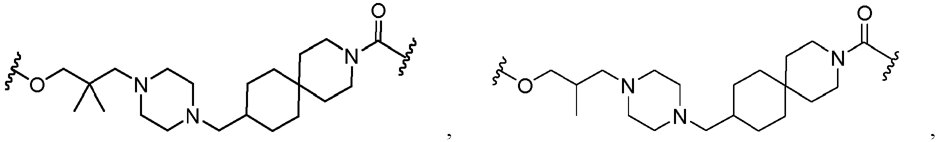

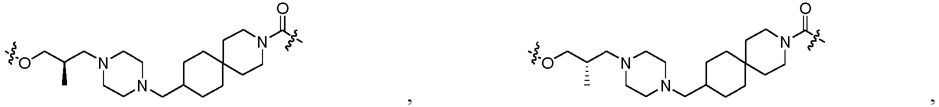

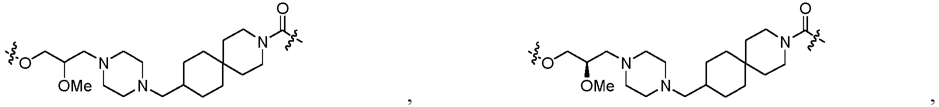

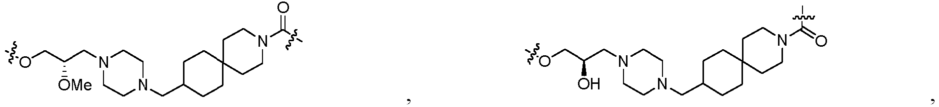

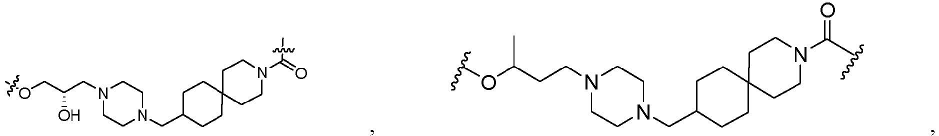

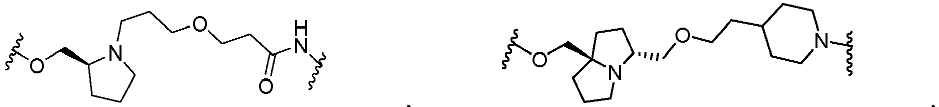

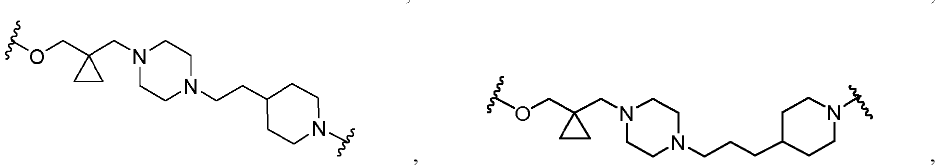

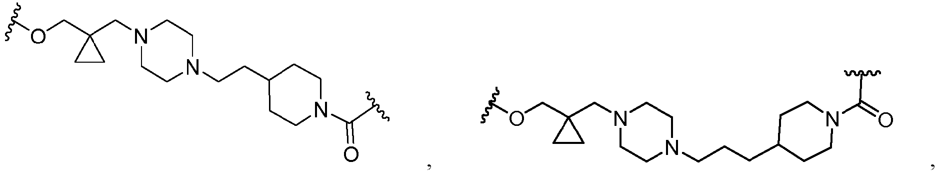

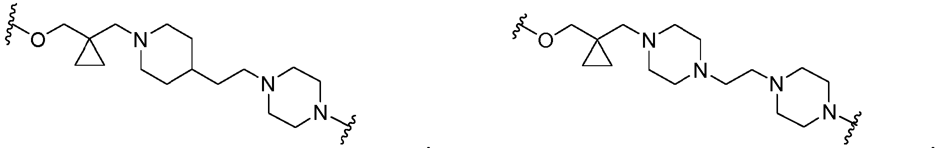

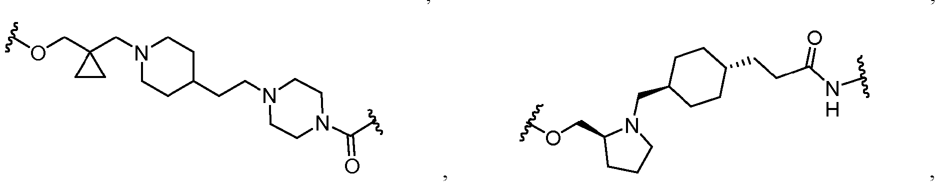

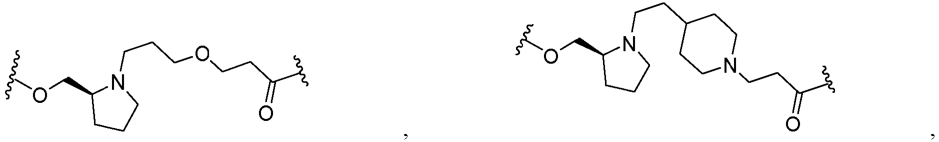

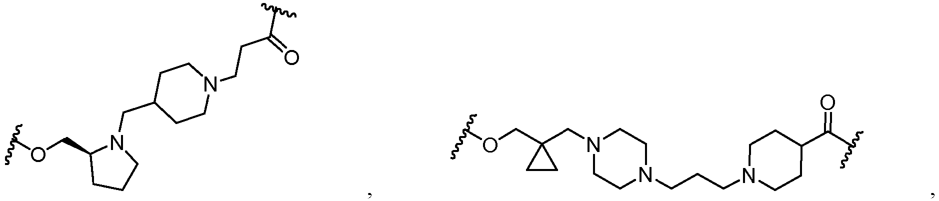

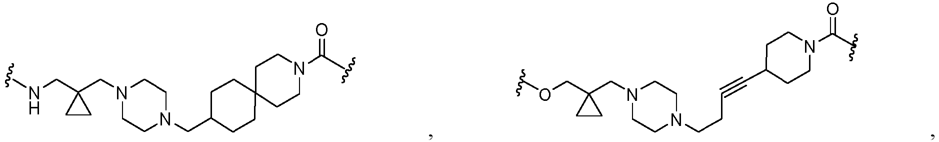

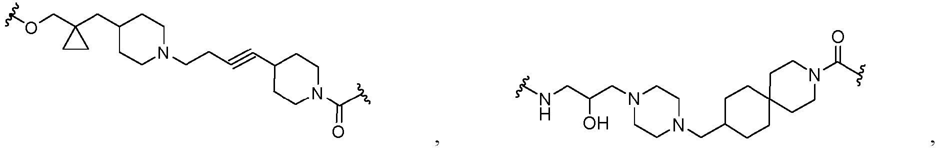

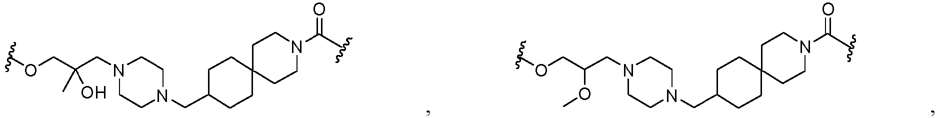

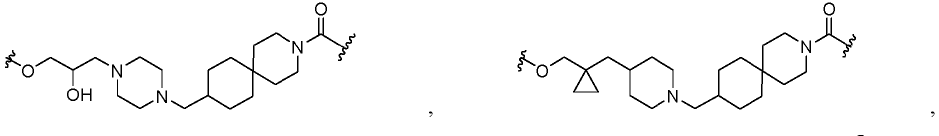

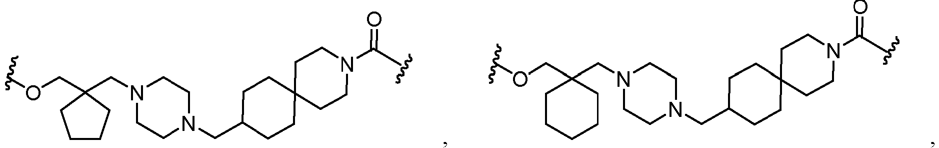

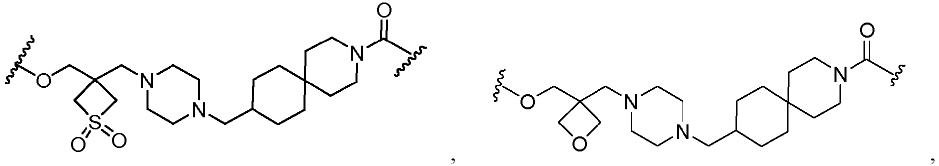

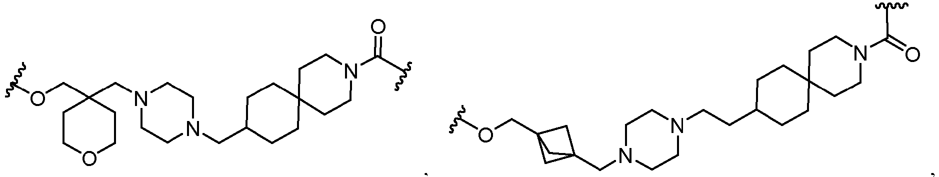

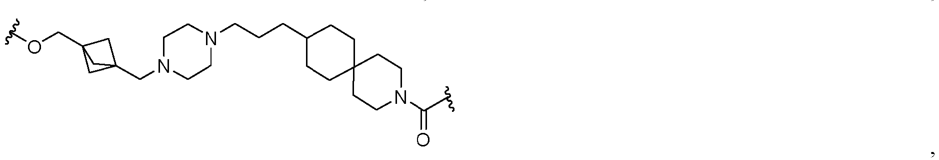

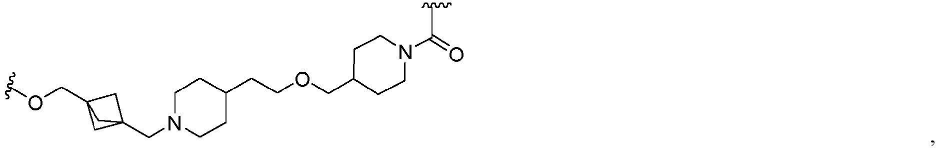

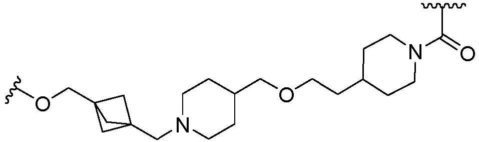

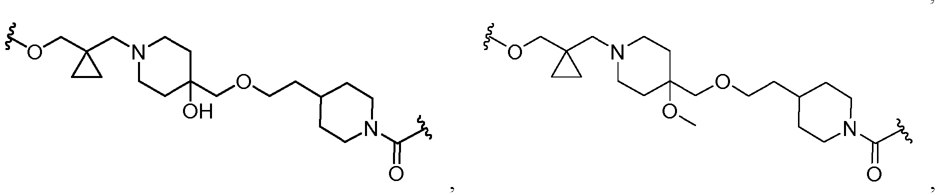

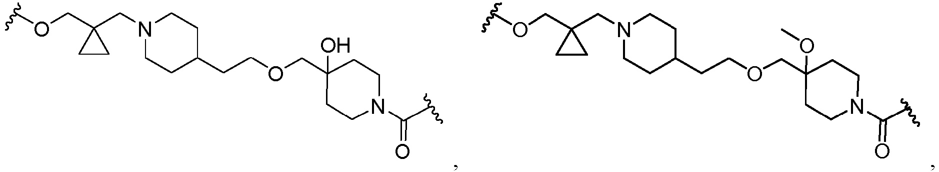

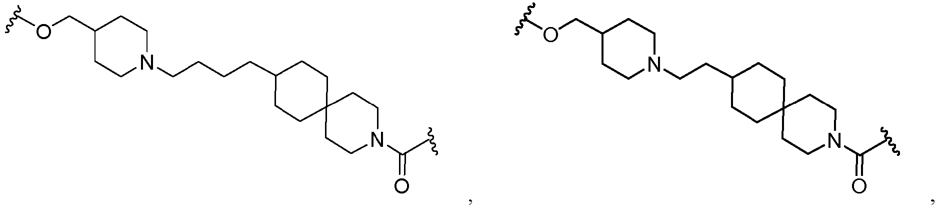

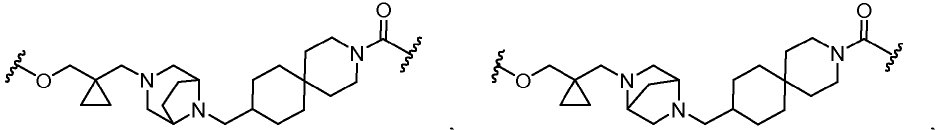

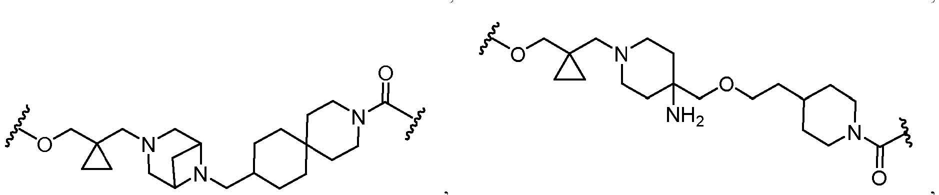

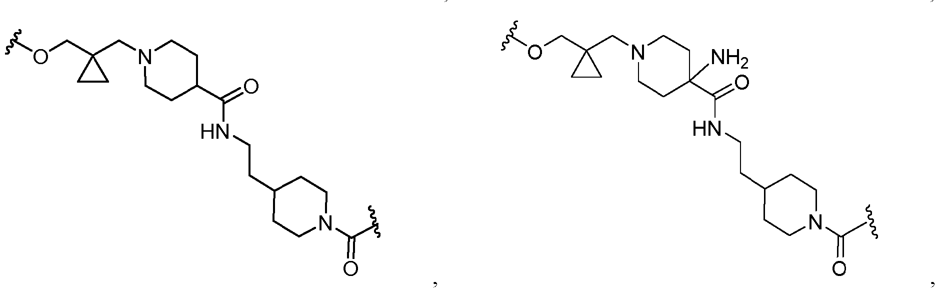

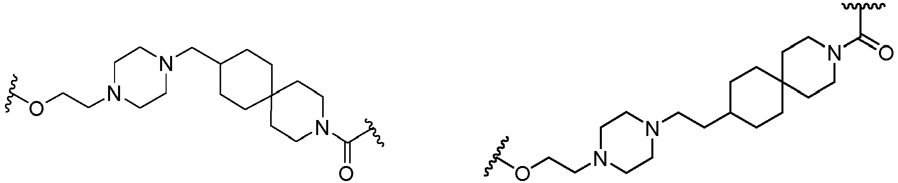

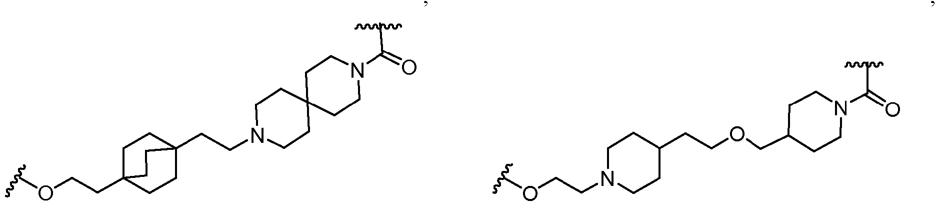

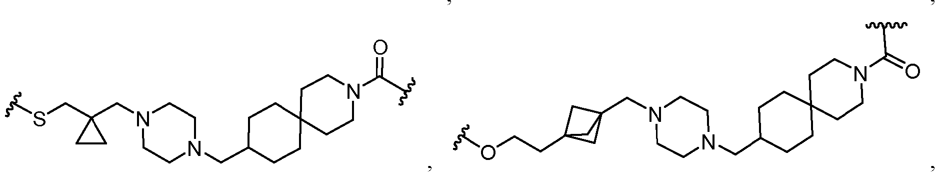

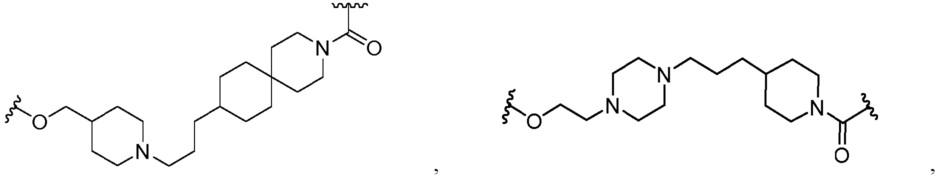

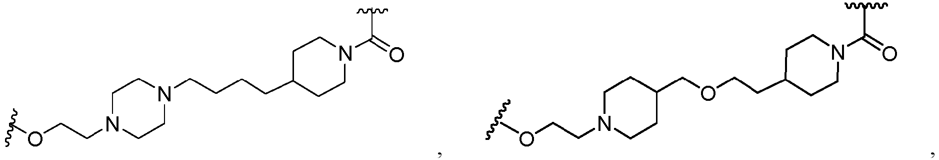

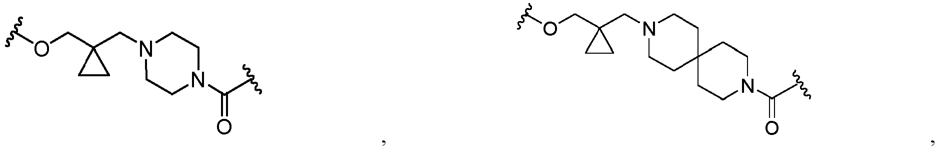

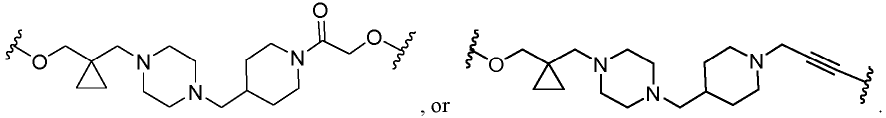

28. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 22 or 26, wherein LA is of any one of the following structures: 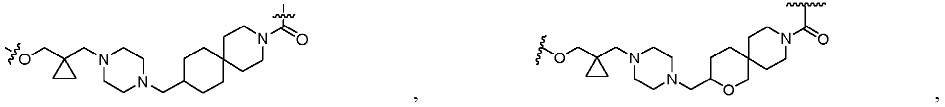 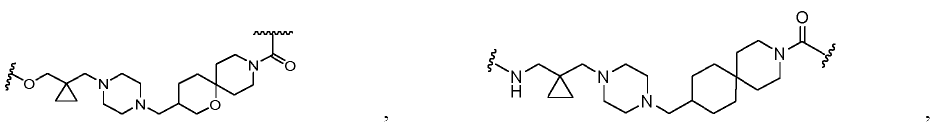 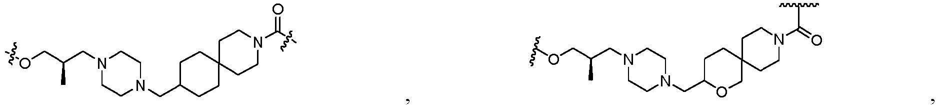 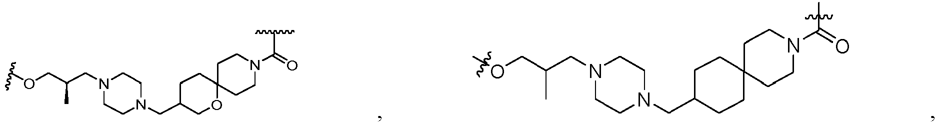 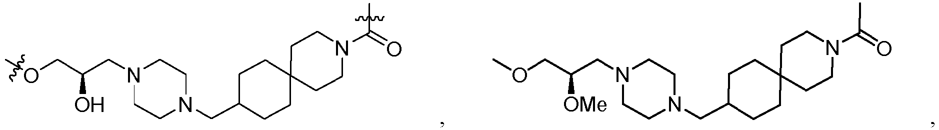 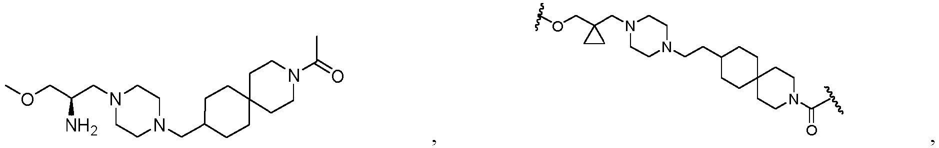 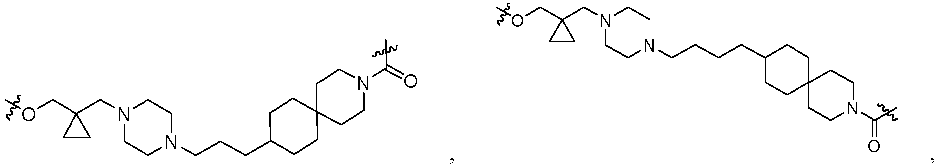 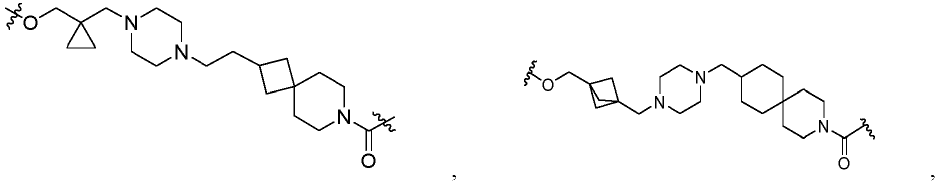 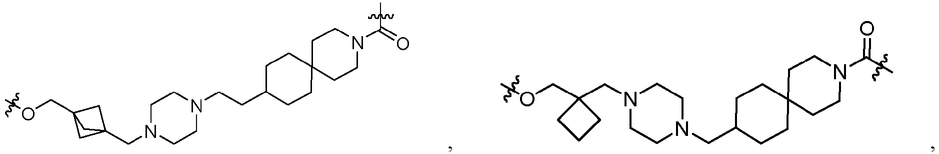 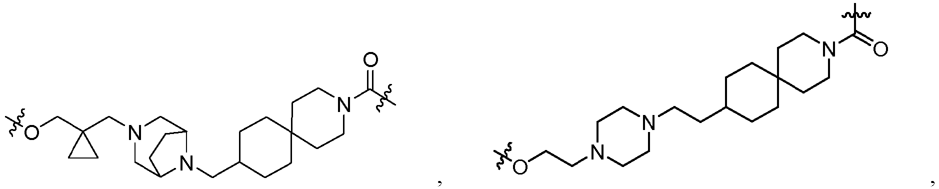 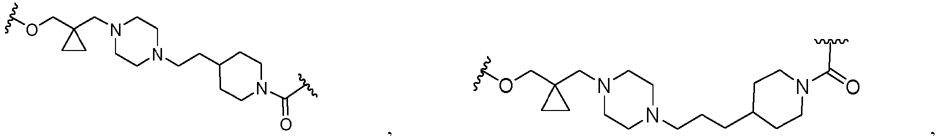 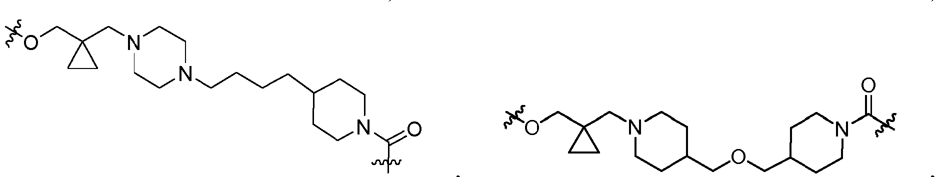 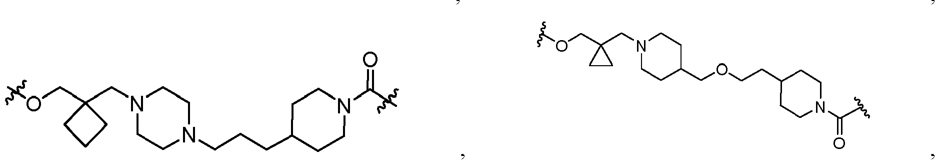 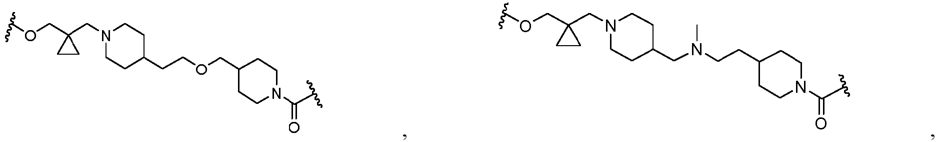 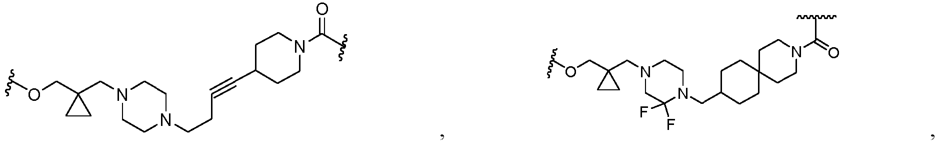 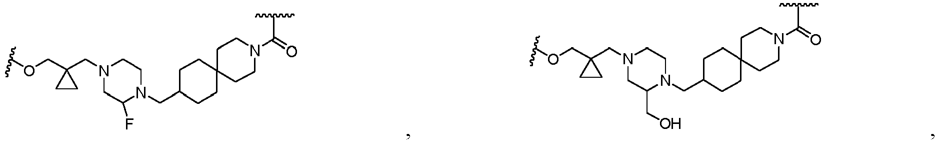 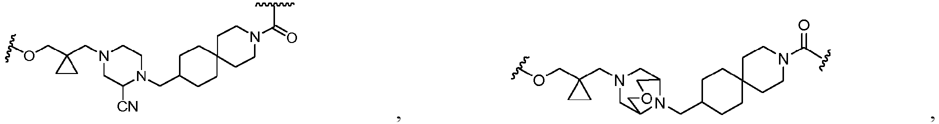 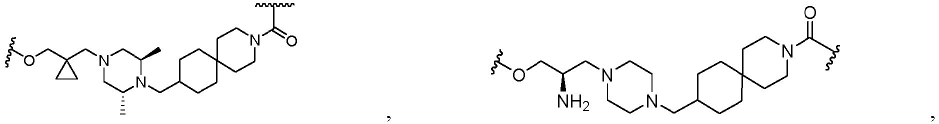 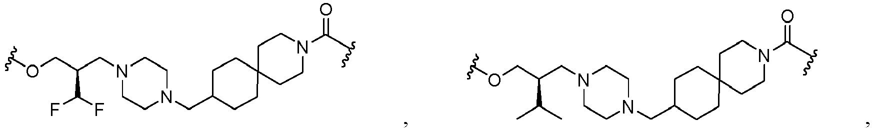 or

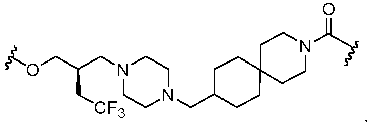

29. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein E1 has a structure of formula E1-1a, E1-1b, E1-1c, E1-1d, E1-1e, E1-1f, E1-1g, E1-1aa, E1-1h, 1-1bb, E1-1cc, E1-1dd, E1-1ee, E1-1ff, E1-1gg, or E1-1hh: 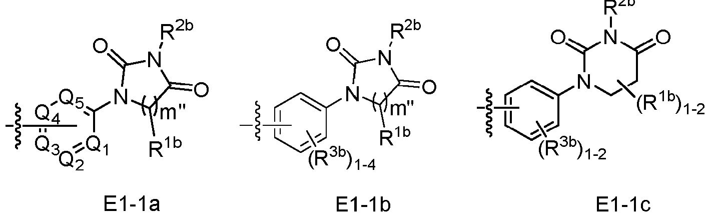 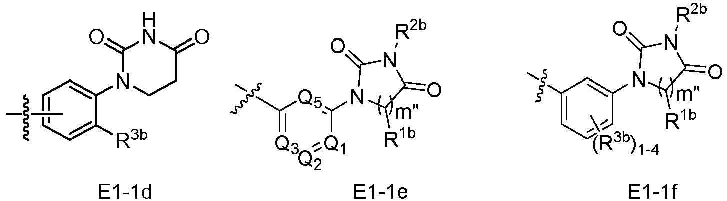 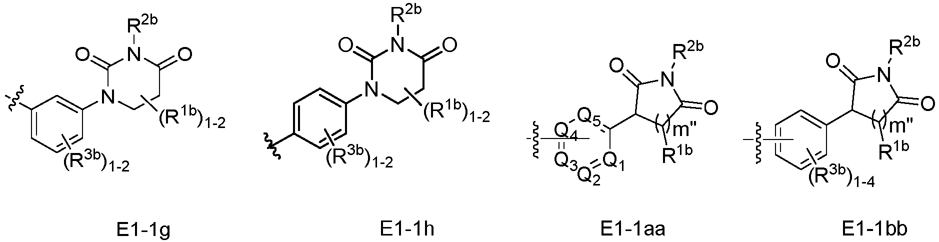 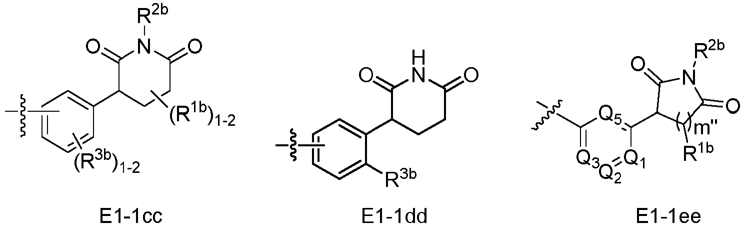 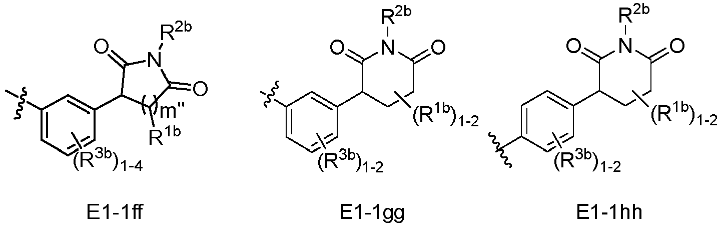 wherein Q₁, Q₂, Q₃, Q₄, Q₅, R^{1b}, R^{2b}, R^{3b}, and m" are as defined and described in claim 1.

30. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 29, wherein E1 has a structure of formula E1-1h", E1-1i', E1-1j', or E1-1h'h': 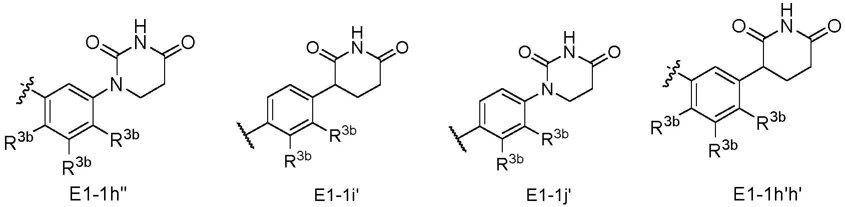 wherein R^{3b} is as defined in claim 24.

31. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to any one of claims 29-30, wherein R^{3b} is hydrogen, halogen, cyano, -OH, -NH₂, C₁-C₆ alkyl, C₃-C₈ cycloalkyl, 3- to 8-membered heterocycloalkyl, -O(C₁-C₆ alkyl), -O(C₃-C₈ cycloalkyl), -O(3- to 8-membered heterocycloalkyl), -N(C₁-C₆ alkyl)₁₋₂, -N(C₃-C₈ cycloalkyl)₁₋₂, or -S(C₁-C₆ alkyl), wherein the C₁-C₆ alkyl, C₃-C₈ cycloalkyl, 3- to 8-membered heterocycloalkyl, -O(C₁-C₆ alkyl), - O(C₃-C₈ cycloalkyl), -O(3- to 8-membered heterocycloalkyl), -N(C₁-C₆ alkyl)₁₋₂, -N(C₃-C₈ cycloalkyl)₁₋₂, or -S(C₁-C₆ alkyl) is optionally substituted with 1-3 halogen, cyano, -OH, and -NH₂.

32. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 29, wherein E1 has a structure of formula E1-1h": 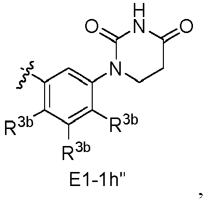 wherein each R^{3b} is independently hydrogen, halogen, C₁-C₆ alkyl, or -O(C₁-C₆ alkyl), the C₁-C₆ alkyl and -O(C₁-C₆ alkyl) being optionally substituted with deuterium, halogen, cyano, -OH, or -NH₂, and the number of substitution is 1, 2, or 3.

33. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 30, wherein E1 is:

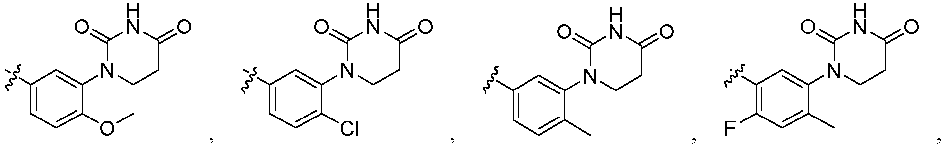

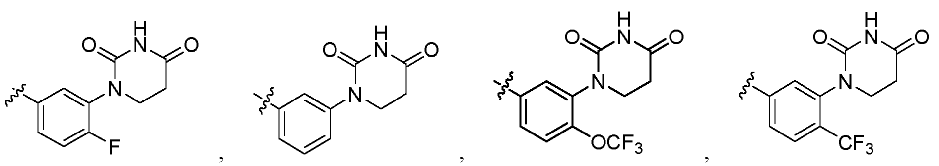

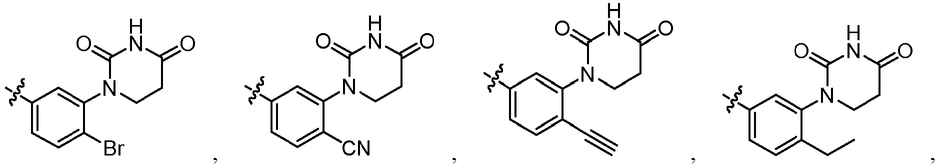

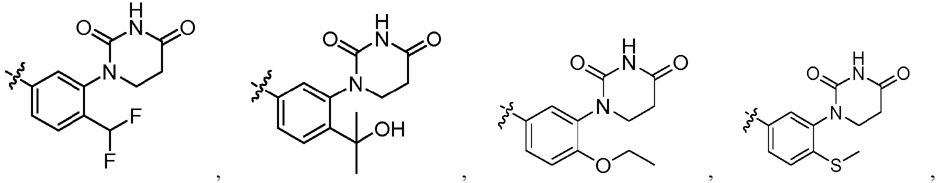

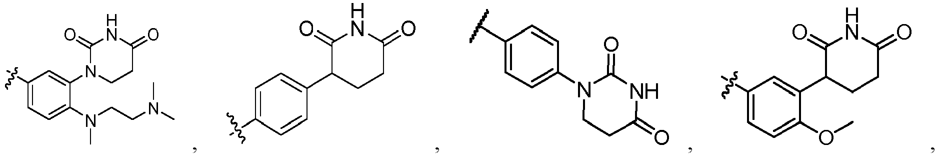

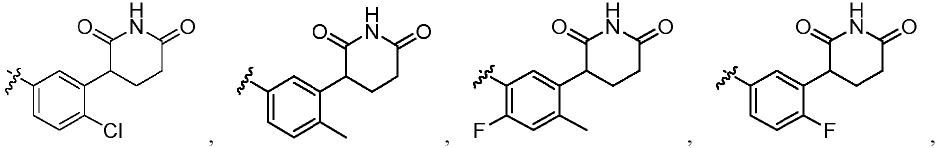

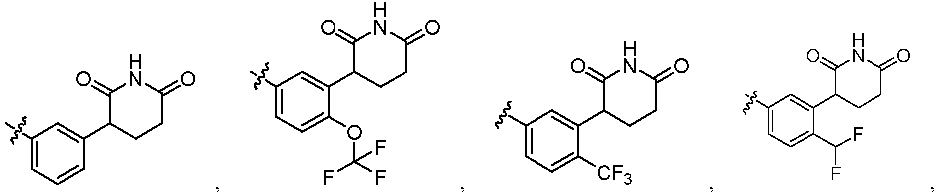

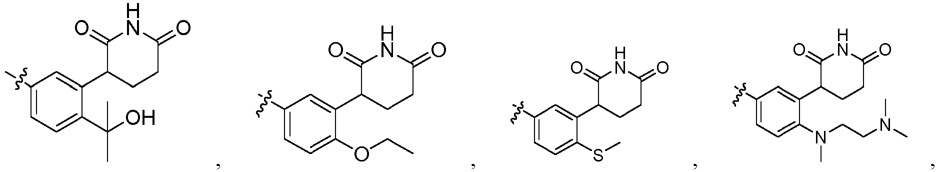

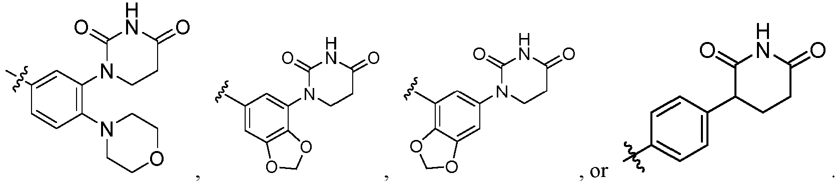

34. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 32, wherein E1 is 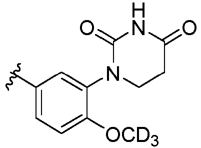 or

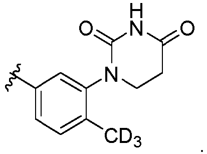

35. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein E2 has a structure of formula E2-1a, E2-1b, E2-1c, E2-1d, E2-1e, or E2-1f: 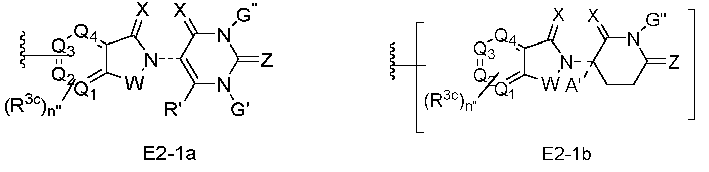 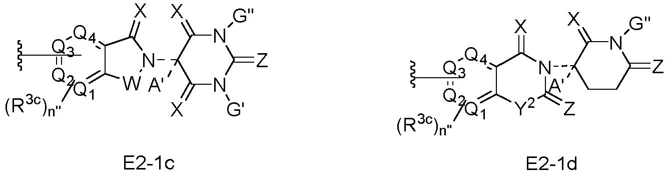 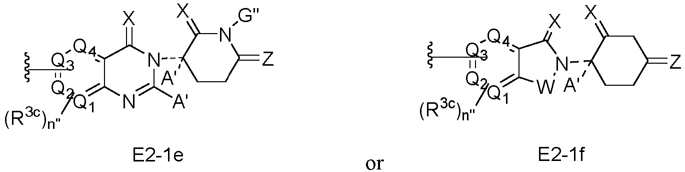 wherein in E2-1a, E2-1b, E2-1c, E2-1d, E2-1e, and E2-1f:
W is CR^{1c}R^{2c}, C(S), C(O), or SO₂;
X is CH₂, O, or S;
Y² is NH, N-(C₁-C₆)alkyl, N-(C₆-C₁₀)aryl, N-(5- to 10-membered)heteroaryl, N-(C₃-C₈)cycloalkyl, N-(3- to 8-membered)heterocycloalkyl, O, or S;
Z is CH₂, O, or S;
G" and G' are each independently hydrogen, deuterium, C₁-C₆ alkyl, OH, C₃-C₆ cycloalkyl, -CH₂-heterocycloalkyl, or -CH₂-phenyl, wherein the C₁-C₆ alkyl, C₃-C₆ cycloalkyl, -CH₂-heterocycloalkyl, or - CH₂-phenyl is optionally substituted with 1-3 groups independently selected from hydroxy, halogen, cyano, and amino;
Q₁, Q₂, Q₃, and Q₄ are each independently CR^{3b} or N;
A' is hydrogen, deuterium, C₁-C₆ alkyl, C₃-C₆ cycloalkyl, or halogen;
R^{1c}, R^{2c}, and R^{3c} are each independently hydrogen, hydroxy, halogen, -NH₂, -N(C₁-C₆ alkyl)₁₋₂, C₁-C₆ alkyl,
C₁-C₆ alkoxy, C₁-C₆ haloalkyl, -CONR_{'}R_{"}, -OR_{'}, -NR_{'}R_{"}, -SR_{'}, -SO₂R_{'}, -SO₂NR_{'}R_{"}, -CR_{'}R_{"}, -CR_{'}NR_{'}R_{"}, aryl, heteroaryl, C₃-C₈ cycloalkyl, 3- to 8-membered heterocycloalkyl, -P(O)(OR_{'})R_{"}, -P(O)R_{'}R_{"}, -OP(O)(OR_{'})R_{"}, -CF₃, -CN, -NR_{'}SO₂NR_{'}R_{"}, -NR_{'}C(O)NR_{'}R_{"}, -C(O)NR_{'}C(O)R_{"}, -NR_{'}C(=N-CN)NR_{'}R_{"}, -C(=N-CN)NR_{'}R_{"}, - NR_{'}C(=N-CN)R_{"}, -NR_{'}C(=C-NO₂)NR_{'}R_{"}, -SO₂NR_{'}COR_{"}, -NO₂, -COR_{'}, -C(C=N-OR_{'})R_{"}, -CR_{'}=CR_{'}R_{"}, -CCR_{'}, -S(C=O)(C=N-R_{'})R_{"}, -SF₅, or -OCF₃;
R_{'} and R_{"} are each independently a bond, hydrogen, deuterium, C₁-C₆ alkyl, C₃-C₈ cycloalkyl, C₆-C₁₀ aryl, 5- to 10-membered heteroaryl, or 3- to 8-membered heterocycloalkyl;
n" is 0, 1, 2, or 3;
----- is a bond, which may be an R stereoisomer, an S stereoisomer, or a non-stereoisomer;
wherein R^{3b} is as described and defined in claim 1.

36. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 35, wherein E2 has a structure of formula E2-1bb, E2-1aa, or E2-1cc: 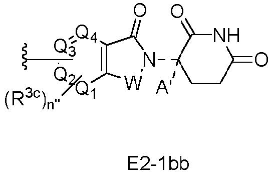 wherein in E2-1bb:
Q₁, Q₂, Q₃, and Q₄ are each independently CR^{3b} or N;
W is C(O) or CH₂;
A' is hydrogen, deuterium, C₁-C₆ alkyl, or halogen;
R^{3c} is hydrogen, deuterium, hydroxy, halogen, cyano, -NH₂, -N(C₁-C₆ alkyl)₁₋₂, C₁-C₆ alkyl, C₁-C₆ alkoxy,
or C₁-C₆ haloalkyl;
n" is 0, 1, 2, or 3;
----- is a bond, which may be an *R* stereoisomer, an *S* stereoisomer, or a non-stereoisomer;
wherein R^{3b} is as described and defined in claim 1; 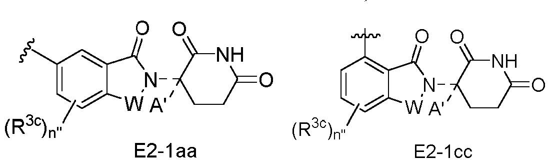
wherein in E2-1aa and E2-1aa:
W is CH₂ or C(O);
A' is hydrogen, methyl, Cl, or F;
each R^{3c} is independently hydrogen, hydroxy, NH₂, C₁-C₆ alkyl, or C₁-C₆ alkoxy;
n" is 0, 1, 2, or 3;
----- is a bond, which may be an *R* stereoisomer, an *S* stereoisomer, or a non-stereoisomer.

37. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 35, wherein E2 is: 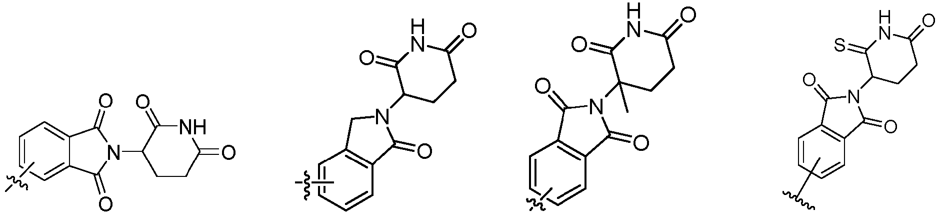 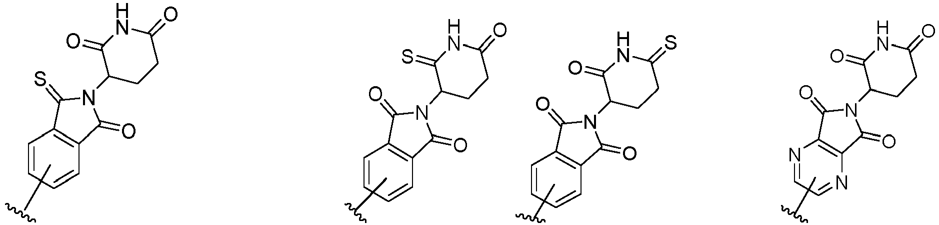 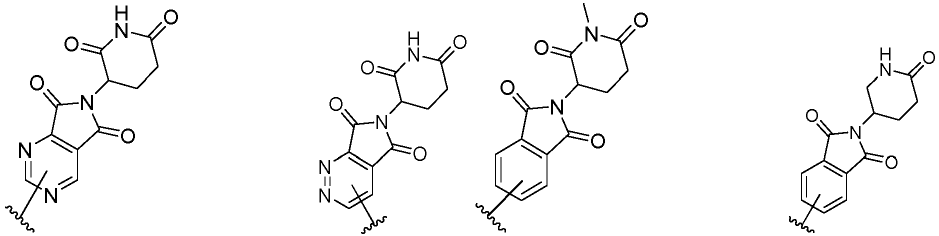 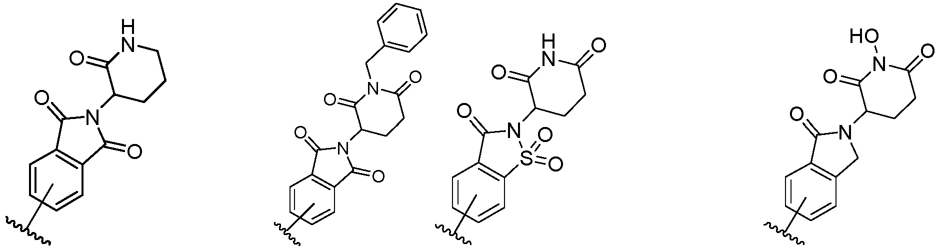 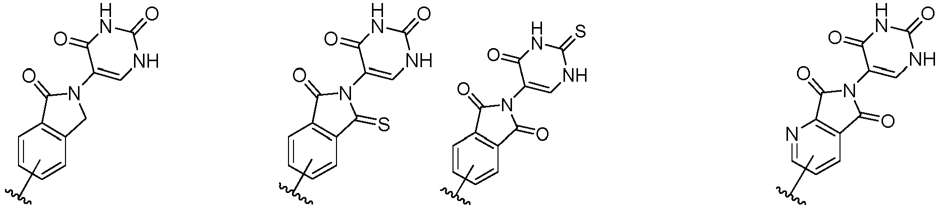 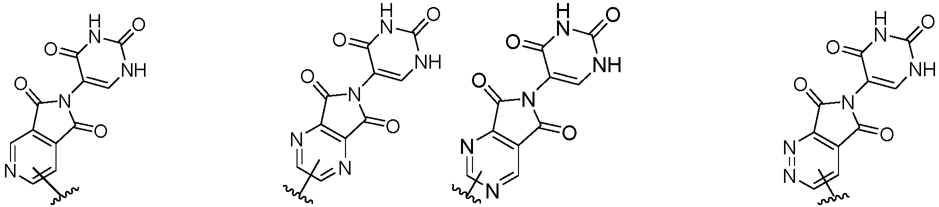 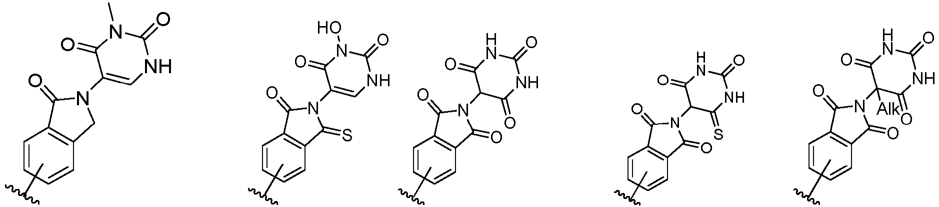 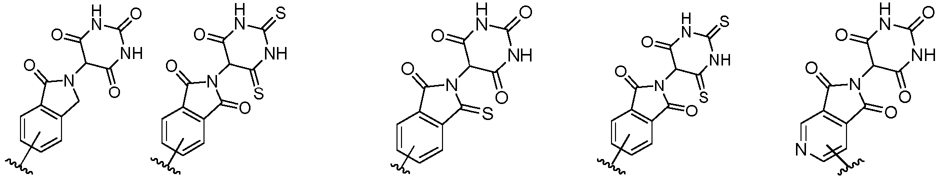 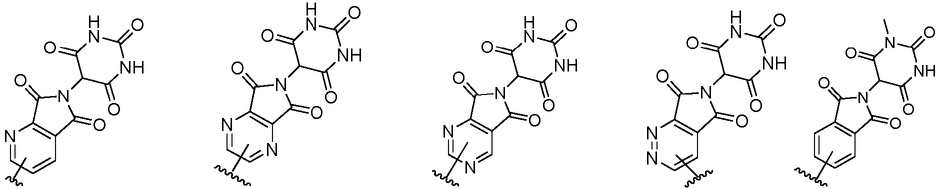 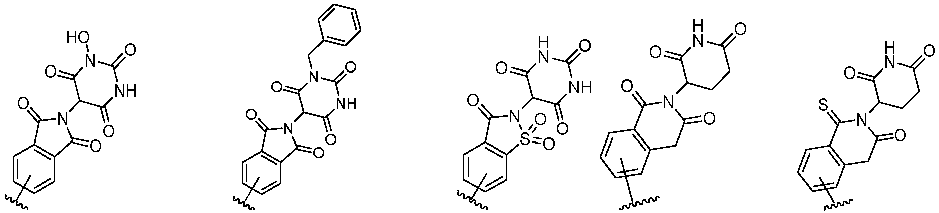 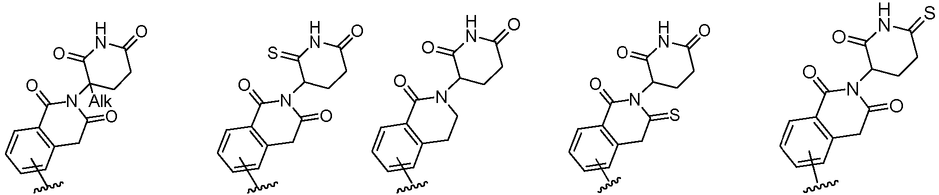 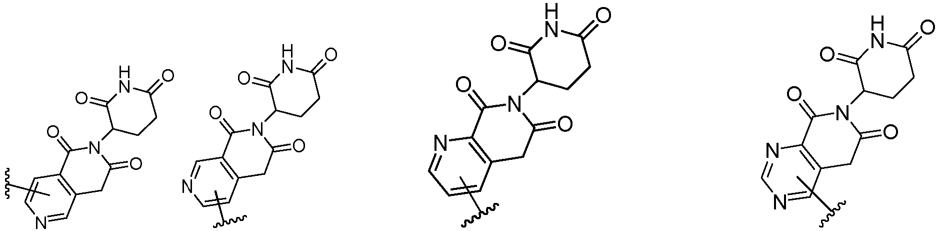 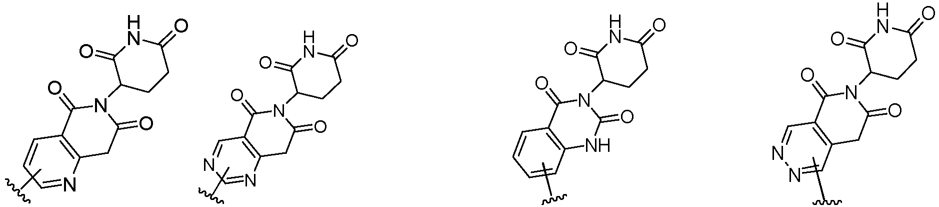 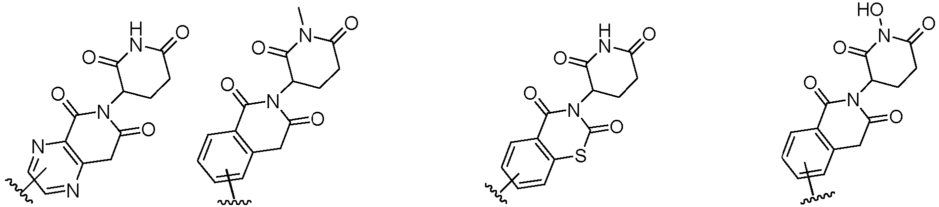 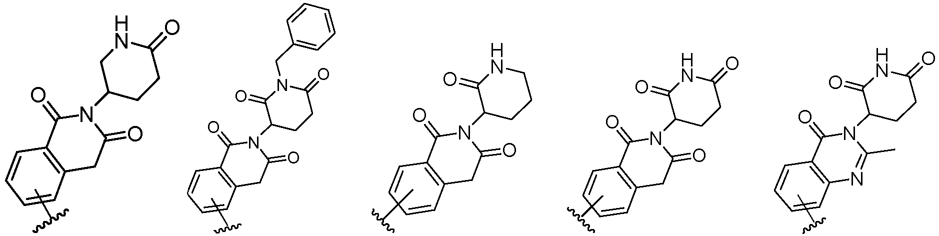 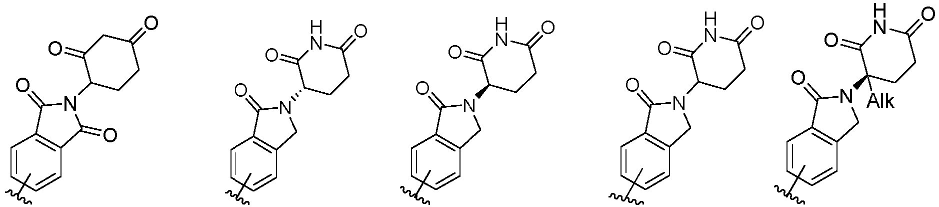 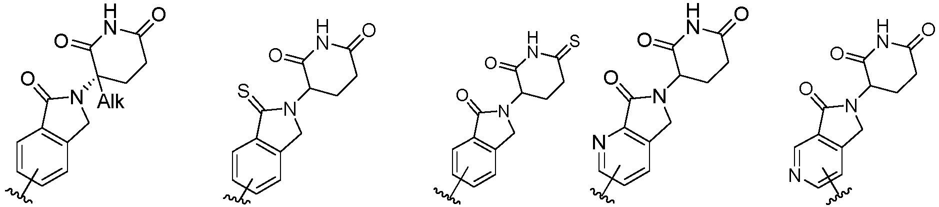 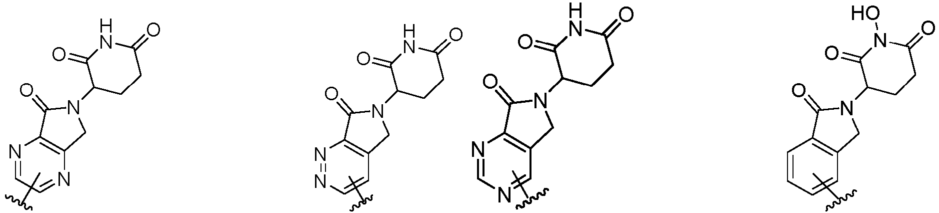 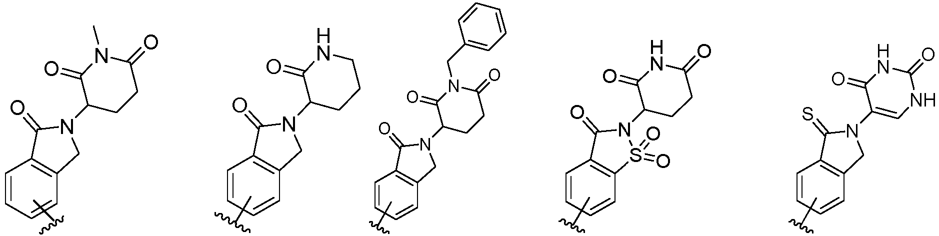 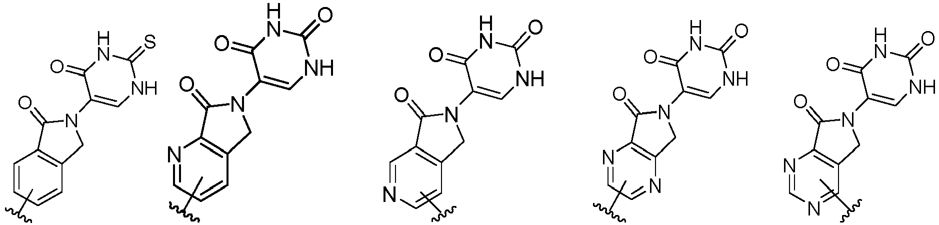 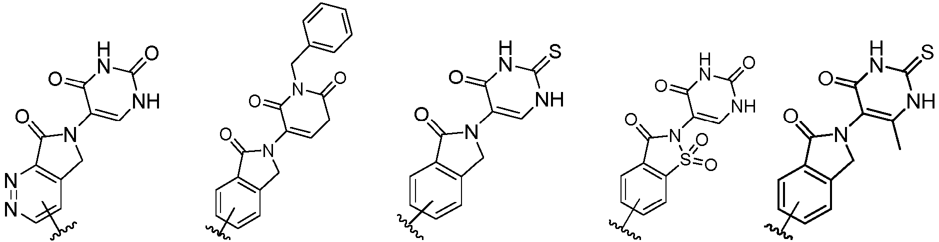 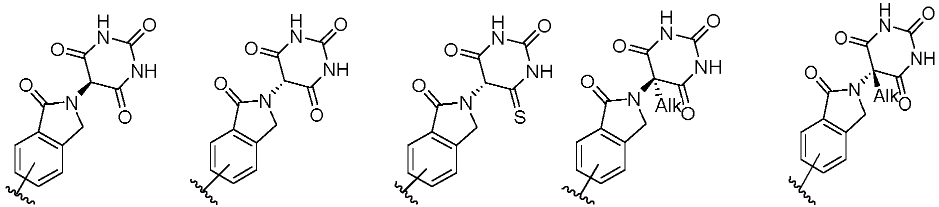 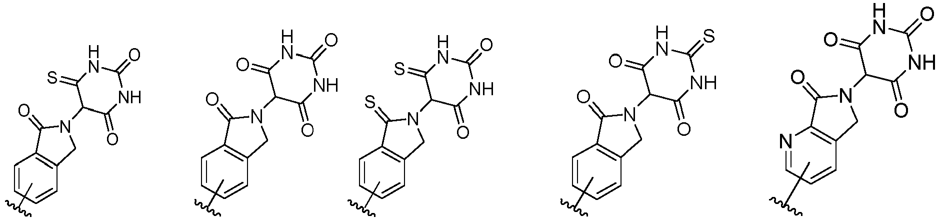 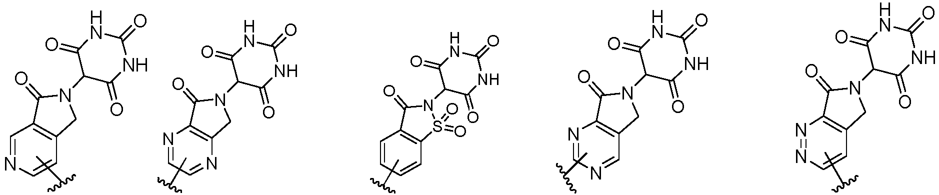 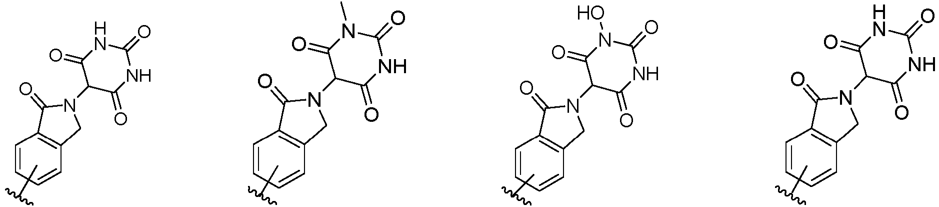 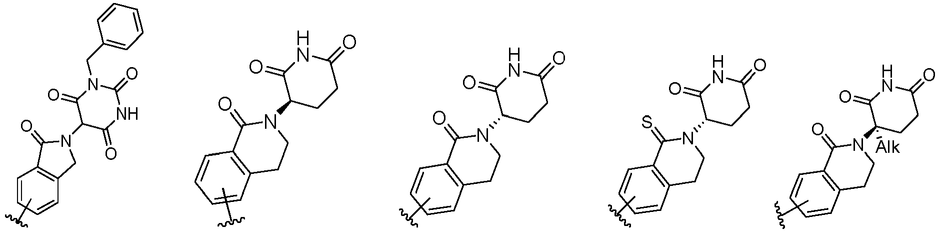 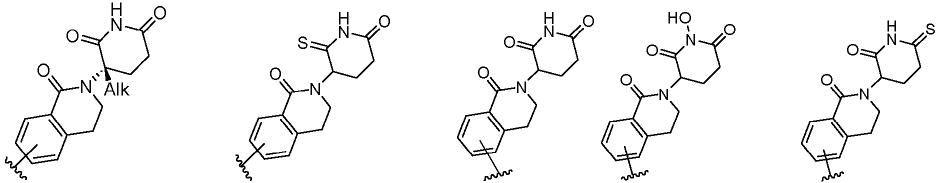 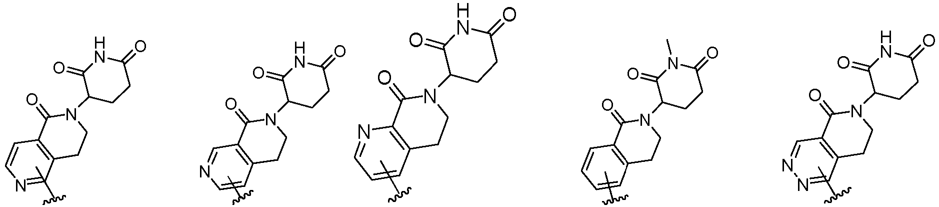 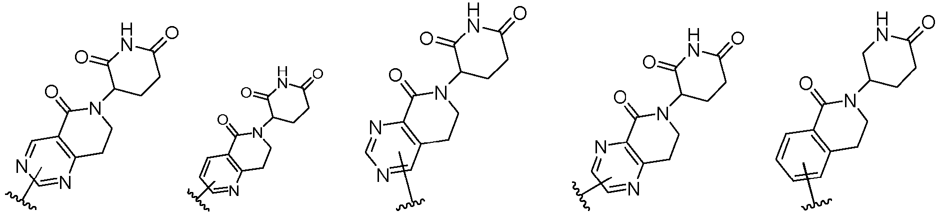 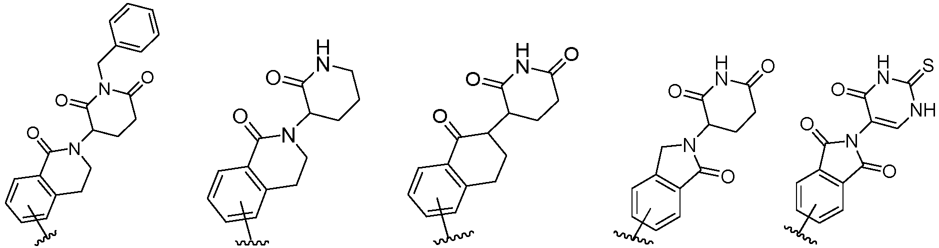 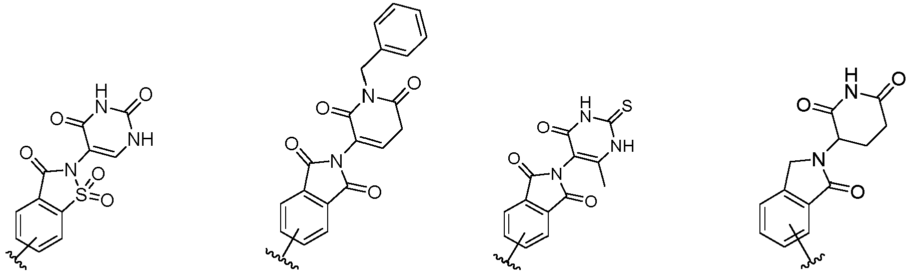 or

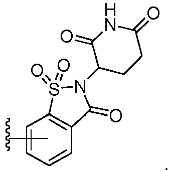

38. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein E3 has a structure of formula E3-1: 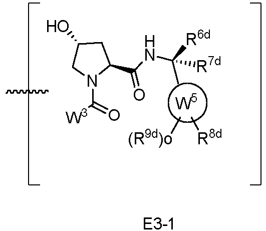
W³ is C₆-C₁₀ aryl, 5- to 10-membered heteroaryl, or 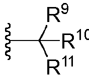
, wherein the aryl or heteroaryl is optionally substituted;
R⁹ and R¹⁰ are each independently hydrogen, C₁-C₆ alkyl, C₃-C₈ cycloalkyl, or 5- to 10-membered heteroaryl, wherein the alkyl, C₃-C₈ cycloalkyl, or 5- to 10-membered heteroaryl is optionally substituted with 1 or more -OH, halogen, or -NH₂;
or R⁹ and R¹⁰, together with the carbon atom linked thereto, form C₃-C₈ cycloalkyl, wherein the C₃-C₈ cycloalkyl is optionally substituted with -OH, halogen, -NH₂, or C₁-C₃ alkyl;
R¹¹ is C₁-C₆ alkyl, C₁-C₆ alkoxy, 3- to 8-membered heterocycloalkyl, C₆-C₁₀ aryl, 5- to 10-membered heteroaryl, or 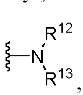 wherein the C₁-C₆ alkyl, C₁-C₆ alkoxy, 3- to 8-membered heterocycloalkyl, C₆-C₁₀ aryl, or 5- to 10-membered heteroaryl is optionally substituted with 1 or more -OH, halogen, or -NH₂; R¹² is selected from H, C(O), and substituted alkyl;
R¹³ is selected from H, C₁-C₆ alkyl, -alkyl CO-, -(cycloalkyl)alkyl CO-, -aralkyl CO-, -aryl CO-, - (heterocycloalkyl)CO-, and arylalkyl, wherein the alkyl, -alkyl CO-, -(cycloalkyl)alkyl CO-, -aralkyl CO-, - aryl CO-, -(heterocycloalkyl)CO-, or arylalkyl is optionally substituted;
R¹⁶ is H, halogen, -OH, C₁-C₆ alkyl, or C₁-C₆ alkoxy, wherein the C₁-C₆ alkyl or C₁-C₆ alkoxy is optionally substituted with halogen;
o is 1, 2, 3, or 4;
wherein R^{8d}, R^{14a}, R^{14b}, and R¹⁵ are as described and defined in claim 1.

39. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 38, wherein E3 has a structure of formula E3-1a, E3-1b, or E3-1c: 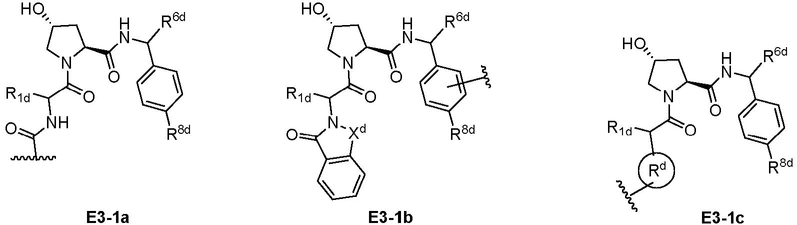 wherein,
R_{1d} is H, ethyl, isopropyl, *tert*-butyl*, sec*-butyl, cyclopropyl, cyclobutyl, cyclopentyl, cyclohexyl, alkyl, hydroxyalkyl, heteroaryl, or haloalkyl, wherein the alkyl, hydroxyalkyl, or heteroaryl is optionally substituted;
R^{6d} is H, -CH₃, -CH₂F, -CH₂OH, ethyl, isopropyl, or cyclopropyl;
R^{8d} is H, halogen, CN, OH, NO₂, C₆-C₁₀ aryl, 5- to 10-membered heteroaryl, C₁-C₆ alkyl, C₁-C₆ alkoxy, C₃-C₈ cycloalkyl, or 3- to 8-membered heterocycloalkyl, wherein the C₆-C₁₀ aryl, 5- to 10-membered heteroaryl, C₁-C₆ alkyl, C₁-C₆ alkoxy, C₃-C₈ cycloalkyl, or 3- to 8-membered heterocycloalkyl is optionally substituted with halogen, -OH, CN, NO₂, or amino;
X^{d} is CH₂ or C(O);
R^{d} is 5- to 6-membered heteroaryl, wherein the 5- to 6-membered heteroaryl is optionally substituted.

40. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to any one of claims 1, 2, 38, and 39, wherein E3 has a structure of formula E3-1aa: 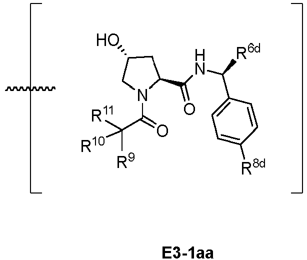 wherein,
R^{6d} is H, -CH₃, -CH₂F, -CH₂OH, ethyl, isopropyl, or cyclopropyl;
R⁹ is H;
R¹⁰ is H, ethyl, isopropyl, *tert*-butyl, *sec*-butyl, cyclopropyl, cyclobutyl, cyclopentyl, or cyclohexyl;
R¹¹ is selected from 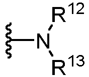 and 5- to 10-membered heteroaryl, wherein the 5- to 10-membered heteroaryl is optionally substituted with 1 or more -OH, halogen, or -NH₂;
R¹² is H or C(O);
R¹³ is H, alkyl, -alkyl CO-, -(cycloalkyl)alkyl CO-, -aralkyl CO-, -aryl CO-, -(heterocycloalkyl)CO-, or arylalkyl, wherein the alkyl, -alkyl CO-, -(cycloalkyl)alkyl CO-, -aralkyl CO-, -aryl CO-, - (heterocycloalkyl)CO-, or arylalkyl is optionally substituted;
R^{8d} is H, halogen, CN, OH, NO₂,

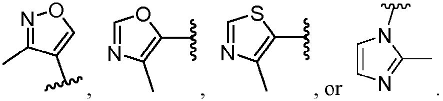

41. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to any one of claims 1, 2, and 38-40, wherein E3 is: 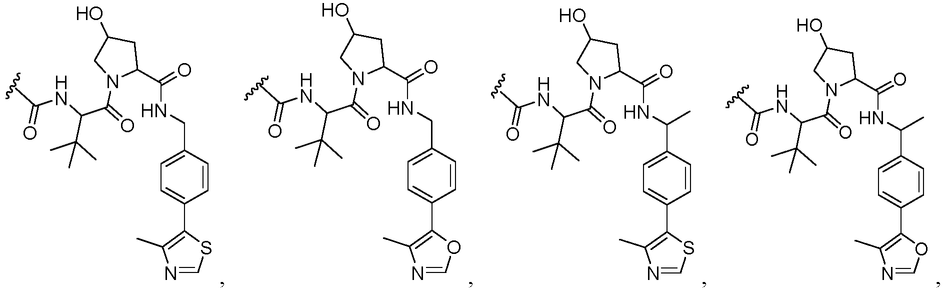 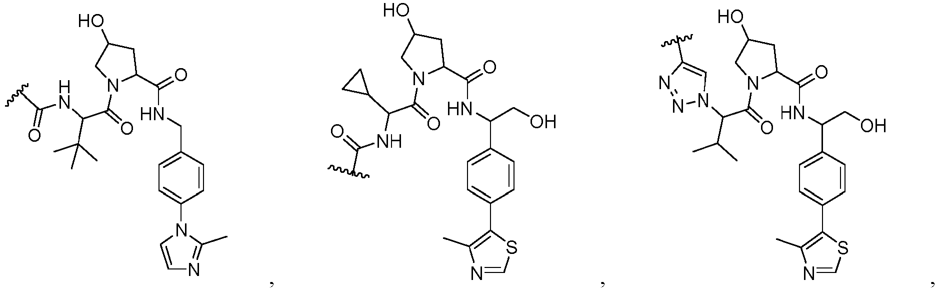 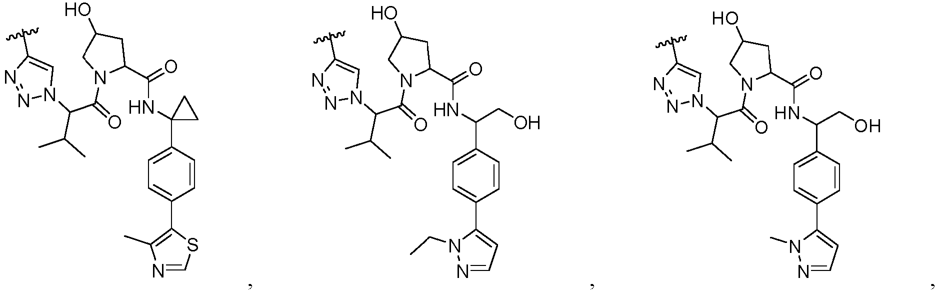 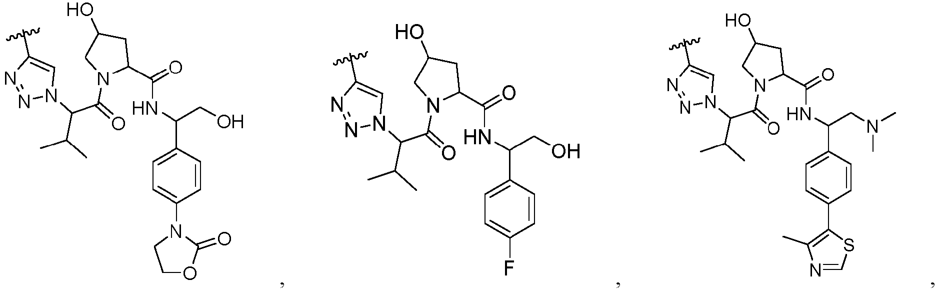 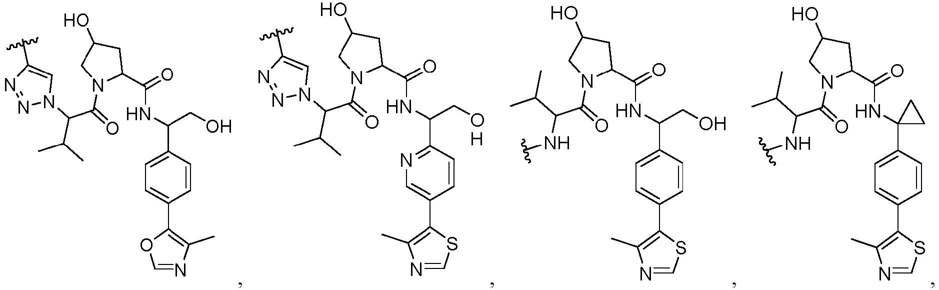 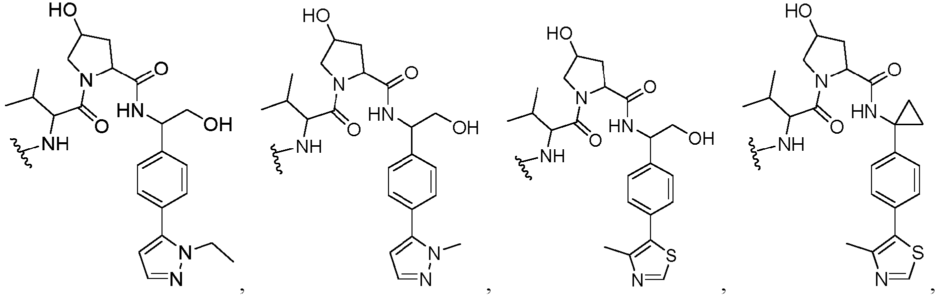 or

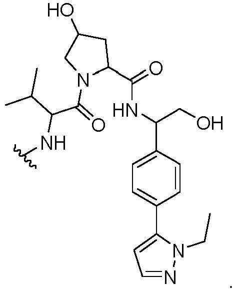

42. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 41, wherein E3 is:

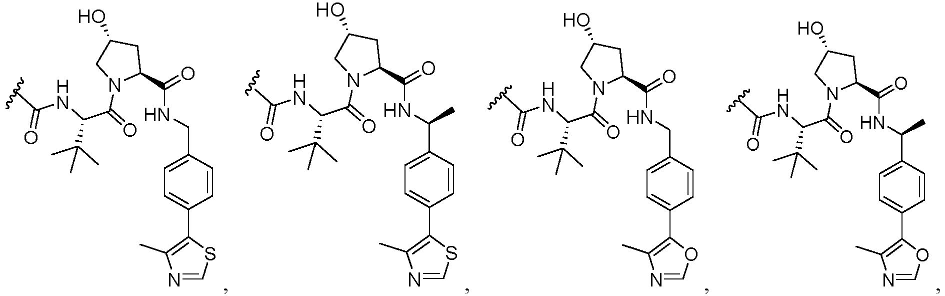

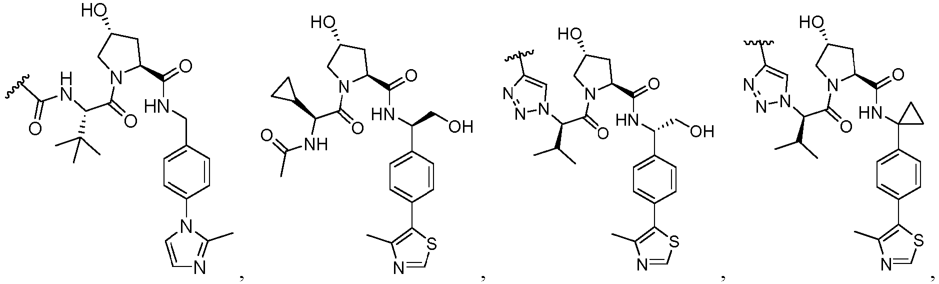

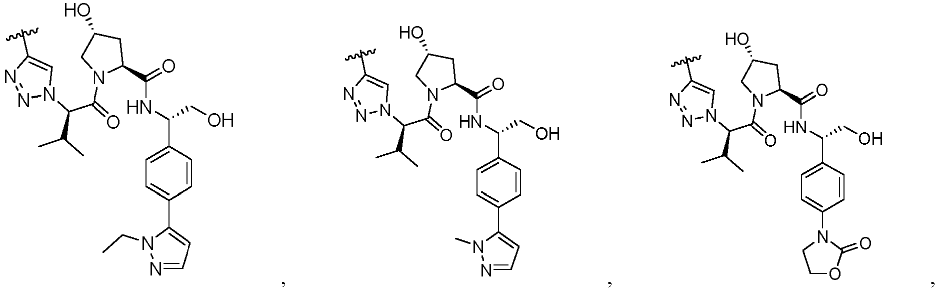

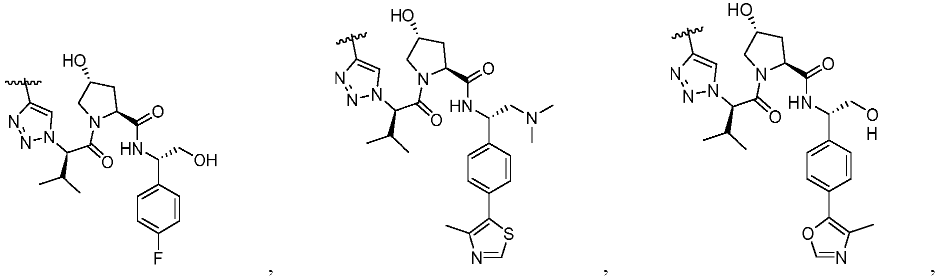

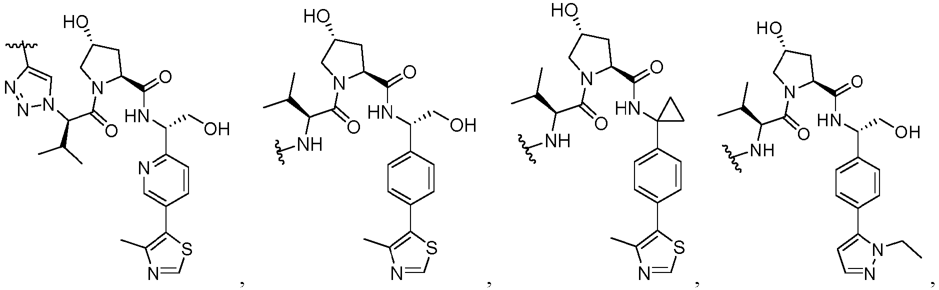

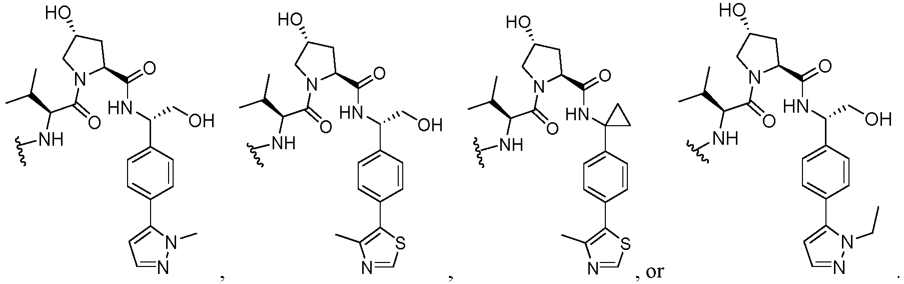

43. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein the compound of formula I or I' satisfies any one of the following conditions:
(1) K is K1-I-a1 or K' is K2-I-a1; 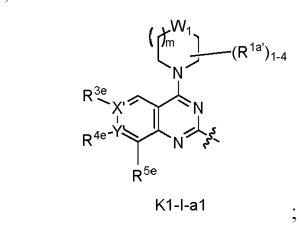
wherein in K1-I-a1, X' is C or N; Y' is C;
R^{3e} is H or absent; R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R^{1a'} is hydrogen, hydroxy, NH₂, halogen, or C₁-C₆ alkyl;
W₁ is CH₂ or O;
m is 0, 1, 2, or 3; 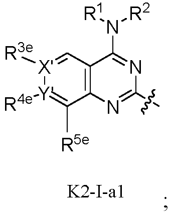
wherein in K2-I-a1, X' is C or N; Y' is C;
R^{3e} is H or absent; R^{4e} is C₆-C₁₀ aryl or 5- to 10-membered heteroaryl, wherein the C₆-C₁₀ aryl or 5- to 10-membered heteroaryl is optionally substituted with 1 or more R^{a'};
R^{a'} is independently selected from halogen, hydroxy, C₁-C₆ alkyl, C₂-C₆ alkynyl, and C₃-C₆ cycloalkyl;
R^{5e} is halogen;
R¹ and R² are independently hydrogen, C₁-C₆ alkyl, or C₃-C₈ cycloalkyl, wherein the C₁-C₆ alkyl or C₃-C₈ cycloalkyl is optionally substituted with one or more halogen, hydroxy, or amino;
L is LA-9 or LA-10; 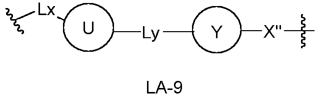
wherein in LA-9:
Lx is -(CH₂)ᵥ-, wherein one or two CH₂ contained in Lx are each independently optionally replaced by -O- or -CR_{d}Rₑ-; -CR_{d}Rₑ- is 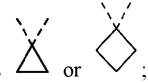 v is 1, 2, 3, 4, 5, or 6;
ring U is 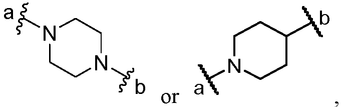 wherein end a is linked to Lx, and end b is linked to Ly;
ring Y is 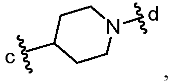 wherein end c is linked to Ly, and end d is linked to X";
Ly is -(CH₂)ₖ-, wherein one or two CH₂ contained in Ly are each independently optionally replaced by -O-, -C≡C-, or -N(C₁-C₆ alkyl)-; k is 1, 2, 3, 4, 5, or 6;
X" is -C(O)-; 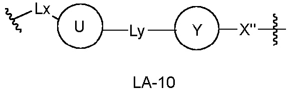
wherein in LA-10:
Lx is -(CH₂)ᵥ-, wherein one or two CH₂ contained in Lx are each independently optionally replaced by -O- or -CR_{d}Rₑ-; -CR_{d}Rₑ- is 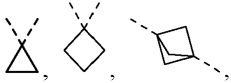 -CH(CH₃)-, -CH(OCH₃)-, -CH(OH)-, or -CH(NH₂)-; v is 1, 2, 3, 4, 5, or 6;
ring U is 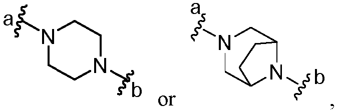 wherein end a is linked to Lx, and end b is linked to Ly;
ring Y is 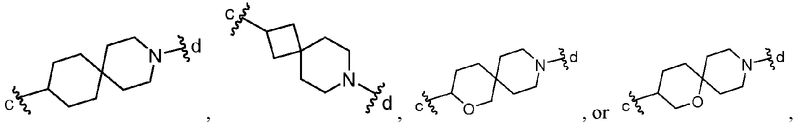
wherein end c is linked to Ly, and end d is linked to X";
Ly is -(CH₂)ₖ-, and k is 1, 2, 3, 4, 5, or 6;
X" is -C(O)-;
E is E1-1h" or E2-1aa; 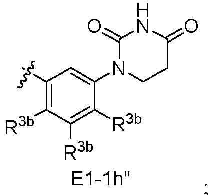
wherein in E1-1h", each R^{3b} is independently hydrogen, halogen, C₁-C₆ alkyl, or -O(C₁-C₆ alkyl), the C₁-C₆ alkyl and -O(C₁-C₆ alkyl) being optionally substituted with deuterium, halogen, cyano, -OH, or -NH₂, and the number of substitution is 1, 2, or 3; 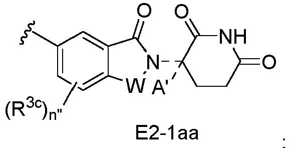
wherein in E2-1aa,
W is CH₂ or C(O);
A' is hydrogen, methyl, Cl, or F;
each R^{3c} is independently selected from hydrogen, hydroxy, NH₂, C₁-C₆ alkyl, and C₁-C₆ alkoxy; n" is 1, 2, 3, or 4;
----- is a bond, which is an R stereoisomer, an S stereoisomer, or a non-stereoisomer;
(2) the compound of formula I or I' is the following compound:

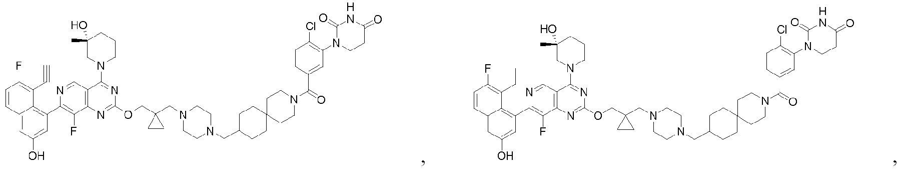

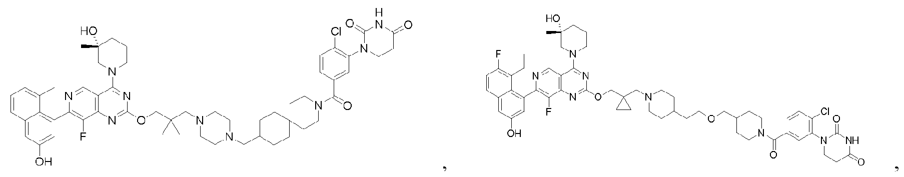

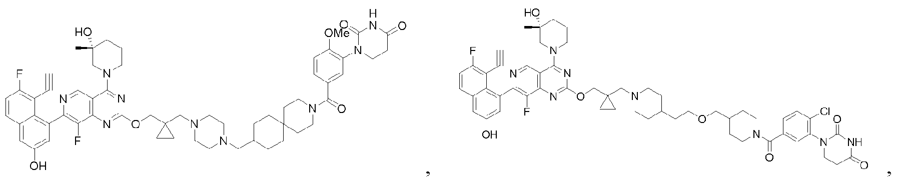

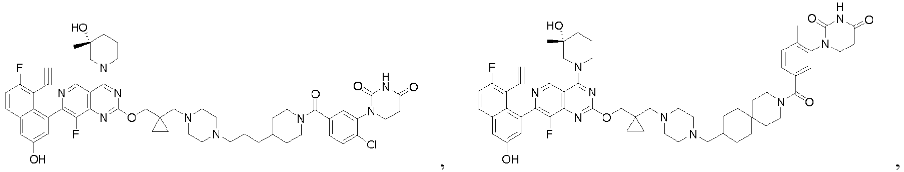

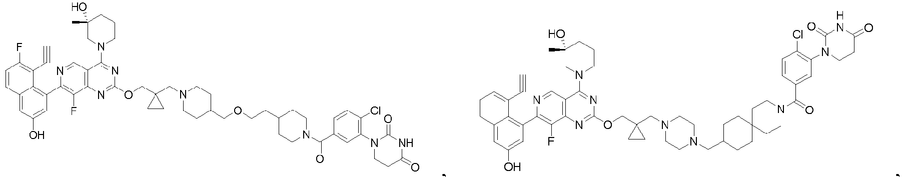

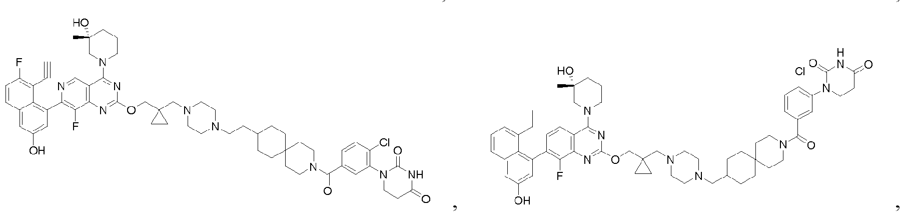

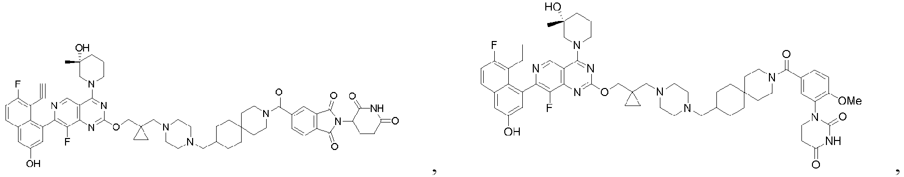

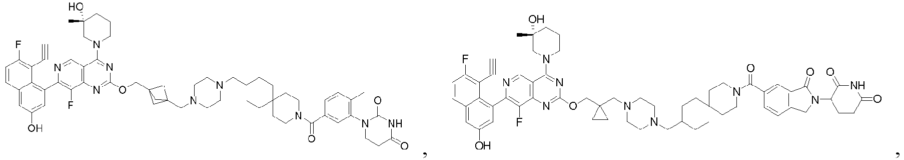

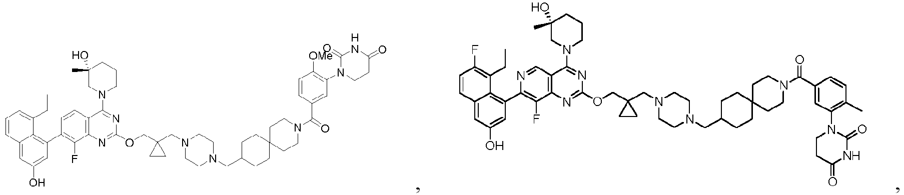

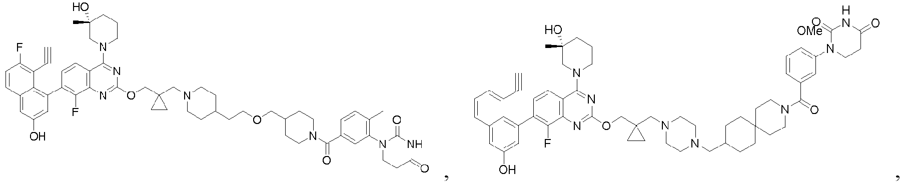

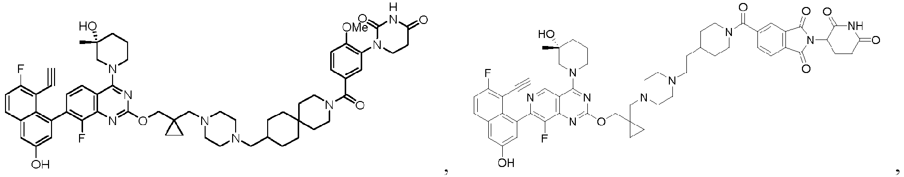

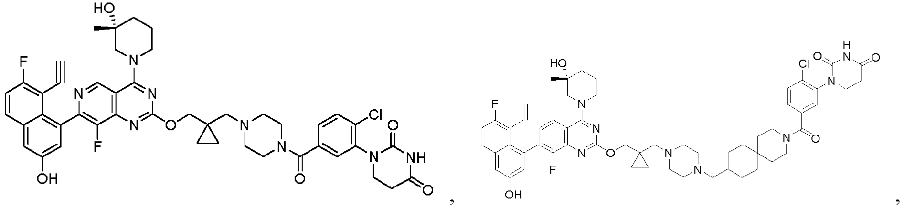

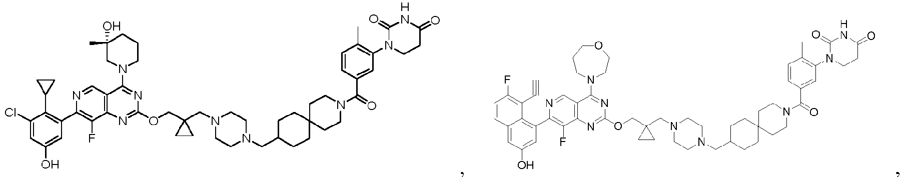

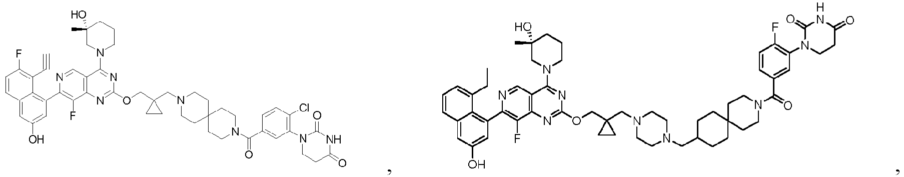

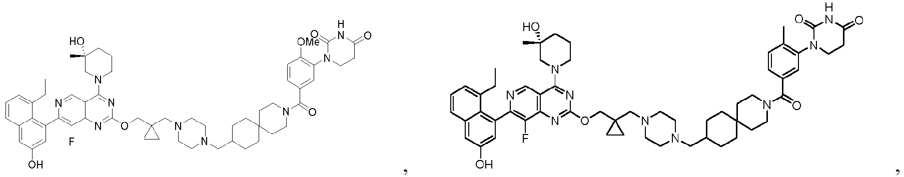

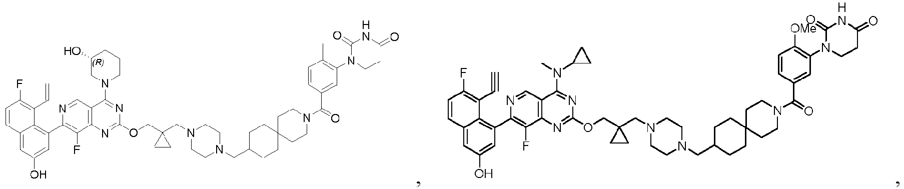

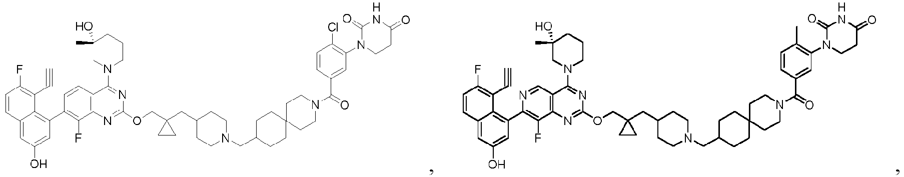

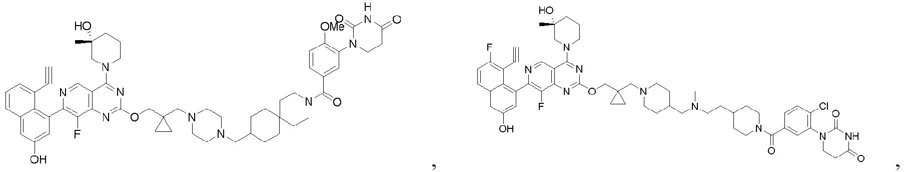

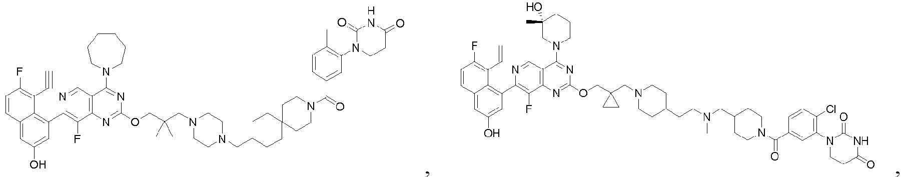

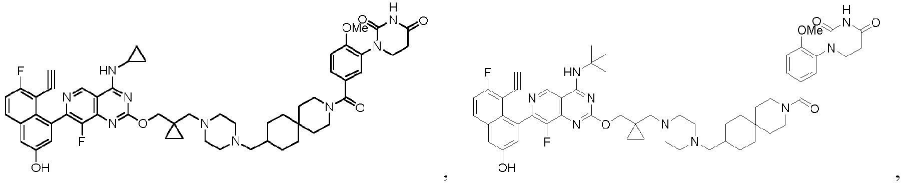

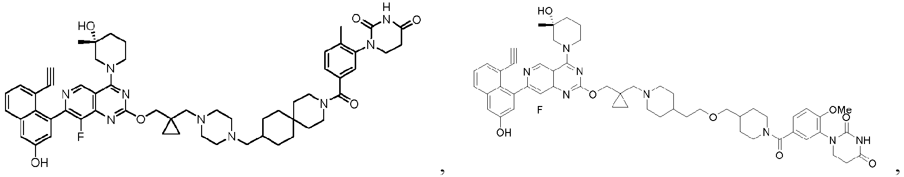

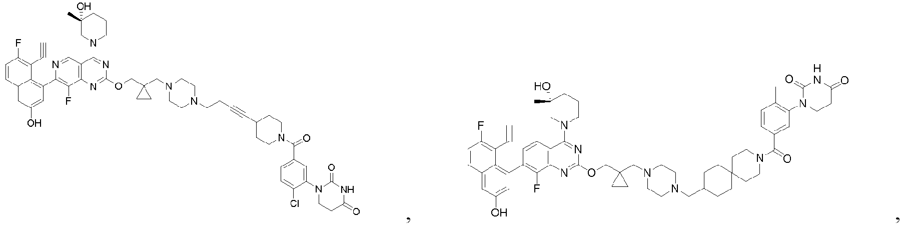

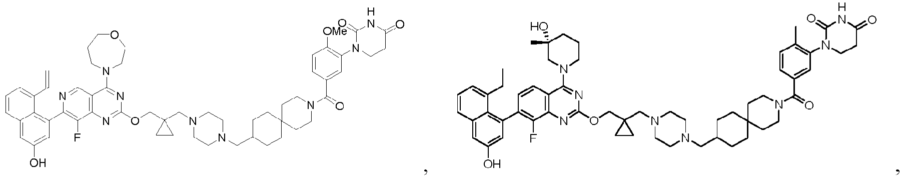

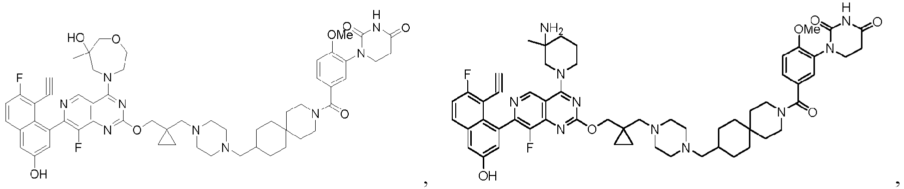

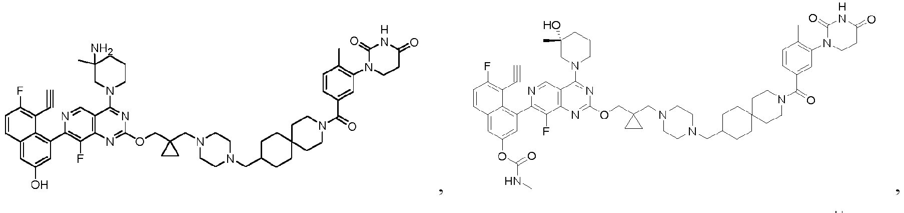

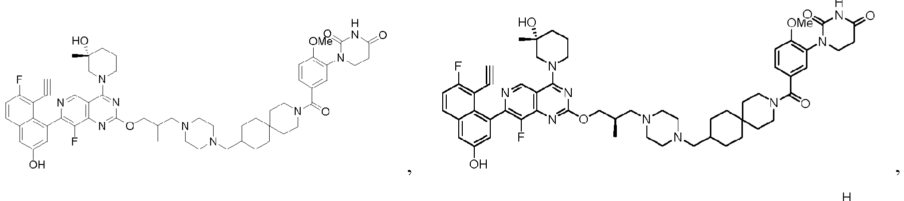

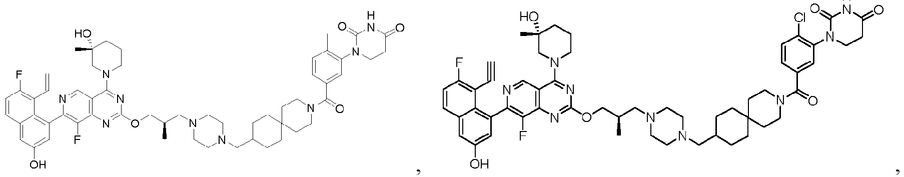

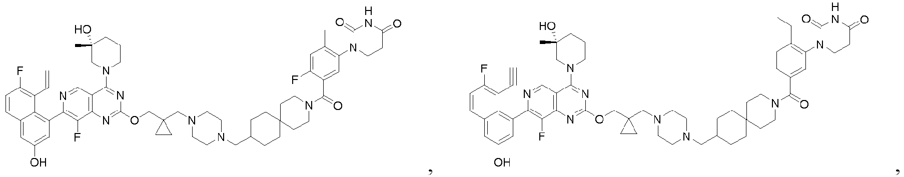

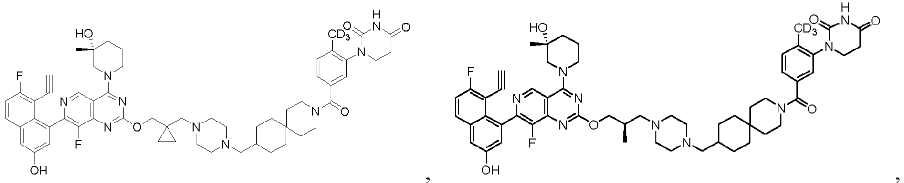

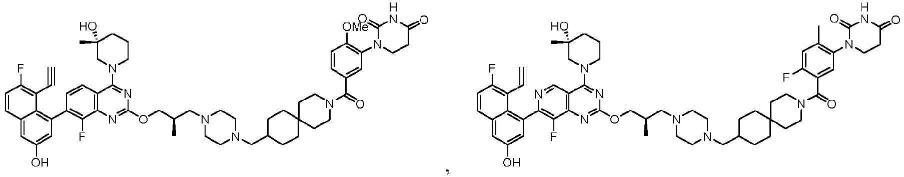

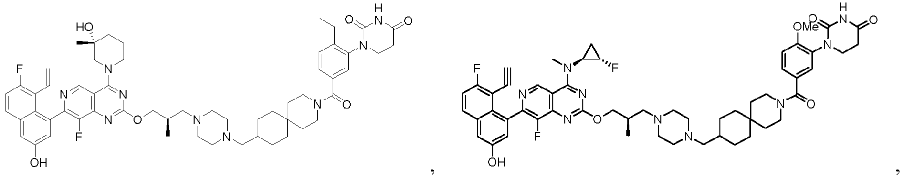

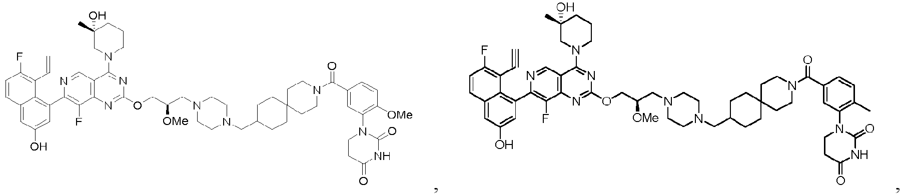

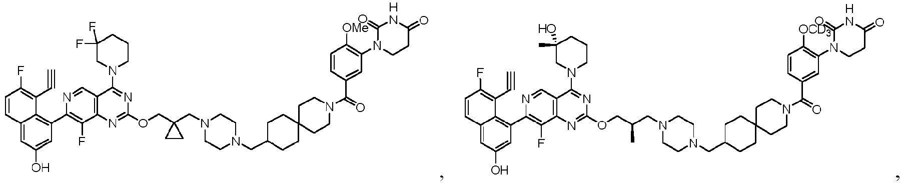

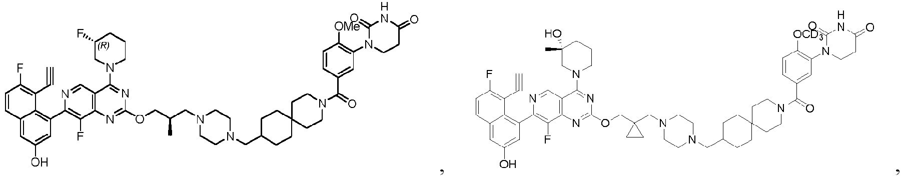

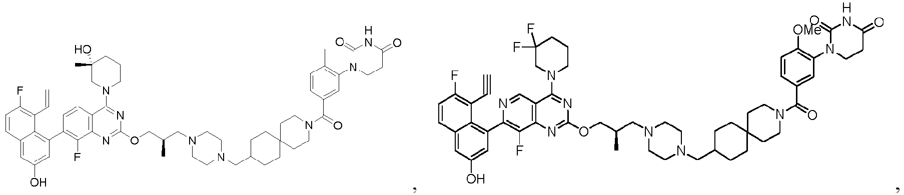

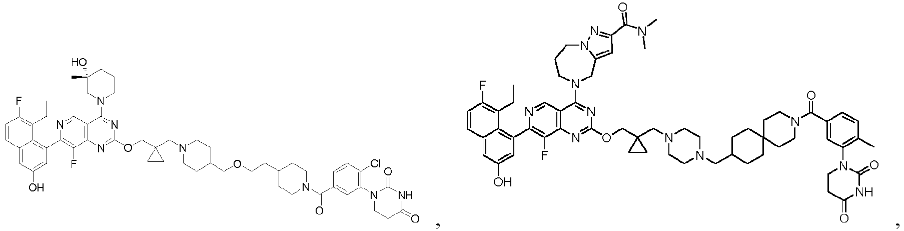

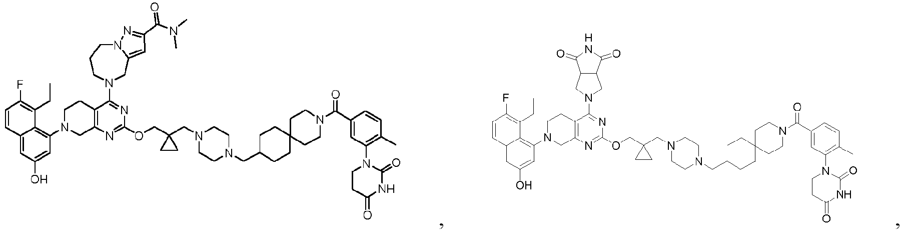

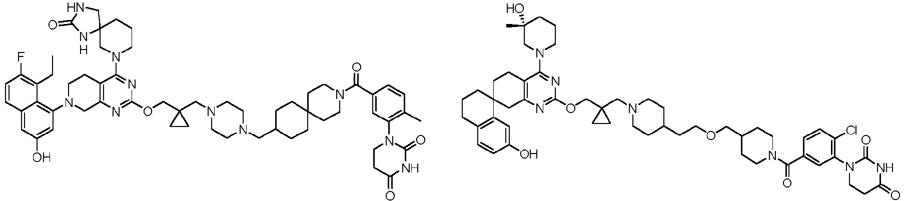

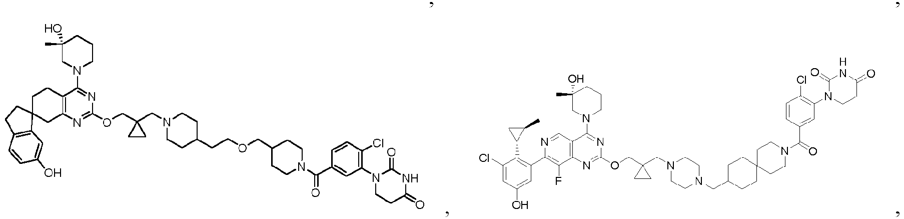

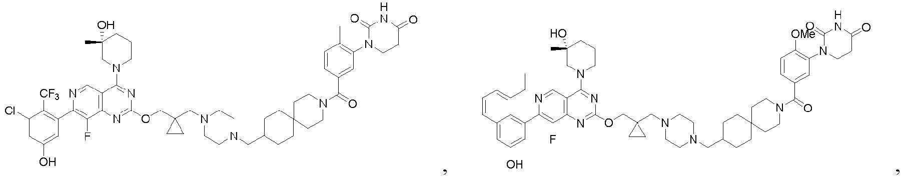

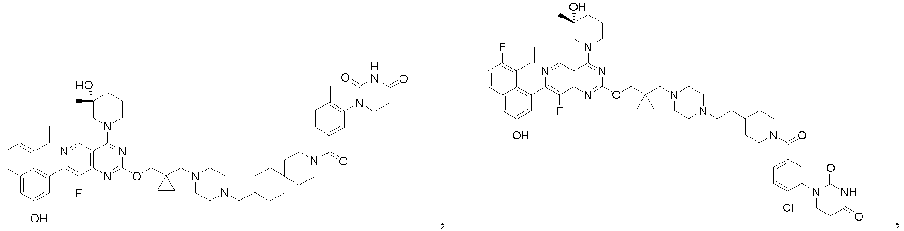

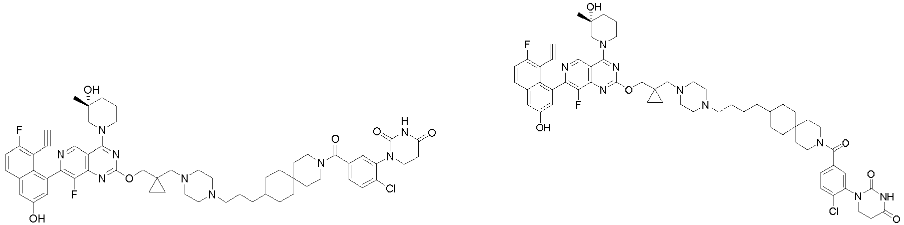

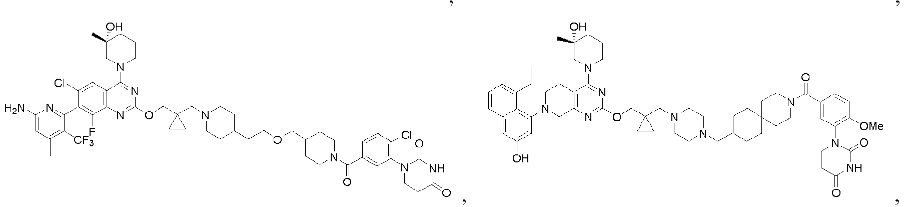

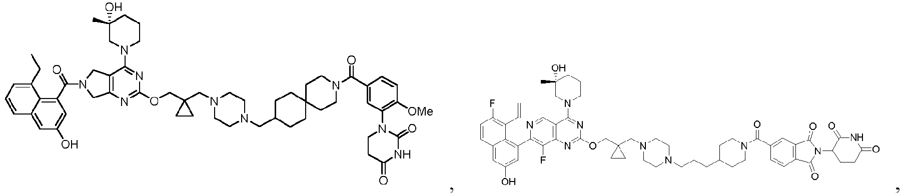

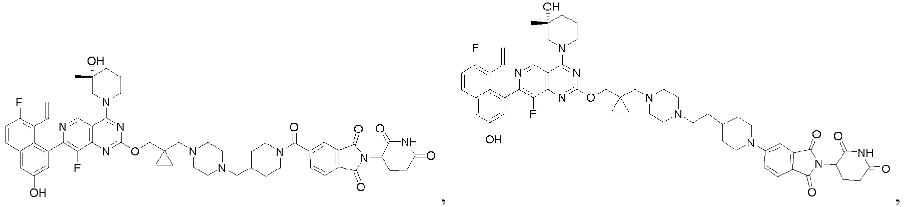

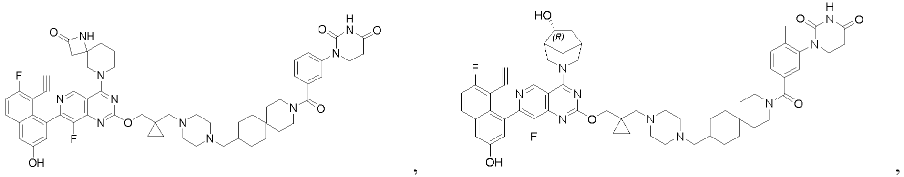

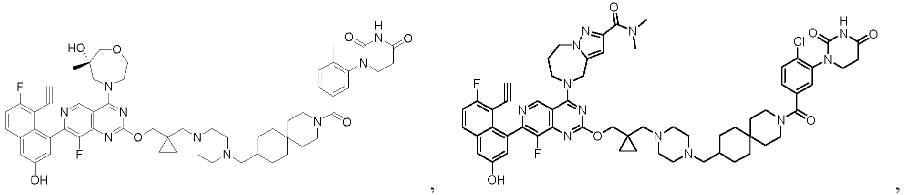

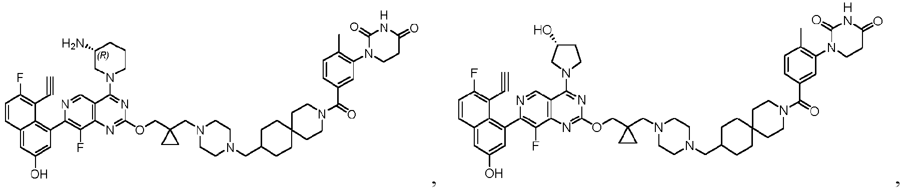

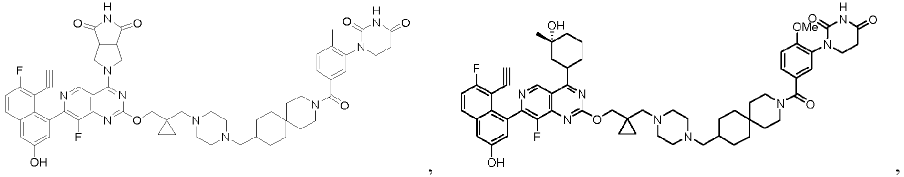

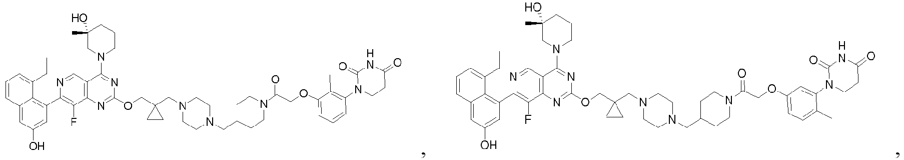

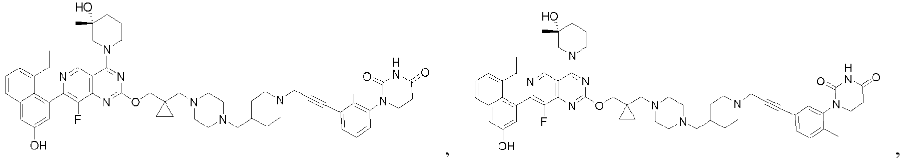

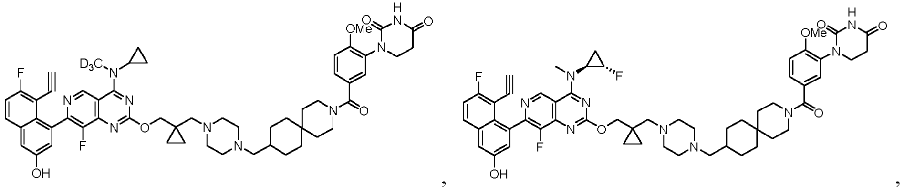

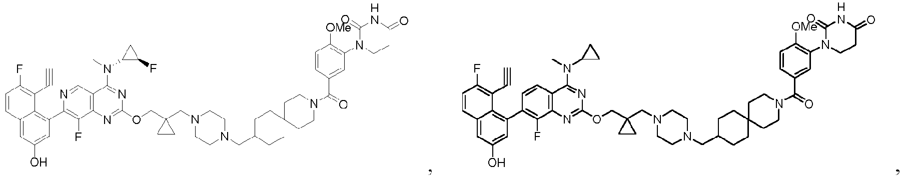

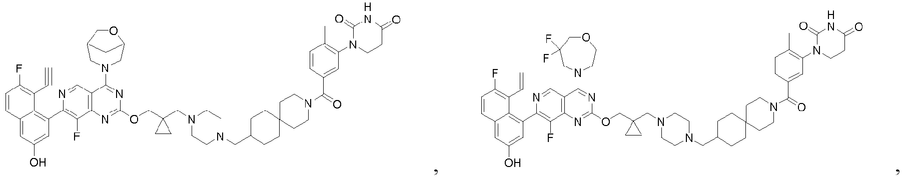

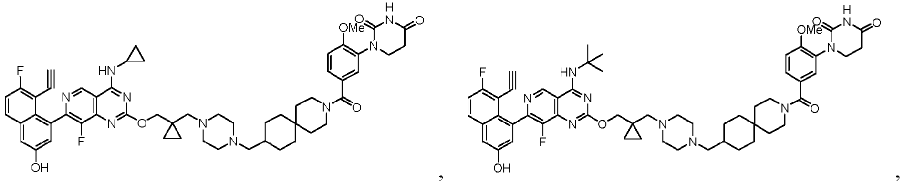

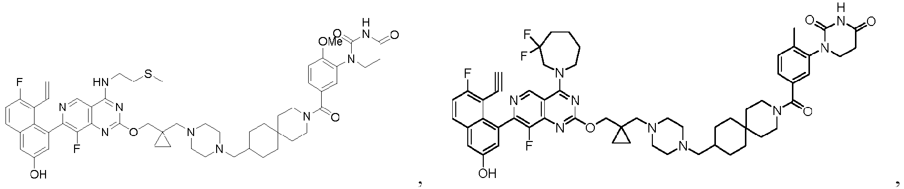

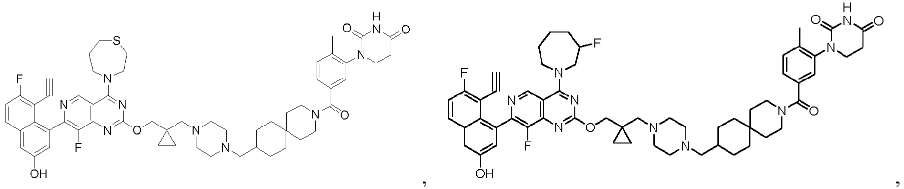

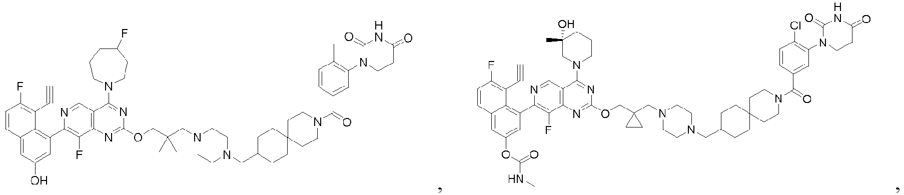

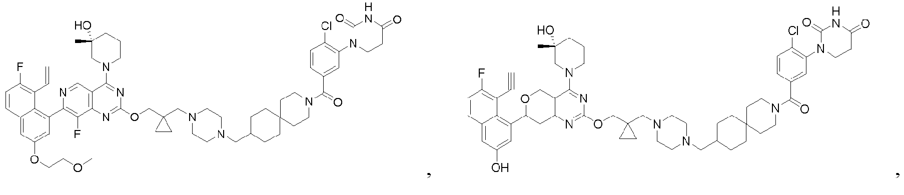

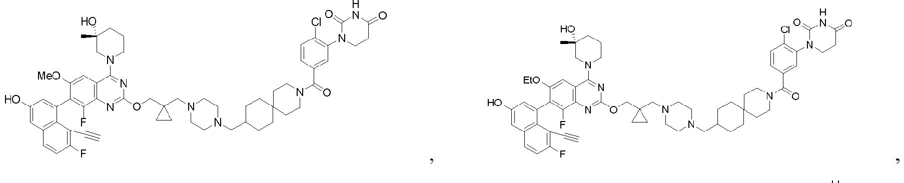

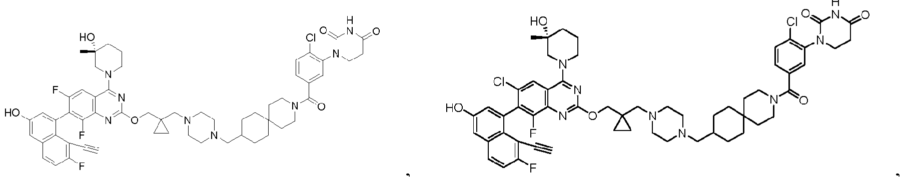

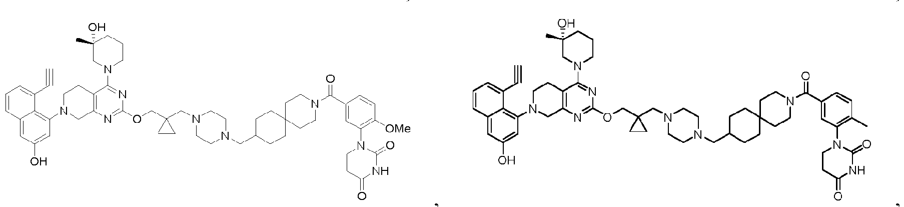

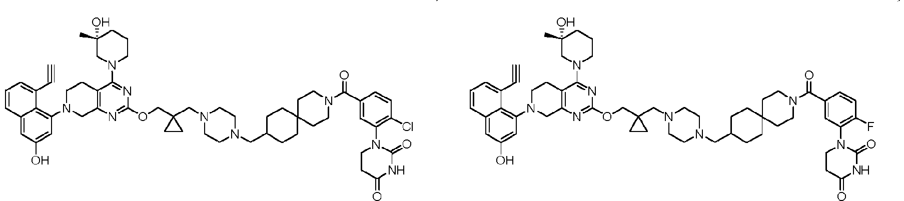

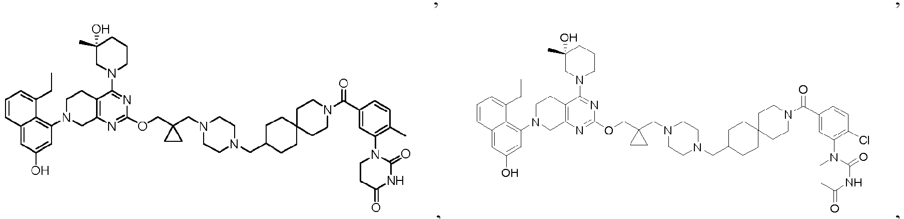

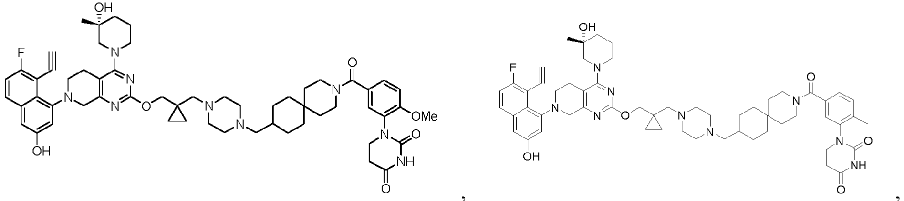

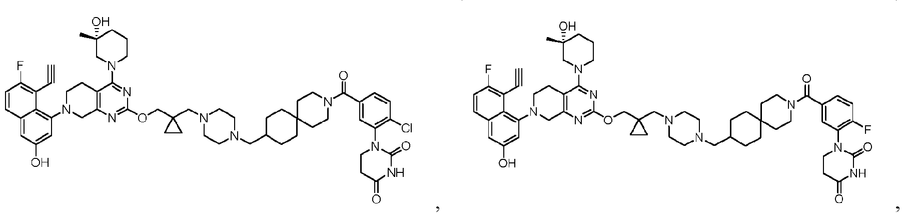

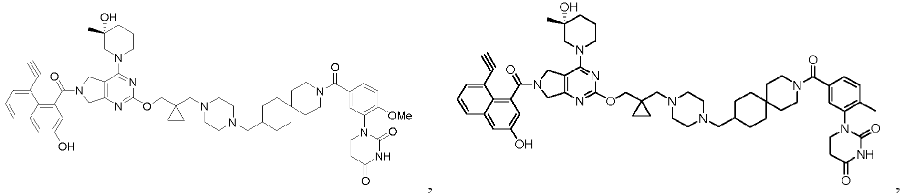

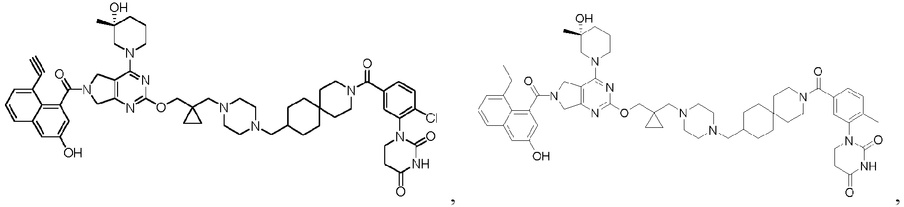

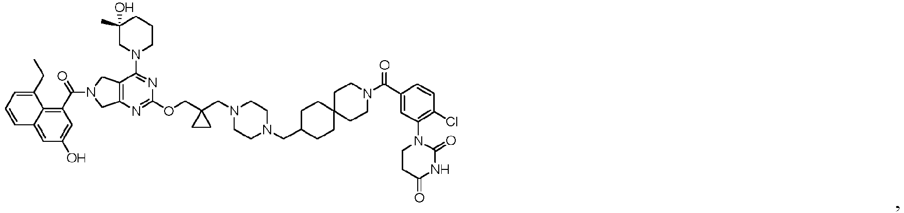

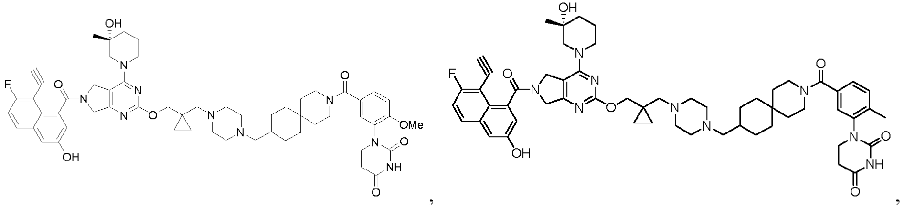

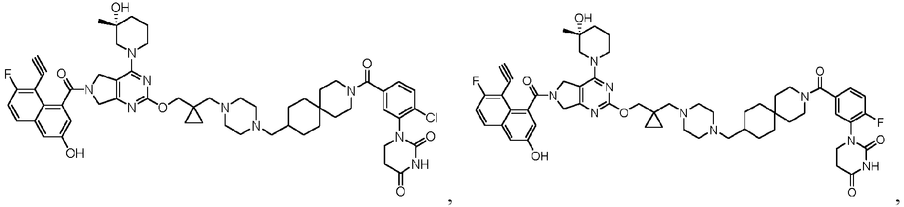

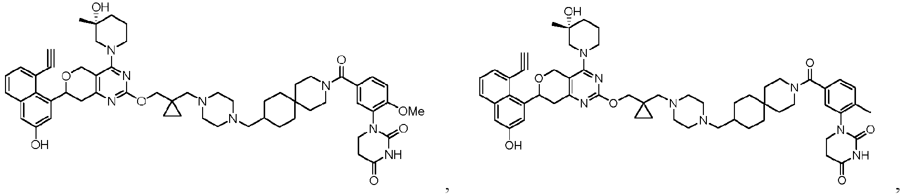

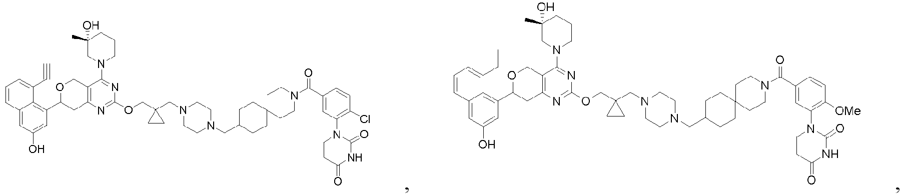

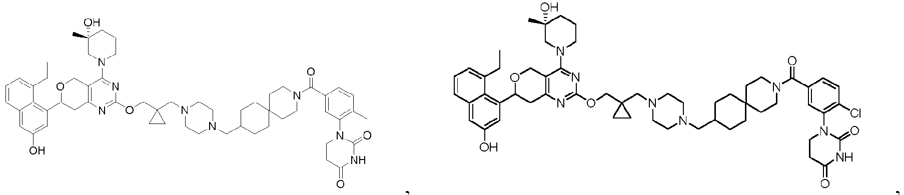

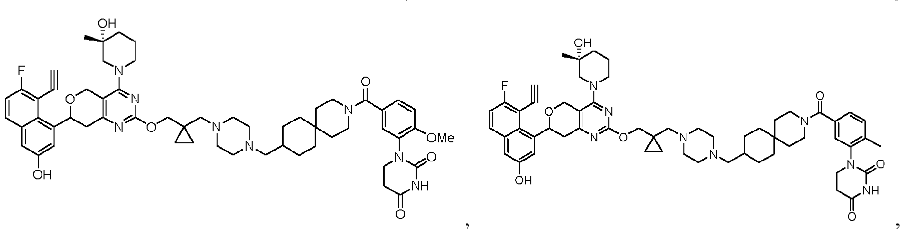

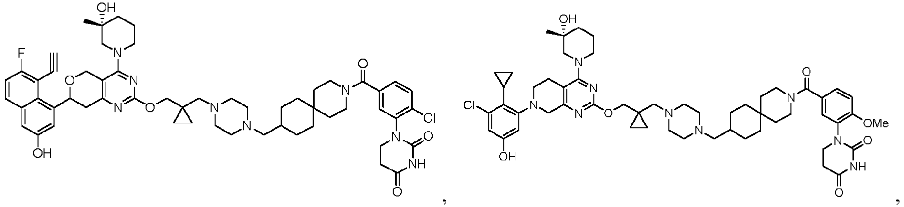

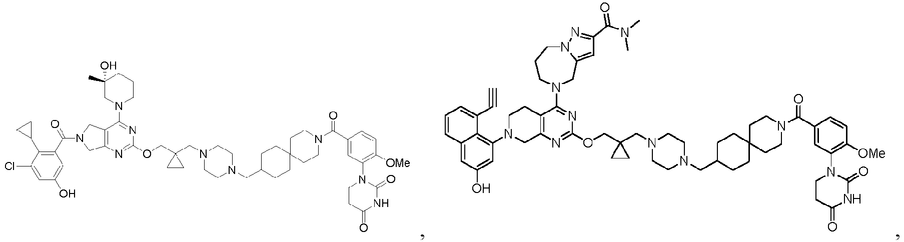

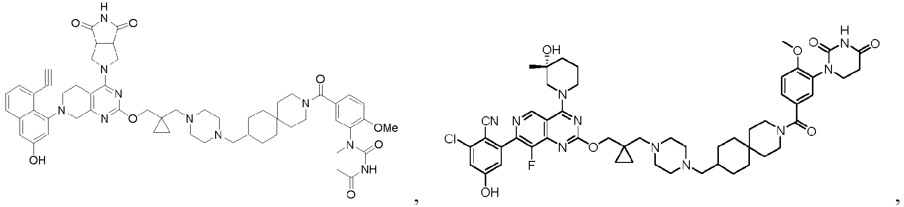

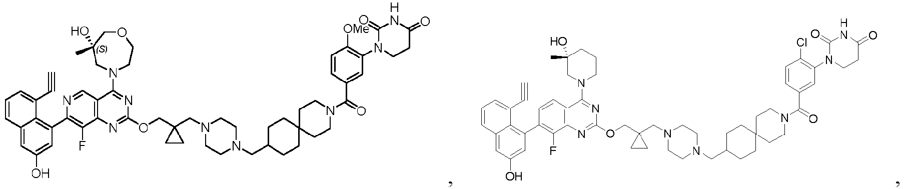

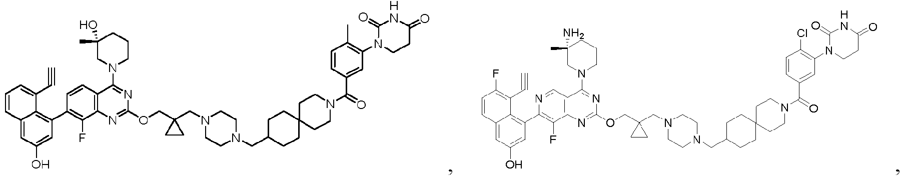

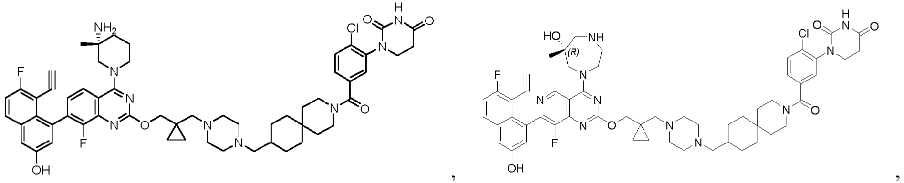

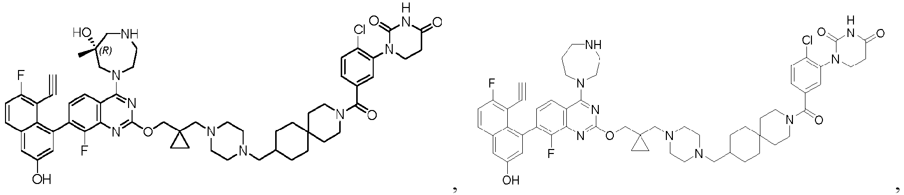

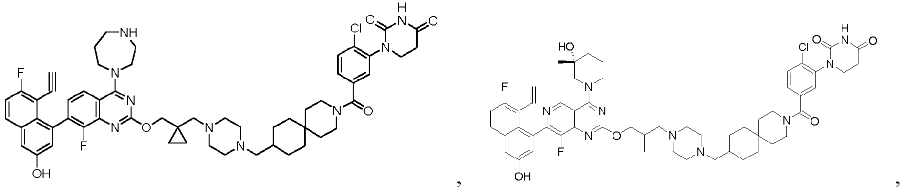

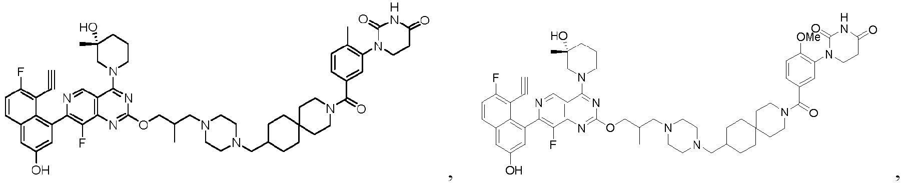

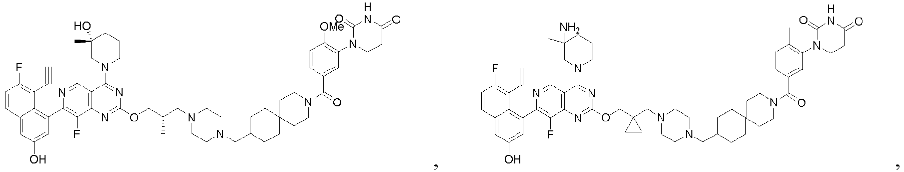

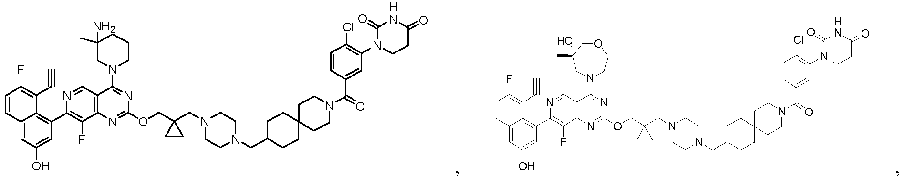

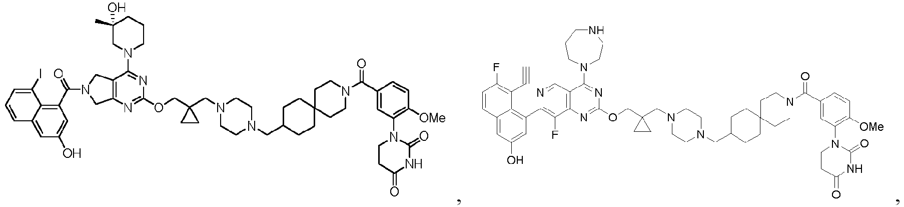

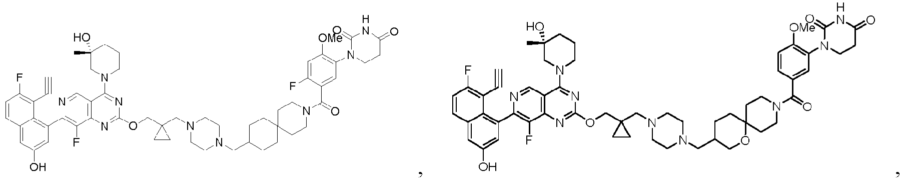

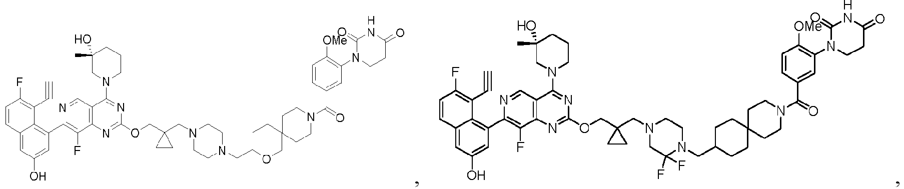

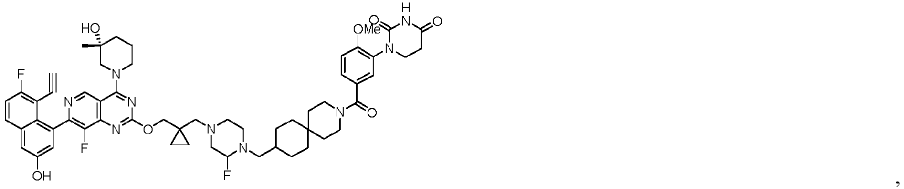

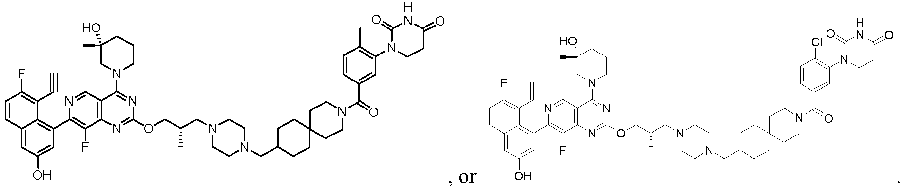

44. A pharmaceutical composition, comprising the compound, and/or the stereoisomer, the enantiomer, the diastereomer, the atropisomer, the deuteride, the hydrate, the solvate or the prodrug thereof and/or the pharmaceutically acceptable salt thereof according to any one of claims 1-43, and a pharmaceutically acceptable carrier, diluent, or excipient.

45. Use of substance X in preparing a medicament, wherein substance X is the compound, and/or the stereoisomer, the enantiomer, the diastereomer, the atropisomer, the deuteride, the hydrate, the solvate or the prodrug thereof and/or the pharmaceutically acceptable salt thereof according to any one of claims 1-43, or the pharmaceutical composition according to claim 44 in preparing a medicament for the treatment or prevention of a KRAS-mediated disease or disorder or cancer.

46. The use according to claim 45, wherein the cancer is selected from:
heart: sarcoma (angiosarcoma, fibrosarcoma, rhabdomyosarcoma, and liposarcoma), myxoma, rhabdomyoma, fibroma, lipoma, and teratoma;
lung: bronchial cancer (squamous cell carcinoma, undifferentiated small cell carcinoma, undifferentiated large cell carcinoma, and adenocarcinoma), bronchioloalveolar carcinoma (bronchiolar carcinoma), bronchial adenoma, sarcoma, lymphoma, chondroma, hamartoma, mesothelioma, lung cancer, and small cell lung cancer;
gastrointestinal tract: esophagus cancer (squamous cell carcinoma, adenocarcinoma, leiomyosarcoma, and lymphoma), stomach (cancer, lymphoma, and leiomyosarcoma), pancreas (ductal adenocarcinoma, insulinoma, glucagonoma, gastrinoma, carcinoid tumor, and vipoma), small intestine (adenocarcinoma, lymphoma, carcinoid tumor, Kaposi's sarcoma, leiomyoma, hemangioma, lipoma, neurofibroma, and fibroma), large intestine (adenocarcinoma, tubular adenoma, villous adenoma, hamartoma, and leiomyoma), appendiceal cancer, gastrointestinal neuroendocrine tumor, esophageal gastric cancer, anal cancer, and gastrointestinal stromal tumor;
genitourinary system: kidney (adenocarcinoma, embryonal carcinosarcoma (Wilms' tumor), lymphoma, and leukemia), bladder and urinary tract (squamous cell carcinoma, transitional cell carcinoma, and adenocarcinoma), prostate (adenocarcinoma and sarcoma), testis (seminoma, teratoma, embryonal carcinoma, teratoma, choriocarcinoma, sarcoma, interstitial cell carcinoma, fibroma, fibroadenoma, adenomatoid tumor, and lipoma), germinal cell tumor, bladder cancer, and prostate cancer;
liver: liver cancer (hepatocellular carcinoma), intrahepatic cholangiocarcinoma, hepatoblastoma, malignant hemangioendothelioma, hepatocellular adenoma, and hemangioma;
biliary tract: cholangiocarcinoma, gallbladder cancer, ampullary cancer, intrahepatic cholangiocarcinoma, and hepatobiliary cancer;
bone: osteogenic sarcoma (osteosarcoma), fibrosarcoma, malignant fibrous histiocytoma, chondrosarcoma, Ewing's sarcoma, malignant lymphoma (reticulosarcoma), multiple myeloma, malignant giant cell tumor, chordoma, chondroma (osteochondral exostosis), benign chondroma, chondroblastoma, chondromyxoid fibroma, osteoid osteoma, giant cell tumor, and bone cancer;
nervous system: skull (osteoma, hemangioma, granuloma, xanthoma, and osteitis deformans), meninges (meningioma, meningosarcoma, and glioma), brain (astrocytoma, medulloblastoma, glioma, ependymoma, germinal cell tumor (pinealoma), glioblastoma multiforme, oligodendroglioma, neurilemmoma, eye cancer, and congenital tumor), spinal neurofibroma, meningioma, glioma, and sarcoma);
gynaecology and obstetrics: uterus (endometrial cancer (serous cystadenocarcinoma, mucinous cystadenocarcinoma, and unclassified cancer), granulosa theca cell tumor, ovarian Sertoli-Leydig cell tumor, dysgerminoma, and malignant teratoma), vulva (squamous cell carcinoma, intraepithelial carcinoma, adenocarcinoma, fibrosarcoma, and melanoma), vagina (clear cell carcinoma, squamous cell carcinoma, botulinum sarcoma (embryonal rhabdomyosarcoma), fallopian tube (cancer), cervical cancer, and endometrioid carcinoma;
hematology: blood (myeloid leukemia (acute and chronic), acute lymphoblastic leukemia, chronic lymphocytic leukemia, myeloproliferative disease, multiple myeloma, and myelodysplastic syndrome), Hodgkin's disease, and non-Hodgkin's lymphoma (malignant lymphoma);
skin: malignant melanoma, basal cell carcinoma, squamous cell carcinoma, Kaposi's sarcoma, dysplastic nevus, lipoma, hemangioma, dermatofibroma, keloid, psoriasis, skin cancer, and non-melanoma; and
adrenal gland: neuroblastoma.

47. The use according to claim 45, wherein the cancer is a cancer associated with KRAS G12A, KRAS G12C, KRAS G12D, KRAS G12R, KRAS G12S, KRAS G12V, KRAS G13D, or KRAS Q61H protein mutation.

48. The use according to claim 47, wherein the KRAS G12A-associated cancer is non-small cell lung cancer, ovarian cancer, or colorectal cancer; the KRAS G12C-associated cancer is non-small cell lung cancer, colorectal cancer, or pancreatic cancer; the KRAS G12D-associated cancer is biliary tract cancer, endometrial cancer, pancreatic cancer, colorectal cancer, non-small cell lung cancer, ovarian cancer, or rectal cancer; the KRAS G12R-associated cancer is pancreatic cancer or non-small cell lung cancer; the KRAS G12S-associated cancer is rectal cancer, intestine adenocarcinoma, or colorectal cancer; the KRAS G12V-associated cancer is pancreatic cancer, colorectal cancer, non-small cell lung cancer, or ovarian cancer; the KRAS G13D-associated cancer is colorectal cancer.

49. A compound of formula S-1, S-2, S-3, or S-4: 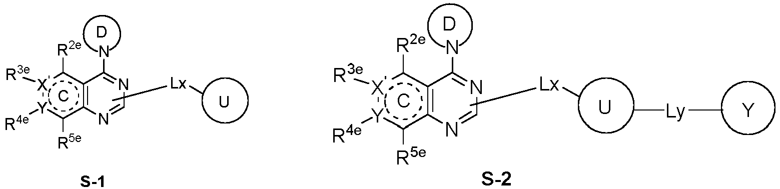 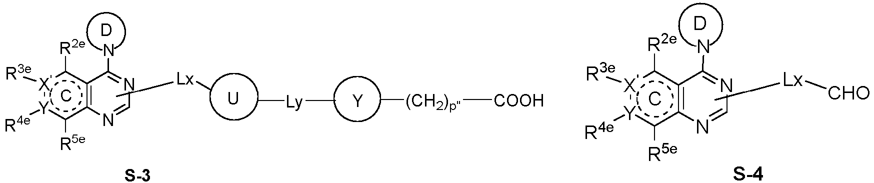 wherein ring C, ring D, R^{2e}, R^{3e}, R^{4e}, and R^{5e} are as defined and described in claim 3; Lx, ring U, Ly, and ring Y, as well as the linking relationships among them are as described in any one of claims 20-28; p" is 1, 2, 3, or 4.

50. The compound according to claim 49, wherein formula S-1 is formula S-1-1, formula S-2 is formula S-2-1, formula S-3 is formula S-3-1, and formula S-4 is formula S-4-1: 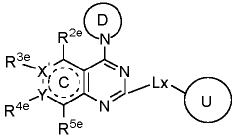 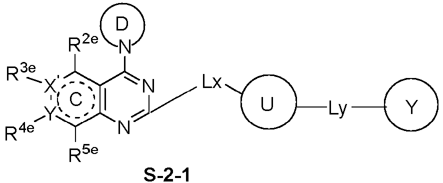 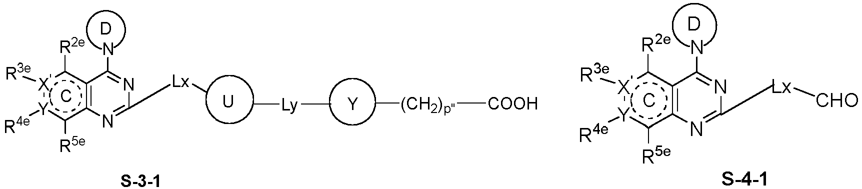 wherein ring C, ring D, R^{2e}, R^{3e}, R^{4e}, R^{5e}, Lx, ring U, Ly, ring Y, and p" are as defined and described in claim 49.

51. The compound of formula S-1, S-2, S-3, or S-4 according to claim 49, wherein the compound of formula S-1, S-2, S-3, or S-4 is selected from 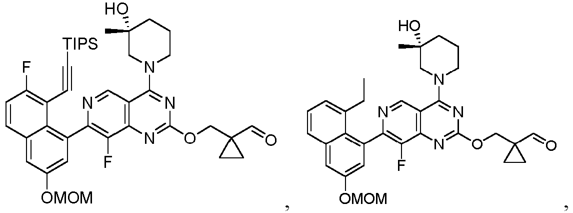 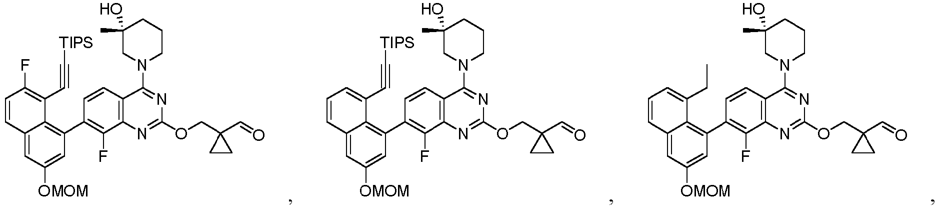 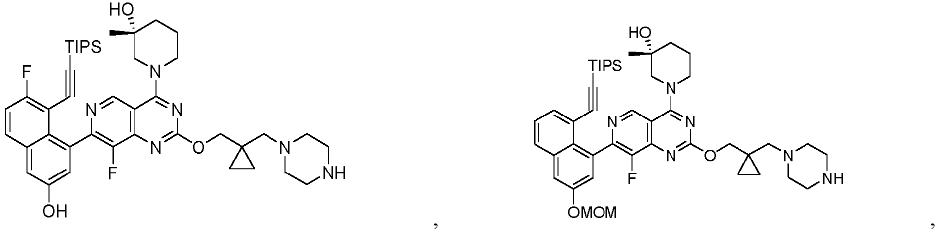 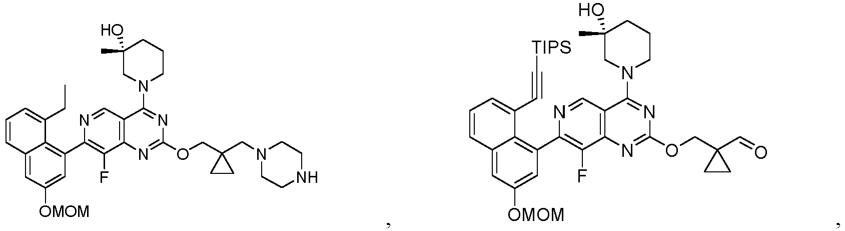 and
